(19) Europäisches Patentamt
European Patent Office
Office européen des brevets

(11) **EP 1 829 876 A1**

(12) **EUROPEAN PATENT APPLICATION**
published in accordance with Art. 158(3) EPC

(43) Date of publication:
**05.09.2007 Bulletin 2007/36**

(21) Application number: **05809668.6**

(22) Date of filing: **24.11.2005**

(51) Int Cl.:
***C07D 471/06*** *(2006.01)* ***A61K 31/4375*** *(2006.01)*
***A61K 31/444*** *(2006.01)* ***A61K 31/4545*** *(2006.01)*
***A61K 31/455*** *(2006.01)* ***A61K 31/496*** *(2006.01)*
***A61K 31/497*** *(2006.01)* ***A61K 31/513*** *(2006.01)*
***A61K 31/5377*** *(2006.01)* ***A61K 45/00*** *(2006.01)*
***A61P 1/02*** *(2006.01)* ***A61P 1/04*** *(2006.01)*
***A61P 1/08*** *(2006.01)* ***A61P 1/16*** *(2006.01)*
***A61P 1/18*** *(2006.01)* ***A61P 3/14*** *(2006.01)*
***A61P 5/00*** *(2006.01)* ***A61P 7/02*** *(2006.01)*
***A61P 7/06*** *(2006.01)* ***A61P 7/12*** *(2006.01)*
***A61P 9/00*** *(2006.01)*

(86) International application number:
**PCT/JP2005/021522**

(87) International publication number:
**WO 2006/057270 (01.06.2006 Gazette 2006/22)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **26.11.2004 JP 2004342042**

(71) Applicant: **Asahi Kasei Pharma Corporation**
**Tokyo 101-8481 (JP)**

(72) Inventors:
- **YAMADA, Rintaro**
  **4170801 (JP)**
- **SETO, Minoru**
  **4170855 (JP)**

(74) Representative: **Forstmeyer, Dietmar et al**
**BOETERS & LIECK**
**Oberanger 32**
**80331 München (DE)**

(54) **NITROGENEOUS TRICYCLIC COMPOUND**

(57) A novel compound represented by the following formula (1) or a salt thereof [wherein $R^1$, $R^5$, $R^6$, $R^7$, and $R^8$ represent hydrogen atom, a halogen atom, hydroxyl group, an alkyl group, an alkenyl group and the like; $X^1 \cdots X^2$ represents $-CH(R^2)-CH(R^3)^-$, $-CH(R^2)-CH(R^3)-CH(R^4)-$, $-C(R^2)=C(R^3)-$, or $-C(R^2)=C(R^3)-CH(R^4)-$ ($R^2$, $R^3$, and $R^4$ represent hydrogen atom, or an alkyl group); $A^1$, $A^{11}$, $A^2$, and $A^{21}$ represent hydrogen atom, or an alkyl group; Y represents $-CH(A^3)-$, $-CH(A^3)-C(A^4)(A^{41})-$, $-CH(A^3)-C(A^4)(A^{41})-C(A^5)(A^{51})-$, or a single bond ($A^3$, $A^4$, $A^{41}$, $A^5$, and $A^{51}$ represent hydrogen atom, or an alkyl group), and Z represents hydroxyl group, or $-N(A^6)(A^{61})$ (As represents hydrogen atom, or an alkyl group, and $A^{61}$ represents hydrogen atom, an alkyl group, a substituted alkyl group and the like)], having an action of potently inhibiting phosphorylation of myosin regulatory light chain.

EP 1 829 876 A1

[Formula 1]

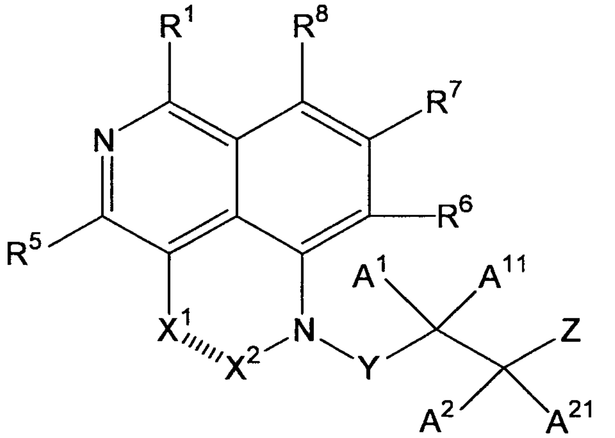

(1)

## Description

[Technical Field]

The present invention relates to a novel nitrogen-containing tricyclic compound or a salt thereof, and a medicament comprising said nitrogen-containing tricyclic compound or a salt thereof as an active ingredient.

[Background Art]

Movements of cells include contraction, migration, release, aggregation and the like, and phosphorylation of the myosin regulatory light chain is important for these cell movements. The myosin regulatory light chain is a subunit having a molecular weight of 20 kDa and constituting myosin, which exists in smooth muscle cells and various non-muscle cells such as neutrophils, leukocytes, platelets and nerve cells of warm-blooded animals (Barany, K., et al., Biochemistry of Smooth Muscle Contraction, pp.21-35, 1996). Myosin existing in smooth muscle cells and various non-muscle cells such as neutrophils, leukocytes, platelets and nerve cells of warm-blooded animals is constituted by a myosin heavy chain subunit having a molecular weight of about 200 kDa, the myosin regulatory light chain subunit having a molecular weight of about 20 kDa, and a myosin constitutive light chain subunit having a molecular weight of about 17 kDa. The myosin regulatory light chain is mainly phosphorylated by the myosin light chain kinase to increase the activity of myosin ATPase existing in the myosin heavy chain subunit (Barany, M., et al., Biochemistry of Smooth Muscle Contraction, pp.321-339, 1996).

It is known that the activated myosin having the increased ATPase activity becomes possible to interact with actin and activates movement apparatuses of cytoskeleton to activate cell movements. That is, it is known that activation of myosin relates to cell contraction (Kamm, K., et al., Annu. Rev. Physiol., 51, pp.299-313, 1989). It is also known that activation of myosin relates to change of cell morphology (Schmidt, J.T. et al., J, Neurobiol., 52 (3), pp.175-188, 2002). It is known that activation of myosin relates to cell migration (Niggli, V., FEBS Lett., 445, pp.69-72, 1999). Further, it is known that activation of myosin relates to cell release (Kitani, S., et al., Biochem. Biophys. Res. Commun., 183, pp. 48-54, 1992). It is further known that activation of myosin relates to cell aggregation (Itoh, K., et al., Biochim. Biophys. Acta., 1136, pp.52-56, 1992). It is also known that activation of myosin relates to cell apoptosis (Mills, J.C. et al., J. Cell Biol., Vol. 140, No. 3, pp.627-636, 1998). Based on these findings, it is considered that an agent which inhibits the phosphorylation of the myosin regulatory light chain suppresses cell contraction, regulates change of cell morphology, suppresses cell migration, suppresses cell release, suppresses cell aggregation and suppresses cell apoptosis.

Cell contraction is deeply involved in diseases relating to contraction of various smooth muscle layers. Examples of such diseases include, for example, hypertension (Samlyo, A.P., et al., Rev. Physiol. Biochem. Pharmacol., Vol. 134, pp.209-234, 1999), angina pectoris (Shimokawa et al., Cardiovasc. Res., Vol. 43, No. 4, pp.1029-1039, 1999; Satoh, H., et al., Jpn. J. Pharmacol., 79 (suppl.), p.211, 1999), cerebral vascular spasm (M. Satoh et al., the 57th General Meeting of Japan Neurosurgical Society, Collection of Abstracts, 153, 1998; N. Ono et al., Pharmacol. Ther., Vol. 82, No. 2-3, pp.123-131, 1991; Shimokawa et al., Cardiovasc. Res., Vol. 43, No. 4, pp.1029-1039, 1999), erectile dysfunction (Andersson, KE. et al., World J. Vrol., 15, pp.14-20, 1997), bronchial asthma (K. Iidzuka, Allergy, 47, 943, 1998; K. Iidzuka et al., Jpn. J. Respirology Society, 37, 196, 1999) and the like.

Change of cell morphology is deeply involved in diseases relating to morphological change of various cells. Examples of the diseases relating to change of cell morphology include, for example, various nerve dysfunctions as those relating to nerve cells. As the nerve dysfunctions, for example, neural damages caused by trauma, neurodegenerative diseases such as Alzheimer's disease, Parkinson's disease, diabetic retinopathy, glaucoma and the like can be exemplified (Arakawa, Y., et al., BIO Clinica, 17 (13), pp.26-28, 2002). Further, cell migration is deeply involved in diseases relating to migration of various cells. Examples of such diseases include, for example, cancer invasion and metastasis (Itoh, K. et al., Nat. Med., Vol. 5, No. 2, pp.221-5, 1999; Keely, P. et al., Trends Cell Biol., Vol. 8, No. 3, pp. 101-6, 1998), nephritis (Fujimoto, O. et al., Journal of Japanese Society of Internal Medicine, 88 (1), pp.148-58, 1998) and the like.

Furthermore, it is considered that cell release is deeply involved in various allergies and the like (Keane-Myers A. et al., Curr. Allergy Asthma Rep., 1(6):550-557, 2001), and further, cell aggregation is considered to be deeply involved in thrombosis and the like (Nakai, K. et al., Blood, Vol. 90, No. 10, pp.3736-42., 1997). Further, it is known that cell apoptosis is involved in neurodegenerative diseases such as Alzheimer's disease, Parkinson's disease and glaucoma, viral diseases, hepatic diseases and the like (Thompson, C.B., Science, Vol. 267, pp.1456-1462, 1995).
Based on these findings, it is considered that the inhibitor of the phosphorylation of myosin regulatory light chain of the present invention, which is an agent inhibiting the phosphorylation of the myosin regulatory light chain, is useful as an active ingredient of a medicament for prophylactic and/or therapeutic treatment of a disease relating to cell contraction, disease relating to change of cell morphology, disease relating to cell migration, disease relating to cell release, disease relating to cell aggregation, and/or disease relating to cell apoptosis.

As agents that inhibit phosphorylation of the myosin regulatory light chain, isoquinoline derivatives are known. It has been reported that 1-(5-isoquinolinesulfonyl)-2-methylpiperazine (H-7) inhibits phosphorylation of the myosin regulatory light chain of mesenteric artery (Suzuki, A. et al., Br. J. Pharmacol., 109, pp.703-712, 1993), iris smooth muscle (Howe, P.H. et al., Biochem J., 255, pp.423-429, 1988), and astrocyte (Mobley P.L., et al., Exp. Cell Res., 214, pp.55-66, 1994). 5-Substituted isoquinoline derivatives that inhibit phosphorylation of the myosin regulatory light chain are also known (International Patent Publication No. 2004/009555). However, all of these compounds are bicyclic compounds.

[Disclosure of the Invention]

[Object to be Achieved by the Invention]

An object of the present invention is to provide a novel compound having an action of strongly inhibiting phosphorylation of the myosin regulatory light chain.

[Means for Achieving the Object]

In order to achieve the aforementioned object, the inventors of the present invention synthesized various novel compounds and studied pharmacological actions thereof. As a result, it was found that the compounds represented by the following formula (1) and salts thereof had the desired pharmacological action, and were useful as an active ingredient of a medicament for prophylactic and/or therapeutic treatment of diseases relating to cell contraction, those relating to change of cell morphology, those relating to cell migration, those relating to cell release, those relating to cell aggregation, and those relating to cell apoptosis. The compounds of the present invention are compounds having a tricyclic structure, and have a structure different from that of the isoquinoline derivatives disclosed in the above-mentioned publications (International Patent Publication No. 2004/009555; Suzuki, A. et al., Br. J. Pharmacol., 109, pp.703-712, 1993; Howe, P.H. et al., Biochem J., 255, pp.423-429, 1988; Mobley P.L., et al., Exp. Cell Res., 214, pp.55-66, 1994). The present invention was achieved on the basis of these findings.

The present invention thus provides the compounds and salts thereof described below.

1. A compound represented by the following formula (1) or a salt thereof:

[Formula 1]

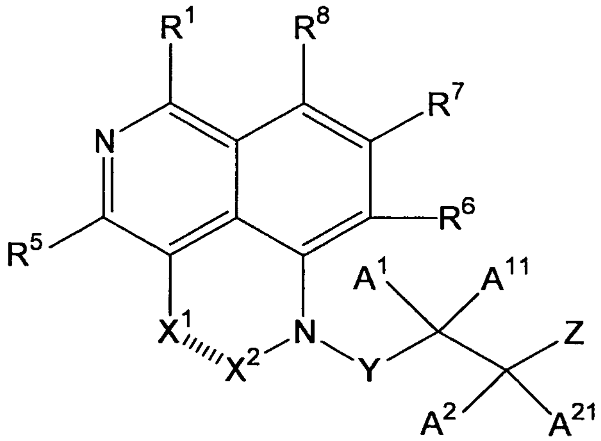

(1)

[wherein $R^1$, $R^5$, $R^6$, $R^7$, and $R^8$ independently represent hydrogen atom, a halogen atom, hydroxyl group, an alkyl group, an alkenyl group, an alkynyl group, an alkoxy group, amino group, an alkylamino group, or an arylamino group;
$X^1 \cdots X^2$ represents $-CH(R^2)-CH(R^3)-$, $-CH(R^2)-CH(R^3)-CH(R^4)-$, $-C(R^2)=C(R^3)-$, or $-C(R^2)=C(R^3)-CH(R^4)-$;
$R^2$, $R^3$, and $R^4$ independently represent hydrogen atom, or an alkyl group;
$A^1$, $A^{11}$, $A^2$, and $A^{21}$ independently represent hydrogen atom, or an alkyl group;
Y represents $-CH(A^3)-$, $-CH(A^3)-C(A^4)(A^{41})-$, $-CH(A^3)-C(A^4)(A^{41})-C(A^5)(A^{51})-$, or a single bond;
$A^3$, $A^4$, $A^{41}$, $A^5$, and $A^{51}$ independently represent hydrogen atom, or an alkyl group;
Z represents hydroxyl group, or $-N(A^6)(A^{61})$;
$A^6$ represents hydrogen atom, or an alkyl group, $A^{61}$ represents hydrogen atom, an alkyl group, an alkyl group

substituted with an aryl group, an alkyl group substituted with an aromatic heterocyclic group, an alkyl group substituted with carboxyl group, an alkyl group substituted with cyano group, an alkyl group substituted with hydroxyl group, an alkyl group substituted with an alkoxy group, an alkyl group substituted with a substituted alkoxy group, an alkyl group substituted with amino group, an alkyl group substituted with an alkylamino group, an alkyl group substituted with aminocarbonyl group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$, $-X^3-$ represents carbonyl group, $-SO_2-$, -C(=O)-O-, $-C(=O)-N(A^8)-$, or $-C(=S)-N(A^8)-$, $A^7$ represents hydrogen atom, an alkyl group, an alkyl group substituted with hydroxyl group, an alkyl group substituted with amino group, an alkyl group substituted with an alkylamino group, an alkyl group substituted with aminocarbonyl group, an alkyl group substituted with an acylamino group, or an alkyl group substituted with cyano group, $A^{71}$ and $A^8$ independently represent hydrogen atom, an alkyl group, an alkyl group substituted with an aryl group, an alkyl group substituted with a heterocyclic group, an alkyl group substituted with hydroxyl group, an alkyl group substituted with amino group, an alkyl group substituted with an alkylamino group, an alkyl group substituted with an alkoxy group, an alkyl group substituted with an acylamino group, a cycloalkyl group, an aryl group, or an aromatic heterocyclic group, or $A^7$ and $A^{71}$ may combine together to become an alkylene group, or an alkylene group substituted with an alkyl group to form a ring, or $A^{71}$ and $A^8$ may combine together to become an alkylene group, -alkylene-O-alkylene-, -alkylene-NH-alkylene-, or -alkylene-N (alkyl)-alkylene- to form a ring (wherein said alkylene moiety may be substituted with an alkyl group); and groups in each of one or more combinations selected from the group consisting of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ may bind to each other to form a 5- or 6-membered ring].

2. The compound or a salt thereof according to 1, wherein a compound represented by the formula (1) mentioned in 1, wherein:

$R^1$ is hydrogen atom, chlorine atom, or hydroxyl group,
R5, $R^6$, $R^7$, and $R^8$ are hydrogen atoms, and
$A^{61}$ is hydrogen atom, an alkyl group, an alkyl group substituted with an aryl group, an alkyl group substituted with carboxyl group, an alkyl group substituted with cyano group, an alkyl group substituted with hydroxyl group, an alkyl group substituted with an alkoxy group, an alkyl group substituted with amino group, an alkyl group substituted with an alkylamino group, an alkyl group substituted with aminocarbonyl group, or an alkyl group of which end is substituted with $N(A^7)(-X^3-A^{71})$ (where $-X^3-$ is carbonyl group, and $A^{71}$ is an alkyl group, or an alkyl group substituted with an aryl group) is excluded.

3. The compound or a salt thereof according to 1, wherein a compound represented by the formula (1) mentioned in 1, wherein:

$R^1$ is hydrogen atom, chlorine atom, or hydroxyl group,
$R^5$, $R^6$, $R^7$, and $R^8$ are hydrogen atoms, and
$A^{61}$ is hydrogen atom, an alkyl group, an alkyl group substituted with an aryl group (wherein the aryl group is an unsubstituted aryl group, or an aryl group substituted with one or more substituents selected from the group consisting of an alkyl group, and a halogen atom), an alkyl group substituted with carboxyl group, an alkyl group substituted with cyano group, an alkyl group substituted with hydroxyl group, an alkyl group substituted with an alkoxy group, an alkyl group substituted with amino group, an alkyl group substituted with an alkylamino group, an alkyl group substituted with aminocarbonyl group, or an alkyl group of which end is substituted with $N(A^7)(-X^3-A^{71})$(wherein $-X^3-$ is carbonyl group, and $A^{71}$ is an alkyl group, or an alkyl group substituted with an aryl group) is excluded.

4. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group.
5. The compound or a salt thereof according to 1, wherein $R^1$ is fluorine atom, bromine atom, iodine atom, an alkyl

group, an alkenyl group, an alkynyl group, an alkoxy group, amino group, an alkylamino group, or an arylamino group, and $X^1 \cdots X^2$ is $-CH(R^2)-CH(R^3)-$, or $-C(R^2)=C(R^3)-$.

6. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, and $X^1 \cdots X^2$ is $-CH(R^2)-CH(R^3)-$.

7. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, and $X^1 \cdots X^2$ is ethylene group.

8. The compound or a salt thereof according to 1, wherein $R^1$ is hydrogen atom, chlorine atom, or hydroxyl group, and any one of $R^5$, $R^6$, $R^7$, and $R^8$ is not hydrogen atom.

9. The compound or a salt thereof according to 1, wherein $R^1$ is hydrogen atom, chlorine atom, or hydroxyl group, any one of $R^5$, $R^6$, $R^7$, and $R^8$ is not hydrogen atom, and $X^1 \cdots X^2$ is $-CH(R^2)-CH(R^3)-$, or $-C(R^2)=C(R^3)-$.

10. The compound or a salt thereof according to 1, wherein $R^1$ is hydrogen atom, chlorine atom, or hydroxyl group, any one of $R^5$, $R^6$, $R^7$, and $R^8$ is not hydrogen atom, and $X^1 \cdots X^2$ is $-CH(R^2)-CH(R^3)-$.

11. The compound or a salt thereof according to 1, wherein $R^1$ is hydrogen atom, chlorine atom, or hydroxyl group, any one of $R^5$, $R^6$, $R^7$, and $R^8$ is not hydrogen atom, and $X^1 \cdots X^2$ is ethylene group.

12. The compound or a salt thereof according to 1, wherein $R^1$ is hydrogen atom, or hydroxyl group, and any one of $R^5$, $R^6$, $R^7$, and $R^8$ is not hydrogen atom.

13. The compound or a salt thereof according to 1, wherein $R^1$ is hydrogen atom, or hydroxyl group, any one of $R^5$, $R^6$, $R^7$, and $R^8$ is not hydrogen atom, and $X^1 \cdots X^2$ is $-CH(R^2)-CH(R^3)-$, or $-C(R^2)=C(R^3)-$.

14. The compound or a salt thereof according to 1, wherein $R^1$ is hydrogen atom, or hydroxyl group, any one of $R^5$, $R^6$, $R^7$, and $R^8$ is not hydrogen atom, and $X^1 \cdots X^2$ is $-CH(R^2)-CH(R^3)-$.

15. The compound or a salt thereof according to 1, wherein $R^1$ is hydrogen atom, or hydroxyl group, any one of $R^5$, $R^6$, $R^7$, and $R^8$ is not hydrogen atom, and $X^1 \cdots X^2$ is ethylene group.

16. The compound or a salt thereof according to 1, wherein $R^1$ is hydrogen atom, chlorine atom, or hydroxyl group, and all of $R^5$, $R^6$, $R^7$, and $R^8$ are hydrogen atoms

17. The compound or a salt thereof according to 1, wherein $R^1$ is hydrogen atom, chlorine atom, or hydroxyl group, all of $R^5$, $R^6$, $R^7$, and $R^8$ are hydrogen atoms, and $X^1 \cdots X^2$ is $-CH(R^2)-CH(R^3)-$, or $-C(R^2)=C(R^3)-$.

18. The compound or a salt thereof according to 1, wherein $R^1$ is hydrogen atom, chlorine atom, or hydroxyl group, all of $R^5$, $R^6$, $R^7$, and $R^8$ are hydrogen atoms, and $X^1 \cdots X^2$ is $-CH(R^2)-CH(R^3)-$.

19. The compound or a salt thereof according to 1, wherein $R^1$ is hydrogen atom, chlorine atom, or hydroxyl group, all of $R^5$, $R^6$, $R^7$, and $R^8$ are hydrogen atoms, and $X^1 \cdots X^2$ is ethylene group.

20. The compound or a salt thereof according to 1, wherein $R^1$ is hydrogen atom, or hydroxyl group, and all of $R^5$, $R^6$, $R^7$, and $R^8$ are hydrogen atoms

21. The compound or a salt thereof according to 1, wherein $R^1$ is hydrogen atom, or hydroxyl group, all of $R^5$, $R^6$, $R^7$, and $R^8$ are hydrogen atoms, and $X^1 \cdots X^2$ is $-CH(R^2)-CH(R^3)-$, or $-C(R^2)=C(R^3)-$.

22. The compound or a salt thereof according to 1, wherein $R^1$ is hydrogen atom, or hydroxyl group, all of $R^5$, $R^6$, $R^7$, and $R^8$ are hydrogen atoms, and $X^1 \cdots X^2$ is $-CH(R^2)-CH(R^3)-$.

23. The compound or a salt thereof according to 1, wherein $R^1$ is hydrogen atom, or hydroxyl group, all of $R^5$, $R^6$, $R^7$, and $R^8$ are hydrogen atoms, and $X^1 \cdots X^2$ is ethylene group.

24. The compound or a salt thereof according to 1, wherein $A^{61}$ is an alkyl group substituted with an aryl group, an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents carbonyl group, $-SO_2-$, $-C(=O)-O-$, $-C(=O)-N(A^8)-$, or $-C(=S)-N(A^8)-$).

**25.** The compound or a salt thereof according to 1, wherein $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents $-SO_2-$, -C(=O)-O-, $-C(=O)-N(A^8)-$, or $-C(=S)-N(A^8)-$).

26. The compound or a salt thereof according to 1, wherein $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom).

27. The compound or a salt thereof according to 1, wherein $A^{61}$ is an alkyl group of which end is substituted with $N(A^7)(-X^3-A^{71})$ (wherein $-X^3-$ represents $-SO_2-$, -C(=O)-O-, $-C(=O)-N(A^8)-$, or $-C(=S)-N(A^8)-$).

28. The compound or a salt thereof according to 1, wherein $A^{61}$ is an alkyl group of which end is substituted with $N(A^7)(-X^3-A^{71})$ (wherein $-X^3-$ represents carbonyl group).

29. The compound or a salt thereof according to 1, wherein $A^{61}$ is an alkyl group substituted with an aromatic heterocyclic group.

30. The compound or a salt thereof according to 1, wherein $A^{61}$ is an alkyl group substituted with a substituted alkoxy group.

31. The compound or a salt thereof according to 1, wherein $A^{61}$ is an alkyl group substituted with an alkylaminocarbonyloxy group.

32. The compound or a salt thereof according to 1, wherein $A^{61}$ is an alkyl group substituted with a cycloalkylaminocarbonyloxy group.

33. The compound or a salt thereof according to 1, wherein $A^{61}$ is an alkyl group substituted with an arylaminocarbonyloxy group.

34. The compound or a salt thereof according to 1, wherein $A^{61}$ is an alkyl group substituted with mercapto group.

35. The compound or a salt thereof according to 1, wherein $A^{61}$ is an alkyl group substituted with an alkylthio group.

36. The compound or a salt thereof according to 1, wherein $A^{61}$ is an alkyl group substituted with an acylthio group.

37. The compound or a salt thereof according to 1, wherein $A^{61}$ is a cycloalkyl group. 38. The compound or a salt thereof according to 1, wherein $A^{61}$ is a substituted cycloalkyl group.

39. The compound or a salt thereof according to 1, wherein $A^{61}$ is carbamimidoyl group. 40. The compound or a salt thereof according to 1, wherein $A^{61}$ is an alkylcarbonyl group.

41. The compound or a salt thereof according to 1, wherein $A^{61}$ is an arylcarbonyl group.

42. The compound or a salt thereof according to 1, wherein $A^{61}$ is a non-aromatic heterocyclic group.

43. The compound or a salt thereof according to 1, wherein $A^{61}$ is a substituted non-aromatic heterocyclic group.

44. The compound or a salt thereof according to 1, wherein $R^1$ is hydrogen atom, chlorine atom, or hydroxyl group, and $A^{61}$ is an alkyl group substituted with an aryl group, an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents carbonyl group, $-SO_2-$, -C(=O)-O-, $-C(=O)-N(A^8)-$, or $-C(=S)-N(A^8)-$).

45. The compound or a salt thereof according to 1, wherein $R^1$ is hydrogen atom, chlorine atom, or hydroxyl group,

and $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents $-SO_2-$, -C(=O)-O-, -C(=O)-N($A^8$)-, or -C(=S)-N($A^8$)-).

46. The compound or a salt thereof according to 1, wherein $R^1$ is hydrogen atom, chlorine atom, or hydroxyl group, and $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom).

47. The compound or a salt thereof according to 1, wherein $R^1$ is hydrogen atom, chlorine atom, or hydroxyl group, and $A^{61}$ is an alkyl group of which end is substituted with $N(A^7)(-X^3-A^{71})$ (wherein $-X^3-$ represents $-SO_2-$, -C(=O)-O-, -C(=O)-N($A^8$)-, or -C(=S)-N($A^8$)-).

48. The compound or a salt thereof according to 1, wherein $R^1$ is hydrogen atom, chlorine atom, or hydroxyl group, and $A^{61}$ is an alkyl group of which end is substituted with $N(A^7)(-X^3-A^{71})$ (wherein $-X^3-$ represents carbonyl group).

49. The compound or a salt thereof according to 1, wherein $R^1$ is hydrogen atom, chlorine atom, or hydroxyl group, and $A^{61}$ is an alkyl group substituted with an aromatic heterocyclic group.

50. The compound or a salt thereof according to 1, wherein $R^1$ is hydrogen atom, chlorine atom, or hydroxyl group, and $A^{61}$ is an alkyl group substituted with a substituted alkoxy group.

51. The compound or a salt thereof according to 1, wherein $R^1$ is hydrogen atom, chlorine atom, or hydroxyl group, and $A^{61}$ is an alkyl group substituted with an alkylaminocarbonyloxy group.

52. The compound or a salt thereof according to 1, wherein $R^1$ is hydrogen atom, chlorine atom, or hydroxyl group, and $A^{61}$ is an alkyl group substituted with a cycloalkylaminocarbonyloxy group.

53. The compound or a salt thereof according to 1, wherein $R^1$ is hydrogen atom, chlorine atom, or hydroxyl group, and $A^{61}$ is an alkyl group substituted with an arylaminocarbonyloxy group.

54. The compound or a salt thereof according to 1, wherein $R^1$ is hydrogen atom, chlorine atom, or hydroxyl group, and $A^{61}$ is an alkyl group substituted with mercapto group.

55. The compound or a salt thereof according to 1, wherein $R^1$ is hydrogen atom, chlorine atom, or hydroxyl group, and $A^{61}$ is an alkyl group substituted with an alkylthio group.

56. The compound or a salt thereof according to 1, wherein $R^1$ is hydrogen atom, chlorine atom, or hydroxyl group, and $A^{61}$ is an alkyl group substituted with an acylthio group.

57. The compound or a salt thereof according to 1, wherein $R^1$ is hydrogen atom, chlorine atom, or hydroxyl group, and $A^{61}$ is a cycloalkyl group.

58. The compound or a salt thereof according to 1, wherein $R^1$ is hydrogen atom, chlorine atom, or hydroxyl group, and $A^{61}$ is a substituted cycloalkyl group.

59. The compound or a salt thereof according to 1, wherein $R^1$ is hydrogen atom, chlorine atom, or hydroxyl group, and $A^{61}$ is carbamimidoyl group.

60. The compound or a salt thereof according to 1, wherein $R^1$ is hydrogen atom, chlorine atom, or hydroxyl group, and $A^{61}$ is an alkylcarbonyl group.

61. The compound or a salt thereof according to 1, wherein $R^1$ is hydrogen atom, chlorine atom, or hydroxyl group, and $A^{61}$ is an arylcarbonyl group.

62. The compound or a salt thereof according to 1, wherein $R^1$ is hydrogen atom, chlorine atom, or hydroxyl group, and $A^{61}$ is a non-aromatic heterocyclic group.

63. The compound or a salt thereof according to 1, wherein $R^1$ is hydrogen atom, chlorine atom, or hydroxyl group, and $A^{61}$ is a substituted non-aromatic heterocyclic group.

64. The compound or a salt thereof according to 1, wherein $R^1$ is hydrogen atom, or hydroxyl group, and $A^{61}$ is an alkyl group substituted with an aryl group, an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents carbonyl group, $-SO_2-$, -C(=O)-O-, $-C(=O)-N(A^8)-$, or $-C(=S)-N(A^8)-$).

65. The compound or a salt thereof according to 1, wherein $R^1$ is hydrogen atom, or hydroxyl group, and $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents $-SO_2-$, -C(=O)-O-, $-C(=O)-N(A^8)-$, or $-C(=S)-N(A^8)-$).

66. The compound or a salt thereof according to 1, wherein $R^1$ is hydrogen atom, or hydroxyl group, and $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom).

67. The compound or a salt thereof according to 1, wherein $R^1$ is hydrogen atom, or hydroxyl group, and $A^{61}$ is an alkyl group of which end is substituted with $N(A^7)(-X^3-A^{71})$ (wherein $-X^3-$ represents $-SO_2-$, -C(=O)-O-, $-C(=O)-N(A^8)-$, or $-C(=S)-N(A^8)-$).

68. The compound or a salt thereof according to 1, wherein $R^1$ is hydrogen atom, or hydroxyl group, and $A^{61}$ is an alkyl group of which end is substituted with $N(A^7)(-X^3-A^{71})$ (wherein $-X^3-$ represents carbonyl group).

69. The compound or a salt thereof according to 1, wherein $R^1$ is hydrogen atom, or hydroxyl group, and $A^{61}$ is an alkyl group substituted with an aromatic heterocyclic group.

70. The compound or a salt thereof according to 1, wherein $R^1$ is hydrogen atom, or hydroxyl group, and $A^{61}$ is an alkyl group substituted with a substituted alkoxy group. 71. The compound or a salt thereof according to 1, wherein $R^1$ is hydrogen atom, or hydroxyl group, and $A^{61}$ is an alkyl group substituted with an alkylaminocarbonyloxy group.

72. The compound or a salt thereof according to 1, wherein $R^1$ is hydrogen atom, or hydroxyl group, and $A^{61}$ is an alkyl group substituted with a cycloalkylaminocarbonyloxy group.

73. The compound or a salt thereof according to 1, wherein $R^1$ is hydrogen atom, or hydroxyl group, and $A^{61}$ is an alkyl group substituted with an arylaminocarbonyloxy group.

74. The compound or a salt thereof according to 1, wherein $R^1$ is hydrogen atom, or hydroxyl group, and $A^{61}$ is an alkyl group substituted with mercapto group.

75. The compound or a salt thereof according to 1, wherein $R^1$ is hydrogen atom, or hydroxyl group, and $A^{61}$ is an alkyl group substituted with an alkylthio group.

76. The compound or a salt thereof according to 1, wherein $R^1$ is hydrogen atom, or hydroxyl group, and $A^{61}$ is an alkyl group substituted with an acylthio group.

77. The compound or a salt thereof according to 1, wherein $R^1$ is hydrogen atom, or hydroxyl group, and $A^{61}$ is a cycloalkyl group.

78. The compound or a salt thereof according to 1, wherein $R^1$ is hydrogen atom, or hydroxyl group, and $A^{61}$ is a substituted cycloalkyl group.

79. The compound or a salt thereof according to 1, wherein $R^1$ is hydrogen atom, or hydroxyl group, and $A^{61}$ is carbamimidoyl group.

80. The compound or a salt thereof according to 1, wherein $R^1$ is hydrogen atom, or hydroxyl group, and $A^{61}$ is an alkylcarbonyl group.

81. The compound or a salt thereof according to 1, wherein $R^1$ is hydrogen atom, or hydroxyl group, and $A^{61}$ is an

arylcarbonyl group.

82. The compound or a salt thereof according to 1, wherein $R^1$ is hydrogen atom, or hydroxyl group, and $A^{61}$ is a non-aromatic heterocyclic group.

83. The compound or a salt thereof according to 1, wherein $R^1$ is hydrogen atom, or hydroxyl group, and $A^{61}$ is a substituted non-aromatic heterocyclic group.

84. The compound or a salt thereof according to 1, wherein $X^1 \cdots X^2$ is $-CH(R^2)-CH(R^3)-$, or $-C(R^2)=C(R^3)-$, and $A^{61}$ is an alkyl group substituted with an aryl group, an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents carbonyl group, $-SO_2-$, $-C(=O)-O-$, $-C(=O)-N(A^8)-$, or $-C(=S)-N(A^8)-$).

85. The compound or a salt thereof according to 1, wherein $X^1 \cdots X^2$ is $-CH(R^2)-CH(R^3)-$, or $-C(R^2)=C(R^3)-$, and $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents $-SO_2-$, $-C(=O)-O-$, $-C(=O)-N(A^8)-$, or $-C(=S)-N(A^8)-$).

86. The compound or a salt thereof according to 1, wherein $X^1 \cdots X^2$ is $-CH(R^2)-CH(R^3)-$, or $-C(R^2)=C(R^3)-$, and $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom).

87. The compound or a salt thereof according to 1, wherein $X^1 \cdots X^2$ is $-CH(R^2)-CH(R^3)-$, or $-C(R^2)=C(R^3)-$, and $A^{61}$ is an alkyl group of which end is substituted with $N(A^7)(-X^3-A^{71})$ (wherein $-X^3-$ represents $-SO_2-$, $-C(=O)-O-$, $-C(=O)-N(A^8)-$, or $-C(=S)-N(A^8)-$).

88. The compound or a salt thereof according to 1, wherein $X^1 \cdots X^2$ is $-CH(R^2)-CH(R^3)-$, or $-C(R^2)=C(R^3)-$, and $A^{61}$ is an alkyl group of which end is substituted with $N(A^7)(-X^3-A^{71})$ (wherein $-X^3-$ represents carbonyl group).

89. The compound or a salt thereof according to 1, wherein $X^1 \cdots X^2$ is $-CH(R^2)-CH(R^3)-$, or $-C(R^2)=C(R^3)-$, and $A^{61}$ is an alkyl group substituted with an aromatic heterocyclic group.

90. The compound or a salt thereof according to 1, wherein $X^1 ... X^2$ is $-CH(R^2)-CH(R^3)-$, or $-C(R^2)=C(R3)-$, and $A^{61}$ is an alkyl group substituted with a substituted alkoxy group.

91. The compound or a salt thereof according to 1, wherein $X^1 \cdots X^2$ is $-CH(R^2)-CH(R^3)-$, or $-C(R^2)=C(R^3)-$, and $A^{61}$ is an alkyl group substituted with an alkylaminocarbonyloxy group.

92. The compound or a salt thereof according to 1, wherein $X^1 \cdots X^2$ is $-CH(R^2)-CH(R^3)-$, or $-C(R^2)=C(R^3)-$, and $A^{61}$ is an alkyl group substituted with a cycloalkylaminocarbonyloxy group.

**93.** The compound or a salt thereof according to 1, wherein $X^1 \cdots X^2$ is $-CH(R^2)-CH(R^3)-$, or $-C(R^2)=C(R^3)-$, and $A^{61}$ is an alkyl group substituted with an arylaminocarbonyloxy group.

94. The compound or a salt thereof according to 1, wherein $X^1 \cdots X^2$ is $-CH(R^2)-CH(R^3)-$, or $-C(R^2)=C(R^3)-$, and $A^{61}$ is an alkyl group substituted with mercapto group.

95. The compound or a salt thereof according to 1, wherein $X^1 \cdots X^2$ is $-CH(R^2)-CH(R^3)-$, or $-C(R^2)=C(R^3)-$, and $A^{61}$ is an alkyl group substituted with an alkylthio group.

96. The compound or a salt thereof according to 1, wherein $X^1 \cdots X^2$ is $-CH(R^2)-CH(R^3)-$, or $-C(R^2)=C(R^3)-$, and $A^{61}$

is an alkyl group substituted with an acylthio group.
97. The compound or a salt thereof according to 1, wherein $X^1 \cdots X^2$ is $-CH(R^2)-CH(R^3)-$, or $-C(R^2)=C(R^3)-$, and $A^{61}$ is a cycloalkyl group.
98. The compound or a salt thereof according to 1, wherein $X^1 \cdots X^2$ is $-CH(R^2)-CH(R^3)-$, or $-C(R^2)=C(R^3)-$, and $A^{61}$ is a substituted cycloalkyl group.
99. The compound or a salt thereof according to 1, wherein $X^1 ... X^2$ is $-CH(R^2)-CH(R^3)-$, or $-C(R^2)=C(R^3)-$, and $A^{61}$ is carbamimidoyl group.
100. The compound or a salt thereof according to 1, wherein $X^1 ... X^2$ is $-CH(R^2)-CH(R^3)-$, or $-C(R^2)=C(R^3)-$, and $A^{61}$ is an alkylcarbonyl group.

101. The compound or a salt thereof according to 1, wherein $X^1 ... X^2$ is $-CH(R^2)-CH(R^3)-$, or $-C(R^2)=C(R^3)^-$, and $A^{61}$ is an arylcarbonyl group.
102. The compound or a salt thereof according to 1, wherein $X^1 \cdots X^2$ is $-CH(R^2)-CH(R^3)-$, or $-C(R^2)=C(R^3)^-$; and $A^{61}$ is a non-aromatic heterocyclic group.
103. The compound or a salt thereof according to 1, wherein $X^1 ... X^2$ is $-CH(R^2)-CH(R^3)-$, or $-C(R^2)=C(R^3)-$, and $A^{61}$ is a substituted non-aromatic heterocyclic group.
104. The compound or a salt thereof according to 1, wherein $X^1 \cdots X^2$ is $-CH(R^2)-CH(R^3)-$, and $A^{61}$ is an alkyl group substituted with an aryl group, an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents carbonyl group, $-SO_2-$, $-C(=O)-O-$, $-C(=O)-N(A^8)-$, or $-C(=S)-N(A^8)-$).

105. The compound or a salt thereof according to 1, wherein $X^1 \cdots X^2$ is $-CH(R^2)-CH(R^3)-$, and $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents $-SO_2-$, $-C(=O)-O-$, $-C(=O)-N(A^8)-$, or $-C(=S)-N(A^8)-$).
106. The compound or a salt thereof according to 1, wherein $X^1 \cdots X^2$ is $-CH(R^2)-CH(R^3)-$, and $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom).

107. The compound or a salt thereof according to 1, wherein $X^1 \cdots X^2$ is $-CH(R^2)-CH(R^3)-$, and $A^{61}$ is an alkyl group of which end is substituted with $N(A^7)(-X^3-A^{71})$ (wherein $-X^3-$ represents $-SO_2-$, $-C(=O)-O-$, $-C(=O)-N(A^8)-$, or $-C(=S)-N(A^8)-$).
108. The compound or a salt thereof according to 1, wherein $X^1 \cdots X^2$ is $-CH(R^2)-CH(R^3)-$, and $A^{61}$ is an alkyl group of which end is substituted with $N(A^7)(-X^3-A^{71})$ (wherein $-X^3-$ represents carbonyl group).
109. The compound or a salt thereof according to 1, wherein $X^1 \cdots X^2$ is $-CH(R^2)-CH(R^3)-$, and $A^{61}$ is an alkyl group substituted with an aromatic heterocyclic group.
110. The compound or a salt thereof according to 1, wherein $X^1 \cdots X^2$ is $-CH(R^2)-CH(R^3)-$, and $A^{61}$ is an alkyl group substituted with a substituted alkoxy group.
111. The compound or a salt thereof according to 1, wherein $X^1 \cdots X^2$ is $-CH(R^2)-CH(R^3)-$, and $A^{61}$ is an alkyl group substituted with an alkylaminocarbonyloxy group.
112. The compound or a salt thereof according to 1, wherein $X^1 \cdots X^2$ is $-CH(R^2)-CH(R^3)-$, and $A^{61}$ is an alkyl group

substituted with a cycloalkylaminocarbonyloxy group.
113. The compound or a salt thereof according to 1, wherein $X^1\cdots X^2$ is -CH($R^2$)-CH($R^3$)-, and $A^{61}$ is an alkyl group substituted with an arylaminocarbonyloxy group.
114. The compound or a salt thereof according to 1, wherein $X^1\cdots X^2$ is -CH($R^2$)-CH($R^3$)-, and $A^{61}$ is an alkyl group substituted with mercapto group.
115. The compound or a salt thereof according to 1, wherein $X^1 ... X^2$ is -CH($R^2$)-CH($R^3$)-, and $A^{61}$ is an alkyl group substituted with an alkylthio group.

116. The compound or a salt thereof according to 1, wherein $X^1\cdots X^2$ is -CH($R^2$)-CH($R^3$)-, and $A^{61}$ is an alkyl group substituted with an acylthio group.
117. The compound or a salt thereof according to 1, wherein $X^1\cdots X^2$ is -CH($R^2$)-CH($R^3$)-, and $A^{61}$ is a cycloalkyl group.
118. The compound or a salt thereof according to 1, wherein $X^1\cdots X^2$ is -CH($R^2$)-CH($R^3$)-, and $A^{61}$ is a substituted cycloalkyl group.
119. The compound or a salt thereof according to 1, wherein $X^1\cdots X^2$ is -CH($R^2$)-CH($R^3$)-, and $A^{61}$ is carbamimidoyl group.
120. The compound or a salt thereof according to 1, wherein $X^1\cdots X^2$ is -CH($R^2$)-CH($R^3$)-, and $A^{61}$ is an alkylcarbonyl group.
121. The compound or a salt thereof according to 1, wherein $X^1\cdots X^2$ is -CH($R^2$)-CH($R^3$)-, and $A^{61}$ is an arylcarbonyl group.
122. The compound or a salt thereof according to 1, wherein $X^1\cdots X^2$ is CH($R^2$)-CH($R^3$)- , and $A^{61}$ is a non-aromatic heterocyclic group.
123. The compound or a salt thereof according to 1, wherein $X^1\cdots X^2$ is -CH($R^2$)-CH($R^3$)-, and $A^{61}$ is a substituted non-aromatic heterocyclic group.

124. The compound or a salt thereof according to 1, wherein $X^1\cdots X^2$ is ethylene group, and $A^{61}$ is an alkyl group substituted with an aryl group, an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with N($A^7$)(-$X^3$-$A^{71}$) (wherein -$X^3$- represents carbonyl group, -$SO_2$- -C(=O)-O-, -C(=O)-N($A^8$)-, or -C(=S)-N($A^8$)-).
125. The compound or a salt thereof according to 1, wherein $X^1 ... X^2$ is ethylene group, and $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with N($A^7$)(-$X^3$-$A^{71}$) (wherein -$X^3$- represents -$SO_2$-, -C(=O)-O-, -C(=O)-N($A^8$)-, or -C(=S)-N($A^8$)-).

126. The compound or a salt thereof according to 1, wherein $X^1\cdots X^2$ is ethylene group, and $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom).
127. The compound or a salt thereof according to 1, wherein $X^1\cdots X^2$ is ethylene group, and $A^{61}$ is an alkyl group of which end is substituted with N($A^7$)(-$X^3$-$A^{71}$) (wherein -$X^3$- represents -$SO_2$-, -C(=O)-O-, -C(=O)-N($A^8$)-, or -C(=S)-N ($A^8$)-).
128. The compound or a salt thereof according to 1, wherein $X^1\cdots X^2$ is ethylene group, and $A^{61}$ is an alkyl group of which end is substituted with N($A^7$)(-$X^3$-$A^{71}$) (wherein -$X^3$- represents carbonyl group).

129. The compound or a salt thereof according to 1, wherein $X^1 \cdots X^2$ is ethylene group, and $A^{61}$ is an alkyl group substituted with an aromatic heterocyclic group.
130. The compound or a salt thereof according to 1, wherein $X^1 \cdots X^2$ is ethylene group, and $A^{61}$ is an alkyl group substituted with a substituted alkoxy group.
131. The compound or a salt thereof according to 1, wherein $X^1 \cdots X^2$ is ethylene group, and $A^{61}$ is an alkyl group substituted with an alkylaminocarbonyloxy group.
132. The compound or a salt thereof according to 1, wherein $X^1 \cdots X^2$ is ethylene group, and $A^{61}$ is an alkyl group substituted with a cycloalkylaminocarbonyloxy group.

133. The compound or a salt thereof according to 1, wherein $X^1 \cdots X^2$ is ethylene group, and $A^{61}$ is an alkyl group substituted with an arylaminocarbonyloxy group.
134. The compound or a salt thereof according to 1, wherein $X^1 \cdots X^2$ is ethylene group, and $A^{61}$ is an alkyl group substituted with mercapto group.
135. The compound or a salt thereof according to 1, wherein $X^1 \cdots X^2$ is ethylene group, and $A^{61}$ is an alkyl group substituted with an alkylthio group.
136. The compound or a salt thereof according to 1, wherein $X^1 \cdots X^2$ is ethylene group, and $A^{61}$ is an alkyl group substituted with an acylthio group.
137. The compound or a salt thereof according to 1, wherein $X^1 \cdots X^2$ is ethylene group, and $A^{61}$ is a cycloalkyl group.
138. The compound or a salt thereof according to 1, wherein $X^1 \cdots X^2$ is ethylene group, and $A^{61}$ is a substituted cycloalkyl group.
139. The compound or a salt thereof according to 1, wherein $X^1 \cdots X^2$ is ethylene group, and $A^{61}$ is carbamimidoyl group.
140. The compound or a salt thereof according to 1, wherein $X^1 \cdots X^2$ is ethylene group, and $A^{61}$ is an alkylcarbonyl group.
141. The compound or a salt thereof according to 1, wherein $X^1 \cdots X^2$ is ethylene group, and $A^{61}$ is an arylcarbonyl group.
142. The compound or a salt thereof according to 1, wherein $X^1 \cdots X^2$ is ethylene group, and $A^{61}$ is a non-aromatic heterocyclic group.
143. The compound or a salt thereof according to 1, wherein $X^1 \cdots X^2$ is ethylene group, and $A^{61}$ is a substituted non-aromatic heterocyclic group.

144. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, and $A^{61}$ is an alkyl group substituted with an aryl group, an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents carbonyl group, $-SO_2-$, -C(=O)-O-, $-C(=O)-N(A^8)-$, or $-C(=S)-N(A^8)-$).

145. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, and $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents $-SO_2-$, -C(=O)-O-, $-C(=O)-N(A^8)-$, or $-C(=S)-N(A^8)-$).

146. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, and $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom).
147. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, and $A^{61}$ is an alkyl group of which end is substituted with $N(A^7)(-X^3-A^{71})$ (wherein $-X^3-$ represents $-SO_2$ -C(=O)-O-, $-C(=O)-N(A^8)-$, or $-C(=S)-N(A^8)-$).
148. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, and $A^{61}$ is an alkyl group of which end is substituted with $N(A^7)(-X^3-A^{71})$ (wherein $-X^3-$ represents carbonyl group).
149. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, and $A^{61}$ is an alkyl group substituted with an aromatic heterocyclic group.

150. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 ... X^2$ is ethylene group, and $A^{61}$ is an alkyl group substituted with a substituted alkoxy group. 151. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, and $A^{61}$ is an alkyl group substituted with an alkylaminocarbonyloxy group.
152. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, and $A^{61}$ is an alkyl group substituted with a cycloalkylaminocarbonyloxy group.
153. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, and $A^{61}$ is an alkyl group substituted with an arylaminocarbonyloxy group.
154. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, and $A^{61}$ is an alkyl group substituted with mercapto group.

155. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, and $A^{61}$ is an alkyl group substituted with an alkylthio group.
156. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 ... X^2$ is ethylene group, and $A^{61}$ is an alkyl group substituted with an acylthio group.
157. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, and $A^{61}$ is a cycloalkyl group.
158. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 ... X^2$ is ethylene group, and $A^{61}$ is a substituted cycloalkyl group.
159. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, and $A^{61}$ is carbamimidoyl group.

160. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$... $X^2$ is ethylene group, and $A^{61}$ is an alkylcarbonyl group.
161. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$···$X^2$ is ethylene group, and $A^{61}$ is an arylcarbonyl group.
162. The compound or a salt thereof according to 1, wherein $R^1$ is hydrogen atom, or hydroxyl group, any one of $R^5$, $R^6$, $R^7$, and $R^8$ is not hydrogen atom, $X^1$···$X^2$ is ethylene group, and $A^{61}$ is an alkyl group substituted with an aryl group, an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents carbonyl group, $-SO_2-$, -C(=O)-O-, $-C(=O)-N(A^8)-$, or $-C(=S)-N(A^8)-$).

163. The compound or a salt thereof according to 1, wherein $R^1$ is hydrogen atom, or hydroxyl group, any one of $R^5$, $R^6$, $R^7$, and $R^8$ is not hydrogen atom, $X^1$···$X^2$ is ethylene group, and $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents $-SO_2-$, -C(=O)-O-, $-C(=O)-N(A^8)-$, or $-C(=S)-N(A^8)-$).
164. The compound or a salt thereof according to 1, wherein $R^1$ is hydrogen atom, or hydroxyl group, any one of $R^5$, $R^6$, $R^7$, and $R^8$ is not hydrogen atom, $X^1$···$X^2$ is ethylene group, and $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom).

165. The compound or a salt thereof according to 1, wherein $R^1$ is hydrogen atom, or hydroxyl group, any one of $R^5$, $R^6$, $R^7$, and $R^8$ is not hydrogen atom, $X^1$... $X^2$ is ethylene group, and $A^{61}$ is an alkyl group of which end is substituted with $N(A^7)(-X3-A^{71})$ (wherein $-X^3-$ represents $-SO_2-$ -C(=O)-O-, $-C(=O)-N(A^8)-$, or $-C(=S)-N(A^8)-$).
166. The compound or a salt thereof according to 1, wherein $R^1$ is hydrogen atom, or hydroxyl group, any one of $R^5$, $R^6$, $R^7$, and $R^8$ is not hydrogen atom, $X^1$ ... $X^2$ is ethylene group, and $A^{61}$ is an alkyl group of which end is substituted with $N(A^7)(-X^3-A^{71})$ (wherein $-X^3-$ represents carbonyl group).
167. The compound or a salt thereof according to 1, wherein $R^1$ is hydrogen atom, or hydroxyl group, any one of $R^5$, $R^6$, $R^7$, and $R^8$ is not hydrogen atom, $X^1$ ... $X^2$ is ethylene group, and $A^{61}$ is an alkyl group substituted with an aromatic heterocyclic group.
168. The compound or a salt thereof according to 1, wherein $R^1$ is hydrogen atom, or hydroxyl group, any one of $R^5$, $R^6$, $R^7$, and $R^8$ is not hydrogen atom, $X^1$···$X^2$ is ethylene group, and $A^{61}$ is an alkyl group substituted with a substituted alkoxy group.
169. The compound or a salt thereof according to 1, wherein $R^1$ is hydrogen atom, or hydroxyl group, any one of $R^5$, $R^6$, $R^7$, and $R^8$ is not hydrogen atom, $X^1$···$X^2$ is ethylene group, and $A^{61}$ is an alkyl group substituted with an alkylaminocarbonyloxy group. 170. The compound or a salt thereof according to 1, wherein $R^1$ is hydrogen atom, or hydroxyl group, any one of $R^5$, $R^6$, $R^7$, and $R^8$ is not hydrogen atom, $X^1$···$X^2$ is ethylene group, and $A^{61}$ is an alkyl group substituted with a cycloalkylaminocarbonyloxy group.

171. The compound or a salt thereof according to 1, wherein $R^1$ is hydrogen atom, or hydroxyl group, any one of $R^5$, $R^6$, $R^7$, and $R^8$ is not hydrogen atom, $X^1 \cdots X^2$ is ethylene group, and $A^{61}$ is an alkyl group substituted with an arylaminocarbonyloxy group.
172. The compound or a salt thereof according to 1, wherein $R^1$ is hydrogen atom, or hydroxyl group, any one of $R^5$, $R^6$, $R^7$, and $R^8$ is not hydrogen atom, $X^1 \cdots X^2$ is ethylene group, and $A^{61}$ is an alkyl group substituted with mercapto group.
173. The compound or a salt thereof according to 1, wherein $R^1$ is hydrogen atom, or hydroxyl group, any one of $R^5$, $R^6$, $R^7$, and $R^8$ is not hydrogen atom, $X^1 \cdots X^2$ is ethylene group, and $A^{61}$ is an alkyl group substituted with an alkylthio group.
174. The compound or a salt thereof according to 1, wherein $R^1$ is hydrogen atom, or hydroxyl group, any one of $R^5$, $R^6$, $R^7$, and $R^8$ is not hydrogen atom, $X^1 \cdots X^2$ is ethylene group, and $A^{61}$ is an alkyl group substituted with an acylthio group.
175. The compound or a salt thereof according to 1, wherein $R^1$ is hydrogen atom, or hydroxyl group, any one of $R^5$, $R^6$, $R^7$, and $R^8$ is not hydrogen atom, $X^1 \cdots X^2$ is ethylene group, and $A^{61}$ is a cycloalkyl group.
176. The compound or a salt thereof according to 1, wherein $R^1$ is hydrogen atom, or hydroxyl group, any one of $R^5$, $R^6$, $R^7$, and $R^8$ is not hydrogen atom, $X^1 \cdots X^2$ is ethylene group, and $A^{61}$ is a substituted cycloalkyl group.
177. The compound or a salt thereof according to 1, wherein $R^1$ is hydrogen atom, or hydroxyl group, any one of $R^5$, $R^6$, $R^7$, and $R^8$ is not hydrogen atom, $X^1 \cdots X^2$ is ethylene group, and $A^{61}$ is carbamimidoyl group.

178. The compound or a salt thereof according to 1, wherein $R^1$ is hydrogen atom, or hydroxyl group, any one of $R^5$, $R^6$, $R^7$, and $R^8$ is not hydrogen atom, $X^1$ ... $X^2$ is ethylene group, and $A^{61}$ is an alkylcarbonyl group.
179. The compound or a salt thereof according to 1, wherein $R^1$ is hydrogen atom, or hydroxyl group, any one of $R^5$, $R^6$, $R^7$, and $R^8$ is not hydrogen atom, $X^1$ ... $X^2$ is ethylene group, and $A^{61}$ is an arylcarbonyl group.
180. The compound or a salt thereof according to 1, wherein $R^1$ is hydrogen atom, or hydroxyl group, any one of $R^5$, $R^6$, $R^7$, and $R^8$ is not hydrogen atom, $X^1 \cdots X^2$ is ethylene group, and $A^{61}$ is a non-aromatic heterocyclic group.
181. The compound or a salt thereof according to 1, wherein $R^1$ is hydrogen atom, or hydroxyl group, any one of $R^5$, $R^6$, $R^7$, and $R^8$ is not hydrogen atom, $X^1 \cdots X^2$ is ethylene group, and $A^{61}$ is a substituted non-aromatic heterocyclic group.
182. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, and groups in one or more combinations selected from the group consisting of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ bind to each other to form a 5- or 6-membered ring

183. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is $-CH(R^2)-CH(R^3)-$, or $-C(R^2)=C(R^3)-$, and groups in one or more combinations selected from the group consisting of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ bind to each other to form a 5- or 6-membered ring
184. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is $-CH(R^2)-CH(R^3)-$, and groups in one or more combinations selected from the group consisting of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ bind to each other to form a 5- or 6-membered ring
185. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, and groups in one or more combinations selected from the group consisting of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ bind to each other to form a 5- or 6-membered ring

186. The compound or a salt thereof according to 1, wherein $R^1$ is hydrogen atom, chlorine atom, or hydroxyl group, any one of $R^5$, $R^6$, $R^7$, and $R^8$ is not hydrogen atom, and groups in one or more combinations selected from the

group consisting of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ bind to each other to form a 5- or 6-membered ring 187. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1$ ... $X^2$ is -CH($R^2$)-CH($R^3$)-, or -C($R^2$)=C($R^3$)-, and groups in one or more combinations selected from the group consisting of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ bind to each other to form a 5. or 6-membered ring

188. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1$ ··· $X^2$ is -CH($R^2$)-CH($R^3$)-, and groups in one or more combinations selected from the group consisting of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ bind to each other to form a 5- or 6-membered ring

189. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1$ ··· $X^2$ is ethylene group, and groups in one or more combinations selected from the group consisting of combinations of $A^6$ and $A^3$, $A^6$ and $A^4$, $A^6$ and $A^1$, $A^6$ and $A^2$, $A^2$ and A3, $A^2$ and $A^4$, $A^6$ and $A^5$, $A^3$ and $A^1$, and $A^5$ and $A^1$ bind to each other to form a 5- or 6-membered ring

190. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

191. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$···$X^2$ is -CH($R^2$)-CH($R^3$)-, or -C($R^2$)=C($R^3$)-, and the moiety of the formula (2) mentioned later is represented by the formula (2-1), (2-2), (2-3), (2-4-t), (2-4-c), (2-5-t), (2-5-c), (2-6-t), (2-6-c), or (2-7)

192: The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$···$X^2$ is -CH($R^2$)-CH($R^3$)-, and the moiety of the formula (2) mentioned later is represented by the formula (2-1), (2-2), (2-3), (2-4-t), (2-4-c), (2-5-t), (2-5-c), (2-6-t), (2-6-c), or (2-7) 193. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$···$X^2$ is ethylene group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

194. The compound or a salt thereof according to 1, wherein $R^1$ is hydrogen atom, chlorine atom, or hydroxyl group, any one of $R^5$, $R^6$, $R^7$, and $R^8$ is not hydrogen atom, and the moiety of the formula (2) mentioned later is represented by the formula (2-1), (2-2), (2-3), (2-4-t), (2-4-c), (2-5-t), (2-5-c), (2-6-t), (2-6-c), or (2-7)

195. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1$ ···$X^2$ is -CH($R^2$)-CH($R^3$)-, or -C($R^2$)=C($R^3$)-, and the moiety of the formula (2) mentioned later is represented by the formula (2-1), (2-2), (2-3), (2-4-t), (2-4-c), (2-5-t), (2-5-c), (2-6-t), (2-6-c), or (2-7)

196. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1$ ···$X^2$ is -CH($R^2$)-CH($R^3$)-, and the moiety of the formula (2) mentioned later is represented by the formula (2-1), (2-2), (2-3), (2-4-t), (2-4-c), (2-5-t), (2-5-c), (2-6-t), (2-6-c), or (2-7)

197. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1$ ···$X^2$ is ethylene group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

198. The compound or a salt thereof according to 1, wherein $R^1$ is hydrogen atom, or hydroxyl group, any one of $R^5$, $R^6$, $R^7$, and $R^8$ is not hydrogen atom, and the moiety of the formula (2) mentioned later is represented by the formula (2-1), (2-2), (2-3), (2-4-t), (2-4-c), (2-5-t), (2-5-c), (2-6-t), (2-6-c), or (2-7)

199. The compound or a salt thereof according to 1, wherein $R^1$ is hydrogen atom, or hydroxyl group, any one of $R^5$, $R^6$, $R^7$, and $R^8$ is not hydrogen atom, $X^1 \cdots X^2$ is $-CH(R^2)-CH(R^3)-$, or $-C(R^2)=C(R^3)-$, and the moiety of the formula (2) mentioned later is represented by the formula (2-1), (2-2), (2-3), (2-4-t), (2-4-c), (2-5-t), (2-5-c), (2-6-t), (2-6-c), or (2-7)
200. The compound or a salt thereof according to 1, wherein $R^1$ is hydrogen atom, or hydroxyl group, any one of $R^5$, $R^6$, $R^7$, and $R^8$ is not hydrogen atom, $X^1 \cdots X^2$ is $-CH(R^2)-CH(R^3)-$, and the moiety of the formula (2) mentioned later is represented by the formula (2-1), (2-2), (2-3), (2-4-t), (2-4-c), (2-5-t), (2-5-c), (2-6-t), (2-6-c), or (2-7) 201. The compound or a salt thereof according to 1, wherein $R^1$ is hydrogen atom, or hydroxyl group, any one of $R^5$, $R^6$, $R^7$, and $R^8$ is not hydrogen atom, $X^1 \cdots X^2$ is ethylene group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

202. The compound or a salt thereof according to 1, wherein $X^1 \cdots X^2$ is ethylene group, $A^{61}$ is an alkyl group substituted with an aryl group, an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents carbonyl group, $-SO_2-$, $-C(=O)-O-$, $-C(=O)-N(A^8)-$, or $-C(=S)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

203. The compound or a salt thereof according to 1, wherein $X^1 \cdots X^2$ is ethylene group, $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents $-SO_2-$, $-C(=O)-O-$, $-C(=O)-N(A^8)-$, or $-C(=S)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

204. The compound or a salt thereof according to 1, wherein $X^1 \cdots X^2$ is ethylene group, $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), and the moiety of the formula (2) mentioned later is represented by the formula (2-1), (2-2), (2-3), (2-4-t), (2-4-c), (2-5-t), (2-5-c), (2-6-t), (2-6-c), or (2-7)
205. The compound or a salt thereof according to 1, wherein $X^1 \cdots X^2$ is ethylene group, $A^{61}$ is an alkyl group of which end is substituted with $N(A^7)(-X^3-A^{71})$ (wherein $-X^3-$ represents $-SO_2-$, $-C(=O)-O-$, $-C(=O)-N(A^8)-$, or $-C(=S)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)
206. The compound or a salt thereof according to 1, wherein $X^1 \cdots X^2$ is ethylene group, $A^{61}$ is an alkyl group of which end is substituted with $N(A^7)(-X^3-A^{71})$ (wherein $-X^3-$ represents carbonyl group), and the moiety of the formula (2) mentioned later is represented by the formula (2-1), (2-2), (2-3), (2-4-t), (2-4-c), (2-5-t), (2-5-c), (2-6-t), (2-6-c), or (2-7)
207. The compound or a salt thereof according to 1, wherein $X^1 \cdots X^2$ is ethylene group, $A^{61}$ is an alkyl group substituted with an aromatic heterocyclic group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

208. The compound or a salt thereof according to 1, wherein $X^1$... $X^2$ is ethylene group, $A^{61}$ is an alkyl group substituted with a substituted alkoxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
209. The compound or a salt thereof according to 1, wherein $X^1 \cdots X^2$ is ethylene group, $A^{61}$ is an alkyl group substituted with an alkylaminocarbonyloxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
210. The compound or a salt thereof according to 1, wherein $X^1 \cdots X^2$ is ethylene group, $A^{61}$ is an alkyl group substituted with a cycloalkylaminocarbonyloxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
211. The compound or a salt thereof according to 1, wherein $X^1 \cdots X^2$ is ethylene group, $A^{61}$ is an alkyl group substituted with an arylaminocarbonyloxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
212. The compound or a salt thereof according to 1, wherein $X^1 \cdots X^2$ is ethylene group, $A^{61}$ is an alkyl group substituted with mercapto group, and the moiety of the formula (2), mentioned later is represented by the formula (2-1), (2-2), (2-3), (2-4-t), (2-4-c), (2-5-t), (2-5-c), (2-6-t), (2-6-c), or (2-7)

213. The compound or a salt thereof according to 1, wherein $X^1 \cdots X^2$ is ethylene group, $A^{61}$ is an alkyl group substituted with an alkylthio group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
214. The compound or a salt thereof according to 1, wherein $X^1 \cdots X^2$ is ethylene group, $A^{61}$ is an alkyl group substituted with an acylthio group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
215. The compound or a salt thereof according to 1, wherein $X^1 \cdots X^2$ is ethylene group, $A^{61}$ is a cycloalkyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
216. The compound or a salt thereof according to 1, wherein $X^1 \cdots X^2$ is ethylene group, $A^{61}$ is a substituted cycloalkyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

217. The compound or a salt thereof according to 1, wherein $X^1 \cdots X^2$ is ethylene group, $A^{61}$ is carbamimidoyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
218. The compound or a salt thereof according to 1, wherein $X^1 \cdots X^2$ is ethylene group, $A^{61}$ is an alkylcarbonyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
219. The compound or a salt thereof according to 1, wherein $X^1 \cdots X^2$ is ethylene group, $A^{61}$ is an arylcarbonyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
220. The compound or a salt thereof according to 1, wherein $X^1 \cdots X^2$ is ethylene group, $A^{61}$ is a non-aromatic heterocyclic group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
221. The compound or a salt thereof according to 1, wherein $X^1 \cdots X^2$ is ethylene group, $A^{61}$ is a substituted non-aromatic heterocyclic group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

222. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, $A^{61}$ is an alkyl group substituted with an aryl group, an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl

group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents carbonyl group, $-SO_2-$, -C(=O)-O-, $-C(=O)-N(A^8)-$, or $-C(=S)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

223. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents $-SO_2-$, -C(=O)-O-, $-C(=O)-N(A^8)-$, or $-C(=S)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

224**.** The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), and the moiety of the formula (2) mentioned later is represented by the formula (2-1), (2-2), (2-3), (2-4-t), (2-4-c), (2-5-t), (2-5-c), (2-6-t), (2-6-c), or (2-7)
225. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 ... X^2$ is ethylene group, $A^{61}$ is an alkyl group of which end is substituted with $N(A^7)(-X^3-A^{71})$ (wherein $-X^3-$ represents $-SO_2-$, -C(=O)-O-, $-C(=O)-N(A^8)-$, or $-C(=S)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

226. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, $A^{61}$ is an alkyl group of which end is substituted with $N(A^7)(-X^3-A^{71})$ (wherein $-X^3-$ represents carbonyl group), and the moiety of the formula (2) mentioned later is represented by the formula (2-1), (2-2), (2-3), (2-4-t), (2-4-c), (2-5-t), (2-5-c), (2-6-t), (2-6-c), or (2-7)
227. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, $A^{61}$ is an alkyl group substituted with an aromatic heterocyclic group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

**228.** The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1 \cdots X^2$ is ethylene group, $A^{61}$ is an alkyl group substituted with a substituted alkoxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

229. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$···$X^2$ is ethylene group, $A^{61}$ is an alkyl group substituted with an alkylaminocarbonyloxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
230. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$···$X^2$ is ethylene group, $A^{61}$ is an alkyl group substituted with a cycloalkylaminocarbonyloxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

231. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$··· $X^2$ is ethylene group, $A^{61}$ is an alkyl group substituted with an arylaminocarbonyloxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
232. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$···$X^2$ is ethylene group, $A^{61}$ is an alkyl group substituted with mercapto group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
233. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$···$X^2$ is ethylene group, $A^{61}$ is an alkyl group substituted with an alkylthio group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

234. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$...$X^2$ is ethylene group, $A^{61}$ is an alkyl group substituted with an acylthio group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
235. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$...$X^2$ is ethylene group, $A^{61}$ is a cycloalkyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
236. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$··· $X^2$ is ethylene group, $A^{61}$ is a substituted cycloalkyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

237. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$···$X^2$ is ethylene group, $A^{61}$ is carbamimidoyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
238. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$···$X^2$ is ethylene group, $A^{61}$ is an alkylcarbonyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
239. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$···$X^2$ is ethylene group, $A^{61}$ is an arylcarbonyl group, and the moiety of the formula (2) mentioned later is

represented by the formula (2-1), (2-2), (2-3), (2-4-t), (2-4-c), (2-5-t), (2-5-c), (2-6-t), (2-6-c), or (2-7)

240. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$···$X^2$ is ethylene group, $A^{61}$ is a non-aromatic heterocyclic group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

241. The compound or a salt thereof according to 1, wherein $R^1$ is fluorine atom, bromine atom, iodine atom, an alkyl group, an alkenyl group, an alkynyl group, an alkoxy group, amino group, an alkylamino group, or an arylamino group, $X^1$···$X^2$ is ethylene group, $A^{61}$ is a substituted non-aromatic heterocyclic group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

242. The compound or a salt thereof according to 1, wherein $R^1$ is hydrogen atom, or hydroxyl group, $X^1$··· $X^2$ is ethylene group, $A^{61}$ is an alkyl group substituted with an aryl group, an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents carbonyl group, $-SO_2-$, -C(=O)-O-, $-C(=O)-N(A^8)-$, or $-C(=S)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

243. The compound or a salt thereof according to 1, wherein $R^1$ is hydrogen atom, or hydroxyl group, $X^1$···$X^2$ is ethylene group, $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents $-SO_2$ -C(=O)-O-, $-C(=O)-N(A^8)-$, or $-C(=S)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

244. The compound or a salt thereof according to 1, wherein $R^1$ is hydrogen atom, or hydroxyl group, $X^1$···$X^2$ is ethylene group, $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), and the moiety of the formula (2) mentioned later is represented by the formula (2-1), (2-2), (2-3), (2-4-t), (2-4-c), (2-5-t), (2-5-c), (2-6-t), (2-6-c), or (2-7)

245. The compound or a salt thereof according to 1, wherein $R^1$ is hydrogen atom, or hydroxyl group, $X^1$···$X^2$ is ethylene group, $A^{61}$ is an alkyl group of which end is substituted with $N(A^7)(-X^3-A^{71})$ (wherein $-X^3-$ represents $-SO_2-$, -C(=O)-O-, $-C(=O)-N(A^8)-$, or $-C(=S)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

246. The compound or a salt thereof according to 1, wherein $R^1$ is hydrogen atom, or hydroxyl group, $X^1$···$X^2$ is ethylene group, $A^{61}$ is an alkyl group of which end is substituted with $N(A^7)(-X^3-A^{71})$ (wherein $-X^3-$ represents carbonyl group), and the moiety of the formula (2) mentioned later is represented by the formula (2-1), (2-2), (2-3), (2-4-t), (2-4-c), (2-5-t), (2-5-c), (2-6-t), (2-6-c), or (2-7)

247. The compound or a salt thereof according to 1, wherein $R^1$ is hydrogen atom, or hydroxyl group, $X^1$···$X^2$ is ethylene group, $A^{61}$ is an alkyl group substituted with an aromatic heterocyclic group, and the moiety of the formula (2) mentioned later is represented by the formula (2-1), (2-2), (2-3), (2-4-t), (2-4-c), (2-5-t), (2-5-c), (2-6-t), (2-6-c),

or (2-7)

248. The compound or a salt thereof according to 1, wherein $R^1$ is hydrogen atom, or hydroxyl group, $X^1 \cdots X^2$ is ethylene group, $A^{61}$ is an alkyl group substituted with a substituted alkoxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

249. The compound or a salt thereof according to 1, wherein $R^1$ is hydrogen atom, or hydroxyl group, $X^1 \cdots X^2$ is ethylene group, $A^{61}$ is an alkyl group substituted with an alkylaminocarbonyloxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

250. The compound or a salt thereof according to 1, wherein $R^1$ is hydrogen atom, or hydroxyl group, $X^1 \cdots X^2$ is ethylene group, $A^{61}$ is an alkyl group substituted with a cycloalkylaminocarbonyloxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

251. The compound or a salt thereof according to 1, wherein $R^1$ is hydrogen atom, or hydroxyl group, $X^1 \cdots X^2$ is ethylene group, $A^{61}$ is an alkyl group substituted with an arylaminocarbonyloxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

252. The compound or a salt thereof according to 1, wherein $R^1$ is hydrogen atom, or hydroxyl group, $X^1 \cdots X^2$ is ethylene group, $A^{61}$ is an alkyl group substituted with mercapto group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

253. The compound or a salt thereof according to 1, wherein $R^1$ is hydrogen atom, or hydroxyl group, $X^1 \cdots X^2$ is ethylene group, $A^{61}$ is an alkyl group substituted with an alkylthio group, and the moiety of the formula (2) mentioned later is represented by the formula (2-1), (2-2), (2-3), (2-4-t), (2-4-c), (2-5-t), (2-5-c), (2-6-t), (2-6-c), or (2-7) 254. The compound or a salt thereof according to 1, wherein $R^1$ is hydrogen atom, or hydroxyl group, $X^1 ... X^2$ is ethylene group, $A^{61}$ is an alkyl group substituted with an acylthio group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

255. The compound or a salt thereof according to 1, wherein $R^1$ is hydrogen atom, or hydroxyl group, $X^1 \cdots X^2$ is ethylene group, $A^{61}$ is a cycloalkyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

256. The compound or a salt thereof according to 1, wherein $R^1$ is hydrogen atom, or hydroxyl group, $X^1 \cdots X^2$ is ethylene group, $A^{61}$ is a substituted cycloalkyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

257. The compound or a salt thereof according to 1, wherein $R^1$ is hydrogen atom, or hydroxyl group, $X^1 \cdots X^2$ is ethylene group, and $A^{61}$ is carbamimidoyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

258. The compound or a salt thereof according to 1, wherein $R^1$ is hydrogen atom, or hydroxyl group, $X^1 \cdots X^2$ is ethylene group, $A^{61}$ is an alkylcarbonyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

259. The compound or a salt thereof according to 1, wherein $R^1$ is hydrogen atom, or hydroxyl group, $X^1 \cdots X^2$ is ethylene group, $A^{61}$ is an arylcarbonyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

260. The compound or a salt thereof according to 1, wherein $R^1$ is hydrogen atom, or hydroxyl group, $X^1 \cdots X^2$ is ethylene group, $A^{61}$ is a non-aromatic heterocyclic group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

261. The compound or a salt thereof according to 1, wherein $R^1$ is hydrogen atom, or hydroxyl group, $X^1 \cdots X^2$ is ethylene group, $A^{61}$ is a substituted non-aromatic heterocyclic group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

262. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1$ ···$X^2$ is ethylene group, $A^{61}$ is an alkyl group substituted with an aryl group, an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents carbonyl group, $-SO_2-$, -C(=O)-O-, $-C(=O)-N(A^8)-$, or $-C(=S)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

263. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1$ ···$X^2$ is ethylene group, $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents $-SO_2-$, -C(=O)-O-, $-C(=O)-N(A^8)-$, or $-C(=S)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

264. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1$ w$X^2$ is ethylene group, $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), and the moiety of the formula (2) mentioned later is represented by the formula (2-1), (2-2), (2-3), (2-4-t), (2-4-c), (2-5-t), (2-5-c), (2-6-t), (2-6-c), or (2-7)
265. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1$ ··· $X^2$ is ethylene group, $A^{61}$ is an alkyl group of which end is substituted with $N(A^7)(-X^3-A^{71})$ (wherein $-X^3-$ represents $-SO_2-$, -C(=O)-O-, $-C(=O)-N(A^8)-$, or $-C(=S)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

266. The compound or a salt thereof according to 1, wherein $R^1$ is hydrogen atom, chlorine atom, or hydroxyl group, any one of $R^6$, $R^6$, $R^7$, and $R^8$ is not hydrogen atom, $X^1$ ···$X^2$ is ethylene group, $A^{61}$ is an alkyl group of which end is substituted with $N(A^7)(-X^3-A^{71})$ (wherein $-X^3-$ represents carbonyl group), and the moiety of the formula (2) mentioned later is represented by the formula (2-1), (2-2), (2-3), (2-4-t), (2-4-c), (2-5-t), (2-5-c), (2-6-t), (2-6-c), or (2-7)
267. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1$ ···$X^2$ is ethylene group, $A^{61}$ is an alkyl group substituted with an aromatic heterocyclic group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
268. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1$ ··· $X^2$ is ethylene group, $A^{61}$ is an alkyl group substituted with a substituted alkoxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

269. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1 \cdots X^2$ is ethylene group, $A^{61}$ is an alkyl group substituted with an alkylaminocarbonyloxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
270. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1 \cdots X^2$ is ethylene group, $A^{61}$ is an alkyl group substituted with a cycloalkylaminocarbonyloxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
271. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1 \cdots X^2$ is ethylene group, $A^{61}$ is an alkyl group substituted with an arylaminocarbonyloxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

272. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1 \cdots X^2$ is ethylene group, $A^{61}$ is an alkyl group substituted with mercapto group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
273. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1 \cdots X^2$ is ethylene group, $A^{61}$ is an alkyl group substituted with an alkylthio group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
274. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1 \cdots X^2$ is ethylene group, $A^{61}$ is an alkyl group substituted with an acylthio group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
275. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1 \cdots X^2$ is ethylene group, $A^{61}$ is a cycloalkyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

276. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1 \cdots X^2$ is ethylene group, $A^{61}$ is a substituted cycloalkyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
277. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1 \cdots X^2$ is ethylene group, $A^{61}$ is carbamimidoyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
278. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1 \cdots X^2$ is ethylene group, $A^{61}$ is an alkylcarbonyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
279. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1 \cdots X^2$ is ethylene group, $A^{61}$ is an arylcarbonyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

280. The compound or a salt thereof according to 1, wherein $R^1$ is hydrogen atom, chlorine atom, or hydroxyl group, any one of $R^5$, $R^6$, $R^7$, and $R^8$ is not hydrogen atom, $X^1 \cdots X^2$ is ethylene group, $A^{61}$ is a non-aromatic heterocyclic group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
281. The compound or a salt thereof according to 1, wherein $R^1$ is hydrogen atom, chlorine atom, or hydroxyl group,

any one of $R^5$, $R^6$, $R^7$, and $R^8$ is not hydrogen atom, $X^1 \cdots X^2$ is ethylene group, $A^{61}$ is a substituted non-aromatic heterocyclic group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

282. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with an aryl group, an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents carbonyl group, $-SO_2-$, -C(=O)-O-, $-C(=O)-N(A^8)-$, or $-C(=S)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

283. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), an alkyl group substituted with an aromatic heterocyclic group, an alkyl group substituted with a substituted alkoxy group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$ (wherein $-X^3-$ represents $-SO_2-$, -C(=O)-O-, $-C(=O)-N(A^8)-$, or $-C(=S)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

284. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with an aryl group (wherein the aryl moiety is not unsubstituted, or is not substituted with any one of an alkyl group, and a halogen atom, or is not substituted with both of an alkyl group, and a halogen atom), and the moiety of the formula (2) mentioned later is represented by the formula (2-1), (2-2), (2-3), (2-4-t), (2-4-c), (2-5-t), (2-5-c), (2-6-t), (2-6-c), or (2-7)
285. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group of which end is substituted with $N(A^7)(-X^3-A^{71})$ (wherein $-X^3-$ represents $-SO_2-$, -C(=O)-O-, $-C(=O)-N(A^8)-$, or $-C(=S)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

286. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group of which end is substituted with $N(A^7)(-X^3-A^{71})$ (wherein $-X^3-$ represents carbonyl group), and the moiety of the formula (2) mentioned later is represented by the formula (2-1), (2-2), (2-3), (2-4-t), (2-4-c), (2-5-t), (2-5-c), (2-6-t), (2-6-c), or (2-7)
287. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with an aromatic heterocyclic group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
288. The compound or a salt thereof according to 1, wherein $R^1$ is hydrogen atom, or hydroxyl group, all of $R^5$, $R^6$,

$R^7$, and $R^8$ are hydrogen atoms, $X^1 \cdots X^2$ is ethylene group, $A^{61}$ is an alkyl group substituted with a substituted alkoxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

289. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with an alkylaminocarbonyloxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

290. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with a cycloalkylaminocarbonyloxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

291. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with an arylaminocarbonyloxy group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

292. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with mercapto group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

293. The compound or a salt thereof according to 1, wherein $R^1$ is hydrogen atom, or hydroxyl group, all of $R^5$, $R^6$, $R^7$, and $R^8$ are hydrogen atoms, $X^1 \ldots X^2$ is ethylene group, $A^{61}$ is an alkyl group substituted with an alkylthio group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

294. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with an acylthio group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

295. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is a cycloalkyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

296. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is a substituted cycloalkyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

297. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is carbamimidoyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

298. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkylcarbonyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

299. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an arylcarbonyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

300. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is a non-aromatic heterocyclic group, and the moiety

of the formula (2) mentioned later is represented by the formula (2-1), (2-2), (2-3), (2-4-t), (2-4-c), (2-5-t), (2-5-c), (2-6-t), (2-6-c), or (2-7)

301. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is a substituted non-aromatic heterocyclic group, and the moiety of the formula (2) mentioned later is represented by the formula (2-1), (2-2), (2-3), (2-4-t), (2-4-c), (2-5-t), (2-5-c), (2-6-t), (2-6-c), or (2-7)
302. The compound or a salt thereof according to 1, wherein $X^1 \cdots X^2$ is ethylene group, $A^{61}$ is an alkyl group of which end is substituted with $N(A^7)(-X^3-A^{71})$ (wherein $-X^3-$ represents $-SO_2-$, or $-C(=O)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)
303. The compound or a salt thereof according to 1, wherein $X^1 \cdots X^2$ is ethylene group, $A^{61}$ is an alkyl group of which end is substituted with $N(A^7)(-X^3-A^{71})$ (wherein $-X^3-$ represents $-SO_2-$), and the moiety of the formula (2) mentioned later is represented by the formula (2-1), (2-2), (2-3), (2-4-t), (2-4-c), (2-5-t), (2-5-c), (2-6-t), (2-6-c), or (2-7) 304. The compound or a salt thereof according to 1, wherein $X^1 \cdots X^2$ is ethylene group, $A^{61}$ is an alkyl group of which end is substituted with $N(A^7)(-X^3-A^{71})$ (wherein $-X^3-$ represents $-C(=O)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

305. The compound or a salt thereof according to 1, wherein $X^1 \cdots X^2$ is ethylene group, $A^{61}$ is an alkyl group of which end is substituted with $N(A^7)(-X^3-A^{71})$ (wherein $A^7$ represents hydrogen atom, $-X^3-$ represents $-SO_2-$, or $-C(=O)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)
306. The compound or a salt thereof according to 1, wherein $X^1 ... X^2$ is ethylene group, $A^{61}$ is an alkyl group of which end is substituted with $N(A^7)(-X^3-A^{71})$ (wherein $A^7$ represents hydrogen atom, $-X^3-$ represents $-SO_2-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)
307. The compound or a salt thereof according to 1, wherein $X^1 ... X^2$ is ethylene group, $A^{61}$ is an alkyl group of which end is substituted with $N(A^7)(-X^3-A^{71})$ (wherein $A^7$ represents hydrogen atom, $-X^{3-}$ represents $-C(=O)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)
308. The compound or a salt thereof according to 1, wherein $R^1$ is hydrogen atom, chlorine atom, or hydroxyl group, $X^1 \cdots X^2$ is ethylene group, $A^{61}$ is an alkyl group of which end is substituted with $N(A^7)(-X^3-A^{71})$ (wherein $-X^{3-}$ represents $-SO_2-$, or $-C(=O)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

309. The compound or a salt thereof according to 1, wherein $R^1$ is hydrogen atom, chlorine atom, or hydroxyl group, $X^1 \cdots X^2$ is ethylene group, $A^{61}$ is an alkyl group of which end is substituted with $N(A^7)(-X^3-A^{71})$ (wherein $-X^3-$ represents $-SO_2-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)
310. The compound or a salt thereof according to 1, wherein $R^1$ is hydrogen atom, chlorine atom, or hydroxyl group, $X^1 ... X^2$ is ethylene group, $A^{61}$ is an alkyl group of which end is substituted with $N(A^7)(-X^3-A^{71})$ (wherein $-X^3-$ represents $-C(=O)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)
311. The compound or a salt thereof according to 1, wherein $R^1$ is hydrogen atom, chlorine atom, or hydroxyl group, $X^1 ... X^2$ is ethylene group, $A^{61}$ is an alkyl group of which end is substituted with $N(A^7)(-X^3-A^{71})$ (wherein $A^7$ represents hydrogen atom, $-X^{3-}$ represents $-SO_2-$, or $-C(=O)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

312. The compound or a salt thereof according to 1, wherein $R^1$ is hydrogen atom, chlorine atom, or hydroxyl group, $X^1 \cdots X^2$ is ethylene group, $A^{61}$ is an alkyl group of which end is substituted with $N(A^7)(-X^3-A^{71})$ (wherein $A^7$ represents

hydrogen atom, $-X^3-$ represents $-SO_2-$), and the moiety of the formula (2) mentioned later is represented by the formula (2-1), (2-2), (2-3), (2-4-t), (2-4-c), (2-5-t), (2-5-c), (2-6-t), (2-6-c), or (2-7) 313. The compound or a salt thereof according to 1, wherein $R^1$ is hydrogen atom, chlorine atom, or hydroxyl group, $X^1\cdots X^2$ is ethylene group, $A^{61}$ is an alkyl group of which end is substituted with $N(A^7)(-X^3-A^{71})$ (wherein $A^7$ represents hydrogen atom, $-X^3-$ represents $-C(=O)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

314. The compound or a salt thereof according to 1, wherein $R^1$ is hydrogen atom, or hydroxyl group, $X^1\cdots X^2$ is ethylene group, $A^{61}$ is an alkyl group of which end is substituted with $N(A^7)(-X^3-A^{71})$ (wherein $-X^3-$ represents $-SO_2-$, or $-C(=O)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

315. The compound or a salt thereof according to 1, wherein $R^1$ is hydrogen atom, or hydroxyl group, $X^1\cdots X^2$ is ethylene group, $A^{61}$ is an alkyl group of which end is substituted with $N(A^7)(-X^3-A^{71})$ (wherein $-X^3-$ represents $-SO_2-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

316. The compound or a salt thereof according to 1, wherein $R^1$ is hydrogen atom, or hydroxyl group, $X^1...X^2$ is ethylene group, $A^{61}$ is an alkyl group of which end is substituted with $N(A^7)(-X^3-A^{71})$ (wherein $-X^3-$ represents $-C(=O)-N(A^8)-$), and the moiety of the formula (2) mentioned later is represented by the formula (2-1); (2-2), (2-3), (2-4-t), (2-4-c), (2-5-t), (2-5-c), (2-6-t), (2-6-c), or (2-7)

317. The compound or a salt thereof according to 1, wherein $R^1$ is hydrogen atom, or hydroxyl group, $X^1\cdots X^2$ is ethylene group, $A^{61}$ is an alkyl group of which end is substituted with $N(A^7)(-X^3-A^{71})$ (wherein $A^7$ represents hydrogen atom, $-X^3-$ represents $-SO_2-$, or $-C(=O)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

318. The compound or a salt thereof according to 1, wherein $R^1$ is hydrogen atom, or hydroxyl group, $X^1...X^2$ is ethylene group, $A^{61}$ is an alkyl group of which end is substituted with $N(A^7)(-X^3-A^{71})$ (wherein $A^7$ represents hydrogen atom, $-X^3-$ represents $-SO_2-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

319. The compound or a salt thereof according to 1, wherein $R^1$ is hydrogen atom, or hydroxyl group, $X^1\cdots X^2$ is ethylene group, $A^{61}$ is an alkyl group of which end is substituted with $N(A^7)(-X^3-A^{71})$ (wherein $A^7$ represents hydrogen atom, $-X^3-$ represents $-C(=O)-N(A^8)-$), and the moiety of the formula (2) mentioned later is represented by the formula (2-1), (2-2), (2-3), (2-4-t), (2-4-c), (2-5-t), (2-5-c), (2-6-t), (2-6-c), or (2-7)

320. The compound or a salt thereof according to 1, wherein $X^1\cdots X^2$ is ethylene group, $A^{61}$ is a substituted cyclobutyl group, or a substituted cyclohexyl group, and the moiety of the formula (2) mentioned later is represented by the formula (2-1), (2-2), (2-3), (2-4-t), (2-4-c), (2-5-t), (2-5-c), (2-6-t), (2-6-c), or (2-7)

321. The compound or a salt thereof according to 1, wherein $X^1\cdots X^2$ is ethylene group, $A^{61}$ is a cyclobutyl group substituted at the 3-position, or a cyclohexyl group substituted at the 4-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)

322. The compound or a salt thereof according to 1, wherein $R^1$ is hydrogen atom, or hydroxyl group, $X^1\cdots X^2$ is ethylene group, $A^{61}$ is a cyclobutyl group substituted at the 3-position, or a cyclohexyl group substituted at the 4-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)

323. The compound or a salt thereof according to 1, wherein $R^1$ is hydrogen atom, or hydroxyl group, $X^1\cdots X^2$ is ethylene group, $A^{61}$ is a cyclobutyl group substituted at the 3-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)

324. The compound or a salt thereof according to 1, wherein $R^1$ is hydrogen atom, or hydroxyl group, $X^1\cdots X^2$ is ethylene group, $A^{61}$ is a cyclohexyl group substituted at the 4-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)

325. The compound or a salt thereof according to 1, wherein $R^1$ is hydrogen atom, or hydroxyl group, $X^1 \cdots X^2$ is ethylene group, $A^{61}$ is a cyclobutyl group substituted with hydroxyl group, amino group, an alkylamino group, an alkylsulfonylamino group, or an acylamino group at the 3-position, or a cyclohexyl group substituted with hydroxyl group, amino group, an alkylamino group, an alkylsulfonylamino group, or an acylamino group at the 4-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)
326. The compound or a salt thereof according to 1, wherein $R^1$ is hydrogen atom, or hydroxyl group, $X^1 \cdots X^2$ is ethylene group, $A^{61}$ is a cyclobutyl group substituted with hydroxyl group, amino group, an alkylamino group, an alkylsulfonylamino group, or an acylamino group at the 3-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)
327. The compound or a salt thereof according to 1, wherein $R^1$ is hydrogen atom, or hydroxyl group, $X^1 \cdots X^2$ is ethylene group, $A^{61}$ is a cyclohexyl group substituted with hydroxyl group, amino group, an alkylamino group, an alkylsulfonylamino group, or an acylamino group at the 4-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)

328. The compound or a salt thereof according to 1, wherein $R^1$ is hydrogen atom, or hydroxyl group, $X^1 \cdots X^2$ is ethylene group, $A^{61}$ is a cyclobutyl group substituted with hydroxyl group at the 3-position, or a cyclohexyl group substituted with hydroxyl group at the 4-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)
329. The compound or a salt thereof according to 1, wherein $R^1$ is hydrogen atom, or hydroxyl group, $X^1 \cdots X^2$ is ethylene group, $A^{61}$ is a cyclobutyl group substituted with hydroxyl group at the 3-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)
330. The compound or a salt thereof according to 1, wherein $R^1$ is hydrogen atom, or hydroxyl group, $X^1 \cdots X^2$ is ethylene group, $A^{61}$ is a cyclohexyl group substituted with hydroxyl group at the 4-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)

331. The compound or a salt thereof according to 1, wherein $R^1$ is hydrogen atom, or hydroxyl group, $X^1 \cdots X^2$ is ethylene group, $A^{61}$ is a cyclobutyl group substituted with amino group at the 3-position, or a cyclohexyl group substituted with hydroxyl group, amino group, an alkylamino group, or an acylamino group at the 4-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)
332. The compound or a salt thereof according to 1, wherein $R^1$ is hydrogen atom, or hydroxyl group, $X^1 ... X^2$ is ethylene group, $A^{61}$ is a cyclobutyl group substituted with amino group at the 3-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)
333. The compound or a salt thereof according to 1, wherein $R^1$ is hydrogen atom, or hydroxyl group, $X^1 \cdots X^2$ is ethylene group, $A^{61}$ is a cyclohexyl group substituted with amino group at the 4-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)
334. The compound or a salt thereof according to 1, wherein $R^1$ is hydrogen atom, or hydroxyl group, $X^1 \cdots X^2$ is ethylene group, $A^{61}$ is a cyclobutyl group substituted with an alkylamino group at the 3-position, or a cyclohexyl group substituted with hydroxyl group, amino group, an alkylamino group, or an acylamino group at the 4-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)

**335.** The compound or a salt thereof according to 1, wherein $R^1$ is hydrogen atom, or hydroxyl group, $X^1 \cdots X^2$ is ethylene group, $A^{61}$ is a cyclobutyl group substituted with an alkylamino group at the 3-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)
336. The compound or a salt thereof according to 1, wherein $R^1$ is hydrogen atom, or hydroxyl group, $X^1 ... X^2$ is

ethylene group, $A^{61}$ is a cyclohexyl group substituted with an alkylamino group at the 4-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)

337. The compound or a salt thereof according to 1, wherein $R^1$ is hydrogen atom, or hydroxyl group, $X^1 \cdots X^2$ is ethylene group, $A^{61}$ is a cyclobutyl group substituted with an acylamino group at the 3-position, or a cyclohexyl group substituted with hydroxyl group, amino group, an alkylamino group, or an acylamino group at the 4-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)

338. The compound or a salt thereof according to 1, wherein $R^1$ is hydrogen atom, or hydroxyl group, $X^1 \cdots X^2$ is ethylene group, $A^{61}$ is a cyclobutyl group substituted with an acylamino group at the 3-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)

339. The compound or a salt thereof according to 1, wherein $R^1$ is hydrogen atom, or hydroxyl group, $X^1 \cdots X^2$ is ethylene group, $A^{61}$ is a cyclohexyl group substituted with an acylamino group at the 4-position, and the moiety of the formula (2) mentioned later is represented by the formula (2-1), (2-2), (2-3), (2-4-t), (2-4-c), (2-5-t), (2-5-c), (2-6-t), (2-6-c), or (2-7)

340. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with an aryl group, and the moiety of the formula (2) mentioned later is represented by the formula (2-7)

341. The compound or a salt thereof according to 1 or 340, which is the compound of Example 1-4, 1-41, 1-42, 1-43, 1-44, 1-45, 1-46, 1-47, 1-48, 1-50, 1-51, 1-52, 1-53, 1-54, 1-55, 1-56, 1-57, 1-59, 1-60, 1-61, 1-62, 1-63, 1-64, 1-65, 1-66, 1-241, 1-242, 1-299, 1-314, 1-315, 1-334, 1-371, 1-372, 1-373, 1-374, 1-375, 1-376, 1-377, 1-378, 1-380, 1-381, 1-382, 1-383, 1-384, 1-385, 1-386, 1-387, 1-389, 1-390, 1-391, 1-392, 1-393, 1-394, 1-395, 1-396, 1-571, 1-572, 1-629, 1-644, or 1-645.

342. The compound or a salt thereof according to 1 or 340, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with a substituted aryl group, and the moiety of the formula (2) mentioned later is represented by the formula (2-7).

343. The compound or a salt thereof according to 1, 340 or 342, which is the compound of Example 1-4, 1-42, 1-43, 1-44, 1-45, 1-46, 1-47, 1-48, 1-50, 1-51, 1-52, 1-53, 1-54, 1-55, 1-56, 1-57, 1-59, 1-60, 1-61, 1-62, 1-63, 1-64, 1-65, 1-66, 1-241, 1-242, 1-299, 1-314, 1-315, 1-334, 1-372, 1-373, 1-374, 1-375, 1-376, 1-377, 1-378, 1-380, 1-381, 1-382, 1-383, 1-384, 1-385, 1-386, 1-387, 1-389, 1-390, 1-391, 1-392, 1-393, 1-394, 1-395, 1-396, 1-571, 1-572, 1-629, 1-644, or 1-645.

344. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with a substituted aryl group, and the moiety of the formula (2) mentioned later is represented by the formula (2-1).

345. The compound according to 1, or 344, which is the compound of Example 3-4, 3-43, 3-44, 3-45, 3-46, 3-47, 3-48, 3-50, 3-51,3-52, 3-53, 3-54, 3-55, 3-56, 3-57, 3-59, 3-60, 3-61, 3-62, 3-63, 3-64, 3-65, 3-66, 3-334, 3-373, 3-374, 3-375, 3-376, 3-377, 3-378, 3-380, 3-381,3-382, 3-383, 3-384, 3-385, 3-386, 3-387, 3-389, 3-390, 3-391,3-392, 3-393, 3-394, 3-395, 3-396, or 3-397.

346. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with an aryl group, wherein the aryl group may be substituted with a heterocyclic group, hydroxyl group, an alkoxy group which may be halogenated, an alkylenedioxy group, amino group, an alkylamino group, an alkylamino group substituted with hydroxyl group, an alkylamino group substituted with an alkyl group substituted with hydroxyl group, an alkylcarbonylamino group, or an alkylsulfonylamino group, and the moiety of the formula (2) mentioned later is represented by the formula (2-1), or (2-7).

347. The compound or a salt thereof according to 1, or 346, which is the compound of Example 1-4, 1-43, 1-44, 1-45, 1-46, 1-47, 1-48, 1-50, 1-51, 1-52, 1-53, 1-54, 1-55, 1-56, 1-57, 1-59, 1-60, 1-61, 1-62, 1-63, 1-64, 1-65, 1-66, 1-334, 1-373, 1-374, 1-375, 1-376, 1-377, 1-378, 1-380, 1-381, 1-382, 1-383, 1-384, 1-385, 1-386, 1-387, 1-389,

1-390, 1-391, 1-392, 1-393, 1-394, 1-395, 1-396, 3-4, 3-43, 3-44, 3-45, 3-46, 3-47, 3-48, 3-50, 3-51,3-52, 3-53, 3-54, 3-55, 3-56, 3-57, 3-59, 3-60, 3-61,3-62, 3-63, 3-64, 3-65, 3-66, 3-334, 3-373, 3-374, 3-375, 3-376, 3-377, 3-378, 3-380, 3-381,3-382, 3-383, 3-384, 3-385, 3-386, 3-387, 3-389, 3-390, 3-391,3-392, 3-393, 3-394, 3-395, or 3-396.

348. The compound or a salt thereof according to 1, or 346, wherein $R^1$ is hydrogen atom, or hydroxyl group, all of $R^5$, $R^6$, $R^7$, and $R^8$ are hydrogen atoms, $X^1...X^2$ is ethylene group, $A^{61}$ is an alkyl group substituted with an aryl group, wherein the aryl group may be substituted with hydroxyl group, an alkoxy group, an alkylenedioxy group, and the moiety of the formula (2) mentioned later is represented by the formula (2-1), or (2-7);
349. The compound or a salt thereof according to 1, 346, or 348, which is the compound of Example 1-4, 1-43, 1-44, 1-45, 1-46, 1-65, 1-66, 1-334, 1-373, 1-374, 1-375, 1-376, 1-389, 1-390, 1-391, 1-392, 1-393, 1-394, 1-395, 1-396, 3-4, 3-43, 3-44, 3-45, 3-46, 3-65, 3-66, 3-334, 3-373, 3-374, 3-375, 3-376, 3-389, 3-390, 3-391,3-392, 3-393, 3-394, 3-395, or 3-396.
350. The compound or a salt thereof according to 1, wherein $R^1$ is hydrogen atom, or hydroxyl group, all of $R^5$, $R^6$, $R^7$, and $R^8$ are hydrogen atoms, $X^1...X^2$ is ethylene group, $A^{61}$ is an alkyl group substituted with an aromatic heterocyclic group, and the moiety of the formula (2) mentioned later is represented by the formula (2-1), or (2-7)

351. The compound or a salt thereof according to 1, or 350, which is the compound of Example 1-67, 1-68, 1-69, 1-72, 1-397, 1-398, 1-399, 1-402, 3-67, 3-68, 3-69, 3-72, 3-397, 3-398, 3-399, or 3-402.
352. The compound or a salt thereof according to 1, wherein $R^1$ is hydrogen atom, or hydroxyl group, all of $R^5$, $R^6$, $R^7$, and $R^8$ are hydrogen atoms, $X^1\cdots X^2$ is ethylene group, $A^{61}$ is an alkyl group substituted with hydroxyl group, and the moiety of the formula (2) mentioned later is represented by the formula (2-2), (2-4-t), or (2-4-c)
353. The compound or a salt thereof according to 1, or 352, which is the compound of Example 2-11,2-79, 2-80, 2-82, 2-86, 2-88, 2-341,2-409, 2-410, 2-412, 2-416, 2-418, 5-3, 5-27, 5-28, 5-30, 5-31, 5-289, 5-303, 5-327, 5-328, 5-330, 5-331, or 5-589.
354. The compound or a salt thereof according to 1, or 352, wherein $R^1$ is hydrogen atom, or hydroxyl group, all of $R^5$, $R^6$, $R^7$, and $R^8$ are hydrogen atoms, $X^1\cdots X^2$ is ethylene group, $A^{61}$ is an alkyl group substituted with one hydroxyl group, and the moiety of the formula (2) mentioned later is represented by the formula (2-2), (2-4-t), or (2-4-c).

355. The compound or a salt thereof according to 1, 352, or 354, which is the compound of Example 2-79, 2-80, 2-86, 2-88, 2-409, 2-410, 2-416, 2-418, 5-3, 5-27, 5-28, 5-31, 5-303, 5-327, 5-328, or 5-331.
**356.** The compound or a salt thereof according to 1, or 352, wherein $R^1$ is hydrogen atom, or hydroxyl group, all of $R^5$, $R^6$, $R^7$, and $R^8$ are hydrogen atoms, $X^1\cdots X^2$ is ethylene group, $A^{61}$ is an alkyl group substituted with two hydroxyl groups, and the moiety of the formula (2) mentioned later is represented by the formula (2-2), (2-4-t), or (2-4-c).
357. The compound or a salt thereof according to 1, 352, or 356, which is the compound of Example 2-11,2-82, 2-341,2-412, 5-30, 5-289, 5-330, or 5-589.
358. The compound or a salt thereof according to 1, wherein $R^1$ is hydrogen atom, or hydroxyl group, all of $R^5$, $R^6$, $R^7$, and $R^8$ are hydrogen atoms, $X^1\cdots X^2$ is ethylene group, $A^{61}$ is an alkyl group substituted with amino group, or an alkyl group substituted with an alkylamino group, and the moiety of the formula (2) mentioned later is represented by the formula (2.2), (2-4-t), or (2.4.c)
359. The compound or a salt thereof according to 1, or 358, which is the compound of Example 2-21,2-83, 2-84, 2-85, 2-129, 2-133, 2-137, 2-138, 2-139, 2-141,2-144, 2-148, 2-149, 2-150, 2-151,2-351,2-413, 2-414, 2-415, 2-459, 2-463, 2-467, 2-468, 2-469, 2-471,2-474, 2-478, 2-479, 2-480, 2-481,2-523, 2-524, 5-53, 5-59, 5-60, 5-61, 5-63, 5-67, 5-71, 5-72, 5-73, 5-74, 5-353, 5-359, 5-360, 5-361, 5-363, 5-367, 5-371, 5-372, 5-373, or 5-374.

360. The compound or a salt thereof according to 1, or 358, wherein $R^1$ is hydrogen atom, or hydroxyl group, all of $R^5$, $R^6$, $R^7$, and $R^8$ are hydrogen atoms, $X^1...X^2$ is ethylene group, $A^{61}$ is an alkyl group substituted with two amino groups, or an alkyl group substituted with two alkylamino groups, and the moiety of the formula (2) mentioned later is represented by the formula (2-2).
361. The compound or a salt thereof according to 1, 358, or 360, which is the compound of Example 2-21, or 2-351.
362. The compound or a salt thereof according to 1, or 358, wherein $R^1$ is hydrogen atom, or hydroxyl group, all of

$R^5$, $R^6$, $R^7$, and $R^8$ are hydrogen atoms, $X^1 \cdots X^2$ is ethylene group, $A^{61}$ is an alkyl group substituted with amino group (wherein the alkyl group is substituted with one or more hydroxyl groups), or an alkyl group substituted with an alkylamino group (wherein the alkyl group is substituted with one or more hydroxyl groups), and the moiety of the formula (2) mentioned later is represented by the formula (2-2), (2-4-t), or (2-4-c).

363. The compound or a salt thereof according to 1, 358, or 362, which is the compound of Example 2-83, 2-84, 2-85, 2-129, 2-133, 2-137, 2-138, 2-139, 2-413, 2-414, 2-415, 2-459, 2-463, 2-467, 2-468, 2-469, 5-53, 5-59, 5-60, 5-61, 5-353, 5-359, 5-360, or 5-361.

364. The compound or a salt thereof according to 1, or 358, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with an alkylamino group substituted with hydroxyl group, and the moiety of the formula (2) mentioned later is represented by the formula (2-2), (2-4-t), or (2-4-c).

365. The compound or a salt thereof according to 1, 358, or 364, which is the compound of Example 2-149, 2-150, 2-151,2-479, 2-480, 2-481, 5-72, 5-73, 5-74, 5-372, 5-373, or 5-374.

366. The compound or a salt thereof according to 1, or 358, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is pentyl group substituted with amino group, or pentyl group substituted with an alkylamino group, and the moiety of the formula (2) mentioned later is represented by the formula (2-2).

367. The compound or a salt thereof according to 1, 358, or 366, which is the compound of Example 2-141,2-144, 2-148, 2-471,2-474, or 2-478.

368. The compound or a salt thereof according to 1, or 358, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is butyl group substituted with amino group, or butyl group substituted with an alkylamino group, and the moiety of the formula (2) mentioned later is represented by the formula (2-4-t), or (2-4-c).

369. The compound or a salt thereof according to 1, 358, or 368, which is the compound of Example 5-63, 5-67, 5-71, 5-363, 5-367, or 5-371.

370. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group of which end is substituted with N ($A^7$)(-C(=O)-$A^{71}$), $A^{71}$ is an alkyl group, an alkyl group substituted with an aryl group, an alkyl group substituted with a heterocyclic group, an alkyl group substituted with hydroxyl group, an alkyl group substituted with amino group, an alkyl group substituted with an alkylamino group, an alkyl group substituted with an alkoxy group, an alkyl group substituted with an acylamino group, an aryl group, or an aromatic heterocyclic group, and the moiety of the formula (2) mentioned later is represented by the formula (2-2), (2-4-t), or (2-4-c)

371. The compound or a salt thereof according to 1, or 370, which is the compound of Example 2-20, 2-24, 2-25, 2-130, 2-134, 2-159, 2-160, 2-161,2-162, 2-163, 2-164, 2-165, 2-166, 2-167, 2-173, 2-174, 2-175, 2-176, 2-177, 2-179, 2-180, 2-181,2-182, 2-183, 2-184, 2-185, 2-186, 2-350, 2-354, 2-355, 2-460, 2-464, 2-489, 2-490, 2-491,2-492, 2-493, 2-494, 2-495, 2-496, 2-497, 2-503, 2-504, 2-505, 2-506, 2-507, 2-509, 2-510, 2-511,2-512, 2-513, 2-514, 2-515, 2-516, 5-54, 5-79, 5-84, 5-85, 5-86, 5-87, 5-88, 5-89, 5-90, 5-91, 5-92, 5-93, 5-94, 5-100, 5-101, 5-102, 5-103, 5-104, 5-106, 5-107, 5-108, 5-109, 5-110, 5-111, 5-112, 5-113, 5-114, 5-115, 5-116, 5-117, 5-118, 5-119, 5-120, 5-121, 5-122, 5-354, 5-379, 5-384, 5-385, 5-386, 5-387, 5-388, 5-389, 5-390, 5-391, 5-392, 5-393, 5-394, 5-400, 5-401, 5-402, 5-403, 5-404, 5-406, 5-407, 5-408, 5-409, 5-410, 5-411, 5-412, 5-413, 5-414, 5-415, 5-416, 5-417, 5-418, 5-419, 5-420, 5-421, or 5-422.

372. The compound or a salt thereof according to 1, or 370, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group of which end is substituted with N($A^7$)(-C(=O)-$A^{71}$), $A^{71}$ is an alkyl group, an alkyl group substituted with an aryl group, an alkyl group substituted with a heterocyclic group, an alkyl group substituted with hydroxyl group, an alkyl group substituted with amino group, an alkyl group substituted with an alkylamino group, an alkyl group substituted with an alkoxy group, an alkyl group substituted with an acylamino group, an aryl group, or an aromatic heterocyclic group, $A^7$ is not hydrogen atom, and the moiety of the formula (2) mentioned later is represented by the formula (2-2), (2-4-t), or (2-4-c).

373. The compound or a salt thereof according to 1, 370, or 372, which is the compound of Example 2-179, 2-180, 2-181,2-182, 2-183, 2-184, 1-185, 2-186, 2-509, 2-510, 2-511,2-512, 2-513, 2-514, 2-515, 2-516, 2-649, 5-106, 5-107, 5-108, 5-109, 5-110, 5-111, 5-112, 5-113, 5-114, 5-115, 5-116, 5-117, 5-118, 5-119, 5-120, 5-121, 5-122,

5-406, 5-407, 5-408, 5-409, 5-410, 5-411, 5-412, 5-413, 5-414, 5-415, 5-416, 5-417, 5-418, 5-419, 5-420, 5-421, or 5-422.

374. The compound or a salt thereof according to 1, or 370, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group of which end is substituted with $N(A^7)(-C(=O)-A^{71})$, $A^{71}$ is an alkyl group substituted with hydroxyl group, an alkyl group substituted with amino group, an alkyl group substituted with an alkylamino group, an alkyl group substituted with an alkoxy group, or an alkyl group substituted with an acylamino group, and the moiety of the formula (2) mentioned later is represented by the formula (2-2), (2-4-t), or (2-4-c).
375. The compound or a salt thereof according to 1, 370, or 374, which is the compound of Example 2-24, 2-25, 2-134, 2-159, 2-160, 2-166, 2-167, 2-354, 2-355, 2-464, 2-489, 2-490, 2-496, 2-497, 5-84, 5-85, 5-86, 5-92, 5-93, 5-94, 5-384, 5-385, 5-386, 5-392, 5-393, or 5-394.

376. The compound or a salt thereof according to 1, 370, or 372, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group of which end is substituted with $N(A^7)(-C(=O)-A^{71})$, $A^{71}$ is an alkyl group, an alkyl group substituted with an aryl group, an alkyl group substituted with a heterocyclic group, an alkyl group substituted with hydroxyl group, an alkyl group substituted with amino group, an alkyl group substituted with an alkylamino group, an alkyl group substituted with an alkoxy group, an alkyl group substituted with an acylamino group, an aryl group, or an aromatic heterocyclic group, $A^7$ is methyl group, and the moiety of the formula (2) mentioned later is represented by the formula (2-2), (2-4-t), or (2-4-c).
377. The compound or a salt thereof according to 1, 370, 372, or 376, which is the compound of Example 2-179, 2-180, 2-181,2-182, 2-509, 2-510, 2-511,2-512, 5-106, 5-107, 5-108, 5-109, 5-110, 5-406, 5-407, 5-408, 5-409, or 5-410.

378. The compound or a salt thereof according to 1, 370, or 372, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group of which end is substituted with $N(A^7)(-C(=O)-A^{71})$, $A^{71}$ is an alkyl group, an alkyl group substituted with an aryl group, an alkyl group substituted with a heterocyclic group, an alkyl group substituted with hydroxyl group, an alkyl group substituted with amino group, an alkyl group substituted with an alkylamino group, an alkyl group substituted with an alkoxy group, an alkyl group substituted with an acylamino group, an aryl group, or an aromatic heterocyclic group, $A^7$ is an alkyl group substituted with hydroxyl group, or an alkyl group substituted with cyano group, and the moiety of the formula (2) mentioned later is represented by the formula (2-2), (2-4-t), or (2-4-c).
379. The compound or a salt thereof according to 1, 370, 372, or 378, which is the compound of Example 2-183, 2-184, 1-185, 2-186, 2-513, 2-514, 2-515, 2-516, 5-111, 5-112, 5-113, 5-114, 5-115, 5-116, 5-117, 5-118, 5-119, 5-120, 5-121, 5-122, 5-411, 5-412, 5-413, 5-414, 5-415, 5-416, 5-417, 5-418, 5-419, 5-420, 5-421, or 5-422.

380. The compound or a salt thereof according to 1, or 370, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group of which end is substituted with $N(A^7)(\cdot C(=O)-A^{71})$, $A^{71}$ is an aryl group, and the moiety of the formula (2) mentioned later is represented by the formula (2-2), (2-4-t), or (2-4-c).
381. The compound or a salt thereof according to 1, 370, or 380, which is the compound of Example 2-161,2-491, 5-87, or 5-387.
382. The compound or a salt thereof according to 1, or 370, wherein $R^1$ is hydrogen atom, or hydroxyl group, all of $R^5$, $R^6$, $R^7$, and $R^8$ are hydrogen atoms, $X^1 ... X^2$ is ethylene group, $A^{61}$ is an alkyl group of which end is substituted with $N(A^7)(-C(=O)-A^{71})$, $A^{71}$ is an aromatic heterocyclic group, and the moiety of the formula (2) mentioned later is represented by the formula (2-2), (2-4-t), or (2-4-c).
383. The compound or a salt thereof according to 1, 370, or 382, which is the compound of Example 2-162, 2-163, 2-164, 2-165, 2-492, 2-493, 2-494, 2-495, 5-88, 5-89, 5-90, 5-91, 5-388, 5-389, 5-390, or 5-391.

384. The compound or a salt thereof according to 1, or 370, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with two of N $(A^7)(-C(=O)-A^{71})$ at the end, $A^{71}$ is an alkyl group, an alkyl group substituted with an aryl group, an alkyl group substituted with a heterocyclic group, an alkyl group substituted with hydroxyl group, an alkyl group substituted with amino group, an alkyl group substituted with an alkylamino group, an alkyl group substituted with an alkoxy group, an alkyl group substituted with an acylamino group, an aryl group, or an aromatic heterocyclic group, and the moiety of the formula (2) mentioned later is represented by the formula (2-2), (2-4-t), or (2-4-c).
385. The compound or a salt thereof according to 1, 370, or 384, which is the compound of Example 2-20, 2-350, 5-79, or 5-379.
386. The compound or a salt thereof according to 1, or 370, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is n-butyl group of which end is substituted with $N(A^7)(-C(=O)-A^{71})$, $A^{71}$ is an alkyl group, an alkyl group substituted with an aryl group, an alkyl group substituted with a heterocyclic group, an alkyl group substituted with hydroxyl group, an alkyl group substituted with amino group, an alkyl group substituted with an alkylamino group, an alkyl group substituted with an alkoxy group, an alkyl group substituted with an acylamino group, an aryl group, or an aromatic heterocyclic group, $A^7$ is hydrogen atom, and the moiety of the formula (2) mentioned later is represented by the formula (2-2), (2-4-t), or (2-4-c).
387. The compound or a salt thereof according to 1, 370, or 386, which is the compound of Example 2-173, 2-174, 2-175, 2-176, 2-177, 2-503, 2-504, 2-505, 2-506, 2-507, 5-100, 5-101, 5-102, 5-103, 5-104, 5-400, 5-401, 5-402, 5-403, or 5-404.

388. The compound or a salt thereof according to 1, or 370, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group of which end is substituted with $N(A^7)(-C(=O)-A^{71})$(wherein the alkyl group is substituted with one or more hydroxyl groups), $A^{71}$ is an alkyl group, an alkyl group substituted with an aryl group, an alkyl group substituted with a heterocyclic group, an alkyl group substituted with hydroxyl group, an alkyl group substituted with amino group, an alkyl group substituted with an alkylamino group, an alkyl group substituted with an alkoxy group, an alkyl group substituted with an acylamino group, an aryl group, or an aromatic heterocyclic group, and the moiety of the formula (2) mentioned later is represented by the formula (2-2), (2-4-t), or (2-4-c).
389. The compound or a salt thereof according to 1, 370, or 388, which is the compound of Example 2-130, 2-460, 5-54, or 5-354.
390. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is a cycloalkyl group, and the moiety of the formula (2) mentioned later is represented by the formula (2-2), (2-4-t), or (2-4-c)
391. The compound or a salt thereof according to 1, or 390, which is the compound of Example 2-12, 2-27, 2-28, 2-38, 2-39, 2-40, 2-187, 2-188, 2-189, 2-190, 2-191,2-215, 2-216, 2-217, 2-218, 2-342, 2-357, 2-358, 2-368, 2-369, 2-370, 2-517, 2-518, 2-519, 2-521,2-545, 2-546, 2-547, 2-548, 5-13, 5-123, 5-124, 5-125, 5-146, 5-313, 5-423, 5-424, 5-425, or 5-446.

392. The compound or a salt thereof according to 1, or 390, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is a substituted cycloalkyl group, and the moiety of the formula (2) mentioned later is represented by the formula (2-2), (2-4-t), or (2-4-c).
393. The compound or a salt thereof according to 390, or 392, which is the compound of Example 2-12, 2-27, 2-28, 2-187, 2-188, 2-189, 2-190, 2-191,2-215, 2-216, 2-217, 2-218, 2-342, 2-357, 2-358, 2-517, 2-518, 2-519, 2-521,2-545, 2-546, 2-547, 2-548, 5-123, 5-124, 5-125, 5-146, 5-423, 5-424, 5-425, or 5-446.
394. The compound or a salt thereof according to 390, or 392, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is a cycloalkyl group substituted with amino group, or a cycloalkyl group substituted with alkylamino group, and the moiety of the formula (2) mentioned later is represented by the formula (2-2), (2-4-t), or (2-4-c).
395. The compound or a salt thereof according to 1, 390, 392, or 394, which is the compound of Example 2-27, 2-188, 2-216, 2-218, 2-357, 2-518, 2-546, 2-548, 5-124, or 5-424.

396. The compound or a salt thereof according to 1, 390, or 392, wherein $R^1$ is hydrogen atom, or hydroxyl group,

all of $R^5$, $R^6$, $R^7$, and $R^8$ are hydrogen atoms, $X^1 \cdots X^2$ is ethylene group, $A^{61}$ is a cycloalkyl group substituted with hydroxyl group, and the moiety of the formula (2) mentioned later is represented by the formula (2-2), (2-4-t), or (2-4-c).
397. The compound or a salt thereof according to 1, 390, 392. or 396, which is the compound of Example 2-12, 2-187, 2-342, 2-517, 5-123, or 5-423.
**398.** The compound or a salt thereof according to 1, 390, or 392, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is a cycloalkyl group substituted with an alkylcarbonylamino group, a cycloalkyl group substituted with an arylcarbonylamino group, or a cycloalkyl group substituted with an alkylsulfonylamino group, and the moiety of the formula (2) mentioned later is represented by the formula (2-2), (2-4-t), or (2-4-c).
399. The compound or a salt thereof according to 1, 390, 392, or 398, which is the compound of Example 2-28, 2-189, 2-190, 2-191,2-215, 2-217, 2-358, 2-519, 2-520, 2-521,2-545, 2-547, 5-125, 5-146, 5-425, or 5-446.

400. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is a 4- to 8-membered non-aromatic heterocyclic group containing one or more hetero atoms, and the moiety of the formula (2) mentioned later is represented by the formula (2-1), (2-2), (2-3), (2-4-t), (2-4-c), (2-5-t), (2-5-c), (2-6-t), (2-6-c), or (2-7). 401. The compound or a salt thereof according to 1, or 400, which is the compound of Example 1-23, 1-192, 1-193, 1-194, 1-318, 1-319, 1-353, 1-522, 1-523, 1-524, 1-648, 1-649, 2-23, 2-192, 2-193, 2-194, 2-318, 2-319, 2-353, 2-522, 2-523, 2-524, 2-648, 2-649, 3-23, 3-192, 3-193, 3-194, 3-318, 3-319, 3-353, 3-522, 3-523, 3-524, 3-648, 3-649, 4-23, 4-192, 4-193, 4-194, 4-318, 4-319, 4-353, 4-522, 4-523, 4-524, 4-648, 4-649, 5-290, 5-590, 6-290, 6-590, 7-290, or 7-590.

**402.** The compound or a salt thereof according to 1, or 400, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is a 4- to 8-membered non-aromatic heterocyclic group containing one hetero atom, or a substituted non-aromatic heterocyclic group, and the moiety of the formula (2) mentioned later is represented by the formula (2-1), (2-2), (2-3), or (2-7).
403. The compound or a salt thereof according to 1, 400, or 402, which is the compound of Example 1-192, 1-193, 1-194, 1-318, 1-319, 1-522, 1-523, 1-524, 1-648, 1-649, 2-192, 2-193, 2-194, 2-318, 2-319, 2-522, 2-523, 2-524, 2-648, 2-649, 3-192, 3-193, 3-194, 3-318, 3-319, 3-522, 3-523, 3-524, 3-648, 3-649, 4-192, 4-193, 4-194, 4-318, 4-319, 4-522, 4-523, 4-524, 4-648, or 4-649.

404. The compound or a salt thereof according to 1, 400, or 402, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is a 5- to 8-membered saturated non-aromatic heterocyclic group containing one nitrogen atom (said nitrogen atom may be substituted with an alkyl group), oxygen atom, or sulfur atom, and the moiety of the formula (2) mentioned later is represented by the formula (2-1), (2-2), (2-3), or (2-7).
405. The compound or a salt thereof according to 1, 400, 402, or 404, which is the compound of Example 1-192, 1-193, 1-194, 1-318, 1-522, 1-523, 1-524, 1-648, 2-192, 2-193, 2-194, 2-318, 2-522, 2-523, 2-524, 2-648, 3-192, 3-193, 3-194, 3-318, 3-522, 3-523, 3-524, 3-648, 4-192, 4-193, 4-194, 4-318, 4-522, 4-523, 4-524, or 4-648.

406. The compound or a salt thereof according to 1, 400, or 402, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is a 4- to 8-membered saturated non-aromatic heterocyclic group containing one nitrogen atom (said nitrogen atom is substituted with an alkylcarbonyl group, an arylcarbonyl group, an alkylsulfonyl group, or an arylsulfonyl group), and the moiety of the formula (2) mentioned later is represented by the formula (2-1), (2-2), (2-3), or (2-7).
407. The compound or a salt thereof according to 1, 400, 402, or 406, which is the compound of Example 1-319, 1-649, 2-319, 2-649, 3-319, 3-649, 4-319, or 4-649.
408. The compound or a salt thereof according to 1, or 400, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is a 6- to 8-membered saturated non-aromatic heterocyclic group containing two hetero atoms (nitrogen atoms may be substituted with an alkyl group), and the moiety of the formula (2) mentioned later is represented by the formula (2-1), (2-2), (2-3), (2-4-t), (2-4-c), (2-5-t),

(2-5-c), (2-6-t), (2-6-c), or (2-7).
409. The compound or a salt thereof according to 1, 400, or 408, which is the compound of Example 1-23, 1-353, 2-23, 2-353, 3-23, 3-353, 4-23, 4-353, 5-290, 5-590, 6-290, 6-590, 7-290, or 7-590.

410. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with an aryl group, wherein the aryl group may be substituted with a lower alkyl group, and the moiety of the formula (2) mentioned later is represented by the formula (2-1), or (2-7)
411. The compound or a salt thereof according to 1, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with a substituted aryl group, and the moiety of the formula (2) mentioned later is represented by the formula (2-1)
412. The compound or a salt thereof according to 1, or 411, which is the compound of Example 3-4, 3-42, 3-43, 3-44, 3-45, 3-46, 3-47, 3-48, 3-50, 3-51,3-52, 3-53, 3-54, 3-55, 3-56, 3-57, 3-59, 3-60, 3-61,3-62, 3-63, 3-64, 3-65, 3-66, 3-241,3-242, 3-299, 3-314, 3-315, 3-334, 3-372, 3-373, 3-374, 3-375, 3-376, 3-377, 3-378, 3-380, 3-381,3-382, 3-383, 3-384, 3-385, 3-386, 3-387, 3-389, 3-390, 3-391,3-392, 3-393, 3-394, 3-395, 3-396, 3-571,3-572, 3-629, 3-644, or 3-645.

413. The compound or a salt thereof according to 1, or 410, which is the compound of Example 1-241, 1-242, 1-299, 1-571, 1-572, 1-629, 3-241,3-242, 3-299, 3-571,3-572, or 3-629.
414. The compound or a salt thereof according to 1, or 400, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with an aryl group, wherein the aryl group may be substituted with a lower alkyl group, and the moiety of the formula (2) mentioned later is represented by the formula (2-1).
415. The compound or a salt thereof according to 1, or 410, wherein $R^1$ is hydrogen atom, or hydroxyl group, all of $R^5$, $R^6$, $R^7$, and $R^8$ are hydrogen atoms, $X^1 \cdots X^2$ is ethylene group, $A^{61}$ is an alkyl group substituted with an aryl group, wherein the aryl group may be substituted with a lower alkyl group, and the moiety of the formula (2) mentioned later is represented by the formula (2-7).
416. The compound or a salt thereof according to 1, 410, or 414, which is the compound of Example 1-241, 1-242, 1-299, 1-571, 1-572, or 1-629.
417. The compound or a salt thereof according to 1, 410, or 415, which is the compound of Example 3-241,3-242, 3-299, 3-571,3-572, or 3-629.

From another aspect, the present invention provides a medicament containing a compound represented by the aforementioned formula (1) or a physiologically acceptable salt thereof as an active ingredient. The aforementioned medicament has an inhibitory action on phosphorylation of myosin regulatory light chain, and is useful as a medicament for prophylactic and/or therapeutic treatment of, for example, a disease relating to cell contraction, disease relating to change of cell morphology, disease relating to cell migration, disease relating to cell release, disease relating to cell aggregation, and/or disease relating to cell apoptosis and the like.
More specifically, there are provided a medicament for decreasing phosphorylation amount of myosin regulatory light chain in a cell, which comprises a compound represented by the aforementioned formula (1) or a physiologically acceptable salt thereof as an active ingredient, a medicament having a cell contraction inhibitory action, which comprises a compound represented by the aforementioned formula (1) or a physiologically acceptable salt thereof as an active ingredient, a medicament having an action to regulate change of cell morphology, which comprises a compound represented by the aforementioned formula (1) or a physiologically acceptable salt thereof as an active ingredient, a medicament having a cell migration inhibitory action, which comprises a compound represented by the aforementioned formula (1) or a physiologically acceptable salt thereof as an active ingredient, a medicament having a cell release inhibitory action, which comprises a compound represented by the aforementioned formula (1) or a physiologically acceptable salt thereof as an active ingredient, a medicament having a cell aggregation inhibitory action, which comprises a compound represented by the aforementioned formula (1) or a physiologically acceptable salt thereof as an active ingredient, a medicament having a cell apoptosis inhibitory action, which comprises a compound represented by the aforementioned formula (1) or a physiologically acceptable salt thereof as an active ingredient, and a medicament for inhibiting the Rho/Rho kinase pathway, which comprises a compound represented by the aforementioned formula (1) or a physiologically acceptable salt thereof as an active ingredient.
The present invention also provides an inhibitor of the phosphorylation of myosin regulatory light chain containing a

compound represented by the aforementioned formula (1) or a physiologically acceptable salt thereof, and an inhibitor of the Rho/Rho kinase pathway comprising a compound represented by the aforementioned formula (1) or a physiologically acceptable salt thereof.

The present invention further provides a medicament comprising a combination of a compound represented by the aforementioned formula (1) or a physiologically acceptable salt thereof and a jointly-used drug.

The aforementioned combined medicament is useful as a medicament for prophylactic and/or therapeutic treatment of, for example, a disease relating to cell contraction, a disease relating to change of cell morphology, a disease relating to cell migration, a disease relating to cell release, a disease relating to cell aggregation, and/or a disease relating to cell apoptosis and the like. More specifically, the present invention also provides a medicament for use in prophylactic and/or therapeutic treatment of a disease relating to cell contraction, which comprises a combination of a compound represented by the aforementioned formula (1) or a physiologically acceptable salt thereof and a jointly-used drug, a medicament for use in prophylactic and/or therapeutic treatment of a disease relating to change of cell morphology, which comprises a combination of a compound represented by the aforementioned formula (1) or a physiologically acceptable salt thereof and a jointly-used drug, a medicament for use in prophylactic and/or therapeutic treatment of a disease relating to cell migration, which comprises a combination of a compound represented by the aforementioned formula (1) or a physiologically acceptable salt thereof and a jointly-used drug, a medicament for use in prophylactic and/or therapeutic treatment of a disease relating to cell release, which comprises a combination of a compound represented by the aforementioned formula (1) or a physiologically acceptable salt thereof and a jointly-used drug, a medicament for use in prophylactic and/or therapeutic treatment of a disease relating to cell aggregation, which comprises a combination of a compound represented by the aforementioned formula (1) or a physiologically acceptable salt thereof and a jointly-used drug, and a medicament for use in prophylactic and/or therapeutic treatment of a disease relating to cell apoptosis, which comprises a combination of a compound represented by the aforementioned formula (1) or a physiologically acceptable salt thereof and a jointly-used drug.

From another aspect, the present invention provides use of a compound represented by the aforementioned formula (1) or a physiologically acceptable salt thereof for manufacture of the aforementioned medicaments.

The present invention also provides a method for prophylactic and/or therapeutic treatment of a disease relating to cell contraction, disease relating to change of cell morphology, disease relating to cell migration, disease relating to cell release, disease relating to cell aggregation, and/or disease relating to cell apoptosis and the like, which comprises the step of administrating a prophylactically and/or therapeutically effective amount of a compound represented by the aforementioned formula (1) or a physiologically acceptable salt thereof to a mammal including human, a method for decreasing phosphorylation amount of myosin regulatory light chain in a cell, which comprises the step of administrating an effective amount of a compound represented by the aforementioned formula (1) or a physiologically acceptable salt thereof to a mammal including human, a method for inhibiting cell contraction, which comprises the step of administrating an effective amount of a compound represented by the aforementioned formula (1) or a physiologically acceptable salt thereof to a mammal including human, a method for regulating change of cell morphology, which comprises the step of administrating an effective amount of a compound represented by the aforementioned formula (1) or a physiologically acceptable salt thereof to a mammal including human, a method for inhibiting cell migration, which comprises the step of administrating an effective amount of a compound represented by the aforementioned formula (1) or a physiologically acceptable salt thereof to a mammal including human, a method for inhibiting cell release, which comprises the step of administrating an effective amount of a compound represented by the aforementioned formula (1) or a physiologically acceptable salt thereof to a mammal including human, a method for inhibiting cell aggregation, which comprises the step of administrating an effective amount of a compound represented by the aforementioned formula (1) or a physiologically acceptable salt thereof to a mammal including human, a method for inhibiting cell apoptosis, which comprises the step of administrating an effective amount of a compound represented by the aforementioned formula (1) or a physiologically acceptable salt thereof to a mammal including human, and a method for inhibiting the Rho/Rho kinase pathway, which comprises the step of administrating an effective amount of a compound represented by the aforementioned formula (1) or a physiologically acceptable salt thereof to a mammal including human.

[Effect of the Invention]

The compounds of the present invention represented by the formula (1) have inhibitory activity against the myosin regulatory light chain phosphorylation, and are useful as active ingredients of medicaments for prophylactic and/or therapeutic treatment of, for example, diseases relating to contraction of various cells, diseases relating to morphological change of various cells, diseases relating to migration of various cells, diseases relating to release of various cells, diseases relating to aggregation of various cells, diseases relating to apoptosis of various cells, diseases relating to abnormal gene expression in various cells and the like.

[Best Mode for Carrying out the Invention]

The alkyl group mentioned in this specification may be a linear or branched alkyl group, and for example, a lower alkyl group is preferred. The lower alkyl group include linear or branched alkyl groups having 1 to 6 carbon atoms, and specific examples are, for example, methyl group, ethyl group, propyl group, isopropyl group, butyl group, secondary butyl group, tertiary butyl group, pentyl group, 2-methylbutyl group, hexyl group and the like. As the lower alkyl group in $R^2$, $R^3$, $R^4$, $A^1$, $A^{11}$, $A^2$, $A^{21}$, $A^3$, $A^4$, $A^{41}$, A5, $A^{51}$, $A^6$, and $A^{61}$, methyl group, or ethyl group is independently preferred. The "cycloalkyl" is a saturated alicyclic hydrocarbon group, and examples include, for example, a monocyclic $C_{3-9}$ cycloalkyl such as cyclopropyl, cyclobutyl, cyclopentyl, cyclohexyl, cycloheptyl, cyclooctyl, and cyclononyl, bicyclic or tricyclic $C_{6-10}$ cycloalkyl such as adamantly and the like. Examples of the "lower cycloalkyl" include, for example, a $C_{3-6}$ cycloalkyl and the like. Examples of the "aryl" include a monocyclic or condensed polycyclic aromatic hydrocarbon group. For example, a $C_{6-14}$ aryl group such as phenyl, naphthyl, anthryl, phenanthryl, and acenaphthylenyl, are preferred, phenyl, 1-naphthyl, 2-naphthyl and the like are especially preferred, and phenyl is particularly preferred.

Examples of the "heterocyclic group" include, for example, an aromatic heterocyclic group and a saturated or unsaturated non-aromatic heterocyclic group (aliphatic heterocyclic group) containing 1 to 3 kinds (preferably 1 to 2 kinds) of at least one (preferably 1 to 4, more preferably 1 or 2) hetero atoms selected from oxygen atom, sulfur atom, nitrogen atom and the like as atoms constituting the ring system (ring atoms). Examples of the "aromatic heterocyclic group" include, for example, a 5-or 6-membered aromatic monocyclic heterocyclic group such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, and triazinyl. Examples also include a 8- to 12-membered condensed aromatic heterocyclic group (preferably a heterocyclic group comprising a 5-or 6-membered aromatic monocyclic heterocyclic group among those mentioned above condensing to benzene ring, or a heterocyclic group comprising two of the same or different 5- or 6-membered aromatic monocyclic heterocyclic groups among those mentioned above, more preferably a heterocyclic group comprising a 5- or 6-membered aromatic monocyclic heterocyclic group among those mentioned above condensing to benzene ring), such as benzofuranyl, isobenzofuranyl, benzo[b]thienyl, indolyl, isoindolyl, 1H-indazolyl, benzindazolyl, benzoxazolyl, 1,2-benzisoxazolyl, benzothiazolyl, benzopyranyl, 1,2-benzisothiazolyl, 1H-benzotriazolyl, quinolyl, isoquinolyl, cinnolinyl, quinazolinyl, quinoxalinyl, phthalazinyl, naphthylidinyl, purinyl, pteridinyl, carbazolyl, α-carbolinyl, β-carbolinyl, γ-carbolinyl, acridinyl, phenoxazinyl, phenothiazinyl, indolidinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo [4,3-a]pyridyl, and 1,2,4-triazolo[4,3-b]pyridazinyl.

Examples of the "non-aromatic heterocyclic group" include, for example, a 3- to 8-membered (preferably 5- or 6-membered) saturated or unsaturated (preferably saturated) non-aromatic heterocyclic group (aliphatic heterocyclic group), such as oxylanyl, azetidinyl, oxetanyl, thietanyl, pyrrolidinyl, tetrahydrofuryl, tetrahydrothienyl, piperidinyl, tetrahydropyranyl, tetrahydrothiopyranyl, morpholinyl, thiomorpholinyl, piperazinyl, 2(1H)-oxotetrahydropyrimidyl, and homopiperazinyl, a non-aromatic heterocyclic group corresponding to the aforementioned aromatic monocyclic heterocyclic group or aromatic condensed heterocyclic group a part or all of which double bonds are saturated, such as 1,2,3,4-tetrahydroquinolyl, and 1,2,3,4-tetrahydroisoquinolyl and the like. Examples of $R^1$, $R^5$, $R^6$, $R^7$, and $R^8$ independently include hydrogen atom, a halogen atom, hydroxyl group, an alkyl group, an alkenyl group, an alkynyl group, an alkoxyl group, amino group, an alkylamino group, and an aryl amino group. Examples of the halogen atom referred to herein include fluorine atom, chlorine atom, bromine atom, and iodine atom, and fluorine atom, chlorine atom, or bromine atom are preferred.

Examples of $R^1$ include hydrogen atom, chlorine atom, and hydroxyl group. Preferred examples of $R^1$ include hydrogen atom, and hydroxyl group. It is also preferred that these two groups are chosen as $R^1$, and it is also preferred that either hydrogen atom or hydroxyl group is selected.
Examples of $R^1$ include fluorine atom, bromine atom, iodine atom, an alkyl group, an alkenyl group, an alkynyl group, an alkoxyl group, amino group, an alkylamino group, and an arylamino group.
It is preferred that all of $R^5$, $R^6$, $R^7$, and $R^8$ are hydrogen atoms.
It is further preferred that arbitrary three of $R^5$, $R^6$, $R^7$, and $R^8$ are hydrogen atoms.
As for $R^1$, $R^5$, $R^6$, $R^7$, and $R^8$, examples of the "alkyl group" include the aforementioned lower alkyl group and the like, and preferred examples include methyl, and ethyl. It is preferred that one of $R^1$, $R^5$, $R^6$, $R^7$, or $R^8$ is an alkyl group, and the others are hydrogen atoms. It is also preferred that, for example, $R^1$ is hydroxyl group, one of $R^5$, $R^6$, $R^7$, or $R^8$ is an alkyl group, and the others are hydrogen atoms.

As for $R^1$, $R^5$, $R^6$, $R^7$, and $R^8$, examples of the "alkenyl group" include a $C_{2-6}$ alkenyl group, such as vinyl, allyl, isopropenyl, 2-methylallyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, and 5-hexenyl, and preferred examples include vinyl and allyl. It is preferred that, for example, one of $R^1$, $R^5$, $R^6$, $R^7$, and $R^8$ is an alkenyl group, and the others are hydrogen atoms. It is also preferred that, for

example, $R^1$ is hydroxyl group, one of $R^5$, $R^6$, $R^7$, and $R^8$ is an alkenyl group, and the others are hydrogen atoms.

As for $R^1$, $R^5$, $R^6$, $R^7$, and $R^8$, examples of the "alkynyl group" include, for example, a $C_{2-6}$ alkynyl group such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, and 5-hexynyl, and preferred examples include ethynyl and 1-propynyl. It is preferred that, for example, one of $R^1$, $R^5$, $R^6$, $R^7$, and $R^8$ is an alkynyl group, and the others are hydrogen atoms. It is also preferred that, for example, $R^1$ is hydroxyl group, one of $R^5$, $R^6$, $R^7$, and $R^8$ is an alkynyl group, and the others are hydrogen atoms.

As for $R^1$, $R^5$, $R^6$, $R^7$, and $R^8$, examples of the "alkoxyl group" include, for example, a $C_{1-4}$ alkoxyl group such as methoxy, ethoxy, 1-propoxy, isopropoxy, 1-butoxy, isobutoxy, and t-butoxy and the like, and preferred example include methoxy and ethoxy. It is preferred that, for example, one of $R^1$, $R^5$, $R^6$, $R^7$, or $R^8$ is an alkoxyl group, and the others are hydrogen atoms. It is also preferred that, for example, $R^1$ is hydroxyl group, one of $R^5$, $R^6$, $R^7$, or $R^8$ is an alkoxyl group, and the others are hydrogen atoms.

As for $R^1$, $R^5$, $R^6$, $R^7$, and $R^8$, examples of the "alkylamino group" include a mono(lower alkyl)amino group, and a di(lower alkyl)amino groups. Examples of the mono(lower alkyl)amino group include, for example, a mono($C_{1-6}$) alkylamino group such as methylamino, ethylamino, and propylamino, and examples of the di(lower alkyl)amino group include a di($C_{1-6}$)alkylamino groups such as dimethylamino, and diethylamino. It is preferred that, for example, one of $R^1$, $R^5$, $R^6$, $R^7$, or $R^8$ is an alkylamino group, and the others are hydrogen atoms. It is also preferred that, for example, $R^1$ is hydroxyl group, one of $R^5$, $R^6$, $R^7$, and $R^8$ is an alkylamino group, and the others are hydrogen atoms.

As for $R^1$, $R^5$, $R^6$, $R^7$, and $R^8$, the "aryl amino group" is an amino group substituted with an aryl group, and examples include, for example, a monoarylamino group, and a diarylamino groups. Examples of the aryl group in the aryl amino group include the aforementioned groups, and preferred examples include phenylamino. It is preferred that, for example, one of $R^1$, $R^5$, $R^6$, $R^7$, or $R^8$ is an arylamino group, and the others are hydrogen atoms. It is also preferred that, for example, $R^1$ is hydroxyl group, one of $R^5$, $R^6$, $R^7$, or $R^8$ is an aryl amino group, and the others are hydrogen atoms.

Examples of $-X^1\cdots X^2-$ include $-CH(R^2)-CH(R^3)-$, $-CH(R^2)-CH(R^3)-CH(R^4)-$, $-C(R^2) =C(R^3)-$, and $-C(R^2)=C(R^3)-CH(R^4)-$. As $-X^1\cdots X^2-$, $-CH(R^2)-CH(R^3)-$, $-CH(R^2)-CH(R^3)-CH(R^4)-$, or $-C(R^2)=C(R^3)-$ is preferred, $-CH(R^2)-CH(R^3)-$, or $-C(R^2)=C(R^3)-$ is particularly preferred. $R^2$, $R^3$, and $R^4$ may be the same or different, and they are preferably represent hydrogen atom, or a lower alkyl group, more preferably hydrogen atom, or methyl group. It is particularly preferred that all of these substituents are hydrogen atoms, and it is also preferred that an arbitrary one of these substituents is methyl group, and all of the remaining substituents are hydrogen atoms. Preferred example of $-CH(R^2)-CH(R^3)-$ include $-CH_2-CH_2-$, $-CH(CH_3)-CH_2-$, and $-CH_2-CH(CH_3)-$, and most preferred examples include $-CH_2-CH_2-$ (ethylene group). Preferred examples of $-C(R^2)=C(R^3)-$ include $-CH=CH-$, $-C(CH_3)=CH-$, and $-CH=C(CH_3)-$.

$A^1$, $A^{11}$, $A^2$, and $A^{21}$ may be the same or different, and they preferably represent hydrogen atom, or a lower alkyl group, more preferably hydrogen atom, or methyl group. It is particularly preferred that all of these substituents are hydrogen atoms, and it is also preferred that an arbitrary one of these substituents is methyl group, and all of the remaining substituents are hydrogen atoms.

Y represents $-CH(A^3)-$, $-CH(A^3)-C(A4)(A4i)-$, $-CH(A^3)-C(A^4)(A^{41})-C(A^5) A^{51})-$, or a single bond, and as for $-CH(A^3)-C(A^4)(A^{41})-$, and $-CH(A^3)-C(A^4)(A^{41})-C(A^5)(A^{51})-$, $-CH(A^3)-$ binds to N (nitrogen atom) bonding to $X^2$. As Y, $-CH(A^3)-$, $-CH(A^3)-C(A^4)(A^{41})-$, or a single bond is preferred.

$A^3$ preferably represents hydrogen atom, or a lower alkyl group, and a more preferred example is hydrogen atom. $A^3$ is also preferably a lower alkyl group, and particularly preferred examples are methyl group, and ethyl group.

It is preferred that $A^4$ and $A^{41}$ independently represent hydrogen atom, or a lower alkyl group, and it is preferred that, for example, both of them represent hydrogen atom. It is also preferred that either one of $A^4$ and $A^{41}$ is hydrogen atom, and the other is a lower alkyl group. Further, it is also preferred that $A^4$ 4 and $A^{41}$ are lower alkyl groups, which may be the same or different. Preferred examples of the lower alkyl group as $A^4$ or $A^{41}$ are methyl group, and ethyl group. It is preferred that $A^5$ and $A^{51}$ independently represent hydrogen atom, or a lower alkyl group, and it is preferred that, for example, both of them are hydrogen atoms. It is also preferred that either one of $A^5$ and $A^{51}$ is hydrogen atom, and the other is a lower alkyl group. Further, it is also preferred that $A^5$ and $A^{51}$ are lower alkyl groups, which may be the same or different. Preferred examples of the lower alkyl group as $A^5$ or $A^{51}$ are methyl group, and ethyl group.

Z represents hydroxyl group, or $N(A^6)(A^{61})$. Z is preferably hydroxyl group. It is also preferred that Z is $N(A^6)(A^{61})$. In such a compound, $A^6$ is hydrogen atom or an alkyl group, preferably hydrogen atom, or a lower alkyl group, most preferably hydrogen atom, or methyl group. It is also preferred that, for example, $A^6$ represents either one of them, or a combination of two of them.

$A^{61}$ represents hydrogen atom, an alkyl group, an alkyl group substituted with an aryl group, an alkyl group substituted with an aromatic heterocyclic group, an alkyl group substituted with carboxyl group, an alkyl group substituted with cyano group, an alkyl group substituted with hydroxyl group, an alkyl group substituted with an alkoxy group, an alkyl group substituted with a substituted alkoxy group, an alkyl group substituted with amino group, an alkyl group substituted with an alkylamino group, an alkyl group substituted with aminocarbonyl group, an alkyl group substituted with an alkylaminocarbonyloxy group, an alkyl group substituted with a cycloalkylaminocarbonyloxy group, an alkyl group

substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$.

Examples of $A^{61}$ include, for example, hydrogen atom, a lower alkyl group, a lower alkyl group substituted with an aryl group, a lower alkyl group substituted with carboxyl group, a lower alkyl group substituted with cyano group, a lower alkyl group substituted with hydroxyl group, a lower alkyl group substituted with a lower alkoxy group, a lower alkyl group substituted with amino group, a lower alkyl group substituted with a lower alkylamino group, a lower alkyl group substituted with aminocarbonyl group, a lower alkyl group substituted with alkylaminocarbonyloxy group, a lower alkyl group substituted with a cycloalkylaminocarbonyloxy group, a lower alkyl group substituted with an arylaminocarbonyloxy group, a lower alkyl group substituted with mercapto group, a lower alkyl group substituted with a lower alkylthio group, a lower alkyl group substituted with an acylthio group, a $C_{3-9}$ cycloalkyl group, a substituted $C_{3-9}$ cycloalkyl group, carbamimidoyl group, a lower alkylcarbonyl group, a 4- to 8-membered non-aromatic heterocyclic group, a substituted 4- to 8-membered non-aromatic heterocyclic group, and a lower alkyl group of which end is substituted with $N(A^7)(-X^3-A^{71})$.
$A^{61}$ is preferably hydrogen atom.
Further, $A^{61}$ is also preferably a lower alkyl group, and this lower alkyl group is most preferably methyl group, or ethyl group. It is also preferred that, for example, $A^{61}$ represents either one of them, or a combination of two of them.
$A^{61}$ is also preferably an a lower alkyl group substituted with an aryl group.

Examples of the lower alkyl group substituted with an aryl group herein referred to include a $C_{7-10}$ aralkyl group and the like, and examples include benzyl group, and 2-phenylethyl group. Particularly preferred examples include benzyl group. These groups may be substituted with a lower alkyl group, a halogen atom, a lower alkyl group substituted with a halogen atom, an alkyl group substituted with carboxyl group, an aryl group, a heterocyclic group, hydroxyl group, an alkoxy group which may be halogenated, an alkylenedioxy group, an alkyl group substituted with an alkoxycarbonyl group, nitro group, amino group, an alkylamino group, an alkyl group substituted with hydroxyl group, an alkylamino group substituted with hydroxyl group, an alkylamino group substituted with an alkyl group substituted with hydroxyl group, an alkylcarbonylamino group, an alkylamino group substituted with an alkylcarbonyl group, an alkylsulfonylamino group, an arylsulfonylamino group, an alkyl-NH-C(=O)-NH group, a substituted alkyl-NH-C(=O)-NH group, or the like. One to four, preferably one or two, of these groups may substitute on the aryl ring (provided that only one alkylenedioxy group can substitute on the aryl ring), and when two or more substituents substitute, the substituents are independent from each other, or one another, and may be the same or different. Further, when the aryl ring is phenyl, the substitution positions of these substituents may be, for example, 2-, 3-, 4-, 5-, or 6-position. When there is one substituent, the substitution position is, for example, 2-, 3-, or 4-position, 3-position, or 4-position is preferred, and the 4-position is most preferred. When there are two substituents, the substitution positions are preferably (2-position, 3-position), (2-position, 4-position), (2-position, 5-position), (3-position, 4-position), or (3-position, 5-position), particularly preferably (3-position, 4-position), or (3-position, 5-position).

Preferred examples of the substituent of the "lower alkyl group substituted with an aryl group" include a lower alkyl group, and particularly preferred examples include methyl group, and ethyl group. Preferred examples of the substituent of the "lower alkyl group substituted with an aryl group" also include a halogen atom, and particularly preferred examples include fluorine atom, chlorine atom, and bromine atom. Preferred examples of the substituent of the lower alkyl group substituted with an aryl group also include a lower alkyl group substituted with a halogen atom, and examples of the "lower alkyl group substituted with a halogen atom" include, for example, a lower alkyl group having 1 to 5 halogen atoms (for example, fluorine atom, chlorine atom, bromine atom, or iodine atom and the like), and specific examples include, chloromethyl, difluoromethyl, trichloromethyl, trifluoromethyl, 2-bromoethyl, 2,2,2-trifluoroethyl, 3,3,3-trifluoropropyl and the like. Examples of the substituent of the "lower alkyl group substituted with an aryl group" include an alkyl group substituted with carboxyl group, and particularly preferred examples of the substituent of the "alkyl group substituted with carboxyl group" include a lower alkyl group substituted with one or two (preferably one) carboxyl groups, and specific examples include carboxymethyl, 2-carboxyethyl, 3-carboxypropyl and the like. Preferred examples of the substituent of the "lower alkyl group substituted with an aryl group" also include an aryl group. The "aryl" is the same as that described above. Examples of the substituent of the "lower alkyl group substituted with an aryl group" also include a heterocyclic group. Examples of the "heterocyclic group" include an aromatic heterocyclic group, a saturated or unsaturated non-aromatic heterocyclic group (aliphatic heterocyclic group) and the like, as described above. As the "aromatic heterocyclic group", for example, a 5- or 6- membered heterocyclic group such as furyl, thienyl, pyrrolyl, oxazolyl, isoxazolyl, thiazolyl, isothiazolyl, imidazolyl, and triazolyl is preferred, and as the "saturated or unsaturated non-aromatic heterocyclic group, a 5- or 6- membered heterocyclic group such as pyrrolidinyl, morpholinyl, piperidinyl, and piperazinyl is preferred. As the substituent of the "lower alkyl group substituted with an aryl group", hydroxyl group is also preferred.

As the substituent of the "lower alkyl group substituted with an aryl group", an alkoxy group which may be halogenated is also preferred, and particularly preferred examples include a lower alkoxy group (for example, a $C_{1-6}$ alkoxy group such as methoxy, ethoxy, propoxy, isopropoxy, n-butoxy, isobutoxy, sec-butoxy, and tert-butoxy) which

may have 1 to 5 halogen atoms (for example, fluorine atom, chlorine atom, bromine atom, iodine atom and the like), and examples include methoxy, difluoromethoxy, trifluoromethoxy, ethoxy, 2,2,2-trifluoroethoxy, n-propoxy, isopropoxy, n-butoxy, 4,4,4-trifluorobutoxy, isobutoxy, sec-butoxy, pentyloxy, hexyloxy and the like. As the substituent of the "lower alkyl group substituted with an aryl group", an alkylenedioxy group is also preferred, and examples include, for example, methylenedioxy, ethylenedioxy and the like. As the substituent of the "lower alkyl group substituted with an aryl group", an alkyl group substituted with an alkoxycarbonyl group is also preferred, and the number of the alkoxycarbonyl as the substituent is preferably 1 or 2. A lower alkyl group substituted with a lower alkoxycarbonyl group is particularly preferred, and specific examples include methoxycarbonylmethyl, 2-(methoxycarbonyl)ethyl, 3-(methoxycarbonyl)propyl, ethoxycarbonylmethyl, 2-(ethoxycarbonyl)ethyl, and 3-(ethoxycarbonyl)propyl. As the substituent of the "lower alkyl group substituted with an aryl group", nitro group is also preferred. As the substituent of the "lower alkyl group substituted with an aryl group", amino group is also preferred.

As the substituent of the "lower alkyl group substituted with an aryl group", an alkylamino group is also preferred, and examples of the "alkylamino group" include, for example, a mono(lower alkyl)amino group, and a di(lower alkyl) amino group. Examples of the mono(lower alkyl)amino group include, for example, a mono($C_{1-6}$ alkyl)amino group such as methylamino, ethylamino, and propylamino and the like, and examples of the di(lower alkyl)amino group include, for example, a di($C_{1-6}$ alkyl)amino group such as dimethylamino, and diethylamino and the like. As the substituent of the "lower alkyl group substituted with an aryl group", an alkyl group substituted with hydroxyl group is also preferred, and a lower alkyl group substituted with hydroxyl group is particularly preferred. Specific examples include hydroxymethyl, 2-hydroxyethyl, 3-hydroxypropyl, 2-hydroxypropyl, 4-hydroxybutyl and the like. As the substituent of the "lower alkyl group substituted with an aryl group", an alkylamino group substituted with hydroxyl group is also preferred. The "alkylamino group substituted with hydroxyl group" means an amino group substituted with one or two alkyl groups substituted with hydroxyl group. An amino group substituted with one or two lower alkyl groups substituted with hydroxyl group is particularly preferred, and specific examples include 2-(2-hydroxyethyl)amino, 3-(3-hydroxypropyl)amino, 2-(3-hydroxypropyl)amino, 4-(4-hydroxybutyl)amino, di(2-hydroxyethyl)amino, bis(3-hydroxypropyl)amino and the like. As the substituent of the "lower alkyl group substituted with an aryl group", a (hydroxy-substituted alkyl)(alkyl)amino group is also preferred. The "(hydroxy-substituted alkyl)(alkyl)amino group" means amino group substituted with one alkyl group substituted with hydroxyl group and one alkyl group, and an amino group substituted with one lower alkyl group substituted with hydroxyl group and one lower alkyl group is particularly preferred. Specific examples include (2-hydroxyethyl) (methyl)amino, (3-hydroxypropyl)(methyl)amino, (2-hydroxypropyl)(methyl)amino, (4-hydroxybutyl)(methyl)amino, (2-hydroxyethyl)(ethyl)amino, (3-hydroxypropyl)(ethyl)amino, (2-hydroxypropyl)(ethyl)amino, (4-hydroxybutyl)(ethyl)amino and the like.

As the substituent of the "lower alkyl group substituted with an aryl group", an alkylcarbonylamino group is also preferred, and a lower alkylcarbonylamino group is particularly referred. Specific examples include a $C_{1-6}$ alkylcarbonylamino group such as acetylamino, propionylamino, and butyrylamino. As the substituent of the "lower alkyl group substituted with an aryl group", an alkylamino group substituted with an alkylcarbonyl group is also preferred. The "alkylamino group substituted with an alkylcarbonyl group" means an amino group substituted with one alkylcarbonyl group and an alkyl group, and an amino group substituted with one lower alkylcarbonyl group and one lower alkyl group is particularly preferred. Specific examples include a $C_{2-8}$ alkylcarbonylamino group such as acetyl(methyl)amino, propionyl(methyl)amino, butyryl(methyl)amino, acetyl(ethyl)amino, propionyl(ethyl)amino, and butyryl(ethyl)amino. As the substituent of the "lower alkyl group substituted with an aryl group", an alkylsulfonylamino group is also preferred, and a lower alkylsulfonylamino group is particularly preferred. Specific examples include methylsulfonylamino, ethylsulfonylamino, propylsulfonylamino, isopropylsulfonylamino and the like. As the substituent of the "lower alkyl group substituted with an aryl group", an arylsulfonylamino group is also preferred. The aryl in the "arylsulfonylamino" is the same as that described above, and as specific examples of the arylsulfonylamino, phenylsulfonylamino, 1-naphthylsulfonylamino, 2-naphthylsulfonylamino and the like are preferred, and phenylsulfonylamino is particularly preferred.

As the substituent of the "lower alkyl group substituted with an aryl group", an alkyl-NH-C(=O)-NH group is also preferred, and a lower alkyl-NH-C(=O)-NH- group is particularly preferred. Specific examples include methyl-NH-C(=O)-NH-, ethyl-NH-C(=O)-NH-, propyl-NH-C(=O)-NH-, isopropyl-NH-C(=O)-NH-, butyl-NH-C(=O)-NH-, isobutyl-NH-C(=O)-NH-, tert-butyl-NH-C(=O)-NH- and the like. As the substituent of the "lower alkyl group substituted with an aryl group", a substituted alkyl-NH-C(=O)-NH- group is also preferred, and a substituted lower alkyl-NH-C(=O)-NH- group is particularly preferred, and examples include, for example, a hydroxyl group-substituted lower alkyl-NH-C(=O)-NH- group, an amino group-substituted lower alkyl-NH-C(=O)-NH- group, an alkylamino group-substituted lower alkyl-NH-C(=O)-NH- group and the like. As the amino group-substituted lower alkyl-NH-C(=O)-NH- group, a (lower alkyl substituted with one or two hydroxyl groups (preferably one hydroxyl group))-NH-C(=O)-NH- group is preferred, and such a group of which end is substituted with hydroxyl group is preferred. Specific examples include 2-hydroxyethyl-NH-C(=O)-NH-, 3-hydroxypropyl-NH-C(=O)-NH-, 4-hydroxybutyl-NH-C(=O)-NH- and the like. As the amino group-substituted lower alkyl-NH-C(=O)-NH- group, a (lower alkyl substituted with one or two amino groups (preferably one amino group))-NH-C (=O)-NH- group is preferred, and such a group of which end is substituted with amino group is preferred. Specific

examples include 2-aminoethyl-NH-C(=O)-NH-, 3-aminopropyl-NH-C(=O)-NH-, 4-aminobutyl-NH-C(=O)-NH- and the like. As the alkylamino group-substituted lower alkyl-NH-C(=O)-NH- group, a (lower alkyl substituted with one or two (preferably one) monoalkylamino groups or dialkylamino groups)-NH-C(=O)-NH- group is preferred, and such a group of which end is substituted with an alkylamino group is preferred. Specific examples include 2-methylaminoethyl-NH-C(=O)-NH-, 3-methylaminopropyl-NH-C(=O)-NH-, 4-methylaminobutyl-NH-C(=O)-NH-, 2-(dimethylamino)ethyl-NH-C(=O)-NH-, 3-(dimethylamino)propyl-NH-C(=O)-NH-, 4-(dimethylamino)butyl-NH-C(=O)-NH- and the like.

As $A^{61}$, an alkyl group substituted with an aromatic heterocyclic group is also preferred. Further, as the "alkyl group substituted with an aromatic heterocyclic group", a lower alkyl group substituted with an aromatic heterocyclic group is preferred. The aromatic heterocyclic group referred to herein is the same as that described above, and specific examples of the "alkyl group substituted with an aromatic heterocyclic group" include 2-furylmethyl, 3-furylmethyl, 2-thienylmethyl, 3-thienylmethyl, 2-pyrrolylmethyl, 3-pyrrolylmethyl and the like.

As $A^{61}$, a lower alkyl group substituted with carboxyl group is also preferred. In this group, the lower alkyl group may be substituted with one or more carboxyl groups, and a lower alkyl group substituted with one of carboxyl group is generally preferred. Preferred examples include carboxymethyl, 2-carboxyethyl, 3-carboxypropyl, and 4-carboxybutyl. In particular, carboxymethyl and 2-carboxyethyl are preferred. Preferred examples also include either one of or a combination of any two of the aforementioned groups.

As $A^{61}$, a lower alkyl group substituted with cyano group is also preferred. In this group, the lower alkyl group may be substituted with one or more cyano groups. A lower alkyl group substituted with one of cyano group is generally preferred, and preferred examples include cyanomethyl, 2-cyanoethyl, 3-cyanopropyl, and 4-cyanobutyl.

As $A^{61}$, a lower alkyl group substituted with hydroxyl group is also preferred. In this group, the lower alkyl group may be substituted with one or more hydroxyl groups. A lower alkyl group substituted with one of hydroxyl group is generally preferred, and preferred examples include 2-hydroxyethyl, 3-hydroxypropyl, and 4-hydroxybutyl. In particular, 2-hydroxyethyl, and 3-hydroxypropyl are preferred. Preferred examples also include either one of or a combination of any two of these groups. A lower alkyl group substituted with two of hydroxyl groups is also preferred, and examples include 2,3-dihydroxypropyl, 3,4-dihydroxybutyl, 2-(1,3-dihydroxy)propyl, 2-(3,4-dihydroxy)butyl, 2,3-dihydroxybutyl, 2-(1,5-dihydroxy)pentyl, 3-(1,5-dihydroxy)pentyl and the like.

As $A^{61}$, a lower alkyl group substituted with a lower alkoxyl group is also preferred. The lower alkoxyl group include linear or branched alkoxyl groups having 1 to 4 carbon atoms, and specific examples include, for example, methoxy group, ethoxy group, propoxy group, isopropoxy group and the like. Methoxy group and ethoxy group are preferred. The lower alkyl group may be substituted with one or more lower alkoxyl groups. A lower alkyl group substituted with one of lower alkoxyl group is generally preferred, and preferred examples include 2-methoxyethyl, 3-methoxypropyl, 2-ethoxyethyl, 3-ethoxypropyl, and 4-methoxybutyl. In particular, 2-methoxyethyl and 3-methoxypropyl are preferred. Preferred examples also include either one of or a combination of any two of these groups.

As $A^{61}$, a lower alkyl group substituted with a substituted alkoxy group is also preferred. Examples of the "substituted an alkoxy group" herein referred to include an alkoxy group substituted with hydroxyl group, an alkoxy group, amino group, an alkylamino group, an acylamino group, mercapto group, alkylthio group, or an acylthio group. One or two of these groups may independently substitute on the alkoxy group, and an alkoxy group substituted with one of these groups is generally preferred. Preferred examples of the "lower alkyl group substituted with a substituted alkoxy group" include a lower alkyl group substituted with an alkoxy group substituted with hydroxyl group, and specific examples include 2-(2-hydroxyethoxy)ethyl, 2-(3-hydroxypropoxy)ethyl, 3-(2-hydroxyethoxy)propyl, 3-(3-hydroxypropoxy)propyl and the like. Preferred examples of the "lower alkyl group substituted with a substituted alkoxy group" further include a lower alkyl group substituted with an alkoxy group substituted with an alkoxy group, and preferred examples of both alkoxy groups include a lower alkyloxy. Specific examples of the lower alkyl group substituted with an alkoxy group substituted with an alkoxy group include 2-(2-methoxyethoxy)ethyl, 2-(3-methoxypropoxy)ethyl, 3-(2-methoxyethoxy)propyl, 3-(3-methoxypropoxy)propyl, 2-(2-ethoxyethoxy)ethyl, 2-(3-ethoxypropoxy)ethyl, 3-(2-ethoxyethoxy)propyl, 3-(3-ethoxypropoxy)propyl and the like.

Preferred examples of the "lower alkyl group substituted with a substituted alkoxy group" include a lower alkyl group substituted with an alkoxy group substituted with amino group, and specific examples include 2-(2-aminoethoxy)ethyl, 2-(3-aminopropoxy)ethyl, 3-(2-aminoethoxy)propyl, 3-(3-aminopropoxy)propyl and the like. Preferred examples of the "lower alkyl group substituted with a substituted alkoxy group" further include a lower alkyl group substituted with an alkoxy group substituted with an alkylamino group, and examples of the "alkylamino group" in this group include, for example, a mono(lower alkyl)amino group, and a di(lower alkyl)amino group. Examples of the mono(lower alkyl)amino group include, for example, a mono($C_{1-6}$ alkyl)amino group such as methylamino, ethylamino, and propylamino and the like. Examples of the di(lower alkyl)amino group include, for example, a di($C_{1-6}$ alkyl)amino group such as dimethylamino, and diethylamino and the like. Thus, specific examples of the lower alkyl group substituted with an alkoxy group substituted with an alkylamino group include a lower alkyl group substituted with an alkoxy group substituted with a mono(lower alkyl)amino group such as 2-(2-(methylamino)ethoxy)ethyl, 2-(3-(methylamino)propoxy)ethyl, 3-(2-(methylamino)ethoxy)propyl, 3-(3-(methylamino)propoxy)propyl, 2-(2-(ethylamino)ethoxy)ethyl, 2-(3-(ethylamino)propoxy)ethyl,

3-(2-(ethylamino)ethoxy)propyl, and 3-(3-(ethylamino)propoxy)propyl, and a lower alkyl group substituted with an alkoxy group substituted with a di(lower alkyl)amino group such as 2-(2-(dimethylamino)ethoxy)ethyl, 2-(3-(dimethylamino) propoxy)ethyl, 3-(2-(dimethylamino)ethoxy)propyl, 3-(3-(dimethylamino)propoxy)propyl, 2-(2-(diethylamino)ethoxy) ethyl, 2-(3-(diethylamino)propoxy)ethyl, 3-(2-(diethylamino)ethoxy)propyl, and 3-(3-(diethylamino)propoxy)propyl.

Preferred examples of the "lower alkyl group substituted with a substituted alkoxy group" include a lower alkyl group substituted with an alkoxy group substituted with an acylamino group, and examples of the "acylamino group" in this group include, for example, an alkylcarbonylamino group, and an arylcarbonylamino group ("alkyl" and "aryl" herein referred to are the same as those described above), and particularly preferred examples include an alkylcarbonylamino group. Specific examples of the lower alkyl group substituted with an alkoxy group substituted with an acylamino group include 2-(2-(acetylamino)ethoxy)ethyl, 2-(3-(acetylamino)propoxy)ethyl, 3-(2-(acetylamino)ethoxy)propyl, 3-(3-(acetylamino)propoxy)propyl, 2-(2-(propionylamino)ethoxy)ethyl, 2-(3-(propionylamino)propoxy)ethyl, 3-(2-(propionylamino)ethoxy)propyl, 3-(3-(propionylamino)propoxy)propyl and the like. Preferred examples of the "lower alkyl group substituted with a substituted alkoxy group" further include a lower alkyl group substituted with an alkoxy group substituted with mercapto group, and specific examples include 2-(2-mercaptoethoxy)ethyl, 2-(3-mercaptopropoxy)ethyl, 3-(2-mercaptoethoxy)propyl, 3-(3-mercaptopropoxy)propyl and the like. Preferred examples the "lower alkyl group substituted with a substituted alkoxy group" further include a lower alkyl group substituted with an alkoxy group substituted with an alkylthio group, and preferred examples of the alkylthio group in this group include a lower alkylthio. Specific examples of the lower alkyl group substituted with an alkoxy group substituted with an alkylthio group include 2-(2-(methylthio) ethoxy)ethyl, 2-(3-(methylthio)propoxy)ethyl, 3-(2-(methylthio)ethoxy)propyl, 3-(3-(methylthio)propoxy)propyl, 2-(2-(ethylthio)ethoxy)ethyl, 2-(3-(ethylthio)propoxy)ethyl, 3-(2-(ethylthio)ethoxy)propyl, 3-(3-(ethylthio)propoxy)propyl and the like.

Preferred examples of the "lower alkyl group substituted with a substituted alkoxy group" include a lower alkyl group substituted with an alkoxy group substituted with an acylthio group. Examples of the "acylthio group" in this group include, for example, an alkylcarbonylthio group, and an arylcarbonylthio group ("alkyl" and "aryl" herein referred to are the same as those described above), and particularly preferred examples include an alkylcarbonylthio group. Specific examples of the lower alkyl group substituted with an alkoxy group substituted with an acylthio group include 2-(2-(acetylthio)ethoxy)ethyl, 2-(3-(acetylthio)propoxy)ethyl, 3-(2-(acetylthio)ethoxy)propyl, 3-(3-(acetylthio)propoxy)propyl, 2-(2-(propionylthio)ethoxy)ethyl, 2-(3-(propionylthio)propoxy)ethyl, 3-(2-(propionylthio)ethoxy)propyl, 3-(3-(propionylthio)propoxy)propyl and the like.

As $A^{61}$, a lower alkyl group substituted with amino group is also preferred. The lower alkyl group may be substituted with one or more amino groups, and a lower alkyl group substituted with one amino group is generally preferred. Preferred examples include 2-aminoethyl, 3-aminopropyl, 2-aminopropyl, and 4-aminobutyl, and 2-aminoethyl, and 3-aminopropyl are particularly preferred. Further, the "lower alkyl group substituted with amino group" may be further substituted with hydroxyl group. The lower alkyl group is usually substituted with one hydroxyl group. Specific examples include 2-amino-3-hydroxypropyl, 3-amino-2-hydroxypropyl, 3-amino-4-hydroxybutyl and the like.

As $A^{61}$, a lower alkyl group substituted with a lower alkylamino group is also preferred. The lower alkylamino group include a monoalkylamino group and dialkylamino group substituted with one or two lower alkyl groups. As for the dialkylamino group, the alkyl groups may be the same or different. The lower alkyl group may be substituted with one or more of the amino groups, and a lower alkyl group substituted with one alkylamino group is generally preferred. Preferred examples include 2-(methylamino)ethyl, 2-(dimethylamino)ethyl, 3-(methylamino)propyl, 3-(dimethylamino) propyl, 4-(methylamino)butyl, and 4-(dimethylamino)butyl, and 2-(methylamino)ethyl, 2-(dimethylamino)ethyl, 3-(methylamino)propyl, and 3-(dimethylamino)propyl are particularly preferred. Further, the "lower alkyl group substituted with a lower alkylamino group" may be further substituted with hydroxyl group. The lower alkyl group is usually substituted with one hydroxyl group, and specific examples include 2-(methylamino)-3-hydroxypropyl, 3-(methylamino)-2-hydroxypropyl, 3-(methylamino)-4-hydroxybutyl, 2-(dimethylamino)-3-hydroxypropyl, 3-(dimethylamino)-2-hydroxypropyl, 3-(dimethylamino)-4-hydroxybutyl and the like.

As $A^{61}$, a lower alkyl group substituted with aminocarbonyl group is also preferred. The lower alkyl group may be substituted with one or more aminocarbonyl groups. A lower alkyl group substituted with one of aminocarbonyl group is generally preferred, and preferred examples include, for example, aminocarbonylmethyl and aminocarbonylethyl. Preferred examples also include either one of or a combination of any two of these groups.

As $A^{61}$, an alkyl group substituted with an alkylaminocarbonyloxy group is also preferred. The alkylaminocarboxyoxy group herein referred to means an alkyl-NH-C(=O)-O-, and a lower alkyl-NH-C(=O)-O- is preferred. Thus, preferred specific examples of the alkyl group substituted with an alkylaminocarbonyloxy group include 2-(methylaminocarbonyloxy)ethyl, 2-(ethylaminocarbonyloxy)ethyl, 2-(propylaminocarbonyloxy)ethyl, 2-(isopropylaminocarbonyloxy)ethyl, 3-(methylaminocarbonyloxy)propyl, 3-(ethylaminocarbonyloxy)propyl, 3-(propylaminocarbonyloxy)propyl, 3-(isopropylaminocarbonyloxy)propyl, 4-(methylaminocarbonyloxy)butyl, 4-(ethylaminocarbonyloxy)butyl, 4-(propylaminocarbonyloxy)butyl, 4-(isopropylaminocarbonyloxy)butyl and the like.

As $A^{61}$, an alkyl group substituted with a cycloalkylaminocarbonyloxy group is also preferred. The cycloalkylaminocarboxyoxy group herein referred to means a cycloalkyl-NH-C(=O)-O-, and a lower cycloalkyl-NH-C(=O)-O- is preferred. Preferred specific examples of the alkyl group substituted with a cycloalkylaminocarbonyloxy group include 2-(cyclopropylaminocarbonyloxy)ethyl, 2-(cyclobutylaminocarbonyloxy)ethyl, 2-(cyclopentylaminocarbonyloxy)ethyl, 2-(cyclohexylaminocarbonyloxy)ethyl, 3-(cyclopropylaminocarbonyloxy)propyl, 3-(cyclobutylaminocarbonyloxy)propyl, 3-(cyclopentylaminocarbonyloxy)propyl, 3-(cyclohexylaminocarbonyloxy)propyl, 4-(cyclopropylaminocarbonyloxy) butyl, 4-(cyclobutylaminocarbonyloxy)butyl, 4-(cyclopentylaminocarbonyloxy)butyl, 4-(cyclohexylaminocarbonyloxy) butyl and the like.

As $A^{61}$, an alkyl group substituted with an arylaminocarbonyloxy group is also preferred. The arylaminocarboxyoxy group herein referred to means an aryl-NH-C(=O)-O- (aryl is the same as that described above), and preferred specific examples of the alkyl group substituted with an arylaminocarbonyloxy group include 2-(phenylaminocarbonyloxy) ethyl, 3-(phenylaminocarbonyloxy)propyl, 4-(phenylaminocarbonyloxy)butyl, 5-(phenylaminocarbonyloxy)pentyl and the like.

Further, as $A^{61}$, an alkyl group substituted with mercapto group is also preferred, and a lower alkyl group substituted with mercapto group is particularly preferred. In this group, the lower alkyl group may be substituted with one or more mercapto groups, and an alkyl group substituted with one mercapto group is generally preferred For example, 2-mercaptoethyl, 3-mercaptopropyl, 4-mercaptobutyl, 5-mercaptopentyl and the like are preferred. Preferred examples also include either one of or a combination of any two of the aforementioned groups.

As $A^{61}$, an alkyl group substituted with an alkylthio group is also preferred. A lower alkyl group substituted with an alkylthio group is preferred, and a lower alkyl group substituted with a lower alkylthio group is particularly preferred. In this group, the lower alkyl group may be substituted with one or more alkylthio groups, and an alkyl group substituted with one alkylthio group is generally preferred. For example, 2-(methylthio)ethyl, 2-(ethylthio)ethyl, 3-(methylthio)propyl, 3-(ethylthio)propyl, 4-(methylthio)butyl, 4-(ethylthio)butyl, 5-(methylthio)pentyl, 5-(ethylthio)pentyl and the like are preferred. Preferred examples also include either one of or a combination of any two of the aforementioned groups.

As $A^{61}$, an alkyl group substituted with an acylthio group is also preferred. A lower alkyl group substituted with an acylthio group is preferred, and a lower alkyl group substituted with a $C_{2-6}$ alkanoylthio group is particularly preferred. In this group, the lower alkyl group may be substituted with one or more acylthio groups, and an alkyl group substituted with one acylthio group is generally preferred. For example, 2-(acetylthio)ethyl, 2-(propionylthio)ethyl, 3-(acetylthio) propyl, 3-(propionylthio)propyl, 4-(acetylthio)butyl, 4-(propionylthio)butyl, 5-(acetylthio)pentyl, 5-(propionylthio)pentyl and the like are preferred. Preferred examples also include either one of or a combination of any two of the aforementioned groups.

As $A^{61}$, a cycloalkyl group is also preferred. The cycloalkyl is the same as that described above, and a lower cycloalkyl group is preferred.

As $A^{61}$, a substituted cycloalkyl group is also preferred. A cycloalkyl group substituted with hydroxyl group, an alkoxy group, amino group, an alkylamino group, an acylamino group, mercapto group, alkylthio group, an acylthio group, an alkylsulfonylamino group, or the like is particularly preferred. The cycloalkyl group may be independently substituted with one or two of these groups, and a cycloalkyl, group substituted with one of these group is generally preferred. Preferred examples include a cycloalkyl group substituted with hydroxyl group, and specific examples include 3-hydroxycyclobutyl, 3-hydroxycyclohexyl, 4-hydroxycyclohexyl and the like. Further, preferred examples include a cycloalkyl group substituted with an alkoxy group, and preferred examples of the alkoxy group in this group include a lower alkyloxy. Specific examples of the cycloalkyl group substituted with an alkoxy group include 3-methoxycyclobutyl, 3-methoxycyclohexyl, 4-methoxycyclohexyl, 3-ethoxycyclobutyl, 3-ethoxycyclohexyl, 4-ethoxycyclohexyl and the like. Preferred examples further include a cycloalkyl group substituted with amino group, and specific examples include 3-aminocyclobutyl, 3-aminocyclohexyl, 4-aminocyclohexyl and the like. Preferred examples further include a cycloalkyl group substituted with an alkylamino group, and examples of the "alkylamino group" in this group include a mono(lower alkyl)amino group, and a di(lower alkyl)amino group. Examples of the mono(lower alkyl)amino group include, for example, a mono($C_{1-6}$) alkylamino group such as methylamino, ethylamino, and propylamino and the like, and examples of the di(lower alkyl) amino group include a di($C_{1-6}$ alkyl)amino group such as dimethylamino, and diethylamino and the like. Thus, specific examples of the cycloalkyl group substituted with an alkylamino group include a cycloalkyl group substituted with a mono (lower alkyl)amino group such as 3-(methylamino)cyclobutyl, 3-(methylamino)cyclohexyl, 4-(methylamino)cyclohexyl, 3-(ethylamino)cyclobutyl, 3-(ethylamino)cyclohexyl, and 4-(ethylamino)cyclohexyl, and a cycloalkyl group substituted with a di(lower alkyl)amino group such as 3-(dimethylamino)cyclobutyl, 3-(dimethylamino)cyclohexyl, 4-(dimethylamino) cyclohexyl, 3-(diethylamino)cyclobutyl, 3-(diethylamino)cyclohexyl, and 4-(diethylamino)cyclohexyl.

Preferred examples further include a cycloalkyl group substituted with an acylamino group, and examples of the "acylamino group" in this group include an alkylcarbonylamino group, and an arylcarbonylamino group ("alkyl" and "aryl" herein referred to are the same as those described above). An alkylcarbonylamino group is particularly preferred. Specific examples of the cycloalkyl group substituted with an acylamino group include 3-(acetylamino)cyclobutyl, 3-(acetylamino)cyclohexyl, 4-(acetylamino)cyclohexyl, 3-(propionylamino)cyclobutyl, 3-(propionylamino)cyclohexyl, 4-(propionylamino)cyclohexyl, 3-(butyrylamino)cyclobutyl, 3-(butyrylamino)cyclohexyl, 4-(butyrylamino)cyclohexyl,

3-(isobutyrylamino)cyclobutyl, 3-(isobutyrylamino)cyclohexyl, 4-(isobutyrylamino)cyclohexyl and the like, and particularly preferred examples include 3-(acetylamino)cyclobutyl, 3-(acetylamino)cyclohexyl, and 4-(acetylamino)cyclohexyl. Preferred examples further include a cycloalkyl group substituted with mercapto group, and specific examples include 3-mercaptocyclobutyl, 3-mercaptocyclohexyl, 4-mercaptocyclohexyl and the like. Preferred examples further include a cycloalkyl group substituted with an alkylthio group, and preferred examples of the alkylthio group in this group include a lower alkylthio. Specific examples of the cycloalkyl group substituted with an alkylthio group include 3-(methylthio) cyclobutyl, 3-(methylthio)cyclohexyl, 4-(methylthio)cyclohexyl, 3-(ethylthio)cyclobutyl, 3-(ethylthio)cyclohexyl, 4-(ethylthio)cyclohexyl and the like.

Preferred examples further include a cycloalkyl group substituted with an acylthio group, and examples of the "acylthio group" in this group include, for example, an alkylcarbonylthio group, and an arylcarbonylthio group ("alkyl" and "aryl" herein referred to are the same as those described above), and an alkylcarbonylthio group is particularly preferred. Specific examples of the cycloalkyl group substituted with an acylthio group include 3-(acetylthio)cyclobutyl, 3-(acetylthio)cyclohexyl, 4-(acetylthio)cyclohexyl, 3-(propionylthio)cyclobutyl, 3-(propionylthio)cyclohexyl, 4-(propionylthio)cyclohexyl, 3-(butyrylthio)cyclobutyl, 3-(butyrylthio)cyclohexyl, 4-(butyrylthio)cyclohexyl, 3-(isobutyrylthio)cyclobutyl, 3-(isobutyrylthio)cyclohexyl, 4-(isobutyrylthio)cyclohexyl and the like, and particularly preferred examples include 3-(acetylthio)cyclobutyl, 3-(acetylthio)cyclohexyl, and 4-(acetylthio)cyclohexyl. Preferred examples further include a cycloalkyl group substituted with an alkylsulfonylamino group, and examples of the alkylsulfonylamino group in this group include a lower alkylsulfonyl. Specific examples of the cycloalkyl group substituted with an alkylsulfonylamino group include 3-(methylsulfonylamino)cyclobutyl, 3-(methylsulfonylamino)cyclohexyl, 4-(methylsulfonylamino)cyclohexyl, 3-(ethylsulfonylamino)cyclobutyl, 3-(ethylsulfonylamino)cyclohexyl, 4-(ethylsulfonylamino)cyclohexyl and the like.

Further, preferred examples of $A^{61}$ include carbamimidoyl group.

As $A^{61}$, an alkylcarbonyl group is also preferred, a lower alkylcarbonyl group is particularly preferred, and specific examples include acetyl, propionyl, butyryl, and isobutyryl.

As $A^{61}$, an arylcarbonyl group is also preferred. The "aryl" in this group is the same as that described above. Examples of the arylcarbonyl group include, for example, phenylcarbonyl, 1-naphthylcarbonyl, 2-naphthylcarbonyl and the like.

As $A^{61}$, a non-aromatic heterocyclic group is also preferred. The non-aromatic heterocyclic group is the same as that described above. A 4- to 8-membered non-aromatic heterocyclic group is preferred, and particularly preferred examples include a 4- to 6-membered non-aromatic heterocyclic group. The non-aromatic heterocyclic group comprises one or more hetero atoms, and a non-aromatic heterocyclic group comprising one or two hetero atoms is generally preferred. Specific examples include 3-azetidinyl, 3-oxetanyl, 3-thietanyl, 3-pyrrolidinyl, 3-tetrahydrofuryl, 3-tetrahydrothienyl, 3-piperidinyl, 3-tetrahydropyranyl, 3-tetrahydrothiopyranyl, 4-piperidinyl, 4-tetrahydropyranyl, 4-tetrahydrothiopyranyl, 6-homopiperazinyl and the like, and 3-azetidinyl, 3-oxetanyl, 3-thietanyl, 3-pyrrolidinyl, 3-tetrahydrofuryl, 3-tetrahydrothienyl, 3-piperidinyl, 3-tetrahydropyranyl, 3-tetrahydrothiopyranyl, 4-piperidinyl, 4-tetrahydropyranyl, and 4-tetrahydrothiopyranyl are particularly preferred.

As $A^{61}$, a substituted non-aromatic heterocyclic group comprising one or two nitrogen atoms is also preferred. As the substituted non-aromatic heterocyclic group comprising one or two nitrogen atoms, a 4- to 8-membered non-aromatic heterocyclic group is preferred, and particularly preferred examples include a 4- to 6-membered non-aromatic heterocyclic group. When the non-aromatic heterocyclic group is a non-aromatic heterocyclic group comprising one nitrogen atom, it is preferred that the nitrogen atom constituting the ring is substituted. Examples of the substituent include lower alkyl, lower alkylcarbonyl, arylcarbonyl, lower alkylsulfonyl, arylsulfonyl and the like. Methyl, ethyl, propyl, acetyl, benzoyl, methanesulfonyl, benzenesulfonyl and the like are preferred, and particularly preferred examples include methyl, acetyl, and methanesulfonyl. Specific examples of the non-aromatic heterocyclic group comprising one nitrogen atom and substituted with these group include 3-azetidin-1-yl, 3-pyrrolidin-1-yl, 3-piperidin-1-yl, 4-piperidin-1-yl and the like. Further, when the non-aromatic heterocyclic group is a substituted non-aromatic heterocyclic group comprising two nitrogen atoms, the alkylene moiety of the non-aromatic heterocyclic group may be substituted with one or more oxo groups, and it is generally preferred that the alkylene moiety is substituted with one oxo group. Preferred examples include, for example, tetrahydropyrimidin-2(1H)-on-5-yl, 1,3-dimethyl-tetrahydropyrimidin-2(1H)-on-5-yl and the like.

As $A^{61}$, an alkyl group of which end is substituted with $N(A^7)(-X^3-A^{71})$ is also preferred. $-X^3-$ represents carbonyl group, $-SO_2-$, -C(=O)-O-, $-C(=O)-N(A^8)-$, or $-C(=S)-N(A^8)-$, and $A^7$ represents hydrogen atom, an alkyl group, an alkyl group substituted with hydroxyl group, an alkyl group substituted with amino group, an alkyl group substituted with an alkylamino group, an alkyl group substituted with aminocarbonyl group, an alkyl group substituted with an acylamino group, or an alkyl group substituted with cyano group, and $A^{71}$ and $A^8$ independently represent hydrogen atom, an alkyl group, an alkyl group substituted with an aryl group, an alkyl group substituted with a heterocyclic group, an alkyl group substituted with hydroxyl group, an alkyl group substituted with amino group, an alkyl group substituted with an alkylamino group, an alkyl group substituted with an alkoxy group, an alkyl group substituted with an acylamino group, a cycloalkyl group, an aryl group, or an aromatic heterocyclic group, or $A^7$ and $A^{71}$ may together become an alkylene group, or an alkylene group substituted with an alkyl group to form a ring, or $A^{71}$ and $A^8$ may together become an alkylene group,

-alkylene-O-alkylene-, -alkylene-NH-alkylene-, or -alkylene-N(alkyl)-alkylene- to form a ring.

As $A^7$, hydrogen atom is preferred.

As $A^7$, an alkyl group, an alkyl group substituted with hydroxyl group, an alkyl group substituted with amino group, an alkyl group substituted with an alkylamino group, an alkyl group substituted with an alkylcarbonyl group, or an alkyl group substituted with cyano group is also preferred, and more preferred examples of the alkyl group for these alkyl groups include a lower alkyl group.

Preferred examples of $A^7$ include a lower alkyl group, and particularly preferred examples include methyl, and ethyl.

Preferred examples of $A^7$ also include a lower alkyl group substituted with hydroxyl group. In this group, the lower alkyl group may be substituted with one or more hydroxyl groups, and a lower alkyl group substituted with one of hydroxyl group is generally preferred. Specific examples include 2-hydroxyethyl, 3-hydroxypropyl, 2-hydroxypropyl, 4-hydroxybutyl and the like, and particularly preferred examples include 2-hydroxyethyl, and 3-hydroxyethyl.

Preferred examples of $A^7$ also include a lower alkyl group substituted with amino group. In this group, the lower alkyl group may be substituted with one or more amino groups, and a lower alkyl group substituted with one of amino group is generally preferred. Specific examples include 2-aminoethyl, 3-aminopropyl, 2-aminopropyl, 4-aminobutyl and the like, and particularly preferred examples include 2-aminoethyl, and 3-aminoethyl.

As $A^7$, a lower alkyl group substituted with a lower alkylamino group is also preferred. The "lower alkylamino group" include a monoalkylamino group and dialkylamino group substituted with one or two lower alkyl groups. As for the dialkylamino group, the alkyl groups may be the same or different. The lower alkyl group may be substituted with one or more of the amino groups, and a lower alkyl group substituted with one alkylamino group is generally preferred. Preferred examples include 2-(methylamino)ethyl, 2-(dimethylamino)ethyl, 3-(methylamino)propyl, 3-(dimethylamino)propyl, 4-(methylamino)butyl, and 4-(dimethylamino)butyl, particularly preferred examples include 2-(methylamino)ethyl, and 3-(methylamino)propyl.

Preferred examples of $A^7$ also include a lower alkyl group substituted with aminocarbonyl group. In this group, the lower alkyl group may be substituted with one or more aminocarbonyl groups, and a lower alkyl group substituted with one of aminocarbonyl group is generally preferred. Preferred examples include, for example, aminocarbonylmethyl, 2-aminocarbonylethyl, and 3-aminocarbonylpropyl.

As $A^7$, a lower alkyl group substituted with an acylamino group is also preferred. In this group, the lower alkyl group may be substituted with one or more acylamino groups, and a lower alkyl group substituted with one acylamino group is generally preferred. Examples of the "acylamino group" in this group include, for example, an alkylcarbonylamino group, and an arylcarbonylamino group ("alkyl" and "aryl" herein referred to are the same as those described above), and particularly preferred examples include an alkylcarbonylamino group. Specific examples of the lower alkyl group substituted with an acylamino group include 2-(acetylamino)ethyl, 3-(acetylamino)propyl, 2-(propionylamino)ethyl, 3-(propionylamino)propyl and the like.

Preferred examples of $A^7$ also include a lower alkyl group substituted with cyano group. In this group, the lower alkyl group may be substituted with one or more cyano groups, and a lower alkyl group substituted with one of cyano group is generally preferred. Specific examples include cyanomethyl, 2-cyanoethyl, 1-cyanoethyl, 3-cyanopropyl, 2-cyanopropyl, 4-cyanobutyl and the like, and particularly preferred examples include cyanomethyl, and 2-cyanoethyl.

As $-X^3-$, carbonyl group is preferred.

As $-X^3-$, $-SO_2-$, -C(=O)-O-, $-C(=O)-N(A^8)-$, and $-C(=S)-N(A^8)-$ are also preferred, and $-SO_2-$, and $-C(=O)-N(A^8)-$ are particularly preferred.

As $A^{61}$, an alkyl group of which end is substituted with $N(A^7)(-X^3-A^{71})$ (wherein $-X^3-$ represents carbonyl group) is preferred.

Preferred examples of $A^{71}$ include hydrogen atom.

As $A^{71}$, an alkyl group, an alkyl group substituted with an aryl group, an alkyl group substituted with a heterocyclic group, an alkyl group substituted with hydroxyl group, an alkyl group substituted with amino group, an alkyl group substituted with an alkylamino group, an alkyl group substituted with an alkoxy group, and an alkyl group substituted with an acylamino group are preferred, and more preferred examples of the alkyl group for these alkyl groups include a lower alkyl group.

Further , as $A^{61}$, a lower alkyl group of which end is substituted with $N(A^7)(-X^3-A^{71})$ is also preferred. In this group, $-X^3-$, $A^7$ and $A^{71}$ are the same as those described above. As for the substitution with $N(A^7)(-X^3-A^{71})$, the lower alkyl group may be substituted with one or more of $N(A^7)(-X^3-A^{71})$, and the lower alkyl group substituted with one of $N(A^7)(-X^3-A^{71})$ is generally preferred.

Preferred examples of $A^{71}$ include a lower alkyl group, and specific examples include methyl, ethyl, propyl, isopropyl, n-butyl, isobutyl, tert-butyl and the like. Particularly preferred examples include methyl, ethyl and the like. When $-X^3-$ is carbonyl group, or $-SO_2-$ the lower alkyl group may be substituted with 1 to 5 halogen atoms (for example, fluorine atom, chlorine atom, bromine atom, iodine atom and the like). Preferred specific examples include chloromethyl, difluoromethyl, trichloromethyl, trifluoromethyl, 2,2,2-trifluoroethyl, 3,3,3-trifluoropropyl, 4,4,4-trifluorobutyl and the like, and more preferred examples include trifluoromethyl, and 2,2,2-trifluoroethyl.

Preferred examples of $A^{71}$ also include a lower alkyl group substituted with an aryl group (aryl is the same as

that described above), and specific examples include benzyl, 2-phenylethyl, 1-naphthylmethyl, 2-naphthylmethyl and the like. Preferred are benzyl, 2-phenylethyl and the like. The "aryl ring" moiety of these groups may be substituted with a group similar to $A^{61}$ mentioned above.

Preferred examples of $A^{71}$ also include a lower alkyl group substituted with a heterocyclic group (heterocyclic group is the same as that described above), and examples of the heterocyclic group in this group include "an aromatic heterocyclic group", and a "non-aromatic heterocyclic group". As for the "lower alkyl group substituted with a heterocyclic group", specific examples of the lower alkyl group substituted with an aromatic heterocyclic group include 2-furylmethyl, 3-furylmethyl, 2-thienylmethyl, 3-thienylmethyl, 2-pyrrolylmethyl, 3-pyrrolylmethyl and the like. Further, as for the "lower alkyl group substituted with a heterocyclic group", specific examples of the lower alkyl group substituted with a non-aromatic heterocyclic group include oxylanylmethyl, azetidinylmethyl, pyrrolidinylmethyl, tetrahydrofurylmethyl, thiolanylmethyl, piperidylmethyl, tetrahydropyranylmethyl, morpholinylmethyl, thiomorpholinylmethyl, piperazinylmethyl and the like.

Preferred examples of $A^{71}$ also include a lower alkyl group substituted with hydroxyl group. In this group, the lower alkyl group may be substituted with one or more hydroxyl groups, and a lower alkyl group substituted with one of hydroxyl group is generally preferred. Specific examples include 2-hydroxyethyl, 3-hydroxypropyl, 2-hydroxypropyl, 4-hydroxybutyl and the like, and particularly preferred examples include 2-hydroxyethyl, and 3-hydroxyethyl.

Preferred examples of $A^{71}$ include a lower alkyl group substituted with amino group. In this group, the lower alkyl group may be substituted with one or more amino groups, and a lower alkyl group substituted with one of amino group is generally preferred. Specific examples include, 2-aminoethyl, 3-aminopropyl, 2-aminopropyl, 4-aminobutyl and the like, and particularly preferred examples include 2-aminoethyl, and 3-aminoethyl.

As $A^{71}$, a lower alkyl group substituted with a lower alkylamino group is also preferred. The "lower alkylamino group" herein referred to include a monoalkylamino group and dialkylamino group, which are substituted with one or two lower alkyl groups. As for the dialkylamino group, the alkyl groups may be the same or different. The lower alkyl group may be substituted with one or more of the amino groups, and a lower alkyl group substituted with one alkylamino group is generally preferred. Preferred examples include 2-(methylamino)ethyl, 2-(dimethylamino)ethyl, 3-(methylamino)propyl, 3-(dimethylamino)propyl, 4-(methylamino)butyl, and 4-(dimethylamino)butyl, and particularly preferred examples include 2-(methylamino)ethyl, and 3-(methylamino)propyl.

As $A^{71}$, a lower alkyl group substituted with an alkoxy group is also preferred. The lower alkoxy group include linear or branched alkoxy having 1 to 4 carbon atoms. Specific examples include, for example, methoxy group, ethoxy group, propoxy group, isopropoxy group and the like, and methoxy group, and ethoxy group are preferred. The lower alkyl group may be substituted with one or more lower alkoxy groups, and a lower alkyl group substituted with one alkoxy group is generally preferred. Preferred examples include 2-methoxyethyl, 3-methoxypropyl, 2-ethoxyethyl, 3-ethoxypropyl, and 4-methoxybutyl, and 2-methoxyethyl, and 3-methoxypropyl are particularly preferred.

As $A^{71}$, a lower alkyl group substituted with an acylamino group is also preferred. In this group, the lower alkyl group may be substituted with one or more acylamino groups, and a lower alkyl group substituted with one acylamino group is generally preferred. Examples of the "acylamino group" in this group include an alkylcarbonylamino group, and an arylcarbonylamino group ("alkyl" and "aryl" herein referred to are the same as those described above).

As $A^{71}$, a lower alkyl group substituted with an alkylcarbonylamino group is also preferred. In this group, the lower alkyl group may be substituted with one or more alkylcarbonylamino groups, and a lower alkyl group substituted with one of alkylcarbonylamino group is generally preferred. Specific examples include acetylaminomethyl, 2-(acetylamino)ethyl, 3-(acetylamino)propyl, 4-(acetylamino)butyl, propionylaminomethyl, 2-(propionylamino)ethyl, 3-(propionylamino)propyl, 4-(propionylamino)butyl and the like.

As $A^{71}$, a lower alkyl group substituted with an arylcarbonylamino group is also preferred. In this group, the lower alkyl group may be substituted with one or more arylcarbonylamino groups, and a lower alkyl group substituted with one of arylcarbonylamino group is generally preferred. Specific examples include benzoylaminomethyl, 2-(benzoylamino)ethyl, 3-(benzoylamino)propyl, 4-(benzoylamino)butyl and the like.

As $A^{71}$, a cycloalkyl group is also preferred. The cycloalkyl is the same as that described above. A lower cycloalkyl group is preferred, and particularly preferred examples include cyclopropyl, cyclobutyl, cyclopentyl, cyclohexyl and the like. As $A^{71}$, an aryl group is also preferred. The aryl is the same as that described above, and preferred examples include phenyl, 1-naphthyl, 2-naphthyl and the like. As $A^{71}$, an aromatic heterocyclic group is also preferred. The aromatic heterocyclic group is the same as that described above, and specific examples include 2-furyl, 3-furyl, 2-thienyl, 3-thienyl, 2-pyrrolyl, 3-pyrrolyl and the like.

It is also preferred that $A^7$ and $A^{71}$ together become an alkylene group, or an alkylene group substituted with an alkyl group to form a ring together with N to which $A^7$ and $A^{71}$ bind and $-X^3-$. When $A^7$ and $A^{71}$ together become an alkylene group substituted with an alkyl group to form a ring, the alkyl group is preferably a lower alkyl group, and examples of the lower alkyl group include, methyl, and ethyl. More preferred examples include methyl. Further, the ring formed by $A^7$ and $A^{71}$ is preferably a 4- to 7-membered ring, and particularly preferred examples include 4-, 5-, and 6-membered rings.

Preferred examples of $A^8$ include hydrogen atom.
As $A^8$, an alkyl group, an alkyl group substituted with an aryl group, an alkyl group substituted with a heterocyclic group, an alkyl group substituted with hydroxyl group, an alkyl group substituted with amino group, an alkyl group substituted with an alkylamino group, an alkyl group substituted with an alkoxy group, and an alkyl group substituted with an acylamino group are also preferred, and more preferred examples of the alkyl group of these alkyl groups include a lower alkyl group.

Preferred examples of $A^8$ include a lower alkyl group. Specific examples include methyl, ethyl, propyl, isopropyl, n-butyl, isobutyl, tert-butyl and the like, and particularly preferred examples include methyl, ethyl and the like.
Preferred examples of $A^8$ include a lower alkyl group substituted with an aryl group (aryl is the same as that described above). Specific examples include benzyl, 2-phenylethyl, 1-naphthylmethyl, 2-naphthylmethyl and the like, and preferred examples include benzyl, 2-phenylethyl and the like. The "aryl ring" moiety of these groups may be substituted with a group similar to $A^{61}$ mentioned above.
Preferred examples of $A^8$ include a lower alkyl group substituted with a heterocyclic group (heterocyclic group is the same as that described above), and the heterocyclic group in this group include an "aromatic heterocyclic group", and a "non-aromatic heterocyclic group". As for the "lower alkyl group substituted with a heterocyclic group", specific examples of the lower alkyl group substituted with an aromatic heterocyclic group include 2-furylmethyl, 3-furylmethyl, 2-thienylmethyl, 3-thienylmethyl, 2-pyrrolylmethyl, 3-pyrrolylmethyl and the like. Further, as for the "lower alkyl group substituted with a heterocyclic group", specific examples of the lower alkyl group substituted with a non-aromatic heterocyclic group include oxylanylmethyl, azetidinylmethyl, pyrrolidinylmethyl, tetrahydrofurylmethyl, thiolanylmethyl, piperidylmethyl, tetrahydropyranylmethyl, morpholinylmethyl, thiomorpholinylmethyl, piperazinylmethyl and the like.

Preferred examples of $A^8$ also include a lower alkyl group substituted with hydroxyl group. In this group, the lower alkyl group may be substituted with one or more hydroxyl groups, and a lower alkyl group substituted with one of hydroxyl group is generally preferred. Specific examples include 2-hydroxyethyl, 3-hydroxypropyl, 2-hydroxypropyl, 4-hydroxybutyl and the like, and particularly preferred examples include 2-hydroxyethyl, and 3-hydroxyethyl.
Preferred examples of $A^8$ include a lower alkyl group substituted with amino group. In this group, the lower alkyl group may be substituted with one or more amino groups, and a lower alkyl group substituted with one of amino group is generally preferred. Specific examples include, 2-aminoethyl, 3-aminopropyl, 2-aminopropyl, 4-aminobutyl and the like, and particularly preferred examples include 2-aminoethyl, and 3-aminoethyl.
As $A^8$, a lower alkyl group substituted with a lower alkylamino group is also preferred. In this group, the lower alkyl group may be substituted with one or more lower alkylamino groups, and a lower alkyl group substituted with one of lower alkylamino group is generally preferred. The "lower alkylamino group" herein referred to include a monoalkylamino group, and dialkylamino group, which are substituted with one or two lower alkyl groups. As for the dialkylamino group, the alkyl groups may be the same or different. The lower alkyl group may be substituted with one or more of the amino groups, and a lower alkyl group substituted with one alkylamino group is generally preferred. Preferred examples include 2-(methylamino)ethyl, 2-(dimethylamino)ethyl, 3-(methylamino)propyl, 3-(dimethylamino)propyl, 4-(methylamino)butyl, and 4-(dimethylamino)butyl, and particularly preferred examples include 2-(methylamino)ethyl, and 3-(methylamino)propyl.

As $A^8$, a lower alkyl group substituted with an alkoxy group is also preferred. The lower alkoxy group include linear or branched alkoxy having 1 to 4 carbon atoms. Specific examples include, for example, methoxy group, ethoxy group, propoxy group, isopropoxy group and the like, and methoxy group, and ethoxy group are preferred. The lower alkyl group may be substituted with one or more lower alkoxy groups, and a lower alkyl group substituted with one alkoxy group is generally preferred. Preferred examples include 2-methoxyethyl, 3-methoxypropyl, 2-ethoxyethyl, 3-ethoxypropyl, and 4-methoxybutyl, and 2-methoxyethyl, and 3-methoxypropyl are particularly preferred.
As $A^8$, a lower alkyl group substituted with an acylamino group is also preferred. In this group, the lower alkyl group may be substituted with one or more acylamino groups, and a lower alkyl group substituted with one acylamino group is generally preferred. Examples of the "acylamino group" in this group include an alkylcarbonylamino group, and an arylcarbonylamino group ("alkyl" and "aryl" herein referred to are the same as those described above).
As $A^8$, a lower alkyl group substituted with an alkylcarbonylamino group is also preferred. In this group, the lower alkyl group may be substituted with one or more alkylcarbonylamino groups, and a lower alkyl group substituted with one of alkylcarbonylamino group is generally preferred. Specific examples include acetylaminomethyl, 2-(acetylamino)ethyl, 3-(acetylamino)propyl, 4-(acetylamino)butyl, propionylaminomethyl, 2-(propionylamino)ethyl, 3-(propionylamino)propyl, 4-(propionylamino)butyl and the like.

As $A^8$, a lower alkyl group substituted with an arylcarbonylamino group is also preferred. In this group, the lower alkyl group may be substituted with one or more arylcarbonylamino groups, and a lower alkyl group substituted with one of arylcarbonylamino group is generally preferred. Specific examples include benzoylaminomethyl, 2-(benzoylamino)ethyl, 3-(benzoylamino)propyl, 4-(benzoylamino)butyl and the like.
As $A^8$, a cycloalkyl group is also preferred. The cycloalkyl is the same as that described above. A lower cycloalkyl group is preferred, and particularly preferred examples include cyclopropyl, cyclobutyl, cyclopentyl, cyclohexyl and the like. As $A^8$, an aryl group is also preferred. The aryl is the same as that described above, and preferred examples include phenyl,

1-naphthyl, 2-naphthyl and the like. As $A^8$, an aromatic heterocyclic group is also preferred. The aromatic heterocyclic group is the same as that described above, and specific examples include, 2-furyl, 3-furyl, 2-thienyl, 3-thienyl, 2-pyrrolyl, 3-pyrrolyl and the like.

It is also preferred that $A^{71}$ and $A^8$ together become an alkylene group, -alkylene-O-alkylene-, -alkylene-NH-alkylene-, or -alkylene-N(alkyl)-alkylene- to form a ring (the alkylene moiety mentioned above may be substituted with an alkyl group). When $A^{71}$ and $A^8$ combine together to form a ring, and have an alkyl group on the alkylene moiety, the alkyl group is preferably a lower alkyl group, and examples of the lower alkyl group include, methyl, and ethyl. More preferred examples include methyl. Further, the ring formed by $A^{71}$ and $A^8$ combined together to become alkylene is preferably a 4- to 7-membered ring, and particularly preferred examples include 4-, 5-, and 6-membered rings. The ring formed by $A^{71}$ and $A^8$ combined together to become -alkylene-O-alkylene-, -alkylene-NH-alkylene-, or -alkylene-N (alkyl)-alkylene- is preferably a 6- or 7-membered ring.

When $A^7$ and $A^{71}$ combine together to form a ring, it is preferred that $A^{71}$ and $A^8$ do not combine together to form a ring. Groups in each of one or more combinations selected from the group consisting of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ may bind to each other to form a 5- or 6-membered ring, particularly preferably a 6-membered ring. In this group, it is particularly preferred that one 5- or 6-membered ring is formed with one of the aforementioned combinations. The ring is preferably consists of carbon atoms except for the N atom to which $A^3$ binds. When a ring is formed with $A^6$ and $A^1$, $A^6$ and $A^2$, or $A^6$ and $A^3$, or a ring is formed with $A^2$ and $A^3$, $A^{11}$ and $A^{21}$ are preferably hydrogen atoms, and the ring is most preferably a saturated ring.

Further, when Y is a single bond, and Z is $-N(A^6)(A^{61})$, it is preferred that, for example, the groups of $A^6$ and $A^1$ bind to each other to form a 5- or 6-membered ring.
Moreover, when Y is $-CH(A^3)-$, and Z is $-N(A^6)(A^{61})$, it is preferred that, for example, the groups of $A^6$ and $A^3$ bind to each other to form a 6-membered ring.
Furthermore, when Y is $-CH(A^3)-C(A^4)(A^{41})$, and Z is $-N(A^6)(A^{61})$, it is preferred that, for example, the groups of $A^2$ and $A^3$ bind to each other to form a 6-membered ring.

Specifically, examples of the structure represented by the formula (2):

[Formula 2]

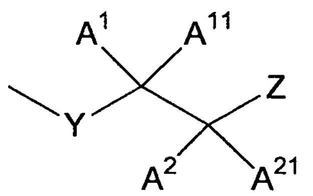

(2)

[the bond on the left of Y binds to N (nitrogen atom) bonding to $X^2$], which is a partial structure in the formula (1), wherein a ring is formed with the aforementioned combinations, include the followings structures containing a 5- or 6-membered ring, i.e., groups represented by the formula, (2-1), formula (2-2), formula (2-3), formula (2-4-t), formula (2-4-c), formula (2-5-t), formula (2-5-c), formula (2-6-t), formula (2-6-c), and formula (2-7).

[Formula 3]

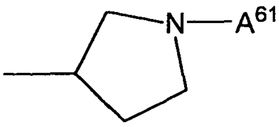

(2-1)

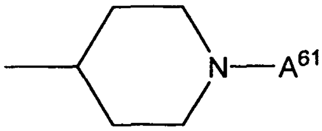

(2-2)

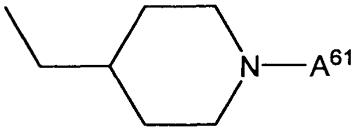

(2-3)

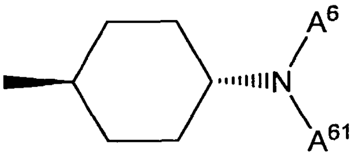

(2-4-t)

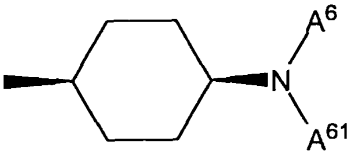

(2-4-c)

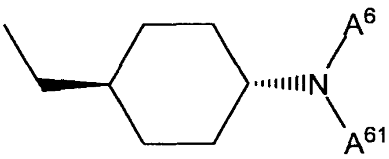

(2-5-t)

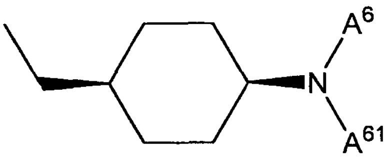

(2-5-c)

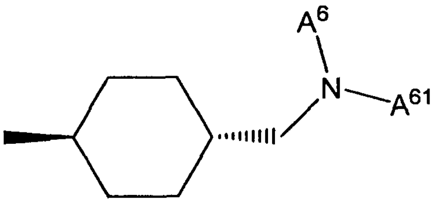

(2-6-t)

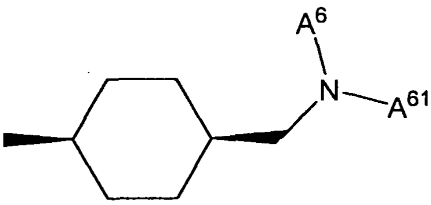

(2-6-c)

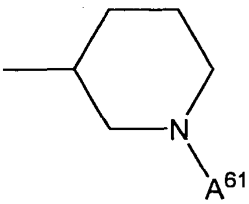

(2-7)

In the formulas, $A^6$ represents hydrogen atom, or an alkyl group, $A^{61}$ represents hydrogen atom, an alkyl group, an alkyl group substituted with an aryl group, an alkyl group substituted with an aromatic heterocyclic group, an alkyl group substituted with carboxyl group, an alkyl group substituted with cyano group, an alkyl group substituted with hydroxyl group, an alkyl group substituted with an alkoxy group, an alkyl group substituted with a substituted alkoxy group, an

alkyl group substituted with amino group, an alkyl group substituted with an alkylamino group, an alkyl group substituted with aminocarbonyl group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$, wherein $-X^3-$ represents carbonyl group, $-SO_2-$, -C(=O)-O-, -C(=O)-N$(A^8)$-, or -C(=S)-N$(A^8)$-, $A^7$ represents hydrogen atom, an alkyl group, an alkyl group substituted with hydroxyl group, an alkyl group substituted with amino group, an alkyl group substituted with an alkylamino group, an alkyl group substituted with aminocarbonyl group, an alkyl group substituted with an acylamino group, or an alkyl group substituted with cyano group, and $A^{71}$ and $A^8$ independently represent hydrogen atom, an alkyl group, an alkyl group substituted with an aryl group, an alkyl group substituted with a heterocyclic group, an alkyl group substituted with hydroxyl group, an alkyl group substituted with amino group, an alkyl group substituted with an alkylamino group, an alkyl group substituted with an alkoxy group, an alkyl group substituted with an acylamino group, a cycloalkyl group, an aryl group, or an aromatic heterocyclic group, or $A^7$ and $A^{71}$ combine together to become an alkylene group, or an alkylene group substituted with an alkyl group to form a ring, or $A^{71}$ and $A^8$ combine together to become an alkylene group, -alkylene-O-alkylene-, -alkylene-NH-alkylene-, or -alkylene-N(alkyl)-alkylene- to form a ring (wherein said alkylene moiety may be substituted with an alkyl group). The leftmost single bond in each formula binds to N (nitrogen atom) binding to $X^2$ in the formula (1).

In the groups of these formulas, the bonds in the cyclohexane ring are in the trans-conformation in the groups represented by the formula (2-4-t), formula (2-5-t), and formula (2-6-t), or cis-conformation in the groups represented by the formula (2-4-c), formula (2-5-c), and formula (2-6-c).
Among them, the groups represented by the formula (2-1), formula (2-2), formula (2-4-t), formula (2-4-c), and formula (2-7) are preferred, and the groups represented by the formula (2-1), formula (2-2), formula (2-4-t), and formula (2-7) are particularly preferred. Preferred examples also include either one of or a combination of any two of these groups. Preferred examples of $A^6$ and $A^{61}$ are as mentioned above.

The compounds of the present invention represented by the formula (1) may have one or more asymmetric carbons, and stereoisomers based on such asymmetric carbons such as optical antipodes and diastereoisomer may exist. The stereoisomers in pure forms, any mixtures, racemates and the like of the stereoisomers all fall within the scope of the present invention. Further, when the compounds of the present invention have an olefinic double bond or a cyclic structure, two or more kinds of stereoisomers may exist, and such stereoisomers in pure forms, any mixtures and the like of such stereoisomers all fall within the scope of the present invention. Furthermore, the compounds of the present invention represented by the formula (1) may exist as tautomers. Existence of such tautomers is apparent to those skilled in the art, and such tautomers all fall within the scope of the present invention.

The compounds of the present invention may also exist as salts. Forms of the salts are not particularly limited. Acid addition salts are generally formed, or base addition salts may be formed depending on the types of substituents. The types of physiologically acceptable salts are well known to those skilled in the art, and examples include, for example, those described by Berge et al. in J. Pharm. Sci., 66, 1-19 (1977). Examples of the acid addition salts include, for example, mineral acid salts such as hydrochlorides, hydrobromides, hydroiodides, nitrates, sulfates, and hydrogensulfates, phosphates, hydrogenphosphates, organic acid salts such as acetates, trifluoroacetates, gluconates, lactates, salicylates, citrates, tartrates, ascorbates, succinates, maleates, fumarates, formates, benzoates, methanesulfonates, ethanesulfonates, benzenesulfonates and p-toluenesulfonates. Where one or more substituents contain an acidic moiety, examples of suitable pharmacologically acceptable base addition salts include, for example, metal salts such as sodium salts, potassium salts, magnesium salts, lithium salts, calcium salts, aluminum salts and zinc salts, and salts of organic amines such as ethanolamine.

Methods for preparation of the compounds represented by the formula (1) are not particularly limited. For example, they can be prepared according to the methods described below (Preparation methods 1 to 4).

(Preparation method 1)

The compounds represented by the formula (1) can be prepared from a compound represented by the following formula (A):

[Formula 4]

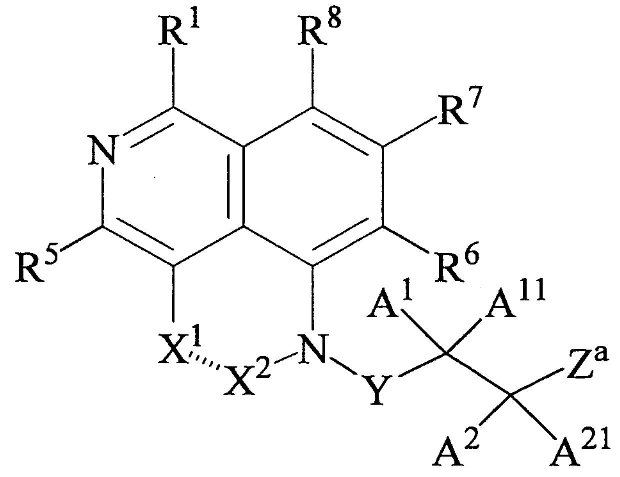

(A)

[wherein $R^1$, $R^5$, $R^6$, $R^7$, and $R^8$ independently represent hydrogen atom, a halogen atom, hydroxyl group, an alkyl group, an alkenyl group, an alkynyl group, an alkoxy group, amino group, an alkylamino group, or an arylamino group; $X^1$...$X^2$ represents $-CH(R^2)-CH(R^3)-$, $-CH(R^2)-CH(R3)-CH(R^4)-$, $-C(R^2)=C(R^3)-$, or $-C(R^2)=C(R^3)-CH(R^4)-$;
$R^2$, $R^3$, and $R^4$ independently represent hydrogen atom, or an alkyl group;
$A^1$, $A^{11}$, $A^2$, and $A^{21}$ independently represent hydrogen atom, or an alkyl group;
Y represents $-CH(A^3)-$, $-CH(A^3)-C(A^4)(A^{41})-$, $-CH(A^3)-C(A^4)(A^{41})-C(A^5)(A^{51})-$, or a single bond;
$A^3$, $A^4$, $A^{41}$, $A^5$, or $A^{51}$ independently represent hydrogen atom, or an alkyl group;
$Z^a$ represents $-O(PG^1)$, $-OH$, $-N(A^6)(PG^2)$, $-NH(A^6)$, $-N(A^6)(A^{62})$, or $-N(A^6)(A^{63})$;
$PG^1$ represents a protective group of hydroxyl group, $PG^2$ represents a protective group of amino group;
$A^6$ represents hydrogen atom, or an alkyl group;
$A^{62}$ represents an alkyl group, an alkyl group substituted with an aryl group, an alkyl group substituted with an aromatic heterocyclic group, an alkyl group substituted with carboxyl group, an alkyl group substituted with carboxyl group protected with a protective group of carboxyl group $PG^3$, an alkyl group substituted with cyano group, an alkyl group substituted with hydroxyl group, an alkyl group substituted with hydroxyl group protected with $PG^1$, an alkyl group substituted with an alkoxy group, an alkyl group substituted with a substituted alkoxy group, an alkyl group substituted with amino group, an alkyl group substituted with amino group protected with $PG^2$, an alkyl group substituted with an alkylamino group, an alkyl group substituted with an alkylamino group protected with $PG^2$, an alkyl group substituted with aminocarbonyl group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with mercapto group protected with a protective group of mercapto group $PG^4$, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$, where $-X^3-$ represents carbonyl group, $-SO_2-$, $-C(=O)-O-$, $-C(=O)-N(A^8)-$, or $-C(=S)-N(A^8)-$, $A^7$ represents hydrogen atom, an alkyl group, an alkyl group substituted with hydroxyl group, an alkyl group substituted with amino group, an alkyl group substituted with an alkylamino group, an alkyl group substituted with aminocarbonyl group, an alkyl group substituted with an acylamino group, or an alkyl group substituted with cyano group, and $A^{71}$ and $A^8$ independently represent hydrogen atom, an alkyl group, an alkyl group substituted with an aryl group, an alkyl group substituted with a heterocyclic group, an alkyl group substituted with hydroxyl group, an alkyl group substituted with amino group, an alkyl group substituted with an alkylamino group, an alkyl group substituted with an alkoxy group, an alkyl group substituted with an acylamino group, a cycloalkyl group, an aryl group, or an aromatic heterocyclic group, or $A^7$ and $A^{71}$ may combine together to become an alkylene group, or an alkylene group substituted with an alkyl group to form a ring, or $A^{71}$ and $A^8$ may combine together to become an alkylene group, -alkylene-O-alkylene-,
-alkylene-NH-alkylene-, or -alkylene-N(alkyl)-alkylene- to form a ring (wherein said alkylene moiety may be substituted with an alkyl group);
$A^{63}$ represents an alkyl group of which end is substituted with $NH(A^7)$;
$PG^3$ represents a protective group of carboxyl group, $PG^4$ represents a protective group of mercapto group; and groups in one or more combinations selected from the group consisting of combinations of $A^6$ and $A^3$, $A^6$ and $A^4$, $A^6$

and $A^1$, $A^6$ and $A^2$, $A^2$ and $A^3$, $A^2$ and $A^4$, $A^6$ and $A^5$, $A^3$ and $A^1$, and $A^5$ and $A^1$ may bind to each other to form a 5- or 6-membered ring] by removing a protective group of the compound (Step 1-1).

**[0185]** [0185] The $PG^1$ group used herein is not particularly limited so long that the group can protect hydroxyl group, does not react in reactions in this preparation process other than the deprotection step, and further can be easily removed. Preferred examples of the protective group of hydroxyl group include a trialkylsilyl group such as tert-butyldimethylsilyl group (TBDMS group), an acyl group such as acetyl group, benzyl group (Bn group), and tetrahydropyranyl (THP) group, and particularly preferred examples include Bn group and THP group.

The $PG^2$ group is not particularly limited so long that the group can protect amino group, does not react in reactions in this preparation process other than the deprotection step, and further can be easily removed. Preferred examples include t-butoxycarbonyl group (Boc group), benzyloxycarbonyl group (Cbz group), benzyl group (Bn group), phthaloyl group, and triphenylmethyl group, and particularly preferred examples include Boc group, Cbz group, and Bn group.

The $PG^3$ group is not particularly limited so long that the group can protect carboxyl group, does not react in reactions in this preparation process other than the deprotection step, and further can be easily removed. Examples include alkyl groups, and specifically, tert-butyl group is preferred, for example.

The $PG^4$ group is not particularly limited so long that the group can protect mercapto group, does not react in reactions in this preparation process other than the deprotection step, and further can be easily removed. Preferred examples include an acyl group such as acetyl group (Ac group), benzyl group (Bn group), triphenylmethyl group, methoxymethyl group, and tetrahydropyranyl group (THP group), and particularly preferred examples include Ac group, and THP group.

In the aforementioned deprotection steps, when $PG^1$, $PG^2$, $PG^3$, or $PG^4$ exists in a compound of the formula (A), the group can be removed by a known reaction depending on a kind of the protective group. Methods for the deprotection are apparent to those skilled in the art by referring to prior art described in, for example, Greene, T.W. and Wuts, P.G.M. "Protective Groups in Organic Synthesis", John Wiley and Sons Inc. (3rd edition); and Kocienski, P.J., "Protecting Groups", Georg Thieme Verlag (1994).

More specific explanation will be set forth below. For example, a method of preparing the compounds of the formula (1) by removing Bn group from a compound of the formula (A) wherein $PG^1$ represents Bn group can be performed by using known reduction conditions of hydrogenation. Examples of the method include a method performed in an alcohol, ethyl acetate, an ether solvent such as 1,4-dioxane, or a mixed solvent thereof, and examples of catalyst include, for example, palladium/carbon. Examples of the reaction include a method of performing the reaction at 0 to 80°C, preferably 10 to 40°C.

For example, examples of a method of preparing the compounds of the formula (1), by removing THP group from a compound of the formula (A) wherein $PG^1$ represents THP group, include a method utilizing acidolysis. Examples of the acid include mineral acids, and specific examples are hydrochloric acid, sulfuric acid, nitric acid, phosphoric acid and the like, and hydrochloric acid is preferred. The acid is preferably used in an amount of 1 to 100 fold moles. Examples of the solvent include water, alcohols, ether type solvents such as 1,4-dioxane, and mixed solvents thereof. The reaction is preferably performed in the temperature range of from room temperature to reflux temperature of the solvent.

For example, a method of preparing the compounds of the formula (1) by removing Boc group from a compound of the formula (A) wherein $PG^2$ represents Boc group can be performed by using known acidic conditions. As for the solvent used for the reaction, the reaction can be performed, for example, without solvent, or in water, an alcohol, acetonitrile, an ether solvent such as 1,4-dioxane, or a mixed solvent thereof. As the acid, a mineral acid and organic acid can be used. Specific examples include hydrochloric acid, sulfuric acid, nitric acid, acetic acid, methanesulfonic acid, phosphoric acid and the like, and hydrochloric acid is preferred. The acid is preferably used in an amount of 1 to 100 fold moles based on the compound of the formula (A). The reaction is preferably performed in the temperature range of from room temperature to the reflux temperature of the solvent. Alternatively, the removal of Boc group can be performed by using trifluoroacetic acid. Examples of this method include a method of using trifluoroacetic acid alone, and a method of using trifluoroacetic acid as a mixed solvent system with water or dichloromethane. The reaction is performed, for example, in the temperature range of from 0 to 100°C, preferably from room temperature to 50°C. As for the amount of trifluoroacetic acid, 1 to 100 fold moles are preferably used based on the compound of the formula (A).

Further, the method of preparing the compounds of the formula (1) by removing Cbz group (or Bn group) from a compound of the formula (A) wherein $PG^2$ represents Cbz group or Bn group can be performed by using known reduction conditions of hydrogenation. Examples of the method include a method performed in an alcohol, ethyl acetate, an ether type solvent such as 1,4-dioxane, or a mixed solvent thereof, and examples of catalyst include, for example, palladium/carbon. The reaction is performed, for example, at 0 to 100°C, preferably 10 to 80°C.

Further, the method of preparing the compounds of the formula (1) by removing tert-butyl group from a compound of the formula (A) wherein $PG^3$ represents tert-butyl group can be performed by known acidolysis. Examples of the acid include mineral acids. Specific examples include hydrochloric acid, sulfuric acid, nitric acid, phosphoric acid and the like, and hydrochloric acid is preferred. As for the amount of the acid used, it is preferable to use 1 to 100 fold moles. Examples of the solvent used for the reaction include, for example, water, alcohols, ether solvents such as 1,4-dioxane and mixed solvents thereof, and 1,4-dioxane is preferred. The reaction is preferably performed in the temperature range of from

room temperature to the reflux temperature of the solvent.

The method of preparing the compounds of the formula (1) by removing Ac group form a compound of the formula (A) wherein $PG^4$ represents Ac group can be performed by a known decomposition method using aqueous alkali or aqueous ammonia. Examples of the alkali include, for example, sodium hydroxide, potassium hydroxide, potassium carbonate, sodium carbonate, sodium methoxide and the like, and sodium hydroxide is preferred. The concentration of the aqueous alkali is, for example, 0.001 to 5 N, preferably 0.05 to 3 N. The concentration of aqueous ammonia is preferably, for example, 1 to 30%. Examples of the solvent used for the reaction include, for example, water, an alcohol, an ether type solvent such as tetrahydrofuran, and 1,4-dioxane, and a mixed solvent of these, and a mixed solvent of water and methanol is preferred. The reaction is preferably performed at a temperature in the range of from 0°C to the reflux temperature of the solvent.

Furthermore, examples of the method of preparing the compounds of the formula (1) by removing THP from a compound of the formula (A) wherein $PG^4$ represents the THP group include known decomposition methods using aqueous silver nitrate (see, for example, G.F. Holland, L.A. Cohen, J. Am. Chem. Soc., 80, 3765 (1958) and the like), or hydrogen bromide/acetic acid (see, for example, K. Hammerstrom, W. Lunkenheimer, H. Zahn, Makromol. Chem., 41, 133 (1970) and the like).

When any of hydroxyl group, amino group, carboxyl group, and mercapto group exists in the compound of the formula (A), a protective group corresponding to them, $PG^1$, $PG^2$, $PG^3$, or $PG^4$, is introduced into the compound, and it is preferable to carry out deprotection for this group in order to obtain a compound of the formula (1). Further, one or more kinds of these protective groups may be introduced into the compound of the formula (A) as the case may be, and when two or more protective groups exist, it is also preferable to carry out deprotection with a combination of the methods mentioned for Step 1-1. When $Z^a$ represents the same group as that represented by Z in the general formula (1), such compounds of the formula (A) constitute a part of the compounds of the formula (1), and thus Step 1-1 mentioned above is unnecessary.

The compounds of the formula (A) can be classified into compounds represented by the following formula (A-1):

[Formula 5]

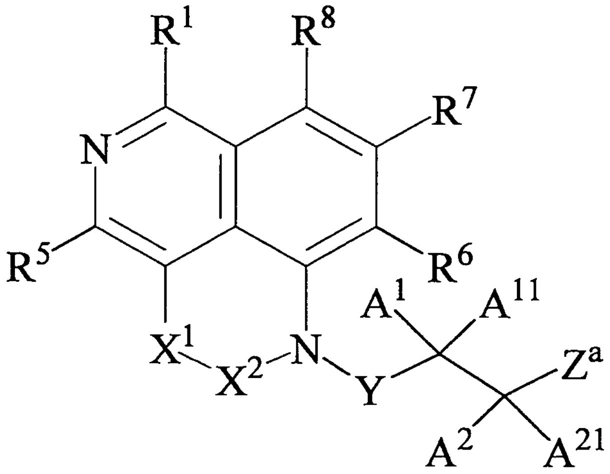

(A-1)

[wherein Y, $A^1$, $A^{11}$, $A^2$, $A^{21}$, $R^1$, $R^5$, $R^6$, $R^7$, $R^8$, and $Z^a$ have the same meanings as those defined above, and $X^1$-$X^2$ represents $-CH(R^2)-CH(R^3)-$, or $-CH(R^2)-CH(R^3)-CH(R^4)-$], and compounds represented by the following formula (A-2):

[Formula 6]

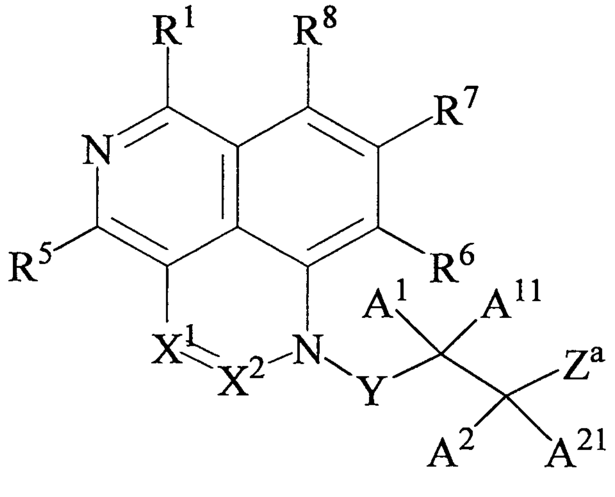

(A-2)

[wherein Y, $A^1$, $A^{11}$, $A^2$, $A^{21}$, $Z^a$, $R^1$, $R^5$, $R^6$, $R^7$, and $R^8$ have the same meanings as those defined above, and $X^1$=$X^2$ represents -C($R^2$)=C($R^3$)-, or -C($R^2$)=C($R^3$)-CH($R^4$)-].

The compounds of the formula (A-2) can be prepared by dehydrogenation of a compound of the formula (A-1) in an inert solvent (Step 1-1-1). As the catalyst, for example, palladium catalysts such as 5% palladium/carbon, 10% palladium/carbon, or palladium black, and sulfur are preferred. Examples of the inert solvent include xylene, mesitylene, toluene and the like, and xylene is preferred. The reaction temperature is 60°C or higher, preferably 120 to 150°C.

The substituents on the aromatic ring of the compound of the formula (1), $R^1$, $R^5$, $R^6$, $R^7$, and $R^8$, can be introduced depending on the type thereof by a suitable combination of known reactions, for example, electrophilic substitution reactions such as halogenation and nitration, subsequent conversion of nitro group into amino group by reduction, conversion of amino group into a halogen atom, hydroxyl group, or the like, and conversion of a halogen atom into an alkyl group, an alkenyl group, an aryl group, amino group, an alkylamino group, an arylamino group, hydroxyl group, an alkoxyl group, or the like. The methods will be specifically mentioned later.

The compounds of the formula (A-1) mentioned above can be classified into the following two types of compounds, depending on the types of $R^1$, $R^5$, R6, $R^7$, and $R^8$; Specifically, i) compounds represented by the following formula (A-a):

[Formula 7]

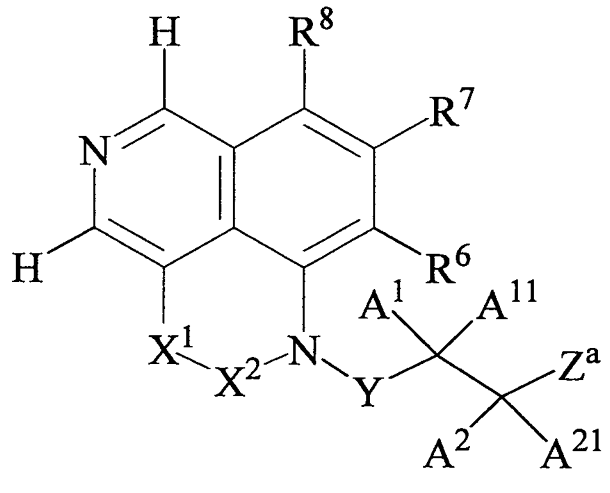

(A-a)

[wherein Y, $A^1$, $A^{11}$, $A^2$, $A^{21}$, $Z^a$, $R^6$, $R^7$, $R^8$, and $X^1$-$X^2$ have the same meanings as those defined above], which correspond to the compounds of the formula (A-1) wherein $R^1$, and $R^5$ are hydrogen atoms, and ii) compounds represented

by the following formula (A-d):

[Formula 8]

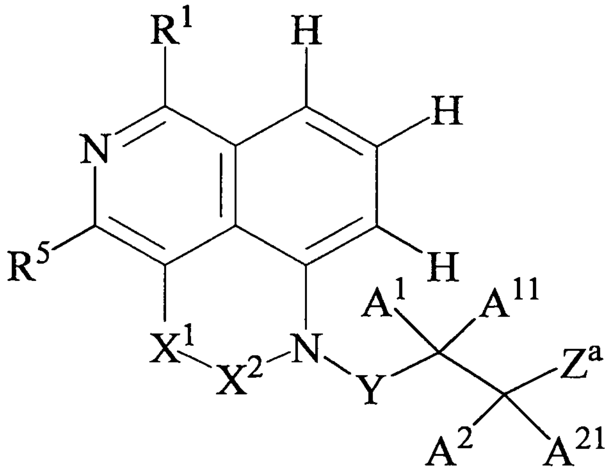

(A-d)

[wherein Y, $A^1$, $A^{11}$, $A^2$, $A^{21}$, $Z^a$, $R^1$, $R^5$, and $X^1$-$X^2$ have the same meanings as those defined above], which correspond to the compounds of the formula (A-1) wherein $R^6$, $R^7$, and $R^8$ are hydrogen atoms.

(Preparation method 1-a)

The compounds of the aforementioned formula (A-a) can be prepared, for example, as follows. Specifically, the compounds can be prepared by deprotecting (corresponding to the deprotection for the protective group of amino group in Step 1-1) a compound of the following formula (A-a-1):

[Formula 9]

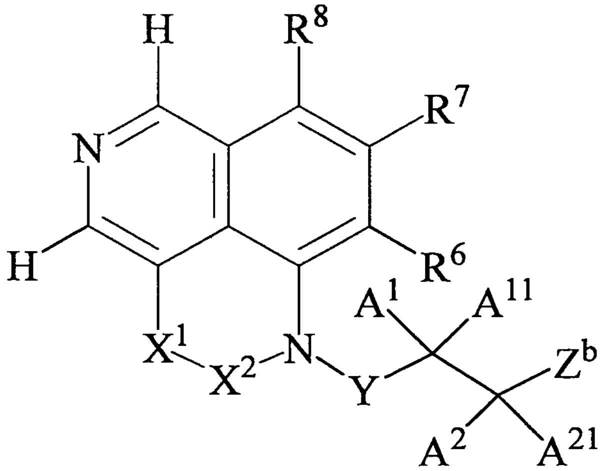

(A-a-1)

[wherein Y, $A^1$, $A^{11}$, $A^2$, $A^{21}$, $R^6$, $R^7$, $R^8$, and $X^1$-$X^2$ have the same meanings as those defined above, $Z^b$ is -N($A^6$)($PG^2$), and $A^6$ and $PG^2$ have the same meanings as those defined above], which corresponds to a compound of the formula (A-a) wherein $Z^a$ is -N($A^6$)($PG^2$), to prepare a compound of the following formula (A-a-2):

[Formula 10]

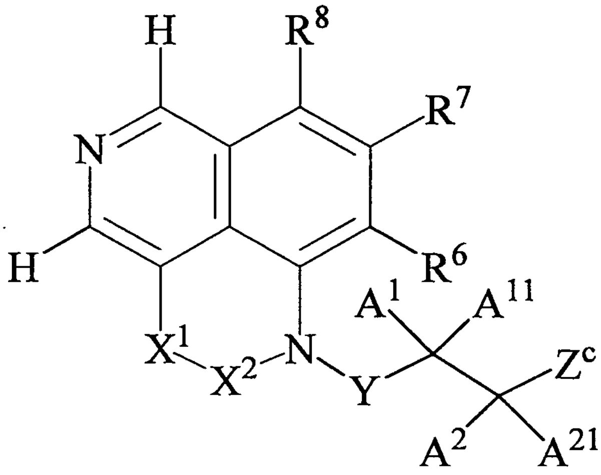

(A-a-2)

[wherein Y, $A^1$, $A^{11}$, $A^2$, $A^{21}$, $X^1$-$X^2$, $R^6$, $R^7$, and $R^8$ have the same meanings as those defined above, $Z^c$ is -NH($A^6$), and $A^6$ has the same meaning as that defined above], which corresponds to a compound of the formula (A-a) wherein $Z^a$ is -NH($A^6$) (which constitutes a part of the compounds of the formula (1)) (Step 1-2-1), and further reacting this compound with a compound represented as $A^{62}$-W [wherein $A^{62}$ has the same meaning as defined above, and W represents a leaving group] (Step 1-2-2).

For the deprotection step performed for the preparation of a compound of the formula (A-a-2) from a compound of the formula (A-a-1), an ordinary deprotection reaction can be utilized as explained above for the $PG^2$ group.
W in $A^{62}$-W used for the preparation of a compound of the formula (A-a) wherein $Z^a$ is -NH($A^6$) from a compound of the formula (A-a-2) is not particularly limited so long as W is a leaving group. Examples include, for example, a halogen atom, an alkylsulfonyloxy group, and an arylsulfonyloxy group, preferred examples include chlorine atom, bromine atom, iodine atom, methanesulfonyloxy group, and p-toluenesulfonyloxy group, particularly preferred examples are chlorine atom, bromine atom, and iodine atom, and still more particularly preferred examples are chlorine atom, and bromine atom.
Conditions of the reaction for preparing the compounds of the formula (A-a) wherein $Z^a$ is -N($A^6$)($A^{62}$) from a compound of the formula (A-a-2) are as follows. Specifically, the reaction is usually performed in the presence of a base, and a mineral base is preferred. Examples include potassium carbonate, sodium carbonate, cesium carbonate, sodium hydrogencarbonate, potassium hydroxide, and sodium hydroxide, and potassium carbonate is particularly preferred.
The compound represented as $A^{62}$-W is preferably used in an amount of 1 fold mole or more, particularly preferably 2 to 10 fold moles, based on the compound of the aforementioned formula (A-a-2).
As the reaction solvent, an inert solvent, for example, an alcoholic solvent such as methanol and ethanol, N,N-dimethylformamide, N,N-dimethylacetamide, N-methylpyrrolidone, sulfolane, tetrahydrofuran, 1,4-dioxane, acetone, 2-butanone, dimethyl sulfoxide, acetonitrile and the like can be used alone, or as a mixed solvent thereof, and water, dimethylformamide and acetone are preferred.
The reaction temperature is, for example, -10°C or higher, preferably 10 to 40°C. The reaction time is, for example, usually 0.5 hour or more, preferably 2 to 10 hours.

Further, the compounds of the following formula (A-b):

[Formula 11]

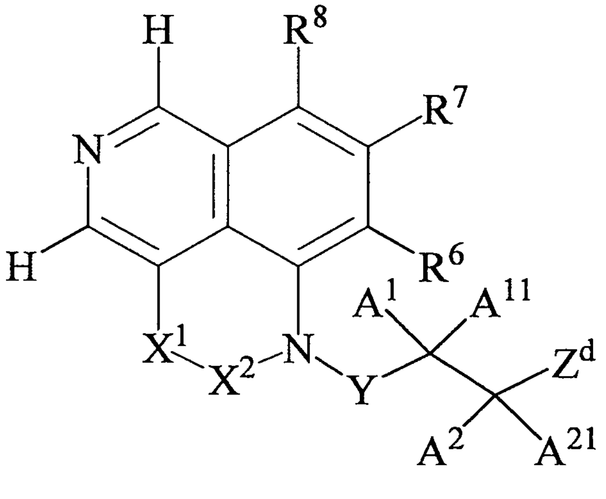

(A-b)

[wherein Y, $A^1$, $A^{11}$, $A^2$, $A^{21}$, $X^1$-$X^2$, $R^6$, $R^7$, and $R^8$ have the same meanings as those defined above, $Z^d$ is -N($A^6$)($A^{63}$), and $A^6$ and $A^{63}$ have the same meanings as those defined above], which correspond to the compounds of the aforementioned formula (A-a) wherein $Z^a$ in the formula (A-a) is -N($A^6$)($A^{63}$), can be prepared by, for example, reacting a compound of the formula (A-a-2) with a compound represented as $A^{63}$-W [wherein $A^{63}$, and W have the same meanings as those defined above] (Step 1-2-2-1). Examples of this step include Step 1-2-2 mentioned above performed by using $A^{63}$-W instead of $A^{62}$-W.

For example, the compounds of the formula (A-a) wherein $Z^a$ is -N($A^6$)($A^{62}$), and $A^{62}$ is an alkyl group of which end is substituted with N($A^7$)(-$X^3$-$A^{71}$) [wherein -$X^3$- is carbonyl group, and $A^7$ and $A^{71}$ have the same meanings as defined above, provided that a compound where $A^7$ and $A^{71}$ combine together to become an alkylene group, or an alkylene group substituted with an alkyl group to form a ring is excluded] can be prepared by reacting the compound of the formula (A-b) obtained above with an acylation reagent suitably corresponding to an target compound to be prepared (Step 1-2-3).

Examples of the acylation reagent include carboxylic acid chlorides, carboxylic acid anhydrides, carboxylic acid active esters, carboxylic acids and the like. Examples of acetylation reagent include, for example, acetyl chloride, acetic anhydride, acetic acid active esters, acetic acid and the like. Examples of the carboxylic acid active esters include carboxylic acid succinimides, imidazole carboxylates, carboxylic acid 4-nitrophenyl esters, carboxylic acid pentafluorophenyl esters and the like. The acylation reagent is usually used preferably in an amount of 1 or more fold moles, most preferably 1.1 to 10 fold moles, based on the compound of the aforementioned formula (A-b). When a carboxylic acid is directly used as the acylation reagent, it is usually preferable to perform the reaction in the presence of a dehydration condensing agent. Examples of the dehydration condensing agent include N,N-dicyclohexylcarbodiimide, N,N-diisopropylcarbodiimide, 1-ethyl-3-(3-dimethylaminopropyl)carbodiimide (EDC), 2-chloro-1-methylpyridinium iodide, 2-(1H-benzotriazol-1-yl)-1,1,3,3-tetramethyluronium tetrafluoroborate (TBTU), benzotriazol-1-yloxytris(dimethylamino)phosphonium hexafluorophosphate (BOP), 2-(1H-benzotriazol-1-yl)-1,1,3,3-tetramethyluronium hexafluorophosphate (HBTU), O-(N-succinimidyl)-1,1,3,3-tetramethyluronium tetrafluoroborate (TSTU), benzotriazol-1-yloxytrispyrrolidinophosphonium hexafluorophosphate (PyBOP), bromotrispyrrolidinophosphonium hexafluorophosphate (PyBrOP), tetramethylfluoroformamidinium hexafluorophosphate (TFFH), 2-chloro-1-methylpyridinium iodide, 2,2-dipyridyl disulfide/triphenylphosphine, diethyl azodicarboxylate/triphenylphosphine and the like. These dehydration condensing agent is usually preferably used in an amount of 1 or more fold moles, most preferably 1.1 to 10 fold moles, based on the compound of the aforementioned formula (A-b).

The acylation reaction is also preferably performed, for example, in the presence of an additive such as 1-hydroxybenzotriazole (HOBt), 1-hydroxy-7-azabenzotriazole (HOAt), N-hydroxysuccinimide (HOSu), 4-nitrophenol (HONp) and pentafluorophenol (HOPfp). As for the amount of the additive, preferably 0.01 to 10 fold moles or more, most preferably 0.1 to 5 fold moles, are usually used based on the compound of the aforementioned formula (A-b).

The acylation reaction is also preferably performed, for example, in the presence of an organic tertiary amine such as triethylamine, N,N-diisopropylethylamine, pyridine, 4-dimethylaminopyridine, and 1,8-diazabicyclo[5.4.0]undec-7-ene, or an inorganic base such as potassium carbonate, sodium carbonate, cesium carbonate, sodium hydrogencarbonate, potassium hydroxide, and sodium hydroxide. As for the amount of the base, preferably 0.01 to 10 fold moles or more, most preferably 0.1 to 5 fold moles, are usually used based on the compound of the aforementioned formula (A-b).

As the reaction solvent, an inert solvent, for example, water, an alcoholic solvent such as tert-butanol, N,N-dimethylformamide, N,N-dimethylacetamide, N-methylpyrolidone, tetrahydrofuran, 1,4-dioxane, 1,2-dimethoxyethane, dichloromethane, chloroform, benzene, toluene, ethyl acetate, dimethyl sulfoxide, sulfolane, acetonitrile, or the like can be used as each kind, or a mixed solvent thereof.

The reaction temperature is, for example, -10°C or higher, preferably 10 to 40°C. The reaction time is, for example, usually 0.5 hour or more, preferably 2 to 10 hours.

For example, the compounds of the formula (A-a) wherein $A^{62}$ is "an alkyl group of which end is substituted with $N(A^7)(-X^3-A^{71})$" [wherein $-X^3-$ represents carboxyl group], and $A^7$ and $A^{71}$ together become an alkylene group, or an alkylene group substituted with an alkyl group to form a ring can be prepared in the same manner as Step 1-2-2 by reacting the "compound of the formula (A-b) wherein $A^7$ is hydrogen atom" obtained above as a starting material with a compound represented as $(PG^3)O-X^3-A^{72}$ [wherein $PG^3$ has the same meaning as defined above, $-X^3-$ is carbonyl group, and $A^{72}$ represents an alkyl group of which end is substituted with a leaving group (the alkyl group may be substituted with another alkyl group)], then removing $PG^3$ by the aforementioned known method, and cyclizing the resultant using a dehydration condensing agent similar to those used in the aforementioned acylation reaction (Step 1-2-4). The leaving group is the same as that described above.

For example, the compounds of the formula (A-a) wherein $Z^a$ is $-N(A^6)(A^{62})$, and $A^{62}$ is an alkyl group of which end is substituted with $N(A^7)(-X^3-A^{71})$ [where $-X^3-$ represents $-SO_2-$, and $A^7$, and $A^{71}$ have the same meanings as those defined above, provided that a compound where $A^7$ and $A^{71}$ combine together to become an alkylene group, or an alkylene group substituted with an alkyl group to form a ring is excluded ($A^7$ preferably represents hydrogen atom, or an alkyl group, and $A^{71}$ preferably represents an alkyl group, aralkyl group, or an aryl group)] can be prepared by reacting the compound of the formula (A-b) obtained above with a sulfonylation reagent suitably corresponding to an target compound to be prepared in the presence of a base by a known method (Step 1-2-5). Examples of the sulfonylation reagent include sulfonyl chloride and sulfonic acid anhydride, and sulfonic acid anhydride is particularly preferred. Preferred examples of the base include an organic tertiary amine such as triethylamine, N,N-diisopropylethylamine, pyridine, 4-dimethylaminopyridine, and 1,8-diazabicyclo[5.4.0]undec-7-ene, and an inorganic base such as potassium carbonate, sodium carbonate, cesium carbonate, sodium hydrogencarbonate, potassium hydroxide, and sodium hydroxide. The amount of the base is preferably 0.01 to 10 fold moles or more, particularly preferably 0.1 to 5 fold moles. As the reaction solvent, an inert solvent, for example, water, an alcoholic solvent such as tert-butanol, N,N-dimethylformamide, N,N-dimethylacetamide, N-methylpyrolidone, tetrahydrofuran, 1,4-dioxane, 1,2-dimethoxyethane, dichloromethane, chloroform, benzene, toluene, ethyl acetate, dimethyl sulfoxide, sulfolane, acetonitrile, or the like can be used independently, or a mixed solvent thereof. The reaction temperature is, for example, -10°C or higher, preferably 10 to 40°C. The reaction time is, for example, usually 0.5 hour or more, preferably 2 to 10 hours.

For example, the compounds of the formula (A-a) wherein $Z^a$ is $-N(A^6)(A^{62})$, and $A^{62}$ is an alkyl group of which end is substituted with $N(A^7)(-X^3-A^{71})$ [where $-X^3-$ represents -C(=O)-O-, or $-C(=O)-N(A^8)-$, and $A^7$, and $A^{71}$ have the same meanings as those defined above, provided that a compound where $A^7$ and $A^{71}$ combine together to become an alkylene group, or an alkylene group substituted with an alkyl group to form a ring, or $A^{71}$ and $A^8$ combine together to become an alkylene group, -alkylene-O-alkylene-, -alkylene-NH-alkylene-, or -alkylene-N(alkyl)-alkylene- to form a ring is excluded] can be prepared by deriving the compound of the formula (A-b) obtained above into an active ester, and reacting the active ester with an alcohol or amine suitably corresponding to an target compound to be prepared (Steps 1-2-6 and 1-2-7). Example of the reagent used for preparing the active ester include 4-nitrophenyl chloroformate, 4-nitrophenyl carbonate, 1,1'-carbonyldiimidazole and the like, and preferred examples include 4-nitrophenyl chloroformate. The active esterification regent is preferably used in an amount of 1 fold mole or more, particularly preferably 1.1 to 10 fold moles, based on the compound of the formula (A-b) mentioned above.

The acylation reaction is also preferably performed, for example, in the presence of an organic tertiary amine such as triethylamine, N,N-diisopropylethylamine, pyridine, 4-dimethylaminopyridine, and 1,8-diazabicyclo[5.4.0]undec-7-ene, or an inorganic base such as potassium carbonate, sodium carbonate, cesium carbonate, sodium hydrogencarbonate, potassium hydroxide, and sodium hydroxide. As for the amount of the base, preferably 0.01 to 10 fold moles or more, most preferably 0.1 to 5 fold moles, are usually used based on the compound of the aforementioned formula (A-b). As the reaction solvent, an inert solvent such as N,N-dimethylformamide, N,N-dimethylacetamide, N-methylpyrolidone, tetrahydrofuran, 1,4-dioxane, 1,2-dimethoxyethane, dichloromethane, chloroform, benzene, toluene, dimethyl sulfoxide, sulfolane, acetonitrile, or the like can be used independently, or as a mixed solvent thereof. The reaction temperature is, for example, -10°C or higher, preferably 10 to 40°C. The reaction time is, for example, usually 0.5 hour or more, preferably 2 to 10 hours.

Further, the target compound can be prepared by successively reacting the active ester prepared as described above with an alcohol or amine suitably corresponding to an target compound to be prepared under the same conditions as mentioned above.

For example, the compounds of the formula (A-a) wherein $Z^a$ is $-N(A^6)(A^{62})$, and $A^{62}$ is an alkyl group of which end is substituted with $N(A^7)(-X^3-A^{71})$ [where $-X^3-$ represents $-C(=S)-N(A^8)-$, and $A^7$, and $A^{71}$ have the same meanings as those defined above, provided that a compound where $A^7$ and $A^{71}$ combine together to become an alkylene group,

or an alkylene group substituted with an alkyl group to form a ring, or $A^{71}$ and $A^8$ combine together become an alkylene group, -alkylene-O-alkylene-, -alkylene-NH-alkylene-, or -alkylene-N(alkyl)-alkylene- to form a ring is excluded] can be prepared by deriving a compound of the formula (A-b) into an active ester using 1,1'-thiocarbonyldiimidazole instead of 1,1'-carbonyldiimidazole used in Steps 1-2-6 and 1-2-7, and reacting the active ester with an amine suitably corresponding to an target compound to be prepared under similar conditions (Steps 1-2-8).
For example, the compounds of the formula (A-a) wherein $Z^a$ is -N($A^6$)($A^{62}$), and $A^{62}$ is "an alkyl group of which end is substituted with an alkylaminocarbonyloxy group, an alkyl group substituted with a cycloalkylaminocarbonyloxy group, or an alkyl group substituted with an arylaminocarbonyloxy group" can be prepared from a compound of the formula (A-a) wherein $Z^a$ is an alkyl group substituted with hydroxyl group in the same manner as that of Steps 1-2-6 and 1-2-7. Specifically, the compounds can be prepared by allowing a regent such as 4-nitrophenyl chloroformate, 4-nitrophenyl carbonate, or 1,1'-carbonyldiimidazole to react on a compound of the formula (A-a) wherein $Z^a$ is an alkyl group substituted with hydroxyl group to convert the compound into an active ester, and successively reacting the active ester with an alkylamine, cycloalkylamine, or arylamine suitably corresponding to an target compound to be prepared (Steps 1-2-9).
For example, the compounds of the formula (A·a·1) mentioned above can be prepared from a compound of the following formula (A-c) :

[Formula 12]

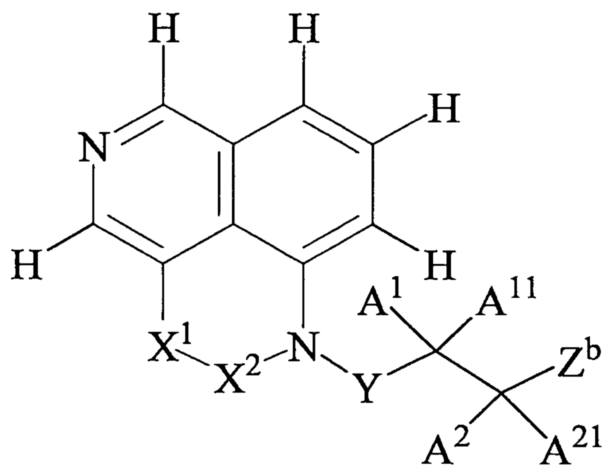

(A-c)

[wherein Y, $A^1$, $A^{11}$, $A^2$, $A^{21}$, $Z^b$, and $X^1$-$X^2$ have the same meanings as those defined above], which corresponds to a compound of the formula (A-a-1) wherein $R^6$, $R^7$, and $R^8$ are hydrogen atoms, for example, as follows.
First, the compounds represented by the following formula (A-g):

[Formula 13]

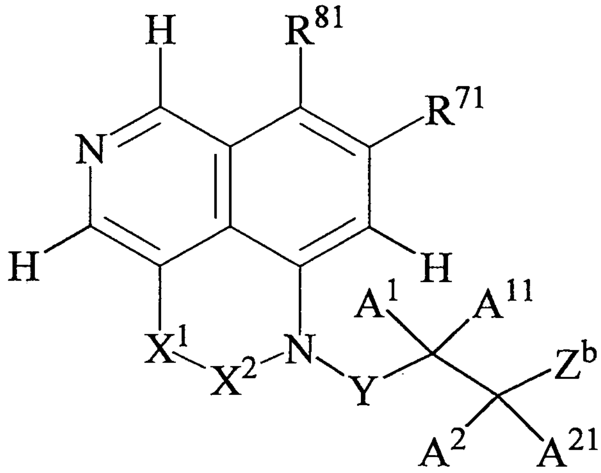

(A-g)

[wherein Y, $A^1$, $A^{11}$, $A^2$, $A^{21}$, $Z^b$, and $X^1$-$X^2$ have the same meanings as those defined above, $R^{71}$, and $R^{81}$ independently represent hydrogen atom, or a halogen atom (chlorine atom, bromine atom, iodine atom and the like)], which correspond to the compounds of the formula (A-a-1) wherein $R^6$ is hydrogen atom, $R^7$ is $R^{71}$, and $R^8$ is $R^{81}$), can be obtained by allowing a halogenation regent such as N-bromosuccinimide, N-chlorosuccinimide, and N-iodosuccinimide in an inert solvent such as N,N-dimethylformamide, N,N-dimethylacetamide, N-methylpyrrolidone, N,N'-dimethylimidazolidinone, and sulfolane (Step 1-3). Preferred examples of the inert solvent include N,N-dimethylformamide. Examples of the method include a method of using the halogenation regent in an amount of 1 fold mole or more, preferably 1 to 10 fold moles, based on the compound of the formula (A-c). Examples of the method include a method of performing the reaction at a reaction temperature of -10 to 120°C, preferably about 0 to 80°C.

Further, the compounds represented by the following formula (A-h):

[Formula 14]

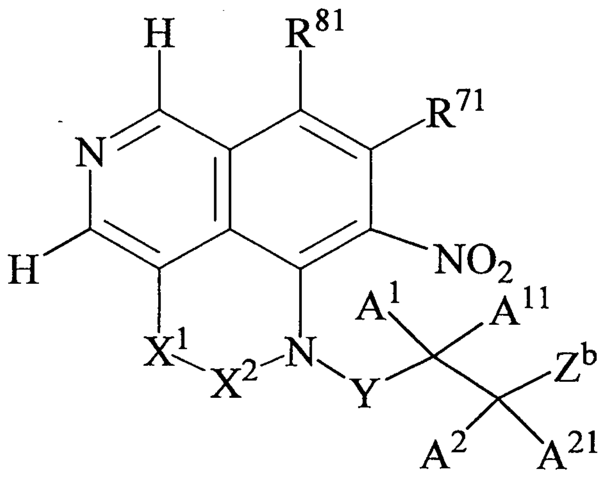

(A-h)

[wherein Y, $A^1$, $A^{11}$, $A^2$, $A^{21}$, $R^{71}$, $R^{81}$, $Z^b$, and $X^1$-$X^2$ have the same meanings as those defined above] can be obtained by nitrating the compound of the formula (A-g) obtained as described above (Step 1-4).

Further, the compounds represented by the following formula (A-i):

[Formula 15]

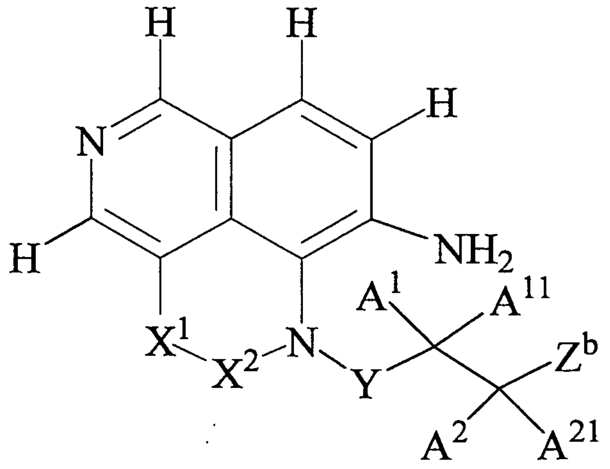

(A-i)

[wherein Y, $A^1$, $A^{11}$, $A^2$, $A^{21}$, $Z^b$, and $X^1$-$X^2$ have the same meanings as those defined above] can be obtained by reducing the nitro group of the compound of the formula (A-h) obtained as described above with hydrogen in a solvent, for example, an alcohol such as methanol and ethanol, an ether such as tetrahydrofuran and 1,4-dioxane, water, ethyl acetate, or the like, used independently, or in a solvent consisting of a suitable combination of these in the presence of a base such as triethylamine, N,N-diisopropylethylamine, and pyridine, and a platinum group catalyst such as palladium/carbon, palladium black, palladium hydroxide, platinum oxide, and platinum black (Step 1-5). Preferred examples of the method for this reaction include a method of performing the reaction in the presence of triethylamine in an amount of 1 to 3 fold moles and 1 to 50% by weight of palladium/carbon catalyst in a methanol solvent at a temperature of 10 to 80°C under a hydrogen atmosphere of ordinary pressure to 3 atmospheres. Steps 1-3 to 1-5 can be performed by referring to known literatures (for example, Kucznierz, R., Dickhaut, J, Leinert, H., Von Der Saal, W., Synth. Commun., 29, 1617 (1999); Ping Chen, Bioorganic & Medicinal Chemistry Letters, 13, 1345 (2003)).

Further, the compounds represented by the following formula (A-i) obtained as described above can be subjected to, for example, a known diazotization-Sandmeyer reaction (see, for example, Denny, William., et al, J. Med. Chem., 2002, 740; Kulka, J. Am. Chem. Soc., 75, 3597 (1953) and the like) to obtain the compounds represented by the following formula (A-j):

[Formula 16]

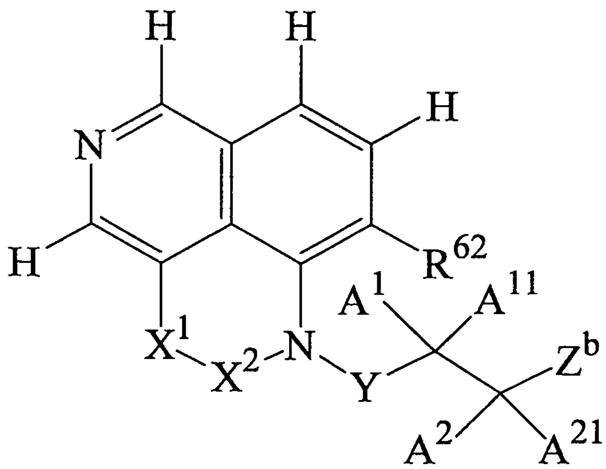

(A-j)

[wherein Y, $A^1$, $A^{11}$, $A^2$, $A^{21}$, $Z^b$, and $X^1$-$X^2$ have the same meanings as those defined above, and $R^{62}$ is a halogen atom].

Further, by subjecting the compounds of the formula (A-g), (A-j), or the like to the known Suzuki-Miyaura reaction using a palladium catalyst or a nickel catalyst (alkyl group, alkenyl group; for example, Kirchhoff, J.H., Netherton,

M.R., Hills, I.D., Fu, G.C., J. Am. Chem. Soc., 124, 13662 (2002) and the like), Negishi reaction (alkyl group; Dai, C., Fu, G.C., J. Am. Chem. Soc., 123, 2719 (2001) and the like), Stille reaction (alkenyl group; Carreno, M.C., Mahugo, R.H-S.J., J. Org. Chem., 64, 1387 (1999) and the like), Heck reaction (alkenyl group; for example, Selvakumar, K., Zapf, A., Beller, M., Org. Lett., 4, 3031 (2002); Raggon, J.W., Snyder, W.M., Organic Process Research & Development, 6, 67 (2002) and the like), Sonogashira reaction (alkynyl group and the like; Hundertmark, T., Littke, A.F., Buchwald, S.L., Fu, G.C., Org. Lett., 2, 1729 (2000) and the like), Buchwald reaction (amino group, alkylamino group, arylamino group, alkoxyl group and the like; for example, Klapars, A., Huang, X., Buchwald, S.L., J. Am. Chem. Soc., 124, 7421 (2002), Buchwald, S.L., Zim, D., Org. Lett., 5, 2413 (2003), Toracca, KE., Huang, X., Parrish, C.A., Buchwald, S.L., J. Am. Chem. Soc., 123, 10770 (2001) and the like), the compounds of the formula (A-a-1) mentioned above can be prepared ("coupling method using catalyst"). Hydroxyl group can also be prepared by dealkylating an alkoxyl group according to a known method.

The compounds of the formula (A-a-1) obtained above can be converted into the compounds represented by the formula (A-a) as described above. For example, the conversion can be performed by any of Steps 1-2-1, 1-2-2, 1-2-2-1, 1-2-3, 1-2-4, 1-2-5, 1-2-6, 1-2-7, 1-2-8, and 1-2-9, or an arbitrary combination of these.

(Preparation method 1-b)

The compounds of the formula (A-d) mentioned above can be prepared as follows. For example, the compounds of the formula (A-d) wherein $Z^a$ is -N($A^6$)($A^{62}$) can be prepared by deprotecting (corresponding to the deprotection of protective group of amino group in Step 1-1) a compound of the following formula (A-c-1):

[Formula 17].

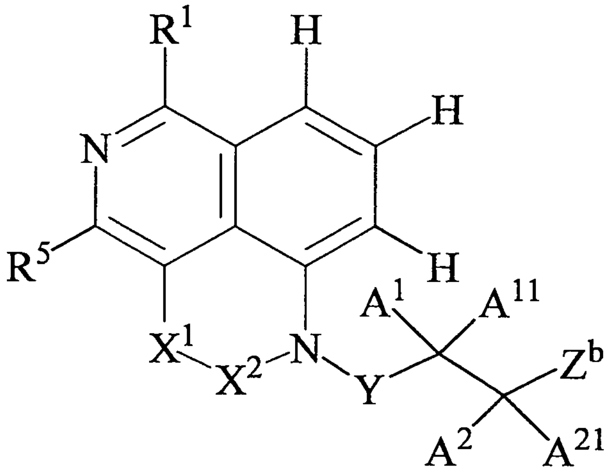

(A-c-1)

[wherein Y, $A^1$, $A^{11}$, $A^2$, $A^{21}$, $R^1$, $R^5$, $Z^b$, and $X^1$-$X^2$ have the same meanings as those defined above], which corresponds to a compound of the formula (A-d) wherein $Z^a$ is -N($A^6$)($PG^2$), to prepare a compound of the following formula (A-d-1):

[Formula 18]

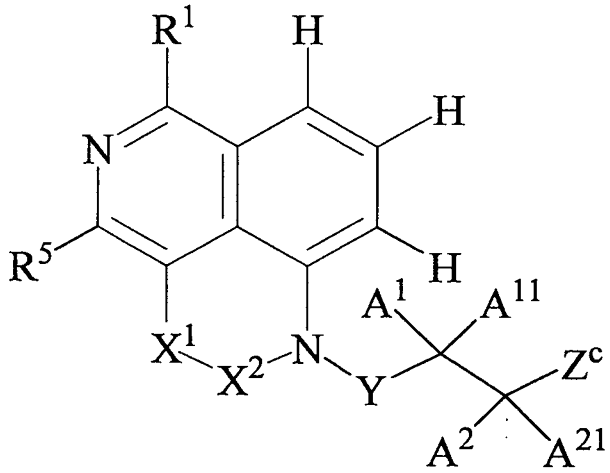

(A-d-1)

[wherein Y, $A^1$, $A^{11}$, $A^2$, $A^{21}$, $R^1$, $R^5$, $Z^c$, and $X^1$-$X^2$ have the same meanings as those defined above], which corresponds to a compound of the formula (A-d) wherein $Z^a$ is -NH($A^6$) (constituting a part of the compounds of formula (1) (Step 1-7-1), and further reacting this compound with a compound represented as $A^{62}$-W [wherein $A^{62}$ has the same meaning as defined above, and W represents a leaving group] (Step 1-7-2). A method similar to that of Step 1-2-1 can be used for Step 1-7-1, and a method similar to that of Step 1-2-2 can be used for Step 1-7-2.

Further, the compounds of the following formula (A-e):

[Formula 19]

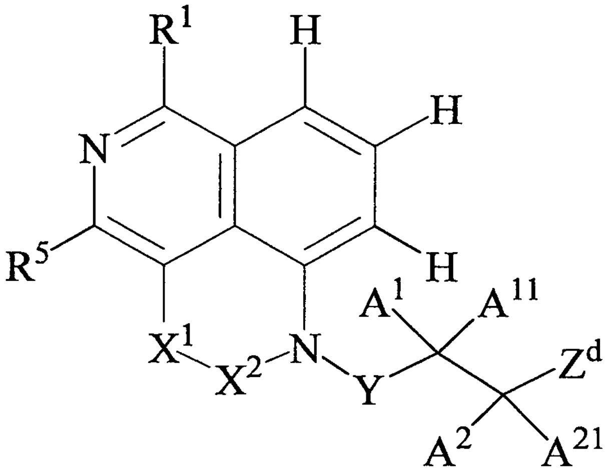

(A-e)

[wherein Y, $A^1$, $A^{11}$, $A^2$, $A^{21}$, $R^1$, $R^5$, $Z^d$, and $X^1$-$X^2$ have the same meanings as those defined above], which correspond to the compounds of the formula (A-d) wherein $Z^a$ in the formula (A-d) is -N($A^6$)($A^{63}$), can be prepared by, for example, reacting a compound of the formula (A-d-1) with a compound represented as $A^{63}$-W [wherein $A^{63}$ and W have the same meaning as defined above] (Step 1-7-2-1). This step can be performed in the same manner as that of Step 1-2-2-1 mentioned above.

Further, the compounds of the formula (A-d) wherein $Z^a$ is -N($A^6$)($A^{62}$), and $A^{62}$ is an alkyl group of which end is substituted with N($A^7$)(-$X^3$-$A^{71}$) can be prepared by, for example, the same methods as those of Steps 1-2-3 to 1-2-8 (Steps 1-7-3 to 1-7-8, respectively).

Further, the compounds of the formula (A-d) wherein $Z^a$ is -N($A^6$)($A^{62}$), and $A^{62}$ is an alkyl group of which end is substituted

with an alkylaminocarbonyloxy group, an alkyl group substituted with a cycloalkylaminocarbonyloxy group, or an alkyl group substituted with an arylaminocarbonyloxy group can be prepared from a compound of the formula (A-d) wherein $Z_a$ is an alkyl group substituted with hydroxyl group, for example, in the same manner as that of Step 1-2-9 mentioned above (Step 1-7-9).

The compounds represented by the formula (A-c-1) can be prepared by subjecting a compound represented by the following formula (A-k):

[Formula 20]

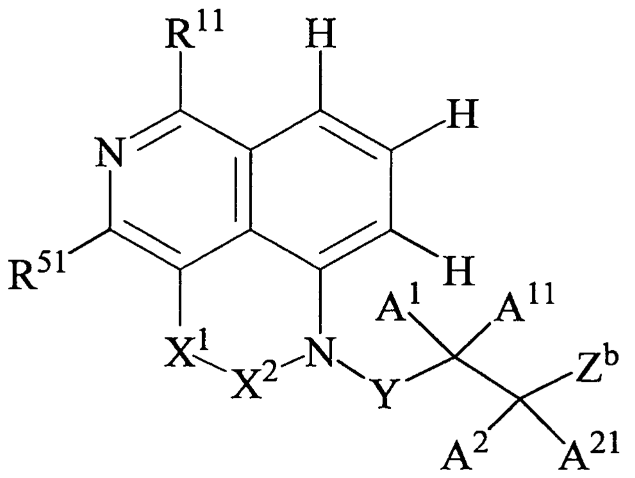

(A-k)

[wherein Y, $A^1$, $A^{11}$, $A^2$, $A^{21}$, $Z^b$, and $X^1$-$X^2$ have the same meanings as those defined above, and $R^{11}$, and $R^{51}$ are hydrogen atoms, or independently represent hydrogen atom, or a halogen atom] to the aforementioned "coupling method using catalyst".

Further, for example, the compounds of the formula (A-c-1) wherein $R^{11}$, and $R^{51}$ independently represent hydrogen atom, or hydroxyl group can also be prepared by hydrolyzing a compounds of the formula (A-k) wherein $R^{11}$, and $R^{51}$ independently represent hydrogen atom, or a halogen atom in a mineral acid (Step 1-8). Examples of the mineral acid include hydrochloric acid, sulfuric acid, nitric acid, and perchloric acid, and preferred examples include hydrochloric acid. The reaction temperature is, for example, -10 to 150°C, preferably 20 to 120°C.

The compounds of the formula (A-c) mentioned above constitute a part of the compounds of the formula (A-k), and in this case, $R^{11}$, and $R^{51}$ represent hydrogen atom.

Further, the compounds represented by the formula (A-k) can be prepared by cyclizing a compound represented by the following formula (B):

[Formula 21]

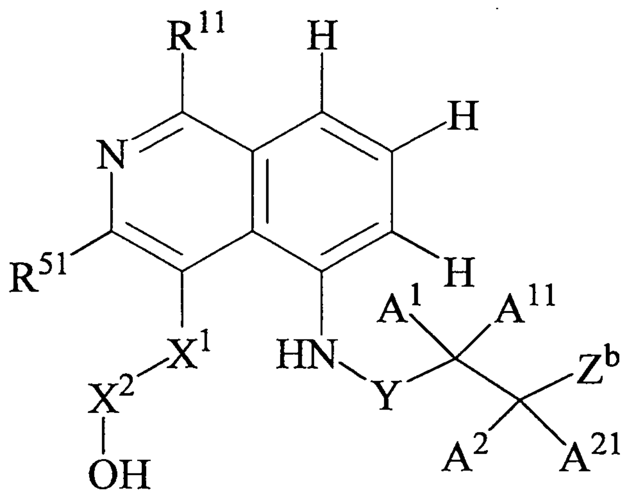

(B)

[wherein Y, $A^1$, $A^{11}$, $A^2$, $A^{21}$, $Z^b$, and $X^1$-$X^2$ have the same meanings as those defined above, amd $R^1$, and $R^5$ independently represent hydrogen atom, or a halogen atom] (Step 1-9).

Examples of the cyclization method include a method of performing the cyclization in the presence of a phosphorus reagent and an azo compound, and a method of reacting the compound with an alkylsulfonyl chloride, an arylsulfonyl chloride, an alkylsulfonic acid anhydride or an arylsulfonic acid anhydride in the presence of a base, and a preferred method is a method of performing the cyclization in the presence of a phosphorus reagent and an azo compound in an inert solvent (see, for example, Tsunoda et al., Chemistry Letters, 539 (1994); or Mitsunobu, 0., Synthesis, 1 (1981)). Examples of the inert solvent include, for example, tetrahydrofuran, toluene, and dichloromethane, and a preferred example is tetrahydrofuran. Examples of the phosphorus reagent include, for example, triphenylphosphine, and tri(n-butyl)phosphine. Example of the azo compound include, for example, diethyl azodicarboxylate, di(iso-propyl) azodicarboxylate, and 1,1'-azobis(N,N-dimethylformamide). Each of the phosphorus reagent and the azo compound may be the same or different, and is used in an amount of 1 fold mole or more, preferably 2 to 4 fold moles, based on the compound of the formula (B). The reaction temperature is, for example, -10°C or higher, preferably about 0 to 60°C.

The compounds represented by formula (A·k) mentioned above can be prepared by hydration of a compound represented by the following formula (C):

[Formula 22]

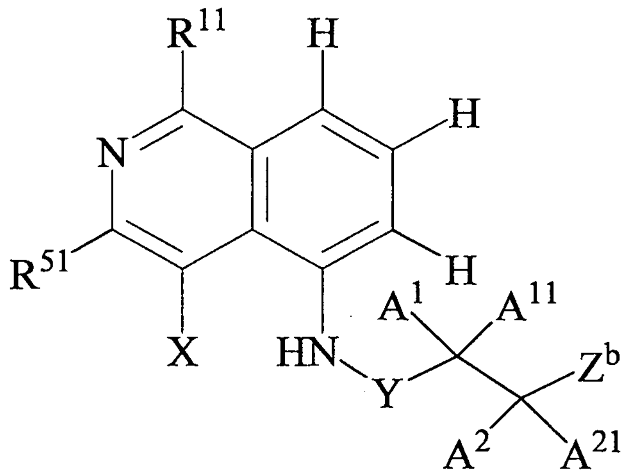

(C)

[wherein Y, $A^1$, $A^{11}$, $A^2$, $A^{21}$, $R^{11}$, $R^{51}$, and $Z^b$ have the same meanings as those defined above, and X represents -C($R^2$)=CH($R^3$), or -CH($R^2$)-C($R^3$)=CH($R^4$)] (Step 1-10).

For example, a compound of the formula (C) can be hydroborated with a boron reagent, then oxidized and

hydrolyzed to obtain the compound. As for the hydroboration, examples of the boron reagent include dicyclohexylborane, disyamyl borane, thexyl borane, catechol borane, 9-borabicyclo[3.3.1]nonane (9-BBN) dimer, 9-BBN monomer and the like, and 9-BBN dimer, and 9-BBN monomer are preferred. The boron reagent is preferably used in an amount of usually 1 fold mole or more, preferably about 2 to 5 fold moles. Examples of the solvent include ether type solvents such as tetrahydrofuran and 1,4-dioxane and the like, and tetrahydrofuran is preferred. The reaction temperature is 0°C to the boiling temperature of the solvent used, preferably 10 to 60)°C. The reaction time is 2 hours or more, preferably 10 to 20 hours. As for the subsequent oxidization and hydrolysis, examples of the oxidizing agent include 30% aqueous hydrogen peroxide, sodium peroxoborate, N-methylmorpholine N-oxide, triethylamine N-oxide and the like, and 30% aqueous hydrogen peroxide, and sodium peroxoborate are preferred. The oxidizing agent is used in an amount of, for example, 1 fold mole or more, preferably 2 to 20 fold moles. The reaction time is, for example, 0.25 to 10 hours, preferably 0.5 to 4 hours. Then, the hydrolysis is performed under an alkaline condition, and examples of the alkali include aqueous sodium hydroxide, aqueous potassium hydroxide and the like. The alkali is used in an amount of, for example, usually 2 to 100 fold moles, preferably 3 to 20 fold moles, and the reaction time is, for example, 2 hours or more, preferably 2 to 4 hours.

The compounds represented by the formula (C) can be prepared from a compound represented by the following formula (D):

[Formula 23]

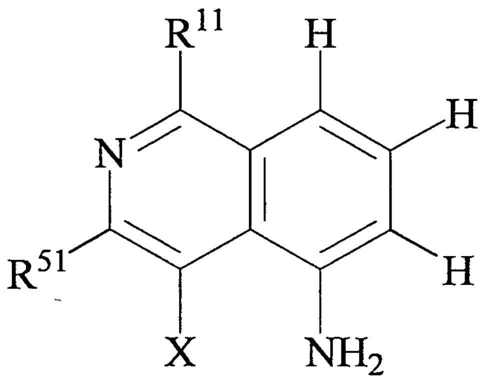

(D)

[wherein $R^{11}$, $R^{51}$, and X have the same meanings as those defined above], and a carbonyl compound represented by the following formula (E):

[Formula 24]

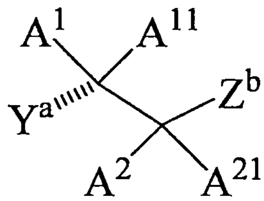

(E)

[wherein the bond represented by the broken line represents a single bond, or a double bond;
when the bond represented by the broken line is a single bond, $Y^a$ represents $-C(A^3)=O$, $-C(A^4)(A^{41})-C(A^3)=O$, or $-C(A^5)(A^{51})-C(A^4)(A^{41})-C(A^3)=O$,
$A^{11}$, $A^{21}$, $A^{41}$, and $A^{51}$ have the same meanings as those defined above;
when the bond represented by the broken line is a double bond, $Y^a$ is oxygen atom, $A^{11}$ is hydrogen atom, $A^{21}$, $A^{41}$, and $A^{51}$ have the same meanings as those defined above; and
$A^1$, $A^2$, $Z^a$, $A^3$, $A^4$, and $A^5$ have the same meanings as those defined above, provided that when any of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded, which carbonyl compound is commercially available, or can be prepared (Step

1-11).

Specifically, the compounds of the formula (C) can be prepared by allowing a reducing agent to react on a compound of the formula (D) and a compound of the formula (E) in a solvent, for example, a halogenated hydrocarbon such as dichloromethane, 1,2-dichloroethane, and chloroform, an ether such as 1,4-dioxane and tetrahydrofuran, an alcohol such as methanol, ethanol, and isopropanol, benzene, toluene, N,N-dimethylformamide, N,N-dimethylacetamide, N-methylpyrrolidone, acetic acid and the like independently used, or a mixed solvent consisting of a suitable combination of these. Examples of the reducing agent include a metal hydride reducing agent such as sodium borohydride, potassium borohydride, lithium borohydride, zinc borohydride, sodium cyanoborohydride, sodium triacetoxyborohydride, borane/tetrahydrofuran complex, borane/pyridine complex, borane/triethylamine complex, borane/dimethyl sulfide complex, and lithium triethylborohydride, and a preferred example is sodium triacetoxyborohydride. Based on the compound of the formula (D), the reducing agent is used in an amount of, for example, 0.5 fold mole or more, preferably 1 to 20 fold moles. The reaction temperature is, for example, 0°C or higher, preferably 10 to 80°C. The reaction time is, for example, 0.1 hour or more, preferably 0.3 to 48 hours.

Preferred examples of the method include a method of performing the reduction by a known method under a hydrogen gas atmosphere in the presence of a platinum group catalyst, such as palladium/carbon, palladium hydroxide, platinum oxide, and platinum black.

The compounds of the formula (D) can be prepared from i) a compound represented by the following formula (F-a):

[Formula 25]

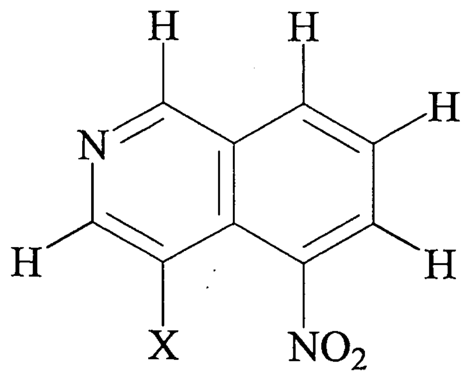

(F-a)

[wherein X has the same meanings as that defined above], or ii) a compound represented by the following formula (F-b):

[Formula 26]

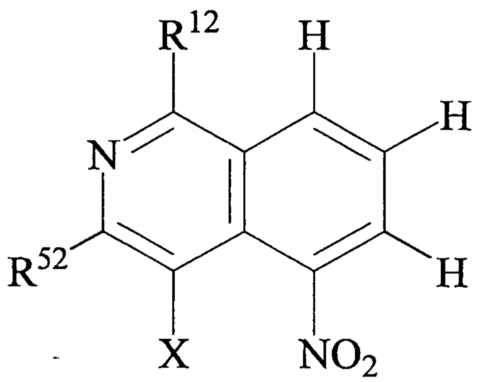

(F-b)

[wherein $R^{12}$, and $R^{52}$ independently represent hydrogen atom, or a halogen atom (chlorine atom, bromine atom, iodine atom and the like, preferably chlorine atom), and X has the same meaning as that defined above].

For the preparation of the compounds of the formula (D) from a compound of the formula (F-a) (Step 1-12-1), and the preparation of the compounds of the formula (D) from a compound of the formula (F-b) (Step 1-12-2), a known

reduction reaction of nitro group can be used. This reduction is preferably carried out in an inert solvent. Examples of the inert solvent include alcohols, ethers, and esters, preferred examples include esters, and especially preferred examples include ethyl acetate. Examples of the reduction reagent include tin (divalent) reagents. Preferred examples of the tin (divalent) reagents include stannous chloride, and hydrate thereof. The reaction temperature is, for example, -20°C or higher, preferably 10 to 50°C. The reaction time is, for example, 2 hours or more, preferably 4 to 15 hours.

The compounds of the formula (F-b) can be prepared by allowing a halogenation regent to react on a compound of the following formula (F-c):

[Formula 27]

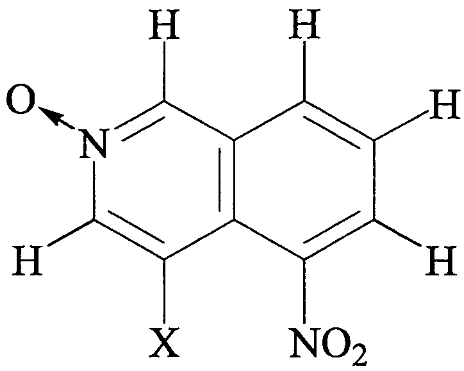

(F-c)

[wherein X has the same meaning as that defined above] for halogenation (Step 1-13). Examples of the halogenation reagent include phosphorous oxychloride, phosphorus trichloride, phosphorus pentachloride, phosphorus oxybromide, phosphorus tribromide, phosphorus pentabromide and the like, and preferred examples include phosphorous oxychloride. The halogenation reagent is preferably used in an amount of 0.1 fold mole or more, particularly preferably 1 to 10 fold moles, based on the compound of the formula (F-c). As for the solvent, examples of the method include a method of performing the reaction without solvent, or in an inert solvent, and preferred examples of the method include a method of performing the reaction without solvent, or using dichloromethane, 1,2-dichloroethane, chloroform, or toluene as the solvent. The reaction is preferably performed at room temperature or a higher temperature.

The compounds of the formula (F-c) can be prepared by oxidizing a compound of the formula (F-a) (Step 1-14). Examples of oxidizing agent include aqueous hydrogen peroxide, sodium periodate, sodium perborate, 3-chloroperbenzoic acid, ruthenium trichloride, and dimethyldioxirane, and preferred examples include 3-chloroperbenzoic acid. The oxidizing agent is preferably used in an amount of 0.1 fold mole or more, particularly preferably 1 to 20 fold moles, based on the compound of the formula (F-a). Examples of the solvent include acetic acid, trifluoroacetic acid, dichloromethane, 1,2-dichloroethane, chloroform, acetonitrile, acetone, trichlorofluoromethane, benzene, 1,4-dioxane, tert-butanol, water, and mixed solvents of these, and preferred examples include chloroform.

Further, the compounds represented of the formula (F-a) can be prepared by reacting known 4-bromo-5-nitroisoquinoline (Reference Example 1) with a tin compound represented by the following formula (G):

[Formula 28]. $X\text{-}SnBu_3$ (G)

[wherein X has the same meaning as defined above, and Bu represents n-butyl] (Step 1-15). Examples of the tin compound represented by the formula (G) include those commercially available and those known from literatures (see, for example, Seyferth et al., Chem. Ind., 402 (1959); J. Amer. Chem. Soc., 361 (1962); and J. Amer. Chem. Soc., 515 (1957)). The amount of this tin compound is, for example, 1 fold mole or more, preferably 1 to 3 fold moles, based on the compound of the formula (E).

As for the preparation of the compounds of the formula (F-a) by the coupling reaction of 4-bromo-5-nitroisoquinoline, and a compound of the formula (G), preferable examples include, for example, the following two kinds of reaction conditions.

The first reaction condition corresponds to a method of performing the reaction in toluene or an ether type solvent in the presence of tetrakis(triphenylphosphine)palladium(0) as a catalyst, and 2,6-di(tert-butyl)-4-cresol (BHT) as a polymerization inhibitor. Based on 4-bromo-5-nitroisoquinoline, tetrakis(triphenylphosphine)palladium(0) is used in an amount

of, for example, 0.001 fold mole or more, preferably 0.01 to 0.2 fold mole, and BHT is used in an amount of, for example, 0.001 fold mole or more, preferably 0.005 to 0.01 fold mole. As the solvent, toluene or 1,4-dioxane is preferred, and the reaction temperature is, for example, 10°C or higher, preferably 80 to 120°C.
The second reaction condition corresponds to a method of performing the reaction in an ether type solvent in the presence of a palladium compound such as tetrakis(triphenylphosphine)palladium(O), palladium(II) acetate, or tris(dibenzylideneacetone)dipalladium(O), or a phosphorus compound such as triphenylphosphine or tri(tert-butyl)phosphine, and cesium fluoride as an additive. As the palladium compound, tris(dibenzylideneacetone)dipalladium(0) is preferred, and tri (tert-butyl)phosphine is preferred as the phosphorus compound. The solvent is preferably 1,4-dioxane. Based on 4-bromo-5-nitroisoquinoline, the palladium compound is used in an amount of, for example, 0.001 fold mole or more, preferably 0.01 to 0.2 fold mole, and the phosphorus compound is preferably used in an amount of about 4 fold moles. Cesium fluoride is preferably used in an amount of about 1 to 3 fold moles based on the tin compound of the formula (G). The reaction temperature is, for example, 10°C or higher, preferably 60 to 100°C. As for these reactions, Gregory, C, Fu et al., Angew. Chem. Int. Ed., 2411 (1999) can be referred to.

The compounds of the following formula (D-a):

[Formula 29]

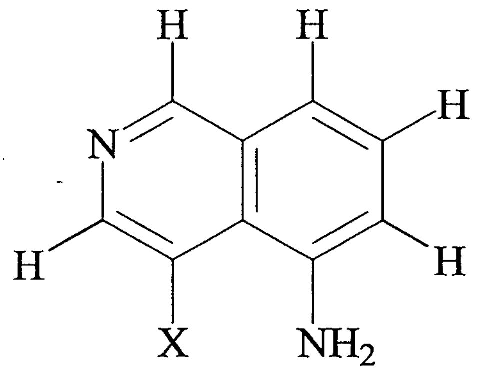

(D-a)

[wherein X has the same meaning as that defined above], which correspond to the compounds represented by the formula (D) wherein $R^{11}$, and $R^{51}$ are both hydrogen atoms, can also be parepared from 5-amino-4-bromoisoquinoline (Reference Example 2), and a compound of formula (G) mentioned above in the same manner as that of Step 1-15. The compounds of formula (D-a) can also be derived into compounds of the formula (D) by successively performing methods similar to those of Step 1-14, and Step 1-13.
The compounds of the formula (A) wherein $Z^a$ is -N($A^6$)($A^{62}$), and $A^{62}$ is carbamimidoyl group can be prepared by, for example, guanidylation of a compound of the formula (A) wherein $Z^a$ is -NH($A^6$) according to a known method (see, for example, Drake Brian, Patek Marcel, Lebl Michael, Synthesis, 6, 579 (1994) and the like) (Step 1-16).
Further, the compounds of the formula (A) wherein $Z^a$ is -N($A^6$)($A^{62}$), and $A^{62}$ is an alkylcarbonyl group, or an arylcarbonyl group can be prepared by, for example, reacting a compound of the formula (A) wherein $Z^a$ is -NH($A^6$) with an acylation regent suitably corresponding to an target compound to be prepared in the same manner as that of Step 1-2-3 (Step 1-17).

(Preparation Method 2)

The compounds represented by the following formula (1-a):

[Formula 30]

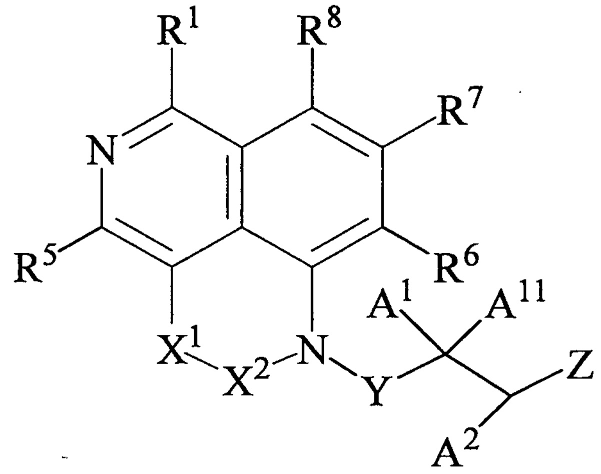

(1-a)

[wherein $R^1$, $R^5$, $R^6$, $R^7$, $R^8$, $A^{11}$, and $X^1$-$X^2$ have the same meanings as those defined above; and Y, $A^1$, $A^2$, and Z have the same meanings as those defined above, provided that when any of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded], which correspond to the compounds of the formula (1) wherein $A^{21}$ is hydrogen atom, can be prepared by deprotection of a protective group of a compound represented by the following formula (A-m):

[Formula 31]

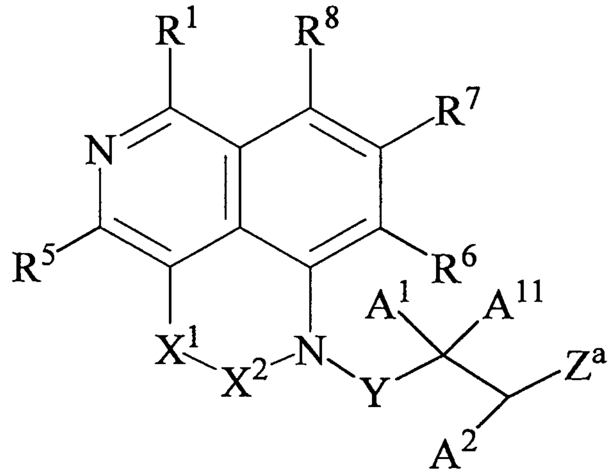

(A-m)

[wherein $R^1$, $R^5$, $R^6$, $R^7$, $R^8$, $A^{11}$, and $X^1$-$X^2$ have the same meanings as those defined above; and Y, $A^1$, $A^2$, and $Z^a$ have the same meanings as those defined above, provided that when any of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded], which correspond to the compounds of the formula (A-1) wherein $A^{21}$ is hydrogen atom, if the protective group is present, in the same manner as that of Step 1-1 (Step 2-1). When Z and $Z^a$ in the formula (1-a) represent the same group, the compounds of the formula (A-m) constitute a part of the compounds of the formula (1-a), and Step 2-1 mentioned above is unnecessary.

The compounds represented by the formula (A-m) can be prepared from a compound represented by the following formula (H):

[Formula 32]

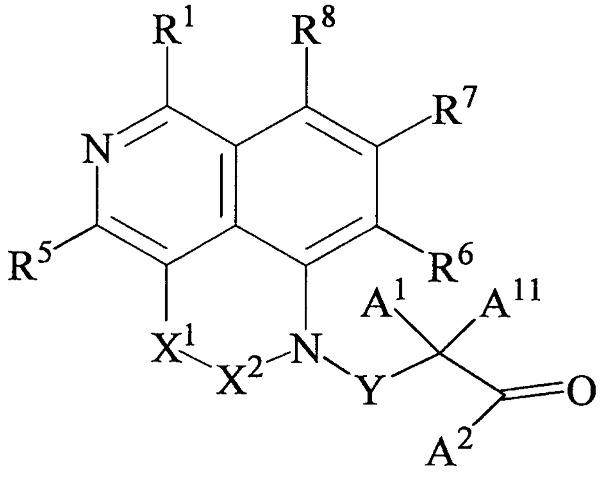

(H)

[wherein $R^1$, $R^5$, $R^6$, $R^7$, $R^8$, $A^{11}$, and $X^1$-$X^2$ have the same meanings as those defined above; and Y, $A^1$, $A^2$, and $Z^a$ have the same meanings as those defined above, provided that when any of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded].

First, when the compounds of the formula (A-m) are prepared, examples of the method include a method of allowing a reducing agent to react on a compound of the formula (H) in a solvent (Step 2-2-1). Examples of the reducing agent include a metal hydride reducing agent such as sodium borohydride, zinc borohydride, borane/tetrahydrofuran complex, borane/pyridine complex, borane/triethylamine complex, borane/dimethyl sulfide complex, and lithium triethylboride, and preferred examples include sodium borohydride. Based on the compound of the formula (H), sodium borohydride is used in an amount of, for example, 0.5 fold mole or more, preferably 1 to 20 fold moles. Examples of the solvent include alcohols such as methanol, ethanol, and isopropanol, ethers such as tetrahydrofuran, 1,2- dimethoxy ethane, and 1,4-dioxane, dichloromethane, and N,N-dimethylformamide, and preferred examples include methanol, and ethanol. The reaction temperature is, for example, 0°C or higher, preferably 10°C to the reflux temperature of the solvent. The reaction time is, for example, 0.1 hour or more, preferably 0.5 to 12 hours.

Further, the compounds of the formula (A-m) wherein $Z^a$ is -NH($A^6$), -N($A^6$)($A^{62}$), or -N($A^6$)($A^{63}$) [wherein $A^6$, $A^{62}$, and $A^{63}$ have the same meanings as those defined above, provided that a compound wherein $A^{62}$ is carbamimidoyl group, an alkylcarbonyl group, or an arylcarbonyl group is excluded] can be prepared by reductively reacting a compound represented by the formula N($A^6$)$H_2$, NH($A^6$)($A^{62}$), or NH($A^6$)($A^{63}$) with a compound of the formula (H) for amination (Step 2-2-2). The reductive amination reaction can be performed by referring to a known method. Examples of the method include, for example, a method of allowing a compound of the formula NH($A^6$)($A^{61}$) mentioned above in an amount of 1 fold mole or more, preferably 1 to 10 fold moles, based on the compound of the formula (H), to react on the compound of the formula (H) in a 1,2-dichloroethane solvent in the presence of a reducing agent such as sodium triacetoxyborohydride in an amount of 0.5 fold mole or more, preferably 1 to 10 fold mole, based on the compound of the formula (H) at a temperature of 0°C or higher, preferably room temperature to 60°C. A method of performing the reaction by using acetic acid as a solvent or in an amount of about 0.1 to 10 fold moles is also preferred. Further, preferred examples also include a method of performing the reaction in the presence of a dehydrating agent such as anhydrous sodium sulfate, anhydrous magnesium sulfate, and molecular sieves.

Further, the compounds of the formula (A-m) wherein $Z^a$ is -N($A^6$)($A^{62}$), and $A^{62}$ is an alkyl group of which end is substituted with N($A^7$)(-$X^3$-$A^{71}$) [wherein -$X^3$- represents carbonyl group, -$SO_2$ -C(=O)-O-, -C(=O)-N($A^8$)-, or -C(=S)-N($A^8$)-, and $A^7$, $A^{71}$, and $A^8$ have the same meanings as those defined above] can be prepared from the compound of the formula (A-m) wherein $Z^a$ is -N($A^6$)($A^{63}$) obtained above in the same manner as that of Preparation method 1, Steps 1-2-3 to 1-2-8 (Steps 2-2-3 to 2-2-8, respectively). Further, it is also preferable to prepare the compounds by directly reacting a compound of N($A^6$)($A^{62}$) wherein $A^{62}$ is an alkyl group of which end is substituted with N($A^7$)(-$X^3$-$A^{71}$) in the reductive amination of the compound of the formula (H) and NH($A^6$)($A^{62}$).

Further, the compounds of the formula (A-m) wherein $Z^a$ is -N($A^6$)($A^{62}$), and $A^{62}$ is an alkyl group of which end is substituted with an alkylaminocarbonyloxy group, an alkyl group substituted with a cycloalkylaminocarbonyloxy group, or an alkyl group substituted with an arylaminocarbonyloxy group can be prepared in the same manner as that of Step 2-2-2 mentioned above by reductively reacting a compound of the formula (H) with NH($A^6$)($A^{62}$) wherein $A^{62}$ is an alkyl

group substituted with hydroxyl group for amination (Step 2-2-9), subsequently converting the aminated compound into an active ester in the same manner as that of Step 1-2-9 mentioned above, and then reacting the active ester with an alkylamine, cycloalkylamine, or arylamine suitably corresponding to an target compound to be prepared (Step 2-2-10). Further, it is also preferable to attain the preparation by directly reacting a compound of in $NH(A^6)(A^{62})$ wherein $A^{62}$ is an alkyl group of which end is substituted with an alkylaminocarbonyloxy group, an alkyl group substituted with a cycloalkylaminocarbonyloxy group, or an alkyl group substituted with an arylaminocarbonyloxy group in the reductive amination of the compound of the formula (H) mentioned above.
Further, the compounds of the formula (A-m) wherein $Z^a$ is $-N(A^6)(A^{62})$, and $A^{62}$ is carbamimidoyl group can be prepared from a compound of the formula (A-m) wherein $Z^a$ is $-NH(A^6)$ in the same manner as that of Step 1-16 (Step 2-3).
The compounds of the formula (A-m) wherein $Z^a$ is $-N(A^6)(A^{62})$, and $A^{62}$ is an alkylcarbonyl group, or an arylcarbonyl group can be prepared from a compound of the formula (A-m) wherein $Z^a$ is $-NH(A^6)$ in the same manner as that of Step 1-2-3 (Step 2-4).

The compounds represented by the following formula (1-b):

[Formula 33]

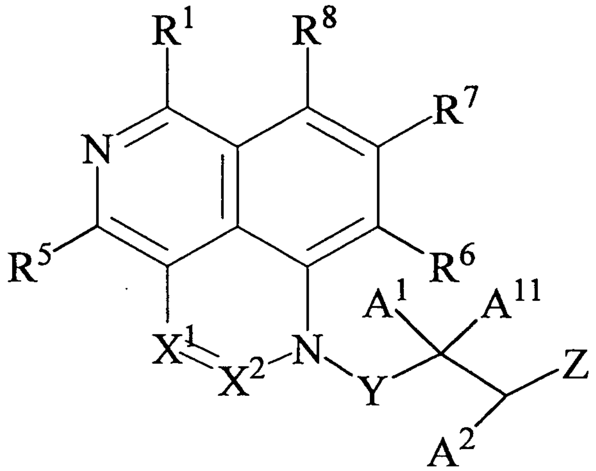

(1-b)

[wherein $R^1$, $R^5$, $R^6$, $R^7$, $R^8$, $X^1$=$X^2$, All, and Z have the same meanings as those defined above; and Y, $A^1$, and $A^2$ have the same meanings as those defined above, provided that when any of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded] can be prepared from a compound of the formula (1-a). Specifically, the compounds of the formula (1-b) can be prepared by dehydrogenating a compound of the formula (1-a) in the same manner as that of Step 1-1-1 (Step 2-5).

The compounds of the formula (H) can be prepared form a compound represented by the following formula (J):

[Formula 34]

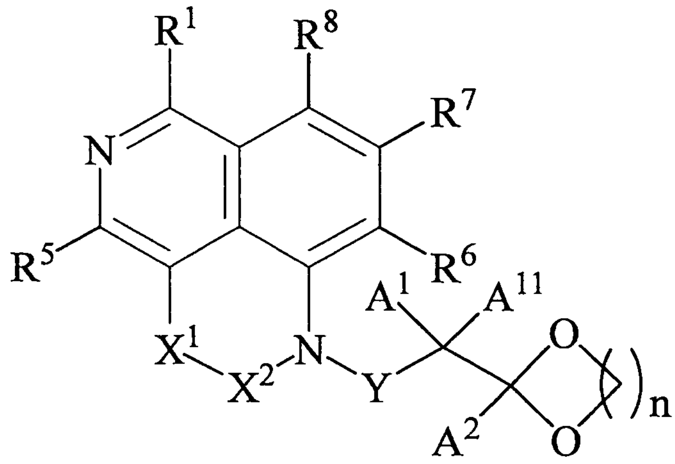

(J)

[wherein n represents 2 or 3;
$R^1$, $R^5$, $R^6$, $R^7$, $R^8$, $A^{11}$, and $X^1$-$X^2$ have the same meanings as those defined above; and Y, $A^1$, and $A^2$ have the same meanings as those defined above, provided that when any of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded] (Step 2-6). This step is a method of performing the reaction in a solvent in the presence of an acid catalyst. Examples of the solvent include alcohols such as methanol, ethanol, tert-butanol, and ethylene glycol, ethers such as tetrahydrofuran, 1,2-dimethoxyethane, and 1,4-dioxane, nitromethane, dimethyl sulfoxide, N,N-dimethylformamide, N,N-dimethylacetamide, 1-methylpyrolidone, sulfolane, acetic acid, and water, and methanol, ethanol, tert-butanol, tetrahydrofuran, and 1,4-dioxane are preferred. Examples of the acid include mineral acids such as hydrochloric acid, sulfuric acid, and nitric acid, methanesulfonic acid, p-toluenesulfonic acid, trifluoromethanesulfonic acid, trifluoroacetic acid, perchloric acid and the like, and hydrochloric acid, and perchloric acid are preferred. The reaction temperature is, for example, 0°C or higher, preferably 10 to 120°C. The reaction time is, for example, 0.1 hour or more, preferably 0.5 to 12 hours.

The compounds of the formula (J) mentioned above can be classified depending on the types of $R^1$, $R^5$, $R^6$, $R^7$, and $R^8$ into the following two types of compounds, Specifically, i) compounds represented by the following formula (J-a):

[Formula 35]

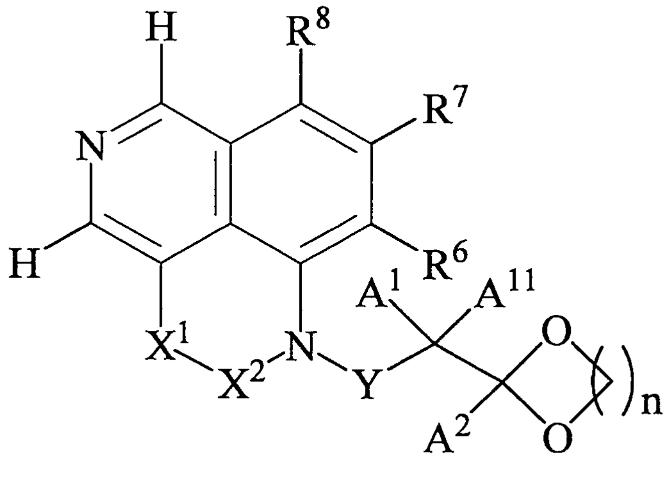

(J-a)

[wherein n, $A^{11}$, $R^6$, $R^7$, and $R^8$ have the same meanings as those defined above; and
Y, $A^1$, and $A^2$ have the same meanings as those defined above, provided that when any of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded], which correspond to the compounds of the formula (J) wherein $R^1$, and $R^5$ are hydrogen

atoms, and ii) compounds represented by the following formula (J-b):

[Formula 36]

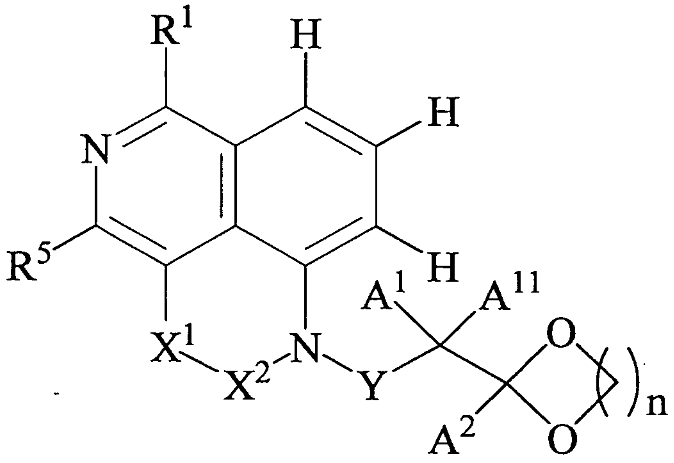

(J-b)

[wherein n, $A^{11}$, $R^1$, and $R^5$ have the same meanings as those defined above; and Y, $A^1$, and $A^2$ have the same meanings as those defined above, provided that when any of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded], which correspond to the compounds of the formula (J) wherein $R^6$, $R^7$, and $R^8$ are hydrogen atoms.

(Preparation method 2-a)

The compounds of the formula (J-a) can be prepared as follows. Specifically, the compounds can be prepared from a compound represented by the following formula (J-c):

[Formula 37]

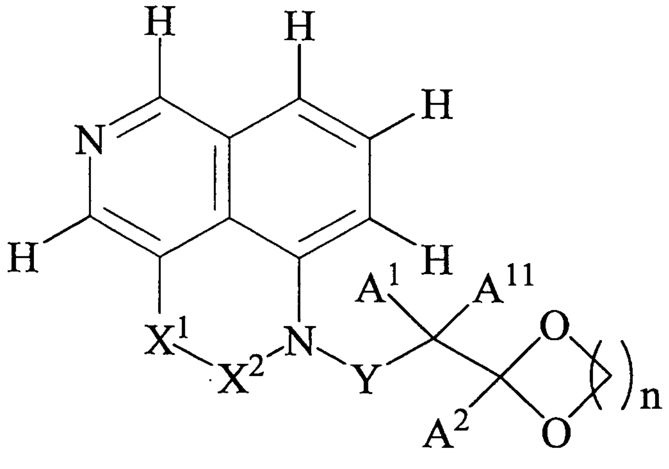

(J-c)

[wherein n, $A^{11}$, and $X^1$-$X^2$ have the same meanings as those defined above; and Y, $A^1$, and $A^2$ have the same meanings as those defined above, provided that when any of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded], which correspond to the compounds of the formula (J) wherein $R^1$, $R^5$, $R^6$, $R^7$, and $R^8$ are hydrogen atoms, by a combination of Steps 1-3, 1-4, 1-5, 1-6 of Preparation method 1, the aforementioned "coupling method using catalyst" and the like.

(Preparation method 2-b)

The compounds of the formula (J-b) mentioned above can be prepared as follows. Specifically, the compounds can be prepared from a compound of the following formula (J-d):

[Formula 38]

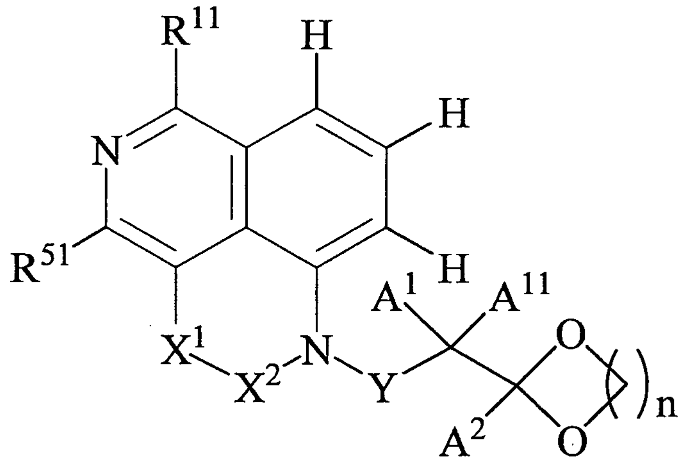

(J-d)

[wherein $R^{11}$, and $R^{51}$ independently represent hydrogen atom, or a halogen atom, n, $A^{11}$, and $X^1$-$X^2$ have the same meanings as those defined above; and

Y, $A^1$, and $A^2$ have the same meanings as those defined above, provided that when any of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded], which correspond to the compounds of the formula (J) wherein $R^1$, and $R^5$ independently represent hydrogen atom, or a halogen atom, and $R^6$, $R^7$, and $R^8$ are hydrogen atoms, according to Step 1-8 of Preparation method 1, or by further combining the aforementioned "coupling method using catalyst" and the like.

The compound of the formula (J-c) constitute a part of the compounds of the formula (A-d), in which $R^{11}$, and $R^{51}$ represent hydrogen atom.

The compounds of the formula (J-d) can be prepared by cyclizing a compound represented by the following formula (K) :

[Formula 39]

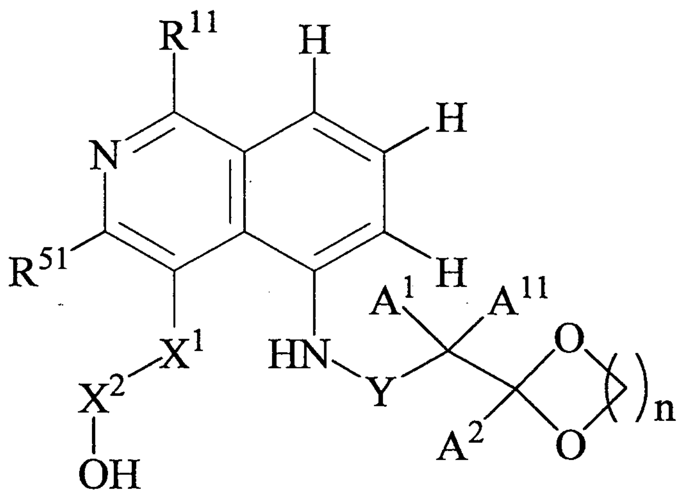

(K)

[wherein n, $R^{11}$, $R^{51}$, $A^{11}$, and $X^1$-$X^2$ have the same meanings as those defined above; and

Y, $A^1$, and $A^2$ have the same meanings as those defined above, provided that when any of combinations of $A^6$ and $A^3$,

$A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded] in the same manner as that of Step 1-9 mentioned above (Step 2-7).

Further, the compounds of the formula (K) can be prepared by hydration of a compound of the following formula (L):

[Formula 40]

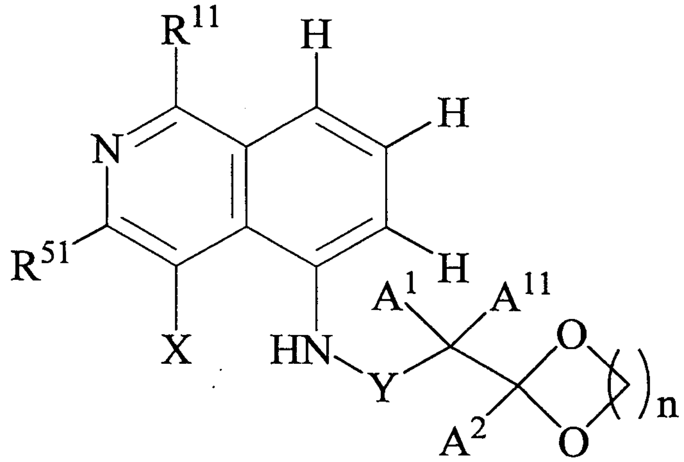

(L)

[wherein X represents $-C(R^2)=CH(R^3)$, or $-CH(R^2)-C(R^3)=CH(R^4)$, n, $R^{11}$, $R^{51}$, and $A^{11}$ have the same meanings as those defined above; and Y, $A^1$, and $A^2$ have the same meanings as those defined above, provided that when any of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded] in the same manner as that of Step 1-10 (Step 2-8).

Further, the compounds of formula (L) can be prepared by performing reductive amination using a compound represented by the formula (D), and a compound represented by the following formula (N):

[Formula 41]

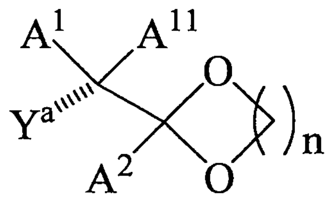

(N)

[wherein n, and $A^{11}$ have the same meanings as those defined above; and $Y^a$, $A^1$, and $A^2$ have the same meanings as those defined above, provided that when any of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded], which is commercially available, or can be prepared, instead of the compound of the formula (E), in the same manner as that of Step 1-11 (Step 2-9).

(Preparation method 3)

The compounds represented by the following formula (A-k-1):

[Formula 42]

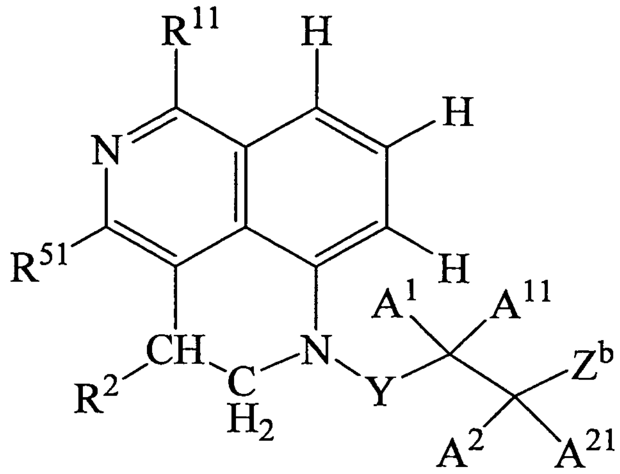

(A-k-1)

[wherein $R^{11}$, $R^{51}$, $R^2$, $A^{11}$, and $A^{21}$ have the same meanings as those defined above; and
Y, $A^1$, $A^2$, and $Z^b$ have the same meanings as those defined above, provided that when any of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded], which correspond to the compounds of the formula (A-k) wherein $X^1$-$X^2$ is $-CH(R^2)-CH_2-$, constitute a part of the compounds of the formula (A-k), and can be prepared as described in Preparation method 1. However, they can also be prepared as follows, an alternative method.

Specifically, they can be prepared by directly cyclizing a compound represented by the following formula (C-a):

[Formula 43]

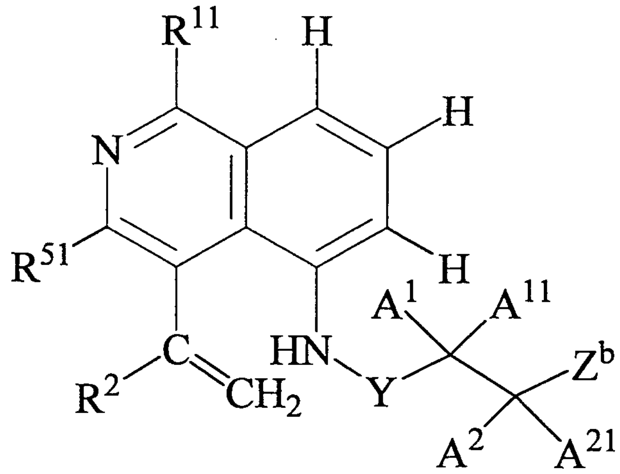

(C-a)

[wherein $R^{11}$, $R^{51}$, $R^2$, $A^{11}$, and $A^{21}$ have the same meanings as those defined above; and
Y, $A^1$, $A^2$, and $Z^b$ have the same meanings as those defined above, provided that when any of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded] (Step 3-1).

Examples of the method for the cyclization include a method of allowing a base to react on the compound in an inert solvent. Examples of the inert solvent include ether type solvents such as tetrahydrofuran, 1,2-dimethoxyethane, and 1,4-dioxane, benzene, toluene, dimethyl sulfoxide, N,N-dimethylformamide, 1-methylpyrolidone, and sulfolane, and tetrahydrofuran, and 1,4-dioxane are preferred. Examples of the base include alkali metals such as sodium and potassium, alkali metal hydrides such as sodium hydride, and potassium hydride, alkali metal alkoxides such as sodium methoxide, potassium methoxide, sodium ethoxide, sodium isopropoxide, sodium tert-butoxide, and potassium tert-butoxide, organic metal bases such as methyl lithium, n-butyl lithium, phenyl lithium, tert-butyl lithium, lithium diisopropylamide, sodium bis(trimethylsilyl)amide, potassium bis(trimethylsilyl)amide, and lithium 2,2,6,6-tetramethylpiperizide and the like, potassium, potassium hydride, potassium tert-butoxide, and potassium bis(trimethylsilyl)amide are preferred, and potassium

tert-butoxide is particularly preferred.
The amount of the base used is, for example, 0.01 fold mole or more, preferably 0.1 to 5 fold moles, based on the compound of the formula (C-a). The reaction temperature is, for example, 0°C or higher, preferably 10 to 120°C. The reaction time is, for example, 0.001 hour or more, preferably 0.01 to 5 hours.

The compounds represented by the formula (C-a) constitute a part of the compounds represented by the formula (C) mentioned above, and the method for the preparation from 4-bromo-5-nitroisoquinoline is the same as that described above (Step 1-15), provided that a tin compound the following formula (G-a):

[Formula 44]

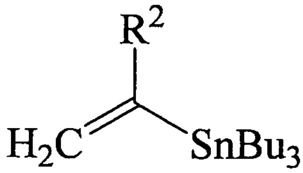

(G-a)

[wherein $R^2$, and Bu have the same meanings as those defined above], which constitutes a part of the tin compounds represented by the formula (G), can be used in the preparation of the compounds of the formula (F-a), or (F-b) from 4-bromo-5-nitroisoquinoline.
The compounds of the formula (A-k-1) constitute a part of the compounds of the formula (A-k) as described above, and therefore they can be derived into the compounds of the formula (1) wherein $X^1 \cdots X^2$ is $-CH(R^2)-CH_2-$ by such preparation steps starting from a compound of the formula (A-k) as described in Preparation method 1.

(Preparation method 4)

The compounds represented by the following formula (J-d-1):

[Formula 45]

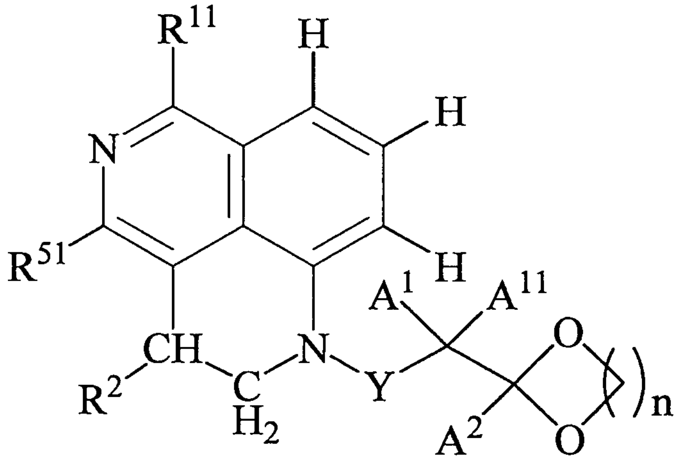

(J-d-1)

[wherein n, $R^2$, $R^{11}$, $R^{51}$, and $A^{11}$ have the same meanings as those defined above; and
Y, $A^1$, and $A^2$ have the same meanings as those defined above, provided that when any of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded] constitute a part of the compounds of the formula (J-d), and can be prepared as described in Preparation method 2. However, they can also be prepared as follows, an alternative method.

Specifically, they can be prepared by cyclizing a compound represented by the following formula (L-a) :

[Formula 46]

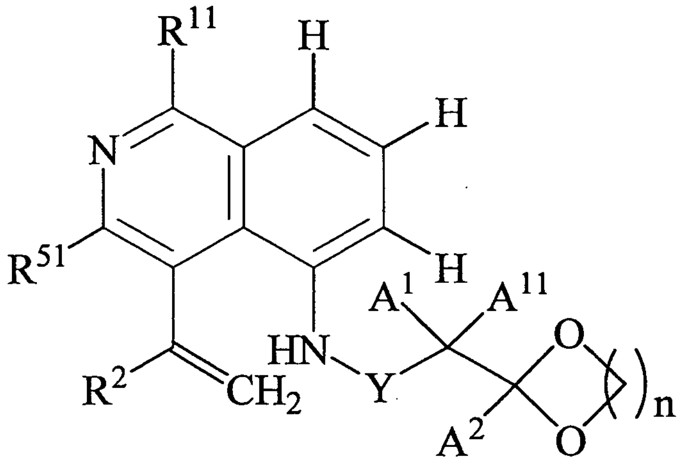

(L-a)

[wherein n, $R^2$, $R^{11}$, $R^{51}$, and $A^{11}$ have the same meanings as those defined above; and Y, $A^1$, and $A^2$ have the same meanings as those defined above, provided that when any of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ is not present, said combination is excluded] in the same manner as that of Step 3-1 (Step 4-1).

The compounds of the formula (L-a) constitute a part of the compounds of the formula (L) mentioned above, and the preparation method thereof is the same as that described above. Further, the compounds of the formula (J-d-1) constitute a part of the compounds of the formula (J-d) as described above, and therefore they can be derived into the compounds of the formula (1) wherein $X^1 \cdots X^2$ is $-CH(R^2)-CH_2-$ by such preparation steps starting from a compound of the formula (J-d) as described in Preparation method 2.

The compounds of the present invention represented by the aforementioned formula (1) and physiologically acceptable salts thereof have cell movement inhibitory actions on the basis of inhibition against phosphorylation of the myosin regulatory light chain in the cells, and are preferred as active ingredients of medicaments.

Among the cell movement inhibitory actions of the compounds of the present invention, the cell contraction inhibitory action can be confirmed by measuring vasoconstriction inhibitory activity, tracheal relaxation activity, intraocular pressure reducing activity, respiratory tract constriction inhibitory activity, or the like. The action to regulate change of cell morphology can be confirmed by, for example, measuring neurite outgrowth of nerve cells, or the like. The inhibitory action on cell migration (the action will be abbreviated as "cell migration inhibitory action") can be confirmed by measuring neutrophil migration inhibitory activity, respiratory tract inflammation suppressing activity, or the like. The cell release inhibitory action can be confirmed by measuring the chemical mediator releasing amount from neutrophils. The cell aggregation inhibitory action can be confirmed by measuring platelet aggregation inhibitory activity, or the like. Further, the apoptosis inhibitory action can be confirmed by, for example, giving stimulation to induce apoptosis to cells and then measuring cell viability or occurring frequencies of morphological changes of cells characteristic to apoptosis such as nuclear condensation, nuclear fragmentation, and blebbing of cells.

However, since the cell movement inhibitory actions on the basis of the inhibition of phosphorylation of the myosin regulatory light chain in the cells are known to be associated with various biological actions as described in the section of related art in the specification, the cell movement inhibitory actions must be construed in their broadest sense including the aforementioned cell contraction inhibitory action, action to regulate change of cell morphology, cell migration inhibitory action, cell release inhibitory action, cell aggregation inhibitory action, and apoptosis inhibitory action.

For example, the compounds of the present invention represented by the aforementioned formula (1) and salts thereof have an inhibitory activity against phosphorylation of the myosin regulatory light chain (see, Test Example 1 of the specification), vasoconstriction inhibitory activity (see, Test Example 2 in the specification), antigen stimulation-induced respiratory tract constriction suppressing activity (see, Test Example 3 in the specification), intraocular pressure reducing activity (see, Test Example 4 in the specification), neurite outgrowth activity (see, Test Example 5 in the specification), neutrophil migration inhibitory activity (see, Test Example 6 in the specification), chronic respiratory tract inflammation suppressing activity (see, Test Example 7 in the specification), acute respiratory tract inflammation inhibitory activity (see, Test Example 8 in the specification), tracheal relaxation activity (see, Test Example 11 in the specification), and

constriction elicitor-induced respiratory tract constriction suppressing activity (see, Test Example 12 in the specification). Further, as demonstrated by the test examples, the compounds represented by the aforementioned formula (1) and salts thereof have notably higher vasoconstriction inhibitory activity, antigen stimulation-induced respiratory tract constriction inhibitory activity, intraocular pressure reducing activity, neurite outgrowth activity, neutrophil migration inhibitory activity, chronic respiratory tract inflammation suppressing activity, acute respiratory tract inflammation inhibitory activity, tracheal relaxation activity, and constriction elicitor-induced respiratory tract constriction suppressing activity as compared with the conventional isoquinoline compounds. Therefore, the compounds represented by the aforementioned formula (1) and salts thereof are useful as active ingredients of medicaments for prophylactic and/or therapeutic treatment of diseases relating to contraction of various cells, diseases relating to morphological change of various cells, diseases relating to migration of various cells, diseases relating to release of various cells, diseases relating to aggregation of various cells, and/or diseases relating to apoptosis of various cells and the like.

Although it is not intended to be bound by any specific theory, action mechanism of the compounds of the present invention represented by the aforementioned general formula (1) and salts thereof can be presumed as follows. It is known that increase of the amount of phosphorylated myosin regulatory light chain activates the actomyosin system, which is a movement apparatus of cytoskeleton, and activates cell movements. Therefore, it is considered that the phosphorylation reaction of myosin regulatory light chain is important for cell movements (Kamm, K., et al., Annu. Rev. Physiol., 51, pp.299-313, 1989; Niggli, V., FEBS Lett., 445, pp.69-72, 1999; Itoh, K., et al., Biochim. Biophys. Acta., 1136, pp.52-56, 1992; Kitani, S., et al., Biochem. Biophys. Res. Commun., 183, pp.48-54, 1992). Measurement of the amount of phosphorylated myosin regulatory light chain in the cells revealed that the compounds represented by the aforementioned formula (1) and salts thereof decrease the amount of phosphorylated myosin regulatory light chain in the cells (refer to Test Example 1 in the specification).

It is known that the amount of phosphorylated myosin regulatory light chain in the cells is determined by activated states of two reaction routes including Reaction route 1 and Reaction route 2 described below (Fukata, Y., et al., Trends Pharmacol. Sci., 22, pp.32-39, 2001).

<Reaction route 1>
Increase of intracellular calcium concentration → Activation of myosin light chain kinase → Increase of amount of phosphorylated myosin regulatory light chain
<Reaction route 2>
Activation of low molecular weight G protein Rho → Activation of Rho kinase → Phosphorylation (inactivation) of myosin phosphatase → Increase of amount of phosphorylated myosin regulatory light chain

It is considered that a compound that inhibits Reaction route 1 and/or Reaction route 2 mentioned above has an activity for decreasing the amount of phosphorylated myosin regulatory light chain. In order to estimate whether either or both of Reaction route 1 and Reaction route 2 mentioned above are the target site for the compounds of the present invention represented by the aforementioned formula (1) and salts thereof, effects of the compounds of the present invention represented by the aforementioned formula (1) and salts thereof on increase of intracellular calcium concentration and activity of myosin light chain kinase were examined. As a result, it was found that the compounds of the present invention and salts thereof gave no influence on the increase of intracellular calcium concentration (see, Test Example 9), and did not inhibit the myosin light chain kinase activity (see, Test Example 10). Therefore, it is presumed that the compounds of the formula (1) according to the present invention do not inhibit Reaction route 1 mentioned above, but inhibit Reaction route 2 mentioned above to decrease the amount of phosphorylated myosin regulatory light chain. Thus, the compounds of the present invention can be used as inhibitors of the Rho/Rho kinase pathway. The inhibition of Reaction route 2 mentioned above by the compounds of the present invention represented by the aforementioned formula (1) and salts thereof may be confirmed by measuring the inhibitory activity for the Rho kinase activity, or alternatively, by measuring the inhibitory activity for the phosphorylation reaction of myosin phosphatase.

The activity of Rho kinase can be measured by the method disclosed in WO01/56988. More specifically, ATP ($\gamma$ $^{32}$P-ATP) is added to a substrate (Ribosomal S6 kinase substrate) together with a commercially available Rho kinase (Upstate) to start the enzymatic reaction and phosphorylate the substrate. The substrate is adsorbed on filter paper, and ATP is washed off with the phosphate buffer. Then, the amount of the phosphorylated substrate is measured by using a liquid scintillation counter. The inhibitory activity of the compounds of the present invention represented by the aforementioned formula (1) for the Rho kinase activity can be determined by adding the compounds before starting the enzymatic reaction, and measuring suppression of the phosphorylation amount of the substrate. The phosphorylation reaction of myosin phosphatase can be measured by, for example, using an antibody specifically recognizing the phosphorylated myosin phosphatase (Feng, J. et al., J. Biol. Chem., 274, pp.37385-37390, 1999). More specifically, proteins including myosin phosphatase are extracted from a tissue, subjected to electrophoresis on acrylamide gel, and transferred to a nitrocellulose membrane. The proteins are reacted with antibodies specifically recognizing phosphorylated myosin phosphatase to detect the amount of phosphorylated myosin phosphatase. The inhibitory activity on the phosphorylation

reaction of myosin phosphatase can be determined by adding the compounds before starting the extraction from the tissue, and measuring suppression of the phosphorylation amount of the myosin phosphatase.

It is considered that the compounds of the present invention represented by the aforementioned formula (1) and salts thereof inhibit the Rho/Rho kinase pathway, which is Reaction route 2 mentioned above, and exhibit more potent cell contraction inhibitory activity and cell migration inhibitory activity compared with the conventional isoquinoline compounds. It is known that the Rho/Rho kinase route plays an important role for cell contraction and cell migration. Other than the above, it has been reported that the Rho/Rho kinase pathway controls a variety of cellular functions such as aggregation, release, production, division, apoptosis, and gene expression in various cell lines (Fukata, Y., et al., Trends in Pharmacological Sciences, 22, pp.32-39, 2001; Murata T., et al., J. Hepatotol., 35, pp.474-481, 2001; Ohnaka, K., et al., Biochem. Biophys. Res. Commun., 287, pp.337-342, 2001; Yuhong, S., et al., Exp. Cell Res., 278, pp.45-52, 2002; Arakawa, Y. et al., BIO Clinica, 17(13), pp.26-28, 2002; Inoue, M. et al., Nat. Med., 10, pp.712-718, 2004). Therefore, the compounds of the present invention which inhibit the Rho/Rho kinase pathway exhibit, based on that effect, more potent cell contraction inhibitory activity (Test Examples 2, 3, 4, 11, and 12), cell morphology change regulating activity (Test Example 5), cell migration inhibitory activity (Test Examples 6, 7, and 8), cell release inhibitory activity, cell aggregation inhibitory activity, apoptosis inhibitory activity, and activity of regulating gene expression compared with the conventional isoquinoline compounds, and are useful as active ingredients of medicaments for prophylactic and/or therapeutic treatment of diseases relating to contraction of various cells, diseases relating to morphological change of various cells, diseases relating to migration of various cells, diseases relating to release from various cells, diseases relating to aggregation of various cells, diseases relating to apoptosis of various cells, and/or diseases relating to abnormal gene expression in various cells (Jikken Igaku (Experimental Medicine) Vol. 17, 7, 1999).

Examples of the diseases relating to contraction of various cells include, for example, as those relating to vascular smooth muscles, hypertension, arteriosclerosis, cerebral circulatory disturbance, brain function disorder with the aforementioned disease (mental disorder, memory disorder, dementia, delirium, poriomania, dyskinesia and the like), dizziness, auditory disturbance, cardiac diseases, pokkuri-byou (sudden death), disturbances of peripheral circulation, disturbances of retinal circulation, renal failure and the like, as those relating to airway smooth muscles, asthma, acute respiratory distress syndrome (ARDS), pulmonary emphysema, peripheral respiratory tract disease, chronic bronchitis, chronic obstructive pulmonary disease (COPD) and the like (Ueki, J. et al., Gendai Iryo (Contemporary Medical Care), Vol.34, No.9, pp.87-92, 2002), as those relating to digestive tract smooth muscles, vomiting, chronic gastritis, reflux esophagitis, irritable bowel syndrome and the like, as those relating to smooth muscle cells existing in eyes, glaucoma and the like, as those relating to vitreum of eyes, vitreoretinal diseases and the like (Hirayama, K., et al., Preliminary Published Abstracts of the 42nd Congress of the Vitreoretina Society of Japan), as those relating to smooth muscles of bladder and urethra, dysuria, pollakiuria, incontinence and the like, as those relating to smooth muscles of uterus, gestational toxicosis, threatened premature delivery, abortion and the like, and as those relating to smooth muscles of penis, erectile dysfunction is known. However, the diseases are not limited to the aforementioned examples.

More precisely, examples of hypertension include essential hypertension, renal hypertension, renovascular hypertension, hypertension during pregnancy, endocrine hypertension, cardiovascular hypertension, neurogenic hypertension, iatrogenic hypertension, pulmonary hypertension and the like, and examples of arteriosclerosis include those in which pathological change is observed in major arteries in whole body such as coronary artery, aorta abdominalis, renal artery, carotid artery, ophthalmic artery, and cerebral artery. Examples of cerebral circulatory disturbance include cerebral thrombosis, cerebral infarction, cerebral hemorrhage, transient brain ischemic attack, hypertensive encephalopathy, cerebral arteriosclerosis, subdural hemorrhage, epidural hemorrhage, subarachnoid hemorrhage, brain hypoxia, cerebral edema, encephalitis, brain tumor, head injury, mental disorder, metabolic intoxication, drug intoxication, transient asphyxia, deep anesthesia in operation and the like. The cardiac diseases include congestive heart failure, acute myocardial infarction, previous myocardial infarction, subendocardial infarction, right ventricular infarction, atypical myocardial infarction, ischemic cardiomyopathy, variant angina pectoris, stable angina, effort angina, coronary vasospasm, postinfarction angina, unstable angina pectoris, arrhythmia, and acute cardiac death.

The peripheral circulatory disturbances include aortic diseases such as Buerger's disease, arteriosclerotic obliteration, and Raynaud's syndrome, venous diseases such as venous thrombosis and thrombophlebitis, hyperviscosity syndrome, frostbite and chilblain, psychoesthesia and hypnagogic disturbance due to feeling of cold, bedsore, cleft, and alopecia. Examples of the retinal circulatory disturbances include retinal vascular obstruction, arteriosclerotic retinopathy, vasospastic retinopathy, hypertonic fundus, hypertensive retinopathy, renal retinopathy, hypertensive neuroretinopathy, diabetic retinopathy and the like. Glaucoma includes primary glaucoma, secondary glaucoma, developmental glaucoma, childhood secondary glaucoma and the like, as well as more narrowly classified types of the foregoings, including primary open-angle glaucoma, primary angle-closure glaucoma, mixed-type glaucoma, ocular hypertension and the like (Japanese Journal of Ophthalmology, vol. 107, No. 3, 2003). Further, examples of the vitreoretinal diseases include retinal detachment, retinoschisis, vitreoretinal interface syndrome, retinal pigment epitheliosis, macular hole, phacomatosis, vitreous hemorrhage, retinal circulatory disturbances and the like (the vitreoretinal diseases mentioned herein include more narrowly classified diseases belonging to each of the categories according to the pathological typology described

in Shin Zusetsu Rinsho Ganka Koza (Illustrative Lecture of Clinical Ophthalmology, New Edition), Ed. By Tano, Y., Araie, M., et al, Vol. 5, Vitreoretinal Diseases, MEDICAL VIEW, 2003). The urinary disturbances include dysuria, bladder neck contracture, bladder neck occlusion, urethral syndrome, detrusor sphincter dyssynergia, unstable bladder, chronic prostatitis, chronic cystitis, prostate pain, Hinman's syndrome, Fowler's syndrome, psychogenic dysuria, drug-induced dysuria, dysuria with aging and the like. The erectile dysfunction include organic erectile dysfunction accompanying diseases of diabetes mellitus, arteriosclerosis, hypertension, multiple-sclerotic cardiac diseases, hyperlipidemia, depression and the like, functional erectile dysfunction, erectile dysfunction with aging, and erectile dysfunction after spinal cord injury or radical prostatectomy.

Examples of the diseases relating to morphological change of various cells include, for example, various nerve dysfunctions as those relating to nerve cells. As the nerve dysfunctions, for example, neural damages caused by trauma (spinal cord injury and the like), neurodegenerative diseases such as Alzheimer's disease, Parkinson's disease, diabetic retinopathy, and glaucoma and the like can be exemplified. Glaucoma refers to the same as that mentioned above.

Examples of the diseases relating to migration of various cells include, for example, as those relating to cancer cells, infiltration and metastasis of cancer. Examples of those relating to vascular endothelial cells include angiogenesis, neovascular maculopathy, macular edema and the like (the macular diseases mentioned herein include more narrowly classified diseases belonging to each of the categories according to the pathological typology described in Shin Zusetsu Rinsho Ganka Koza (Illustrative Lecture of Clinical Ophthalmology, New Edition), Ed. By Tano, Y., Araie, M., et al, Vol. 5, Vitreoretinal Diseases, MEDICAL VIEW, 2003). Examples of those relating to leukocytes include bacterial infection, allergic hypersensitive diseases (e.g., bronchial asthma, atopic dermatitis, pollinosis, anaphylactic shock and the like), collagen diseases (e.g., rheumatoid arthritis, systemic lupus erythematodes, multiple sclerosis, Sjogren's disease and the like), angiitis, inflammatory bowel diseases (e.g., ulcerative colitis, Crohn's disease and the like), ischemic reperfusion injury of visceral organs, traumatic spinal cord injury, pneumonia, hepatitis, nephritis, pancreatitis, otitis media, sinusitis, arthritis (for example, osteoarthritis, gout and the like can be exemplified), fibrosis, AIDS, adult T-cell leukemia, rejection after organ transplantation (graft versus host reaction), vascular restenosis, and endotoxin shock. Example of the cancer include myelocytic leukemia, lymphatic leukemia, gastric cancer, carcinoma of the colon and rectum, lung cancer, pancreatic carcinoma, hepatocellular carcinoma, carcinoma of the esophagus, ovarian cancer, breast cancer, skin cancer, head and neck cancer, cancer of the testicles, neuroblastoma, urinary tract epithelial cancer, multiple myeloma, carcinoma uteri, melanoma, brain tumor and the like. Examples of hepatitis include hepatitis by virus infection (e.g., hepatitis B, hepatitis C and the like), and alcoholic hepatitis. Examples of the pneumonia include chronic obstructive pulmonary disease (COPD) and interstitial pneumonia, which may shift to fibrosis. Examples of nephritis include chronic nephritic syndrome, asymptomatic proteinuria, acute nephritic syndrome, nephrotic syndrome, IgA nephropathy, pyelonephritis, glomerulonephritis and the like. Fibrosis include chronic pathological changes characterized by excess deposition of connective tissue proteins in lung, skin, heart, liver, pancreas, kidney and the like. The major pathological conditions are pulmonary fibrosis, hepatic fibrosis, and skin fibrosis. However, fibrosis is not limited to these examples. In hepatic fibrosis, viral hepatitis progresses by infection of, in particular, hepatitis B virus or hepatitis C virus, thus hepatic cells cause necrosis, and thereby fibrosis progresses, which means macronodular hepatic cirrhosis. Further, hepatic fibrosis also includes micronodular hepatic cirrhosis caused by progress of alcoholic hepatitis.

Examples of diseases relating to release of various cells include, as those relating to leukocytes, for example, allergic diseases.

Examples of the allergic diseases include asthma, atopic dermatitis, allergic conjunctivitis, allergic arthritis, allergic rhinitis, allergic pharyngitis and the like.

Examples of the diseases relating to aggregation of various cells include, as those relating to platelets, for example, thrombosis.

Thrombosis include the aforementioned circulatory disturbances of major arteries, major veins and peripheral arteries and veins in whole body, as well as shock caused by hemorrhage, drug intoxication, or endotoxin, disseminated intravascular coagulation (DIC) following it, and multiple organ failure (MOF).

Examples of the diseases relating to apoptosis of various cells include, as those relating to nerves, for example, neurodegenerative diseases such as Alzheimer's disease, Parkinson's disease, diabetic peripheral neuropathy, retinopathy, amyotrophic lateral sclerosis due to cerebral ischemia, pigmented retinitis, and cerebellar degeneration, and glaucoma. Examples of glaucoma are mentioned above. AIDS, and fulminant hepatitis are examples of disease relating to viruses, chronic heart failure due to myocardial ischemia is an example of diseases relating to smooth muscles, myelodysplasia, aplastic anemia, sideroblastic anemia, and graft-versus-host disease (GVHD) after organ transplantation are examples of diseases relating to blood, arthrosteitis, and osteoporosis is an example of diseases relating to bones.

Examples of the diseases relating to abnormal gene expression of various cells include, for example, bone diseases as those relating to bone cells, AIDS as one relating to virus, cancers as those relating to cancer cells, and neurogenic pain (also called neuropathic pain) as one relating to nerve cells.

Examples of the bone diseases include osteoporosis, hypercalcemia, bone Paget's disease, renal osteodystrophy, rheumatoid arthritis, osteoarthritis, osteogenesis imperfecta tarda, bone damage, periodontal bone disorder and the like.

Examples of AIDS include acquired immunodeficiency syndrome caused by human immunodeficiency virus (HIV) infection. Examples of the cancers include gastric cancer, carcinoma of the colon and rectum, hepatocellular carcinoma, pancreatic carcinoma, lung cancer, leukemia, malignant lymphoma, carcinoma uteri, ovarian cancer, breast cancer, skin cancer and the like. Neurogenic pain include peripheral neurogenic pain and central neurogenic pain. Examples of peripheral neurogenic pain include, for example, traumatic nerve injury, ischemic neuropathy, multiple neurosis, nerve-plexus injury, compression of nerve root, stump pain after dismemberment, postherpetic neuralgia, trigeminal neuralgia and the like, and examples of central neurogenic pain include, for example, cerebral apoplexy, multiple sclerosis, spinal cord injury, epilepsy and the like (Yuge T. et al., Medical Care Practice of Anesthesiology 6, Current Situation of Neuropathic Pain, Bunko-do, 2002).

Usefulness of the compounds of the present invention represented by the aforementioned formula (1) and salts thereof as active ingredients of medicaments for prophylactic and/or therapeutic treatment of hypertension can be confirmed by, for example, administering the compound to various hypertension model animals or the like. Examples of hypertension animal models include spontaneous hypertensive rat (SHR), renal hypertensive rat, DOCA-salt hypertensive rat and the like (Uehata, M. et al., Nature, 389, 990-994, 1997). A compound is orally, intravenously or intraperitoneally administered to a hypertension model animal at a dose of 0.1 to 1,000 mg/kg, preferably 0.1 to 100 mg/kg, and the diastolic blood pressure is measured. The usefulness as a medicament for hypertension can be confirmed based on an action of reducing the diastolic blood pressure.

Usefulness of the compounds of the present invention represented by the aforementioned formula (1) and salts thereof as active ingredients of medicaments for prophylactic and/or therapeutic treatment of pulmonary hypertension can be confirmed by using, for example, a rat model of pulmonary hypertension created by administering monocrotaline to a rat for 2 to 3 weeks (Ito, K.M. et al., Am. J. Physiol., 279, H1786-H1795, 2000). A compound is orally, intravenously or intraperitoneally administered to a model animal of pulmonary hypertension at a dose of 0.1 to 1,000 mg/kg, preferably 0.1 to 100 mg/kg, and the intrapulmonary pressure is measured. The usefulness as a medicament for pulmonary hypertension can be confirmed based on an action of decreasing the intrapulmonary pressure.

Usefulness of the compounds of the present invention represented by the aforementioned formula (1) and salts thereof as active ingredients of medicaments for prophylactic and/or therapeutic treatment of arteriosclerosis can be confirmed by using, for example, a rat model of L-NAME-induced arteriosclerosis (Cir. Res. 89(5):415-21, 2001), a rat model of balloon-induced neointimal formation (Sawada N. et al., Circulation 101 (17):2030-3, 2000) or the like. A compound is orally, intravenously or intraperitoneally administered to a model animal of arteriosclerosis at a dose of 0.1 to 1,000 mg/kg, preferably 0.1 to 100 mg/kg, and thickening of arteries is observed. The usefulness as a medicament for arteriosclerosis can be confirmed based on an action of suppressing neointimal formation in arteries.

Usefulness of the compounds of the present invention represented by the aforementioned formula (1) and salts thereof as active ingredients of medicaments for prophylactic and/or therapeutic treatment of cerebral circulatory dysfunction can be confirmed by using, for example, a gerbil model of hippocampal neuronal death (Kirino et al., Brain Res., 239, 57-69, 1982) or the like. A compound is orally, intravenously or intraperitoneally administered to the model animal at a dose of 0.1 to 1,000 mg/kg, preferably 0.1 to 100 mg/kg, and the amount of energy-related substances and survival period of gerbil, or inhibition of late-onset of neuronal death is measured. The usefulness as a medicament for cerebral circulatory dysfunction can be confirmed based on actions for maintaining, improving and activating cerebral metabolic ability, brain and nerve protective action, and action for suppressing formation of cerebral infarction.

Usefulness of the compounds of the present invention represented by the aforementioned formula (1) and salts thereof as active ingredients of medicaments for prophylactic and/or therapeutic treatment of cardiac diseases can be confirmed by using, for example, a rat model of myocardial infarction based on the ligation of artery (Xia Q.G. et al., Cardiovasc. Res., 49(1):110-7, 2001) or the like. Effectiveness as a medicament for cardiac diseases can be confirmed by orally, intravenously or intraperitoneally administering a compound to the model animal at a dose of 0.1 to 1,000 mg/kg, preferably 0.1 to 100 mg/kg, and observing a cardiac tissue fixed by formalin perfusion after ischemic reperfusion.

Usefulness of the compounds of the present invention represented by the aforementioned formula (1) and salts thereof as active ingredients of medicaments for prophylactic and/or therapeutic treatment of disturbances of peripheral circulation can be confirmed by using, for example, a rat model of bedsore (Pierce S.M. et al., Am. J. Physiol. Heart Circ. Physiol., 281(1):H67-74, 2001) or the like. Effectiveness as a medicament for bedsore (peripheral circulatory disturbance) can be confirmed by orally, intravenously or intraperitoneally administering a compound to the model animal at a dose of 0.1 to 1,000 mg/kg, preferably 0.1 to 100 mg/kg, compressing the hind leg skin at a pressure of 50 mmHg, and then observing a tissue of necrotic area of the lesion or measuring epithelial blood flow of the same.

Usefulness of the compounds of the present invention represented by the aforementioned formula (1) and salts thereof as active ingredients of medicaments for prophylactic and/or therapeutic treatment of disturbances of retinal circulation can be confirmed by using, for example, rabbit model of rose bengal-mediated argon laser retinal vein photothrombosis (Jpn. J. Ophthalmol., 45(4):359-62, 2001), or the like. Effectiveness as a medicament for retinal circulatory disturbance can be confirmed by orally, intravenously or intraperitoneally administering a compound to the model animal at a dose of 0.1 to 1,000 mg/kg, preferably 0.1 to 100 mg/kg, and comparing the degree of retinal circulatory disturbance

with that of a control based on count of laser spots to evaluate degree of the action and sustainment of the action.
Usefulness of the compounds of the present invention represented by the aforementioned formula (1) and salts thereof as active ingredients of medicaments for prophylactic and/or therapeutic treatment of renal failure can be confirmed by using, for example, a rat model of one-kidney, one-clip renal hypertension (Kiso to Rinsho, 30, 511-524, 1996). Effectiveness as a medicament for renal failure can be confirmed by orally, intravenously or intraperitoneally administering a compound to the model animal at a dose of 0.1 to 1,000 mg/kg, preferably 0.1 to 100 mg/kg, and measuring the diuretic effect.

Usefulness of the compounds of the present invention represented by the aforementioned formula (1) and salts thereof as active ingredients of medicaments for prophylactic and/or therapeutic treatment of asthma, for example, bronchial asthma, can be confirmed by using, for example, suppression of constriction of a trachea isolated from an animal (Kunihiko Iizuka, Allergy, 47:943, 1998; Kunihiko Iizuka, Akihiro Yoshii, Jpn. J. Respirol. Soc., 37:196, 1999.), antigen-stimulation induced respiratory tract constriction model, antigen-stimulation induced chronic respiratory tract inflammation model (Henderson, W.R., et al., Am. J. Respir. Cric. Care Med., 165(1), pp.108-116, 2002), constriction elicitor-induced respiratory tract constriction model (histamine, acetylcholine and the like are generally used, Daniela, S. et al., J. Pharmacol. Exp. Ther., 297(1), pp.280-290, 2001), LPS-induced acute respiratory tract inflammation model, inhibition of human peripheral blood leucocyte migration and the like. The usefulness as a medicament for bronchial asthma can be confirmed by orally, intravenously or intraperitoneally administering a compound to the model animal at a dose of 0.1 to 1,000 mg/kg, preferably 0.1 to 100 mg/kg, and measuring elevation of airway resistance caused by tracheal constriction or relaxation, antigen stimulation, histamine inhalation, or acetylcholine inhalation, migrating leucocyte count in bronchoalveolar lavage fluid and the like, or performing histological analysis.
Usefulness of the compounds of the present invention represented by the aforementioned formula (1) and salts thereof as active ingredients of medicaments for prophylactic and/or therapeutic treatment of irritable bowel syndrome can be confirmed by administering the compounds to a stress burden model animal, or the like. Examples of the stress burden model animal include, for example, a rat model of arresting stress (Miyata, K. et al., J. Pharmacol. Exp. Ther., 259, pp. 815-819, 1991), a CRH-administered rat model (Miyata, K. et al., Am. J. Physiol., 274, G827-831, 1998) and the like. A compound is orally, intravenously or intraperitoneally administered to a stress burden model animal at a dose of 0.1 to 1,000 mg/kg, preferably 0.1 to 100 mg/kg, and counting the number of fecal pellets. The usefulness as a medicament for curative medicine of irritable bowel syndrome can be confirmed based on effect for reducing the number of fecal pellets.

Usefulness of the compounds of the present invention represented by the aforementioned formula (1) and salts thereof as active ingredients of medicaments for prophylactic and/or therapeutic treatment of glaucoma can be confirmed by, for example, measuring intraocular pressure of a rabbit, cat or monkey after administration of the medicaments by instillation (Surv. Ophthalmol. 41:S9-S18, 1996). The usefulness as a medicament for glaucoma can be confirmed by instilling or orally, intravenously or intraperitoneally administering a compound to a locally anesthetized rat or monkey model animal at a dose of 0.1 to 1,000 mg/kg, preferably 0.1 to 100 mg/kg, and measuring the intraocular pressure over time using an tonometer to evaluate degree of the intraocular pressure reducing activity, or sustainment of the intraocular pressure reducing activity.
Usefulness of the compounds of the present invention represented by the aforementioned formula (1) and salts thereof as active ingredients of medicaments for prophylactic and/or therapeutic treatment of vitreoretinal diseases can be confirmed by a known method, for example, the methods described in Oshima, Y. et al., Gene Ther., 9(18), pp.1214-20, 2002; and Ito, S., et al., Graefes Arch. Clin. Exp. Ophthalmol., 237(8), pp.691-6., 1999. The usefulness as a medicament for vitreoretinal diseases can be confirmed by orally, intravenously, intraperitoneally, or intraocularly administering (direct administration to vitreum or retina) a compound to a rabbit in which retinal detachment is induced by cell transfer to the vitreoretinal interface, vitrectomy, or the like at a dose of 0.1 to 1,000 mg/kg, preferably 0.1 to 100 mg/kg, and evaluating amelioration of the pathological conditions on the basis of histological analysis.
Usefulness of the compounds of the present invention represented by the aforementioned formula (1) and salts thereof as active ingredients of medicaments for prophylactic and/or therapeutic treatment of dysuria can be confirmed by using, for example, a model of rhythmic bladder contraction (Kaneko S. et al., Folia Pharmacol. Japon, Vol. 93(2), 55-60, 1989; Nomura N. et al., Folia Pharmacol. Japon, Vol. 94(3), 173-, 1989.) or the like. The usefulness as a medicament for urinary disturbance can be confirmed by orally, intravenously or intraperitoneally administering a compound to an anesthetized rat or dog at a dose of 0.1 to 1,000 mg/kg, preferably 0.1 to 100 mg/kg, and measuring the number of rhythmic contraction of filled bladder (micturition).

Usefulness of the compounds of the present invention represented by the aforementioned formula (1) and salts thereof as active ingredients of medicaments for prophylactic and/or therapeutic treatment of erectile dysfunction can be confirmed by a known method, for example, the method described in J. Uro., 151, 797-800, 1994. A compound is dissolved in a hydrophilic ointment, 30 mg of the ointment was applied to a rat penis, and the rat is held in an acrylic cylinder for 10 minutes so that the rat was not able to lick the penis. The rat is moved to an acrylic cage of 30 cm x 30 cm, and videotaped for 60 minutes from the side and the bottom of the cage. Then, the number of erection of the penis per 30 minutes can be counted to confirm the usefulness as a medicament for erectile dysfunction.

Usefulness of the compounds of the present invention represented by the aforementioned formula (1) and salts thereof as active ingredients of medicaments for suppressing cancer metastasis and invasion can be confirmed by, for example, the method described in Cancer Res., 55:3551-3557 (1995). The usefulness as a medicament for cancer metastasis and invasion can be confirmed by orally, intravenously or intraperitoneally administering a compound at a dose of 0.1 to 1,000 mg/kg, preferably 0.1 to 100 mg/kg, to a nude mouse transplanted with human cancer cell suspension transplantable to immunodeficient mice at the same site (spontaneous metastasis model), and measuring the metastasized lesion.

Usefulness of the compounds of the present invention represented by the aforementioned formula (1) and salts thereof as active ingredients of medicaments for prophylactic and/or therapeutic treatment of collagen disease can be confirmed by using, for example, collagen-induced arthritis model of a rat or mouse (Griffith, M.M. et al., Arthritis Rheumatism, 24: 781, 1981; Wooley, P.H. et al., J. Exp. Med., 154:688, 1981). The usefulness as a medicament for collagen disease can be confirmed by orally, intravenously or intraperitoneally administering a compound to the model mouse or rat at a dose of 0.1 to 1,000 mg/kg, preferably 0.1 to 100 mg/kg, and measuring footpad volume or progression of bone destruction.

Usefulness of the compounds of the present invention represented by the aforementioned formula (1) and salts thereof as active ingredients of medicaments for prophylactic and/or therapeutic treatment of inflammatory bowel disease can be confirmed by using a rat model of idiopathic ulcerative colitis induced by subserosal injection of acetic acid, a model of sodium dextransulfate-induced colitis, a model of trinitrobenzenesulfonic acid-induced colitis (Kojima et al., Folia. Pharmacol. Jpn., 118, 123-130, 2001), or the like. The usefulness as a medicament for inflammatory bowel disease can be confirmed by, for example, orally, intravenously or intraperitoneally administering a compound at a dose of 0.1 to 1,000 mg/kg, preferably 0.1 to 100 mg/kg, to a rat in which colitis is induced by intraintestinal injection of acetic acid, dissecting the rat after several days to two weeks, then observing and measuring the ulcer area of the intestinal epithelium, and amount of leucotriene B4 in a colon homogenate.

Usefulness of the compounds of the present invention represented by the aforementioned formula (1) and salts thereof as active ingredients of medicaments for prophylactic and/or therapeutic treatment of spinal cord injury can be confirmed by using, for example, a rat model of spinal cord ablation (Sayer F.T. et al., Exp. Neurol., 175(1):282-96, 2002) or the like. Effectiveness as a medicament for spinal cord injury can be confirmed by orally, intravenously, intraperitoneally, or intraspinally administering a compound to the model animal at a dose of 0.1 to 1,000 mg/kg, preferably 0.1 to 100 mg/kg, and, after several weeks, examining a tissue of the spinal cord with a microscope to measure a degree of nerve regeneration.

Usefulness of the compounds of the present invention represented by the aforementioned formula (1) and salts thereof as active ingredients of medicaments for prophylactic and/or therapeutic treatment of pneumonia can be confirmed by using, for example, a mouse model of OVA-induced chronic pneumonia (Henderson W.R. et al., Am. J. Respir. Crit. Care Med., 165(1):108-116, 2002), a mouse model of LPS-induced acute pneumonia (Gonzales de Moraes, VL., et al., Br. J. Pharmacol., 123, pp.631-6, 1998), or the like. Effectiveness as a medicament for pneumonia can be confirmed by orally, intravenously or intraperitoneally administering a compound to the model animal at a dose of 0.1 to 1,000 mg/kg, preferably 0.1 to 100 mg/kg, and evaluating change in number of eosinophils or monocytes in the pulmonary cavity, and histological findings of inflammation.

Usefulness of the compounds of the present invention represented by the aforementioned formula (1) and salts thereof as active ingredients of medicaments for prophylactic and/or therapeutic treatment of hepatitis can be confirmed by using a mouse model of endotoxin-induced liver injury according to, for example, the method described in J. Immunol., 159, 3961-3967, 1997. The usefulness as a medicament for hepatitis can be confirmed by orally, intravenously or intraperitoneally administering a compound to the mouse model of endotoxin-induced liver injury at a dose of 0.1 to 1,000 mg/kg, preferably 0.1 to 100 mg/kg, and measuring the plasmic transaminase level or amount of hydroxyproline in a hepatic tissue, which are indicators of liver function, or performing histological analysis.

Usefulness of the compounds of the present invention represented by the aforementioned formula (1) and salts thereof as active ingredients of medicaments for prophylactic and/or therapeutic treatment of pancreatitis can be confirmed by using, for example, a mouse model of cerulein inducted acute pancreatitis (Niedirau, C. et al., Gastroenterology 88 (5 Pt 1):1192-1204, 1985) or the like. Effectiveness as a medicament for pancreatitis can be confirmed by orally, intravenously or intraperitoneally administering a compound to the model animal at a dose of 0.1 to 1,000 mg/kg, preferably 0.1 to 100 mg/kg, and measuring the serum amylase activity, or weight of pancreas.

Usefulness of the compounds of the present invention represented by the aforementioned formula (1) and salts thereof as active ingredients of medicaments for prophylactic and/or therapeutic treatment of nephritis can be confirmed by using, for example, a nephritis rat model prepared by administering anti-GBM antibodies obtained by immunizing a rabbit with a GBM fraction derived from a rat to a rat (WO01/56988), or the like. A compound is orally, intravenously or intraperitoneally administered to the nephritis rat model at a dose of 0.1 to 1,000 mg/kg, preferably 0.1 to 100 mg/kg, and the urinary proteins are measured. The usefulness as a medicament for nephritis can be confirmed based on an action of reducing the urinary protein level.

Usefulness of the compounds of the present invention represented by the aforementioned formula (1) and salts

thereof as active ingredients for suppressing allograft rejection at the time of organ transplantation can be confirmed by using, for example, a rat model of skin transplantation, rat model of heart transplantation (Ochiai T. et al., Transplant. Proc., 19, 1284-1286, 1987), or the like. Effectiveness as a medicament for suppressing rejection at the time of organ transplantation can be confirmed by orally, intravenously or intraperitoneally administering a compound to a model animal at a dose of 0.1 to 1,000 mg/kg, preferably 0.1 to 100 mg/kg, and estimating the graft survival ratio.
Usefulness of the compounds of the present invention represented by the aforementioned formula (1) and salts thereof as active ingredients of medicaments for prophylactic and/or therapeutic treatment of rheumatoid arthritis can be confirmed by using collagen-induced arthritis model of a rat or mouse (Griffith, M.M. et al., Arthritis Rheumatism, 24:781, 1981; Wooley, P.H. et al., J. Exp. Med., 154:688, 1981). The usefulness as a medicament for rheumatoid arthritis can be confirmed by orally, intravenously or intraperitoneally administering a compound to a model mouse or model rat at a dose of 0.1 to 1,000 mg/kg, preferably 0.1 to 100 mg/kg, and measuring footpad volume or progression of bone destruction.

Usefulness of the compounds of the present invention represented by the aforementioned formula (1) and salts thereof as active ingredients of medicaments for prophylactic and/or therapeutic treatment of chronic obstructive pulmonary disease (COPD) can be confirmed by using, for example, suppression of constriction of a trachea isolated from an animal, an antigen stimulation-induced respiratory tract constriction model, a constriction elicitor-induced respiratory tract constriction model (histamine, acetylcholine and the like are generally used), antigen stimulation-induced chronic respiratory tract inflammation model, a mouse model of LPS-induced acute respiratory tract inflammation, a tobacco smoke exposition model (Fuchigami J. et al., 73rd Meeting of Japanese Pharmacological Society, Collection of Abstracts, 2000), inhibition of chemotaxis of human peripheral leucocytes or the like. The usefulness as a medicament for COPD can be confirmed by orally, intravenously or intraperitoneally administering a compound to any of the model animals mentioned above at a dose of 0.1 to 1,000 mg/kg, preferably 0.1 to 100 mg/kg, and measuring tracheal constriction or relaxation, change in airway resistance, migrating leucocyte count in bronchoalveolar lavage fluid, change in number of number of eosinophils or monocytes in the pulmonary cavity, or histological findings of inflammation.
Usefulness of the compounds of the present invention represented by the aforementioned formula (1) and salts thereof as active ingredients of medicaments for prophylactic and/or therapeutic treatment of hepatic fibrosis can be confirmed by using a carbon tetrachloride-induced hepatic fibrosis model according to, for example, the method described in J. Hepatol., 35(4), 474-481, 2001. The usefulness as a medicament for hepatic fibrosis can be confirmed by orally, intravenously or intraperitoneally administering a compound to the hepatic fibrosis model at a dose of 0.1 to 1,000 mg/kg, preferably 0.1 to 100 mg/kg, and measuring the plasmic transaminase level, or amount of hydroxyproline in a hepatic tissue, which are indicators of liver function, or performing histological analysis.
Usefulness of the compounds of the present invention represented by the aforementioned formula (1) and salts thereof as active ingredients of medicaments for prophylactic and/or therapeutic treatment of pulmonary fibrosis can be confirmed by using an animal model of Bleomycin-induced pulmonary fibrosis according to the method described in, for example, Am. J. Respir. Crit. Care Med., 163(1), pp.210-217, 2001. The usefulness as a medicament for pulmonary fibrosis can be confirmed by orally, intravenously or intraperitoneally administering a compound to the pulmonary fibrosis mouse model at a dose of 0.1 to 1,000 mg/kg, preferably 0.1 to 100 mg/kg, and measuring respiratory function, or amount of hydroxyproline in a pulmonary tissue.

Usefulness of the compounds of the present invention represented by the aforementioned formula (1) and salts thereof as active ingredients of medicaments for prophylactic and/or therapeutic treatment of allergy can be confirmed by using an atopic dermatitis mouse model or the like according to the method described in, for example, Allergy, 50 (12) 1152-1162, 2001. The usefulness as a medicament for allergy can be confirmed by orally, intravenously or intraperitoneally administering a compound to an NC/Nga mouse pretreated with a surfactant or an organic solvent at a dose of 0.1 to 1,000 mg/kg, preferably 0.1 to 100 mg/kg, when eruption is induced in the mouse by using housedust mite antigens, and measuring the plasmic IgE level, number of eosinophils and the like.
Usefulness of the compounds of the present invention represented by the aforementioned formula (1) and salts thereof as active ingredients of medicaments for prophylactic and/or therapeutic treatment of thrombosis can be confirmed by using, for example, a rabbit model of experimentally-induced venous thrombus (Maekawa, T. et al., Trombos. Diathes. Haemorrh., 60, pp.363-370, 1974), or the like. Effectiveness as a medicament for thrombosis can be confirmed by orally, intravenously or intraperitoneally administering a compound to the model animal at a dose of 0.1 to 1,000 mg/kg, preferably 0.1 to 100 mg/kg, and estimating the incidence of thrombus.
Usefulness of the compounds of the present invention represented by the aforementioned formula (1) as active ingredients of medicaments for prophylactic and/or therapeutic treatment of Alzheimer's disease can be confirmed by using, for example, an in vitro culture system of nerve cells derived from rat embryos (Yankner, B.A. et al., Science, 250, pp. 279-282, 1990), or the like. Effectiveness as a medicament for Alzheimer's disease can be confirmed by adding 0.1 to 1 mM, preferably 0.1 to 100 μM, of a compound, and measuring suppression ratio for cell death induced by beta-amyloid proteins.

Usefulness of the compounds of the present invention represented by the aforementioned formula (1) and salts

thereof as active ingredients of medicaments for prophylactic and/or therapeutic treatment of bone disease can be confirmed by using, for example, a mouse model of osteoporosis prepared by ovariectomy (OVX mouse, Golub, L.M. et al., Ann. N.Y. Acad. Sci., 878, pp.290-310, 1999). A compound is orally, intravenously or intraperitoneally administered to the OVX mouse at a dose of 0.1 to 1,000 mg/kg, preferably 0.1 to 100 mg/kg, and deciduous dental roots are measured, or weight of skeletal bones is measured. The usefulness as a medicament for periodontal bone disorder or osteoporosis can be confirmed based on an action for suppressing periodontal breakdowns, or an action for suppressing skeletal bone weight loss.

Usefulness of the compounds of the present invention represented by the aforementioned formula (1) and salts thereof as active ingredients of medicaments for prophylactic and/or therapeutic treatment of AIDS can be confirmed by using, for example, a rhesus monkey model of SIV-infection (Crub S. et al., Acta Neuropathol., 101(2), pp.85-91, 2001) or the like. Effectiveness as a medicament for AIDS can be confirmed by orally, intravenously or intraperitoneally administering a compound to the model animal at a dose of 0.1 to 1,000 mg/kg, preferably 0.1 to 100 mg/kg, and quantifying the SIV mRNA level in blood.

Usefulness of the compounds of the present invention represented by the aforementioned formula (1) and salts thereof as active ingredients of medicaments for prophylactic and/or therapeutic treatment of cancer can be confirmed by using, for example, a mouse model of ultraviolet ray irradiation-induced skin cancer, a nude mouse model of tumor xenograft (Orengo I.F. et al., Arch Dermatol., 138(6), pp.823-824, 2002; Ki D.W. et al., Anticancer Res., 22(2A), pp.777-788, 2002) or the like. Effectiveness as a medicament for cancer can be confirmed by orally, intravenously or intraperitoneally administering a compound to a model animal at a dose of 0.1 to 1,000 mg/kg, preferably 0.1 to 100 mg/kg, and observing progression or reduction of the grafted cancer tissues on the body surface.

Usefulness of the compounds of the present invention represented by the aforementioned formula (1) and salts thereof as active ingredients of medicaments for prophylactic and/or therapeutic treatment of neurogenic pain can be confirmed by using, for example, a spared nerve injury model (Bennett, GJ. et al., Pain, 33(1) pp.87-107 (1988)), or the lie. Effectiveness as a medicament for neurogenic pain can be confirmed by orally, intravenously or intraperitoneally administering a compound to a model animal at a dose of 0.1 to 1,000 mg/kg, preferably 0.1 to 100 mg/kg, and measuring change of response to pain caused by thermal or physical stimulation.

As described above, the compounds of the present invention represented by the formula (1) are useful as the medicaments mentioned above.

Further, when test compounds of the compounds of the present invention or salts thereof were introduced into wells of a 96-well plate at a concentration three times higher than the $IC_{50}$ values obtained in Test Example 1, and the cell suspension prepared in Test Example 1 was added at a density of $10^6$/well, incubated for 30 minutes at room temperature and stained with trypan blue to determine the survival rates of the cells, a viability as high as 90% or more was observed in all the wells. Furthermore, when the compounds of the present invention or salts thereof were orally administered to mice every day at a dose of 30 mg/kg for 5 days, death was not observed. Therefore, the compounds of the present invention had no particular problem also in safety.

As the active ingredients of the medicaments of the present invention, the compounds represented by the aforementioned formula (1), and physiologically acceptable salts thereof are preferred.

The aforementioned substance, per se, may be administrated as the medicament of the present invention. A pharmaceutical composition containing one or more kinds of the aforementioned substances as the active ingredients and one or more kinds of pharmaceutical additives can be generally prepared and administrated orally or parenterally (e.g., intravenous administration, intramuscular administration, subcutaneous administration, transdermal administration, intrapulmonary administration, intranasal administration, instillation, intraurethral administration, intravaginal administration, sublingual administration, intrarectal administration and the like) to human or an animal other than human. The aforementioned pharmaceutical composition can be prepared in a dosage form suitable for an intended administration route. More specifically, examples of the pharmaceutical composition suitable for oral administration include oral drug products (tablets, film-coated tablets, intraoral collapsing tablets, hard capsules, soft capsules, powders, fine granules, granules, dry syrups, syrups, pills, troches and the like), and examples of the pharmaceutical composition suitable for parenteral administration include injections (liquid dosage forms, lyophilized dosage forms, suspensions and the like), inhalants, suppositories, transdermally absorbed agents (e.g., tapes), ointments, ophthalmic solutions, ophthalmic ointments, ophthalmic membrane adherent agents and the like. For glaucoma, preferred examples of the dosage form include oral drug products, ophthalmic solutions, ophthalmic ointments, and ophthalmic membrane adherent agents. Further, preferred dosage forms for bronchial asthma or chronic obstructive pulmonary disease include oral drug products, inhalants (for example, a method of inhaling powder of the pharmaceutical composition or a liquid dosage form prepared by dissolving or suspending the pharmaceutical composition in a solvent as it is, or inhaling mist thereof by using a sprayer called atomizer or nebulizer), and transdermal preparations.

These pharmaceutical compositions can be prepared in a conventional manner by using pharmaceutical additives generally used in this field (e.g., excipients, disintegrants, binders, lubricants, colorants, buffering agents, coating agents, flavors, fragrances, emulsifying agents, isotonic agents, solubilizing agents, preservatives, viscosity improvers,

pH adjusters and the like). Examples of the excipients include saccharides such as lactose, sucrose, and trehalose, sugar alcohols such as D-mannitol, erythritol, xylitol, and sorbitol, starches such as maize starch, crystalline cellulose, calcium hydrogenphosphate and the like, examples of the disintegrants include starches, partially pregelatinized starch, carmellose and metal salts thereof, croscarmellose sodium, sodium carboxymethyl starch, agar powder, crospovidone, low substituted hydroxypropylcellulose and the like, examples of the binders include hydroxypropylmethylcellulose, hydroxypropylcellulose, polyvinyl alcohol, methylcellulose, ethylcellulose, popidone, acacia powder, pullulan, pregelatinized starch and the like, and examples of the lubricants include stearic acid and metal salts thereof, talc, silicic acid and metal salts thereof, salt-hardened oil, sucrose fatty acid esters, sodium laurylsulfate, sodium stearyl fumarate and the like

When solid pharmaceutical compositions are prepared, there are used pharmaceutical additives including, for example, sucrose, lactose, glucose, fructose, trehalose, D-mannitol, sorbitol, erythritol, xylitol, maltitol, maize starch, potato starch, wheat starch, rice starch, crystalline cellulose, carmellose, carmellose calcium, low substituted hydroxypropylcellulose, croscarmellose sodium, crospovidone, dextrin, cyclodextrin, dextran, agar, xanthane gum, guar gum, rosin, acacia, hydroxypropylcellulose, hydroxypropylmethylcellulose, methylcellulose, ethylcellulose, polyvinyl alcohol, povidone, pregelatinized starch, partly pregelatinized starch, pullulan, pectin, polysorbate, polyethylene glycol, propylene glycol, glycerol, magnesium stearate, talc, light anhydrous silicic acid, hydrated silicon dioxide, kaolin, sucrose fatty acid esters, sodium laurylsulfate, silicic acid, aluminum silicate, magnesium aluminometasilicate, calcium carbonate, sodium hydrogencarbonate, sodium chloride, sodium citrate, citric acid, succinic acid, tartaric acid, hydrogenated castor oil, hydrogenated tallow, stearic acid, cetanol, olive oil, orange oil, soybean oil, cacao butter, carnauba wax, paraffin, vaseline, triacetin, triethyl citrate, iron oxide, caramel, tartrazine, vanillin, carmellose sodium, cellulose derivatives such as hydroxypropylmethylcellulose phthalate, hydroxypropylmethylcellulose acetate succinate, carboxymethylethylcellulose, carboxyvinyl polymer, cellulose acetate phthalate, cellulose acetate trimellitate, ethylcellulose, and cellulose acetate, polyethylene glycol, gelatin, shellac, methacrylic acid and derivatives thereof as well as copolymers thereof, ethylcellulose aqueous dispersion (Aquacoat), silicone oil, triacetin and the like. The tablets can be tablets having usual surfaces of the tablets as required, and examples include sugar coated tablets, enteric coating tablets, film-coated tablets, bilayer tablets, and multilayer tablets.

When semi-solid pharmaceutical compositions are prepared, there are used pharmaceutical additives including, for example, animal fats and oils (olive oil, maize oil, castor oil and the like), mineral fats and oils (vaseline, white petrolatum, solid paraffin and the like), waxes (jojoba oil, carnauba wax, beeswax and the like), partially or totally synthesized glycerol fatty acid esters. Examples of commercial products include Witepsol (Dynamit Nobel), Pharmasol (Nippon Oil & Fats) and the like.

When liquid pharmaceutical compositions are prepared, pharmaceutical additives including, for example, sodium chloride, glucose, sorbitol, glycerol, olive oil, propylene glycol, ethyl alcohol and the like can be used. When injections are prepared, sterile liquid media, for example, physiological saline, isotonic solutions, oily liquids such as sesame oil and soybean oil are used. Further, if necessary, suitable suspending agents such as carboxymethylcellulose sodium, nonionic surfactants, solubilizing agents such as benzyl benzoate and benzyl alcohol and the like may be used together. When eye drops are prepared, they can be prepared as aqueous liquids or aqueous solutions. For example, aqueous solutions can be prepared by using a sterile aqueous solution for injections. To these liquids for instillation, various additives such as buffers (borate buffers, acetate buffers, carbonate buffers and the like are preferred in view of reduction of stimulus), isotonic agents (for example, sodium chloride, potassium chloride and the like can be mentioned), preservatives (for example, methyl paraoxybenzoate, ethyl paraoxybenzoate, benzyl alcohol, chlorobutanol and the like can be mentioned), viscosity improvers (for example, methylcellulose, sodium carboxymethylcellulose and the like can be mentioned) and the like may be optionally added. As for preparation of inhalants, when the composition is inhaled as powder, for example, preparation of the aforementioned solid pharmaceutical composition can be referred to, and the obtained powder is preferably further pulverized. Further, when the composition is inhaled as a liquid, preferable methods include a method of preparing the pharmaceutical composition by referring to the aforementioned preparation of solid pharmaceutical composition to prepare a solid composition and dissolving the solid in distilled water or a suitable solvent to obtain a medicament solution upon use, or a method of preparing the pharmaceutical composition by referring to the aforementioned preparation of liquid pharmaceutical composition to obtain a medicament solution. The size of particles in the aforementioned powder or medicament solution is preferably a particle size suitable for inhalation, and the upper limit of the size is, for example, preferably 100 $\mu$ m or less, more preferably 50 $\mu$ m or less, particularly preferably 10 $\mu$ m or less. The lower limit of the particle size is not particularly limited, and a smaller particle size is more preferred.

A content of the active ingredient in the aforementioned pharmaceutical composition can be suitably chosen depending on a dosage form. Although the lower limit of the content of the active ingredient is not particularly limited so long as the effect of the present invention is exhibited,the limit is preferably 0.0001% by weight or more, 0.001% by weight or more, 0.01% by weight or more, 0.1% by weight or more, or 1% by weight or more, based on the total weight of the composition. Although the upper limit of the content of the active ingredient is not also particularly limited so long as the effect of the present invention is exhibited, the limit is preferably 100% by weight or less, 80% by weight or less,

50% by weight or less, 10% by weight or less, 5% by weight or less, 1% by weight or less, or 0.1% by weight or less. A dose of the medicament of the present invention can be suitably determined for each administration in consideration of the age, weight, sexuality of a patient, the type of a disease, the severity of pathological condition and the like. The lower limit is, for example, preferably 0.01 mg or more, 0.1 mg or more, or 1 mg or more. The upper limit is, for example, preferably 1000 mg or less, 500 mg or less, 100 mg or less, or 30 mg or less. These doses can be administered once in a day or several times a day as divided portions.

(i) The compound represented by the aforementioned formula (1) or a salt thereof mentioned above can be suitably used in combination with one or more other drugs.

Further, (ii) the compound or a salt thereof according to 1 wherein the groups of $R^1$, $R^5$, $R^6$, $R^7$, $R^8$, and $A^{61}$ are constituted by a combination of $R^1$ being hydrogen atom, chlorine atom, or hydroxyl group, $R^5$, $R^6$, $R^7$, and $R^8$ being hydrogen atoms, and $A^{61}$ being hydrogen atom, an alkyl group, an alkyl group substituted with an aryl group, an alkyl group substituted with carboxyl group, an alkyl group substituted with cyano group, an alkyl group substituted with hydroxyl group, an alkyl group substituted with an alkoxy group, an alkyl group substituted with amino group, an alkyl group substituted with an alkylamino group, an alkyl group substituted with aminocarbonyl group, or an alkyl group of which end is substituted with $N(A^7)(-X^3-A^{71})$ (where $-X^3-$ is carbonyl group, and $A^{71}$ is an alkyl group, or an alkyl group substituted with an aryl group) can also be used in combination with one or more other drugs.

A drug used for the combination with the compound (i) or (ii) is hereinafter referred to as a jointly-used drug. As the jointly-used drug, for example, drugs in various molecular forms such as low molecular weight compounds, low molecular peptides, polypeptides, nucleic acid oligomers, peptide-nucleic acid (PNA) oligomers, and antibodies can be used, and the drug can be chosen depending on administration object, administration route, objective disease and the like from various drugs of which objective diseases are diseases relating to contraction of various cells, diseases relating to morphological change of various cells, diseases relating to migration of various cells, diseases relating to release from various cells, diseases relating to aggregation of various cells, diseases relating to apoptosis of various cells, diseases relating to abnormal gene expression in various cells and the like (provided that (i) and (ii) are excluded).

A medicament characterized by comprising (i) and a jointly-used drug in combination may sometimes be more preferred compared with a pharmaceutical composition comprising (i) solely as an active ingredient. Such characteristic feature can be understood by any kind of more preferred result provided by a medicament comprising (i) and a jointly-used drug in combination compared with a pharmaceutical composition comprising solely (i) in any test method described below. In the following explanations, explanations for a medicament comprising (i) and a jointly-used drug is also applied to a medicament comprising (ii) and a jointly-used drug, and for that purpose, (i) can be read as (ii).

The following test methods are examples for indicating that usefulness of a medicament based on the aforementioned combination can be demonstrated on the basis of the prophylactic and/or therapeutic effect on, for example, glaucoma, chronic obstructive pulmonary disease, bronchial asthma, and spinal cord injury, and the methods are not intended to indicate that the usefulness of the medicament based on the combination is limited to these applications.

For example, it can be concluded that a medicament based on the aforementioned combination is useful as a medicament for prophylactic and/or therapeutic treatment of glaucoma. First, usefulness of (i) can be confirmed by a result obtained by evaluation of (i) in the aforementioned test methods. Further, usefulness of a medicament based on the combination, such as enhancement of an intraocular pressure reducing action, and extension of duration of an intraocular pressure reducing action, can be confirmed by a result obtained by evaluating each compound combined with each jointly-used drug, for example, a prostaglandin-relating agent such as isopropylunoprostone and latanoprost, a carbonic anhydrase inhibitor such as dorzolamide hydrochloride, brinzolamide hydrochloride, and acetazolamide hydrochloride, an adrenergic receptor blocker such as bunazosin hydrochloride, timolol maleate, carteolol hydrochloride, befunolol hydrochloride, betaxolol hydrochloride and nipradilol hydrochloride.

It can also be concluded that a medicament based on the aforementioned combination is useful as a medicament for prophylactic and/or therapeutic treatment of chronic obstructive pulmonary disease (COPD). First, usefulness of (i) can be confirmed by a result obtained by evaluation of (i) in the aforementioned test methods. Further, higher usefulness of a medicament based on the combination can be confirmed by a result obtained by evaluating each compound combined with each jointly-used drug, for example, a β -adrenergic receptor stimulant such as salbutamol sulfate, terbutaline sulfate, fenoterol hydrobromide, formoterol fumarate, and salmeterol xinafoate, an anticholinergic agent such as tiotropium bromide, ipratropium bromide, and oxitropium bromide, a xanthine derivative such as theophylline and aminophylline, a steroid such as beclometasone dipropionate, fluticasone propionate, and budesonide,.

Further, it can be concluded that a medicament based on the aforementioned combination is useful as a medicament for prophylactic and/or therapeutic treatment of bronchial asthma. First, usefulness of (i) can be confirmed by a result obtained by evaluation of (i) in the aforementioned test methods. Further, higher usefulness of a medicament based on the combination can be confirmed by a result obtained by evaluating each compound combined with each

jointly-used drug, for example, a β -adrenergic receptor stimulant such as salbutamol sulfate, terbutaline sulfate, fenoterol hydrobromide, formoterol fumarate, and salmeterol xinafoate, a steroid such as beclometasone dipropionate, fluticasone propionate, and budesonide, a lipid mediator inhibitor such as seratrodast, ramatroban, ozagrel hydrochloride, pranlukast, montelukast sodium, and zafirlukast and the like.

Furthermore, it can be concluded that a medicament based on the aforementioned combination is useful as a medicament for prophylactic and/or therapeutic treatment of spinal cord injury. First, usefulness of (i) can be confirmed by a result obtained by evaluation of (i) in the aforementioned test methods. Further, higher usefulness of a medicament based on the combination can be confirmed by a result obtained by evaluating each compound combined with each jointly-used drug, for example, a steroid such as methylprednisolone succinate, a cannabinoid such as cannabinol and tetrahydrocannabinol, 4-aminopyridine and the like.

The aforementioned medicament based on the combination include, for example, a medicament comprising (i) and a jointly-used drug which are simultaneously administered, and a medicament comprising (i) and a jointly-used drug which are administered with an interval period within which each efficacy is expected. Further, a medicament prepared in a single form in which (i) and a jointly-used drug are mixed, and a medicament comprising (i) and a jointly-used drug which are prepared in separate forms are also included. Furthermore, a medicament wherein (i) and a jointly-used drug are administered via the same route, and a medicament wherein (i) and a jointly-used drug are separately administered are also included.

In the aforementioned medicament based on the combination, a mixing ratio of (i) and a jointly-used drug, a form of (i) and a jointly-used drug after mixing wherein (i) and the jointly-used drug are prepared in a single form and the like can be suitably determined depending on a purpose of administration, an administration route, a disease to be treated, symptoms, physicochemical properties of the drug, easiness of administration and the like, and a dose thereof can be suitably chosen on the basis of, for example, clinically used doses of (i) and the jointly-used drug.

When (i) and a jointly-used drug are prepared in a single form, the aforementioned pharmaceutical additives may be used in addition to (i) and the jointly-used drug to prepare a pharmaceutical composition, and a preferred form such as oral agents, injections (solution, suspension and the like), fusion drips, inhalants, suppositories, transdermally absorbed agents (e.g., tapes), ointments, ophthalmic solutions, ophthalmic ointments, ophthalmic membrane adherent agents and the like can be prepared and used.

When (i) and a jointly-used drug are prepared in separate forms, each of (i) and the jointly-used drug can be prepared in a preferred from in the same manner as mentioned above and used.

The medicament characterized by comprising (i) and a jointly-used drug in combination can be used as an agent for prophylactic and/or therapeutic treatment of various diseases. The diseases are preferably those relating to contraction of various cells, and among the diseases relating to contraction of various cells, preferred examples include glaucoma, bronchial asthma, or chronic obstructive pulmonary disease. Further, any kind of the medicament characterized by comprising (i) and a jointly-used drug in combination is also preferred as an agent for prophylactic and/or therapeutic treatment of diseases relating to migration of various cells, and the disease is also preferably bronchial asthma, or chronic obstructive pulmonary disease among the diseases relating to migration of various cells. Further, any kind of the medicament characterized by comprising (i) and a jointly-used drug in combination is also preferred as an agent for prophylactic and/or therapeutic treatment of diseases relating to morphological change of various cells. Among the diseases relating to morphological change of various cells, the disease is more preferably a neurological disorder, and the neurological disorder is more preferably spinal cord injury.

The following [1] to [32] are encompassed within the scope of the present invention.

[1] A medicament comprising (i) and a drug having an intraocular pressure reducing action and/or a drug having an optic nerve protective action in combination.

[2] A medicament comprising (i) and any one or more of an adrenergic receptor stimulant, a prostaglandin-related agent, a carbonic anhydrase inhibitor, an adrenergic receptor blocker, a cholinesterase inhibitor, a calcium antagonist, a Rho kinase inhibitor, an angiotensin II receptor antagonist, and an NMDA receptor blocker in combination.

[3] A medicament comprising (i) and a drug having an intraocular pressure reducing action in combination.

[4] A medicament comprising (i) and any one or more of a prostaglandin-related agent, a carbonic anhydrase inhibitor, an adrenergic receptor blocker, and an NMDA receptor blocker.

[5] The medicament according to [4], which is an agent for prophylactic and/or therapeutic treatment of glaucoma.

[6] A medicament comprising (i) and a drug having an optic nerve protective action in combination.

[7] The medicament according to [4] wherein the prostaglandin-related agent mentioned in [4] is any one of latanoprost, bimatoprost, travoprost, isopropylunoprostone, and tafluprost.

[8] The medicament according to [4] wherein the carbonic anhydrase inhibitor mentioned in [4] is any one of dorzolamide hydrochloride, brinzolamide hydrochloride, and acetazolamide hydrochloride.

[9] The medicament according to [4] wherein the adrenergic receptor blocker mentioned in [4] is any one of bunazosin hydrochloride, timolol maleate, carteolol hydrochloride, levobunolol hydrochloride, betaxolol hydrochloride, nipradilol hydrochloride, and befunolol hydrochloride.

[10] A method for therapeutic and/or prophylactic treatment of glaucoma, which uses the medicament according to any one of [1] to [9].

[11] A medicament comprising (i) and a drug having a tracheal dilational action and/or an anti-inflammatory action in combination.

[12] A medicament comprising (i) and any one of a β 2- adrenergic receptor stimulant, an NK antagonist, a xanthine derivative, an anticholinergic agent, a phosphodiesterase inhibitor, a steroid, or an elastase inhibitor in combination.

[13] A medicament comprising (i) and any one of a β 2- adrenergic receptor stimulant, a xanthine derivative, an anticholinergic agent, and a steroid in combination.

[14] The medicament according to [13] wherein the medicament mentioned in [13] is an agent for prophylactic and/or therapeutic treatment of chronic obstructive pulmonary disease.

[15] A medicament comprising (i) and an antibiotic in combination.

[16] A medicament comprising (i) and an expectorant in combination.

[17] The medicament according to [13] wherein the β 2- adrenergic receptor stimulant mentioned in [13] is any one of salbutamol sulfate, terbutaline sulfate, fenoterol hydrobromide, formoterol fumarate, and salmeterol xinafoate.

[18] The medicament according to [13] wherein the xanthine derivative mentioned in [13] is any one of theophylline, and aminophylline.

[19] The medicament according to [13] wherein the anticholinergic agent mentioned in [13] is any one of tiotropium bromide, ipratropium bromide, and oxitropium bromide.

[20] The medicament according to [13] wherein the steroid mentioned in [13] is any one of beclometasone dipropionate, fluticasone propionate, and budesonide.

[21] A method for therapeutic and/or prophylactic treatment of chronic obstructive pulmonary disease, which uses the medicament according to any one of [10] to [20].

[22] A medicament comprising (i) and a steroid in combination.

[23] A medicament comprising (i) and a cannabinoid in combination.

[24] A medicament comprising (i) and a potassium channel blocker in combination.

[25] A medicament comprising (i) and a hypoxanthine derivative in combination.

[26] The medicament according to any one of [22] to [25] wherein the medicament according to any one of [22] to [25] is an agent for therapeutic and/or prophylactic treatment of spinal cord injury.

[27] The medicament according to [22] wherein the steroid mentioned in [22] is methylprednisolone succinate.

[28] The medicament according to [23] wherein the cannabinoid mentioned in [23] is any one of cannabinol, tetrahydrocannabinol, and a physiologically acceptable salt thereof.

[29] The medicament according to [24] wherein the potassium channel blocker mentioned in [24] is either 4-aminopyridine, or a physiologically acceptable salt thereof.

[30] The medicament according to [25] wherein the hypoxanthine derivative mentioned in [25] is leteprinim potassium.

[31] A medicament comprising (i) and a drug having a central nerve regeneration action in combination.

[32] A method for therapeutic and/or prophylactic treatment of spinal cord injury, which uses the medicament according any one of [22] to [31].

The inventions of [1] to [32] mentioned above wherein (i) is replaced with (ii) are also fall within the scope of the present invention, and all of these inventions are preferred.

The compound (i) has an intraocular pressure reducing action as demonstrated in Test Example 4, and it has been confirmed that the medicaments of [1], [2], and [3] mentioned above induce enhancement of the intraocular pressure reducing action, extension of duration of the intraocular pressure reducing action and the like compared with a pharmaceutical composition containing (i) alone as an active ingredient as demonstrated in Test Example 13. Thus, they are preferred as agents for therapeutic and/or prophylactic treatment of glaucoma.

Further, a medicament comprising (i) and an agent for therapeutic and/or prophylactic treatment of glaucoma in combination is also preferred. As the agent for therapeutic and/or prophylactic treatment of glaucoma, for example, drugs having an intraocular pressure reducing action, drugs having an optic nerve protective action, drugs having an intraocular

pressure reducing action, and/or an optic nerve protective action and the like are known.
Among the jointly-used drugs according to the present invention, the drug having an intraocular pressure reducing action is not particularly limited so long as the drug has an intraocular pressure reducing action. Examples include adrenergic receptor stimulants, prostaglandin-related agents, carbonic anhydrase inhibitors (also abbreviated as CAI), adrenergic receptor blockers, cholinesterase inhibitors, calcium antagonists (AI Report, Cima Science Journal, 2002), Rho kinase inhibitors (Honjo, M. et al., Invest. Ophthalmol. Vis. Sci., 42 (1), pp.137-44 (2001); Honjo, M. et al., Arch. Ophthalmol. 119 (8), pp.1171-8 (2001)), angiotensin II receptor antagonists (Inoue, T. et al., Current Eye Res., 23 (2), pp. 133-8 (2001)) and the like, and any one or more of these drugs can be used. When two or more kinds of jointly-used drugs (the same shall apply to (i) and (ii)) are selected, two or more kinds of the drugs may be selected from drugs belonging to the same classification, or one or more kind of the drugs may be selected from drugs belonging to each of different classifications. The drugs are preferably selected from those belonging to different classifications. Further, as the drug having an intraocular pressure reducing action, one or more kinds of drugs among prostaglandin-related agents, carbonic anhydrase inhibitors, and adrenergic receptor blockers are preferred. This medicament is preferred as an agent for therapeutic and/or prophylactic treatment of glaucoma.

Among the jointly-used drugs used in the present invention, the drug having an optic nerve cell protective action means a drug having an action of protecting optic nerves, and examples include drugs having an action of protecting optic nerve cells based on an action of suppressing cell death as well as an action of improving eyeground vascular flow. The action of suppressing cell death of optic nerve cells can be confirmed as an action of suppressing cell death induced via a glutamate receptor in an exo vivo culture system using retinal nerve cells extracted from a rat or the like by adding NMDA (Hahn et al., Proc. Natl. Acad. Sci. USA, 85, 6556. (1998)), and the action of improving the eyeground vascular flow can be confirmed by, for example, quantitatively analyzing change of the eyeground vascular flow in human, rabbit, monkey, or the like administered with the drug using the laser speckle method (Tamaki, Y. et al., Surv. Ophthalmol., 42 (Suppl. 1), S52-S63. (1997)).
Examples of the drug having an optic nerve protective action include adrenergic receptor stimulants (Wheeler, LA. et al., Eur. J. Ophthalmol., 11 (Suppl. 2) 403-11. (2001)), adrenergic receptor blockers (Wood, JP., et al., Exp. Eye Res. 76 (4), 505-16. (2003) and the like), calcium antagonists (Toriu, N. et al., Exp. Eye Res., 70 (4)), 475-84. (2000)), NMDA receptor blockers (Kim, TW. et al., Korean J. Ophthalmol., 16 (1), 1-7. (2002)), prostaglandin related agents (Tamaki, Y. et al., and J. Ocul. Pharmacol. Ther., 17 (5), 403-11. (2001) and the like), carbonic anhydrase inhibitors (Harris, A. et al., J. Ocul. Pharmacol. Ther., 15,189-197. (1999)), angiotensin II receptor antagonists (Inoue, T. et al., Ophthalmic Res., 35, pp.351-4. (2003)) and the like, and any one or more of these drugs can be used.

Moreover, besides drugs having either of the intraocular pressure reducing action and the optic nerve protective action, drugs both having the intraocular pressure reducing action and the optic nerve protective action as a single agent are also known. Examples of such drugs include, for example, adrenergic receptor stimulants, prostaglandin-related agents, carbonic anhydrase inhibitors, adrenergic receptor blockers, calcium antagonists and the like, and any one or more these drugs may be used.
Specifically, among the jointly-used drugs used for the present invention, the drugs having an intraocular pressure reducing action and/or drugs having an optic nerve protective action can be divided into the aforementioned drugs having an intraocular pressure reducing action, drugs having an optic nerve protective action, and drugs having both of an intraocular pressure reducing action and an optic nerve protective action as a single agent. Examples of the drugs having an intraocular pressure reducing action and/or drugs having an optic nerve protective action include, for example, one or more of adrenergic receptor stimulants, prostaglandin-related agents, carbonic anhydrase inhibitors, adrenergic receptor blockers, cholinesterase inhibitors, calcium antagonists, Rho kinase inhibitors, angiotensin II receptor antagonists, NMDA receptor blockers and the like. Furthermore, preferred examples include one or more of prostaglandin-related agents, carbonic anhydrase inhibitors, adrenergic receptor blockers, NMDA receptor blockers and the like.

In the specification, the adrenergic receptors include, for example, those of α 1A-subtype considered to be involved in control of constriction and relaxation of various smooth muscles, α 1B-subtype, α 1D-subtype, α 2A-subtype, α 2B-subtype, α 2C-subtype, β 1-subtype, β 2-subtype, and β 3-subtype (Kurose et al., Protein Nucleic Acid Enzyme, Vol. 42, No. 3, pp.316-26. (1997)).
In the specification, the adrenergic receptor stimulants means a medicament that acts as an agonist against at least one of the aforementioned adrenergic receptors , and has an regulatory action on various smooth muscles. Examples of the regulatory action on various smooth muscles include, for example, an intraocular pressure reducing action, and a tracheal dilational action. The medicaments herein refer to mean those exhibiting an intraocular pressure reducing action. Depending on the degree of selectivity for the adrenergic receptors, for example, those selectively acting on α -receptors may be described as α -stimulants, those selectively acting on α 2-receptors may be described as α 2-stimulants and the like.
In the present invention, examples of the adrenergic receptor stimulants include, for example, non-selective sympathetic nerve stimulants, α 1-stimulants, and α 2-stimulants. Epinephrine, dipivefrin (U.S. Patent No. 3,809,714), apraclonidine (U.S. Patent No. 4,517,199), brimonidine (U.S. Patent No. 4,517,199), and physiologically acceptable salts thereof are

preferred, and epinephrine hydrochloride, dipivefrin hydrochloride, apraclonidine, and brimonidine tartrate are preferred. Any one of these drug is preferred, or any two or more are also preferred.

The prostaglandin-related agents are roughly classified into prostaglandin receptor (FP receptor) binding prostaglandins (Richard, MB.et al., Annu. Rev.Pharmacol. Toxicol., 41, pp.661-90. (2001)), and metabolic type prostaglandins. In addition, derivatives thereof such as isopropylunoprostone can be exemplified. The prostaglandin receptor (FP receptor) binding prostaglandins are prostaglandins having an ability to bind with an FP receptor, and generally, they are often naturally-occurring prostaglandins or compounds having similar structures. The prostaglandin-related agents preferably further exhibit an intraocular pressure reducing action, and preferably have a curative effect for glaucoma. Examples of naturally occurring prostaglandins include, for example, prostaglandin F 2 α and the like. Examples of the FP receptor binding prostaglandins having a structure similar to that of naturally occurring prostaglandins include, for example, latanoprost, travoprost, bimatoprost, tafluprost and the like.

The metabolic type prostaglandins mean compounds produced by metabolism of the FP receptor binding prostaglandins in the living bodies.

Therefore, in the present invention, as the prostaglandin-related agents, isopropylunoprostone (U.S. Patent No. 5,627,209), latanoprost (U.S. Patent No. 5,296,504), travoprost (U.S. Patent No. 5,510,383), bimatoprost (U.S. Patent No. 6,403,649), tafluprost, and physiologically acceptable salts thereof are preferred, and isopropylunoprostone, latanoprost, travoprost, bimatoprost, and tafluprost are preferred. Any one of these drugs is preferred, or any two or more are also preferred.

In the specification, the carbonic anhydrase inhibitors mean isozymes of carbonic anhydrase having at least type II and/or type IV enzyme inhibitory action. Examples of the carbonic anhydrase inhibitors include, for example, dorzolamide (European Patent Publication No. 296879), brinzolamide (U.S. Patent No. 5,378,703), acetazolamide (U.S. Patent No. 2,554,816), and physiologically acceptable salts thereof, and dorzolamide hydrochloride, brinzolamide hydrochloride, and acetazolamide hydrochloride are preferred. Any one of these drugs is preferred, or any two or more are also preferred.

In the specification, the adrenergic receptors are the same as those mentioned above. The adrenergic receptor blockers means drugs that act as an antagonist against at least one adrenergic receptor, and have an action of regulating various smooth muscles. The action of regulating various smooth muscles preferably means an action exhibiting an intraocular pressure reducing action. Depending on the degree of selectivity for adrenergic receptors, they may be described as α -blockers, β -blockers, or α β -blockers. Examples of the adrenergic receptor blockers include, for example, α -blockers, β -blockers, and α β -blockers, bunazosin (British Patent Application Publication No. 1398455), timolol (U.S. Patent No. 5,354,860), carteolol (U.S. Patent No. 3,953,456), befunolol (U.S. Patent No. 4,515,977), betaxolol (U.S. Patent No. 4,252,984), nipradilol (Japanese Patent Publication (KOKOKU) No. 60-54317), Levobunolol (U.S. Patent No. 5,426,227), and physiologically acceptable salts thereof are preferred, and bunazosin hydrochloride, timolol maleate, carteolol hydrochloride, befunolol hydrochloride, betaxolol hydrochloride, and nipradilol hydrochloride are also preferred. Any one of these drugs is preferred, or any two or more are also preferred.

Examples of the cholinesterase inhibitors include, for example, demecarium, physostigmine (U.S. Patent No. 4,791,107), echothiophate, and physiologically acceptable salts thereof, and physostigmine sulfate and the like are preferred. Any one of these drugs is preferred, or any two or more are also preferred.

Examples of the calcium antagonists include, iganidipine (U.S. Patent No. 2,554,.16), lomerizine (Japanese Patent Unexamined Publication (KOKAI) No. 60-222472), and physiologically acceptable salts thereof, and iganidipine hydrochloride, and lomerizine hydrochloride are also preferred. Any one of these drugs is preferred, or any two or more are also preferred.

Examples of the Rho kinase inhibitors include, for example,
(+)-trans-4-(1-aminoethyl)-1-(4-pyridylcarbamoyl)cyclohexane (Y-27632),
(+)-trans-N-(pyrrolo[1H[2,3-b]pyridin-4-yl)-4-(1-aminoethyl)cyclohexanecarboxamide,
(R)-(+)-N-(4-pyridyl)-4-(1-aminoethyl)benzamide,
(R)-(+)-N-(1H-pyrrolo[2,3-b]pyridin-4-yl)-4-(1-aminoethyl)benzamide (Y-39983) (International Patent Publication WO02/083175), HA-1077 (Nagumo, H. et al., Am. J. Physiol. Cell Physiol., 278 (1) pp.C57-65. (2000)), and physiologically acceptable salts thereof, and Y-27632 hydrochloride, Y-39983 hydrochloride, and HA-1077 hydrochloride are preferred. Any one of these drugs is preferred, or any two or more are also preferred.

In the specification, the angiotensin II receptors include those of $AT_{1A}$, $AT_{1B}$, $AT_2$, $AT_3$, and $AT_4$ subtypes, which bind to angiotensin II to be involved in blood pressure regulating action and the like, and the angiotensin II receptor antagonists mean drugs having a binding activity at least to $AT_1$ receptor, which antagonizes against angiotensin II, to attenuate the action of angiotensin II. Examples of the angiotensin II receptor antagonists include, for example, olmesartan (CS-088) (U.S. Patent No. 5,616,599), and physiologically acceptable salts thereof, and olmesartan is preferred. Any one of these drugs is preferred, or any two or more are preferred.

Examples of the NMDA receptor antagonists include, for example, memantine (U.S. Patent No. 4,122,193), and physiologically acceptable salts thereof, and memantine hydrochloride is preferred. Any one of these drugs is preferred, or

any two or more are also preferred.
Moreover, a method for therapeutic and/or prophylactic treatment of glaucoma, which uses any of the medicaments mentioned above is also preferred.

When the aforementioned medicaments are used as instillation, one to ten drops, preferably one or two drops (the volume of one drop is about 50 μL), as a dose for one administration, can be preferably administered about 1 to 6 times a day.
When the object of the administration is human, for example, the jointly-used drug is generally used preferably in an amount of 0.001 to 100 parts by weight with 1 weight part of (i). More specifically, although the lower limit of the amount of the jointly-used drug is not particularly limited so long as the effect of the present invention is exhibited, the jointly-used drug is used in an amount of, for example, 0.00001 part by weight or more, preferably 0.0001 part by weight or more, more preferably 0.001 part by weight or more, further preferably 0.01 part by weight or more, or 0.1 part by weight or more, or 0.5 part by weight or more, based on 1 part by weight of (i). Although the upper limit of the amount of the jointly-used drug is not also particularly limited, the jointly-used drug is generally used in an amount of 100 parts by weight or less, preferably 50 parts by weight or less, more preferably 25 parts by weight or less, or 10 parts by weight or less, or 5 parts by weight or less.

As for chronic obstructive pulmonary disease, which is one of the diseases relating to constriction of various cells, or diseases relating to migration of various cells, (i) has a respiratory tract constriction suppressing action as shown in Test Example 3, neutrophil migration inhibitory action as shown in Test Example 6, respiratory tract inflammation suppressing action as shown in Test Example 7, and lung inflammation suppressing action as shown in Test Example 8, and therefore the medicaments of [11] and [12] mentioned above are preferred as a therapeutic and/or prophylactic treatment of chronic obstructive pulmonary disease.
Among the jointly-used drugs used for the present invention, examples of the drugs having a tracheal dilational action include, for example, β 2-adrenergic receptor stimulants, neurokinin (also abbreviated as NK) antagonists, xanthine derivatives, anticholinergic agents, phosphodiesterase inhibitors (Masakazu Ichinose, Respiratory Disease New Approach 8, Obstructive Pulmonary Diseases, MEDICAL VIEW, 2002), Rho kinase inhibitors (Iizuka, K. et al., Eur. J. Pharmacol., 406 (2), pp.273-9. (2000)); Japanese Patent Unexamined Publication (KOKAI) No. 03-258763), leukotriene antagonists and the like, and any of one or more of these drugs can be used. A medicament comprising (i) and any one or two of drugs among a β 2 adrenergic receptor stimulant, an NK antagonist, a xanthine derivative, an anticholinergic agent, and a phosphodiesterase inhibitor in combination is also preferred.

In the present invention, the anti-inflammatory action means an action of improving inflammation image of chronic obstructive pulmonary disease, and examples include, for example, suppression of infiltration of leucocytes such as neutrophiles and mast cells into the lung (Gizycki, ML. et al., Thorax, 57 (9), 799-803. (2002)), suppression of degradation of tissue elastin or collagen (Culpitt, SV. et al., Am. J. Respir. Crit. Care Med., 165, 1371-76 (2002)), suppression of production of cytokines such as TNF- α, IL-8, and GM-CSF (Profita, M. et al., Thorax, 58(7), 573-9 (2003) ) and the like. Examples of the drugs having an anti-inflammatory action include, for example, steroids, xanthine derivatives, phosphodiesterase inhibitors, elastase inhibitors and the like, and any one or more of these drugs can be used. A medicament comprising (i) and any one or more drugs among a steroid, a xanthine derivative, a phosphodiesterase inhibitor, and an elastase inhibitor is also preferred.
Moreover, besides the drugs having either one of the tracheal dilational action and the anti-inflammatory action, drugs having both a tracheal dilational action and an anti-inflammatory action as a single agent are also known. Examples of such drugs include xanthine derivatives, and phosphodiesterase inhibitors, and any one or two or more of these drugs can be used.

Specifically, in the present invention, the drugs having a tracheal dilational action and/or an anti-inflammatory action can be divided into the aforementioned drugs having a tracheal dilational action, drugs having an anti-inflammatory action, and drugs having both a tracheal dilational action and an anti-inflammatory action as a single agent. As such drugs, for example, any one or more of a β2-adrenergic receptor stimulant, an NK antagonist, a xanthine derivative, an anticholinergic agent, a phosphodiesterase inhibitor, a Rho kinase inhibitors, a steroid, an elastase inhibitor, and a leukotriene receptor antagonist can be used, and any one or more of a β2. adrenergic receptor stimulant, a xanthine derivative, an anticholinergic agent, a phosphodiesterase inhibitors, a steroid, and a leukotriene receptor antagonist are preferably used. These medicaments are preferred as a therapeutic and/or prophylactic treatment of chronic obstructive pulmonary disease.

In the present invention, the medicaments of [15] and [16] mentioned above are also preferred as a therapeutic and/or prophylactic treatment of chronic obstructive pulmonary disease from another aspect.
In the present invention, examples of preferred β 2-adrenergic receptor stimulants include, for example, salbutamol, terbutaline, fenoterol, formoterol, salmeterol (International Patent Publication WO95/01324), and physiologically acceptable salts thereof, and salbutamol sulfate, terbutaline sulfate, fenoterol hydrobromide, formoterol fumarate, and salmeterol xinafoate are also preferred. Any one of these drugs is preferred, or any two or more are also preferred.
In the present invention, preferred examples of the NK antagonists include, for example, talnetant, and physiologically

acceptable salts thereof, and talnetant and the like are also preferred (U.S. Patent No. 5,811,553). Any one of these drugs is preferred, or any two or more are also preferred.

In the specification, the xanthine derivatives means structural analogues of xanthine. Examples of preferred xanthine derivatives include, for example, theophylline, aminophyllines, and physiologically acceptable salts thereof, and theophylline, and aminophylline are preferred. Any one of these drugs is preferred, or any two or more are also preferred.

In the specification, the anticholinergic agents means agents that acts as an antagonist against at least the M3 subtype among the three subtypes (M1, M2, M3) of muscarine receptors, and exhibit a tracheaectasy action (Masakazu Ichinose, Respiratory Disease New Approach 8, Obstructive Pulmonary Disease, pp.68-76, 2003, Medical View). Examples of preferred anticholinergic agents include, for example, tiotropium (U.S. Patent No. 5,610,163), ipratropium (U.S. Patent No. 3,505,337), oxitropium (Banholzer, VR. et al., Arzneimittelforschung, 35 (1A) 217-28. (1985)), and pharmacologically acceptable salts thereof. Any one of these drugs is preferred, or any two or more are also preferred. Examples of the aforementioned physiologically acceptable salts include tiotropium bromide, ipratropium bromide, and oxitropium bromide (Ba253).

In the present invention, as steroids used as agents for therapeutic and/or prophylactic treatment of chronic obstructive pulmonary disease, steroids that can be prepared as inhalants are preferred. As such steroids, for example, beclometasone, fluticasone (British Patent No. 2088877), budesonide (Japanese Patent Unexamined Publication (KOKAI) No. 2000-128897), and physiologically acceptable salts thereof are preferred, and beclometasone dipropionate, fluticasone propionate, and budesonide are preferred. Any one of these drugs is preferred, or any two or more are also preferred.

In the present invention, preferred examples of the PDE inhibitors include, for example, PDE4 inhibitors, and preferred examples include, for example, cilomilast (Griswold, DE. et al., J. Pharmacol. Exp. Ther., 287 (2), 705-11. (1998)), roflumilast (Bundschuh, DS. et al., J. Pharmacol. Exp. Ther., 297 (1) 280-90. (2001), arophylline (European Patent Publication No. 0435811), and physiologically acceptable salts thereof. Furthermore, preferred examples include cilomilast, roflumilast, and arophylline. Any one of these drugs is preferred, or any two or more are also preferred.

In the present invention, preferred examples of the elastase inhibitors include, for example, sivelestat (Kawabata, K. et al., Biochem. Biophys. Res. Commun., 177 (2), 814-20. (1991), midesteine (MR889) (Baici, A. et al., Biochem. Pharmacol., 39 (5), 919-24 (1990)), physiologically acceptable salts thereof and the like. Sivelestat sodium, and midesteine are also preferred. Any one of these drugs is preferred, or any two or more are also preferred.

In the present invention, the leukotriene receptor antagonist will be described later.

In the present invention, as the antibiotics, for example, macrolide antibiotics are preferred, and preferred examples include erythromycin and the like.

In the present invention, as the expectorants, for example, N-acetylcysteine and the like are preferred.

When the aforementioned medicaments are used as an inhalant, they can be administered about 1 to 6 times a day by spraying a spraying amount once to 10 times, preferably once or twice, for each administration. When an object of the administration is a human, for example, (i) is generally preferably used in an amount of 0.0 1 μg to 100 mg per a single spraying. More specifically, although the lower limit of the amount of the jointly-used drug is not particularly limited so long as the effect of the present invention is exhibited, the jointly-used drug is used in an amount of, for example, 0.0001 μg or more, preferably 0.001 μg or more, more preferably 0.01 μg or more, or 0.1 μg or more, or 0.5 μg or more. Although the upper limit of the amount of the jointly-used drug is not also particularly limited, it is generally used in an amount of 100 mg or less, preferably 10 mg or less, more preferably 5 mg or less, or 1 mg or less, or 500 μ g or less.

Further, when the medicament is used as, for example, an oral agent or an injection, a single dose can be administered about once to 10 times a day.

When an object of the administration is a human, for example, (i) is generally used preferably in an amount of 0.0001 to 5000 mg/kg at one time. More specifically, although the lower limit of the amount of the jointly-used drug is not particularly limited so long as the effect of the present invention is exhibited, the jointly-used drug is used in an amount of, for example, 0.000001 mg/kg or more, preferably 0.00001 mg/kg or more, more preferably 0.0001 mg/kg or more, or 0.001 mg/kg or more, or 0.01 mg/kg or more, or 0.1 mg/kg or more. Although the upper limit of the amount of the jointly-used drug is not also particularly limited, it is generally used in an amount of 5000 mg/kg or less, preferably 1000 mg/kg or less, more preferably 100 mg/kg or less, or 50 mg/kg or less, or 20 mg/kg or less.

As for bronchial asthma, which is one of the diseases relating to constriction of various cells, or the diseases relating to migration of various cells, (i) has a respiratory tract constriction suppressing action as shown in Test Example 3, neutrophil migration inhibitory action as shown in Test Example 6, and respiratory tract inflammation suppressing action as shown in Test Example 7. Therefore, a medicament comprising (i) and any one or more of a β 2-adrenergic receptor stimulant, an anticholinergic agent, a Rho kinase inhibitor, a steroid, a xanthine derivative, a phosphodiesterase inhibitor, a chemical mediator release inhibitor, an antihistamine, a lipid mediator inhibitor, a leukotriene receptor antagonist, and a Th2 cytokine production inhibitor in combination is preferred as agent for therapeutic and/or prophylactic treatment of bronchial asthma, and the medicaments of [13] and [15] to [20] mentioned above are preferred as agents

for therapeutic and/or prophylactic treatment of bronchial asthma.
In the present invention, as the β 2- adrenergic receptor stimulants, anticholinergic agents, Rho kinase inhibitors, steroids, xanthine derivatives, phosphodiesterase inhibitors, and leukotriene receptor antagonists, for example, those containing the same active ingredient as that of those used for therapeutic and/or prophylactic treatment of chronic obstructive pulmonary disease may be used. Any one of these drugs is preferred, or any two or more are also preferred.

In the specification, the chemical mediator means, for example, a substance which is intracellularly stored in mast cells and/or basophiles, and released upon a stimulation to the cells to cause various phenomena such as increased vascular permeability, contraction of various smooth muscles, increased mucous secretion, and increased migration of immune cells, a substance which is produced from a lipid such as arachidonic acid, and released upon a stimulation to the cells to cause various phenomena such as increased vascular permeability, contraction of various smooth muscles, increased mucous secretion, and increased migration of immune cells (lipid mediator), or the like. Examples of the former substances include histamine, serotonin, leukotriene and the like, and examples of the latter include leukotrienes, thromboxane, prostaglandin D2, platelet activating factor (PAF) and the like (Ogura T. et al., Key Word 1994-'95, Sentan Igaku-Sha, 1994).
In the specification, the chemical mediator release inhibitors mean those inhibiting the release of the chemical mediators stored in mast cells and/or basophiles upon a stimulation to the cells. In the present invention, examples of the chemical mediator release inhibitors include, for example, cromolyn (Japanese Patent Unexamined Publication (KOKAI) No. 62-81380), ibudilast (Japanese Patent Publication (KOKOKU) No. 52-29318), tranilast (Japanese Patent Unexamined Publication (KOKAI) No. 5-097825), pemirolast (Japanese Patent Unexamined Publication (KOKAI) No. 2003-073378), physiologically acceptable salts thereof and the like, and sodium cromoglicate, ibudilast, tranilast, and pemirolast potassium are more preferred. Any one of these drugs is preferred, or any two or more are also preferred.

In the specification, the lipid mediator inhibitors mean drugs which attenuate physiological actions mediated by the lipid mediators by a mechanism of inhibiting a function of an enzyme essential for the biosynthesis of the aforementioned chemical mediators under physiological conditions, or a mechanism of antagonistically inhibiting binding of the lipid mediators to a receptor thereof, and examples include thromboxane-A2 synthetase inhibitors, thromboxane A2 receptor antagonists, leukotriene receptor antagonists and the like.
In the specification, the thromboxane-A2 synthetase inhibitors mean those inhibiting a function of an enzyme catalyzing generation of thromboxane A2 from prostaglandin H2. Further, the thromboxane receptor antagonists mean drugs that antagonistically inhibit binding of thromboxane A2 to thromboxane A2 receptor to attenuate the physiological activities thereof.
In the specification, the leukotriene receptors include those of BLT1, BLT2, CysLT1, and CysLT2 subtypes (Izumi, T. et al., J. Biochem., 132, pp.1-6. (2002)), and the leukotriene receptor antagonists mean drugs that antagonistically inhibit binding of leukotriene $B_4$, leukotriene $C_4$, leukotriene $D_4$, or leukotriene $E_4$ to at least one of the aforementioned receptor subtypes to attenuate the physiological functions thereof. In the present invention, examples of the lipid mediator inhibitors include thromboxane-A2 synthetase inhibitors, thromboxane A2 receptor antagonists, and leukotriene antagonists are preferred, and for example, seratrodast, ramatroban (European Patent No. 242518), ozagrel (U.S. Patent No. 4,226,878), pranlukast (European Patent Publication No. 0173516), montelukast (European Patent No. 480717), zafirlukast (U.S. Patent No. 4,859,692), and physiologically acceptable salts thereof, and seratrodast, ramatroban, ozagrel hydrochloride, pranlukast, montelukast sodium, and zafirlukast are preferred. Any one of these drugs is preferred, or any two or more are also preferred.

In the specification, the antihistamines mean those antagonistically inhibiting binding of histamine to at least the histamine H1 receptor to attenuate the physiological functions thereof. In the present invention, examples of the antihistamines include, for example, epinastine (Japanese Patent Publication (KOKOKU) No. 3-66311), ketotifen, fexofenadine (International Patent Publication WO97/23213), oxatomide (Japanese Patent Publication (KOKOKU) No. 62-31707), cetirizine (British Patent No. 2225321), olopatadine (U.S. Patent No. 5116863), azelastine (Japanese Patent Unexamined Publication (KOKAI) No. 7-215968), bepotastine (Japanese Patent Unexamined Publication (KOKAI) No. 10-237070), emedastine (Japanese Patent Publication (KOKOKU) No. 2-24821), physiologically acceptable salts thereof and the like, and epinastine hydrochloride, ketotifen fumarate, fexofenadine hydrochloride, oxatomide, cetirizine hydrochloride, olopatadine hydrochloride, azelastine hydrochloride, bepotastine besilate, and emedastine difumarate are preferred. Any one of these drugs is preferred, or any two or more are also preferred.
In the specification, Th2 cytokines mean interleukin-4, interleukin-5, interleukin-6, interleukin-10, interleukin-13 and the like, which are produced by type 2 helper T cells, and the Th2 cytokine production inhibitors mean drugs that regulate the production of at least one of the cytokines. Examples of the Th2 cytokine production inhibitors include, for example, suplatast (Japanese Patent Publication (KOKOKU) No. 3-70698), and physiologically acceptable salts thereof, and suplatast tosilate is preferred. Any one of these drugs is preferred, or any two or more are also preferred.

When the aforementioned medicaments are used as an inhalant, they can be administered about 1 to 6 times a day by spraying a spraying amount once to 10 times, preferably once or twice, for a single administration.
When an object of the administration is a human, for example, (i) is generally used preferably for each spraying in an

amount of 0.01 μ g to 100 mg. More specifically, although the lower limit of the amount of the jointly-used drug is not particularly limited so long as the effect of the present invention is exhibited, the jointly-used drug is used in an amount of, for example, 0.0001 μg or more, preferably 0.001 μg or more, more preferably 0.01 μg or more, or 0.1 μg or more, or 0.5 μg or more. Although the upper limit of the amount of the jointly-used drug is not also particularly limited, it is generally used in an amount of 100 mg or less, preferably 10 mg or less, more preferably 5 mg or less, or 1 mg or less, or 500 μ g or less.

Further, when the medicament is used as, for example, an oral agent or an injection, a single dose can be administered about once to 10 times a day.

When an object of the administration is a human, for example, (i) is generally used preferably in an amount of 0.0001 to 5000 mg/kg at one time. More specifically, although the lower limit of the amount of the jointly-used drug is not particularly limited so long as the effect of the present invention is exhibited, the jointly-used drug is used in an amount of, for example, 0.000001 mg/kg or more, preferably 0.00001 mg/kg or more, more preferably 0.0001 mg/kg or more, or 0.001 mg/kg or more, or 0.01 mg/kg or more, or 0.1 mg/kg or more. Although the upper limit of the amount of the jointly-used drug is not also particularly limited, the drug is generally used in an amount of 5000 mg/kg or less, preferably 1000 mg/kg or less, more preferably 100 mg/kg or less, or 50 mg/kg or less, or 20 mg/kg or less.

The compound (i) has a neurite outgrowth activity as shown in Test Example 5, and neutrophil migration inhibitory action as shown in Test Example 6. Accordingly, the medicaments of [15], and [22] to [25] mentioned above are preferred as agents for therapeutic and/or prophylactic treatment of spinal cord injury, which is one of traumatic nerve injuries, among neurological disorders, which constitute a class of diseases relating to morphological change of various cells.

A medicament comprising (i) and a drug having a central nerve regeneration action in combination is also preferred as an agent for therapeutic and/or prophylactic treatment of spinal cord injury.

In the present invention, as the antibiotics used for therapeutic and/or prophylactic treatment of spinal cord injury, for example, those comprising the same active ingredient as those used for therapeutic and/or prophylactic treatment of chronic obstructive pulmonary disease.

In the present invention, the steroids used for therapeutic and/or prophylactic treatment of spinal cord injury may comprise the same or different active ingredient as those used for therapeutic and/or prophylactic treatment of chronic obstructive pulmonary disease. Steroids with which an oral agent and an injection can be prepared are preferred. As the steroids, for example, methylprednisolone, and physiologically acceptable salts thereof are preferred, and methylprednisolone succinate and the like are preferred.

In the specification, cannabinoids mean cannabinol and structural analogues thereof, and for example, cannabinol, tetrahydrocannabinol, physiologically acceptable salts thereof and the like are preferred.

In the specification, the potassium channel blockers mean those acting on at least one of the voltage-dependent potassium channels to inhibit the functions thereof, and for example, 4-aminopyridine, physiologically acceptable salts thereof and the like are preferred.

In the specification, the hypoxanthine derivatives mean structural analogues of hypoxanthine, for example, leteprinim (AIT-082) (U.S. Patent No. 6,288,069), and physiologically acceptable salts thereof are preferred, and leteprinim potassium is preferred.

In the specification, regeneration of central nerve means a neurite outgrowth action for central nerve cells, action of neutralizing central nerve axonal growth inhibitor and the like, and examples of drugs having a central nerve regeneration action include, for example, NGF (nerve growth factor), Rho kinase inhibitors (Tanaka, T. et al., J. Cell Biol., 158 (2), pp.321-329 (2002)), BDGF (brain-derived nerve growth factor, Kishino, A., et al., Exp. Neurol., 144 (2), pp.273-86 (1997) ), FGF (fibroblast growth factor, Rabchevsky, AG., et al., J. Neurotrauma., 16(9), pp.817-30. (1999)) and the like.

In the specification, the central nerve axonal growth inhibitors means substances that inhibit axonal growth of central nerves under physiological conditions, and examples include myelin-related proteins, substances originated from glial scars formed in injury lesions (semaphorin, chondroitin sulfate and the like) and the like (Nakamura M. et al., Journal of Spine and Spinal Cord, Vol. 16, No. 4, pp.284-290. (2003)).

Further, a method for therapeutic and/or prophylactic treatment of spinal cord injury using any of the medicaments mentioned above is also preferred.

Further, when the medicament is used as, for example, an oral agent or an injection, a single dose can be administered about once to 10 times a day.

When the object of the administration is human, for example, (i) is generally used preferably in an amount of 0.0001 to 5000 mg/kg at one time. More specifically, although the lower limit of the amount of the jointly-used drug is not particularly limited so long as the effect of the present invention is exhibited, the jointly-used drug is used in an amount of, for example, 0.000001 mg/kg or more, preferably 0.00001 mg/kg or more, more preferably 0.0001 mg/kg or more, or 0.001 mg/kg or more, or 0.01 mg/kg or more, or 0.1 mg/kg or more. Although the upper limit of the amount of the jointly-used drug is not also particularly limited, it is generally used in an amount of 5000 mg/kg or less, preferably 1000 mg/kg or less, more preferably 100 mg/kg or less, or 50 mg/kg or less, or 20 mg/kg or less.

[Examples]

Hereafter, the present invention will be further specifically explained with reference to examples. However, the present invention is not limited to the following examples.
For thin layer chromatography (TLC), Precoated Silica Gel 60 F254 (produced by Merck) was used. After development with chloroform:methanol (100:1 to 4:1), or n-hexane:ethyl acetate (100:1 to 1:10), spots were observed by UV irradiation (254 nm) or coloration with ninhydrine or phosphomolybdic acid. For drying organic solvent, anhydrous magnesium sulfate or anhydrous sodium sulfate was used. For flash column chromatography, Silica gel 60N (spherical shape, neutral, 40 to 100 μ m, produced by Kanto Chemicals) was used. For preparative thin layer chromatography (PTLC), Precoated Silica Gel 60 F254 (20 x 20 cm, thickness: 2 mm, produced by Merck) was used. For the measurement of nuclear magnetic resonance (NMR) spectra, the measurement was performed by using Gemini-300 (FT-NMR, produced by Varian), or AL-300 (FT-NMR, produced by JOEL). As a solvent, deuterated chloroform was used, unless otherwise indicated. Chemical shifts were measured by using tetramethylsilane (TMS) as an internal standard, and indicated with δ (ppm), and binding constant was indicated with J (Hz). Mass spectrum (MS) was measured by liquid chromatography-mass spectrometry (LC-MS). Platform-LC type mass spectrometry apparatus (produced by Micromass) was used as the mass spectrometer, and the measurement was performed by the electrospray ionization (ESI) method. As the liquid chromatography apparatus, an apparatus produced by GILSON was used. As the separation column, Mightysil RP-18 GP 50-4.6 (produced by Kanto Chemicals) was used. Elution was generally performed at a flow rate of 2 ml/minute using a linear gradient of 5 to 100% (v/v) Solution B [acetonitrile containing 0.1% (v/v) acetic acid] in Solution A [water containing 0.1% (v/v) acetic acid] from 0 minute to 5 minutes as the solvent.

Reference Example 1: 4-Bromo-5-nitroisoquinoline

With vigorous stirring, concentrated sulfuric acid (36 ml) was added with 4-bromoisoquinoline (10.0 g, Tokyo Kasei Kogyo) to such an extent that the temperature should not exceed 10°C and stirred for a while to attain complete dissolution. Potassium nitrate (4.9 g, Kanto Chemicals) was dissolved in concentrated sulfuric acid (20 ml), added dropwise to the aforementioned solution at a temperature below -5°C and further stirred for 2 hours while maintaining that temperature. Disappearance of 4-bromoisoquinoline was confirmed by thin layer chromatography (n-hexane:ethyl acetate = 1:1), and then the reaction mixture was slowly poured into cold aqueous ammonia (200 ml, Wako Pure Chemical Industries) with vigorous stirring. The reaction mixture was stirred for 15 minutes and then extracted three times with ethyl acetate (150 ml for each time), and the combined organic layer was washed successively with water (250 ml) and saturated brine (250 ml) and dried over anhydrous sodium sulfate. The solvent was evaporated under reduced pressure, and the residue was recrystallized with ethyl acetate to obtain the title compound (5.9 g) as thick yellow needle-like crystals.

Reference Example 2: 5-Amino-4-bromoisoquinoline

4-Bromo-5-nitroisoquinoline (1.0 g) obtained in Reference Example 1 and stannous chloride dihydrate (4.5 g, Wako Pure Chemical Industries) were suspended in ethanol (30 ml), added with concentrated hydrochloric acid (2.3 ml) and stirred at 80°C for 30 minutes and at room temperature for further 12 hours. The reaction mixture was adjusted to pH 12 with addition of 2 N aqueous sodium hydroxide. The target compound was extracted three times with ethyl acetate (100 ml for each time), and the combined organic layer was washed with water (200 ml) and saturated brine (200 ml) and dried over anhydrous sodium sulfate. The solvent was evaporated under reduced pressure, and then the residue was purified by silica gel column chromatography (n-hexane:ethyl acetate = 3:1) to obtain the title compound (493 mg) as yellow powdery crystals.
$^1$H-NMR ($CDCl_3$) δ (ppm): 5.23 (2H, br.s), 6.92 (1H, dd, J=1.6, 7.3Hz), 7.38 (2H, m), 8.50 (1H, s), 8.98 (1H, s)

The compounds of the examples are shown in Tables 1 to 7 mentioned later (in the tables, numerals indicated in the columns of "Exp. No." are example numbers, Me represents methyl, and Bn represents benzyl). The compounds of Table 1 have the structure of the following formula (1-A), the compounds of Table 2 have the structure of the following formula (1-B), the compounds of Table 3 have the structure of the following formula (1-C), the compounds of Table 4 have the structure of the following formula (1-D), the compounds of Table 5 have the structure of the following formula (1-E), the compounds of Table 6 have a structure of the following formula (1-F), and the compounds of Table 7 have the structure of the following formula (1-G), respectively.

[Formula 47]

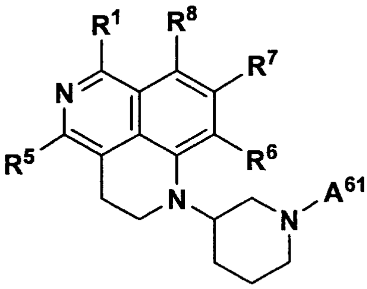
(1-A)

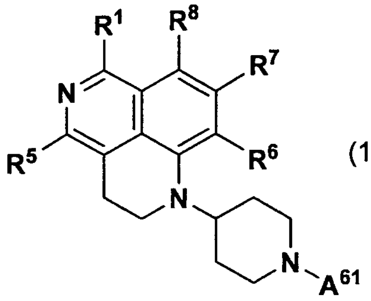
(1-B)

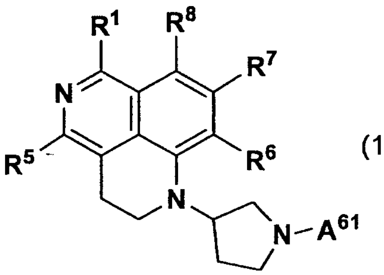
(1-C)

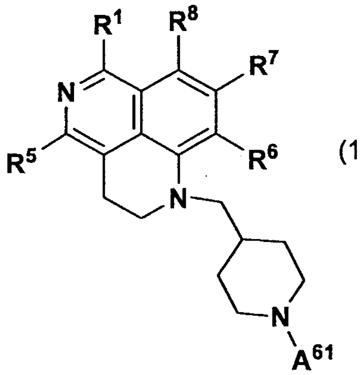
(1-D)

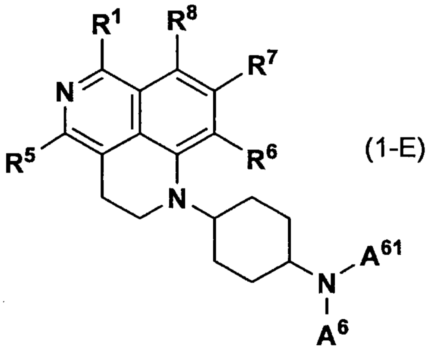
(1-E)

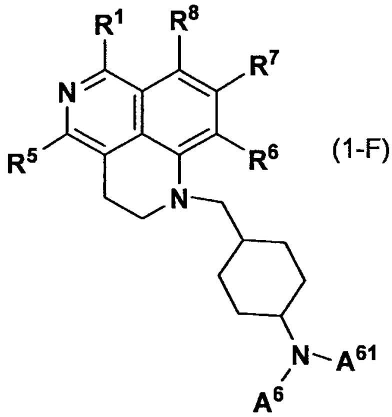
(1-F)

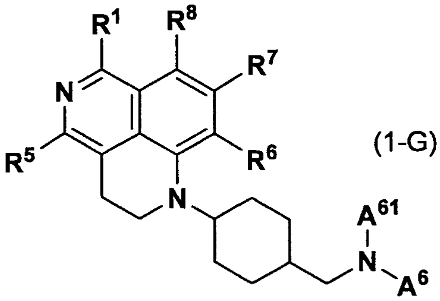
(1-G)

Example 1-1: 3-(2,3-Dihydro-1,5-diazaphenalen-1-yl)piperidine

Step A

5-Amino-4-vinylisoquinoline (Intermediate 1)

A suspension of 5-amino-4-bromoisoquinoline obtained in Reference Example 1 (10 g), tri(n-butyl)vinyltin (21.0 ml), tetrakis(triphenylphosphine)palladium(0) (1.04 g,), and 2,6-di-tert-butyl-p-cresol (11.3 mg) in toluene (90 ml) was stirred at 110°C for 15 hours. The reaction mixture was cooled to room temperature, then added with 10% aqueous potassium fluoride (90 ml), and stirred for 4 hours. The reaction mixture was added with ethyl acetate (100 ml), and the precipitates were removed by filtration. Then, the organic layer was separated, and dried over anhydrous magnesium sulfate. The solvent was evaporated under reduced pressure, and then the residue was purified by silica gel column chromatography (n-hexane:ethyl acetate = 3:1) to obtain the title compound (7.00 g).

Step B

3-(4-Vinyl-5-isoquinolyl)amino-1-(tert-butoxycarbonyl)piperidine (Intermediate 2)

A solution of 5-amino-4-vinylisoquinoline (Intermediate 1, 20.08 g) obtained in Step A mentioned above, and tert-butyl 3-oxo-1-piperidinecarboxylate (47.01 g, AstaTech) in 1,2-dichloroethane (1000 ml) was added with glacial acetic acid (13.5 ml), and then added with sodium triacetoxyborohydride (50.00 g, Aldrich), and stirred at room temperature for 48 hours. The reaction mixture was cooled on an ice bath, and slowly added with saturated aqueous sodium hydrogencarbonate (1000 ml) for neutralization (pH 7). The organic layer was separated, and the aqueous layer was extracted with ethyl acetate (1000 ml). The combined organic layer was dried over anhydrous magnesium sulfate, and the solvent was evaporated under reduced pressure. The residue was purified by silica gel column chromatography (n-hexane:ethyl acetate = 2:1) to obtain the title compound (32.63 g).

Step C

3-(2,3-Dihydro-1,5-diazaphenalen-1-yl)piperidine-1-carboxylic acid tert-butyl ester (Intermediate 3)

A suspension of 3-(4-vinyl-5-isoquinolyl)amino-1-(tert-butoxycarbonyl)-piperidine (Intermediate 2, 6.50 g) obtained in Step B mentioned above, and potassium tert-butoxide (4.10 g) in tetrahydrofuran (92 ml) was stirred at 50°C for 1 hour. The reaction mixture was added with water (100 ml), saturated aqueous ammonium chloride (50 ml), and ethyl acetate (100 ml), and the precipitates were separated by filtration. Then, the organic layer was separated, and dried over anhydrous sodium sulfate. The solvent was evaporated under reduced pressure, and then the residue was purified by silica gel column chromatography (n-hexane:ethyl acetate = 1:2) to obtain the title compound (4.45 g).
MS (m/z): 354 (MH+)

Step D

3-(2,3-Dihydro-1,5-diazaphenalen-1-yl)piperidine

3-(2,3-Dihydro-1,5-diazaphenalen-1-yl)piperidine-1-carboxylic acid tert-butyl ester (Intermediate 3, 24.42 g) obtained in the step mentioned above was added with a 4 N hydrogen chloride/1,4-dioxane solution (320 ml, Kokusan Kagaku), and stirred at 50°C for 2 hours. The solvent was evaporated from the reaction mixture under reduced pressure to obtain the title compound as a hydrochloride (21.97 g).
$^1$H-NMR (DMSO-$d_6$) δ (ppm): 1.89-2.03 (4H, m), 2.78-2.91 (1H, m), 3.02-3.42 (6H, m), 3.53-3.61 (1H, m), 4.48-4.55 (1H, m), 7.60 (1H, d, J=7.8Hz), 7.72 (1H, d, J=7.8Hz), 7.80 (1H, t, J=7.8Hz), 8.36 (1H, s), 9.51 (1H, brs), 9.56 (1H, s)
MS (m/z): 254 (MH+)

Example 1-2: 1-Methyl-3-(2,3-dihydro-1,5-diazaphenalen-1-yl)piperidine

A solution of 3-(2,3-dihydro-1,5-diazaphenalen-1-yl)piperidine (53 mg) obtained in Example 1, Step D in ethanol (1.5 ml) and 37% formalin (1.5 ml, Wako Pure Chemical Industries) was added with 10% palladium/carbon (5 mg), and stirred at 70°C for 8 hours under hydrogen atmosphere. The reaction mixture was filtered through Celite, and then the solvent was evaporated under reduced pressure. The residue was purified by silica gel column chromatography (chloroform:methanol = 9:1) to obtain the title compound (35 mg).

MS (m/z): 268 (MH+)

Example 1-3: 1-(Cyclopentylmethyl)-3-(2,3-dihydro-1,5-diazaphenalen-1-yl)piperidine

The compound of Example 1-3 can be prepared by Method A or Method B mentioned below.

(Method A) A suspension of the hydrochloride of 3-(2,3-dihydro-1,5-diazaphenalen-1-yl)piperidine obtained in Example 1, Step D (326 mg), and potassium carbonate (690 mg, Wako Pure Chemical Industries) in N,N-dimethylformamide (8 ml) was added with cyclopentylmethyl bromide (490 mg, Tokyo Kasei Kogyo), and stirred at room temperature for 18 hours. The reaction mixture was poured into ice water, and extracted 4 times with ethyl acetate (5 ml for each time). The combined organic layer was washed twice with saturated brine (10 ml for each time), and dried over anhydrous magnesium sulfate. The organic layer was filtered through Celite, and then the solvent was evaporated under reduced pressure. The residue was purified by silica gel column chromatography (chloroform: methanol = 20:1) to obtain the title compound (243 mg).
(Method B) A solution of 3-(2,3-dihydro-1,5-diazaphenalen-1-yl)piperidine (253 mg) obtained in Example 1, Step D, and cyclopentanecarboxyaldehyde (185 mg, Tokyo Kasei Kogyo) in 1,2-dichloroethane (8 ml) was added with sodium triacetoxyborohydride (300 mg), and stirred at room temperature for 12 hours. The reaction mixture was added with saturated aqueous sodium hydrogencarbonate, and extracted twice with dichloromethane (5 ml for each time). The combined organic layer was washed with saturated brine (10 ml), and dried over anhydrous magnesium sulfate. The organic layer was filtered through Celite, and then the solvent was evaporated under reduced pressure. The residue was purified by silica gel column chromatography (chloroform:methanol = 20:1) to obtain the title compound (289 mg). MS (m/z): 336 (MH+)

Example 1-4: 1-(4-Methoxybenzyl)-3-(2,3-dihydro-1,5-diazaphenalen-1-yl)piperidine

A solution of 3-(2,3-dihydro-1,5-diazaphenalen-1-yl)piperidine (253 mg) obtained in Example 1, Step D, and 4-methoxybenzaldehyde (200 mg, Tokyo Kasei Kogyo) in 1,2-dichloroethane (8 ml) was added with sodium triacetoxyborohydride (300 mg), and stirred at room temperature for 12 hours. The reaction mixture was added with saturated aqueous sodium hydrogencarbonate, and extracted twice with dichloromethane (5 ml for each time). The combined organic layer was washed with saturated brine (10 ml), and dried over anhydrous magnesium sulfate. The organic layer was filtered through Celite, and then the solvent was evaporated under reduced pressure. The residue was purified by silica gel column chromatography (chloroform:methanol = 20:1) to obtain the title compound (320 mg).
MS (m/z): 374 (MH+)

Example 1-5: 1-Phenyl-3-(2,3-dihydro-1,5-diazaphenalen-1-yl)piperidine

A suspension of 3-(2,3-dihydro-1,5-diazaphenalen-1-yl)piperidine (253 mg) obtained in Example 1, Step D, bromobenzene (235 mg, Tokyo Kasei Kogyo), tris(dibenzylideneacetone)dipalladium(0) (46 mg, Aldrich), bis-di(tert-butyl)(2-biphenyl)phosphine (60 mg, Strem), and sodium tert-butoxide (110 mg, Tokyo Kasei Kogyo) in toluene (5 ml) was stirred at 80°C for 3 hours. The organic layer was filtered through Celite, and then the solvent was evaporated under reduced pressure. The residue was purified by silica gel column chromatography (chloroform:methanol = 20:1) to obtain the title compound (242 mg).
MS (m/z): 330 (MH+)

Example 1-6: 2-[3-(2,3-Dihydro-1,5-diazaphenalen-1-yl)piperidin-1-yl]acetic acid

The compound of Example 1-6 can be prepared by Method A or Method B mentioned below .

(Method A) By using hydrochloride of 3-(2,3-dihydro-1,5-diazaphenalen-1-yl)piperidine obtained in Example 1, Step D, alkylation was performed with tert-butyl bromoacetate (Tokyo Kasei Kogyo) according to the method described in Example 2, and then deprotection was performed for the protective group according to the method described in Example 1, Step D to obtain the title compound as a hydrochloride.
(Method B) A suspension of the hydrochloride of 3-(2,3-dihydro-1,5-diazaphenalen-1-yl)piperidine (326 mg) obtained in Example 1, Step D, and potassium carbonate (690 mg) in N,N-dimethylformamide (8 ml) was added with tert-butyl acrylate (305 mg, Tokyo Kasei Kogyo), and stirred at room temperature 18 hours. The reaction mixture was poured into ice water, and extracted 4 times with ethyl acetate (5 ml for each time). The combined organic layer was washed twice with saturated brine (10 ml for each time), and dried over anhydrous magnesium sulfate. The organic layer was filtered through Celite, and then the solvent was evaporated under reduced pressure. The residue

was purified by silica gel column chromatography (chloroform:methanol = 20:1), and then deprotection was performed for the protective group according to the method described in Example 1, Step D to obtain the title compound as a hydrochloride.
MS (m/z): 312 (MH+)

Example 1-7: 3-[3-(2,3-Dihydro-1,5-diazaphenalen-1-yl)piperidin-1-yl]propanoic acid

A suspension of the hydrochloride of 3-(2,3-dihydro-1,5-diazaphenalen-1-yl)piperidine (96 mg) obtained in Example 1, Step D, and potassium carbonate (228 mg) in N,N-dimethylformamide (2.7 ml) was added with tert-butyl acrylate (174 μ 1, Aldrich), and stirred at room temperature for 40 hours. The reaction mixture was added with acetone (10 ml), insoluble solids were removed by filtration, and then the solvent was evaporated under reduced pressure. The residue was purified by silica gel column chromatography (chloroform:methanol = 20:1), and then deprotection was performed for the protective group according to the method described in Example 1, Step D to obtain the title compound (51 mg).
MS (m/z): 326 (MH+)

Example 1-8: 2-[3-(2,3-Dihydro-1,5-diazaphenalen-1-yl)piperidin-1-yl]acetonitrile

By using hydrochloride of 3-(2,3-dihydro-1,5-diazaphenalen-1-yl)piperidine obtained in Example 1, Step D, alkylation was performed with 2-bromoacetonitrile according to the method described in Example 1-3, Method A to obtain the title compound.
MS (m/z): 293 (MH+)

Example 1-9: 3-[3-(2,3-Dihydro-1,5-diazaphenalen-1-yl)piperidin-1-yl]propanenitrile

By using hydrochloride of 3-(2,3-dihydro-1,5-diazaphenalen-1-yl)piperidine obtained in Example 1, Step D, alkylation was performed with acrylonitrile according to the method described in Example 1-7 to obtain the title compound.
MS (m/z): 307 (MH+)

Example 1-10: 1-(2-Hydroxyethyl)-3-(2,3-dihydro-1,5-diazaphenalen-1-yl)piperidine

3 N Aqueous sodium hydroxide (4 ml) and ethanol (4 ml) were added with hydrochloride of 3-(2,3-dihydro-1,5-diazaphenalen-1-yl)piperidine (326 mg) obtained in Example 1-1, Step D, then added with 2-bromoethanol (142 μl, Tokyo Kasei Kogyo), or 2-chloroethanol (134 μl, Tokyo Kasei Kogyo), and stirred at 40°C for 7 hours. The reaction mixture was extracted 4 times with chloroform (4 ml for each time), and the organic layer was washed 3 times with water (5 ml for each time), and then dried over anhydrous magnesium sulfate. The organic layer was filtered through Celite, and then and the solvent was evaporated under reduced pressure. The residue was purified by silica gel column chromatography (chloroform:methanol = 10:1) to obtain the title compound (166 mg).
$^1$H-NMR (DMSO-$d_6$) δ (ppm): 1.88-1.95 (2H, m), 2.28-2.38 (2H, m), 3.05-3.20 (6H, m), 3.35-3.45 (2H, m), 3.62-3.68 (2H, m), 3.79-3.85 (2H, m), 4.25-4.35 (1H, m), 7.43 (1H, d, J=8.1Hz), 7.67 (1H, d, J=8.1Hz), 7.78 (1H, t, J=8.1Hz), 8.36 (1H, s), 9.53 (1H, s)
MS (m/z): 298 (MH+)

Example 1-11: 2-[3-(2,3-Dihydro-1,5-diazaphenalen-1-yl)piperidin-1-yl]-1,3-propanediol

By using 3-(2,3-dihydro-1,5-diazaphenalen-1-yl)piperidine (253 mg) obtained in Example 1-1, Step D, reductive alkylation was performed with 2,2-dimethyl-1,3-dioxolan-5-one (Tokyo Kasei Kogyo) according to the method described in Example 1-4, and then the resultant was dissolved in tetrahydrofuran (6 ml), added with 3 N hydrochloric acid (2 ml), and stirred at 50°C for 6 hours. The solvent was evaporated under reduced pressure, and the residue was neutralized with saturated aqueous sodium hydrogencarbonate. The mixture was extracted 3 times with ethyl acetate (5 ml for each time), and the organic layer was washed with saturated brine (10 ml), and then dried over anhydrous magnesium sulfate. The organic layer was filtered through Celite, and then the solvent was evaporated under reduced pressure. The residue was purified by silica gel column chromatography (chloroform:methanol = 10:1) to obtain the title compound (258 mg).
MS (m/z): 328 (MH+)

Example 1-12: 1-(4-Hydroxycyclohexyl)-3-(2,3-dihydro-1,5-diazaphenalen-1-yl)piperidine

A solution of 3-(2,3-dihydro-1,5-diazaphenalen-1-yl)piperidine (326 mg) obtained in Example 1-1, Step D, and

1,4-cyclohexanedione monoethylene ketal (200 mg, Tokyo Kasei Kogyo) in 1,2-dichloroethane (10 ml) was added with sodium triacetoxyborohydride (300 mg), and stirred at room temperature 8 hours. The reaction mixture was washed with saturated aqueous sodium hydrogencarbonate, and dried over anhydrous magnesium sulfate. The organic layer was filtered through Celite, and then the solvent was evaporated under reduced pressure. The residue was purified by silica gel column chromatography (n-hexane:ethyl acetate = 1:2). Then, the purified residue was dissolved in 3 N hydrochloric acid (10 ml), and stirred at room temperature for 12 hours. The solvent was evaporated under reduced pressure, and the residue was neutralized with saturated aqueous sodium hydrogencarbonate, and then extracted 3 times with ethyl acetate (10 ml for each time). The combined organic layer was washed with saturated brine (20 ml), and dried over anhydrous magnesium sulfate. The organic layer was filtered through Celite, and then the solvent was evaporated under reduced pressure. Then, the residue was dissolved in methanol (8 ml), added with sodium borohydride (60 mg, Wako Pure Chemical Industries) under cooling on an ice bath, and stirred at the same temperature for 2 hours. The reaction mixture was carefully added with water, and then extracted 3 times with ethyl acetate (10 ml for each time). The combined organic layer was washed with saturated brine (20 ml), and dried over anhydrous magnesium sulfate. The organic layer was filtered through Celite, and then the solvent was evaporated under reduced pressure. The residue was purified by silica gel column chromatography (chloroform:methanol = 20:1) to obtain the title compound (195 mg).
MS (m/z): 352 (MH+)

Example 1-13: 1-(2-Methoxyethyl)-3-(2,3-dihydro-1,5-diazaphenalen-1-yl)piperidine

By using hydrochloride of 3-(2,3-dihydro-1,5-diazaphenalen-1-yl)piperidine obtained in Example 1-1, Step D, alkylation was performed with 2-bromoethyl methyl ether (Tokyo Kasei Kogyo) according to the method described in Example 1-3, Method A to obtain the title compound.
MS (m/z): 312 (MH+)

Example 1-14: 1-(2-Pivaloyloxyethyl)-3-(2,3-dihydro-1,5-diazaphenalen-1-yl)piperidine

A solution of 1-(2-hydroxyethyl)-3-(2,3-dihydro-1,5-diazaphenalen-1-yl)piperidine obtained in Example 1-10, and N,N-diisopropylethylamine (95 mg, Tokyo Kasei Kogyo) in dichloromethane (4 ml) was added with pivalic anhydride (112 mg, Tokyo Kasei Kogyo), and stirred at room temperature 1 hour. The reaction mixture was washed with saturated brine (4 ml), and dried over anhydrous magnesium sulfate. The organic layer was filtered through Celite, and then the solvent was evaporated under reduced pressure. The residue was purified by silica gel column chromatography (chloroform:methanol = 30:1) to obtain the title compound (166 mg). MS (m/z): 382 (MH+)

Example 1-15: 1-(2-Acetylthioethyl)-3-(2,3-dihydro-1,5-diazaphenalen-1-yl)piperidine

A solution of 1-(2-hydroxyethyl)-3-(2,3-dihydro-1,5-diazaphenalen-1-yl)piperidine (149 mg) obtained in Example 1-10, thioacetic acid (76 mg, Aldrich), and 1,1'-azobis(N,N-dimethylformamide) (173 mg, Tokyo Kasei Kogyo) in tetrahydrofuran (4 ml) was added with n-(tri)butylphosphine (203 mg, Tokyo Kasei Kogyo) under cooling on an ice bath, and stirred at the same temperature for 1 hour. The reaction mixture was further stirred at room temperature for 12 hours, and then filtered through Celite. The solvent was evaporated under reduced pressure, and the residue was purified by silica gel column chromatography (chloroform:methanol = 30:1) to obtain the title compound.
MS (m/z): 356 (MH+)

Example 1-16: 1-(2-Thioethyl)-3-(2,3-dihydro-1,5-diazaphenalen-1-yl)piperidine

A solution of 1-(2-acetylthioethyl)-3-(2,3-dihydro-1,5-diazaphenalen-1-yl)piperidine (178 mg) obtained in Example 1-15 in methanol (2 ml) was added with 1 N aqueous sodium hydroxide (1 ml) under cooling on an ice bath, and stirred at room temperature 15 hours. The reaction mixture was neutralized with 1 N aqueous ammonium chloride, and extracted 3 times with ethyl acetate (3 ml for each time). The combined organic layer was washed with saturated brine (8 ml), and dried over anhydrous magnesium sulfate. The organic layer was filtered through Celite, and then the solvent was evaporated under reduced pressure. The residue was purified by silica gel column chromatography (chloroform: methanol = 20:1) to obtain the title compound (108 mg).
MS (m/z): 314 (MH+)

Example 1-17: 1-(2-Aminoethyl)-3-(2,3-dihydro-1,5-diazaphenalen-1-yl)piperidine

By using hydrochloride of 3-(2,3-dihydro-1,5-diazaphenalen-1-yl)piperidine obtained in Example 1-1, Step D, alkylation was performed with tert-butyl N-(2-bromoethyl)carbamate (Fluka) according to the method described in Example 1-3, Method A, and then deprotection was performed for the protective group according to the method described in Example 1-1, Step D to obtain the title compound as a hydrochloride.
MS (m/z): 297 (MH+)

Example 1-18: 1-(3-Aminopropyl)-3-(2,3-dihydro-1,5-diazaphenalen-1-yl)piperidine

By using hydrochloride of 3-(2,3-dihydro-1,5-diazaphenalen-1-yl)piperidine obtained in Example 1-1, Step D, alkylation was performed with tert-butyl N-(3-bromopropyl)carbamate (Tokyo Kasei Kogyo) according to the method described in Example 1-3, Method A, and then deprotection was performed for the protective group according to the method described in Example 1-1, Step D to obtain the title compound as a hydrochloride.
MS (m/z): 311 (MH+)

Example 1-19: 1-[3-(Methylamino)propyl]-3-(2,3-dihydro-1,5-diazaphenalen-1-yl)piperidine

A mixed solvent of 3 N aqueous sodium hydroxide (8 ml) and ethanol (15 ml) was added with hydrochloride of 3-(2,3-dihydro-1,5-diazaphenalen-1-yl)piperidine obtained in Example 1-1, Step D (653 mg) under cooling on an ice bath, then added with 4-chlorobutylaldehyde diethyl acetal (1.08 g, Alfa Aesar), stirred at the same temperature for 30 minutes, and then further stirred at 50°C for 12 hours. The reaction mixture was left to cool to room temperature, and then added with chloroform (30 ml) and saturated brine (20 ml), and the organic layer was separated. The aqueous layer was extracted 3 times with chloroform (20 ml for each time), and the combined organic layer was successively washed with water (30 ml), and saturated brine (30 ml) in this order, and dried over anhydrous magnesium sulfate. The organic layer was filtered through Celite, and then the solvent was evaporated under reduced pressure. The residue was purified by silica gel column chromatography (chloroform:methanol = 20:1), added with 5 N hydrochloric acid (20 ml), and stirred at room temperature for 72 hours. The reaction mixture was cooled to 0°C, neutralized with 2 N aqueous sodium hydroxide (pH 7.5), and extracted 3 times with ethyl acetate (30 ml for each time). The combined organic layer was dried over anhydrous magnesium sulfate, and the solvent was evaporated under reduced pressure. The residue was dissolved in methanol (5 ml) and methylene chloride (10 ml), added with a 40% methylamine/methanol solution (8 ml, Tokyo Kasei Kogyo), and stirred at room temperature for 1 hour. Then, the reaction mixture was added with sodium borohydride (45 mg), and stirred at room temperature 16 hours. The reaction mixture was cooled to 0°C, added with water (20 ml), and extracted twice with methylene chloride (60 ml for each time). The combined organic layer was dried over anhydrous magnesium sulfate, and the solvent was evaporated under reduced pressure. The residue was purified by silica gel column chromatography (chloroform:methanol:isopropylamine = 92.0/7.9/0.1) to obtain the title compound (203 mg).
MS (m/z): 325 (MH+)

Example 1-20: 1,3-Bis(acetamino)-2-[3-(2,3-dihydro-1,5-diazaphenalen-1-yl)piperidin-1-yl]propane

By using 3-(2,3-dihydro-1,5-diazaphenalen-1-yl)piperidine obtained in Example 1-1, Step D, reductive alkylation was performed with 1,3-bis(acetamino)acetone (Sailor) according to the method described in Example 1-12 to obtain the title compound.
MS (m/z): 410 (MH+)

Example 1-21: 2-[3-(2,3-Dihydro-1,5-diazaphenalen-1-yl)piperidin-1-yl]-1,3-propanediamine

1,3-Bis(acetamino)-2-[3-(2,3-dihydro-1,5-diazaphenalen-1-yl)piperidin-1-yl]propane (205 mg) obtained in Example 1-1, Step D was dissolved in concentrated hydrochloric acid (5 ml), and stirred at 80°C for 12 hours. The reaction mixture was left to cool to room temperature, and then the solvent was evaporated under reduced pressure to obtain the title compound as a hydrochloride (230 mg).
MS (m/z): 326 (MH+)

Example 1-22: 1-(2-Acetaminoethyl)-3-(2,3-dihydro-1,5-diazaphenalen-1-yl)piperidine

A suspension of the hydrochloride of 1-(2-aminoethyl)-3-(2,3-dihydro-1,5-diazaphenalen-1-yl)piperidine (405 mg) obtained in Example 1-17 in dichloromethane (15 ml) was added with N,N-diisopropylethylamine (650 mg) under cooling on an ice bath, and stirred for 30 minutes. Then, the reaction mixture was added with acetic anhydride (150 mg), and stirred at the same temperature for 2 hours. The reaction mixture was added with water (15 ml), and the organic layer was separated, and dried over anhydrous magnesium sulfate. The organic layer was filtered through Celite, and then the solvent was evaporated under reduced pressure. The residue was purified by silica gel column chromatography (chloroform:methanol = 10:1) to obtain the title compound (320 mg).

MS (m/z): 339 (MH+)

Example 1-23: 5-[3-(2,3-Dihydro-1,5-diazaphenalen-1-yl)piperidin-1-yl]-tetrahydropyrimidin-2(1H)-one

A solution of the hydrochloride of 2-[3-(2,3-dihydro-1,5-diazaphenalen-1-yl)piperidin-1-yl]-1,3-propanediamine (163 mg) obtained in Example 1-1, Step D in pyridine (4 ml) was added with 4-nitrophenyl carbonate (210 mg, Aldrich) under cooling on an ice bath, and stirred at the same temperature for 1 hour. The reaction mixture was further stirred at room temperature for 12 hours, and then the solvent was evaporated under reduced pressure. The reaction mixture was added with saturated brine, and extracted twice with ethyl acetate (10 ml). The combined organic layer was washed with saturated brine, and the residue was purified by silica gel column chromatography (chloroform:methanol = 20:1) to obtain the title compound (188 mg).
MS (m/z): 352 (MH+)

Example 1-24: 1-[2-(2-Acetaminoacetamino)ethyl]-3-(2,3-dihydro-1,5-diazaphenalen- 1-yl)piperidine

A suspension of the hydrochloride of 1-(2-aminoethyl)-3-(2,3-dihydro-1,5-diazaphenalen-1-yl)piperidine (405 mg) obtained in Example 1-17 in dichloromethane (15 ml) was added with N,N-diisopropylethylamine (650 mg) under cooling on an ice bath, and stirred for 30 minutes. Then, the reaction mixture was added with N-acetylglycine (235 mg, Wako Pure Chemical Industries), and dicyclohexylcarbodiimide (360 mg, Wako Pure Chemical Industries), and stirred at room temperature for 12 hours. The reaction mixture was filtered through Celite, and then the filtrate was washed with saturated brine, and dried over anhydrous magnesium sulfate. The organic layer was filtered through Celite, and then the solvent was evaporated under reduced pressure. The residue was purified by silica gel column chromatography (chloroform:methanol = 10:1) to obtain the title compound (318 mg).
MS (m/z): 396 (MH+)

Example 1-25: 1-{2-[2-(Dimethylamino)acetaminoethyl]}-3-(2,3-dihydro-1,5-diazaphenalen-1-yl)piperidine

From 1-(2-aminoethyl)-3-(2,3-dihydro-1,5-diazaphenalen-1-yl)piperidine obtained in Example 1-17 and 2-(dimethylamino)acetic acid, the title compound was obtained according to the method described in Example 1-24.
MS (m/z): 382

Example 1-26: 1-(2-Methanesulfonylaminoethyl)-3-(2,3-dihydro-1,5-diazaphenalen-1-yl)piperidine

1-(2-Aminoethyl)-3-(2,3-dihydro-1,5-diazaphenalen-1-yl)piperidine obtained in Example 1-17 was used to react with methanesulfonic anhydride according to the method described in Example 1-22 to obtain the title compound.
MS (m/z): 375 (MH+)

Example 1-27: 1-(4-Aminocyclohexyl)-3-(2,3-dihydro-1,5-diazaphenalen-1-yl)piperidine

By using 3-(2,3-dihydro-1,5-diazaphenalen-1-yl)piperidine obtained in Example 1-1, Step D and tert-butyl N-(4-oxocyclohexyl)carbamate (AstaTech), reductive alkylation was performed according to the method described in Example 1-12, and then deprotection was performed for the protective group according to the method described in Example 1-1, Step D to obtain the title compound.
MS (m/z): 351 (MH+)

Example 1-28: 1-(4-Acetaminocyclohexyl)-3-(2,3-dihydro-1,5-diazaphenalen- 1-yl)piperidine

1-(4-Aminocyclohexyl)-3-(2,3-dihydro-1,5-diazaphenalen-1-yl)piperidine obtained in Example 1-27 was used to react with acetic anhydride according to the method described in Example 1-22 to obtain the title compound.
MS (m/z): 393 (MH+)

Example 1-29: N-{3-[3-(2,3-Dihydro-1,5-diazaphenalen-1-yl)piperidin-1-yl]propyl}-N'-(2-hydroxyethyl)urea

A solution of 1-(3-aminopropyl)-3-(2,3-dihydro-1,5-diazaphenalen-1-yl)piperidine (310 mg) obtained in Example 1-18 and 4-dimethylaminopyridine (250 mg, Tokyo Kasei Kogyo) in N,N-dimethylformamide (20 ml) was added with 4-nitrophenyl carbonate (450 mg) under cooling on an ice bath, stirred at the same temperature for 30 minutes, and then further stirred at room temperature for 6 hours. The reaction mixture was added with 2-aminoethanol (125 mg, Tokyo Kasei Kogyo) under cooling on an ice bath, and stirred at room temperature for 12 hours. The reaction mixture was

added with saturated brine, and extracted 4 times with ethyl acetate (20 ml for each time). The combined organic layer was washed twice with saturated brine (40 ml for each time), and dried over anhydrous magnesium sulfate. The organic layer was filtered through Celite, and then the solvent was evaporated under reduced pressure. The residue was purified by silica gel column chromatography (chloroform:methanol = 10:1) to obtain the title compound (208 mg).
MS (m/z) : 398 (MH+)

Example 1-30: N-{3-[3-(2,3-Dihydro-1,5-diazaphenalen-1-yl)piperidin-1-yl]propyl}-N'-benzylurea

A solution of 1-(3-aminopropyl)-3-(2,3-dihydro-1,5-diazaphenalen-1-yl)piperidine (155 mg) obtained in Example 1-18 and N,N-diisopropylethylamine (97 mg) in dichloromethane (5 ml) was added with benzyl isocyanate (100 mg, Aldrich) under cooling on an ice bath, and stirred at room temperature 15 hours. The reaction mixture was added with saturated aqueous sodium hydrogencarbonate, and the organic layer was separated, and dried over anhydrous magnesium sulfate. The organic layer was filtered through Celite, and then the solvent was evaporated under reduced pressure. The residue was purified by silica gel column chromatography (chloroform:methanol = 20:1) to obtain the title compound (176 mg).
MS (m/z): 444 (MH+)

Example 1-31: N-{2-(3-(2,3-Dihydro-1,5-diazaphenalen-1-yl)piperidin-1-yl]ethyl}-N'-(2-hydroxyethyl)thiourea

A solution of 1-(2-aminoethyl)-3-(2,3-dihydro-1,5-diazaphenalen-1-yl)piperidine (310 mg) obtained in Example 1-17 and 4-dimethylaminopyridine (250 mg, Tokyo Kasei Kogyo) in tetrahydrofuran (20 ml) was added with 1,1'-thiocarbonyldiimidazole (270 mg, Aldrich) under cooling on an ice bath, stirred at the same temperature for 30 minutes, and then further stirred at room temperature for 12 hours. The reaction mixture was added with 2-aminoethanol (125 mg, Tokyo Kasei Kogyo) under cooling on an ice bath, and stirred at room temperature 15 hours. The reaction mixture was added with saturated brine, and extracted 3 times with ethyl acetate (20 ml for each time).. The combined organic layer was washed twice with saturated brine (40 ml for each time), and dried over anhydrous magnesium sulfate. The organic layer was filtered through Celite, and then the solvent was evaporated under reduced pressure. The residue was purified by silica gel column chromatography (chloroform:methanol = 10:1) to obtain the title compound (83 mg).
MS (m/z): 400 (MH+)

Example 1-32: N-{2-[3-(2,3-Dihydro-1,5-diazaphenalen-1-yl)piperidin-1-yl]ethyl}-N'-phenylthiourea

From 1-(2-aminoethyl)-3-(2,3-dihydro-1,5-diazaphenalen-1-yl)piperidine obtained in Example 1-17 and phenyl isothiocyanate (Aldrich), the title compound was obtained according to the method described in Example 1-30.
MS (m/z): 432 (MH+)

Example 1-33: 2-(Dimethylamino)ethyl N-{2-[3-(2,3-dihydro-1,5-diazaphenalen-1-yl)piperidin-1-yl]ethyl}carbamate

A solution of 1-(2-aminoethyl)-3-(2,3-dihydro-1,5-diazaphenalen-1-yl)piperidine (310 mg) obtained in Example 1-17 and 4-dimethylaminopyridine (250 mg, Tokyo Kasei Kogyo) in N,N-dimethylformamide (20 ml) was added with 4-nitrophenyl carbonate (450 mg, Aldrich) under cooling on an ice bath, stirred at the same temperature for 30 minutes, and then further stirred at room temperature for 6 hours. The reaction mixture was added with 2-(dimethylamino)ethanol (178 mg, Tokyo Kasei Kogyo) under cooling on an ice bath, and stirred at room temperature 24 hours. The reaction mixture was added with saturated brine, and extracted 4 times with ethyl acetate (20 ml for each time). The combined organic layer was washed twice with saturated brine (40 ml for each time), and dried over anhydrous magnesium sulfate. The organic layer was filtered through Celite, and then the solvent was evaporated under reduced pressure. The residue was purified by silica gel column chromatography (chloroform:methanol = 10:1) to obtain the title compound (208 mg).
MS (m/z): 412 (MH+)

Example 1-34: 2-[3-(2,3-Dihydro-1,5-diazaphenalen-1-yl)piperidin-1-yl]ethyl cyclopentylcarbamic acid

A solution of 1-(2-hydroxyethyl)-3-(2,3-dihydro-1,5-diazaphenalen-1-yl)piperidine (156 mg) obtained in Example 1-10 and 4-dimethylaminopyridine (125 mg, Tokyo Kasei Kogyo) in dichloromethane (8 ml) was added with 4-nitrophenyl carbonate (225 mg, Aldrich) under cooling on an ice bath, stirred at the same temperature for 30 minutes, and then further stirred at room temperature for 6 hours. The reaction mixture was added with cyclopentylamine (85 mg, Tokyo Kasei Kogyo) under cooling on an ice bath, and stirred at room temperature 24 hours. The reaction mixture was added with saturated brine, and extracted 4 times with ethyl acetate (8 ml for each time). The combined organic layer was washed twice with saturated brine (20 ml for each time), and dried over anhydrous magnesium sulfate. The organic

layer was filtered through Celite, and then the solvent was evaporated under reduced pressure. The residue was purified by silica gel column chromatography (chloroform:methanol = 10:1) to obtain the title compound (102 mg).
MS (m/z): 409

Example 1-35: 3-(2,3-Dihydro-1,5-diazaphenalen-1-yl)piperidine-1-carboxamidine

From 3-(2,3-dihydro-1,5-diazaphenalen-1-yl)piperidine obtained in Example 1-1, Step D, the title compound was obtained as a hydrochloride by referring to a known literature (for example, Drake Brian, Patek Marcel, Lebl Michael, Synthesis, 6, 579 (1994) and the like).
MS (m/z): 296 (MH+)

Example 1-36: 1-{2-[3-(2,3-Dihydro-1,5-diazaphenalen-1-yl)piperidine]ethyl}-azetidin-2-one

A solution of 1-(2-aminoethyl)-3-(2,3-dihydro-1,5-diazaphenalen-1-yl)piperidine (364 mg) obtained in Example 1-17 and tert-butyl acrylate (450 μl, Tokyo Kasei Kogyo) in N,N-dimethylformamide (12 ml) was added with potassium carbonate (849 mg), and stirred at room temperature 72 hours. The reaction mixture was cooled to 0°C, added with water (30 ml), and extracted 3 times with ethyl acetate (50 ml for each time). The combined organic layer was washed 3 times with saturated brine (50 ml for each time), and dried over anhydrous sodium sulfate. The solvent was evaporated under reduced pressure, and the residue was purified by silica gel column chromatography (ethyl acetate:methanol: isopropylamine = 95:4.9:0.1). Then, the purified residue was added with a 4 N hydrogen chloride/1,4-dioxane solution (4 ml, Kokusan Kagaku), and stirred at room temperature 1.5 hours. The solvent,was evaporated under reduced pressure, and a suspension of the residue in methylene chloride (12 ml) was added with 2-chloro-1-methylpyridinium iodide (64 mg, Aldrich) and triethylamine (138 μl), and stirred at room temperature 48 hours. The solvent was evaporated under reduced pressure, and the residue was purified by silica gel column chromatography (ethyl acetate:methanol:isopropylamine = 95:4.9:0.1), then added with a 4 N hydrogen chloride/1,4-dioxane solution (0.2 ml), and stirred at room temperature 0.5 hour. The solvent was evaporated under reduced pressure to obtain the title compound as a hydrochloride (15 mg).
MS (m/z): 351 (MH+)

Example 1-37: 1-Acetyl-3-(2,3-dihydro-1,5-diazaphenalen-1-yl)piperidine

By using 3-(2,3-dihydro-1,5-diazaphenalen-1-yl)piperidine obtained in Example 1-1, Step D, acetylation was performed according to the method described in Example 1-22 to obtain the title compound.
MS (m/z): 296 (MH+)

Examples 1-38 to 1-72

The compounds of Examples 1-38 to 1-72 were obtained by using 3-(2,3-dihydro-1,5-diazaphenalen-1-yl) piperidine obtained in Example 1, Step D to perform alkylation or reductive alkylation according to the method described in Example 1-3, Method A or Method B, or Example 1-12.

Examples 1-73 and 1-74

The compounds of Examples 1-73 and 1-74 were obtained by using 3-(2,3-dihydro-1,5-diazaphenalen-1-yl) piperidine obtained in Example 1, Step D to perform condensation according to the method described in Example 1-5.

Examples 1-75 to 1-92

The compounds of Examples 1-75 to 1-92 were obtained by using 3-(2,3-dihydro-1,5-diazaphenalen-1-yl) piperidine obtained in Example 1, Step D to perform alkylation according to the method described in Example 1-6.

Example 1-93: 2-[3-(2,3-Dihydro-1,5-diazaphenalen-1-yl)piperidin-1-yl]ethyl N-methylcarbamate

A solution of 1-(2-hydroxyethyl)-3-(2,3-dihydro-1,5-diazaphenalen-1-yl)piperidine (297 mg) obtained in Example 1-10 and 4-dimethylaminopyridine (250 mg, Tokyo Kasei Kogyo) in N,N-dimethylformamide (20 ml) was added with 4-nitrophenyl carbonate (450 mg) under cooling on an ice bath, stirred at the same temperature for 30 minutes, and then further stirred at room temperature for 6 hours. The reaction mixture was added with a 2 N methylamine/tetrahydrofuran solution (2 ml, Aldrich) under cooling on an ice bath, and stirred at room temperature for 12 hours. The reaction

mixture was added with saturated brine, and extracted 4 times with ethyl acetate (20 ml for each time). The combined organic layer was washed twice with saturated brine (40 ml for each time), and dried over anhydrous magnesium sulfate. The organic layer was filtered through Celite, and then the solvent was evaporated under reduced pressure. The residue was purified by silica gel column chromatography (chloroform:methanol = 10:1) to obtain the title compound (201 mg).
MS (m/z): 355 (MH+)

Examples 1-94 to 1-100

The compounds of Examples 1-94 to 1-100 were obtained by using 1-(2-hydroxyethyl)-3-(2,3-dihydro-1,5-diazaphenalen-1-yl)piperidine obtained in Example 1-10, 1-(3-hydroxypropyl)-3-(2,3-dihydro-1,5-diazaphenalen-1-yl) piperidine obtained in Example 1-81, or 1-(4-hydroxybutyl)-3-(2,3-dihydro-1,5-diazaphenalen-1-yl)piperidine obtained in Example 1-87 to perform active esterification and then condensation with an amine according to the method described in Example 1-93.

Examples 1-101 and 1-102

The compounds of Examples 1-101 and 1-102 were obtained by using 1-(3-acetylthiopropyl)-3-(2,3-dihydro-1,5-diazaphenalen-1-yl)piperidine, or 1-(4-acetylthiobutyl)-3-(2,3-dihydro-1,5-diazaphenalen-1-yl)piperidine to perform hydrolysis according to the method described in Example 1-16.

Examples 1-103 to 1-105

The compounds of Examples 1-103 to 1-105 were obtained by using 1-(2-hydroxyethyl)-3-(2,3-dihydro-1,5-diazaphenalen-1-yl)piperidine obtained in Example 1-10, or 1-(3-hydroxypropyl)-3-(2,3-dihydro-1,5-diazaphenalen-1-yl)piperidine obtained in Example 1-81 to perform thioesterification according to the method described in Example 1-15.

Examples 1-106 to 1-108

The compounds of Examples 1-106 to 1-108 were obtained by using 3-(2,3-dihydro-1,5-diazaphenalen-1-yl) piperidine obtained in Example 1-1, Step D to perform alkylation according to the method described in Example 1-10.

Examples 1-109 to 1-124

The compounds of Examples 1-109 to 1-124 were obtained by using 1-(2-hydroxyethyl)-3-(2,3-dihydro-1,5-diazaphenalen-1-yl)piperidine obtained in Example 1-10, 1-(3-hydroxypropyl)-3-(2,3-dihydro-1,5-diazaphenalen-1-yl) piperidine obtained in Example 1-81, or 1-(4-hydroxybutyl)-3-(2,3-dihydro-1,5-diazaphenalen-1-yl)piperidine obtained in Example 1-87 to perform esterification according to the method described in Example 1-24.

Examples 1-125 to 1-136

The compounds of Examples 1-125 to 1-136 were obtained by using 3-(2,3-dihydro-1,5-diazaphenalen-1-yl) piperidine obtained in Example 1-1, Step D to perform alkylation, or alkylation and subsequent ureation according to the methods described in Examples 1-17 and 1-29.

Examples 1-137 to 1-141

The compounds of Examples 1-137 to 1-141 were obtained by using 3-(2,3-dihydro-1,5-diazaphenalen-1-yl) piperidine obtained in Example 1-1, Step D to perform alkylation according to the method described in Example 1-17.

Examples 1-142 to 1-151

The compounds of Examples 1-142 to 1-151 were obtained by using 3-(2,3-dihydro-1,5-diazaphenalen-1-yl) piperidine obtained in Example 1-1, Step D to perform alkylation, deprotection for the protective group, and reductive amination according to the method described in Example 1-19.

Examples 1-152 to 1-154

The compounds of Examples 1-152 to 1-154 were obtained by using 3-(2,3-dihydro-1,5-diazaphenalen-1-yl)piperidine obtained in Example 1-1, Step D to perform alkylation according to the method described in Example 1-6, Method A or Method B.

Examples 1-155 to 1-186

The compounds of Examples 1-155 to 1-186 were obtained by using 3-(2,3-dihydro-1,5-diazaphenalen-1-yl) piperidine obtained in Example 1-1, Step D to perform alkylation, deprotection for the protective group, reductive amination, and esterification according to the methods described in Example 1-19 and Example 1-24.

Example 1-187

The compound of Example 1-187 was obtained by using 3-(2,3-dihydro-1,5-diazaphenalen-1-yl)piperidine obtained in Example 1-1, Step D to perform reductive alkylation, and reduction according to the method described in Example 1-12.

Example 1-188

The compound of Examples 1-188 was obtained by using 3-(2,3-dihydro-1,5-diazaphenalen-1-yl)piperidine obtained in Example 1-1, Step D to perform reductive alkylation, and deprotection for the protective group according to the method described in Example 1-27.

Examples 1-189 to 1-191

The compounds of Examples 1-189 to 1-191 were obtained by using 1-(3-acetaminocyclobutyl)-3-(2,3-dihydro-1,5-diazaphenalen-1-yl)piperidine obtained in Example 1-188, or 1-(4-aminocyclohexyl)-3-(2,3-dihydro-1,5-diazaphenalen-1-yl)piperidine obtained in Example 1-27 to perform esterification according to the method described in Example 1-28.

Examples 1-192 to 1-194

The compounds of Examples 1-192 to 1-194 were obtained by using 3-(2,3-dihydro-1,5-diazaphenalen-1-yl) piperidine obtained in Example 1-1, Step D to perform reductive amination, and then deprotection for the protective group as required according to the method described in Example 1-12, or Example 1-27.

Examples 1-195 to 1-214

The compounds of Examples 1-195 to 1-214 were obtained by using 1-(2-aminoethyl)-3-(2,3-dihydro-1,5-diazaphenalen-1-yl)piperidine obtained in Example 1-17,
1-[2-(methylamino)ethyl]-3-(2,3-dihydro-1,5-diazaphenalen-1-yl)piperidine obtained in Example 1-142, 1-(3-aminopropyl)-3-(2,3-dihydro-1,5-diazaphenalen-1-yl)piperidine obtained in Example 1-18,
1-[3-(methylamino)propyl]-3-(2,3-dihydro-1,5-diazaphenalen-1-yl)piperidine obtained in Example 1-19, 1-(4-aminobutyl)-3-(2,3-dihydro-1,5-diazaphenalen-1-yl)piperidine obtained in Example 1-140,
1-[2-(2-hydroxyethyl)aminoethyl]-3-(2,3-dihydro-1,5-diazaphenalen-1-yl)piperidine obtained in Example 1-149,
1- [3-(2-hydroxyethyl)aminopropyl]-3-(2,3-dihydro-1,5-diazaphenalen-1-yl)piperidine obtained in Example 1-150, or
1- [4-(2-hydroxyethyl)aminobutyl] -3-(2,3-dihydro-1,5-diazaphenalen-1-yl)piperidine obtained in Example 1-151 to perform sulfonylation according to the method described in Example 1-26.

Example 1-215

The compound of Examples 1-215 was obtained by using 1-(3-aminocyclobutyl)-3-(2,3-dihydro-1,5-diazaphenalen-1-yl)piperidine obtained in Example 1-188 to perform sulfonylation according to the method described in Example 1-26.

Example 1-216

The compound of Examples 1-216 was obtained by performing reductive amination with methylamine instead of the reduction of carbonyl group in Example 1-12.

Example 1-217

The compound of Example 1-217 was obtained by using 1-(4-aminocyclohexyl)-3-(2,3-dihydro-1,5-diazaphenalen-1-yl)piperidine obtained in Example 1-28 to perform sulfonylation according to the method described in Example 1-26.

Example 1-218

The compound of Example 1-218 was obtained by performing reductive amination with dimethylamine instead of the reduction of carbonyl group in Example 1-12.

Examples 1-219 to 1-240 and 1-243 to 1-294

The compounds of Examples 1-219 to 1-240 and 1-243 to 1-294 were obtained by using 1-(2-aminoethyl)-3-(2,3-dihydro-1,5-diazaphenalen-1-yl)piperidine obtained in Example 1-17,
1-[2-(methylamino)ethyl]-3-(2,3-dihydro-1,5-diazaphenalen-1-yl)piperidine obtained in Example 1-142, 1-(3-aminopropyl)-3-(2,3-dihydro-1,5-diazaphenalen-1-yl)piperidine obtained in Example 1-18, or
1-[3-(methylamino)propyl]-3-(2,3-dihydro-1,5-diazaphenalen-1-yl)piperidine obtained in Example 1-19 to perform ureation according to the method described in Example 1-29, or 1-30.

Examples 1-241 and 1-242

The compounds of Examples 1-241 and 1-242 were obtained by using 3-(2,3-dihydro-1,5-diazaphenalen-1-yl) piperidine obtained in Example 1-1, Step D to perform reductive alkylation according to the method described in Example 1-3, Method B.

Examples 1-295 to 1-298 and 1-300 to 1-305

The compounds of Examples 1-295 to 1-298 and 1-300 to 1-305 were obtained by using 1-(2-aminoethyl)-3-(2,3-dihydro-1,5-diazaphenalen-1-yl)piperidine obtained in Example 1-17 to perform thioureation according to the method described in Example 1-31 or Example 1-32.

Example 1-299

The compound of Example 1-299 was obtained by using 3-(2,3-dihydro-1,5-diazaphenalen-1-yl)piperidine obtained in Example 1-1, Step D to perform reductive alkylation according to the method described in Example 1-3, Method B.

Example 1-306 to 1-313

The compounds of Examples 1-306 to 1-313 were obtained by using 1-(2-aminoethyl)-3-(2,3-dihydro-1,5-diazaphenalen-1-yl)piperidine obtained in Example 1-17 to perform carbamation according to the method described in Example 1-33.

Examples 1-314 and 1-315

The compounds of Examples 1-314 and 1-315 were obtained by using 3-(2,3-dihydro-1,5-diazaphenalen-1-yl) piperidine obtained in Example 1-1, Step D to perform reductive alkylation according to the method described in Example 1-4.

Examples 1-316 and 1-317

The compounds of Examples 1-316 and 1-317 were obtained by using 1-(2-aminoethyl)-3-(2,3-dihydro-1,5-diazaphenalen-1-yl)piperidine obtained in Example 1-17 to perform alkylation, deprotection for the protective group, and lactamation cyclization according to the method described in Example 1-36.

Example 1-318

The compound of Example 1-318 was obtained by using 3-(2,3-dihydro-1,5-diazaphenalen-1-yl)piperidine obtained in Example 1-1, Step D to perform reductive alkylation according to the method described in Example 1-12, or Example 1-27.

Example 1-319

The compound of Example 1-319 was obtained by using 1-(piperidin-4-yl)-3-(2,3-dihydro-1,5-diazaphenalen-1-yl)piperidine obtained in Example 1-194 to perform esterification according to the method described in Example 1-24.

Example 1-320

The compound of Example 1-320 was obtained by using 3-(2,3-dihydro-1,5-diazaphenalen-1-yl)piperidine obtained in Example 1-1, Step D to perform esterification according to the method described in Example 1-37.

Example 1-321: 6-Chloro-1-(piperidin-3-yl)-2,3-dihydro-1H-1,5-diazaphenalene

Step A

5-Nitro-4-vinylisoquinoline (Intermediate 4)

A suspension of 4-bromo-5-nitroisoquinoline (52 g) obtained in Reference Example 1, tri(n-butyl)vinyltin (105 g), tetrakis(triphenylphosphine)palladium(0) (4.8 g), and 2,6-di-tert-butyl-p-cresol (11.3 mg) in toluene (300 ml) was stirred at 110°C for 3 hours. The reaction mixture was cooled to room temperature, then added with 10% aqueous potassium fluoride (400 ml), and stirred at 0.5 hour. The reaction mixture was added with ethyl acetate (250 ml), the precipitates were separated by filtration, and then the organic layer was separated, and dried over anhydrous magnesium sulfate. The solvent was evaporated under reduced pressure, and then the residue was purified by silica gel column chromatography (n-hexane/ethyl acetate) to obtain the title compound (24 g).
$^1$H-NMR ($CDCl_3$) δ (ppm): 5.48 (1H, d, J=11Hz), 5.63 (1H, d, J=17Hz), 6.87 (1H, dd, J=11Hz, 17Hz), 7.68 (1H, t, J=8.1Hz), 7.94 (1H, d, J=8.1Hz), 8.20 (1H, d, J=8.1Hz), 8.64 (1H, s), 9.28 (1H, s)

Step B

5-Nitro-4-vinylisoquinoline N-oxide (Intermediate 5)

5-Nitro-4-vinylisoquinoline (Intermediate 4, 23 g) obtained in Step A mentioned above was dissolved in dichloromethane (400 ml), then slowly added with m-chloroperbenzoic acid (43 g, Tokyo Kasei Kogyo), and stirred for 3.5 hours. After cooling on ice, the reaction mixture was added with saturated aqueous sodium hydrogencarbonate for neutralization, and then the organic layer was separated, and dried over anhydrous magnesium sulfate. The solvent was evaporated under reduced pressure to obtain the title compound (27 g).

Step C

1-Chloro-5-nitro-4-vinylisoquinoline (Intermediate 6), 3-chloro-5-nitro-4-vinylisoquinoline (Intermediate 7)

5-nitro-4-vinylisoquinoline N-oxide (Intermediate 5, 54.05 g) obtained in Step B mentioned above was suspended in chloroform (300 ml), and added dropwise with phosphorus oxychloride (46.6 ml, Wako Pure Chemical Industries) under cooling on ice. After the addition, the reaction mixture was warmed to 60°C, and stirred at the same temperature for 1 hour. The reaction mixture was poured into ice water, and added with 5 N aqueous sodium hydroxide (500 ml) with stirring for neutralization. The organic layer was separated, and the aqueous layer was extracted with chloroform (300 ml), and then dried over anhydrous magnesium sulfate. The organic layer was filtered through Celite, and then the solvent was evaporated under reduced pressure. The residue was purified by silica gel column chromatography (n-hexane:ethyl acetate = 3:1) to obtain 1-chloro-5-nitro-4-vinylisoquinoline (40.80 g) and 3-chloro-5-nitro-4-vinylisoquinoline (0.68 g).
1-Chloro compound
$^1$H-NMR ($CDCl_3$) δ (ppm): 5.49 (1H, d, J=11Hz), 5.63 (1H, d, J=17Hz), 6.81 (1H, dd, J=11Hz, 17Hz), 7.77 (1H, t, J=8.1Hz), 7.97 (1H, d, J=8.1Hz), 8.36 (1H, s), 8.64 (1H, d, J=8.1Hz)
MS (m/z): 235 (MH+)
3-Chloro compound
$^1$H-NMR ($CDCl_3$) δ (ppm): 5.10 (1H, d, J=17Hz), 5.5.47 (1H, d, J=11Hz), 6.92 (1H, dd, J=11Hz, 17Hz), 7.67 (1H, t,

J=8.1Hz), 8.01 (1H, d, J=8.1Hz), 8.20 (1H, d, J=8.1Hz), 9.11 (1H, s)
MS (m/z): 235 (MH+)

Step D

5-Amino-1-chloro-4-vinylisoquinoline (Intermediate 8)

1-Chloro-5-nitro-4-vinylisoquinoline (Intermediate 6, 15 g) obtained in Step C mentioned above was dissolved in ethyl acetate (700 ml), added with stannous chloride dihydrate (72 g), and stirred for 2 hours. The reaction mixture was poured onto ice, and then added with 5 N aqueous sodium hydroxide, and the organic layer was separated, and dried over anhydrous sodium sulfate. The solvent was evaporated under reduced pressure, and then the residue was purified by silica gel column chromatography (n-hexane:ethyl acetate = 3:1) to obtain the title compound (10 g). $^{1}$H-NMR ($CDCl_3$) δ (ppm): 5.54 (1H, dd, J=1.5, 10.8Hz), 5.65 (1H, dd, J=1.5, 17.1Hz)6.92 (1H, d, J=7.8Hz), 7.41-7.64 (2H, m), 7.77 (1H, d, J=8.7Hz), 7.94 (1H, s) MS (m/z): 205 (MH+)

Step E

3-(1-Chloro-4-vinylisoquinoline-5-yl)aminopiperidine-1-carboxylic acid tert-butyl ester (Intermediate 9)

A suspension of 5-amino-1-chloro-4-vinylisoquinoline (Intermediate 8, 5.14 g) obtained in Step D mentioned above, tert-butyl 3-oxo-1-piperidinecarboxylate (5.00 g), and anhydrous sodium sulfate (21.39 g) in glacial acetic acid (150 ml) was stirred at room temperature for 30 minutes, then added with sodium triacetoxyborohydride (6.38 g) under cooling on an ice bath, and stirred at room temperature for 12 hours. After insoluble solids were removed by filtration through Celite, the solvent was evaporated under reduced pressure. The residue was added with ethyl acetate (200 ml), and slowly added with saturated aqueous sodium hydrogencarbonate under cooling on an ice bath for neutralization (pH 7). After insoluble solids were removed by filtration through Celite, the organic layer was separated, and the aqueous layer was extracted twice with ethyl acetate (100 ml for each time). The combined organic layer was washed with saturated brine (200 ml), and then dried over anhydrous magnesium sulfate. After filtration through Celite, the solvent was evaporated under reduced pressure, and the residue was purified by silica gel column chromatography (n-hexane: ethyl acetate = 4:1) to obtain the title compound (5.46 g).
MS (m/z): 388 (MH+)

Step F

3-(6-Chloro-2,3-dihydro-1,5-diazaphenalen-1-yl)piperidine-1-carboxylic acid tert-butyl ester (Intermediate 10)

A solution of 3-(1-chloro-4-vinylisoquinoline-5-yl)aminopiperidine-1-carboxylic acid tert-butyl ester (Intermediate 9, 5.46 g) obtained in Step E mentioned above in tetrahydrofuran (140 ml) was added with potassium tert-butoxide (3.16 g), and stirred at 40°C for 2 hours. The reaction mixture was left to cool to room temperature, and then added with toluene (280 ml) and then with a saturated solution of citric acid monohydrate (2.96 g) in tetrahydrofuran under cooling on an ice bath for neutralization (pH 7). After insoluble solids were removed by filtration through Celite, the organic layer was separated, and the aqueous layer was extracted twice with ethyl acetate (300 ml for each time). The combined organic layer was washed with saturated brine (300 ml), and dried over anhydrous magnesium sulfate. The organic layer was filtered through Celite, and then the solvent was evaporated under reduced pressure. The residue was purified by silica gel column chromatography (n-hexane:ethyl acetate = 3:1) to obtain the title compound (2.13 g).
MS (m/z): 388 (MH+)

Step G

6-Chloro-1-(piperidin-3-yl)-2,3-dihydro-1H-1,5-diazaphenalene

A solution of 3-(6-chloro-2,3-dihydro-1,5-diazaphenalen-1-yl)piperidine-1-carboxylic acid tert-butyl ester (Intermediate 10, 2.12 g) mentioned above in a 2 N hydrogen chloride/1,4-dioxane solution (220 ml) was stirred at room temperature for 2 hours. The solvent was evaporated under reduced pressure to obtain the title compound as a hydrochloride (1.90 g).
MS (m/z): 288 (MH+)

Example 1-322

The compound of Example 1-322 was obtained according to the method described in Example 1-321.

Example 1-323: 6-Hydroxy-1-(piperidin-3-yl)-2,3-dihydro-1H-1,5-diazaphenalene

The compound of Example 1-323 can be prepared by Method A or Method B mentioned below.

(Method A) A solution of 3-(6-chloro-2,3-dihydro-1,5-diazaphenalen-1-yl)piperidine-1-carboxylic acid tert-butyl ester (Intermediate 10, 3.51 g) obtained in Example 1-321, Step F in concentrated hydrochloric acid (60 ml) was stirred at 90°C for 17 hours. After completion of the reaction, the solvent was evaporated under reduced pressure to obtain the title compound as a hydrochloride (2.11 g).
(Method B) 25% Hydrogen bromide/acetic acid (5 ml, Wako Pure Chemical Industries) was added with 3-(6-methoxy-2,3-dihydro-1,5-diazaphenalen-1-yl)piperidine-1-carboxylic acid tert-butyl ester (Intermediate 14, 192 mg) obtained in Example 1-327, Step A under cooling on an ice bath, stirred at the same temperature for 30 minutes, and further stirred at room temperature 12 hours, and then the solvent was evaporated under reduced pressure to obtain the title compound as a hydrobromide (160 mg).
MS (m/z): 270 (MH+)

Example 1-324: 6-Methyl-1-(piperidin-3-yl)-2,3-dihydro-1H-1,5-diazaphenalene

Step A

3-(6-Methyl-2,3-dihydro-1,5-diazaphenalen-1-yl)piperidine-1-carboxylic acid tert-butyl ester (Intermediate 11)

From 3-(6-chloro-2,3-dihydro-1,5-diazaphenalen-1-yl)piperidine-1-carboxylic acid tert-butyl ester (Intermediate 10) obtained in Example 1-321, Step F, the title compound was obtained by referring to a known publication (for example, Hollenbaugh, Dian L. et al., Bioorg. Med. Chem. Lett., 13, 1345 (2003) and the like).
MS (m/z): 368 (MH+)

Step B

6-Methyl-1-(piperidin-3-yl)-2,3-dihydro-1H-1,5-diazaphenalene

By using 3-(6-methyl-2,3-dihydro-1,5-diazaphenalen-1-yl)piperidine-1-carboxylic acid tert-butyl ester (Intermediate 11) obtained in Step A mentioned above, the title compound was obtained as a hydrochloride according to the method described in Example 1-1, Step D.
MS (m/z): 268 (MH+)

Example 1-325: 6-Vinyl-1-(piperidin-3-yl)-2,3-dihydro-1H-1,5-diazaphenalene

Step A

3-(6-Vinyl-2,3-dihydro-1,5-diazaphenalen-1-yl)piperidine-1-carboxylic acid tert-butyl ester (Intermediate 12)

From 3-(6-chloro-2,3-dihydro-1,5-diazaphenalen-1-yl)piperidine-1-carboxylic acid tert-butyl ester (Intermediate 10) obtained in Example 1-321, Step F, the title compound was obtained by referring to a known publication (for example, Carreno, M.C., Mahugo, R.H-S.J., J. Org. Chem., 64, 1387 (1999) and the like).
MS (m/z) : 380 (MH+)

Step B

6-Vinyl-1-(piperidin-3-yl)-2,3-dihydro-1H-1,5-diazaphenalene

By using 3-(6-vinyl-2,3-dihydro-1,5-diazaphenalen-1-yl)piperidine-1-carboxylic acid tert-butyl ester (Intermediate 12) obtained in Step A mentioned above, the title compound was obtained as a hydrochloride according to the method described in Example 1-1, Step D.
MS (m/z): 280 (MH+)

Example 1-326:

6-(2-Phenylethynyl)-1-(piperidin-3-yl)-2,3-dihydro-1H-1,5-diazaphenalene

Step A

3-[6-(2-Phenylethynyl)-2,3-dihydro-1,5-diazaphenalen-1-yl]piperidine-1-carboxylic acid tert-butyl ester (Intermediate 13)

By using 3-(6-chloro-2,3-dihydro-1,5-diazaphenalen-1-yl)piperidine-1-carboxylic acid tert-butyl ester (Intermediate 10) obtained in Example 1-321, Step F, the title compound was obtained with referring to a known publication (for example, Hundertmark, T., Littke, A.F., Buchwald, S.L., Fu, G.C., Org. Lett., 2, 1729 (2000) and the like).
MS (m/z): 454 (MH+)

Step B

6-(2-Phenylethynyl)-1-(piperidin-3-yl)-2,3-dihydro-1H-1,5-diazaphenalene

By using 3-[6-(2-phenylethynyl)-2,3-dihydro-1,5-diazaphenalen-1-yl]piperidine-1-carboxylic acid tert-butyl ester (Intermediate 13) obtained in Step A mentioned above, the title compound was obtained as a hydrochloride according to the method described in Example 1-1, Step D.
MS (m/z): 354 (MH+)

Example 1-327: 6-Methoxy-1-(piperidin-3-yl)-2,3-dihydro-1H-1,5-diazaphenalene

Step A

3-(6-Methoxy-2,3-dihydro-1,5-diazaphenalen-1-yl)piperidine-1-carboxylic acid tert-butyl ester (Intermediate 14)

By using 3-(6-chloro-2,3-dihydro-1,5-diazaphenalen-1-yl)piperidine-1-carboxylic acid tert-butyl ester (Intermediate 10) obtained in Example 1-321, Step F, the title compound was obtained with referring to a known publication (for example, Toracca, K.E., Huang, X., Parrish, C.A., Buchwald, S.L., J. Am. Chem. Soc., 123, 10770 (2001) and the like).
MS (m/z): 384 (MH+)

Step B

6-Methoxy-1-(piperidin-3-yl)-2,3-dihydro-1H-1,5-diazaphenalene

By using 3-(6-methoxy-2,3-dihydro-1,5-diazaphenalen-1-yl)piperidine-1-carboxylic acid tert-butyl ester (Intermediate 14) obtained in Step A mentioned above, the title compound was obtained as a hydrochloride according to the method described in Example 1-1, Step D.
MS (m/z): 284 (MH+)

Example 1-328: 6-Amino-1-(piperidin-3-yl)-2,3-dihydro-1H-1,5-diazaphenalene

Step A

3-(6-Amino-2,3-dihydro-1,5-diazaphenalen-1-yl)piperidine-1-carboxylic acid tert-butyl ester (Intermediate 15)

By using 3-(6-chloro-2,3-dihydro-1,5-diazaphenalen-1-yl)piperidine-1-carboxylic acid tert-butyl ester (Intermediate 10) obtained in Example 1-321, Step F, the title compound was obtained with referring to a known publication (for example, Klapars, A., Huang, X., Buchwald, S.L., J. Am. Chem. Soc., 124, 7421 (2002) and the like).
MS (m/z): 369 (MH+)

Step B

6-Amino-1-(piperidin-3-yl)-2,3-dihydro-1H-1,5-diazaphenalene

By using 3-(6-amino-2,3-dihydro-1,5-diazaphenalen-1-yl)piperidine-1-carboxylic acid tert-butyl ester (Intermediate 15) obtained in Step A mentioned above, the title compound was obtained as a hydrochloride according to the method described in Example 1-1, Step D.
MS (m/z): 269 (MH+)

Example 1-329:

6-(Methylamino)-1-(piperidin-3-yl)-2,3-dihydro-1H-1,5-diazaphenalene

Step A

3-[6-(Methylamino)-2,3-dihydro-1,5-diazaphenalen-1-yl]piperidine-1-carboxylic acid tert-butyl ester (Intermediate 16)

By using 3-(6-chloro-2,3-dihydro-1,5-diazaphenalen-1-yl)piperidine-1-carboxylic acid tert-butyl ester (Intermediate 10) obtained in Example 1-321, Step F, the title compound was obtained with referring to a known publication (for example, Buchwald, S.L., Zim, D., Org. Lett.5, 2413 (2003) and the like).
MS (m/z): 383 (MH+)

Step B

6-(Methylamino)-1-(piperidin-3-yl)-2,3-dihydro-1H-1,5-diazaphenalene

By using 3-[6-(methylamino)-2,3-dihydro-1,5-diazaphenalen-1-yl]piperidine-1-carboxylic acid tert-butyl ester (Intermediate 16) obtained in Step A mentioned above, the title compound was obtained as a hydrochloride according to the method described in Example 1-1, Step D.
MS (m/z): 283 (MH+)

Example 1-330:

6-(Dimethylamino)-1-(piperidin- 3-yl)-2,3-dihydro-1H-1,5-diazaphenalene

Step A

3-[6-(Dimethylamino)-2,3-dihydro-1,5-diazaphenalen-1-yl]piperidine-1-carboxylic acid tert-butyl ester (Intermediate 17)

By using 3-(6-chloro-2,3-dihydro-1,5-diazaphenalen-1-yl)piperidine-1-carboxylic acid tert-butyl ester (Intermediate 10) obtained in Example 1-321, Step F, the title compound was obtained with referring to a known publication (for example, Buchwald, S.L., Zim, D., Org. Lett., 5, 2413 (2003) and the like).
MS (m/z): 397 (MH+)

Step B

6-(Dimethylamino)-1-(piperidin-3-yl)-2,3-dihydro-1H-1,5-diazaphenalene

From 3-[6-dimethylamino-2,3-dihydro-1,5-diazaphenalen-1-yl]piperidine-1-carboxylic acid tert-butyl ester (Intermediate 17) obtained in Step A mentioned above, the title compound was obtained as a hydrochloride according to the method described in Example 1-1, Step D.
MS (m/z): 297 (MH+)

Example 1-331: 6-Anilino-1-(piperidin-3-yl)-2,3-dihydro-1H-1,5-diazaphenalene

Step A

3-(6-Anilino-2,3-dihydro-1,5-diazaphenalen-1-yl)piperidine-1-carboxylic acid tert-butyl ester (Intermediate 18)

By using 3-(6-chloro-2,3-dihydro-1,5-diazaphenalen-1-yl)piperidine-1-carboxylic acid tert-butyl ester (Intermediate 10) obtained in Example 1-321, Step F, the title compound was obtained with referring to a known publication (for example, Buchwald, S.L., Zim, D., Org. Lett., 5, 2413 (2003) and the like).
MS (m/z) : 445 (MH+)

Step B

6-Anilino-1-(piperidin-3-yl)-2,3-dihydro-1H-1,5-diazaphenalene

By using 3-(6-anilino-2,3-dihydro-1,5-diazaphenalen-1-yl)piperidine-1-carboxylic acid tert-butyl ester (Intermediate 18) obtained in Step A mentioned above, the title compound was obtained as a hydrochloride according to the method described in Example 1-1, Step D.
MS (m/z): 345 (MH+)

Examples 1-332 to 1-650

The compounds of Examples 1-332 to 1-650 were obtained by using 6-chloro-1-(piperidin-3-yl)-2,3-dihydro-1H-1,5-diazaphenalene obtained in Example 1-321, or 6-hydroxy-1-(piperidin-3-yl)-2,3-dihydro-1H-1,5-diazaphenalene obtained in Example 1-323 according to the methods described in Example 1-2 to 1-320.

Example 1-651: 4-Chloro-1-(piperidin-3-yl)-2,3-dihydro-1H-1,5-diazaphenalene

Step A

3-(4-Chloro-2,3-dihydro-1,5-diazaphenalen-1-yl)piperidine-1-carboxylic acid tert-butyl ester (Intermediate 19)

By using 3-chloro-5-nitro-4-vinylisoquinoline (Intermediate 7) obtained in Example 1-321, Step C, the title compound was obtained according to the methods described in Example 1-321, Steps D to F.
MS (m/z): 388 (MH+)

Step B

4-Chloro-1-(piperidin-3-yl)-2,3-dihydro-1H-1,5-diazaphenalene

By using 3-(4-chloro-2,3-dihydro-1,5-diazaphenalen-1-yl)piperidine-1-carboxylic acid tert-butyl ester (Intermediate 19) obtained in Step A mentioned above, the title compound was obtained as a hydrochloride according to the method described in Example 1-1, Step D.
MS (m/z): 288 (MH+)

Example 1-652

The compound of Example 1-652 was obtained according to the method described in Example 1-651.

Example 1-653

The compound of Example 1-653 was obtained according to Method A or Method B of Example 1-323,.

Examples 1-654 to 1-661

The compounds of Example 1-654 to 1-661 were obtained by using 3-(4-chloro-2,3-dihydro-1,5-diazaphenalen-1-yl)piperidine-1-carboxylic acid tert-butyl ester according to the methods described in Examples 1-324 to 1-331.

Example 1-662

The compound of Example 1-662 was obtained according to the method described in Example 1-663 mentioned below.

Example 1-663: 7-Bromo-1-(piperidin-3-yl)-2,3-dihydro-1H-1,5-diazaphenalene

Step A

3-(7-Bromo-2,3-dihydro-1,5-diazaphenalen-1-yl)piperidine-1-carboxylic acid tert-butyl ester (Intermediate 20), and

3-(8-bromo-2,3-dihydro-1,5-diazaphenalen-1-yl)piperidine-1-carboxylic acid tert-butyl ester (Intermediate 21)

A solution of 3-(2,3-dihydro-1,5-diazaphenalen-1-yl)piperidine-1-carboxylic acid tert-butyl ester (Intermediate 3, 1.06 g) obtained in Example 1-1, Step C in N,N-dimethylformamide (9 ml) was added with N-bromosuccinimide (634 mg, Tokyo Kasei Kogyo) under cooling on an ice bath, and stirred at the same temperature for 30 minutes. The reaction mixture was further stirred at room temperature 5 hours, and then the reaction mixture was poured into ice water, and extracted twice with ethyl acetate (100 ml for each time). The combined organic layer was washed twice with saturated brine (100 ml for each time), and dried over anhydrous magnesium sulfate. The organic layer was filtered through Celite, and then the solvent was evaporated under reduced pressure. The residue was purified by silica gel column chromatography (n-hexane:ethyl acetate =2:1), and the obtained solid was recrystallized (heptane/ethyl acetate) to obtain the title compound (7-bromo compound : 429mg, 8-bromo compound: 31 mg).
MS (m/z) : 432 (MH+)

Step B

7-Bromo-1-(piperidin-3-yl)-2,3-dihydro-1H-1,5-diazaphenalene

By using 3-(7-bromo-2,3-dihydro-1,5-diazaphenalen-1-yl)piperidine-1-carboxylic acid tert-butyl ester (Intermediate 20, 215 mg) obtained in Step A mentioned above, the title compound was obtained as a hydrochloride (162 mg) according to the method described in Example 1-1, Step D.
MS (m/z): 332 (MH+)

Examples 1-664 to 1-672

The compounds of Examples 1-664 to 1-672 were obtained by using 3-(7-bromo-2,3-dihydro-1,5-diazaphenalen-1-yl)piperidine-1-carboxylic acid tert-butyl ester obtained in Example 1-663, Step A according to the methods described in Examples 1-323 to 1-331.

Example 1-673

The compound of Example 1-673 was obtained according to the method described in Example 1-674 mentioned below.

Example 1-674: 8-Bromo-1-(piperidin-3-yl)-2,3-dihydro-1H-1,5-diazaphenalene

By using 3-(8-bromo-2,3-dihydro-1,5-diazaphenalen-1-yl)piperidine-1-carboxylic acid tert-butyl ester (Intermediate 21, 29 mg) obtained in Example 1-663, Step A, the title compound was obtained as a hydrochloride (20 mg) according to the method described in Example 1-1, Step D.
MS (m/z): 332 (MH+)

Examples 1-675 to 1-683

The compounds of Examples 1-675 to 1-683 were obtained by using 3-(8-bromo-2,3-dihydro-1,5-diazaphenalen-1-yl)piperidine-1-carboxylic acid tert-butyl ester obtained in Example 1-663, Step A according to the methods described in Examples 1-323 to 1-331.

Examples 1-684 and 1-685

The compounds of Examples 1-684 and 1-685 were obtained from the compound of Example 1-691 mentioned below by referring to a known diazotization-Sandmeyer reaction (for example, Denny, William., et al., J. Med. Chem., 2002, 740; Kulka, J. Am. Chem. Soc., 75, 3597 (1953) and the like).

Examples 1-686 to 1-690

The compounds of Examples 1-686 to 1-690 were obtained by using 3-(9-bromo-2,3-dihydro-1,5-diazaphenalen-1-yl)piperidine-1-carboxylic acid tert-butyl ester obtained in Example 1-685 according to the methods described in Example 1-323 to 1-327.

Example 1-691: 9-Amino-1-(piperidin-3-yl)-2,3-dihydro-1H-1,5-diazaphenalene

Step A

3-(9-Amino-2,3-dihydro-1,5-diazaphenalen-1-yl)piperidine-1-carboxylic acid tert-butyl ester (Intermediate 22)

By using 3-(7-bromo-2,3-dihydro-1,5-diazaphenalen-1-yl)piperidine-1-carboxylic acid tert-butyl ester (Intermediate 20) obtained in Example 1-663, Step A, nitration (9-position), and reduction (bromine atom→hydrogen atom at the 7-position, nitro group→amino group at the 9-position) were performed with referring to a known publication (for example, Kucznierz, R., Dickhaut, J, Leinert, H., Von Der Saal, W., Synth. Commun., 29, 1617 (1999); Ping Chen, Bioorganic & Medicinal Chemistry Letters, 13, 1345 (2003) and the like) to obtain the title compound.
MS (m/z): 369 (MH+)

Step B

9-Amino-1-(piperidin-3-yl)-2,3-dihydro-1H-1,5-diazaphenalene

By using 3-(9-amino-2,3-dihydro-1,5-diazaphenalen-1-yl)piperidine-1-carboxylic acid tert-butyl ester (Intermediate 22) obtained in Step A mentioned above the title compound was obtained as a hydrochloride according to the method described in Example 1-1, Step D.
MS (m/z): 269 (MH+)

Examples 1-692 to 1-694

The compounds of Examples 1-692 to 1-694 were obtained by using 3-(9-bromo-2,3-dihydro-1,5-diazaphenalen-1-yl)piperidine-1-carboxylic acid tert-butyl ester obtained in Example 1-685 according to the methods described in Example 1-329 to 1-331.
[Table 1]

| Exp. No. | $R^1$ | $R^5$ | $R^8$ | $R^7$ | $R^6$ | $A^{61}$ | Mass (MH+) |
|---|---|---|---|---|---|---|---|
| 1-1 | H | H | H | H | H | H | 254 |
| 1-2 | H | H | H | H | H | Me | 268 |
| 1-3 | H | H | H | H | H | a-1 | 336 |
| 1-4 | H | H | H | H | H | a-2 | 374 |
| 1-5 | H | H | H | H | H | $C_6H_5$ | 330 |
| 1-6 | H | H | H | H | H | $CH_2COOH$ | 312 |
| 1-7 | H | H | H | H | H | $CH_2CH_2COOH$ | 326 |
| 1-8 | H | H | H | H | H | $CH_2CN$ | 293 |
| 1-9 | H | H | H | H | H | $CH_2CH_2CN$ | 307 |
| 1-10 | H | H | H | H | H | $CH_2CH_2OH$ | 298 |
| 1-11 | H | H | H | H | H | $CH(CH_2OH)_2$ | 328 |
| 1-12 | H | H | H | H | H | a-3 | 352 |
| 1-13 | H | H | H | H | H | $CH_2CH_2OMe$ | 312 |
| 1-14 | H | H | H | H | H | $CH_2CH_2OCOCMe_3$ | 382 |
| 1-15 | H | H | H | H | H | $CH_2CH_2SCOMe$ | 356 |
| 1-16 | H | H | H | H | H | $CH_2CH_2SH$ | 314 |
| 1-17 | H | H | H | H | H | $CH_2CH_2NH_2$ | 297 |
| 1-18 | H | H | H | H | H | $CH_2CH_2CH_2NH_2$ | 311 |
| 1-19 | H | H | H | H | H | $CH_2CH_2CH_2NHMe$ | 325 |
| 1-20 | H | H | H | H | H | $CH(CH_2NHCOMe)_2$ | 410 |
| 1-21 | H | H | H | H | H | $CH (CH_2NH_2) _2$ | 326 |
| 1-22 | H | H | H | H | H | $CH_2CH_2NHCOMe$ | 339 |
| 1-23 | H | H | H | H | H | a-4 | 352 |
| 1-24 | H | H | H | H | H | $CH_2CH_2NHCOCH_2NHCOMe$ | 396 |
| 1-25 | H | H | H | H | H | $CH_2CH_2NHCOCH_2NMe_2$ | 382 |
| 1-26 | H | H | H | H | H | $CH_2CH_2NHSO_2Me$ | 375 |
| 1-27 | H | H | H | H | H | a-5 | 351 |
| 1-28 | H | H | H | H | H | a-6 | 393 |
| 1-29 | H | H | H | H | H | $CH_2CH_2CH_2NHCONHCH_2CH_2OH$ | 398 |
| 1-30 | H | H | H | H | H | $CH_2CH_2CH_2NHCONHBn$ | 444 |
| 1-31 | H | H | H | H | H | $CH_2CH_2NHCSNHCH_2CH_2OH$ | 400 |
| 1-32 | H | H | H | H | H | $CH_2CH_2NHCSNHC_6H_5$ | 432 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-33 | H | H | H | H | H | $CH_2CH_2NHCOOCH_2CH_2NMe_2$ | 412 |
| 1-34 | H | H | H | H | H | a-7 | 409 |
| 1-35 | H | H | H | H | H | a-8 | 296 |
| 1-36 | H | H | H | H | H | a-9 | 351 |
| 1-37 | H | H | H | H | H | COMe | 296 |
| 1-38 | H | H | H | H | H | a-10 | 308 |
| 1-39 | H | H | H | H | H | a-11 | 322 |
| 1-40 | H | H | H | H | H | a-12 | 336 |
| 1-41 | H | H | H | H | H | Bn | 344 |
| 1-42 | H | H | H | H | H | a-13 | 378 |
| 1-43 | H | H | H | H | H | a-14 | 360 |
| 1-44 | H | H | H | H | H | a-15 | 404 |
| 1-45 | H | H | H | H | H | a-16 | 418 |
| 1-46 | H | H | H | H | H | a-17 | 434 |
| 1-47 | H | H | H | H | H | a-18 | 411 |
| 1-48 | H | H | H | H | H | a-19 | 429 |
| 1-49 | H | H | H | H | H | a-20 | 420 |
| 1-50 | H | H | H | H | H | a-21 | 359 |
| 1-51 | H | H | H | H | H | a-22 | 373 |
| 1-52 | H | H | H | H | H | a-23 | 387 |
| 1-53 | H | H | H | H | H | a-24 | 403 |
| 1-54 | H | H | H | H | H | a-25 | 417 |
| 1-55 | H | H | H | H | H | a-26 | 447 |
| 1-56 | H | H | H | H | H | a-27 | 401 |
| 1-57 | H | H | H | H | H | a-28 | 437 |
| 1-58 | H | H | H | H | H | a-29 | 446 |
| 1-59 | H | H | H | H | H | a-30 | 388 |
| 1-60 | H | H | H | H | H | a-31 | 404 |
| 1-61 | H | H | H | H | H | a-32 | 374 |
| 1-62 | H | H | H | H | H | a-33 | 374 |
| 1-63 | H | H | H | H | H | a-34 | 404 |
| 1-64 | H | H | H | H | H | a-35 | 404 |
| 1-65 | H | H | H | H | H | a-36 | 434 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-66 | H | H | H | H | H | a-37 | 434 |
| 1-67 | H | H | H | H | H | a-38 | 332 |
| 1-68 | H | H | H | H | H | a-39 | 334 |
| 1-69 | H | H | H | H | H | a-40 | 350 |
| 1-70 | H | H | H | H | H | a-41 | 402 |
| 1-71 | H | H | H | H | H | a-42 | 389 |
| 1-72 | H | H | H | H | H | a-43 | 345 |
| 1-73 | H | H | H | H | H | a-44 | 331 |
| 1-74 | H | H | H | H | H | a-45 | 346 |
| 1-75 | H | H | H | H | H | $CH_2CH_2CH_2COOH$ | 340 |
| 1-76 | H | H | H | H | H | $CH_2CH_2CH_2CH_2COOH$ | 354 |
| 1-77 | H | H | H | H | H | $CH_2CH_2CH_2CN$ | 321 |
| 1-78 | H | H | H | H | H | $CH_2CH_2CH_2CH_2CN$ | 335 |
| 1-79 | H | H | H | H | H | $CH_2CH(Me)OH$ | 312 |
| 1-80 | H | H | H | H | H | $CH(Me)CH_2OH$ | 312 |
| 1-81 | H | H | H | H | H | $CH_2CH_2CH_2OH$ | 312 |
| 1-82 | H | H | H | H | H | $CH_2CH$ (OH) $CH_2OH$ | 328 |
| 1-83 | H | H | H | H | H | $CH_2CH$ ($NH_2$) $CH_2OH$ | 327 |
| 1-84 | H | H | H | H | H | $CH_2CH$ (NHMe) $CH_2OH$ | 341 |
| 1-85 | H | H | H | H | H | $CH_2CH$ ($NMe_2$) $CH_2OH$ | 355 |
| 1-86 | H | H | H | H | H | $CH_2CH$ (Me) $CH_2OH$ | 326 |
| 1-87 | H | H | H | H | H | $CH_2CH_2CH_2CH_2OH$ | 326 |
| 1-88 | H | H | H | H | H | $CH_2CH_2CH_2CH_2CH_2OH$ | 340 |
| 1-89 | H | H | H | H | H | a-46 | 466 |
| 1-90 | H | H | H | H | H | $CH(CH_2OH)CH_2CH_2NHCONHC_6H_5$ | 460 |
| 1-91 | H | H | H | H | H | a-47 | 452 |
| 1-92 | H | H | H | H | H | $CH_2CH(OH)CH_2NHCONHC_6H_5$ | 446 |
| 1-93 | H | H | H | H | H | $CH_2CH_2OCONHMe$ | 355 |
| 1-94 | H | H | H | H | H | $CH_2CH_2OCONHC_6H_5$ | 417 |
| 1-95 | H | H | H | H | H | $CH_2CH_2CH_2OCONHMe$ | 369 |
| 1-96 | H | H | H | H | H | a-48 | 437 |
| 1-97 | H | H | H | H | H | $CH_2CH_2CH_2OCONHC_6H_5$ | 431 |
| 1-98 | H | H | H | H | H | $CH_2CH_2CH_2CH_2OCONHMe$ | 383 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-99 | H | H | H | H | H | a-49 | 451 |
| 1-100 | H | H | H | H | H | $CH_2CH_2CH_2CH_2OCONHC_6H_5$ | 445 |
| 1-101 | H | H | H | H | H | $CH_2CH_2CH_2SH$ | 328 |
| 1-102 | H | H | H | H | H | $CH_2CH_2CH_2CH_2SH$ | 342 |
| 1-103 | H | H | H | H | H | $CH_2CH_2SCOC_6H_5$ | 418 |
| 1-104 | H | H | H | H | H | $CH_2CH_2CH_2SCOMe$ | 370 |
| 1-105 | H | H | H | H | H | $CH_2CH_2CH_2SCOC_6H_5$ | 432 |
| 1-106 | H | H | H | H | H | $CH_2CH_2CH_2OMe$ | 326 |
| 1-107 | H | H | H | H | H | $CH_2CH_2CH_2CH_2OMe$ | 340 |
| 1-108 | H | H | H | H | H | $CH_2CH_2CH_2CH_2CH_2OMe$ | 354 |
| 1-109 | H | H | H | H | H | $CH_2CH_2OCOMe$ | 340 |
| 1-110 | H | H | H | H | H | $CH_2CH_2CH_2OCOMe$ | 354 |
| 1-111 | H | H | H | H | H | $CH_2CH_2CH_2CH_2OCOMe$ | 368 |
| 1-112 | H | H | H | H | H | $CH_2CH_2OCOCH_2Me$ | 354 |
| 1-113 | H | H | H | H | H | $CH_2CH_2CH_2OCOCH_2Me$ | 368 |
| 1-114 | H | H | H | H | H | $CH_2CH_2CH_2CH_2OCOCH_2Me$ | 382 |
| 1-115 | H | H | H | H | H | $CH_2CH_2OCOCHMe_2$ | 368 |
| 1-116 | H | H | H | H | H | $CH_2CH_2CH_2OCOCHMe_2$ | 382 |
| 1-117 | H | H | H | H | H | $CH_2CH_2CH_2CH_2OCOCHMe_2$ | 396 |
| 1-118 | H | H | H | H | H | $CH_2CH_2CH_2OCOCMe_3$ | 396 |
| 1-119 | H | H | H | H | H | $CH_2CH_2CH_2CH_2OCOCMe_3$ | 410 |
| 1-120 | H | H | H | H | H | $CH_2CH_2OCOC_6H_5$ | 402 |
| 1-121 | H | H | H | H | H | $CH_2CH_2CH_2OCOC_6H_5$ | 416 |
| 1-122 | H | H | H | H | H | $CH_2CH_2CH_2CH_2OCOC_6H_5$ | 430 |
| 1-123 | H | H | H | H | H | $CH_2CH_2OCOCH_2OH$ | 356 |
| 1-124 | H | H | H | H | H | $CH_2CH_2OCOCH_2NH_2$ | 355 |
| 1-125 | H | H | H | H | H | $CH_2CH_2OCH_2CH_2OMe$ | 356 |
| 1-126 | H | H | H | H | H | $CH_2CH_2OCH_2CH_2OH$ | 342 |
| 1-127 | H | H | H | H | H | $CH_2CH_2CH_2OCH_2CH_2OH$ | 356 |
| 1-128 | H | H | H | H | H | $CH_2CH_2CH_2CH_2OCH_2CH_2OH$ | 370 |
| 1-129 | H | H | H | H | H | $CH_2CH(CH_2OH)NH_2$ | 327 |
| 1-130 | H | H | H | H | H | $CH_2CH(CH_2OH)NHCOMe$ | 369 |
| 1-131 | H | H | H | H | H | a-50 | 452 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-132 | H | H | H | H | H | $CH_2CH\ (CH_2OH)\ NHCONHC_6H_5$ | 446 |
| 1-133 | H | H | H | H | H | $CH\ (CH_2OH)\ CH_2NH_2$ | 327 |
| 1-134 | H | H | H | H | H | $CH\ (CH_2OH)\ CH_2NHCOMe$ | 369 |
| 1-135 | H | H | H | H | H | a-51 | 452 |
| 1-136 | H | H | H | H | H | $CH\ (CH_2OH)\ CH_2NHCONHC_6H_5$ | 446 |
| 1-137 | H | H | H | H | H | $CH_2CH(OH)CH_2NH_2$ | 327 |
| 1-138 | H | H | H | H | H | $CH_2CH(OH)CH_2NHMe$ | 341 |
| 1-139 | H | H | H | H | H | $CH_2CH(OH)CH_2NMe_2$ | 355 |
| 1-140 | H | H | H | H | H | $CH_2CH_2CH_2CH_2NH_2$ | 325 |
| 1-141 | H | H | H | H | H | $CH_2CH_2CH_2CH_2CH_2NH_2$ | 339 |
| 1-142 | H | H | H | H | H | $CH_2CH_2NHMe$ | 311 |
| 1-143 | H | H | H | H | H | $CH_2CH_2CH_2CH_2NHMe$ | 339 |
| 1-144 | H | H | H | H | H | $CH_2CH_2CH_2CH_2CH_2NHMe$ | 353 |
| 1-145 | H | H | H | H | H | $CH_2CH_2NMe_2$ | 325 |
| 1-146 | H | H | H | H | H | $CH_2CH_2CH_2NMe_2$ | 339 |
| 1-147 | H | H | H | H | H | $CH_2CH_2CH_2CH_2NMe_2$ | 353 |
| 1-148 | H | H | H | H | H | $CH_2CH_2CH,CH_2CH_2NMe_2$ | 367 |
| 1-149 | H | H | H | H | H | $CH_2CH_2NHCH_2CH_2OH$ | 341 |
| 1-150 | H | H | H | H | H | $CH_2CH_2CH_2NHCH_2CH_2OH$ | 355 |
| 1-151 | H | H | H | H | H | $CH_2CH_2CH_2CH_2NHCH_2CH_2OH$ | 369 |
| 1-152 | H | H | H | H | H | $CH_2CONH_2$ | 311 |
| 1-153 | H | H | H | H | H | $CH_2CH_2CONH_2$ | 325 |
| 1-154 | H | H | H | H | H | $CH_2CH_2CH_2CONH_2$ | 339 |
| 1-155 | H | H | H | H | H | $CH_2CH_2NHCOCH_2Me$ | 353 |
| 1-156 | H | H | H | H | H | $CH_2CH_2NHCOCH_2CH_2Me$ | 367 |
| 1-157 | H | H | H | H | H | $CH_2CH_2NHCOCRMe_2$ | 367 |
| 1-158 | H | H | H | H | H | $CHCH_2NHCOCMe_3$ | 381 |
| 1-159 | H | H | H | H | H | $CH_2CH_2NHCOCH_2OH$ | 355 |
| 1-160 | H | H | H | H | H | $CH_2CH_2NHCOCH_2OMe$ | 369 |
| 1-161 | H | H | H | H | H | $CH_2CH_2NHCOC_6H_5$ | 401 |
| 1-162 | H | H | H | H | H | a-52 | 402 |
| 1-163 | H | H | H | H | H | a-53 | 407 |
| 1-164 | H | H | H | H | H | a-54 | 391 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-165 | H | H | H | H | H | a-55 | 403 |
| 1-166 | H | H | H | H | H | $CH_2CH_2NHCOCH_2NHCOCH_2CHMe_2$ | 438 |
| 1-167 | H | H | H | H | H | $CH_2CH_2NHCOCH_2NHCOC_6H_5$ | 458 |
| 1-168 | H | H | H | H | H | $CH_2CH_2CH_2NHCOMe$ | 353 |
| 1-169 | H | H | H | H | H | $CH_2CH_2CH_2NHCOCH_2Me$ | 367 |
| 1-170 | H | H | H | H | H | $CH_2CH_2CH_2NHCOCH_2CH_2Me$ | 381 |
| 1-171 | H | H | H | H | H | $CH_2CH_2CH_2NHCOCHMe_2$ | 381 |
| 1-172 | H | H | H | H | H | $CH_2CH_2CH_2NHCOCMe_3$ | 395 |
| 1-173 | H | H | H | H | H | $CH_2CH_2CH_2CH_2NHCOMe$ | 367 |
| 1-174 | H | H | H | H | H | $CH_2CH_2CH_2CH_2NHCOCH_2Me$ | 381 |
| 1-175 | H | H | H | H | H | $CH_2CH_2CH_2CH_2NHCOCH_2CH_2Me$ | 395 |
| 1-176 | H | H | H | H | H | $CH_2CH_2CH_2CH_2NHCOCHMe_2$ | 395 |
| 1-177 | H | H | H | H | H | $CH_2CH_2CH_2CH_2NHCOCMe_3$ | 409 |
| 1-178 | H | H | H | H | H | $CH_2CH_2N$ (Me) COMe | 353 |
| 1-179 | H | H | H | H | H | $CH_2CH_2N(Me)COC_6H_5$ | 415 |
| 1-180 | H | H | H | H | H | $CH_2CH_2CH_2N(Me)COMe$ | 367 |
| 1-181 | H | H | H | H | H | $CH_2CH_2CH_2N(Me)COC_6H_5$ | 429 |
| 1-182 | H | H | H | H | H | $CH_2CH_2CH_2CH_2N(Me)$ COMe | 381 |
| 1-183 | H | H | H | H | H | $CH_2CH_2N$ $(CH_2CH_2OH)$ COMe | 383 |
| 1-184 | H | H | H | H | H | $CH_2CH_2CH_2N$ $(CH_2CH_2OH)$ COMe | 397 |
| 1-185 | H | H | H | H | H | $CH_2CH_2N(CH_2CN)$ COMe | 378 |
| 1-186 | H | H | H | H | H | $CH_2CH_2CH_2N(CH_2CN)$ COMe | 392 |
| 1-187 | H | H | H | H | H | a-56 | 324 |
| 1-188 | H | H | H | H | H | a-57 | 323 |
| 1-189 | H | H | H | H | H | a-58 | 365 |
| 1-190 | H | H | H | H | H | a-59 | 427 |
| 1-191 | H | H | H | H | H | a-60 | 455 |
| 1-192 | H | H | H | H | H | a-61 | 338 |
| 1-193 | H | H | H | H | H | a-62 | 351 |
| 1-194 | H | H | H | H | H | a-63 | 337 |
| 1-195 | H | H | H | H | H | $CH_2CH_2NHSO_2C_6H_5$ | 437 |
| 1-196 | H | H | H | H | H | $CH_2CH_2NHSO_2NMe_2$ | 404 |
| 1-197 | H | H | H | H | H | $CH_2CH_2N$ (Me) $SO_2Me$ | 389 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-198 | H | H | H | H | H | $CH_2CH_2N$ (Me) $SO_2C_6H_5$ | 451 |
| 1-199 | H | H | H | H | H | $CH_2CH_2N(Me)SO_2NMe_2$ | 418 |
| 1-200 | H | H | H | H | H | $CH_2CH_2N$ ($CH_2CH_2OH$) $SO_2Me$ | 405 |
| 1-201 | H | H | H | H | H | $CH_2CH_2CH_2NHSO_2Me$ | 389 |
| 1-202 | H | H | H | H | H | $CH_2CH_2CH_2NHSO_2C_6H_5$ | 451 |
| 1-203 | H | H | H | H | H | $CH_2CH_2CH_2NHSO_2NMe_2$ | 418 |
| 1-204 | H | H | H | H | H | $CH_2CH_2CH_2N$ (Me) $SO_2Me$ | 403 |
| 1-205 | H | H | H | H | H | $CH_2CH_2CH_2N(Me)SO_2C_6H_5$ | 465 |
| 1-206 | H | H | H | H | H | $CH_2CH_2CH_2N$ (Me) $SO_2NMe_2$ | 432 |
| 1-207 | H | H | H | H | H | $CH_2CH_2CH_2N$ ($CH_2CH_2OH$) $SO_2Me$ | 419 |
| 1-208 | H | H | H | H | H | $CH_2CH_2CH_2CH_2NHSO_2Me$ | 403 |
| 1-209 | H | H | H | H | H | $CH_2CH_2CH_2CH_2NHSO_2C_6H_5$ | 465 |
| 1-210 | H | H | H | H | H | $CH_2CH_2CH_2CH_2NHSO_2NMe_2$ | 432 |
| 1-211 | H | H | H | H | H | $CH_2CH_2CH_2CH_2N$ (Me) $SO_2Me$ | 417 |
| 1-212 | H | H | H | H | H | $CH_2CH_2CH_2CH_2N(Me)SO_2C_6H_5$ | 479 |
| 1-213 | H | H | H | H | H | $CH_2CH_2CH_2CH_2N$ (Me) $SO_2NMe_2$ | 446 |
| 1-214 | H | H | H | H | H | $CH_2CH_2CH_2CH_2CH_2N$ ($CH_2CH_2OH$) $SO_2Me$ | 433 |
| 1-215 | H | H | H | H | H | a-64 | 401 |
| 1-216 | H | H | H | H | H | a-65 | 365 |
| 1-217 | H | H | H | H | H | a-66 | 429 |
| 1-218 | H | H | H | H | H | a-67 | 379 |
| 1-219 | H | H | H | H | H | $CH_2CH_2NHCONHMe$ | 354 |
| 1-220 | H | H | H | H | H | $CH_2CH_2NHCONHCH_2Me$ | 368 |
| 1-221 | H | H | H | H | H | $CH_2CH_2NHCONHCH_2CH_2Me$ | 382 |
| 1-222 | H | H | H | H | H | $CH_2CH_2NHCONHCHMe_2$ | 382 |
| 1-223 | H | H | H | H | H | $CH_2CH_2NHCONHCMe_3$ | 396 |
| 1-224 | H | H | H | H | H | a-68 | 408 |
| 1-225 | H | H | H | H | H | a-69 | 422 |
| 1-226 | H | H | H | H | H | a-70 | 436 |
| 1-227 | H | H | H | H | H | a-71 | 450 |
| 1-228 | H | H | H | H | H | a-72 | 474 |
| 1-229 | H | H | H | H | H | $CH_2CH_2NHCONHC_6H_5$ | 416 |
| 1-230 | H | H | H | H | H | a-73 | 446 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-231 | H | H | H | H | H | a-74 | 432 |
| 1-232 | H | H | H | H | H | a-75 | 431 |
| 1-233 | H | H | H | H | H | a-76 | 459 |
| 1-234 | H | H | H | H | H | a-77 | 466 |
| 1-235 | H | H | H | H | H | a-78 | 466 |
| 1-236 | H | H | H | H | H | a-79 | 441 |
| 1-237 | H | H | H | H | H | a-80 | 445 |
| 1-238 | H | H | H | H | H | a-81 | 450 |
| 1-239 | H | H | H | H | H | $CH_2CH_2NHCONHCOC_6H_5$ | 444 |
| 1-240 | H | H | H | H | H | $CH_2CH_2NHCONHSO_2C_6H_5$ | 480 |
| 1-241 | H | H | H | H | H | a-82 | 358 |
| 1-242 | H | H | H | H | H | a-83 | 358 |
| 1-243 | H | H | H | H | H | a-84 | 473 |
| 1-244 | H | H | H | H | H | a-85 | 460 |
| 1-245 | H | H | H | H | H | a-86 | 472 |
| 1-246 | H | H | H | H | H | a-87 | 422 |
| 1-247 | H | H | H | H | H | $CH_2CH_2NHCONHCMe_2CH_2CMe_3$ | 452 |
| 1-248 | H | H | H | H | H | a-88 | 435 |
| 1-249 | H | H | H | H | H | $CH_2CH_2N$ (Me) $CONHC_6H_5$ | 430 |
| 1-250 | H | H | H | H | H | $CH_2CH_2N$ (Me) CON (Me) $C_6H_5$ | 444 |
| 1-251 | H | H | H | H | H | $CH_2CH_2NHCONHCH_2CH_2OH$ | 384 |
| 1-252 | H | H | H | H | H | $CH_2CH_2NHCONHCH_2CH_2OMe$ | 398 |
| 1-253 | H | H | H | H | H | $CH_2CH_2NHCONHCH_2CH_2NMe_2$ | 411 |
| 1-254 | H | H | H | H | H | $CH_2CH_2NHCON$ (Me) $CH_2CH_2OH$ | 398 |
| 1-255 | H | H | H | H | H | $CH_2CH_2NHCON$ $(CH_2CH_2OH)_2$ | 428 |
| 1-256 | H | H | H | H | H | a-89 | 409 |
| 1-257 | H | H | H | H | H | $CH_2CH_2NHCONMe_2$ | 368 |
| 1-258 | H | H | H | H | H | $CH_2CH_2CH_2NHCONHMe$ | 368 |
| 1-259 | H | H | H | H | H | $CH_2CH_2CH_2NHCONHCH_2Me$ | 382 |
| 1-260 | H | H | H | H | H | $CH_2CH_2CH_2NHCONHCH_2CH_2Me$ | 396 |
| 1-261 | H | H | H | H | H | $CH_2CH_2CH_2NHCONHCHMe_2$ | 396 |
| 1-262 | H | H | H | H | H | $CH_2CH_2CH_2NHCONHCMe_3$ | 410 |
| 1-263 | H | H | H | H | H | a-90 | 422 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-264 | H | H | H | H | H | a-91 | 436 |
| 1-265 | H | H | H | H | H | a-92 | 450 |
| 1-266 | H | H | H | H | H | a-93 | 464 |
| 1-267 | H | H | H | H | H | a-94 | 488 |
| 1-268 | H | H | H | H | H | $CH_2CH_2CH_2NHCONHC_6H_5$ | 430 |
| 1-269 | H | H | H | H | H | a-95 | 460 |
| 1-270 | H | H | H | H | H | a-96 | 446 |
| 1-271 | H | H | H | H | H | a-97 | 445 |
| 1-272 | H | H | H | H | H | a-98 | 473 |
| 1-273 | H | H | H | H | H | a-99 | 480 |
| 1-274 | H | H | H | H | H | a-100 | 480 |
| 1-275 | H | H | H | H | H | a-101 | 455 |
| 1-276 | H | H | H | H | H | a-102 | 459 |
| 1-277 | H | H | H | H | H | a-103 | 464 |
| 1-278 | H | H | H | H | H | $CH_2CH_2CH_2NHCONHCOC_6H_5$ | 458 |
| 1-279 | H | H | H | H | H | a-104 | 506 |
| 1-280 | H | H | H | H | H | a-105 | 506 |
| 1-281 | H | H | H | H | H | a-106 | 487 |
| 1-282 | H | H | H | H | H | a-107 | 474 |
| 1-283 | H | H | H | H | H | a-108 | 486 |
| 1-284 | H | H | H | H | H | a-109 | 436 |
| 1-285 | H | H | H | H | H | $CH_2CH_2CH_2NHCONHCMe_2CH_2CMe_3$ | 466 |
| 1-286 | H | H | H | H | H | a-110 | 449 |
| 1-287 | H | H | H | H | H | $CH_2CH_2CH_2N$ (Me) $CONHC_6H_5$ | 444 |
| 1-288 | H | H | H | H | H | $CH_2CH_2CH_2N$ (Me) CON (Me) $C_6H_5$ | 458 |
| 1-289 | H | H | H | H | H | $CH_2CH_2CH_2NHCONHCH_2CH_2OMe$ | 412 |
| 1-290 | H | H | H | H | H | $CH_2CH_2CH_2NHCONHCH_2CH_2NMe_2$ | 425 |
| 1-291 | H | H | H | H | H | $CH_2CH_2CH_2NHCON$ (Me) $CH_2CH_2OH$ | 412 |
| 1-292 | H | H | H | H | H | $CH_2CH_2CH_2NHCON$ $(CH_2CH_2OH)_2$ | 442 |
| 1-293 | H | H | H | H | H | a-111 | 423 |
| 1-294 | H | H | H | H | H | $CH_2CH_2CH_2NHCONMe_2$ | 382 |
| 1-295 | H | H | H | H | H | $CH_2CH_2NHCSNHCH_2Me$ | 384 |
| 1-296 | H | H | H | H | H | $CH_2CH_2NHCSNHCH_2CH_2Me$ | 398 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-297 | H | H | H | H | H | $CH_2CH_2NHCSNHCHMe_2$ | 398 |
| 1-298 | H | H | H | H | H | $CH_2CH_2NHCSNHCMe_3$ | 412 |
| 1-299 | H | H | H | H | H | a-112 | 358 |
| 1-300 | H | H | H | H | H | $CH_2CH_2NHCSNHCH_2CH_2OMe$ | 414 |
| 1-301 | H | H | H | H | H | $CH_2CH_2NHCSNHCH_2CH_2NMe_2$ | 427 |
| 1-302 | H | H | H | H | H | $CH_2CH_2NHCSN$ (Me) $CH_2CH_2OH$ | 414 |
| 1-303 | H | H | H | H | H | $CH_2CH_2NHCSN(CH_2CH_2OH)_2$ | 444 |
| 1-304 | H | H | H | H | H | a-113 | 425 |
| 1-305 | H | H | H | H | H | $CH_2CH_2NHCSNMe_2$ | 384 |
| 1-306 | H | H | H | H | H | $CH_2CH_2NHCOOMe$ | 355 |
| 1-307 | H | H | H | H | H | $CH_2CH_2NHCOOCH_2Me$ | 369 |
| 1-308 | H | H | H | H | H | $CH_2CH_2NHCOOCH_2CH_2Me$ | 383 |
| 1-309 | H | H | H | H | H | $CH_2CH_2NHCOOCHMe_2$ | 383 |
| 1-310 | H | H | H | H | H | a-114 | 423 |
| 1-311 | H | H | H | H | H | $CH_2CH_2NHCOOC_6H_5$ | 417 |
| 1-312 | H | H | H | H | H | $CH_2CH_2NHCOOCH_2CH_2OH$ | 385 |
| 1-313 | H | H | H | H | H | $CH_2CH_2NHCOOCH_2CH_2OMe$ | 399 |
| 1-314 | H | H | H | H | H | a-115 | 378 |
| 1-315 | H | H | H | H | H | a-116 | 378 |
| 1-316 | H | H | H | H | H | a-117 | 365 |
| 1-317 | H | H | H | H | H | a-118 | 379 |
| 1-318 | H | H | H | H | H | a-119 | 354 |
| 1-319 | H | H | H | H | H | a-120 | 379 |
| 1-320 | H | H | H | H | H | $COC_6H_5$ | 358 |
| 1-321 | Cl | H | H | H | H | H | 288 |
| 1-322 | Br | H | H | H | H | H | 332 |
| 1-323 | OH | H | H | H | H | H | 270 |
| 1-324 | Me | H | H | H | H | H | 268 |
| 1-325 | $CH{=}CH_2$ | H | H | H | H | H | 280 |
| 1-326 | $C{\equiv}CC_6H_5$ | H | H | H | H | H | 354 |
| 1-327 | OMe | H | H | H | H | H | 284 |
| 1-328 | $NH_2$ | H | H | H | H | H | 269 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-329 | NHMe | H | H | H | H | H | 283 |
| 1-330 | $NMe_2$ | H | H | H | H | H | 297 |
| 1-331 | $NHC_6H_5$ | H | H | H | H | H | 345 |
| 1-332 | OH | H | H | H | H | Me | 284 |
| 1-333 | OH | H | H | H | H | a-1 | 352 |
| 1-334 | OH | H | H | H | H | a-2 | 390 |
| 1-335 | OH | H | H | H | H | $C_6H_5$ | 346 |
| 1-336 | OH | H | H | H | H | $CH_2COOH$ | 328 |
| 1-337 | OH | H | H | H | H | $CH_2CH_2COOH$ | 342 |
| 1-338 | OH | H | H | H | H | $CH_2CN$ | 309 |
| 1-339 | OH | H | H | H | H | $CH_2CH_2CN$ | 323 |
| 1-340 | OH | H | H | H | H | $CH_2CH_2OH$ | 314 |
| 1-341 | OH | H | H | H | H | $CH(CH_2OH)_2$ | 344 |
| 1-342 | OH | H | H | H | H | a-3 | 368 |
| 1-343 | OH | H | H | H | H | $CH_2CH_2OMe$ | 328 |
| 1-344 | OH | H | H | H | H | $CH_2CH_2OCOCMe_3$ | 398 |
| 1-345 | OH | H | H | H | H | $CH_2CH_2SCOMe$ | 372 |
| 1-346 | OH | H | H | H | H | $CH_2CH_2SH$ | 330 |
| 1-347 | OH | H | H | H | H | $CH_2CH_2NH_2$ | 313 |
| 1-348 | OH | H | H | H | H | $CH_2CH_2CH_2NH_2$ | 327 |
| 1-349 | OH | H | H | H | H | $CH_2CH_2CH_2NHMe$ | 341 |
| 1-350 | OH | H | H | H | H | $CH\ (CH_2NHCOMe)_2$ | 426 |
| 1-351 | OH | H | H | H | H | $CH\ (CH_2NH_2)$ | 342 |
| 1-352 | OH | H | H | H | H | $CH_2CH_2NHCOMe$ | 355 |
| 1-353 | OH | H | H | H | H | a-4 | 368 |
| 1-354 | OH | H | H | H | H | $CH_2CH_2NHCOCH_2NHCOMe$ | 412 |
| 1-355 | OH | H | H | H | H | $CH_2CH_2NHCOCH_2NMe_2$ | 398 |
| 1-356 | OH | H | H | H | H | $CH_2CH_2NHSO_2Me$ | 391 |
| 1-357 | OH | H | H | H | H | a-5 | 367 |
| 1-358 | OH | H | H | H | H | a-6 | 409 |
| 1-359 | OH | H | H | H | H | $CH_2CH_2CH_2NHCONHCH_2CH_2OH$ | 414 |
| 1-360 | OH | H | H | H | H | $CH_2CH_2CH_2NHCONHBn$ | 460 |
| 1-361 | OH | H | H | H | H | $CH_2CH_2NHCSNHCH_2CH_2OH$ | 416 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-362 | OH | H | H | H | H | $CH_2CH_2NHCSNHC_6H_5$ | 448 |
| 1-363 | OH | H | H | H | H | $CH_2CH_2NHCOOCH_2CH_2NMe_2$ | 428 |
| 1-364 | OH | H | H | H | H | a-7 | 425 |
| 1-365 | OH | H | H | H | H | a-8 | 312 |
| 1-366 | OH | H | H | H | H | a-9 | 367 |
| 1-367 | OH | H | H | H | H | COMe | 312 |
| 1-368 | OH | H | H | H | H | a-10 | 324 |
| 1-369 | OH | H | H | H | H | a-11 | 338 |
| 1-370 | OH | H | H | H | H | a-12 | 352 |
| 1-371 | OH | H | H | H | H | Bn | 360 |
| 1-372 | OH | H | H | H | H | a-13 | 394 |
| 1-373 | OH | H | H | H | H | a-14 | 376 |
| 1-374 | OH | H | H | H | H | a-15 | 420 |
| 1-375 | OH | H | H | H | H | a-16 | 434 |
| 1-376 | OH | H | H | H | H | a-17 | 450 |
| 1-377 | OH | H | H | H | H | a-18 | 427 |
| 1-378 | OH | H | H | H | H | a-19 | 445 |
| 1-379 | OH | H | H | H | H | a-20 | 436 |
| 1-380 | OH | H | H | H | H | a-21 | 375 |
| 1-381 | OH | H | H | H | H | a-22 | 389 |
| 1-382 | OH | H | H | H | H | a-23 | 403 |
| 1-383 | OH | H | H | H | H | a-24 | 419 |
| 1-384 | OH | H | H | H | H | a-25 | 433 |
| 1-385 | OH | H | H | H | H | a-26 | 463 |
| 1-386 | OH | H | H | H | H | a-27 | 417 |
| 1-387 | OH | H | H | H | H | a-28 | 453 |
| 1-388 | OH | H | H | H | H | a-29 | 462 |
| 1-389 | OH | H | H | H | H | a-30 | 404 |
| 1-390 | OH | H | H | H | H | a-31 | 420 |
| 1-391 | OH | H | H | H | H | a-32 | 390 |
| 1-392 | OH | H | H | H | H | a-33 | 390 |
| 1-393 | OH | H | H | H | H | a-34 | 420 |
| 1-394 | OH | H | H | H | H | a-35 | 420 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-395 | OH | H | H | H | H | a-36 | 450 |
| 1-396 | OH | H | H | H | H | a-37 | 450 |
| 1-397 | OH | H | H | H | H | a-38 | 348 |
| 1-398 | OH | H | H | H | H | a-39 | 350 |
| 1-399 | OH | H | H | H | H | a-40 | 366 |
| 1-400 | OH | H | H | H | H | a-41 | 418 |
| 1-401 | OH | H | H | H | H | a-42 | 405 |
| 1-402 | OH | H | H | H | H | a-43 | 361 |
| 1-403 | OH | H | H | H | H | a-44 | 347 |
| 1-404 | OH | H | H | H | H | a-45 | 362 |
| 1-405 | OH | H | H | H | H | $CH_2CH_2CH_2COOH$ | 356 |
| 1-406 | OH | H | H | H | H | $CH_2CH_2CH_2CH_2COOH$ | 370 |
| 1-407 | OH | H | H | H | H | $CH_2CH_2CH_2CN$ | 337 |
| 1-408 | OH | H | H | H | H | $CH_2CH_2CH_2CH_2CN$ | 351 |
| 1-409 | OH | H | H | H | H | $CH_2CH(Me)OH$ | 328 |
| 1-410 | OH | H | H | H | H | $CH(Me)CH_2OH$ | 328 |
| 1-411 | OH | H | H | H | H | $CH_2CH_2CH_2OH$ | 328 |
| 1-412 | OH | H | H | H | H | $CH_2CH$ (OH) $CH_2OH$ | 344 |
| 1-413 | OH | H | H | H | H | $CH_2CH$ ($NH_2$) $CH_2OH$ | 343 |
| 1-414 | OH | H | H | H | H | $CH_2CH$ (NHMe) $CH_2OH$ | 357 |
| 1-415 | OH | H | H | H | H | $CH_2CH$ ($NMe_2$) $CH_2OH$ | 371 |
| 1-416 | OH | H | H | H | H | $CH_2CH$ (Me) $CH_2OH$ | 342 |
| 1-417 | OH | H | H | H | H | $CH_2CH_2CH_2CH_2OH$ | 342 |
| 1-418 | OH | H | H | H | H | $CH_2CH_2CH_2CH_2CH_2OH$ | 356 |
| 1-419 | OH | H | H | H | H | a-46 | 482 |
| 1-420 | OH | H | H | H | H | CH ($CH_2OH$) $CH_2CH_2NHCONHC_6H_5$ | 476 |
| 1-421 | OH | H | H | H | H | a-47 | 468 |
| 1-422 | OH | H | H | H | H | $CH_2CH$ (OH) $CH_2NHCONHC_6H_5$ | 462 |
| 1-423 | OH | H | H | H | H | $CH_2CH_2OCONHMe$ | 371 |
| 1-424 | OH | H | H | H | H | $CH_2CH_2OCONHC_6H_5$ | 433 |
| 1-425 | OH | H | H | H | H | $CH_2CH_2CH_2OCONHMe$ | 385 |
| 1-426 | OH | H | H | H | H | a-48 | 453 |
| 1-427 | OH | H | H | H | H | $CH_2CH_2CH_2OCONHC_6H_5$ | 447 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-428 | OH | H | H | H | H | $CH_2CH_2CH_2CH_2OCONHMe$ | 399 |
| 1-429 | OH | H | H | H | H | a-49 | 467 |
| 1-430 | OH | H | H | H | H | $CH_2CH_2CH_2CH_2OCONHC_6H_5$ | 461 |
| 1-431 | OH | H | H | H | H | $CH_2CH_2CH_2SH$ | 344 |
| 1-432 | OH | H | H | H | H | $CH_2CH_2CH_2CH_2SH$ | 358 |
| 1-433 | OH | H | H | H | H | $CH_2CH_2SCOC_6H_5$ | 434 |
| 1-434 | OH | H | H | H | H | $CH_2CH_2CH_2SCOMe$ | 386 |
| 1-435 | OH | H | H | H | H | $CH_2CH_2CH_2SCOC_6H_5$ | 448 |
| 1-436 | OH | H | H | H | H | $CH_2CH_2CH_2OMe$ | 342 |
| 1-437 | OH | H | H | H | H | $CH_2CH_2CH_2CH_2OMe$ | 356 |
| 1-438 | OH | H | H | H | H | $CH_2CH_2CH_2CH_2CH_2OMe$ | 370 |
| 1-439 | OH | H | H | H | H | $CH_2CH_2OCOMe$ | 356 |
| 1-440 | OH | H | H | H | H | $CH_2CH_2CH_2OCOMe$ | 370 |
| 1-441 | OH | H | H | H | H | $CH_2CH_2CH_2CH_2OCOMe$ | 384 |
| 1-442 | OH | H | H | H | H | $CH_2CH_2OCOCH_2Me$ | 370 |
| 1-443 | OH | H | H | H | H | $CH_2CH_2CH_2OCOCH_2Me$ | 384 |
| 1-444 | OH | H | H | H | H | $CH_2CH_2CH_2CH_2OCOCH_2Me$ | 398 |
| 1-445 | OH | H | H | H | H | $CH_2CH_2OCOCHMe_2$ | 384 |
| 1-446 | OH | H | H | H | H | $CH_2CH_2CH_2OCOCHMe_2$ | 398 |
| 1-447 | OH | H | H | H | H | $CH_2CH_2CH_2CH_2OCOCHMe_2$ | 412 |
| 1-448 | OH | H | H | H | H | $CH_2CH_2CH_2OCOCMe_3$ | 412 |
| 1-449 | OH | H | H | H | H | $CH_2CH_2CH_2CH_2OCOCMe_3$ | 426 |
| 1-450 | OH | H | H | H | H | $CH_2CH_2OCOC_6H_5$ | 418 |
| 1-451 | OH | H | H | H | H | $CH_2CH_2CH_2OCOC_6H_5$ | 432 |
| 1-452 | OH | H | H | H | H | $CH_2CH_2CH_2CH_2OCOC_6H_5$ | 446 |
| 1-453 | OH | H | H | H | H | $CH_2CH_2OCOCH_2OH$ | 372 |
| 1-454 | OH | H | H | H | H | $CH_2CH_2OCOCH_2NH_2$ | 371 |
| 1-455 | OH | H | H | H | H | $CH_2CH_2OCH_2CH_2OMe$ | 372 |
| 1-456 | OH | H | H | H | H | $CH_2CH_2OCH_2CH_2OH$ | 358 |
| 1-457 | OH | H | H | H | H | $CH_2CH_2CH_2OCH_2CH_2OH$ | 372 |
| 1-458 | OH | H | H | H | H | $CH_2CH_2CH_2CH_2OCH_2CH_2OH$ | 386 |
| 1-459 | OH | H | H | H | H | $CH_2CH\ (CH_2OH)\ NH_2$ | 343 |
| 1-460 | OH | H | H | H | H | $CH_2CH\ (CH_2OH)\ NHCOMe$ | 385 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-461 | OH | H | H | H | H | a-50 | 468 |
| 1-462 | OH | H | H | H | H | $CH_2CH\ (CH_2OH)\ NHCONHC_6H_5$ | 462 |
| 1-463 | OH | H | H | H | H | $CH(CH_2OH)CH_2NH_2$ | 343 |
| 1-464 | OH | H | H | H | H | $CH\ (CH_2OH)\ CH_2NHCOMe$ | 385 |
| 1-465 | OH | H | H | H | H | a-51 | 468 |
| 1-466 | OH | H | H | H | H | $CH\ (CH_2OH)\ CH_2NHCONHC_6H_5$ | 462 |
| 1-467 | OH | H | H | H | H | $CH_2CH(OH)CH_2NH_2$ | 343 |
| 1-468 | OH | H | H | H | H | $CH_2CH(OH)CH_2NHMe$ | 357 |
| 1-469 | OH | H | H | H | H | $CH_2CH\ (OH)\ CH_2NMe_2$ | 371 |
| 1-470 | OH | H | H | H | H | $CH_2CH_2CH_2CH_2NH_2$ | 341 |
| 1-471 | OH | H | H | H | H | $CH_2CH_2CH_2CH_2CH_2NH_2$ | 355 |
| 1-472 | OH | H | H | H | H | $CH_2CH_2NHMe$ | 327 |
| 1-473 | OH | H | H | H | H | $CH_2CH_2CH_2CH_2NHMe$ | 355 |
| 1-474 | OH | H | H | H | H | $CH_2CH_2CH_2CH_2CH_2NHMe$ | 369 |
| 1-475 | OH | H | H | H | H | $CH_2CH_2NMe_2$ | 341 |
| 1-476 | OH | H | H | H | H | $CH_2CH_2CH_2NMe_2$ | 355 |
| 1-477 | OH | H | H | H | H | $CH_2CH_2CH_2CH_2NMe_2$ | 369 |
| 1-478 | OH | H | H | H | H | $CH_2CH_2CH_2CH_2CH_2NMe_2$ | 383 |
| 1-479 | OH | H | H | H | H | $CH_2CH_2NHCH_2CH_2OH$ | 357 |
| 1-480 | OH | H | H | H | H | $CH_2CH_2CH_2NHCH_2CH_2OH$ | 371 |
| 1-481 | OH | H | H | H | H | $CH_2CH_2CH_2CH_2NHCH_2CH_2OH$ | 385 |
| 1-482 | OH | H | H | H | H | $CH_2CONH_2$ | 327 |
| 1-483 | OH | H | H | H | H | $CH_2CH_2CONH_2$ | 341 |
| 1-484 | OH | H | H | H | H | $CH_2CH_2CH_2CONH_2$ | 355 |
| 1-485 | OH | H | H | H | H | $CH_2CH_2NHCOCH_2Me$ | 369 |
| 1-486 | OH | H | H | H | H | $CH_2CH_2NHCOCH_2CH_2Me$ | 383 |
| 1-487 | OH | H | H | H | H | $CH_2CH_2NHCOCHMe_2$ | 383 |
| 1-488 | OH | H | H | H | H | $CH_2CH_2NHCOCMe_3$ | 397 |
| 1-489 | OH | H | H | H | H | $CH_2CH_2NHCOCH_2OH$ | 371 |
| 1-490 | OH | H | H | H | H | $CH_2CH_2NHCOCH_2OMe$ | 385 |
| 1-491 | OH | H | H | H | H | $CH_2CH_2NHCOC_6H_5$ | 417 |
| 1-492 | OH | H | H | H | H | a-52 | 418 |
| 1-493 | OH | H | H | H | H | a-53 | 423 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-494 | OH | H | H | H | H | a-54 | 407 |
| 1-495 | OH | H | H | H | H | a-55 | 419 |
| 1-496 | OH | H | H | H | H | $CH_2CH_2NHCOCH_2NHCOCH_2CHMe_2$ | 454 |
| 1-497 | OH | H | H | H | H | $CH_2CH_2NHCOCH_2NHCOC_6H_5$ | 474 |
| 1-498 | OH | H | H | H | H | $CH_2CH_2CH_2NHCOMe$ | 369 |
| 1-499 | OH | H | H | H | H | $CH_2CH_2CH_2NHCOCH_2Me$ | 383 |
| 1-500 | OH | H | H | H | H | $CH_2CH_2CH_2NHCOCH_2CH_2Me$ | 397 |
| 1-501 | OH | H | H | H | H | $CH_2CH_2CH_2NHCOCHMe_2$ | 397 |
| 1-502 | OH | H | H | H | H | $CH_2CH_2CH_2NHCOCMe_3$ | 411 |
| 1-503 | OH | H | H | H | H | $CH_2CH_2CH_2CH_2NHCOMe$ | 383 |
| 1-504 | OH | H | H | H | H | $CH_2CH_2CH_2CH_2NHCOCH_2Me$ | 397 |
| 1-505 | OH | H | H | H | H | $CH_2CH_2CH_2CH_2NHCOCH_2CH_2Me$ | 411 |
| 1-506 | OH | H | H | H | H | $CH_2CH_2CH_2CH_2NHCOCHMe_2$ | 411 |
| 1-507 | OH | H | H | H | H | $CH_2CH_2CH_2CH_2NHCOCMe_3$ | 425 |
| 1-508 | OH | H | H | H | H | $CH_2CH_2N$ (Me) COMe | 369 |
| 1-509 | OH | H | H | H | H | $CH_2CH_2N(Me)COC_6H_5$ | 431 |
| 1-510 | OH | H | H | H | H | $CH_2CH_2CH_2N(Me)COMe$ | 383 |
| 1-511 | OH | H | H | H | H | $CH_2CH_2CH_2N$ (Me) $COC_6H_5$ | 445 |
| 1-512 | OH | H | H | H | H | $CH_2CH_2CH_2CH_2N(Me)$ COMe | 397 |
| 1-513 | OH | H | H | H | H | $CH_2CH_2N$ $(CH_2CH_2OH)$ COMe | 399 |
| 1-514 | OH | H | H | H | H | $CH_2CH_2CH_2N(CH_2CH_2OH)$ COMe | 413 |
| 1-515 | OH | H | H | H | H | $CH_2CH_2N$ $(CH_2CN)$ COMe | 394 |
| 1-516 | OH | H | H | H | H | $CH_2CH_2CH_2N$ $(CH_2CN)$ COMe | 408 |
| 1-517 | OH | H | H | H | H | a-56 | 340 |
| 1-518 | OH | H | H | H | H | a-57 | 339 |
| 1-519 | OH | H | H | H | H | a-58 | 381 |
| 1-520 | OH | H | H | H | H | a-59 | 443 |
| 1-521 | OH | H | H | H | H | a-60 | 471 |
| 1-522 | OH | H | H | H | H | a-61 | 354 |
| 1-523 | OH | H | H | H | H | a-62 | 367 |
| 1-524 | OH | H | H | H | H | a-63 | 353 |
| 1-525 | OH | H | H | H | H | $CH_2CH_2NHSO_2C_6H_5$ | 453 |
| 1-526 | OH | H | H | H | H | $CH_2CH_2NHSO_2NMe_2$ | 420 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-527 | OH | H | H | H | H | $CH_2CH_2N$ (Me) $SO_2Me$ | 405 |
| 1-528 | OH | H | H | H | H | $CH_2CH_2N$ (Me) $SO_2C_6H_5$ | 467 |
| 1-529 | OH | H | H | H | H | $CH_2CH_2N$ (Me) $SO_2NMe_2$ | 434 |
| 1-530 | OH | H | H | H | H | $CH_2CH_2N$ $(CH_2CH_2OH)$ $SO_2Me$ | 421 |
| 1-531 | OH | H | H | H | H | $CH_2CH_2CH_2NHSO_2Me$ | 405 |
| 1-532 | OH | H | H | H | H | $CH_2CH_2CH_2NHSO_2C_6H_5$ | 467 |
| 1-533 | OH | H | H | H | H | $CH_2CH_2CH_2NHSO_2NMe_2$ | 434 |
| 1-534 | OH | H | H | H | H | $CH_2CH_2CH_2N$ (Me) $SO_2Me$ | 419 |
| 1-535 | OH | H | H | H | H | $CH_2CH_2CH_2N(Me)SO_2C_6H_5$ | 481 |
| 1-536 | OH | H | H | H | H | $CH_2CH_2CH_2N$ (Me) $SO_2NMe_2$ | 448 |
| 1-537 | OH | H | H | H | H | $CH_2CH_2CH_2N$ $(CH_2CH_2OH)$ $SO_2Me$ | 435 |
| 1-538 | OH | H | H | H | H | $CH_2CH_2CH_2CH_2NHSO_2Me$ | 419 |
| 1-539 | OH | H | H | H | H | $CH_2CH_2CH_2CH_2NHSO_2C_6H_5$ | 481 |
| 1-540 | OH | H | H | H | H | $CH_2CH_2CH_2CH_2NHSO_2NMe_2$ | 448 |
| 1-541 | OH | H | H | H | H | $CH_2CH_2CH_2CH_2N$ (Me) $SO_2Me$ | 433 |
| 1-542 | OH | H | H | H | H | $CH_2CH_2CH_2CH_2N$ (Me) $SO_2C_6H_5$ | 495 |
| 1-543 | OH | H | H | H | H | $CH_2CH_2CH_2CH_2N$ (Me) $SO_2NMe_2$ | 462 |
| 1-544 | OH | H | H | H | H | $CH_2CH_2CH_2CH_2N(CH_2CH_2OH)$ $SO_2Me$ | 449 |
| 1-545 | OH | H | H | H | H | a-64 | 417 |
| 1-546 | OH | H | H | H | H | a-65 | 381 |
| 1-547 | OH | H | H | H | H | a-66 | 445 |
| 1-548 | OH | H | H | H | H | a-67 | 395 |
| 1-549 | OH | H | H | H | H | $CH_2CH_2NHCONHMe$ | 370 |
| 1-550 | OH | H | H | H | H | $CH_2CH_2NHCONHCH_2Me$ | 384 |
| 1-551 | OH | H | H | H | H | $CH_2CH_2NHCONHCH_2CH_2Me$ | 398 |
| 1-552 | OH | H | H | H | H | $CH_2CH_2NBCONHCHMe_2$ | 398 |
| 1-553 | OH | H | H | H | H | $CH_2CH_2NHCONHCMe_3$ | 412 |
| 1-554 | OH | H | H | H | H | a-68 | 424 |
| 1-555 | OH | H | H | H | H | a-69 | 438 |
| 1-556 | OH | H | H | H | H | a-70 | 452 |
| 1-557 | OH | H | H | H | H | a-71 | 466 |
| 1-558 | OH | H | H | H | H | a-72 | 490 |
| 1-559 | OH | H | H | H | H | $CH_2CH_2NHCONHC_6H_5$ | 432 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-560 | OH | H | H | H | H | a-73 | 462 |
| 1-561 | OH | H | H | H | H | a-74 | 448 |
| 1-562 | OH | H | H | H | H | a-75 | 447 |
| 1-563 | OH | H | H | H | H | a-76 | 475 |
| 1-564 | OH | H | H | H | H | a-77 | 482 |
| 1-565 | OH | H | H | H | H | a-78 | 482 |
| 1-566 | OH | H | H | H | H | a-79 | 457 |
| 1-567 | OH | H | H | H | H | a-80 | 461 |
| 1-568 | OH | H | H | H | H | a-81 | 466 |
| 1-569 | OH | H | H | H | H | $CH_2CH_2NHCONHCOC_6H_5$ | 460 |
| 1-570 | OH | H | H | H | H | $CH_2CH_2NHCONHSO_2C_6H_5$ | 496 |
| 1-571 | OH | H | H | H | H | a-82 | 374 |
| 1-572 | OH | H | H | H | H | a-83 | 374 |
| 1-573 | OH | H | H | H | H | a-84 | 489 |
| 1-574 | OH | H | H | H | H | a-85 | 476 |
| 1-575 | OH | H | H | H | H | a-86 | 488 |
| 1-576 | OH | H | H | H | H | a-87 | 438 |
| 1-577 | OH | H | H | H | H | $CH_2CH_2NHCONHCMe_2CH_2CMe_3$ | 468 |
| 1-578 | OH | H | H | H | H | a-88 | 451 |
| 1-579 | OH | H | H | H | H | $CH_2CH_2N(Me)CONHC_6H_5$ | 446 |
| 1-580 | OH | H | H | H | H | $CH_2CH_2N$ (Me) CON (Me) $C_6H_5$ | 460 |
| 1-581 | OH | H | H | H | H | $CH_2CH_2NHCONHCH_2CH_2OH$ | 400 |
| 1-582 | OH | H | H | H | H | $CH_2CH_2NHCONHCH_2CH_2OMe$ | 414 |
| 1-583 | OH | H | H | H | H | $CH_2CH_2NHCONHCH_2CH_2NMe_2$ | 427 |
| 1-584 | OH | H | H | H | H | $CH_2CH_2NHCON$ (Me) $CH_2CH_2OH$ | 414 |
| 1-585 | OH | H | H | H | H | $CH_2CH_2NHCON$ $(CH_2CH_2OH)_2$ | 444 |
| 1-586 | OH | H | H | H | H | a-89 | 425 |
| 1-587 | OH | H | H | H | H | $CH_2CH_2NHCONMe_2$ | 384 |
| 1-588 | OH | H | H | H | H | $CH_2CH_2CH_2NHCONHMe$ | 384 |
| 1-589 | OH | H | H | H | H | $CH_2CH_2CH_2NHCONHCH_2Me$ | 398 |
| 1-590 | OH | H | H | H | H | $CH_2CH_2CH_2NHCONHCH_2CH_2Me$ | 412 |
| 1-591 | OH | H | H | H | H | $CH_2CH_2CH_2NHCONHCHMe_2$ | 412 |
| 1-592 | OH | H | H | H | H | $CH_2CH_2CH_2NHCONHCMe_3$ | 426 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-593 | OH | H | H | H | H | a-90 | 438 |
| 1-594 | OH | H | H | H | H | a-91 | 452 |
| 1-595 | OH | H | H | H | H | a-92 | 466 |
| 1-596 | OH | H | H | H | H | a-93 | 480 |
| 1-597 | OH | H | H | H | H | a-94 | 504 |
| 1-598 | OH | H | H | H | H | $CH_2CH_2CH_2NHCONHC_6H_5$ | 446 |
| 1-599 | OH | H | H | H | H | a-95 | 476 |
| 1-600 | OH | H | H | H | H | a-96 | 462 |
| 1-601 | OH | H | H | H | H | a-97 | 461 |
| 1-602 | OH | H | H | H | H | a-98 | 489 |
| 1-603 | OH | H | H | H | H | a-99 | 496 |
| 1-604 | OH | H | H | H | H | a-100 | 496 |
| 1-605 | OH | H | H | H | H | a-101 | 471 |
| 1-606 | OH | H | H | H | H | a-102 | 475 |
| 1-607 | OH | H | H | H | H | a-103 | 480 |
| 1-608 | OH | H | H | H | H | $CH_2CH_2CH_2NHCONHCOC_6H_5$ | 474 |
| 1-609 | OH | H | H | H | H | a-104 | 522 |
| 1-610 | OH | H | H | H | H | a-105 | 522 |
| 1-611 | OH | H | H | H | H | a-106 | 503 |
| 1-612 | OH | H | H | H | H | a-107 | 490 |
| 1-613 | OH | H | H | H | H | a-108 | 502 |
| 1-614 | OH | H | H | H | H | a-109 | 452 |
| 1-615 | OH | H | H | H | H | $CH_2CH_2CH_2NHCONHCMe_2CH_2CMe_3$ | 482 |
| 1-616 | OH | H | H | H | H | a-110 | 465 |
| 1-617 | OH | H | H | H | H | $CH_2CH_2CH_2N$ (Me) $CONHC_6H_5$ | 460 |
| 1-618 | OH | H | H | H | H | $CH_2CH_2CH_2N(Me)CON(Me)C_6H_5$ | 474 |
| 1-619 | OH | H | H | H | H | $CH_2CH_2CH_2NHCONHCH_2CH_2OMe$ | 428 |
| 1-620 | OH | H | H | H | H | $CH_2CH_2CH_2NHCONHCH_2CH_2NMe_2$ | 441 |
| 1-621 | OH | H | H | H | H | $CH_2CH_2CH_2NHCON$ (Me) $CH_2CH_2OH$ | 428 |
| 1-622 | OH | H | H | H | H | $CH_2CH_2CH_2NHCON$ $(CH_2CH_2OH)_2$ | 458 |
| 1-623 | OH | H | H | H | H | a-111 | 439 |
| 1-624 | OH | H | H | H | H | $CH_2CH_2CH_2NHCONMe_2$ | 398 |
| 1-625 | OH | H | H | H | H | $CH_2CH_2NHCSNHCH_2Me$ | 400 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-626 | OH | H | H | H | H | $CH_2CH_2NHCSNHCH_2CH_2Me$ | 414 |
| 1-627 | OH | H | H | H | H | $CH_2CH_2NHCSNHCHMe_2$ | 414 |
| 1-628 | OH | H | H | H | H | $CH_2CH_2NHCSNHCMe_3$ | 428 |
| 1-629 | OH | H | H | H | H | a-112 | 374 |
| 1-630 | OH | H | H | H | H | $CH_2CH_2NHCSNHCH_2CH_2OMe$ | 430 |
| 1-631 | OH | H | H | H | H | $CH_2CH_2NHCSNHCH_2CH_2NMe_2$ | 443 |
| 1-632 | OH | H | H | H | H | $CH_2CH_2NHCSN$ (Me) $CH_2CH_2OH$ | 430 |
| 1-633 | OH | H | H | H | H | $CH_2CH_2NHCSN$ $(CH_2CH_2OH)_2$ | 460 |
| 1-634 | OH | H | H | H | H | a-113 | 441 |
| 1-635 | OH | H | H | H | H | $CH_2CH_2NHCSNMe_2$ | 400 |
| 1-636 | OH | H | H | H | H | $CH_2CH_2NHCOOMe$ | 371 |
| 1-637 | OH | H | H | H | H | $CH_2CH_2NHCOOCH_2Me$ | 385 |
| 1-638 | OH | H | H | H | H | $CH_2CH_2NHCOOCH_2CH_2Me$ | 399 |
| 1-639 | OH | H | H | H | H | $CH_2CH_2NHCOOCHMe_2$ | 399 |
| 1-640 | OH | H | H | H | H | a-114 | 439 |
| 1-641 | OH | H | H | H | H | $CH_2CH_2NHCOOC_6H_5$ | 433 |
| 1-642 | OH | H | H | H | H | $CH_2CH_2NHCOOCH_2CH_2OH$ | 401 |
| 1-643 | OH | H | H | H | H | $CH_2CH_2NHCOOCH_2CH_2OMe$ | 415 |
| 1-644 | OH | H | H | H | H | a-115 | 394 |
| 1-645 | OH | H | H | H | H | a-116 | 394 |
| 1-646 | OH | H | H | H | H | a-117 | 381 |
| 1-647 | OH | H | H | H | H | a-118 | 395 |
| 1-648 | OH | H | H | H | H | a-119 | 370 |
| 1-649 | OH | H | H | H | H | a-120 | 395 |
| 1-650 | OH | H | H | H | H | $COC_6H_5$ | 374 |
| 1-651 | H | Cl | H | H | H | H | 288 |
| 1-652 | H | Br | H | H | H | H | 332 |
| 1-653 | H | OH | H | H | H | H | 270 |
| 1-654 | H | Me | H | H | H | H | 268 |
| 1-655 | H | $CH=CH_2$ | H | H | H | H | 280 |
| 1-656 | H | $C\equiv CC_6H_5$ | H | H | H | H | 354 |
| 1-657 | H | OMe | H | H | H | H | 284 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-658 | H | $NH_2$ | H | H | H | H | 269 |
| 1-659 | H | NHMe | H | H | H | H | 283 |
| 1-660 | H | $NMe_2$ | H | H | H | H | 297 |
| 1-661 | H | $NHC_6H_5$ | H | H | H | H | 345 |
| 1-662 | H | H | Cl | H | H | H | 288 |
| 1-663 | H | H | Br | H | H | H | 332 |
| 1-664 | H | H | OH | H | H | H | 270 |
| 1-665 | H | H | Me | H | H | H | 268 |
| 1-666 | H | H | $CH=CH_2$ | H | H | H | 280 |
| 1-667 | H | H | $C\equiv CC_6H_5$ | H | H | H | 354 |
| 1-668 | H | H | OMe | H | H | H | 284 |
| 1-669 | H | H | $NH_2$ | H | H | H | 269 |
| 1-670 | H | H | NHMe | H | H | H | 283 |
| 1-671 | H | H | $NMe_2$ | H | H | H | 297 |
| 1-672 | H | H | $NHC_6H_5$ | H | H | H | 345 |
| 1-673 | H | H | H | Cl | H | H | 288 |
| 1-674 | H | H | H | Br | H | H | 332 |
| 1-675 | H | H | H | OH | H | H | 270 |
| 1-676 | H | H | H | Me | H | H | 268 |
| 1-677 | H | H | H | $CH=CH_2$ | H | H | 280 |
| 1-678 | H | H | H | $C\equiv CC_6H_5$ | H | H | 354 |
| 1-679 | H | H | H | OMe | H | H | 284 |
| 1-680 | H | H | H | $NH_2$ | H | H | 269 |
| 1-681 | H | H | H | NHMe | H | H | 283 |
| 1-682 | H | H | H | $NMe_2$ | H | H | 297 |
| 1-683 | H | H | H | $NHC_6H_5$ | H | H | 345 |
| 1-684 | H | H | H | H | C1 | H | 288 |
| 1-685 | H | H | H | H | Br | H | 332 |
| 1-686 | H | H | H | H | OH | H | 270 |
| 1-687 | H | H | H | H | Me | H | 268 |
| 1-688 | H | H | H | H | $CH=CH_2$ | H | 280 |
| 1-689 | H | H | H | H | $C\equiv CC_6H_5$ | H | 354 |
| 1-690 | H | H | H | H | OMe | H | 284 |

(continued)

| Exp. No. | $R^1$ | $R^5$ | $R^8$ | $R^7$ | $R^6$ | $A^{61}$ | Mass (MH+) |
|---|---|---|---|---|---|---|---|
| 1-691 | H | H | H | H | $NH_2$ | H | 269 |
| 1-692 | H | H | H | H | NHMe | H | 283 |
| 1-693 | H | H | H | H | $NMe_2$ | H | 297 |
| 1-694 | H | H | H | H | $NHC_6H_5$ | H | 345 |

The structures of the groups a-1 to a-144 as $A^{61}$ mentioned in Table 1 are shown below (the same shall apply to Tables 2 to 7).

[Formula 48]

a-1 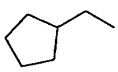
a-2 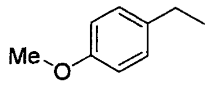
a-3 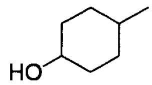

a-4 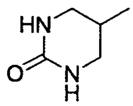
a-5 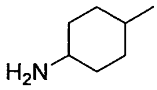
a-6 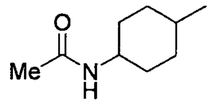

a-7 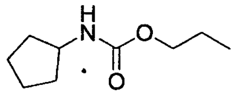
a-8 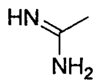
a-9 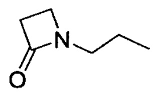

a-10 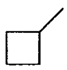
a-11 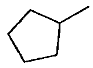
a-12 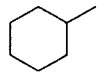

a-13 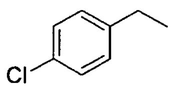
a-14 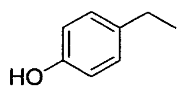
a-15 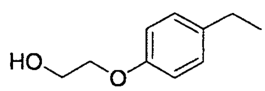

a-16 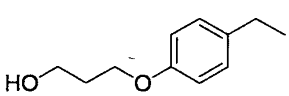
a-17 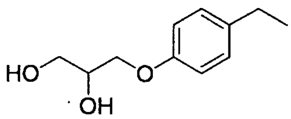
a-18 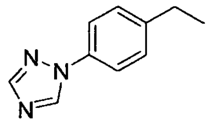

a-19 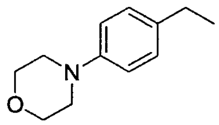
a-20 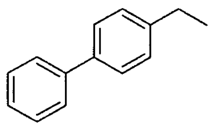
a-21 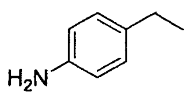

a-22 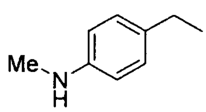
a-23 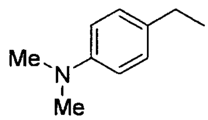
a-24 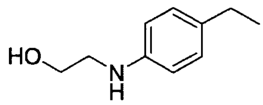

a-25 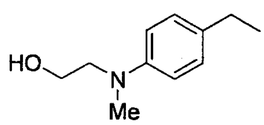
a-26 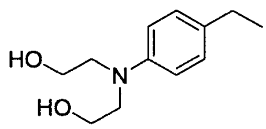
a-27 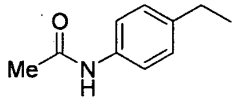

a-28 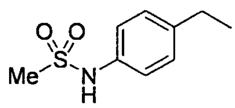
a-29 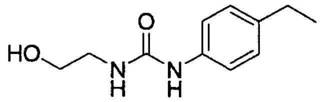
a-30 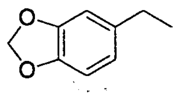

a-31 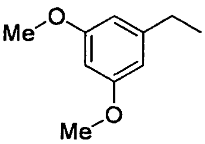 a-32 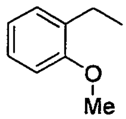 a-33 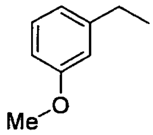

a-34 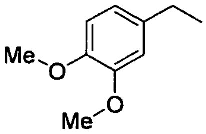 a-35 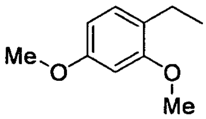 a-36 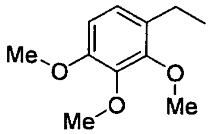

a-37 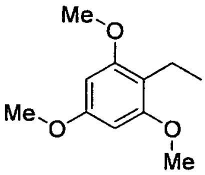 a-38 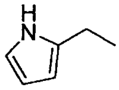 a-39 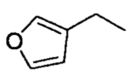

a-40 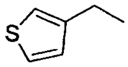 a-41 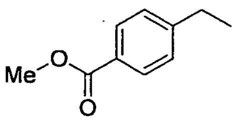 a-42 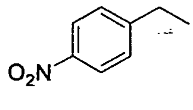

a-43 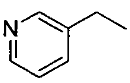 a-44 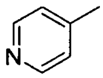 a-45 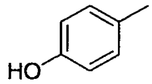

a-46 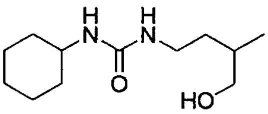 a-47 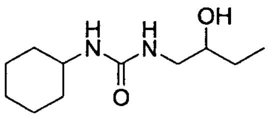 a-48 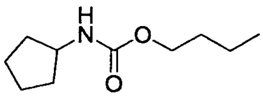

a-49 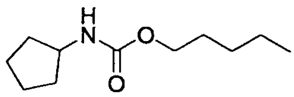 a-50 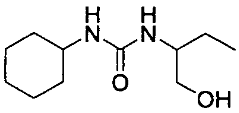 a-51 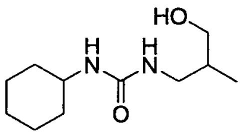

a-52 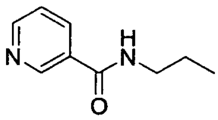 a-53 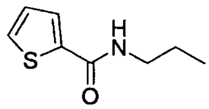 a-54 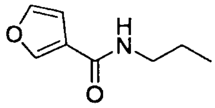

a-55 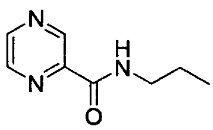 a-56 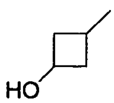 a-57 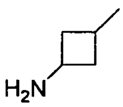

a-58 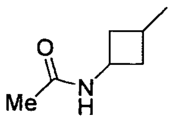 a-59 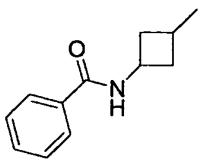 a-60 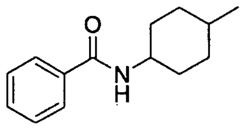

a-61 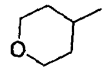

a-62 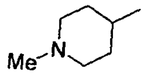

a-63 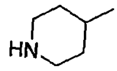

a-64 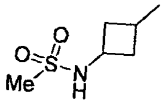

a-65 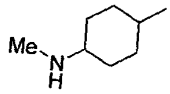

a-66 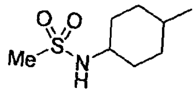

a-67 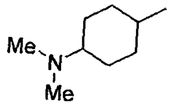

a-68 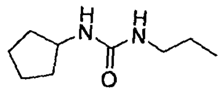

a-69 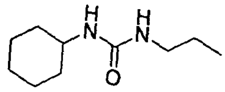

a-70 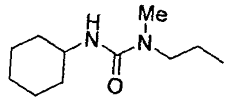

a-71 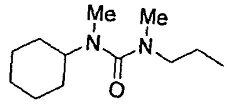

a-72 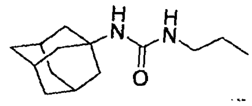

a-73 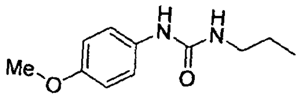

a-74 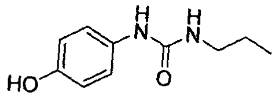

a-75 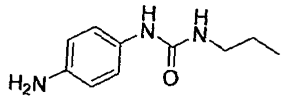

a-76 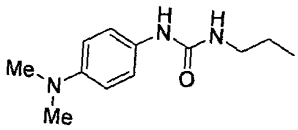

a-77 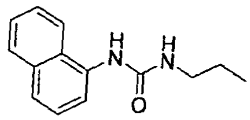

a-78 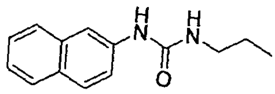

a-79 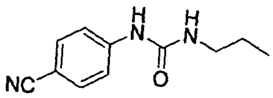

a-80 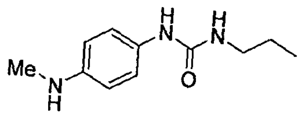

a-81 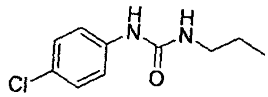

a-82 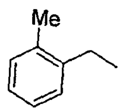

a-83 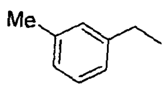

a-84 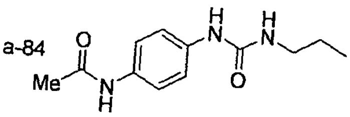

a-85 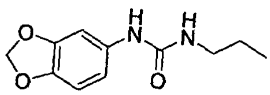

a-86 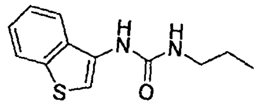

a-87 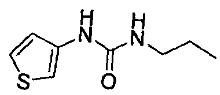

a-88 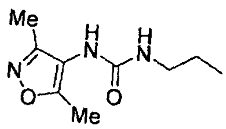

a-89 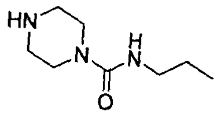

a-90 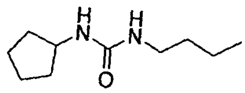

a-91 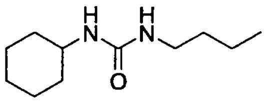 a-92 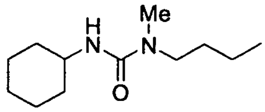 a-93 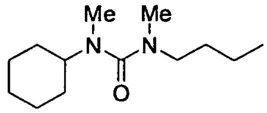

a-94 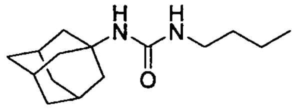 a-95 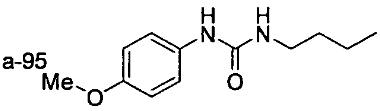 a-96 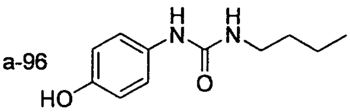

a-97 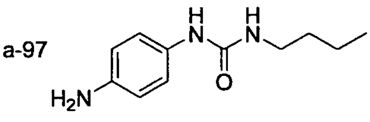 a-98 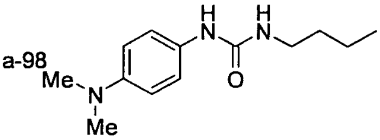 a-99 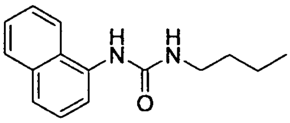

a-100 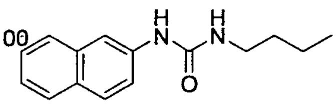 a-101 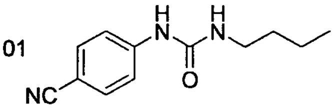 a-102 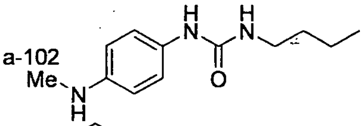

a-103 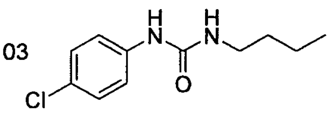 a-104 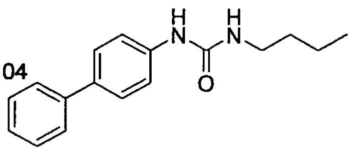 a-105 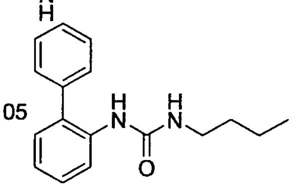

a-106 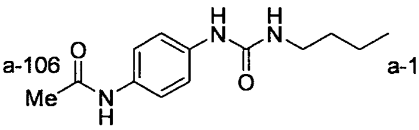 a-107 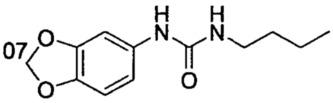 a-108 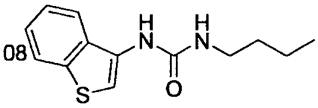

a-109 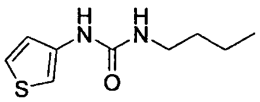 a-110 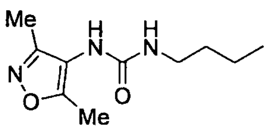 a-111 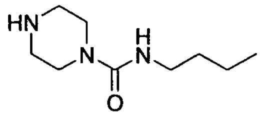

a-112 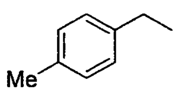 a-113 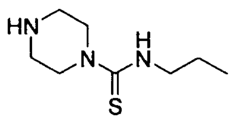 a-114 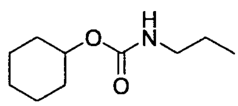

a-115 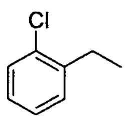 a-116 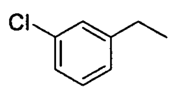 a-117 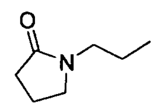

a-118 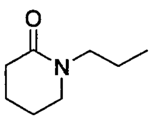 a-119 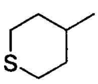 a-120 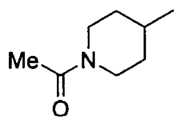

a-121 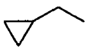 a-122 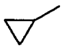 a-123 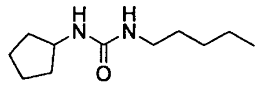

a-124 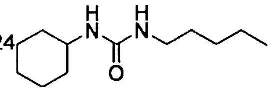 a-125 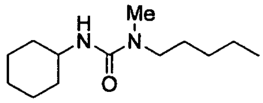 a-126 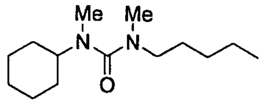

a-127 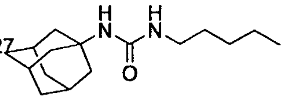 a-128 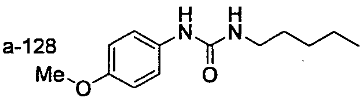 a-129 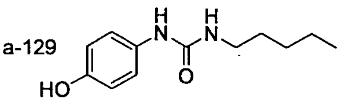

a-130 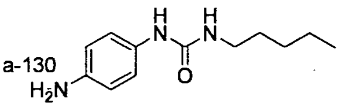 a-131 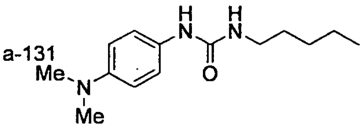 a-132 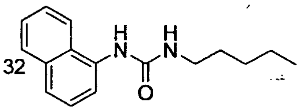

a-133 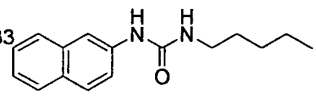 a-134 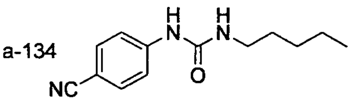 a-135 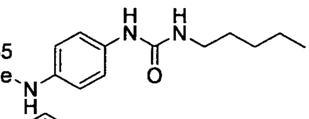

a-136 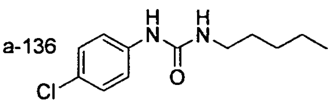 a-137 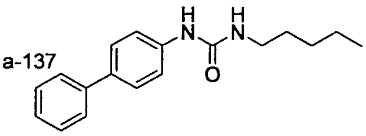 a-138 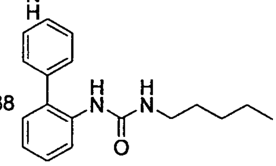

a-139 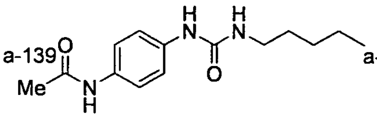 a-140 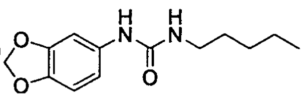 a-141 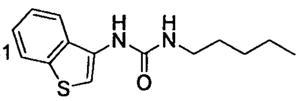

a-142 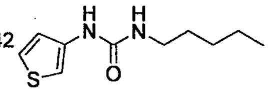 a-143 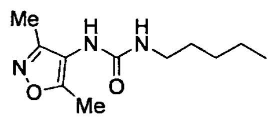 a-144 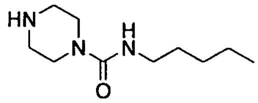

Example 2-1: 4-(2,3-Dihydro-1,5-diazaphenalen-1-yl)piperidine

Step A

4-(2,3-Dihydro-1,5-diazaphenalen-1-yl)piperidine-1-carboxylic acid tert-butyl ester (Intermediate 23)

By using 5-amino-4-vinylisoquinoline (Intermediate 1) obtained in Example 1-1, Step A and tert-butyl 4-oxo-1-piperidinecarboxylate (Aldrich), the title compound was obtained according to the methods described in Example 1-1, Steps B and C.
MS (m/z): 354 (MH+)

Step B

4-(2,3-Dihydro-1,5-diazaphenalen-1-yl)piperidine

By using 4-(2,3-dihydro-1,5-diazaphenalen-1-yl)piperidine-1-carboxylic acid tert-butyl ester (Intermediate 23) obtained in Step A mentioned above, the title compound was obtained as a hydrochloride according to the method described in Example 1-1, Step D.
$^1$H-NMR (DMSO-$d_6$) δ (ppm): 1.85-1.91 (2H, m), 2.03-2.12 (2H, m), 3.05-3.20 (2H, m), 3.21-3.27 (2H, m), 3.37-3.42

(4H, m), 4.24-4.34 (1H, m), 7.45 (1H, d, J=8.1Hz), 7.66 (1H, d, J=8.1Hz), 7.78 (1H, t, J=8.1Hz), 8.35 (1H, s), 9.05 (1H, brs), 9.51 (1H, s)
MS (m/z): 254 (MH+)

Examples 2-2 to 2-320

The compounds of Examples 2-2 to 2-320 were obtained by using 4-(2,3-dihydro-1,5-diazaphenalen-1-yl) piperidine obtained in Example 2-1 according to the methods described in Example 1-2 to 1-320.

Example 2-321: 6-Chloro-1-(piperidin-4-yl)-2,3-dihydro-1H-1,5-diazaphenalene

Step A

4-(6-Chloro-2,3-dihydro-1,5-diazaphenalen-1-yl)piperidine-1-carboxylic acid tert-butyl ester (Intermediate 24)

By using 5-amino-1-chloro-4-vinylisoquinoline (Intermediate 8) obtained in Example 1-321, Step D, the title compound was obtained according to the method described in Example 1-321.
MS (m/z): 388 (MH+)

Step B

6-Chloro-1-(piperidin-4-yl)-2,3-dihydro-1H-1,5-diazaphenalene

By using 4-(6-chloro-2,3-dihydro-1,5-diazaphenalen-1-yl)piperidine-1-carboxylic acid tert-butyl ester (Intermediate 24) obtained in Step A mentioned above, the title compound was obtained as a hydrochloride according to the method described in Example 1-1, Step D.
MS (m/z): 288 (MH+)

Example 2-322

The compound of Example 2-322 was obtained in the same manner as that used for the compound of Example 1-322.

Example 2-323

The compound of Example 2-323 was obtained in the same manner as that used for the compound of Example 1-323.

Examples 2-324 to 2-331

The compounds of Examples 2-324 to 2-331 were obtained by using 4-(6-chloro-2,3-dihydro-1,5-diazaphenalen-1-yl)piperidine-1-carboxylic acid tert-butyl ester (Intermediate 24) obtained in Example 2-321, Step A according to the methods described in Example 1-324 to 1-331.

Examples 2-332 to 2-650

The compounds of Examples 2-332 to 2-650 were obtained by using 6-chloro-1-(piperidin-4-yl)-2,3-dihydro-1H-1,5-diazaphenalene obtained in Example 2-321, or 6-hydroxy-1-(piperidin-4-yl)-2,3-dihydro-1H-1,5-diazaphenalene obtained in Example 2-323 according to the methods described in Example 1-2 to 1-320.

Example 2-651: 4-Chloro-1-(piperidin-4-yl)-2,3-dihydro-1H-1,5-diazaphenalene

Step A

4-(4-Chloro-2,3-dihydro-1,5-diazaphenalen-1-yl)piperidine-1-carboxylic acid tert-butyl ester (Intermediate 25)

By using 3-chloro-5-nitro-4-vinylisoquinoline (Intermediate 7) obtained in Example 1-321, Step C, reduction of the nitro group, reductive alkylation, and cyclization were performed according to the methods described in Example

1-321, Steps D to F to obtain the title compound.
MS (m/z): 388 (MH+)

Step B

4-Chloro-1-(piperidin-4-yl)-2,3-dihydro-1H -1,5-diazaphenalene

By using 4-(4-chloro-2,3-dihydro-1,5-diazaphenalen-1-yl)piperidine-1-carboxylic acid tert-butyl ester (Intermediate 25) obtained in Step C mentioned above, the title compound was obtained as a hydrochloride according to the method described in Example 1-1, Step D.
MS (m/z) : 288 (MH+)

Example 2-652

The compound of Example 2-652 was obtained according to the method described in Example 2-651.

Example 2-653

The compound of Example 2-653 was obtained according to Method A or Method B of Example 1-323,.

Examples 2-654 to 2-661

The compounds of Examples 2-654 to 2-661 were obtained by using 4-(4-chloro-2,3-dihydro-1,5-diazaphenalen-1-yl)piperidine-1-carboxylic acid tert-butyl ester according to the methods described in Example 1-324 to 1-331.

Example 2-662

The compound of Example 2-662 was obtained according to the method described in Example 2-663 mentioned below.

Example 2-663: 7-Bromo-1-(piperidin-4-yl)-2,3-dihydro-1H-1,5-diazaphenalene

Step A

4-(7-Bromo-2,3-dihydro-1,5-diazaphenalen-1-yl)piperidine-1-carboxylic acid tert-butyl ester (Intermediate 26), and

4-(8-bromo-2,3-dihydro-1,5-diazaphenalen-1-yl)piperidine-1-carboxylic acid tert-butyl ester (Intermediate 27)

By using 4-(2,3-dihydro-1,5-diazaphenalen-1-yl)piperidine-1-carboxylic acid tert-butyl ester (Intermediate 24) obtained in Example 2-1, Step A, each of the title compounds was obtained according to the method described in Example 1-663, Step A. MS (m/z): 432 (MH+)

Step B

7-Bromo-1-(piperidin-4-yl)-2,3-dihydro-1H-1,5-diazaphenalene

By using 4-(7-bromo-2,3-dihydro-1,5-diazaphenalen-1-yl)piperidine-1-carboxylic acid tert-butyl ester (Intermediate 26) obtained in Step A mentioned above, the title compound was obtained as a hydrochloride according to the method described in Example 1-1, Step D.
MS (m/z): 332 (MH+)

Examples 2-664 to 2-672

The compounds of Examples 2-664 to 2-672 were obtained by using 4-(7-bromo-2,3-dihydro-1,5-diazaphenalen-1-yl)piperidine-1-carboxylic acid tert-butyl ester obtained in Example 2-663, Step A according to the methods described in Examples 1-323 to 1-331.

Example 2-673

The compound of Example 2-673 was obtained according to the method described in Example 2-674 mentioned below.

Example 2-674: 8-Bromo-1-(piperidin-4-yl)-2,3-dihydro-1H-1,5-diazaphenalene

By using 4-(8-bromo-2,3-dihydro-1,5-diazaphenalen-1-yl)piperidine-1-carboxylic acid tert-butyl ester (Intermediate 27) obtained in Example 2-663, Step A, the title compound was obtained as a hydrochloride according to the method described in Example 1-1, Step D.
MS (m/z): 332 (MH+)

Examples 2-675 to 2-683

The compounds of Examples 2-675 to 2-683 were obtained by using 4-(8-bromo-2,3-dihydro-1,5-diazaphenalen-1-yl)piperidine-1-carboxylic acid tert-butyl ester obtained in Example 2-663, Step A according to the methods described in Examples 1-323 to 1-331.

Examples 2-684 and 2-685

The compounds of Examples 2-684 and 2-685 were obtained from the compound of Example 2-691 mentioned below by referring to a known diazotization-Sandmeyer reaction (for example, Denny, William , et al, J. Med. Chem., 2002, 740; Kulka, J. Am. Chem. Soc., 75, 3597 (1953) and the like).

Examples 2-686 to 2-690

The compounds of Examples 2-686 to 2-690 were obtained by using 4-(9-bromo-2,3-dihydro-1,5-diazaphenalen-1-yl)piperidine-1-carboxylic acid tert-butyl ester obtained in Example 2-685 according to the methods described in Example 1-323 to 1-327.

Example 2-691: 9-Amino-1-(piperidin-4-yl)-2,3-dihydro-1H-1,5-diazaphenalene

Step A

4-(9-Amino-2,3-dihydro-1,5-diazaphenalen-1-yl)piperidine-1-carboxylic acid tert-butyl ester (Intermediate 28)

By using 4-(7-bromo-2,3-dihydro-1,5-diazaphenalen-1-yl)piperidine-1-carboxylic acid tert-butyl ester (Intermediate 26) obtained in Example 2-663, Step A, nitration (9-position), and reduction (bromine atom→hydrogen atom at the 7-position, nitro group→amino group at the 9-position) were performed with referring to a known publication (for example, Kucznierz, R., Dickhaut, J, Leinert, H., Von Der Saal, W., Synth. Commun., 29, 1617 (1999); Ping Chen, Bioorganic & Medicinal Chemistry Letters, 13, 1345 (2003) and the like) to obtain the title compound.
MS (m/z): 369 (MH+)

Step B

9-Amino-1-(piperidin-4-yl)-2, 3-dihydro-1H-1,5-diazaphenalene

By using 4-(9-amino-2,3-dihydro-1,5-diazaphenalen-1-yl)piperidine-1-carboxylic acid tert-butyl ester (Intermediate 28) obtained in Step A mentioned above, the title compound was obtained as a hydrochloride according to the method described in Example 1-1, Step D.
MS (m/z): 269 (MH+)

Examples 2-692 to 2-694

The compounds of Examples 2-692 to 2-694 were obtained by using 4-(9-bromo-2,3-dihydro-1,5-diazaphenalen-1-yl)piperidine-1-carboxylic acid tert-butyl ester obtained in Example 2-685 according to the methods described in Example 1-329 to 1-331.
[Table 2]

| Exp. No. | $R^1$ | $R^5$ | $R^8$ | $R^7$ | $R^6$ | $A^{61}$ | Mass ($MH^+$) |
|---|---|---|---|---|---|---|---|
| 2-1 | H | H | H | H | H | H | 254 |
| 2-2 | H | H | H | H | H | Me | 268 |
| 2-3 | H | H | H | H | H | a-1 | 336 |
| 2-4 | H | H | H | H | H | a-2 | 374 |
| 2-5 | H | H | H | H | H | $C_6H_5$ | 330 |
| 2-6 | H | H | H | H | H | $CH_2COOH$ | 312 |
| 2-7 | H | H | H | H | H | $CH_2CH_2COOH$ | 326 |
| 2-8 | H | H | H | H | H | $CH_2CN$ | 293 |
| 2-9 | H | H | H | H | H | $CH_2CH_2CN$ | 307 |
| 2-10 | H | H | H | H | H | $CH_2CH_2OH$ | 298 |
| 2-11 | H | H | H | H | H | $CH(CH_2OH)_2$ | 328 |
| 2-12 | H | H | H | H | H | a-3 | 352 |
| 2-13 | H | H | H | H | H | $CH_2CH_2OMe$ | 312 |
| 2-14 | H | H | H | H | H | $CH_2CH_2OCOCMe_3$ | 382 |
| 2-15 | H | H | H | H | H | $CH_2CH_2SCOMe$ | 356 |
| 2-16 | H | H | H | H | H | $CH_2CH_2SH$ | 314 |
| 2-17 | H | H | H | H | H | $CH_2CH_2NH_2$ | 297 |
| 2-18 | H | H | H | H | H | $CH_2CH_2CH_2NH_2$ | 311 |
| 2-19 | H | H | H | H | H | $CH_2CH_2CH_2NHMe$ | 325 |
| 2-20 | H | H | H | H | H | $CH(CH_2NHCOMe)_2$ | 410 |
| 2-21 | H | H | H | H | H | $CH(CH_2NH_2)_2$ | 326 |
| 2-22 | H | H | H | H | H | $CH_2CH_2NHCOMe$ | 339 |
| 2-23 | H | H | H | H | H | a-4 | 352 |
| 2-24 | H | H | H | H | H | $CH_2CH_2NHCOCH_2NHCOMe$ | 396 |
| 2-25 | H | H | H | H | H | $CH_2CH_2NHCOCH_2NMe_2$ | 382 |
| 2-26 | H | H | H | H | H | $CH_2CH_2NHSO_2Me$ | 375 |
| 2-27 | H | H | H | H | H | a-5 | 351 |
| 2-28 | H | H | H | H | H | a-6 | 393 |
| 2-29 | H | H | H | H | H | $CH_2CH_2CH_2NHCONHCH_2CH_2OH$ | 398 |
| 2-30 | H | H | H | H | H | $CH_2CH_2CH_2NHCONHBn$ | 444 |
| 2-31 | H | H | H | H | H | $CH_2CH_2NHCSNHCH_2CH_2OH$ | 400 |
| 2-32 | H | H | H | H | H | $CH_2CH_2NHCSNHC_6H_5$ | 432 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-33 | H | H | H | H | H | $CH_2CH_2NHCOOCH_2CH_2NMe_2$ | 412 |
| 2-34 | H | H | H | H | H | a-7 | 409 |
| 2-35 | H | H | H | H | H | a-8 | 296 |
| 2-36 | H | H | H | H | H | a-9 | 351 |
| 2-37 | H | H | H | H | H | COMe | 296 |
| 2-38 | H | H | H | H | H | a-10 | 308 |
| 2-39 | H | H | H | H | H | a-11 | 322 |
| 2-40 | H | H | H | H | H | a-12 | 336 |
| 2-41 | H | H | H | H | H | Bn | 344 |
| 2-42 | H | H | H | H | H | a-13 | 378 |
| 2-43 | H | H | H | H | H | a-14 | 360 |
| 2-44 | H | H | H | H | H | a-15 | 404 |
| 2-45 | H | H | H | H | H | a-16 | 418 |
| 2-46 | H | H | H | H | H | a-17 | 434 |
| 2-47 | H | H | H | H | H | a-18 | 411 |
| 2-48 | H | H | H | H | H | a-19 | 429 |
| 2-49 | H | H | H | H | H | a-20 | 420 |
| 2-50 | H | H | H | H | H | a-21 | 359 |
| 2-51 | H | H | H | H | H | a-22 | 373 |
| 2-52 | H | H | H | H | H | a-23 | 387 |
| 2-53 | H | H | H | H | H | a-24 | 403 |
| 2-54 | H | H | H | H | H | a-25 | 417 |
| 2-55 | H | H | H | H | H | a-26 | 447 |
| 2-56 | H | H | H | H | H | a-27 | 401 |
| 2-57 | H | H | H | H | H | a-28 | 437 |
| 2-58 | H | H | H | H | H | a-29 | 446 |
| 2-59 | H | H | H | H | H | a-30 | 388 |
| 2-60 | H | H | H | H | H | a-31 | 404 |
| 2-61 | H | H | H | H | H | a-32 | 374 |
| 2-62 | H | H | H | H | H | a-33 | 374 |
| 2-63 | H | H | H | H | H | a-34 | 404 |
| 2-64 | H | H | H | H | H | a-35 | 404 |
| 2-65 | H | H | H | H | H | a-36 | 434 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-66 | H | H | H | H | H | a-37 | 434 |
| 2-67 | H | H | H | H | H | a-38 | 332 |
| 2-68 | H | H | H | H | H | a-39 | 334 |
| 2-69 | H | H | H | H | H | a-40 | 350 |
| 2-70 | H | H | H | H | H | a-41 | 402 |
| 2-71 | H | H | H | H | H | a-42 | 389 |
| 2-72 | H | H | H | H | H | a-43 | 345 |
| 2-73 | H | H | H | H | H | a-44 | 331 |
| 2-74 | H | H | H | H | H | a-45 | 346 |
| 2-75 | H | H | H | H | H | $CH_2CH_2CH_2COOH$ | 340 |
| 2-76 | H | H | H | H | H | $CH_2CH_2CH_2CH_2COOH$ | 354 |
| 2-77 | H | H | H | H | H | $CH_2CH_2CH_2CN$ | 321 |
| 2-78 | H | H | H | H | H | $CH_2CH_2CH_2CH_2CN$ | 335 |
| 2-79 | H | H | H | H | H | $CH_2CH$ (Me) OH | 312 |
| 2-80 | H | H | H | H | H | $CH(Me)CH_2OH$ | 312 |
| 2-81 | H | H | H | H | H | $CH_2CH_2CH_2OH$ | 312 |
| 2-82 | H | H | H | H | H | $CH_2CH$ (OH) $CH_2OH$ | 328 |
| 2-83 | H | H | H | H | H | $CH_2CH$ ($NH_2$) $CH_2OH$ | 327 |
| 2-84 | H | H | H | H | H | $CH_2CH$ (NHMe) $CH_2OH$ | 341 |
| 2-85 | H | H | H | H | H | $CH_2CH$ ($NMe_2$) $CH_2OH$ | 355 |
| 2-86 | H | H | H | H | H | $CH_2CH$ (Me) $CH_2OH$ | 326 |
| 2-87 | H | H | H | H | H | $CH_2CH_2CH_2CH_2OH$ | 326 |
| 2-88 | H | H | H | H | H | $CH_2CH_2CH_2CH_2CH_2OH$ | 340 |
| 2-89 | H | H | H | H | H | a-46 | 466 |
| 2-90 | H | H | H | H | H | CH ($CH_2OH$) $CH_2CH_2NHCONHC_6H_5$ | 460 |
| 2-91 | H | H | H | H | H | a-47 | 452 |
| 2-92 | H | H | H | H | H | $CH_2CH$ (OH) $CH_2NHCONHC_6H_5$ | 446 |
| 2-93 | H | H | H | H | H | $CH_2CH_2OCONHMe$ | 355 |
| 2-94 | H | H | H | H | H | $CH_2CH_2OCONHC_6H_5$ | 417 |
| 2-95 | H | H | H | H | H | $CH_2CH_2CH_2OCONHMe$ | 369 |
| 2-96 | H | H | H | H | H | a-48 | 437 |
| 2-97 | H | H | H | H | H | $CH_2CH_2CH_2OCONHC_6H_5$ | 431 |
| 2-98 | H | H | H | H | H | $CH_2CH_2CH_2CH_2OCONHMe$ | 383 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-99 | H | H | H | H | H | a-49 | 451 |
| 2-100 | H | H | H | H | H | $CH_2CH_2CH_2CH_2OCONHC_6H_5$ | 445 |
| 2-101 | H | H | H | H | H | $CH_2CH_2CH_2SH$ | 328 |
| 2-102 | H | H | H | H | H | $CH_2CH_2CH_2CH_2SH$ | 342 |
| 2-103 | H | H | H | H | H | $CH_2CH_2SCOC_6H_5$ | 418 |
| 2-104 | H | H | H | H | H | $CH_2CH_2CH_2SCOMe$ | 370 |
| 2-105 | H | H | H | H | H | $CH_2CH_2CH_2SCOC_6H_5$ | 432 |
| 2-106 | H | H | H | H | H | $CH_2CH_2CH_2OMe$ | 326 |
| 2-107 | H | H | H | H | H | $CH_2CH_2CH_2CH_2OMe$ | 340 |
| 2-108 | H | H | H | H | H | $CH_2CH_2CH_2CH_2CH_2OMe$ | 354 |
| 2-109 | H | H | H | H | H | $CH_2CH_2OCOMe$ | 340 |
| 2-110 | H | H | H | H | H | $CH_2CH_2CH_2OCOMe$ | 354 |
| 2-111 | H | H | H | H | H | $CH_2CH_2CH_2CH_2OCOMe$ | 368 |
| 2-112 | H | H | H | H | H | $CH_2CH_2OCOCH_2Me$ | 354 |
| 2-113 | H | H | H | H | H | $CH_2CH_2CH_2OCOCH_2Me$ | 368 |
| 2-114 | H | H | H | H | H | $CH_2CH_2CH_2CH_2OCOCH_2Me$ | 382 |
| 2-115 | H | H | H | H | H | $CH_2CH_2OCOCHMe_2$ | 368 |
| 2-116 | H | H | H | H | H | $CH_2CH_2CH_2OCOCHMe_2$ | 382 |
| 2-117 | H | H | H | H | H | $CH_2CH_2CH_2CH_2OCOCHMe_2$ | 396 |
| 2-118 | H | H | H | H | H | $CH_2CH_2CH_2OCOCMe_3$ | 396 |
| 2-119 | H | H | H | H | H | $CH_2CH_2CH_2CH_2OCOCMe_3$ | 410 |
| 2-120 | H | H | H | H | H | $CH_2CH_2OCOC_6H_5$ | 402 |
| 2-121 | H | H | H | H | H | $CH_2CH_2CH_2OCOC_6H_5$ | 416 |
| 2-122 | H | H | H | H | H | $CH_2CH_2CH_2CH_2OCOC_6H_5$ | 430 |
| 2-123 | H | H | H | H | H | $CH_2CH_2OCOCH_2OH$ | 356 |
| 2-124 | H | H | H | H | H | $CH_2CH_2OCOCH_2NH_2$ | 355 |
| 2-125 | H | H | H | H | H | $CH_2CH_2OCH_2CH_2OMe$ | 356 |
| 2-126 | H | H | H | H | H | $CH_2CH_2OCH_2CH_2OH$ | 342 |
| 2-127 | H | H | H | H | H | $CH_2CH_2CH_2OCH_2CH_2OH$ | 356 |
| 2-128 | H | H | H | H | H | $CH_2CH_2CH_2CH_2OCH_2CH_2OH$ | 370 |
| 2- 129 | H | H | H | H | H | $CH_2CH\ (CH_2OH)\ NH_2$ | 327 |
| 2-130 | H | H | H | H | H | $CH_2CH\ (CH_2OH)\ NHCOMe$ | 369 |
| 2-131 | H | H | H | H | H | a-50 | 452 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-132 | H | H | H | H | H | $CH_2CH\ (CH_2OH)\ NHCONHC_6H_5$ | 446 |
| 2-133 | H | H | H | H | H | $CH(CH_2OH)CH_2NH_2$ | 327 |
| 2-134 | H | H | H | H | H | $CH\ (CH_2OH)\ CH_2NHCOMe$ | 369 |
| 2-135 | H | H | H | H | H | a-51 | 452 |
| 2-136 | H | H | H | H | H | $CH(CH_2OH)CH_2NHCONHC_6H_5$ | 446 |
| 2-137 | H | H | H | H | H | $CH_2CH(OH)CH_2NH_2$ | 327 |
| 2-138 | H | H | H | H | H | $CH_2CH\ (OH)\ CH_2NHMe$ | 341 |
| 2-139 | H | H | H | H | H | $CH_2CH(OH)CH_2NMe_2$ | 355 |
| 2-140 | H | H | H | H | H | $CH_2CH_2CH_2CH_2NH_2$ | 325 |
| 2-141 | H | H | H | H | H | $CH_2CH_2CH_2CH_2CH_2NH_2$ | 339 |
| 2-142 | H | H | H | H | H | $CH_2CH_2NHMe$ | 311 |
| 2-143 | H | H | H | H | H | $CH_2CH_2CH_2CH_2NHMe$ | 339 |
| 2-144 | H | H | H | H | H | $CH_2CH_2CH_2CH_2CH_2NHMe$ | 353 |
| 2-145 | H | H | H | H | H | $CH_2CH_2NMe_2$ | 325 |
| 2-146 | H | H | H | H | H | $CH_2CH_2CH_2NMe_2$ | 339 |
| 2-147 | H | H | H | H | H | $CH_2CH_2CH_2CH_2NMe_2$ | 353 |
| 2-148 | H | H | H | H | H | $CH_2CH_2CH_2CH_2CH_2NMe_2$ | 367 |
| 2-149 | H | H | H | H | H | $CH_2CH_2NHCH_2CH_2OH$ | 341 |
| 2-150 | H | H | H | H | H | $CH_2CH_2CH_2NHCH_2CH_2OH$ | 355 |
| 2-151 | H | H | H | H | H | $CH_2CH_2CH_2CH_2NHCH_2CH_2OH$ | 369 |
| 2-152 | H | H | H | H | H | $CH_2CONH_2$ | 311 |
| 2-153 | H | H | H | H | H | $CH_2CH_2CONH_2$ | 325 |
| 2-154 | H | H | H | H | H | $CH_2CH_2CH_2CONH_2$ | 339 |
| 2-155 | H | H | H | H | H | $CH_2CH_2NHCOCH_2Me$ | 353 |
| 2-156 | H | H | H | H | H | $CH_2CH_2NHCOCH_2CH_2Me$ | 367 |
| 2-157 | H | H | H | H | H | $CH_2CH_2NHCOCHMe_2$ | 367 |
| 2-158 | H | H | H | H | H | $CH_2CH_2NHCOCMe_3$ | 381 |
| 2-159 | H | H | H | H | H | $CH_2CH_2NHCOCH_2OH$ | 355 |
| 2-160 | H | H | H | H | H | $CH_2CH_2NHCOCH_2OMe$ | 369 |
| 2-161 | H | H | H | H | H | $CH_2CH_2NHCOC_6H_5$ | 401 |
| 2-162 | H | H | H | H | H | a-52 | 402 |
| 2-163 | H | H | H | H | H | a-53 | 407 |
| 2-164 | H | H | H | H | H | a-54 | 391 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-165 | H | H | H | H | H | a-55 | 403 |
| 2-166 | H | H | H | H | H | $CH_2CH_2NHCOCH_2NHCOCH_2CHMe_2$ | 438 |
| 2-167 | H | H | H | H | H | $CH_2CH_2NHCOCH_2NHCOC_6H_5$ | 458 |
| 2-168 | H | H | H | H | H | $CH_2CH_2CH_2NHCOMe$ | 353 |
| 2-169 | H | H | H | H | H | $CH_2CH_2CH_2NHCOCH_2Me$ | 367 |
| 2-170 | H | H | H | H | H | $CH_2CH_2CH_2NHCOCH_2CH_2Me$ | 381 |
| 2-171 | H | H | H | H | H | $CH_2CH_2CH_2NHCOCHMe_2$ | 381 |
| 2-172 | H | H | H | H | H | $CH_2CH_2CH_2NHCOCMe_3$ | 395 |
| 2-173 | H | H | H | H | H | $CH_2CH_2CH_2CH_2NHCOMe$ | 367 |
| 2-174 | H | H | H | H | H | $CH_2CH_2CH_2CH_2NHCOCH_2Me$ | 381 |
| 2-175 | H | H | H | H | H | $CH_2CH_2CH_2CH_2NHCOCH_2CH_2Me$ | 395 |
| 2-176 | H | H | H | H | H | $CH_2CH_2CH_2CH_2NHCOCHMe_2$ | 395 |
| 2-177 | H | H | H | H | H | $CH_2CH_2CH_2CH_2NHCOCMe_3$ | 409 |
| 2-178 | H | H | H | H | H | $CH_2CH_2N$ (Me) COMe | 353 |
| 2-179 | H | H | H | H | H | $CH_2CH_2N$ (Me) $COC_6H_5$ | 415 |
| 2-180 | H | H | H | H | H | $CH_2CH_2CH_2N$ (Me) COMe | 367 |
| 2-181 | H | H | H | H | H | $CH_2CH_2CH_2N$ (Me) $COC_6H_5$ | 429 |
| 2-182 | H | H | H | H | H | $CH_2CH_2CH_2CH_2N$ (Me) COMe | 381 |
| 2-183 | H | H | H | H | H | $CH_2CH_2N(CH_2CH_2OH)COMe$ | 383 |
| 2-184 | H | H | H | H | H | $CH_2CH_2CH_2N$ ($CH_2CH_2OH$) COMe | 397 |
| 2-185 | H | H | H | H | H | $CH_2CH_2N$ ($CH_2CN$) COMe | 378 |
| 2-186 | H | H | H | H | H | $CH_2CH_2CH_2N$ ($CH_2CN$) COMe | 392 |
| 2-187 | H | H | H | H | H | a-56 | 324 |
| 2-188 | H | H | H | H | H | a-57 | 323 |
| 2-189 | H | H | H | H | H | a-58 | 365 |
| 2-190 | H | H | H | H | H | a-59 | 427 |
| 2-191 | H | H | H | H | H | a-60 | 455 |
| 2-192 | H | H | H | H | H | a-61 | 338 |
| 2-193 | H | H | H | H | H | a-62 | 351 |
| 2-194 | H | H | H | H | H | a-63 | 337 |
| 2-195 | H | H | H | H | H | $CH_2CH_2NHSO_2C_6H_5$ | 437 |
| 2-196 | H | H | H | H | H | $CH_2CH_2NHSO_2NMe_2$ | 404 |
| 2-197 | H | H | H | H | H | $CH_2CH_2N$ (Me) $SO_2Me$ | 389 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-198 | H | H | H | H | H | $CH_2CH_2N$ (Me) $SO_2C_6H_5$ | 451 |
| 2-199 | H | H | H | H | H | $CH_2CH_2N(Me)SO_2NMe_2$ | 418 |
| 2-200 | H | H | H | H | H | $CH_2CH_2N$ ($CH_2CH_2OH$) $SO_2Me$ | 405 |
| 2-201 | H | H | H | H | H | $CH_2CH_2CH_2NHSO_2Me$ | 389 |
| 2-202 | H | H | H | H | H | $CH_2CH_2CH_2NHSO_2C_6H_5$ | 451 |
| 2-203 | H | H | H | H | H | $CH_2CH_2CH_2NHSO_2NMe_2$ | 418 |
| 2-204 | H | H | H | H | H | $CH_2CH_2CH_2N$ (Me) $SO_2Me$ | 403 |
| 2-205 | H | H | H | H | H | $CH_2CH_2CH_2N$ (Me) $SO_2C_6H_5$ | 465 |
| 2-206 | H | H | H | H | H | $CH_2CH_2CH_2N$ (Me) $SO_2NMe_2$ | 432 |
| 2-207 | H | H | H | H | H | $CH_2CH_2CH_2N(CH_2CH_2OH)$ $SO_2Me$ | 419 |
| 2-208 | H | H | H | H | H | $CH_2CH_2CH_2CH_2NHSO_2Me$ | 403 |
| 2-209 | H | H | H | H | H | $CH_2CH_2CH_2CH_2NHSO_2C_6H_5$ | 465 |
| 2-210 | H | H | H | H | H | $CH_2CH_2CH_2CH_2NHSO_2NMe_2$ | 432 |
| 2-211 | H | H | H | H | H | $CH_2CH_2CH_2CH_2N$ (Me) $SO_2Me$ | 417 |
| 2-212 | H | H | H | H | H | $CH_2CH_2CH_2CH_2N(Me)SO_2C_6H_5$ | 479 |
| 2-213 | H | H | H | H | H | $CH_2CH_2CH_2CH_2N$ (Me) $SO_2NMe_2$ | 446 |
| 2-214 | H | H | H | H | H | $CH_2CH_2CH_2CH_2N$ ($CH_2CH_2OH$) $SO_2Me$ | 433 |
| 2-215 | H | H | H | H | H | a-64 | 401 |
| 2-216 | H | H | H | H | H | a-65 | 365 |
| 2-217 | H | H | H | H | H | a-66 | 429 |
| 2-218 | H | H | H | H | H | a-67 | 379 |
| 2-219 | H | H | H | H | H | $CH_2CH_2NHCONHMe$ | 354 |
| 2-220 | H | H | H | H | H | $CH_2CH_2NHCONHCH_2Me$ | 368 |
| 2-221 | H | H | H | H | H | $CH_2CH_2NHCONHCH_2CH_2Me$ | 382 |
| 2-222 | H | H | H | H | H | $CH_2CH_2NHCONHCHMe_2$ | 382 |
| 2-223 | H | H | H | H | H | $CH_2CH_2NHCONHCMe_3$ | 396 |
| 2-224 | H | H | H | H | H | a-68 | 408 |
| 2-225 | H | H | H | H | H | a-69 | 422 |
| 2-226 | H | H | H | H | H | a-70 | 436 |
| 2-227 | H | H | H | H | H | a-71 | 450 |
| 2-228 | H | H | H | H | H | a-72 | 474 |
| 2-229 | H | H | H | H | H | $CH_2CH_2NHCONHC_6H_5$ | 416 |
| 2-230 | H | H | H | H | H | a-73 | 446 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-231 | H | H | H | H | H | a-74 | 432 |
| 2-232 | H | H | H | H | H | a-75 | 431 |
| 2-233 | H | H | H | H | H | a-76 | 459 |
| 2-234 | H | H | H | H | H | a-77 | 466 |
| 2-235 | H | H | H | H | H | a-78 | 466 |
| 2-236 | H | H | H | H | H | a-79 | 441 |
| 2-237 | H | H | H | H | H | a-80 | 445 |
| 2-238 | H | H | H | H | H | a-81 | 450 |
| 2-239 | H | H | H | H | H | $CH_2CH_2NHCONHCOC_6H_5$ | 444 |
| 2-240 | H | H | H | H | H | $CH_2CH_2NHCONHSO_2C_6H_5$ | 480 |
| 2-241 | H | H | H | H | H | a-82 | 358 |
| 2-242 | H | H | H | H | H | a-83 | 358 |
| 2-243 | H | H | H | H | H | a-84 | 473 |
| 2-244 | H | H | H | H | H | a-85 | 460 |
| 2-245 | H | H | H | H | H | a-86 | 472 |
| 2-246 | H | H | H | H | H | a-87 | 422 |
| 2-247 | H | H | H | H | H | $CH_2CH_2NHCONHCMe_2CH_2CMe_3$ | 452 |
| 2-248 | H | H | H | H | H | a-88 | 435 |
| 2-249 | H | H | H | H | H | $CH_2CH_2N$ (Me) $CONHC_6H_5$ | 430 |
| 2-250 | H | H | H | H | H | $CH_2CH_2N$ (Me) CON (Me) $C_6H_5$ | 444 |
| 2-251 | H | H | H | H | H | $CH_2CH_2NHCONHCH_2CH_2OH$ | 384 |
| 2-252 | H | H | H | H | H | $CH_2CH_2NHCONHCH_2CH_2OMe$ | 398 |
| 2-253 | H | H | H | H | H | $CH_2CH_2NHCONHCH_2CH_2NMe_2$ | 411 |
| 2-254 | H | H | H | H | H | $CH_2CH_2NHCON$ (Me) $CH_2CH_2OH$ | 398 |
| 2-255 | H | H | H | H | H | $CH_2CH_2NHCON$ $(CH_2CH_2OH)_2$ | 428 |
| 2-256 | H | H | H | H | H | a-89 | 409 |
| 2-257 | H | H | H | H | H | $CH_2CH_2NHCONMe_2$ | 368 |
| 2-258 | H | H | H | H | H | $CH_2CH_2CH_2NHCONHMe$ | 368 |
| 2-259 | H | H | H | H | H | $CH_2CH_2CH_2NHCONHCH_2Me$ | 382 |
| 2-260 | H | H | H | H | H | $CH_2CH_2CH_2NHCONHCH_2CH_2Me$ | 396 |
| 2-261 | H | H | H | H | H | $CH_2CH_2CH_2NHCONHCHMe_2$ | 396 |
| 2-262 | H | H | H | H | H | $CH_2CH_2CH_2NHCONHCMe_3$ | 410 |
| 2-263 | H | H | H | H | H | a-90 | 422 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-264 | H | H | H | H | H | a-91 | 436 |
| 2-265 | H | H | H | H | H | a-92 | 450 |
| 2-266 | H | H | H | H | H | a-93 | 464 |
| 2-267 | H | H | H | H | H | a-94 | 488 |
| 2-268 | H | H | H | H | H | $CH_2CH_2CH_2NHCONHC_6H_5$ | 430 |
| 2-269 | H | H | H | H | H | a-95 | 460 |
| 2-270 | H | H | H | H | H | a-96 | 446 |
| 2-271 | H | H | H | H | H | a-97 | 445 |
| 2-272 | H | H | H | H | H | a-98 | 473 |
| 2-273 | H | H | H | H | H | a-99 | 480 |
| 2-274 | H | H | H | H | H | a-100 | 480 |
| 2-275 | H | H | H | H | H | a-101 | 455 |
| 2-276 | H | H | H | H | H | a-102 | 459 |
| 2-277 | H | H | H | H | H | a-103 | 464 |
| 2-278 | H | H | H | H | H | $CH_2CH_2CH_2NHCONHCOC_6H_5$ | 458 |
| 2-279 | H | H | H | H | H | a-104 | 506 |
| 2-280 | H | H | H | H | H | a-105 | 506 |
| 2-281 | H | H | H | H | H | a-106 | 487 |
| 2-282 | H | H | H | H | H | a-107 | 474 |
| 2-283 | H | H | H | H | H | a-108 | 486 |
| 2-284 | H | H | H | H | H | a-109 | 436 |
| 2-285 | H | H | H | H | H | $CH_2CH_2CH_2NHCONHCMe_2CH_2CMe_3$ | 466 |
| 2-286 | H | H | H | H | H | a-110 | 449 |
| 2-287 | H | H | H | H | H | $CH_2CH_2CH_2N$ (Me) $CONHC_6H_5$ | 444 |
| 2-288 | H | H | H | H | H | $CH_2CH_2CH_2N$ (Me) CON (Me) $C_6H_5$ | 458 |
| 2-289 | H | H | H | H | H | $CH_2CH_2CH_2NHCONHCH_2CH_2OMe$ | 412 |
| 2-290 | H | H | H | H | H | $CH_2CH_2CH_2NHCONHCH_2CH_2NMe_2$ | 425 |
| 2-291 | H | H | H | H | H | $CH_2CH_2CH_2NHCON$ (Me) $CH_2CH_2OH$ | 412 |
| 2-292 | H | H | H | H | H | $CH_2CH_2CH_2NHCON$ $(CH_2CH_2OH)_2$ | 442 |
| 2-293 | H | H | H | H | H | a-111 | 423 |
| 2-294 | H | H | H | H | H | $CH_2CH_2CH_2NHCONMe_2$ | 382 |
| 2-295 | H | H | H | H | H | $CH_2CH_2NHCSNHCH_2Me$ | 384 |
| 2-296 | H | H | H | H | H | $CH_2CH_2NHCSNHCH_2CH_2Me$ | 398 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-297 | H | H | H | H | H | $CH_2CH_2NHCSNHCHMe_2$ | 398 |
| 2-298 | H | H | H | H | H | $CH_2CH_2NHCSNHCMe_3$ | 412 |
| 2-299 | H | H | H | H | H | a-112 | 358 |
| 2-300 | H | H | H | H | H | $CH_2CH_2NHCSNHCH_2CH_2OMe$ | 414 |
| 2-301 | H | H | H | H | H | $CH_2CH_2NHCSNHCH_2CH_2NMe_2$ | 427 |
| 2-302 | H | H | H | H | H | $CH_2CH_2NHCSN (Me) CH_2CH_2OH$ | 414 |
| 2-303 | H | H | H | H | H | $CH_2CH_2NHCSN(CH_2CH_2OH)_2$ | 444 |
| 2-304 | H | H | H | H | H | a-113 | 425 |
| 2-305 | H | H | H | H | H | $CH_2CH_2NHCSNMe_2$ | 384 |
| 2-306 | H | H | H | H | H | $CH_2CH_2NHCOOMe$ | 355 |
| 2-307 | H | H | H | H | H | $CH_2CH_2NHCOOCH_2Me$ | 369 |
| 2-308 | H | H | H | H | H | $CH_2CH_2NHCOOCH_2CH_2Me$ | 383 |
| 2-309 | H | H | H | H | H | $CH_2CH_2NHCOOCHMe_2$ | 383 |
| 2-310 | H | H | H | H | H | a-114 | 423 |
| 2-311 | H | H | H | H | H | $CH_2CH_2NHCOOC_6H_5$ | 417 |
| 2-312 | H | H | H | H | H | $CH_2CH_2NHCOOCH_2CH_2OH$ | 385 |
| 2-313 | H | H | H | H | H | $CH_2CH_2NHCOOCH_2CH_2OMe$ | 399 |
| 2-314 | H | H | H | H | H | a-115 | 378 |
| 2-315 | H | H | H | H | H | a-116 | 378 |
| 2-316 | H | H | H | H | H | a-117 | 365 |
| 2-317 | H | H | H | H | H | a-118 | 379 |
| 2-318 | H | H | H | H | H | a-119 | 354 |
| 2-319 | H | H | H | H | H | a-120 | 379 |
| 2-320 | H | H | H | H | H | $COC_6H_5$ | 358 |
| 2-321 | Cl | H | H | H | H | H | 288 |
| 2-322 | Br | H | H | H | H | H | 332 |
| 2-323 | OH | H | H | H | H | H | 270 |
| 2-324 | Me | H | H | H | H | H | 268 |
| 2-325 | $CH=CH_2$ | H | H | H | H | H | 280 |
| 2-326 | $C\equiv CC_6H_5$ | H | H | H | H | H | 354 |
| 2-327 | OMe | H | H | H | H | H | 284 |
| 2-328 | $NH_2$ | H | H | H | H | H | 269 |
| 2-329 | NHMe | H | H | H | H | H | 283 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-330 | $NMe_2$ | H | H | H | H | H | 297 |
| 2-331 | $NHC_6H_5$ | H | H | H | H | H | 345 |
| 2-332 | OH | H | H | H | H | Me | 284 |
| 2-333 | OH | H | H | H | H | a-1 | 352 |
| 2-334 | OH | H | H | H | H | a-2 | 390 |
| 2-335 | OH | H | H | H | H | $C_6H_5$ | 346 |
| 2-336 | OH | H | H | H | H | $CH_2COOH$ | 328 |
| 2-337 | OH | H | H | H | H | $CH_2CH_2COOH$ | 342 |
| 2-338 | OH | H | H | H | H | $CH_2CN$ | 309 |
| 2-339 | OH | H | H | H | H | $CH_2CH_2CN$ | 323 |
| 2-340 | OH | H | H | H | H | $CH_2CH_2OH$ | 314 |
| 2-341 | OH | H | H | H | H | $CH\ (CH_2OH)_2$ | 344 |
| 2-342 | OH | H | H | H | H | a-3 | 368 |
| 2-343 | OH | H | H | H | H | $CH_2CH_2OMe$ | 328 |
| 2-344 | OH | H | H | H | H | $CH_2CH_2OCOCMe_3$ | 398 |
| 2-345 | OH | H | H | H | H | $CH_2CH_2SCOMe$ | 372 |
| 2-346 | OH | H | H | H | H | $CH_2CH_2SH$ | 330 |
| 2-347 | OH | H | H | H | H | $CH_2CH_2NH_2$ | 313 |
| 2-348 | OH | H | H | H | H | $CH_2CH_2CH_2NH_2$ | 327 |
| 2-349 | OH | H | H | H | H | $CH_2CH_2CH_2NHMe$ | 341 |
| 2-350 | OH | H | H | H | H | $CH\ (CH_2NHCOMe)$ | 426 |
| 2-351 | OH | H | H | H | H | $CH(CH_2NH_2)_2$ | 342 |
| 2-352 | OH | H | H | H | H | $CH_2CH_2NHCOMe$ | 355 |
| 2-353 | OH | H | H | H | H | a-4 | 368 |
| 2-354 | OH | H | H | H | H | $CH_2CH_2NHCOCH_2NHCOMe$ | 412 |
| 2-355 | OH | H | H | H | H | $CH_2CH_2NHCOCH_2NMe_2$ | 398 |
| 2-356 | OH | H | H | H | H | $CH_2CH_2NHSO_2Me$ | 391 |
| 2-357 | OH | H | H | H | H | a-5 | 367 |
| 2-358 | OH | H | H | H | H | a-6 | 409 |
| 2-359 | OH | H | H | H | H | $CH_2CH_2CH_2NHCONHCH_2CH_2OH$ | 414 |
| 2-360 | OH | H | H | H | H | $CH_2CH_2CH_2NHCONHBn$ | 460 |
| 2-361 | OH | H | H | H | H | $CH_2CH_2NHCSNHCH_2CH_2OH$ | 416 |
| 2-362 | OH | H | H | H | H | $CH_2CH_2NHCSNHC_6H_5$ | 448 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-363 | OH | H | H | H | H | $CH_2CH_2NHCOOCH_2CH_2NMe_2$ | 428 |
| 2-364 | OH | H | H | H | H | a-7 | 425 |
| 2-365 | OH | H | H | H | H | a-8 | 312 |
| 2-366 | OH | H | H | H | H | a-9 | 367 |
| 2-367 | OH | H | H | H | H | COMe | 312 |
| 2-368 | OH | H | H | H | H | a-10 | 324 |
| 2-369 | OH | H | H | H | H | a-11 | 338 |
| 2-370 | OH | H | H | H | H | a-12 | 352 |
| 2-371 | OH | H | H | H | H | Bn | 360 |
| 2-372 | OH | H | H | H | H | a-13 | 394 |
| 2-373 | OH | H | H | H | H | a-14 | 376 |
| 2-374 | OH | H | H | H | H | a-15 | 420 |
| 2-375 | OH | H | H | H | H | a-16 | 434 |
| 2-376 | OH | H | H | H | H | a-17 | 450 |
| 2-377 | OH | H | H | H | H | a-18 | 427 |
| 2-378 | OH | H | H | H | H | a-19 | 445 |
| 2-379 | OH | H | H | H | H | a-20 | 436 |
| 2-380 | OH | H | H | H | H | a-21 | 375 |
| 2-381 | OH | H | H | H | H | a-22 | 389 |
| 2-382 | OH | H | H | H | H | a-23 | 403 |
| 2-383 | OH | H | H | H | H | a-24 | 419 |
| 2-384 | OH | H | H | H | H | a-25 | 433 |
| 2-385 | OH | H | H | H | H | a-26 | 463 |
| 2-386 | OH | H | H | H | H | a-27 | 417 |
| 2-387 | OH | H | H | H | H | a-28 | 453 |
| 2-388 | OH | H | H | H | H | a-29 | 462 |
| 2-389 | OH | H | H | H | H | a-30 | 404 |
| 2-390 | OH | H | H | H | H | a-31 | 420 |
| 2-391 | OH | H | H | H | H | a-32 | 390 |
| 2-392 | OH | H | H | H | H | a-33 | 390 |
| 2-393 | OH | H | H | H | H | a-34 | 420 |
| 2-394 | OH | H | H | H | H | a-35 | 420 |
| 2-395 | OH | H | H | H | H | a-36 | 450 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-396 | OH | H | H | H | H | a-37 | 450 |
| 2-397 | OH | H | H | H | H | a-38 | 348 |
| 2-398 | OH | H | H | H | H | a-39 | 350 |
| 2-399 | OH | H | H | H | H | a-40 | 366 |
| 2-400 | OH | H | H | H | H | a-41 | 418 |
| 2-401 | OH | H | H | H | H | a-42 | 405 |
| 2-402 | OH | H | H | H | H | a-43 | 361 |
| 2-403 | OH | H | H | H | H | a-44 | 347 |
| 2-404 | OH | H | H | H | H | a-45 | 362 |
| 2-405 | OH | H | H | H | H | $CH_2CH_2CH_2COOH$ | 356 |
| 2-406 | OH | H | H | H | H | $CH_2CH_2CH_2CH_2COOH$ | 370 |
| 2-407 | OH | H | H | H | H | $CH_2CH_2CH_2CN$ | 337 |
| 2-408 | OH | H | H | H | H | $CH_2CH_2CH_2CH_2CN$ | 351 |
| 2-409 | OH | H | H | H | H | $CH_2CH$ (Me) OH | 328 |
| 2-410 | OH | H | H | H | H | CH(Me)$CH_2OH$ | 328 |
| 2-411 | OH | H | H | H | H | $CH_2CH_2CH_2OH$ | 328 |
| 2-412 | OH | H | H | H | H | $CH_2CH$ (OH) $CH_2OH$ | 344 |
| 2-413 | OH | H | H | H | H | $CH_2CH$ ($NH_2$) $CH_2OH$ | 343 |
| 2-414 | OH | H | H | H | H | $CH_2CH$ (NHMe) $CH_2OH$ | 357 |
| 2-415 | OH | H | H | H | H | $CH_2CH$ ($NMe_2$) $CH_2OH$ | 371 |
| 2-416 | OH | H | H | H | H | $CH_2CH$ (Me) $CH_2OH$ | 342 |
| 2-417 | OH | H | H | H | H | $CH_2CH_2CH_2CH_2OH$ | 342 |
| 2-418 | OH | H | H | H | H | $CH_2CH_2CH_2CH_2CH_2OH$ | 356 |
| 2-419 | OH | H | H | H | H | a-46 | 482 |
| 2-420 | OH | H | H | H | H | CH ($CH_2OH$) $CH_2CH_2NHCONHC_6H_5$ | 476 |
| 2-421 | OH | H | H | H | H | a-47 | 468 |
| 2-422 | OH | H | H | H | H | $CH_2CH$ (OH) $CH_2NHCONHC_6H_5$ | 462 |
| 2-423 | OH | H | H | H | H | $CH_2CH_2OCONHMe$ | 371 |
| 2-424 | OH | H | H | H | H | $CH_2CH_2OCONHC_6H_5$ | 433 |
| 2-425 | OH | H | H | H | H | $CH_2CH_2CH_2OCONHMe$ | 385 |
| 2-426 | OH | H | H | H | H | a-48 | 453 |
| 2-427 | OH | H | H | H | H | $CH_2CH_2CH_2OCONHC_6H_5$ | 447 |
| 2-428 | OH | H | H | H | H | $CH_2CH_2CH_2CH_2OCONHMe$ | 399 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-429 | OH | H | H | H | H | a-49 | 467 |
| 2-430 | OH | H | H | H | H | $CH_2CH_2CH_2CH_2OCONHC_6H_5$ | 461 |
| 2-431 | OH | H | H | H | H | $CH_2CH_2CH_2SH$ | 344 |
| 2-432 | OH | H | H | H | H | $CH_2CH_2CH_2CH_2SH$ | 358 |
| 2-433 | OH | H | H | H | H | $CH_2CH_2SCOC_6H_5$ | 434 |
| 2-434 | OH | H | H | H | H | $CH_2CH_2CH_2SCOMe$ | 386 |
| 2-435 | OH | H | H | H | H | $CH_2CH_2CH_2SCOC_6H_5$ | 448 |
| 2-436 | OH | H | H | H | H | $CH_2CH_2CH_2OMe$ | 342 |
| 2-437 | OH | H | H | H | H | $CH_2CH_2CH_2CH_2OMe$ | 356 |
| 2-438 | OH | H | H | H | H | $CH_2CH_2CH_2CH_2CH_2OMe$ | 370 |
| 2-439 | OH | H | H | H | H | $CH_2CH_2OCOMe$ | 356 |
| 2-440 | OH | H | H | H | H | $CH_2CH_2CH_2OCOMe$ | 370 |
| 2-441 | OH | H | H | H | H | $CH_2CH_2CH_2CH_2OCOMe$ | 384 |
| 2-442 | OH | H | H | H | H | $CH_2CH_2OCOCH_2Me$ | 370 |
| 2-443 | OH | H | H | H | H | $CH_2CH_2CH_2OCOCH_2Me$ | 384 |
| 2-444 | OH | H | H | H | H | $CH_2CH_2CH_2CH_2OCOCH_2Me$ | 398 |
| 2-445 | OH | H | H | H | H | $CH_2CH_2OCOCHMe_2$ | 384 |
| 2-446 | OH | H | H | H | H | $CH_2CH_2CH_2OCOCHMe_2$ | 398 |
| 2-447 | OH | H | H | H | H | $CH_2CH_2CH_2CH_2OCOCHMe_2$ | 412 |
| 2-448 | OH | H | H | H | H | $CH_2CH_2CH_2OCOCMe_3$ | 412 |
| 2-449 | OH | H | H | H | H | $CH_2CH_2CH_2CH_2OCOCMe_3$ | 426 |
| 2-450 | OH | H | H | H | H | $CH_2CH_2OCOC_6H_5$ | 418 |
| 2-451 | OH | H | H | H | H | $CH_2CH_2CH_2OCOC_6H_5$ | 432 |
| 2-452 | OH | H | H | H | H | $CH_2CH_2CH_2CH_2OCOC_6H_5$ | 446 |
| 2-453 | OH | H | H | H | H | $CH_2CH_2OCOCH_2OH$ | 372 |
| 2-454 | OH | H | H | H | H | $CH_2CH_2OCOCH_2NH_2$ | 371 |
| 2-455 | OH | H | H | H | H | $CH_2CH_2OCH_2CH_2OMe$ | 372 |
| 2-456 | OH | H | H | H | H | $CH_2CH_2OCH_2CH_2OH$ | 358 |
| 2-457 | OH | H | H | H | H | $CH_2CH_2CH_2OCH_2CH_2OH$ | 372 |
| 2-458 | OH | H | H | H | H | $CH_2CH_2CH_2CH_2OCH_2CH_2OH$ | 386 |
| 2-459 | OH | H | H | H | H | $CH_2CH$ ($CH_2OH$) $NH_2$ | 343 |
| 2-460 | OH | H | H | H | H | $CH_2CH$ ($CH_2OH$) NHCOMe | 385 |
| 2-461 | OH | H | H | H | H | a-50 | 468 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-462 | OH | H | H | H | H | $CH_2CH\ (CH_2OH)\ NHCONHC_6H_5$ | 462 |
| 2-463 | OH | H | H | H | H | $CH\ (CH_2OH)\ CH_2NH_2$ | 343 |
| 2-464 | OH | H | H | H | H | $CH\ (CH_2OH)\ CH_2NHCOMe$ | 385 |
| 2-465 | OH | H | H | H | H | a-51 | 468 |
| 2-466 | OH | H | H | H | H | $CH\ (CH_2OH)\ CH_2NHCONHC_6H_5$ | 462 |
| 2-467 | OH | H | H | H | H | $CH_2CH\ (OH)\ CH_2NH_2$ | 343 |
| 2-468 | OH | H | H | H | H | $CH_2CH\ (OH)\ CH_2NHMe$ | 357 |
| 2-469 | OH | H | H | H | H | $CH_2CH\ (OH)\ CH_2NMe_2$ | 371 |
| 2-470 | OH | H | H | H | H | $CH_2CH_2CH_2CH_2NH_2$ | 341 |
| 2-471 | OH | H | H | H | H | $CH_2CH_2CH_2CH_2CH_2NH_2$ | 355 |
| 2-472 | OH | H | H | H | H | $CH_2CH_2NHMe$ | 327 |
| 2-473 | OH | H | H | H | H | $CH_2CH_2CH_2CH_2NHMe$ | 355 |
| 2-474 | OH | H | H | H | H | $CH_2CH_2CH_2CH_2CH_2NHMe$ | 369 |
| 2-475 | OH | H | H | H | H | $CH_2CH_2NMe_2$ | 341 |
| 2-476 | OH | H | H | H | H | $CH_2CH_2CH_2NMe_2$ | 355 |
| 2-477 | OH | H | H | H | H | $CH_2CH_2CH_2CH_2NMe_2$ | 369 |
| 2-478 | OH | H | H | H | H | $CH_2CH_2CH_2CH_2CH_2NMe_2$ | 383 |
| 2-479 | OH | H | H | H | H | $CH_2CH_2NHCH_2CH_2OH$ | 357 |
| 2-480 | OH | H | H | H | H | $CH_2CH_2CH_2NHCH_2CH_2OH$ | 371 |
| 2-481 | OH | H | H | H | H | $CH_2CH_2CH_2CH_2NHCH_2CH_2OH$ | 385 |
| 2-482 | OH | H | H | H | H | $CH_2CONH_2$ | 327 |
| 2-483 | OH | H | H | H | H | $CH_2CH_2CONH_2$ | 341 |
| 2-484 | OH | H | H | H | H | $CH_2CH_2CH_2CONH_2$ | 355 |
| 2-485 | OH | H | H | H | H | $CH_2CH_2NHCOCH_2Me$ | 369 |
| 2-486 | OH | H | H | H | H | $CH_2CH_2NHCOCH_2CH_2Me$ | 383 |
| 2-487 | OH | H | H | H | H | $CH_2CH_2NHCOCHMe_2$ | 383 |
| 2-488 | OH | H | H | H | H | $CH_2CH_2NHCOCMe_3$ | 397 |
| 2-489 | OH | H | H | H | H | $CH_2CH_2NHCOCH_2OH$ | 371 |
| 2-490 | OH | H | H | H | H | $CH_2CH_2NHCOCH_2OMe$ | 385 |
| 2-491 | OH | H | H | H | H | $CH_2CH_2NHCOC_6H_5$ | 417 |
| 2-492 | OH | H | H | H | H | a-52 | 418 |
| 2-493 | OH | H | H | H | H | a-53 | 423 |
| 2-494 | OH | H | H | H | H | a-54 | 407 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-495 | OH | H | H | H | H | a-55 | 419 |
| 2-496 | OH | H | H | H | H | $CH_2CH_2NHCOCH_2NHCOCH_2CHMe_2$ | 454 |
| 2-497 | OH | H | H | H | H | $CH_2CH_2NHCOCH_2NHCOC_6H_5$ | 474 |
| 2-498 | OH | H | H | H | H | $CH_2CH_2CH_2NHCOMe$ | 369 |
| 2-499 | OH | H | H | H | H | $CH_2CH_2CH_2NHCOCH_2Me$ | 383 |
| 2-500 | OH | H | H | H | H | $CH_2CH_2CH_2NHCOCH_2CH_2Me$ | 397 |
| 2-501 | OH | H | H | H | H | $CH_2CH_2CH_2NHCOCHMe_2$ | 397 |
| 2-502 | OH | H | H | H | H | $CH_2CH_2CH_2NHCOCMe_3$ | 411 |
| 2-503 | OH | H | H | H | H | $CH_2CH_2CB_2CH_2NHCOMe$ | 383 |
| 2-504 | OH | H | H | H | H | $CH_2CH_2CH_2CH_2NHCOCH_2Me$ | 397 |
| 2-505 | OH | H | H | H | H | $CH_2CH_2CH_2CH_2NHCOCH_2CH_2Me$ | 411 |
| 2-506 | OH | H | H | H | H | $CH_2CH_2CH_2CH_2NHCOCHMe_2$ | 411 |
| 2-507 | OH | H | H | H | H | $CH_2CH_2CH_2CH_2NHCOCMe_3$ | 425 |
| 2-508 | OH | H | H | H | H | $CH_2CH_2N$ (Me) COMe | 369 |
| 2-509 | OH | H | H | H | H | $CH_2CH_2N(Me)COC_6H_5$ | 431 |
| 2-510 | OH | H | H | H | H | $CH_2CH_2CH_2N$(Me) COMe | 383 |
| 2-511 | OH | H | H | H | H | $CH_2CH_2CH_2N(Me)COC_6H_5$ | 445 |
| 2-512 | OH | H | H | H | H | $CH_2CH_2CH_2CH_2N$(Me) COMe | 397 |
| 2-513 | OH | H | H | H | H | $CH_2CH_2N(CH_2CH_2OH)COMe$ | 399 |
| 2-514 | OH | H | H | H | H | $CH_2CH_2CH_2N(CH_2CH_2OH)COMe$ | 413 |
| 2-515 | OH | H | H | H | H | $CH_2CH_2N(CH_2CN)COMe$ | 394 |
| 2-516 | OH | H | H | H | H | $CH_2CH_2CH_2N(CH_2CN)COMe$ | 408 |
| 2-517 | OH | H | H | H | H | a-56 | 340 |
| 2-518 | OH | H | H | H | H | a-57 | 339 |
| 2-519 | OH | H | H | H | H | a-58 | 381 |
| 2-520 | OH | H | H | H | H | a-59 | 443 |
| 2-521 | OH | H | H | H | H | a-60 | 471 |
| 2-522 | OH | H | H | H | H | a-61 | 354 |
| 2-523 | OH | H | H | H | H | a-62 | 367 |
| 2-524 | OH | H | H | H | H | a-63 | 353 |
| 2-525 | OH | H | H | H | H | $CH_2CH_2NHSO_2C_6H_5$ | 453 |
| 2-526 | OH | H | H | H | H | $CH_2CH_2NHSO_2NMe_2$ | 420 |
| 2-527 | OH | H | H | H | H | $CH_2CH_2N(Me)SO_2Me$ | 405 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-528 | OH | H | H | H | H | $CH_2CH_2N$ (Me) $SO_2C_6H_5$ | 467 |
| 2-529 | OH | H | H | H | H | $CH_2CH_2N$ (Me) $SO_2NMe_2$ | 434 |
| 2-530 | OH | H | H | H | H | $CH_2CH_2N(CH_2CH_2OH)SO_2Me$ | 421 |
| 2-531 | OH | H | H | H | H | $CH_2CH_2CH_2NHSO_2Me$ | 405 |
| 2-532 | OH | H | H | H | H | $CH_2CH_2CH_2NHSO_2C_6H_s$ | 467 |
| 2-533 | OH | H | H | H | H | $CH_2CH_2CH_2NHSO_2NMe_2$ | 434 |
| 2-534 | OH | H | H | H | H | $CH_2CH_2CH_2N$ (Me) $SO_2Me$ | 419 |
| 2-535 | OH | H | H | H | H | $CH_2CH_2CH_2N$ (Me) $SO_2C_6H_5$ | 481 |
| 2-536 | OH | H | H | H | H | $CH_2CH_2CH_2N$ (Me) $SO_2NMe_2$ | 448 |
| 2-537 | OH | H | H | H | H | $CH_2CH_2CH_2N(CH_2CH_2OH)$ $SO_2Me$ | 435 |
| 2-538 | OH | H | H | H | H | $CH_2CH_2CH_2CH_2NHSO_2Me$ | 419 |
| 2-539 | OH | H | H | H | H | $CH_2CH_2CH_2CH_2NHSO_2C_6H_5$ | 481 |
| 2-540 | OH | H | H | H | H | $CH_2CH_2CH_2CH_2NHSO_2NMe_2$ | 448 |
| 2-541 | OH | H | H | H | H | $CH_2CH_2CH_2CH_2N(Me)SO_2Me$ | 433 |
| 2-542 | OH | H | H | H | H | $CH_2CH_2CH_2CH_2N$ (Me) $SO_2C_6H_5$ | 495 |
| 2-543 | OH | H | H | H | H | $CH_2CH_2CH_2CH_2N$ (Me) $SO_2NMe_2$ | 462 |
| 2-544 | OH | H | H | H | H | $CH_2CH_2CH_2CH_2N$ $(CH_2CH_2OH)$ $SO_2Me$ | 449 |
| 2-545 | OH | H | H | H | H | a-64 | 417 |
| 2-546 | OH | H | H | H | H | a-65 | 381 |
| 2-547 | OH | H | H | H | H | a-66 | 445 |
| 2-548 | OH | H | H | H | H | a-67 | 395 |
| 2-549 | OH | H | H | H | H | $CH_2CH_2NHCONHMe$ | 370 |
| 2-550 | OH | H | H | H | H | $CH_2CH_2NHCONHCH_2Me$ | 384 |
| 2-551 | OH | H | H | H | H | $CH_2CH_2NHCONHCH_2CH_2Me$ | 398 |
| 2-552 | OH | H | H | H | H | $CH_2CH_2NHCONHCHMe_2$ | 398 |
| 2-553 | OH | H | H | H | H | $CH_2CH_2NHCONHCMe_3$ | 412 |
| 2-554 | OH | H | H | H | H | a-68 | 424 |
| 2-555 | OH | H | H | H | H | a-69 | 438 |
| 2-556 | OH | H | H | H | H | a-70 | 452 |
| 2-557 | OH | H | H | H | H | a-71 | 466 |
| 2-558 | OH | H | H | H | H | a-72 | 490 |
| 2-559 | OH | H | H | H | H | $CH_2CH_2NHCONHC_6H_5$ | 432 |
| 2-560 | OH | H | H | H | H | a-73 | 462 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-561 | OH | H | H | H | H | a-74 | 448 |
| 2-562 | OH | H | H | H | H | a-75 | 447 |
| 2-563 | OH | H | H | H | H | a-76 | 475 |
| 2-564 | OH | H | H | H | H | a-77 | 482 |
| 2-565 | OH | H | H | H | H | a-78 | 482 |
| 2-566 | OH | H | H | H | H | a-79 | 457 |
| 2-567 | OH | H | H | H | H | a-80 | 461 |
| 2-568 | OH | H | H | H | H | a-81 | 466 |
| 2-569 | OH | H | H | H | H | $CH_2CH_2NHCONHCOC_6H_5$ | 460 |
| 2-570 | OH | H | H | H | H | $CH_2CH_2NHCONHSO_2C_6H_5$ | 496 |
| 2-571 | OH | H | H | H | H | a-82 | 374 |
| 2-572 | OH | H | H | H | H | a-83 | 374 |
| 2-573 | OH | H | H | H | H | a-84 | 489 |
| 2-574 | OH | H | H | H | H | a-85 | 476 |
| 2-575 | OH | H | H | H | H | a-86 | 488 |
| 2-576 | OH | H | H | H | H | a-87 | 438 |
| 2-577 | OH | H | H | H | H | $CH_2CH_2NHCONHCMe_2CH_2CMe_3$ | 468 |
| 2-578 | OH | H | H | H | H | a-88 | 451 |
| 2-579 | OH | H | H | H | H | $CH_2CH_2N$ (Me) $CONHC_6H_5$ | 446 |
| 2-580 | OH | H | H | H | H | $CH_2CH_2N$ (Me) CON (Me) $C_6H_5$ | 460 |
| 2-581 | OH | H | H | H | H | $CH_2CH_2NHCONHCH_2CH_2OH$ | 400 |
| 2-582 | OH | H | H | H | H | $CH_2CH_2NHCONHCH_2CH_2OMe$ | 414 |
| 2-583 | OH | H | H | H | H | $CH_2CH_2NHCONHCH_2CH_2NMe_2$ | 427 |
| 2-584 | OH | H | H | H | H | $CH_2CH_2NHCON(Me)CH_2CH_2OH$ | 414 |
| 2-585 | OH | H | H | H | H | $CH_2CH_2NHCON$ $(CH_2CH_2OH)_2$ | 444 |
| 2-586 | OH | H | H | H | H | a-89 | 425 |
| 2-587 | OH | H | H | H | H | $CH_2CH_2NHCONMe_2$ | 384 |
| 2-588 | OH | H | H | H | H | $CH_2CH_2CH_2NHCONHMe$ | 384 |
| 2-589 | OH | H | H | H | H | $CH_2CH_2CH_2NHCONHCH_2Me$ | 398 |
| 2-590 | OH | H | H | H | H | $CH_2CH_2CH_2NHCONHCH_2CH_2Me$ | 412 |
| 2-591 | OH | H | H | H | H | $CH_2CH_2CH_2NHCONHCHMe_2$ | 412 |
| 2-592 | OH | H | H | H | H | $CH_2CH_2CH_2NHCONHCMe_3$ | 426 |
| 2-593 | OH | H | H | H | H | a-90 | 438 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-594 | OH | H | H | H | H | a-91 | 452 |
| 2-595 | OH | H | H | H | H | a-92 | 466 |
| 2-596 | OH | H | H | H | H | a-93 | 480 |
| 2-597 | OH | H | H | H | H | a-94 | 504 |
| 2-598 | OH | H | H | H | H | $CH_2CH_2CH_2NHCONHC_6H_5$ | 446 |
| 2-599 | OH | H | H | H | H | a-95 | 476 |
| 2-600 | OH | H | H | H | H | a-96 | 462 |
| 2-601 | OH | H | H | H | H | a-97 | 461 |
| 2-602 | OH | H | H | H | H | a-98 | 489 |
| 2-603 | OH | H | H | H | H | a-99 | 496 |
| 2-604 | OH | H | H | H | H | a-100 | 496 |
| 2-605 | OH | H | H | H | H | a-101 | 471 |
| 2-606 | OH | H | H | H | H | a-102 | 475 |
| 2-607 | OH | H | H | H | H | a-103 | 480 |
| 2-608 | OH | H | H | H | H | $CH_2CH_2CH_2NHCONHCOC_6H_5$ | 474 |
| 2-609 | OH | H | H | H | H | a-104 | 522 |
| 2-610 | OH | H | H | H | H | a-105 | 522 |
| 2-611 | OH | H | H | H | H | a-106 | 503 |
| 2-612 | OH | H | H | H | H | a-107 | 490 |
| 2-613 | OH | H | H | H | H | a-108 | 502 |
| 2-614 | OH | H | H | H | H | a-109 | 452 |
| 2-615 | OH | H | H | H | H | $CH_2CH_2CH_2NHCONHCMe_2CH_2CMe_3$ | 482 |
| 2-616 | OH | H | H | H | H | a-110 | 465 |
| 2-617 | OH | H | H | H | H | $CH_2CH_2CH_2N$ (Me) $CONHC_6H_5$ | 460 |
| 2-618 | OH | H | H | H | H | $CH_2CH_2CH_2N$(Me) CON (Me) $C_6H_5$ | 474 |
| 2-619 | OH | H | H | H | H | $CH_2CH_2CH_2NHCONHCH_2CH_2OMe$ | 428 |
| 2-620 | OH | H | H | H | H | $CH_2CH_2CH_2NHCONHCH_2CH_2NMe_2$ | 441 |
| 2-621 | OH | H | H | H | H | $CH_2CH_2CH_2NHCON$ (Me) $CH_2CH_2OH$ | 428 |
| 2-622 | OH | H | H | H | H | $CH_2CH_2CH_2NHCON$ $(CH_2CH_2OH)_2$ | 458 |
| 2-623 | OH | H | H | H | H | a-111 | 439 |
| 2-624 | OH | H | H | H | H | $CH_2CH_2CH_2NHCONMe_2$ | 398 |
| 2-625 | OH | H | H | H | H | $CH_2CH_2NHCSNHCH_2Me$ | 400 |
| 2-626 | OH | H | H | H | H | $CH_2CH_2NHCSNHCH_2CH_2Me$ | 414 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-627 | OH | H | H | H | H | $CH_2CH_2NHCSNHCHMe_2$ | 414 |
| 2-628 | OH | H | H | H | H | $CH_2CH_2NHCSNHCMe_3$ | 428 |
| 2-629 | OH | H | H | H | H | a-112 | 374 |
| 2-630 | OH | H | H | H | H | $CH_2CH_2NHCSNHCH_2CH_2OMe$ | 430 |
| 2-631 | OH | H | H | H | H | $CH_2CH_2NHCSNBCH_2CH_2NMe_2$ | 443 |
| 2-632 | OH | H | H | H | H | $CH_2CH_2NHCSN$ (Me) $CH_2CH_2OH$ | 430 |
| 2-633 | OH | H | H | H | H | $CH_2CH_2NHCSN$ $(CH_2CH_2OH)_2$ | 460 |
| 2-634 | OH | H | H | H | H | a-113 | 441 |
| 2-635 | OH | H | H | H | H | $CH_2CH_2NHCSNMe_2$ | 400 |
| 2-636 | OH | H | H | H | H | $CH_2CH_2NHCOOMe$ | 371 |
| 2-637 | OH | H | H | H | H | $CH_2CH_2NHCOOCH_2Me$ | 385 |
| 2-638 | OH | H | H | H | H | $CH_2CH_2NHCOOCH_2CH_2Me$ | 399 |
| 2-639 | OH | H | H | H | H | $CH_2CH_2NHCOOCHMe_2$ | 399 |
| 2-640 | OH | H | H | H | H | a-114 | 439 |
| 2-641 | OH | H | H | H | H | $CH_2CH_2NHCOOC_6H_5$ | 433 |
| 2-642 | OH | H | H | H | H | $CH_2CH_2NHCOOCH_2CH_2OH$ | 401 |
| 2-643 | OH | H | H | H | H | $CH_2CH_2NHCOOCH_2CH_2OMe$ | 415 |
| 2-644 | OH | H | H | H | H | a-115 | 394 |
| 2-645 | OH | H | H | H | H | a-116 | 394 |
| 2-646 | OH | H | H | H | H | a-117 | 381 |
| 2-647 | OH | H | H | H | H | a-118 | 395 |
| 2-648 | OH | H | H | H | H | a-119 | 370 |
| 2-649 | OH | H | H | H | H | a-120 | 395 |
| 2-650 | OH | H | H | H | H | $COC_6H_5$ | 374 |
| 2-651 | H | Cl | H | H | H | H | 288 |
| 2-652 | H | Br | H | H | H | H | 332 |
| 2-653 | H | OH | H | H | H | H | 270 |
| 2-654 | H | Me | H | H | H | H | 268 |
| 2-655 | H | $CH=CH_2$ | H | H | H | H | 280 |
| 2-656 | H | $C\equiv CC_6H_5$ | H | H | H | H | 354 |
| 2-657 | H | OMe | H | H | H | H | 284 |
| 2-658 | H | $NH_2$ | H | H | H | H | 269 |
| 2-659 | H | NHMe | H | H | H | H | 283 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-660 | H | $NMe_2$ | H | H | H | H | 297 |
| 2-661 | H | $NHC_6H_5$ | H | H | H | H | 345 |
| 2-662 | H | H | Cl | H | H | H | 288 |
| 2-663 | H | H | Br | H | H | H | 332 |
| 2-664 | H | H | OH | H | H | H | 270 |
| 2-665 | H | H | Me | H | H | H | 268 |
| 2-666 | H | H | $CH=CH_2$ | H | H | H | 280 |
| 2-667 | H | H | $C= CC_6H_5$ | H | H | H | 354 |
| 2-668 | H | H | OMe | H | H | H | 284 |
| 2-669 | H | H | $NH_2$ | H | H | H | 269 |
| 2-670 | H | H | NHMe | H | H | H | 283 |
| 2-671 | H | H | $NMe_2$ | H | H | H | 297 |
| 2-672 | H | H | $NHC_6H_5$ | H | H | H | 345 |
| 2-673 | H | H | H | Cl | H | H | 288 |
| 2-674 | H | H | H | Br | H | H | 332 |
| 2-675 | H | H | H | OH | H | H | 270 |
| 2-676 | H | H | H | Me | H | H | 268 |
| 2-677 | H | H | H | $CH=CH_2$ | H | H | 280 |
| 2-678 | H | H | H | $C\equiv CC_6H_5$ | H | H | 354 |
| 2-679 | H | H | H | OMe | H | H | 284 |
| 2-680 | H | H | H | $NH_2$ | H | H | 269 |
| 2-681 | H | H | H | NHMe | H | H | 283 |
| 2-682 | H | H | H | $NMe_2$ | H | H | 297 |
| 2-683 | H | H | H | $NHC_6H_5$ | H | H | 345 |
| 2-684 | H | H | H | H | Cl | H | 288 |
| 2-685 | H | H | H | H | Br | H | 332 |
| 2-686 | H | H | H | H | OH | H | 270 |
| 2-687 | H | H | H | H | Me | H | 268 |
| 2-688 | H | H | H | H | $CH=CH_2$ | H | 280 |
| 2-689 | H | H | H | H | $C\equiv CC_6H_5$ | H | 354 |
| 2-690 | H | H | H | H | OMe | H | 284 |
| 2-691 | H | H | H | H | $NH_2$ | H | 269 |
| 2-692 | H | H | H | H | NHMe | H | 283 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 2-693 | H | H | H | H | $NMe_2$ | H | 297 |
| 2-694 | H | H | H | H | $NHC_6H_5$ | H | 345 |

Example 3-1: 3-(2,3-Dihydro-1,5-diazaphenalen-1-yl)pyrrolidine

Step A

3-(2,3-Dihydro-1,5-diazaphenalen-1-yl)pyrrolidine-1-carboxylic acid tert-butyl ester (Intermediate 29)

By using 5-amino-4-vinylisoquinoline (Intermediate 1) obtained in Example 1-1, Step A, and tert-butyl 3-oxo-1-pyrrolidinecarboxylate (AstaTech), the title compound was obtained according to the methods described in Example 1-1, Steps B and C.
MS (m/z): 340 (MH+)

Step B

3-(2, 3-Dihydro-1,5-diazaphenalen-1-yl)pyrrolidine

By using 3-(2,3-dihydro-1,5-diazaphenalen-1-yl)pyrrolidine-1-carboxylic acid tert-butyl ester (Intermediate 29) obtained in Step A mentioned above, the title compound was obtained as a hydrochloride according to the method described in Example 1-1, Step D.
$^1$H-NMR, (DMSO-$d_6$) δ (ppm): 2.06-2.14 (2H, m), 2.23-2.33 (2H, m), 3.20-3.30 (2H, m), 3.30-3.60 (4H, m), 4.90-4.98 (1H, m), 7.34-7.41 (1H, m), 7.66-7.82 (2H, m), 8.36-8.39 (2H, m), 9.53 (1H, s)
MS (m/z): 240 (MH+)

Examples 3-2 to 3-320

The compounds of Examples 3-2 to 3-320 were obtained by using 3-(2,3-dihydro-1,5-diazaphenalen-1-yl) pyrrolidine obtained in Example 3-1 according to the methods described in Example 1-2 to 1-320.

Example 3-321: 6-Chloro-1-(pyrrolidin-3-yl)-2,3-dihydro-1H-1,5-diazaphenalene

Step A

3-(6-Chloro-2,3-dihydro-1,5-diazaphenalen-1-yl)pyrrolidine-1-carboxylic acid tert-butyl ester (Intermediate 30)

By using 5-amino-1-chloro-4-vinylisoquinoline (Intermediate 8) obtained in Example 1-321, Step C, the title compound was obtained according to the method described in Example 1-321.
MS (m/z): 374 (MH+)

Step B

6-Chloro-1-(pyrrolidin-3-yl)-2,3-dihydro-1H-1,5-diazaphenalene

By using 3-(6-chloro-2,3-dihydro-1,5-diazaphenalen-1-yl)pyrrolidine-1-carboxylic acid tert-butyl ester (Intermediate 30) obtained in Step A mentioned above, the title compound was obtained as a hydrochloride according to the method described in Example 1-1, Step D.
MS (m/z): 274 (MH+)

Example 3-322

The compound of Example 3-322 was obtained in the same manner as that used for the compound of Example 1-322.

Example 3-323

The compound of Example 3-323 was obtained in the same manner as that used for the compound of Example 1-323.

Examples 3-324 to 3-331

The compounds of Examples 3-324 to 3-331 were obtained by using 3-(6-chloro-2,3-dihydro-1,5-diazaphenalen-1-yl)pyrrolidine-1-carboxylic acid tert-butyl ester (Intermediate 30) obtained in Example 3-321, Step A according to the methods described in Example 1-324 to 1-331.

Example 3-332 to 3-650

The compounds of Examples 3-332 to 3-650 were obtained by using 6-chloro-1-(pyrrolidin-3-yl)-2,3-dihydro-1H-1,5-diazaphenalene obtained in Example 3-321, or 6-hydroxy-1-(pyrrolidin-3-yl)-2,3-dihydro-1H-1,5-diazaphenalene obtained in Example 3-323 according to the methods described in Examples 1-2 to 1-320.

Example 3-651: 4-Chloro-1-(pyrrolidin-3-yl)-2,3-dihydro-1H-1,5-diazaphenalene

Step A

3-(4-Chloro-2,3-dihydro-1,5-diazaphenalen-1-yl)pyrrolidine-1-carboxylic acid tert-butyl ester (Intermediate 31)

By using 3-chloro-5-nitro-4-vinylisoquinoline (Intermediate 7) obtained in Example 1-321, Step C, the title compound was obtained according to the methods described in Example 1-321, Steps D to F.
MS (m/z): 374 (MH+)

Step B

4-Chloro-1-(pyrrolidin-3-yl)-2,3-dihydro-1H-1,5-diazaphenalene

By using 3-(4-chloro-2,3-dihydro-1,5-diazaphenalen-1-yl)pyrrolidine-1-carboxylic acid tert-butyl ester (Intermediate 31) obtained in Step C mentioned above, the title compound was obtained as a hydrochloride according to the method described in Example 1-1, Step D.
MS (m/z) : 274 (MH+)

Example 3-652

The compound of Example 3-652 was obtained according to the method described in Example 3-651.

Example 3-653

The compound of Example 3-653 was obtained according to Method A or Method B of Example 1-323,.

Examples 3-654 to 3-661

The compounds of Examples 3-654 to 3-661 were obtained by using 3-(6-chloro-2,3-dihydro-1,5-diazaphenalen-1-yl)pyrrolidine-1-carboxylic acid tert-butyl ester according to the methods described in Example 1-324 to 1-331.

Example 3-663: 7-Bromo-1-(pyrrolidin-3-yl)-2,3-dihydro-1H-1,5-diazaphenalene

Step A

3-(7-Bromo-2,3-dihydro-1,5-diazaphenalen-1-yl)pyrrolidine-1-carboxylic acid tert-butyl ester (Intermediate 33), and

3-(8-bromo-2,3-dihydro-1,5-diazaphenalen-1-yl)pyrrolidine-1-carboxylic acid tert-butyl ester (Intermediate 34)

By using 3-(2,3-dihydro-1,5-diazaphenalen-1-yl)pyrrolidine-1-carboxylic acid tert-butyl ester (Intermediate 29) obtained in Example 3-1, Step A, each of the title compounds was obtained according to the method described in Example 1-663, Step A. MS (m/z): 418 (MH+)

Step B

7-Bromo-1-(pyrrolidin-3-yl)-2,3-dihydro-1H-1,5-diazaphenalene

By using 3-(7-bromo-2,3-dihydro-1,5-diazaphenalen-1-yl)pyrrolidine-1-carboxylic acid tert-butyl ester (Intermediate 32) obtained in Step A mentioned above, the title compound was obtained as a hydrochloride according to the method described in Example 1-1, Step D.
MS (m/z) : 318 (MH+)

Examples 3-664 to 3-672

The compounds of Examples 3-664 to 3-672 were obtained by using 3-(7-bromo-2,3-dihydro-1,5-diazaphenalen-1-yl)pyrrolidine-1-carboxylic acid tert-butyl ester obtained in Example 3-663, Step A according to the methods described in Examples 1-323 to 1-331.

Example 3-673

The compound of Example 3-673 was obtained according to the method described in Example 3-674 mentioned below.

Example 3-674: 8-Bromo-1-(pyrrolidin-3-yl)-2,3-dihydro-1H-1,5-diazaphenalene

By using 3-(8-bromo-2,3-dihydro-1,5-diazaphenalen-1-yl)pyrrolidine-1-carboxylic acid tert-butyl ester (Intermediate 33) obtained in Example 3-663, Step A, the title compound was obtained as a hydrochloride according to the method described in Example 1-1, Step D.
MS (m/z): 319 (MH+)

Examples 3-675 to 3-683

The compounds of Examples 3-675 to 3-683 were obtained by using 3-(8-bromo-2,3-dihydro-1,5-diazaphenalen-1-yl)pyrrolidine-1-carboxylic acid tert-butyl ester obtained in Example 3-663, Step A according to the methods described in Examples 1-323 to 1-331.

Examples 3-684 and 3-685

The compounds of Examples 3-684 and 3-685 were obtained from the compound of Example 3-691 mentioned below by referring to a known diazotization-Sandmeyer reaction (for example, Denny, William , et al, J. Med. Chem., 2002, 740; Kulka, J. Am. Chem. Soc., 75, 3597 (1953) and the like).

Examples 3-686 to 3-690

The compounds of Examples 3-686 to 3-690 were obtained by using 3-(9-bromo-2,3-dihydro-1,5-diazaphenalen-1-yl)pyrrolidine-1-carboxylic acid tert-butyl ester obtained in Example 3-685 according to the methods described in Examples 1-323 to 1-327.

Example 3-691: 9-Amino-1-(pyrrolidin-3-yl)-2,3-dihydro-1H-1,5-diazaphenalene

Step A

3-(9-Amino-2,3-dihydro-1,5-diazaphenalen-1-yl)pyrrolidine-1-carboxylic acid tert-butyl ester (Intermediate 34)

By using 3-(7-bromo-2,3-dihydro-1,5-diazaphenalen-1-yl)pyrrolidine-1-carboxylic acid tert-butyl ester (Intermediate 32) obtained in Example 3-663, Step A, nitration (9-position), and reduction (bromine atom→hydrogen atom at the 7-position, nitro group→amino group at the 9-position) were performed with referring to a known publication (for example, Kucznierz, R., Dickhaut, J, Leinert, H., Von Der Saal, W., Synth. Commun., 29, 1617 (1999) ; Ping Chen, Bioorganic & Medicinal Chemistry Letters, 13, 1345 (2003) and the like) to obtain the title compound.
MS (m/z): 355 (MH+)

Step B

9-Amino-1-(pyrrolidin-3-yl)-2,3-dihydro-1H-1,5-diazaphenalene

By using 3-(9-amino-2,3-dihydro-1,5-diazaphenalen-1-yl)pyrrolidine-1-carboxylic acid tert-butyl ester (Intermediate 34) obtained in Step A mentioned above, the title compound was obtained as a hydrochloride according to the method described in Example 1-1, Step D.
MS (m/z): 255 (MH+)

Examples 3-692 to 3-694

The compounds of Examples 3-692 to 3-694 were obtained by using 3-(9-bromo-2,3-dihydro-1,5-diazaphenalen-1-yl)pyrrolidine-1-carboxylic acid tert-butyl ester obtained in Example 3-685 according to the methods described in Examples 1-329 to 1-331.
[Table 3]

| Exp. No. | $R^1$ | $R^5$ | $R^8$ | $R^7$ | $R^6$ | $A^{61}$ | Mass($MH^+$) |
|---|---|---|---|---|---|---|---|
| 3-1 | H | H | H | H | H | H | 240 |
| 3-2 | H | H | H | H | H | Me | 254 |
| 3-3 | H | H | H | H | H | a-1 | 322 |
| 3-4 | H | H | H | H | H | a-2 | 360 |
| 3-5 | H | H | H | H | H | $C_6H_5$ | 316 |
| 3-6 | H | H | H | H | H | $CH_2COOH$ | 298 |
| 3-7 | H | H | H | H | H | $CH_2CH_2COOH$ | 312 |
| 3-8 | H | H | H | H | H | $CH_2CN$ | 279 |
| 3-9 | H | H | H | H | H | $CH_2CH_2CN$ | 293 |
| 3-10 | H | H | H | H | H | $CH_2CH_2OH$ | 284 |
| 3-11 | H | H | H | H | H | $CH(CH_2OH)_2$ | 314 |
| 3-12 | H | H | H | H | H | a-3 | 338 |
| 3-13 | H | H | H | H | H | $CH_2CH_2OMe$ | 298 |
| 3-14 | H | H | H | H | H | $CH_2CH_2OCOCMe_3$ | 368 |
| 3-15 | H | H | H | H | H | $CH_2CH_2SCOMe$ | 342 |
| 3-16 | H | H | H | H | H | $CH_2CH_2SH$ | 300 |
| 3-17 | H | H | H | H | H | $CH_2CH_2NH_2$ | 283 |
| 3-18 | H | H | H | H | H | $CH_2CH_2CH_2NH_2$ | 297 |
| 3-19 | H | H | H | H | H | $CH_2CH_2CH_2NHMe$ | 311 |
| 3-20 | H | H | H | H | H | $CH(CH_2NHCOMe)_2$ | 396 |
| 3=21 | H | H | H | H | H | $CH\ (CH_2NH_2)_2$ | 312 |
| 3-22 | H | H | H | H | H | $CH_2CH_2NHCOMe$ | 325 |
| 3-23 | H | H | H | H | H | a-4 | 338 |
| 3-24 | H | H | H | H | H | $CH_2CH_2NHCOCH_2NHCOMe$ | 382 |
| 3-25 | H | H | H | H | H | $CH_2CH_2NHCOCH_2NMe_2$ | 368 |
| 3-26 | H | H | H | H | H | $CH_2CH_2NHSO_2Me$ | 361 |
| 3-27 | H | H | H | H | H | a-5 | 337 |
| 3-28 | H | H | H | H | H | a-6 | 379 |
| 3-29 | H | H | H | H | H | $CH_2CH_2CH_2NHCONHCH_2CH_2OH$ | 384 |
| 3-30 | H | H | H | H | H | $CH_2CH_2CH_2NHCONHBn$ | 430 |
| 3-31 | H | H | H | H | H | $CH_2CH_2NHCSNHCH_2CH_2OH$ | 386 |
| 3-32 | H | H | H | H | H | $CH_2CH_2NHCSNHC_6H_5$ | 418 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-33 | H | H | H | H | H | $CH_2CH_2NHCOOCH_2CH_2NMe_2$ | 398 |
| 3-34 | H | H | H | H | H | a-7 | 395 |
| 3-35 | H | H | H | H | H | a-8 | 282 |
| 3-36 | H | H | H | H | H | a-9 | 337 |
| 3-37 | H | H | H | H | H | COMe | 282 |
| 3-38 | H | H | H | H | H | a-10 | 294 |
| 3-39 | H | H | H | H | H | a-11 | 308 |
| 3-40 | H | H | H | H | H | a-12 | 322 |
| 3-41 | H | H | H | H | H | Bn | 330 |
| 3-42 | H | H | H | H | H | a-13 | 364 |
| 3-43 | H | H | H | H | H | a-14 | 346 |
| 3-44 | H | H | H | H | H | a-15 | 390 |
| 3-45 | H | H | H | H | H | a-16 | 404 |
| 3-46 | H | H | H | H | H | a-17 | 420 |
| 3-47 | H | H | H | H | H | a-18 | 397 |
| 3-48 | H | H | H | H | H | a-19 | 415 |
| 3-49 | H | H | H | H | H | a-20 | 406 |
| 3-50 | H | H | H | H | H | a-21 | 345 |
| 3-51 | H | H | H | H | H | a-22 | 359 |
| 3-52 | H | H | H | H | H | a-23 | 373 |
| 3-53 | H | H | H | H | H | a-24 | 389 |
| 3-54 | H | H | H | H | H | a-25 | 403 |
| 3-55 | H | H | H | H | H | a-26 | 433 |
| 3-56 | H | H | H | H | H | a-27 | 387 |
| 3-57 | H | H | H | H | H | a-28 | 423 |
| 3-58 | H | H | H | H | H | a-29 | 432 |
| 3-59 | H | H | H | H | H | a-30 | 374 |
| 3-60 | H | H | H | H | H | a-31 | 390 |
| 3-61 | H | H | H | H | H | a-32 | 360 |
| 3-62 | H | H | H | H | H | a-33 | 360 |
| 3-63 | H | H | H | H | H | a-34 | 390 |
| 3-64 | H | H | H | H | H | a-35 | 390 |
| 3-65 | H | H | H | H | H | a-36 | 420 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-66 | H | H | H | H | H | a-37 | 420 |
| 3-67 | H | H | H | H | H | a-38 | 318 |
| 3-68 | H | H | H | H | H | a-39 | 320 |
| 3-69 | H | H | H | H | H | a-40 | 336 |
| 3-70 | H | H | H | H | H | a-41 | 388 |
| 3-71 | H | H | H | H | H | a-42 | 375 |
| 3-72 | H | H | H | H | H | a-43 | 331 |
| 3-73 | H | H | H | H | H | a-44 | 317 |
| 3-74 | H | H | H | H | H | a-45 | 332 |
| 3-75 | H | H | H | H | H | $CH_2CH_2CH_2COOH$ | 326 |
| 3-76 | H | H | H | H | H | $CH_2CH_2CH_2CH_2COOH$ | 340 |
| 3-77 | H | H | H | H | H | $CH_2CH_2CH_2CN$ | 307 |
| 3-78 | H | H | H | H | H | $CH_2CH_2CH_2CH_2CN$ | 321 |
| 3-79 | H | H | H | H | H | $CH_2CH$ (Me) OH | 298 |
| 3-80 | H | H | H | H | H | CH(Me)$CH_2OH$ | 298 |
| 3-81 | H | H | H | H | H | $CH_2CH_2CH_2OH$ | 298 |
| 3-82 | H | H | H | H | H | $CH_2CH$ (OH) $CH_2OH$ | 314 |
| 3-83 | H | H | H | H | H | $CH_2CH$ ($NH_2$) $CH_2OH$ | 313 |
| 3-84 | H | H | H | H | H | $CH_2CH$ (NHMe) $CH_2OH$ | 327 |
| 3-85 | H | H | H | H | H | $CH_2CH$ ($NMe_2$) $CH_2OH$ | 341 |
| 3-86 | H | H | H | H | H | $CH_2CH$ (Me) $CH_2OH$ | 312 |
| 3-87 | H | H | H | H | H | $CH_2CH_2CH_2CH_2OH$ | 312 |
| 3-88 | H | H | H | H | H | $CH_2CH_2CH_2CH_2CH_2OH$ | 326 |
| 3-89 | H | H | H | H | H | a-46 | 452 |
| 3-90 | H | H | H | H | H | CH ($CH_2OH$) $CH_2CH_2NHCONHC_6H_5$ | 446 |
| 3-91 | H | H | H | H | H | a-47 | 438 |
| 3-92 | H | H | H | H | H | $CH_2CH$ (OH) $CH_2NHCONHC_6H_5$ | 432 |
| 3-93 | H | H | H | H | H | $CH_2CH_2OCONHMe$ | 341 |
| 3-94 | H | H | H | H | H | $CH_2CH_2OCONHC_6H_5$ | 403 |
| 3-95 | H | H | H | H | H | $CH_2CH_2CH_2OCONHMe$ | 355 |
| 3-96 | H | H | H | H | H | a-48 | 423 |
| 3-97 | H | H | H | H | H | $CH_2CH_2CH_2OCONHC_6H_5$ | 417 |
| 3-98 | H | H | H | H | H | $CH_2CH_2CH_2CH_2OCONHMe$ | 369 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-99 | H | H | H | H | H | a-49 | 437 |
| 3-100 | H | H | H | H | H | $CH_2CH_2CH_2CH_2OCONHC_6H_5$ | 431 |
| 3-101 | H | H | H | H | H | $CH_2CH_2CH_2SH$ | 314 |
| 3-102 | H | H | H | H | H | $CH_2CH_2CH_2CH_2SH$ | 328 |
| 3-103 | H | H | H | H | H | $CH_2CH_2SCOC_6H_5$ | 404 |
| 3-104 | H | H | H | H | H | $CH_2CH_2CH_2SCOMe$ | 356 |
| 3-105 | H | H | H | H | H | $CH_2CH_2CH_2SCOC_6H_5$ | 418 |
| 3-106 | H | H | H | H | H | $CH_2CH_2CH_2OMe$ | 312 |
| 3-107 | H | H | H | H | H | $CH_2CH_2CH_2CH_2OMe$ | 326 |
| 3-108 | H | H | H | H | H | $CH_2CH_2CH_2CH_2CH_2OMe$ | 340 |
| 3-109 | H | H | H | H | H | $CH_2CH_2OCOMe$ | 326 |
| 3-110 | H | H | H | H | H | $CH_2CH_2CH_2OCOMe$ | 340 |
| 3-111 | H | H | H | H | H | $CH_2CH_2CH_2CH_2OCOMe$ | 354 |
| 3-112 | H | H | H | H | H | $CH_2CH_2OCOCH_2Me$ | 340 |
| 3-113 | H | H | H | H | H | $CH_2CH_2CH_2OCOCH_2Me$ | 354 |
| 3-114 | H | H | H | H | H | $CH_2CH_2CH_2CH_2OCOCH_2Me$ | 368 |
| 3-115 | H | H | H | H | H | $CH_2CH_2OCOCHMe_2$ | 354 |
| 3-116 | H | H | H | H | H | $CH_2CH_2CH_2OCOCHMe_2$ | 368 |
| 3-117 | H | H | H | H | H | $CH_2CH_2CH_2CH_2OCOCHMe_2$ | 382 |
| 3-118 | H | H | H | H | H | $CH_2CH_2CH_2OCOCMe_3$ | 382 |
| 3-119 | H | H | H | H | H | $CH_2CH_2CH_2CH_2OCOCMe_3$ | 396 |
| 3-120 | H | H | H | H | H | $CH_2CH_2OCOC_6H_5$ | 388 |
| 3-121 | H | H | H | H | H | $CH_2CH_2CH_2OCOC_6H_5$ | 402 |
| 3-122 | H | H | H | H | H | $CH_2CH_2CH_2CH_2OCOC_6H_5$ | 416 |
| 3-123 | H | H | H | H | H | $CH_2CH_2OCOCH_2OH$ | 342 |
| 3-124 | H | H | H | H | H | $CH_2CH_2OCOCH_2NH_2$ | 341 |
| 3-125 | H | H | H | H | H | $CH_2CH_2OCH_2CH_2OMe$ | 342 |
| 3-126 | H | H | H | H | H | $CH_2CH_2OCH_2CH_2OH$ | 328 |
| 3-127 | H | H | H | H | H | $CH_2CH_2CH_2OCH_2CH_2OH$ | 342 |
| 3-128 | H | H | H | H | H | $CH_2CH_2CH_2CH_2OCH_2CH_2OH$ | 356 |
| 3-129 | H | H | H | H | H | $CH_2CH(CH_2OH)NH_2$ | 313 |
| 3-130 | H | H | H | H | H | $CH_2CH(CH_2OH)NHCOMe$ | 355 |
| 3-131 | H | H | H | H | H | a-50 | 438 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-132 | H | H | H | H | H | $CH_2CH(CH_2OH)NHCONHC_6H_5$ | 432 |
| 3-133 | H | H | H | H | H | $CH(CH_2OH)CH_2NH_2$ | 313 |
| 3-134 | H | H | H | H | H | $CH(CH_2OH)CH_2NHCOMe$ | 355 |
| 3-135 | H | H | H | H | H | a-51 | 438 |
| 3-136 | H | H | H | H | H | CH ($CH_2OH$) $CH_2NHCONHC_6H_5$ | 432 |
| 3-137 | H | H | H | H | H | $CH_2CH$ (OH) $CH_2NH_2$ | 313 |
| 3-138 | H | H | H | H | H | $CH_2CH(OH)CH_2NHMe$ | 327 |
| 3-139 | H | H | H | H | H | $CH_2CH$ (OH) $CH_2NMe_2$ | 341 |
| 3-140 | H | H | H | H | H | $CH_2CH_2CH_2CH_2NH_2$ | 311 |
| 3-141 | H | H | H | H | H | $CH_2CH_2CH_2CH_2CH_2NH_2$ | 325 |
| 3-142 | H | H | H | H | H | $CH_2CH_2NHMe$ | 297 |
| 3-143 | H | H | H | H | H | $CH_2CH_2CH_2CH_2NHMe$ | 325 |
| 3-144 | H | H | H | H | H | $CH_2CH_2CH_2CH_2CH_2NHMe$ | 339 |
| 3-145 | H | H | H | H | H | $CH_2CH_2NMe_2$ | 311 |
| 3-146 | H | H | H | H | H | $CH_2CH_2CH_2NMe_2$ | 325 |
| 3-147 | H | H | H | H | H | $CH_2CH_2CH_2CH_2NMe_2$ | 339 |
| 3-148 | H | H | H | H | H | $CH_2CH_2CH_2CH_2CH_2NMe_2$ | 353 |
| 3-149 | H | H | H | H | H | $CH_2CH_2NHCH_2CH_2OH$ | 327 |
| 3-150 | H | H | H | H | H | $CH_2CH_2CH_2NHCH_2CH_2OH$ | 341 |
| 3-151 | H | H | H | H | H | $CH_2CH_2CH_2CH_2NHCH_2CH_2OH$ | 355 |
| 3-152 | H | H | H | H | H | $CH_2CONH_2$ | 297 |
| 3-153 | H | H | H | H | H | $CH_2CH_2CONH_2$ | 311 |
| 3-154 | H | H | H | H | H | $CH_2CH_2CH_2CONH_2$ | 325 |
| 3-155 | H | H | H | H | H | $CH_2CH_2NHCOCH_2Me$ | 339 |
| 3-156 | H | H | H | H | H | $CH_2CH_2NHCOCH_2CH_2Me$ | 353 |
| 3-157 | H | H | H | H | H | $CH_2CH_2NHCOCHMe_2$ | 353 |
| 3-158 | H | H | H | H | H | $CH_2CH_2NHCOCMe_3$ | 367 |
| 3-159 | H | H | H | H | H | $CH_2CH_2NHCOCH_2OH$ | 341 |
| 3-160 | H | H | H | H | H | $CH_2CH_2NHCOCH_2OMe$ | 355 |
| 3-161 | H | H | H | H | H | $CH_2CH_2NHCOC_6H_5$ | 387 |
| 3-162 | H | H | H | H | H | a-52 | 388 |
| 3-163 | H | H | H | H | H | a-53 | 393 |
| 3-164 | H | H | H | H | H | a-54 | 377 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-165 | H | H | H | H | H | a-55 | 389 |
| 3-166 | H | H | H | H | H | $CH_2CH_2NHCOCH_2NHCOCH_2CHMe_2$ | 424 |
| 3-167 | H | H | H | H | H | $CH_2CH_2NHCOCH_2NHCOC_2H_2$ | 444 |
| 3-168 | H | H | H | H | H | $CH_2CH_2CH_2NHCOMe$ | 339 |
| 3-169 | H | H | H | H | H | $CH_2CH_2CH_2NHCOCH_2Me$ | 353 |
| 3-170 | H | H | H | H | H | $CH_2CH_2CH_2NHCOCH_2CH_2Me$ | 367 |
| 3-171 | H | H | H | H | H | $CH_2CH_2CH_2NHCOCHMe_2$ | 367 |
| 3-172 | H | H | H | H | H | $CH_2CH_2CH_2NHCOCMe_3$ | 381 |
| 3-173 | H | H | H | H | H | $CH_2CH_2CH_2CH_2NHCOMe$ | 353 |
| 3-174 | H | H | H | H | H | $CH_2CH_2CH_2CH_2NHCOCH_2Me$ | 367 |
| 3-175 | H | H | H | H | H | $CH_2CH_2CH_2CH_2NHCOCH_2CH_2Me$ | 381 |
| 3-176 | H | H | H | H | H | $CH_2CH_2CH_2CH_2NHCOCHMe_2$ | 381 |
| 3-177 | H | H | H | H | H | $CH_2CH_2CH_2CH_2NHCOCMe_3$ | 395 |
| 3-178 | H | H | H | H | H | $CH_2CH_2N$ (Me) COMe | 339 |
| 3-179 | H | H | H | H | H | $CH_2CH_2N$ (Me) $COC_6H_5$ | 401 |
| 3-180 | H | H | H | H | H | $CH_2CH_2CH_2N$ (Me) COMe | 353 |
| 3-181 | H | H | H | H | H | $CH_2CH_2CH_2N(Me)COC_6H_5$ | 415 |
| 3-182 | H | H | H | H | H | $CH_2CH_2CH_2CH_2N$ (Me) COMe | 367 |
| 3-183 | H | H | H | H | H | $CH_2CH_2N(CH_2CH_2OH)COMe$ | 369 |
| 3-184 | H | H | H | H | H | $CH_2CH_2CH_2N$ ($CH_2CH_2OH$) COMe | 383 |
| 3-185 | H | H | H | H | H | $CH_2CH_2N$ ($CH_2CN$) COMe | 364 |
| 3-186 | H | H | H | H | H | $CH_2CH_2CH_2N(CH_2CN)COMe$ | 378 |
| 3-187 | H | H | H | H | H | a-56 | 310 |
| 3-188 | H | H | H | H | H | a-57 | 309 |
| 3-189 | H | H | H | H | H | a-58 | 351 |
| 3-190 | H | H | H | H | H | a-59 | 413 |
| 3-191 | H | H | H | H | H | a-60 | 441 |
| 3-192 | H | H | H | H | H | a-61 | 324 |
| 3-193 | H | H | H | H | H | a-62 | 337 |
| 3-194 | H | H | H | H | H | a-63 | 323 |
| 3-195 | H | H | H | H | H | $CH_2CH_2NHSO_2C_6H_5$ | 423 |
| 3-196 | H | H | H | H | H | $CH_2CH_2NHSO_2NMe_2$ | 390 |
| 3-197 | H | H | H | H | H | $CH_2CH_2N$ (Me) $SO_2Me$ | 375 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-198 | H | H | H | H | H | $CH_2CH_2N$ (Me) $SO_2C_6H_5$ | 437 |
| 3-199 | H | H | H | H | H | $CH_2CH_2N$ (Me) $SO_2NMe_2$ | 404 |
| 3-200 | H | H | H | H | H | $CH_2CH_2N$ ($CH_2CH_2OH$) $SO_2Me$ | 391 |
| 3-201 | H | H | H | H | H | $CH_2CH_2CH_2NHSO_2Me$ | 375 |
| 3-202 | H | H | H | H | H | $CH_2CH_2CH_2NHSO_2C_6H_5$ | 437 |
| 3-203 | H | H | H | H | H | $CH_2CH_2CH_2NHSO_2NMe_2$ | 404 |
| 3-204 | H | H | H | H | H | $CH_2CH_2CH_2N$ (Me) $SO_2Me$ | 389 |
| 3-205 | H | H | H | H | H | $CH_2CH_2CH_2N$ (Me) $SO_2C_6H_5$ | 451 |
| 3-206 | H | H | H | H | H | $CH_2CH_2CH_2N$ (Me) $SO_2NMe_2$ | 418 |
| 3-207 | H | H | H | H | H | $CH_2CH_2CH_2N$ ($CH_2CH_2OH$) $SO_2Me$ | 405 |
| 3-208 | H | H | H | H | H | $CH_2CH_2CH_2CH_2NHSO_2Me$ | 389 |
| 3-209 | H | H | H | H | H | $CH_2CH_2CH_2CH_2NHSO_2C_6H_5$ | 451 |
| 3-210 | H | H | H | H | H | $CH_2CH_2CH_2CH_2NHSO_2NMe_2$ | 418 |
| 3-211 | H | H | H | H | H | $CH_2CH_2CH_2CH_2N$ (Me) $SO_2Me$ | 403 |
| 3-212 | H | H | H | H | H | $CH_2CH_2CH_2CH_2N(Me)SO_2C_6H_5$ | 465 |
| 3-213 | H | H | H | H | H | $CH_2CH_2CH_2CH_2N$ (Me) $SO_2NMe_2$ | 432 |
| 3-214 | H | H | H | H | H | $CH_2CH_2CH_2CH_2N$ ($CH_2CH_2OH$) $SO_2Me$ | 419 |
| 3-215 | H | H | H | H | H | a-64 | 387 |
| 3-216 | H | H | H | H | H | a-65 | 351 |
| 3-217 | H | H | H | H | H | a-66 | 415 |
| 3-218 | H | H | H | H | H | a-67 | 365 |
| 3-219 | H | H | H | H | H | $CH_2CH_2NHCONHMe$ | 340 |
| 3-220 | H | H | H | H | H | $CH_2CH_2NHCONHCH_2Me$ | 354 |
| 3-221 | H | H | H | H | H | $CH_2CH_2NHCONHCH_2CH_2Me$ | 368 |
| 3-222 | H | H | H | H | H | $CH_2CH_2NHCONHCHMe_2$ | 368 |
| 3-223 | H | H | H | H | H | $CH_2CH_2NHCONHCMe_3$ | 382 |
| 3-224 | H | H | H | H | H | a-68 | 394 |
| 3-225 | H | H | H | H | H | a-69 | 408 |
| 3-226 | H | H | H | H | H | a-70 | 422 |
| 3-227 | H | H | H | H | H | a-71 | 436 |
| 3-228 | H | H | H | H | H | a-72 | 460 |
| 3-229 | H | H | H | H | H | $CH_2CH_2NHCONHC_6H_5$ | 402 |
| 3-230 | H | H | H | H | H | a-73 | 432 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-231 | H | H | H | H | H | a-74 | 418 |
| 3-232 | H | H | H | H | H | a-75 | 417 |
| 3-233 | H | H | H | H | H | a-76 | 445 |
| 3-234 | H | H | H | H | H | a-77 | 452 |
| 3-235 | H | H | H | H | H | a-78 | 452 |
| 3-236 | H | H | H | H | H | a-79 | 427 |
| 3-237 | H | H | H | H | H | a-80 | 431 |
| 3-238 | H | H | H | H | H | a-81 | 436 |
| 3-239 | H | H | H | H | H | $CH_2CH_2NHCONHCOC_6H_5$ | 430 |
| 3-240 | H | H | H | H | H | $CH_2CH_2NHCONHSO_2C_6H_5$ | 466 |
| 3-241 | H | H | H | H | H | a-82 | 344 |
| 3-242 | H | H | H | H | H | a-83 | 344 |
| 3-243 | H | H | H | H | H | a-84 | 459 |
| 3-244 | H | H | H | H | H | a-85 | 446 |
| 3-245 | H | H | H | H | H | a-86 | 458 |
| 3-246 | H | H | H | H | H | a-87 | 408 |
| 3-247 | H | H | H | H | H | $CH_2CH_2NHCONHCMe_2CH_2CMe_3$ | 438 |
| 3-248 | H | H | H | H | H | a-88 | 421 |
| 3-249 | H | H | H | H | H | $CH_2CH_2N$ (Me) $CONHC_6H_5$ | 416 |
| 3-250 | H | H | H | H | H | $CH_2CH_2N$ (Me) CON (Me) $C_6H_5$ | 430 |
| 3-251 | H | H | H | H | H | $CH_2CH_2NHCONHCH_2CH_2OH$ | 370 |
| 3-252 | H | H | H | H | H | $CH_2CH_2NHCONHCH_2CH_2OMe$ | 384 |
| 3-253 | H | H | H | H | H | $CH_2CH_2NHCONHCH_2CH_2NMe_2$ | 397 |
| 3-254 | H | H | H | H | H | $CH_2CH_2NHCON(Me)CH_2CH_2OH$ | 384 |
| 3-255 | H | H | H | H | H | $CH_2CH_2NHCON$ $(CH_2CH_2OH)_2$ | 414 |
| 3-256 | H | H | H | H | H | a-89 | 395 |
| 3-257 | H | H | H | H | H | $CH_2CH_2NHCONMe_2$ | 354 |
| 3-258 | H | H | H | H | H | $CH_2CH_2CH_2NHCONHMe$ | 354 |
| 3-259 | H | H | H | H | H | $CH_2CH_2CH_2NHCONHCH_2Me$ | 368 |
| 3-260 | H | H | H | H | H | $CH_2CH_2CH_2NHCONHCH_2CH_2Me$ | 382 |
| 3-261 | H | H | H | H | H | $CH_2CH_2CH_2NHCONHCHMe_2$ | 382 |
| 3-262 | H | H | H | H | H | $CH_2CH_2CH_2NHCONHCMe_3$ | 396 |
| 3-263 | H | H | H | H | H | a-90 | 408 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-264 | H | H | H | H | H | a-91 | 422 |
| 3-265 | H | H | H | H | H | a-92 | 436 |
| 3-266 | H | H | H | H | H | a-93 | 450 |
| 3-267 | H | H | H | H | H | a-94 | 474 |
| 3-268 | H | H | H | H | H | $CH_2CH_2CH_2NHCONHC_6H_5$ | 416 |
| 3-269 | H | H | H | H | H | a-95 | 446 |
| 3-270 | H | H | H | H | H | a-96 | 432 |
| 3-271 | H | H | H | H | H | a-97 | 431 |
| 3-272 | H | H | H | H | H | a-98 | 459 |
| 3-273 | H | H | H | H | H | a-99 | 466 |
| 3-274 | H | H | H | H | H | a-100 | 466 |
| 3-275 | H | H | H | H | H | a-101 | 441 |
| 3-276 | H | H | H | H | H | a-102 | 445 |
| 3-277 | H | H | H | H | H | a-103 | 450 |
| 3-278 | H | H | H | H | H | $CH_2CH_2CH_2NHCONHCOC_6H_5$ | 444 |
| 3-279 | H | H | H | H | H | a-104 | 492 |
| 3-280 | H | H | H | H | H | a-105 | 492 |
| 3-281 | H | H | H | H | H | a-106 | 473 |
| 3-282 | H | H | H | H | H | a-107 | 460 |
| 3-283 | H | H | H | H | H | a-108 | 472 |
| 3-284 | H | H | H | H | H | a-109 | 422 |
| 3-285 | H | H | H | H | H | $CH_2CH_2CH_2NHCONHCMe_2CH_2CMe_3$ | 452 |
| 3-286 | H | H | H | H | H | a-110 | 435 |
| 3-287 | H | H | H | H | H | $CH_2CH_2CH_2N$ (Me) $CONHC_6H_5$ | 430 |
| 3-288 | H | H | H | H | H | $CH_2CH_2CH_2N(Me)CON(Me)C_6H_5$ | 444 |
| 3-289 | H | H | H | H | H | $CH_2CH_2CH_2NHCONHCH_2CH_2OMe$ | 398 |
| 3-290 | H | H | H | H | H | $CH_2CH_2CH_2NHCONHCH_2CH_2NMe_2$ | 411 |
| 3-291 | H | H | H | H | H | $CH_2CH_2CH_2NHCON$ (Me) $CH_2CH_2OH$ | 398 |
| 3-292 | H | H | H | H | H | $CH_2CH_2CH_2NHCON$ ($CH_2CH_2OH$) | 428 |
| 3-293 | H | H | H | H | H | a-111 | 409 |
| 3-294 | H | H | H | H | H | $CH_2CH_2CH_2NHCONMe_2$ | 368 |
| 3-295 | H | H | H | H | H | $CH_2CH_2NHCSNHCH_2Me$ | 370 |
| 3-296 | H | H | H | H | H | $CH_2CH_2NHCSNHCH_2CH_2Me$ | 384 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-297 | H | H | H | H | H | $CH_2CH_2NHCSNHCHMe_2$ | 384 |
| 3-298 | H | H | H | H | H | $CH_2CH_2NHCSNHCMe_3$ | 398 |
| 3-299 | H | H | H | H | H | a-112 | 344 |
| 3-300 | H | H | H | H | H | $CH_2CH_2NHCSNHCH_2CH_2OMe$ | 400 |
| 3-301 | H | H | H | H | H | $CH_2CH_2NHCSNHCH_2CH_2NMe_2$ | 413 |
| 3-302 | H | H | H | H | H | $CH_2CH_2NHCSN(Me)CH_2CH_2OH$ | 400 |
| 3-303 | H | H | H | H | H | $CH_2CH_2NHCSN(CH_2CH_2OH)_2$ | 430 |
| 3-304 | H | H | H | H | H | a-113 | 411 |
| 3-305 | H | H | H | H | H | $CH_2CH_2NHCSNMe_2$ | 370 |
| 3-306 | H | H | H | H | H | $CH_2CH_2NHCOOMe$ | 341 |
| 3-307 | H | H | H | H | H | $CH_2CH_2NHCOOCH_2Me$ | 355 |
| 3-308 | H | H | H | H | H | $CH_2CH_2NHCOOCH_2CH_2Me$ | 369 |
| 3-309 | H | H | H | H | H | $CH_2CH_2NHCOOCHMe_2$ | 369 |
| 3-310 | H | H | H | H | H | a-114 | 409 |
| 3-311 | H | H | H | H | H | $CH_2CH_2NHCOOC_6H_5$ | 403 |
| 3-312 | H | H | H | H | H | $CH_2CH_2NHCOOCH_2CH_2OH$ | 371 |
| 3-313 | H | H | H | H | H | $CH_2CH_2NHCOOCH_2CH_2OMe$ | 385 |
| 3-314 | H | H | H | H | H | a-115 | 364 |
| 3-315 | H | H | H | H | H | a-116 | 364 |
| 3-316 | H | H | H | H | H | a-117 | 351 |
| 3-317 | H | H | H | H | H | a-118 | 365 |
| 3-318 | H | H | H | H | H | a-119 | 340 |
| 3-319 | H | H | H | H | H | a-120 | 365 |
| 3-320 | H | H | H | H | H | $COC_6H_5$ | 344 |
| 3-321 | Cl | H | H | H | H | H | 274 |
| 3-322 | Br | H | H | H | H | H | 318 |
| 3-323 | OH | H | H | H | H | H | 256 |
| 3-324 | Me | H | H | H | H | H | 254 |
| 3-325 | $CH=CH_2$ | H | H | H | H | H | 266 |
| 3-326 | $C\equiv CC_6H_5$ | H | H | H | H | H | 340 |
| 3-327 | OMe | H | H | H | H | H | 270 |
| 3-328 | $NH_2$ | H | H | H | H | H | 255 |
| 3-329 | NHMe | H | H | H | H | H | 269 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-330 | $NMe_2$ | H | H | H | H | H | 283 |
| 3-331 | $NHC_6H_5$ | H | H | H | H | H | 331 |
| 3-332 | OH | H | H | H | H | Me | 270 |
| 3-333 | OH | H | H | H | H | a-1 | 338 |
| 3-334 | OH | H | H | H | H | a-2 | 376 |
| 3-335 | OH | H | H | H | H | $C_6H_5$ | 332 |
| 3-336 | OH | H | H | H | H | $CH_2COOH$ | 314 |
| 3-337 | OH | H | H | H | H | $CH_2CH_2COOH$ | 328 |
| 3-338 | OH | H | H | H | H | $CH_2CN$ | 295 |
| 3-339 | OH | H | H | H | H | $CH_2CH_2CN$ | 309 |
| 3-340 | OH | H | H | H | H | $CH_2CH_2OH$ | 300 |
| 3-341 | OH | H | H | H | H | $CH\ (CH_2OH)\ _2$ | 330 |
| 3-342 | OH | H | H | H | H | a-3 | 354 |
| 3-343 | OH | H | H | H | H | $CH_2CH_2OMe$ | 314 |
| 3-344 | OH | H | H | H | H | $CH_2CH_2OCOCMe_3$ | 384 |
| 3-345 | 0H | H | H | H | H | $CH_2CH_2SCOMe$ | 358 |
| 3-346 | OH | H | H | H | H | $CH_2CH_2SH$ | 316 |
| 3-347 | OH | H | H | H | H | $CH_2CH_2NH_2$ | 299 |
| 3-348 | OH | H | H | H | H | $CH_2CH_2CH_2NH_2$ | 313 |
| 3-349 | OH | H | H | H | H | $CH_2CH_2CH_2NHMe$ | 327 |
| 3-350 | OH | H | H | H | H | $CH(CH_2NHCOMe)_2$ | 412 |
| 3-351 | OH | H | H | H | H | $CH(CH_2NH_2)_2$ | 328 |
| 3-352 | OH | H | H | H | H | $CH_2CH_2NHCOMe$ | 341 |
| 3-353 | OH | H | H | H | H | a-4 | 354 |
| 3-354 | OH | H | H | H | H | $CH_2CH_2NHCOCH_2NHCOMe$ | 398 |
| 3-355 | OH | H | H | H | H | $CH_2CH_2NHCOCH_2NMe_2$ | 384 |
| 3-356 | OH | H | H | H | H | $CH_2CH_2NHSO_2Me$ | 377 |
| 3-357 | OH | H | H | H | H | a-5 | 353 |
| 3-358 | OH | H | H | H | H | a-6 | 395 |
| 3-359 | OH | H | H | H | H | $CH_2CH_2CH_2NHCONHCH_2CHOH$ | 400 |
| 3-360 | OH | H | H | H | H | $CH_2CH_2CH_2NHCONHBn$ | 446 |
| 3-361 | OH | H | H | H | H | $CH_2CH_2NHCSNHCH_2CH_2OH$ | 402 |
| 3-362 | OH | H | H | H | H | $CH_2CH_2NHCSNHC_6H_5$ | 434 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-363 | OH | H | H | H | H | $CH_2CH_2NHCOOCH_2CH_2NMe_2$ | 414 |
| 3-364 | OH | H | H | H | H | a-7 | 411 |
| 3-365 | OH | H | H | H | H | a-8 | 298 |
| 3-366 | OH | H | H | H | H | a-9 | 353 |
| 3-367 | OH | H | H | H | H | COMe | 298 |
| 3-368 | OH | H | H | H | H | a-10 | 310 |
| 3-369 | OH | H | H | H | H | a-11 | 324 |
| 3-370 | OH | H | H | H | H | a-12 | 338 |
| 3-371 | OH | H | H | H | H | Bn | 346 |
| 3-372 | OH | H | H | H | H | a-13 | 380 |
| 3-373 | OH | H | H | H | H | a-14 | 362 |
| 3-374 | OH | H | H | H | H | a-15 | 406 |
| 3-375 | OH | H | H | H | H | a-16 | 420 |
| 3-376 | OH | H | H | H | H | a-17 | 436 |
| 3-377 | OH | H | H | H | H | a-18 | 413 |
| 3-378 | OH | H | H | H | H | a-19 | 431 |
| 3-379 | OH | H | H | H | H | a-20 | 422 |
| 3-380 | OH | H | H | H | H | a-21 | 361 |
| 3-381 | OH | H | H | H | H | a-22 | 375 |
| 3-382 | OH | H | H | H | H | a-23 | 389 |
| 3-383 | OH | H | H | H | H | a-24 | 405 |
| 3-384 | OH | H | H | H | H | a-25 | 419 |
| 3-385 | OH | H | H | H | H | a-26 | 449 |
| 3-386 | OH | H | H | H | H | a-27 | 403 |
| 3-387 | OH | H | H | H | H | a-28 | 439 |
| 3-388 | OH | H | H | H | H | a-29 | 448 |
| 3-389 | OH | H | H | H | H | a-30 | 390 |
| 3-390 | OH | H | H | H | H | a-31 | 406 |
| 3-391 | OH | H | H | H | H | a-32 | 376 |
| 3-392 | OH | H | H | H | H | a-33 | 376 |
| 3-393 | OH | H | H | H | H | a-34 | 406 |
| 3-394 | OH | H | H | H | H | a-35 | 406 |
| 3-395 | OH | H | H | H | H | a-36 | 436 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-396 | OH | H | H | H | H | a-37 | 436 |
| 3-397 | OH | H | H | H | H | a-38 | 334 |
| 3-398 | OH | H | H | H | H | a-39 | 336 |
| 3-399 | OH | H | H | H | H | a-40 | 352 |
| 3-400 | OH | H | H | H | H | a-41 | 404 |
| 3-401 | OH | H | H | H | H | a-42 | 391 |
| 3-402 | OH | H | H | H | H | a-43 | 347 |
| 3-403 | OH | H | H | H | H | a-44 | 333 |
| 3-404 | OH | H | H | H | H | a-45 | 348 |
| 3-405 | OH | H | H | H | H | $CH_2CH_2CH_2COOH$ | 342 |
| 3-406 | OH | H | H | H | H | $CH_2CH_2CH_2CH_2COOH$ | 356 |
| 3-407 | OH | H | H | H | H | $CH_2CH_2CH_2CN$ | 323 |
| 3-408 | OH | H | H | H | H | $CH_2CH_2CH_2CH_2CN$ | 337 |
| 3-409 | OH | H | H | H | H | $CH_2CH(Me) OH$ | 314 |
| 3-410 | OH | H | H | H | H | $CH (Me) CH_2OH$ | 314 |
| 3-411 | OH | H | H | H | H | $CH_2CH_2CH_2OH$ | 314 |
| 3-412 | OH | H | H | H | H | $CH_2CH(OH)CH_2OH$ | 330 |
| 3-413 | OH | H | H | H | H | $CH_2CH (NH_2) CH_2OH$ | 329 |
| 3-414 | OH | H | H | H | H | $CH_2CH(NHMe)CH_2OH$ | 343 |
| 3-415 | OH | H | H | H | H | $CH_2CH (NMe_2) CH_20H$ | 357 |
| 3-416 | OH | H | H | H | H | $CH_2CH(Me)CH_2OH$ | 328 |
| 3-417 | OH | H | H | H | H | $CH_2CH_2CH_2CH_2OH$ | 328 |
| 3-418 | OH | H | H | H | H | $CH_2CH_2CH_2CH_2CH_2OH$ | 342 |
| 3-419 | OH | H | H | H | H | a-46 | 468 |
| 3-420 | OH | H | H | H | H | $CH(CH_2OH)CH_2CH_2NHCONHC_6H_5$ | 462 |
| 3-421 | OH | H | H | H | H | a-47 | 454 |
| 3-422 | OH | H | H | H | H | $CH_2CH (OH) CH_2NHCONHC_6H_5$ | 448 |
| 3-423 | OH | H | H | H | H | $CH_2CH_2OCONHMe$ | 357 |
| 3-424 | OH | H | H | H | H | $CH_2CH_2OCONHC_6H_5$ | 419 |
| 3-425 | OH | H | H | H | H | $CH_2CH_2CH_2OCONHMe$ | 371 |
| 3-426 | OH | H | H | H | H | a-48 | 439 |
| 3-427 | OH | H | H | H | H | $CH_2CH_2CH_2OCONHC_6H_5$ | 433 |
| 3-428 | OH | H | H | H | H | $CH_2CH_2CH_2CH_2OCONHMe$ | 385 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-429 | OH | H | H | H | H | a-49 | 453 |
| 3-430 | OH | H | H | H | H | $CH_2CH_2CH_2CH_2OCONHC_6H_5$ | 447 |
| 3-431 | OH | H | H | H | H | $CH_2CH_2CH_2SH$ | 330 |
| 3-432 | OH | H | H | H | H | $CH_2CH_2CH_2CH_2SH$ | 344 |
| 3-433 | OH | H | H | H | H | $CH_2CH_2SCOC_6H_5$ | 420 |
| 3-434 | OH | H | H | H | H | $CH_2CH_2CH_2SCOMe$ | 372 |
| 3-435 | OH | H | H | H | H | $CH_2CH_2CH_2SCOC_6H_5$ | 434 |
| 3-436 | OH | H | H | H | H | $CH_2CH_2CH_2OMe$ | 328 |
| 3-437 | OH | H | H | H | H | $CH_2CH_2CH_2CH_2OMe$ | 342 |
| 3-438 | OH | H | H | H | H | $CH_2CH_2CH_2CH_2CH_2OMe$ | 356 |
| 3-439 | OH | H | H | H | H | $CH_2CH_2OCOMe$ | 342 |
| 3-440 | OH | H | H | H | H | $CH_2CH_2CH_2OCOMe$ | 356 |
| 3-441 | OH | H | H | H | H | $CH_2CH_2CH_2CH_2OCOMe$ | 370 |
| 3-442 | OH | H | H | H | H | $CH_2CH_2OCOCH_2Me$ | 356 |
| 3-443 | OH | H | H | H | H | $CH_2CH_2CH_2OCOCH_2Me$ | 370 |
| 3-444 | OH | H | H | H | H | $CH_2CH_2CH_2CH_2OCOCH_2Me$ | 384 |
| 3-445 | OH | H | H | H | H | $CH_2CH_2OCOCHMe_2$ | 370 |
| 3-446 | OH | H | H | H | H | $CH_2CH_2CH_2OCOCHMe_2$ | 384 |
| 3-447 | OH | H | H | H | H | $CH_2CH_2CH_2CH_2OCOCHMe_2$ | 398 |
| 3-448 | OH | H | H | H | H | $CH_2CH_2CH_2OCOCMe_3$ | 398 |
| 3-449 | OH | H | H | H | H | $CH_2CH_2CH_2CH_2OCOCMe_3$ | 412 |
| 3-450 | OH | H | H | H | H | $CH_2CH_2OCOC_6H_5$ | 404 |
| 3-451 | OH | H | H | H | H | $CH_2CH_2CH_2OCOC_6H_5$ | 418 |
| 3-452 | OH | H | H | H | H | $CH_2CH_2CH_2CH_2OCOC_6H_5$ | 432 |
| 3-453 | OH | H | H | H | H | $CH_2CH_2OCOCH_2OH$ | 358 |
| 3-454 | OH | H | H | H | H | $CH_2CH_2OCOCH_2NH_2$ | 357 |
| 3-455 | OH | H | H | H | H | $CH_2CH_2OCH_2CH_2OMe$ | 358 |
| 3-456 | OH | H | H | H | H | $CH_2CH_2OCH_2CH_2OH$ | 344 |
| 3-457 | OH | H | H | H | H | $CH_2CH_2CH_2OCH_2CH_2OH$ | 358 |
| 3-458 | OH | H | H | H | H | $CH_2CH_2CH_2CH_2OCH_2CH_2OH$ | 372 |
| 3-459 | OH | H | H | H | H | $CH_2CH\ (CH_2OH)\ NH_2$ | 329 |
| 3-460 | OH | H | H | H | H | $CH_2CH\ (CH_2OH)\ NHCOMe$ | 371 |
| 3-461 | OH | H | H | H | H | a-50 | 454 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-462 | OH | H | H | H | H | $CH_2CH(CH_2OH)NHCONHC_6H_5$ | 448 |
| 3-463 | OH | H | H | H | H | $CH(CH_2OH)CH_2NH_2$ | 329 |
| 3-464 | OH | H | H | H | H | $CH(CH_2OH)CH_2NHCOMe$ | 371 |
| 3-465 | OH | H | H | H | H | a-51 | 454 |
| 3-466 | OH | H | H | H | H | $CH\ (CH_2OH)\ CH_2NHCONHC_6H_5$ | 448 |
| 3-467 | OH | H | H | H | H | $CH_2CH\ (OH)\ CH_2NH_2$ | 329 |
| 3-468 | OH | H | H | H | H | $CH_2CH\ (OH)\ CH_2NHMe$ | 343 |
| 3-469 | OH | H | H | H | H | $CH_2CH\ (OH)\ CH_2NMe_2$ | 357 |
| 3-470 | OH | H | H | H | H | $CH_2CH_2CH_2CH_2NH_2$ | 327 |
| 3-471 | OH | H | H | H | H | $CH_2CH_2CH_2CH_2CH_2NH_2$ | 341 |
| 3-472 | OH | H | H | H | H | $CH_2CH_2NHMe$ | 313 |
| 3-473 | OH | H | H | H | H | $CH_2CH_2CH_2CH_2NHMe$ | 341 |
| 3-474 | OH | H | H | H | H | $CH_2CH_2CH_2CH_2CH_2NHMe$ | 355 |
| 3-475 | OH | H | H | H | H | $CH_2CH_2NMe_2$ | 327 |
| 3-476 | OH | H | H | H | H | $CH_2CH_2CH_2NMe_2$ | 341 |
| 3-477 | OH | H | H | H | H | $CH_2CH_2CH_2CH_2NMe_2$ | 355 |
| 3-478 | OH | H | H | H | H | $CH_2CH_2CH_2CH_2CH_2NMe_2$ | 369 |
| 3-479 | OH | H | H | H | H | $CH_2CH_2NHCH_2CH_2OH$ | 343 |
| 3-480 | OH | H | H | H | H | $CH_2CH_2CH_2NHCH_2CH_2OH$ | 357 |
| 3-481 | OH | H | H | H | H | $CH_2CH_2CH_2CH_2NHCH_2CH_2OH$ | 371 |
| 3-482 | OH | H | H | H | H | $CH_2CONH_2$ | 313 |
| 3-483 | OH | H | H | H | H | $CH_2CH_2CONH_2$ | 327 |
| 3-484 | OH | H | H | H | H | $CH_2CH_2CH_2CONH_2$ | 341 |
| 3-485 | OH | H | H | H | H | $CH_2CH_2NHCOCH_2Me$ | 355 |
| 3-486 | OH | H | H | H | H | $CH_2CH_2NHCOCH_2CH_2Me$ | 369 |
| 3-487 | OH | H | H | H | H | $CH_2CH_2NHCOCHMe_2$ | 369 |
| 3-488 | OH | H | H | H | H | $CH_2CH_2NHCOCMe_3$ | 383 |
| 3-489 | OH | H | H | H | H | $CH_2CH_2NHCOCH_2OH$ | 357 |
| 3-490 | OH | H | H | H | H | $CH_2CH_2NHCOCH_2OMe$ | 371 |
| 3-491 | OH | H | H | H | H | $CH_2CH_2NHCOC_6H_{.}$ | 403 |
| 3-492 | OH | H | H | H | H | a-52 | 404 |
| 3-493 | OH | H | H | H | H | a-53 | 409 |
| 3-494 | OH | H | H | H | H | a-54 | 393 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-495 | OH | H | H | H | H | a-55 | 405 |
| 3-496 | OH | H | H | H | H | $CH_2CH_2NHCOCH_2NHCOCH_2CHMe_2$ | 440 |
| 3-497 | OH | H | H | H | H | $CH_2CH_2NHCOCH_2NHCOC_6H_5$ | 460 |
| 3-498 | OH | H | H | H | H | $CH_2CH_2CH_2NHCOMe$ | 355 |
| 3-499 | OH | H | H | H | H | $CH_2CH_2CH_2NHCOCH_2Me$ | 369 |
| 3-500 | OH | H | H | H | H | $CH_2CH_2CH_2NHCOCH_2CH_2Me$ | 383 |
| 3-501 | OH | H | H | H | H | $CH_2CH_2CH_2NHCOCHMe_2$ | 383 |
| 3-502 | OH | H | H | H | H | $CH_2CH_2CH_2NHCOCMe_3$ | 397 |
| 3-503 | OH | H | H | H | H | $CH_2CH_2CH_2CH_2NHCOMe$ | 369 |
| 3-504 | OH | H | H | H | H | $CH_2CH_2CH_2CH_2NHCOCH_2Me$ | 383 |
| 3-505 | OH | H | H | H | H | $CH_2CH_2CH_2CH_2NHCOCH_2CH_2Me$ | 397 |
| 3-506 | OH | H | H | H | H | $CH_2CH_2CH_2CH_2NHCOCHMe_2$ | 397 |
| 3-507 | OH | H | H | H | H | $CH_2CH_2CH_2CH_2NHCOCMe_3$ | 411 |
| 3-508 | OH | H | H | H | H | $CH_2CH_2N$ (Me) COMe | 355 |
| 3-509 | OH | H | H | H | H | $CH_2CH_2N$ (Me) $COC_6H_5$ | 417 |
| 3-510 | OH | H | H | H | H | $CH_2CH_2CH_2N$ (Me) COMe | 369 |
| 3-511 | OH | H | H | H | H | $CH_2CH_2CH_2N$ (Me) $COC_6H_5$ | 431 |
| 3-512 | OH | H | H | H | H | $CH_2CH_2CH_2CH_2N$ (Me) COMe | 383 |
| 3-513 | OH | H | H | H | H | $CH_2CH_2N$ ($CH_2CH_2OH$) COMe | 385 |
| 3-514 | OH | H | H | H | H | $CH_2CH_2CH_2N$ ($CH_2CH_2OH$) COMe | 399 |
| 3-515 | OH | H | H | H | H | $CH_2CH_2N$ ($CH_2CN$) COMe | 380 |
| 3-516 | OH | H | H | H | H | $CH_2CH_2CH_2N$ ($CH_2CN$) COMe | 394 |
| 3-517 | OH | H | H | H | H | a-56 | 326 |
| 3-518 | OH | H | H | H | H | a-57 | 325 |
| 3-519 | OH | H | H | H | H | a-58 | 367 |
| 3-520 | OH | H | H | H | H | a-59 | 429 |
| 3-521 | OH | H | H | H | H | a-60 | 457 |
| 3-522 | OH | H | H | H | H | a-61 | 340 |
| 3-523 | OH | H | H | H | H | a-62 | 353 |
| 3-524 | OH | H | H | H | H | a-63 | 339 |
| 3-525 | OH | H | H | H | H | $CH_2CH_2NHSO_2C_6H_5$ | 439 |
| 3-526 | OH | H | H | H | H | $CH_2CH_2NHSO_2NMe_2$ | 406 |
| 3-527 | OH | H | H | H | H | $CH_2CH_2N$ (Me) $SO_2Me$ | 391 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-528 | OH | H | H | H | H | $CH_2CH_2N$ (Me) $SO_2C_6H_5$ | 453 |
| 3-529 | OH | H | H | H | H | $CH_2CH_2N(Me)SO_2NMe_2$ | 420 |
| 3-530 | OH | H | H | H | H | $CH_2CH_2N$ ($CH_2CH_2OH$) $SO_2Me$ | 407 |
| 3-531 | OH | H | H | H | H | $CH_2CH_2CH_2NHSO_2Me$ | 391 |
| 3-532 | OH | H | H | H | H | $CH_2CH_2CH_2NHSO_2C_6H_5$ | 453 |
| 3-533 | OH | H | H | H | H | $CH_2CH_2CH_2NHSO_2NMe_2$ | 420 |
| 3-534 | OH | H | H | H | H | $CH_2CH_2CH_2N$ (Me) $SO_2Me$ | 405 |
| 3-535 | OH | H | H | H | H | $CH_2CH_2CH_2N$ (Me) $SO_2C_6H_5$ | 467 |
| 3-536 | OH | H | H | H | H | $CH_2CH_2CH_2N$ (Me) $SO_2NMe_2$ | 434 |
| 3-537 | OH | H | H | H | H | $CH_2CH_2CH_2N$ ($CH_2CH_2OH$) $SO_2Me$ | 421 |
| 3-538 | OH | H | H | H | H | $CH_2CH_2CH_2CH_2NHSO_2Me$ | 405 |
| 3-539 | OH | H | H | H | H | $CH_2CH_2CH_2CH_2NHSO_2C_6H_5$ | 467 |
| 3-540 | OH | H | H | H | H | $CH_2CH_2CH_2CH_2NHSO_2NMe_2$ | 434 |
| 3-541 | OH | H | H | H | H | $CH_2CH_2CH_2CH_2N$ (Me) $SO_2Me$ | 419 |
| 3-542 | OH | H | H | H | H | $CH_2CH_2CH_2CH_2N(Me)SO_2C_6H_5$ | 481 |
| 3-543 | OH | H | H | H | H | $CH_2CH_2CH_2CH_2N$ (Me) $SO_2NMe_2$ | 448 |
| 3-544 | OH | H | H | H | H | $CH_2CH_2CH_2CH_2N$ ($CH_2CH_2OH$) $SO_2Me$ | 435 |
| 3-545 | OH | H | H | H | H | a-64 | 403 |
| 3-546 | OH | H | H | H | H | a-65 | 367 |
| 3-547 | OH | H | H | H | H | a-66 | 431 |
| 3-548 | OH | H | H | H | H | a-67 | 381 |
| 3-549 | OH | H | H | H | H | $CH_2CH_2NHCONHMe$ | 356 |
| 3-550 | OH | H | H | H | H | $CH_2CH_2NHCONHCH_2Me$ | 370 |
| 3-551 | OH | H | H | H | H | $CH_2CH_2NHCONHCH_2CH_2Me$ | 384 |
| 3-552 | OH | H | H | H | H | $CH_2CH_2NHCONHCHMe_2$ | 384 |
| 3-553 | OH | H | H | H | H | $CH_2CH_2NHCONHCMe_3$ | 398 |
| 3-554 | OH | H | H | H | H | a-68 | 410 |
| 3-555 | OH | H | H | H | H | a-69 | 424 |
| 3-556 | OH | H | H | H | H | a-70 | 438 |
| 3-557 | OH | H | H | H | H | a-71 | 452 |
| 3-558 | OH | H | H | H | H | a-72 | 476 |
| 3-559 | OH | H | H | H | H | $CH_2CH_2NHCONHC_6H_5$ | 418 |
| 3-560 | OH | H | H | H | H | a-73 | 448 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-561 | OH | H | H | H | H | a-74 | 434 |
| 3-562 | OH | H | H | H | H | a-75 | 433 |
| 3-563 | OH | H | H | H | H | a-76 | 461 |
| 3-564 | OH | H | H | H | H | a-77 | 468 |
| 3-565 | OH | H | H | H | H | a-78 | 468 |
| 3-566 | OH | H | H | H | H | a-79 | 443 |
| 3-567 | OH | H | H | H | H | a-80 | 447 |
| 3-568 | OH | H | H | H | H | a-81 | 452 |
| 3-569 | OH | H | H | H | H | $CH_2CH_2NHCONHCOC_6H_5$ | 446 |
| 3-570 | OH | H | H | H | H | $CH_2CH_2NHCONHSO_2C_6H_5$ | 482 |
| 3-571 | OH | H | H | H | H | a-82 | 360 |
| 3-572 | OH | H | H | H | H | a-83 | 360 |
| 3-573 | OH | H | H | H | H | a-84 | 475 |
| 3-574 | OH | H | H | H | H | a-85 | 462 |
| 3-575 | OH | H | H | H | H | a-86 | 474 |
| 3-576 | OH | H | H | H | H | a-87 | 424 |
| 3-577 | OH | H | H | H | H | $CH_2CH_2NHCONHCMe_2CH_2CMe_3$ | 454 |
| 3-578 | OH | H | H | H | H | a-88 | 437 |
| 3-579 | OH | H | H | H | H | $CH_2CH_2N$ (Me) $CONHC_6H_5$ | 432 |
| 3-580 | OH | H | H | H | H | $CH_2CH_2N$ (Me) CON (Me) $C_6H_5$ | 446 |
| 3-581 | OH | H | H | H | H | $CH_2CH_2NHCONHCH_2CH_2OH$ | 386 |
| 3-582 | OH | H | H | H | H | $CH_2CH_2NHCONHCH_2CH_2OMe$ | 400 |
| 3-583 | OH | H | H | H | H | $CH_2CH_2NHCONHCH_2CH_2NMe_2$ | 413 |
| 3-584 | OH | H | H | H | H | $CH_2CH_2NHCON(Me)$ $CH_2CH_2OH$ | 400 |
| 3-585 | OH | H | H | H | H | $CH_2CH_2NHCON(CH_2CH_2OH)_2$ | 430 |
| 3-586 | OH | H | H | H | H | a-89 | 411 |
| 3-587 | OH | H | H | H | H | $CH_2CH_2NHCONMe_2$ | 370 |
| 3-588 | OH | H | H | H | H | $CH_2CH_2CH_2NHCONHMe$ | 370 |
| 3-589 | OH | H | H | H | H | $CH_2CH_2CH_2NHCONHCH_2Me$ | 384 |
| 3-590 | OH | H | H | H | H | $CH_2CH_2CH_2NHCONHCH_2CH_2Me$ | 398 |
| 3-591 | OH | H | H | H | H | $CH_2CH_2CH_2NHCONHCHMe_2$ | 398 |
| 3-592 | OH | H | H | H | H | $CH_2CH_2CH_2NHCONHCMe_3$ | 412 |
| 3-593 | OH | H | H | H | H | a-90 | 424 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-594 | OH | H | H | H | H | a-91 | 438 |
| 3-595 | OH | H | H | H | H | a-92 | 452 |
| 3-596 | OH | H | H | H | H | a-93 | 466 |
| 3-597 | OH | H | H | H | H | a-94 | 490 |
| 3-598 | OH | H | H | H | H | $CH_2CH_2CH_2NHCONHC_6H_5$ | 432 |
| 3-599 | OH | H | H | H | H | a-95 | 462 |
| 3-600 | OH | H | H | H | H | a-96 | 448 |
| 3-601 | OH | H | H | H | H | a-97 | 447 |
| 3-602 | OH | H | H | H | H | a-98 | 475 |
| 3-603 | OH | H | H | H | H | a-99 | 482 |
| 3-604 | OH | H | H | H | H | a-100 | 482 |
| 3-605 | OH | H | H | H | H | a-101 | 457 |
| 3-606 | OH | H | H | H | H | a-102 | 461 |
| 3-607 | OH | H | H | H | H | a-103 | 466 |
| 3-608 | OH | H | H | H | H | $CH_2CH_2CH_2NHCONHCOC_6H_5$ | 460 |
| 3-609 | OH | H | H | H | H | a-104 | 508 |
| 3-610 | OH | H | H | H | H | a-105 | 508 |
| 3-611 | OH | H | H | H | H | a-106 | 489 |
| 3-612 | OH | H | H | H | H | a-107 | 476 |
| 3-613 | OH | H | H | H | H | a-108 | 488 |
| 3-614 | OH | H | H | H | H | a-109 | 438 |
| 3-615 | OH | H | H | H | H | $CH_2CH_2CH_2NHCONHCMe_2CH_2CM_3$, | 468 |
| 3-616 | OH | H | H | H | H | a-110 | 451 |
| 3-617 | OH | H | H | H | H | $CH_2CH_2CH_2N$ (Me) $CONHC_6H_5$ | 446 |
| 3-618 | OH | H | H | H | H | $CH_2CH_2CH_2N$ (Me) CON (Me) $C_6H_5$ | 460 |
| 3-619 | OH | H | H | H | H | $CH_2CH_2CH_2NHCONHCH_2CH_2OMe$ | 414 |
| 3-620 | OH | H | H | H | H | $CH_2CH_2CH_2NHCONHCH_2CH_2NMe_2$ | 427 |
| 3-621 | OH | H | H | H | H | $CH_2CH_2CH_2NHCON$ (Me) $CH_2CH_2OH$ | 414 |
| 3-622 | OH | H | H | H | H | $CH_2CH_2CH_2NHCON$ $(CH_2CH_2OH)_2$ | 444 |
| 3-623 | OH | H | H | H | H | a-111 | 425 |
| 3-624 | OH | H | H | H | H | $CH_2CH_2CH_2NHCONMe_2$ | 384 |
| 3-625 | OH | H | H | H | H | $CH_2CH_2NHCSNHCH_2Me$ | 386 |
| 3-626 | OH | H | H | H | H | $CH_2CH_2NHCSNHCH_2CH_2Me$ | 400 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-627 | OH | H | H | H | H | $CH_2CH_2NHCSNHCHMe_2$ | 400 |
| 3-628 | OH | H | H | H | H | $CH_2CH_2NHCSNHCMe_3$ | 414 |
| 3-629 | OH | H | H | H | H | a-112 | 360 |
| 3-630 | OH | H | H | H | H | $CH_2CH_2NHCSNHCH_2CH_2OMe$ | 416 |
| 3-631 | OH | H | H | H | H | $CH_2CH_2NHCSNHCH_2CH_2NMe_2$ | 429 |
| 3-632 | OH | H | H | H | H | $CH_2CH_2NHCSN$ (Me) $CH_2CH_2OH$ | 416 |
| 3-633 | OH | H | H | H | H | $CH_2CH_2NHCSN$ $(CH_2CH_2OH)_2$ | 446 |
| 3-634 | OH | H | H | H | H | a-113 | 427 |
| 3-635 | OH | H | H | H | H | $CH_2CH_2NHCSNMe_2$ | 386 |
| 3-636 | OH | H | H | H | H | $CH_2CH_2NHCOOMe$ | 357 |
| 3-637 | OH | H | H | H | H | $CH_2CH_2NHCOOCH_2Me$ | 371 |
| 3-638 | OH | H | H | H | H | $CH_2CH_2NHCOOCH_2CH_2Me$ | 385 |
| 3-639 | OH | H | H | H | H | $CH_2CH_2NHCOOCHMe_2$ | 385 |
| 3-640 | OH | H | H | H | H | a-114 | 425 |
| 3-641 | OH | H | H | H | H | $CH_2CH_2NHCOOC_6H_5$ | 419 |
| 3-642 | OH | H | H | H | H | $CH_2CH_2NHCOOCH_2CH_2OH$ | 387 |
| 3-643 | OH | H | H | H | H | $CH_2CH_2NHCOOCH_2CH_2OMe$ | 401 |
| 3-644 | OH | H | H | H | H | a-115 | 380 |
| 3-645 | OH | H | H | H | H | a-116 | 380 |
| 3-646 | OH | H | H | H | H | a-117 | 367 |
| 3-647 | OH | H | H | H | H | a-118 | 381 |
| 3-648 | OH | H | H | H | H | a-119 | 356 |
| 3-649 | OH | H | H | H | H | a-120 | 381 |
| 3-650 | OH | H | H | H | H | $COC_6H_5$ | 360 |
| 3-651 | H | Cl | H | H | H | H | 274 |
| 3-652 | H | Br | H | H | H | H | 318 |
| 3-653 | H | OH | H | H | H | H | 256 |
| 3-654 | H | Me | H | H | H | H | 254 |
| 3-655 | H | $CH=CH_2$ | H | H | H | H | 266 |
| 3-656 | H | $C\equiv CC_6H_5$ | H | H | H | H | 340 |
| 3-657 | H | OMe | H | H | H | H | 270 |
| 3-658 | H | $NH_2$ | H | H | H | H | 255 |
| 3-659 | H | NHMe | H | H | H | H | 269 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-660 | H | $NMe_2$ | H | H | H | H | 283 |
| 3-661 | H | $NHC_6H_5$ | H | H | H | H | 331 |
| 3-662 | H | H | Cl | H | H | H | 274 |
| 3-663 | H | H | Br | H | H | H | 318 |
| 3-664 | H | H | OH | H | H | H | 256 |
| 3-665 | H | H | Me | H | H | H | 254 |
| 3-666 | H | H | $CH=CH_2$ | H | H | H | 266 |
| 3-667 | H | H | $C\equiv CC_6H_5$ | H | H | H | 340 |
| 3-668 | H | H | OMe | H | H | H | 270 |
| 3-669 | H | H | $NH_2$ | H | H | H | 255 |
| 3-670 | H | H | NHMe | H | H | H | 269 |
| 3-671 | H | H | $NMe_2$ | H | H | H | 283 |
| 3-672 | H | H | $NHC_6H_5$ | H | H | H | 331 |
| 3-673 | H | H | H | Cl | H | H | 274 |
| 3-674 | H | H | H | Br | H | H | 318 |
| 3-675 | H | H | H | OH | H | H | 256 |
| 3-676 | H | H | H | Me | H | H | 254 |
| 3-677 | H | H | H | $CH=CH_2$ | H | H | 266 |
| 3-678 | H | H | H | $C\equiv CC_6H_5$ | H | H | 340 |
| 3-679 | H | H | H | 0Me | H | H | 270 |
| 3-680 | H | H | H | $NH_2$ | H | H | 255 |
| 3-681 | H | H | H | NHMe | H | H | 269 |
| 3-682 | H | H | H | $NMe_2$ | H | H | 283 |
| 3-683 | H | H | H | $NHC_6H_5$ | H | H | 331 |
| 3-684 | H | H | H | H | C1 | H | 274 |
| 3-685 | H | H | H | H | Br | H | 318 |
| 3-686 | H | H | H | H | OH | H | 256 |
| 3-687 | H | H | H | H | Me | H | 254 |
| 3-688 | H | H | H | H | $CH=CH_2$ | H | 266 |
| 3-689 | H | H | H | H | $C\equiv CC_6H_5$ | H | 340 |
| 3-690 | H | H | H | H | OMe | H | 270 |
| 3-691 | H | H | H | H | $NH_2$ | H | 255 |
| 3-692 | H | H | H | H | NHMe | H | 269 |

(continued)

| Exp. No. | $R^1$ | $R^5$ | $R^8$ | $R^7$ | $R^6$ | $A^{61}$ | Mass($MH^+$) |
|---|---|---|---|---|---|---|---|
| 3-693 | H | H | H | H | $NMe_2$ | H | 283 |
| 3-694 | H | H | H | H | $NHC_6H_5$ | H | 331 |

Example 4-1: 4-(2,3-Dihydro-1,5-diazaphenalen-1-ylmethyl)piperidine

Step A

4-(2,3-Dihydro-1,5-diazaphenalen-1-ylmethyl)piperidine-1-carboxylic acid tert-butyl ester (Intermediate 35)

By using 5-amino-4-vinylisoquinoline (Intermediate 1) obtained in Example 1-1, Step A, and tert-butyl 4-formyl-1-piperidinecarboxylate (PharmaCore), the title compound was obtained according to the methods described in Example 1-1, Step B and C.
MS (m/z): 368 (MH+)

Step B

4-(2,3-Dihydro-1,5-diazaphenalen-1-ylmethyl)piperidine

By using 4-(2,3-dihydro-1,5-diazaphenalen-1-ylmethyl)piperidine-1-carboxylic acid tert-butyl ester (Intermediate 35) obtained in Step A mentioned above, the title compound was obtained as a hydrochloride according to the method described in Example 1-1, Step D.
MS (m/z): 268 (MH+)

Examples 4-2 to 4-320

The compounds of Examples 4-2 to 4-320 were obtained by using 4-(2,3-dihydro-1,5-diazaphenalen-1-ylmethyl) piperidine obtained in Example 4-1 according to the methods described in Examples 1-2 to 1-320.

Example 4-321: 6-Chloro-1-(piperidin-4-ylmethyl)-2,3-dihydro-1H-1,5-diazaphenalene Step A

4-(6-Chloro-2,3-dihydro-1,5-diazaphenalen-1-ylmethyl)piperidine-1-carboxylic acid tert-butyl ester (Intermediate 36)

By using 5-amino-1-chloro-4-vinylisoquinoline (Intermediate 8) obtained in Example 1-321, Step C, the title compound was obtained according to the method described in Example 1-1.
MS (m/z): 402

Step B

6-Chloro-1-(p iperidin-4-ylmethyl)-2,3-dihydro-1H-1,5-diazaphenalene

By using 4-(6-chloro-2,3-dihydro-1,5-diazaphenalen-1-ylmethyl)piperidine-1-carboxylic acid tert-butyl ester (Intermediate 36) obtained in Step A mentioned above, the title compound was obtained as a hydrochloride according to the method described in Example 1-1, Step D.
MS (m/z): 302 (MH+)

Example 4-322

The compound of Example 4-322 was obtained in the same manner as that used for the compound of Example 1-322.

Example 4-323

The compound of Example 4-323 was obtained in the same manner as that used for the compound of Example 1-323.

Examples 4-324 to 4-331

The compounds of Examples 4-324 to 4-331 were obtained by using 4-(6-chloro-2,3-dihydro-1,5-diazaphenalen-1-ylmethyl)piperidine-1-carboxylic acid tert-butyl ester (Intermediate 36) obtained in Example 4-321, Step A according to the methods described in Examples 1-324 to 1-331.

Examples 4-332 to 4-650

The compounds of Examples 4-332 to 4-650 were obtained by using 6-chloro-1-(piperidin-4-ylmethyl)-2,3-dihydro-1H-1,5-diazaphenalene obtained in Example 4-321, or 6-hydroxy-1-(piperidin-4-ylmethyl)-2,3-dihydro-1H-1,5-diazaphenalene obtained in Example 4-323 according to the methods described in Examples 1-2 to 1-320.

Example 4-651: 4-Chloro-1-(piperidin-4-ylmethyl)-2,3-dihydro-1H-1,5-diazaphenalene Step A

4-(4-Chloro-2,3-dihydro-1,5-diazaphenalen-1-ylmethyl)piperidine-1-carboxylic acid tert-butyl ester (Intermediate 37)

By using 3-chloro-5-nitro-4-vinylisoquinoline (Intermediate 7) obtained in Example 1-321, Step C, the title compound was obtained according to the methods described in Example 1-321, Steps D to F.
MS (m/z): 402 (MH+)

Step B

4-Chloro-1-(piperidin-4-ylmethyl)-2,3-dihydro-1H-1,5-diazaphenalene

By using 4-(4-chloro-2,3-dihydro-1,5-diazaphenalen-1-ylmethyl)piperidine-1-carboxylic acid tert-butyl ester (Intermediate 37) obtained in Step C mentioned above, the title compound was obtained as a hydrochloride according to the method described in Example 1-1, Step D.
MS (m/z): 302 (MH+)

Example 4-652

The compound of Example 4-652 was obtained according to the method described in Example 4-651.

Example 4-653

The compound of Example 4-653 was obtained according to Method A or Method B of Example 1-323,.

Examples 4-654 to 4-661

The compounds of Examples 4-654 to 4-661 were obtained by using 4-(4-chloro-2,3-dihydro-1,5-diazaphenalen-1-ylmethyl)piperidine-1-carboxylic acid tert-butyl ester according to the methods described in Examples 1-324 to 1-331.

Example 4-662

The compound of Example 4-662 was obtained according to the method described in Example 4-663 mentioned below.

Example 4-663: 7-Bromo-1-(piperidin-4-yl)methyl-2,3-dihydro-1H-1,5-diazaphenalene Step A

4-(7-Bromo-2,3-dihydro-1,5-diazaphenalen-1-ylmethyl)piperidine-1-carboxylic acid tert-butyl ester (Intermediate 38), and

4-(8-bromo-2,3-dihydro-1,5-diazaphenalen-1-ylmethyl)piperidine-1-carboxylic acid tert-butyl ester (Intermediate 39)

By using 4-(2,3-dihydro-1,5-diazaphenalen-1-ylmethyl)piperidine-1-carboxylic acid tert-butyl ester (Intermediate 35) obtained in Example 4-1, Step A, each of the title compounds was obtained according to the method described in Example 1-663, Step A.
MS (m/z): 446 (MH+)

Step B

7-Bromo-1-(piperidin-4-ylmethyl)-2,3-dihydro-1H-1,5-diazaphenalene

By using 4-(7-bromo-2,3-dihydro-1,5-diazaphenalen-1-yl)piperidine-1-carboxylic acid tert-butyl ester (Intermediate 38) obtained in Step A mentioned above, the title compound was obtained as a hydrochloride according to the method described in Example 1-1, Step D.
MS (m/z): 346 (MH+)

Examples 4-664 to 4-672

The compounds of Examples 4-664 to 4-672 were obtained by using 4-(7-bromo-2,3-dihydro-1,5-diazaphenalen-1-yl)piperidine-1-carboxylic acid tert-butyl ester obtained in Example 4-663, Step A according to the methods described in Examples 1-323 to 1-331.

Example 4-673

The compound of Example 4-673 was obtained according to the method described in Example 4-674 mentioned below.

Example 4-674: 8-Bromo-1-(piperidin-4-ylmethyl)-2,3-dihydro-1H-1,5-diazaphenalene By using

4-(8-bromo-2,3-dihydro-1,5-diazaphenalen-1-ylmethyl)piperidine-1-carboxylic acid tert-butyl ester (Intermediate 39) obtained in Example 4-663, Step A, the title compound was obtained as a hydrochloride according to the method described in Example 1-1, Step D.
MS (m/z) : 346 (MH+)

Examples 4-675 to 4-683

The compounds of Examples 4-675 to 4-683 were obtained by using 4-(8-bromo-2,3-dihydro-1,5-diazaphenalen-1-ylmethyl)piperidine-1-carboxylic acid tert-butyl ester obtained in Example 4-663, Step A according to the methods described in Examples 1-323 to 1-331.

Examples 4-684 and 4-685

The compounds of Examples 4-684 and 4-685 was obtained from the compound of Example 4-691 mentioned below by referring to a known diazotization-Sandmeyer reaction (for example, Denny, William , et al, J. Med. Chem., 2002, 740; Kulka, J. Am. Chem. Soc., 75, 3597 (1953) and the like).

Example 4-691: 9-Amino-1-(piperidin-4-ylmethyl)-2,3-dihydro-1H-1,5-diazaphenalene Step A

4-(9-Amino-2,3-dihydro-1,5-diazaphenalen- -1-ylmethyl)piperidine-1-carboxylic acid tert-butyl ester (Intermediate 40)

By using 4-(7-bromo-2,3-dihydro-1,5-diazaphenalen-1-ylmethyl)piperidine-1-carboxylic acid tert-butyl ester (Intermediate 38) obtained in Example 4-663, Step A, nitration (9-position), and reduction (bromine atom→hydrogen atom at the 7-position, nitro group→amino group at the 9-position) were performed with referring to a known publication (for example, Kucznierz, R., Dickhaut, J, Leinert, H., Von Der Saal, W., Synth. Commun., 29, 1617 (1999); Ping Chen, Bioorganic & Medicinal Chemistry Letters, 13, 1345 (2003) and the like) to obtain the title compound.
MS (m/z): 383 (MH+)

Step B

9-Amino-1-(piperidin-4-ylmethyl)-2,3-dihydro-1H-1,5-diazaphenalene

By using 4-(9-amino-2,3-dihydro-1,5-diazaphenalen-1-ylmethyl)piperidine-1-carboxylic acid tert-butyl ester (Intermediate 40) obtained in Step A mentioned above, the title compound was obtained as a hydrochloride according to the method described in Example 1-1, Step D.
MS (m/z): 283 (MH+)

Examples 4-692 to 4-694

The compounds of Examples 4-692 to 4-694 were obtained by using 4-(9-bromo-2,3-dihydro-1,5-diazaphenalen-1-ylmethyl)piperidine-1-carboxylic acid tert-butyl ester obtained in Example 4-685 according to the methods described in

Examples 1-329 to 1-331.

[Table 4]

| Exp. No. | $R^1$ | $R^5$ | $R^8$ | $R^7$ | $R^6$ | $A^{61}$ | Mass ($MH^+$) |
|---|---|---|---|---|---|---|---|
| 4-1 | H | H | H | H | H | H | 268 |
| 4-2 | H | H | H | H | H | Me | 282 |
| 4-3 | H | H | H | H | H | a-1 | 350 |
| 4-4 | H | H | H | H | H | a-2 | 388 |
| 4-5 | H | H | H | H | H | $C_6H_5$ | 344 |
| 4-6 | H | H | H | H | H | $CH_2COOH$ | 326 |
| 4-7 | H | H | H | H | H | $CH_2CH_2COOH$ | 340 |
| 4-8 | H | H | H | H | H | $CH_2CN$ | 307 |
| 4-9 | H | H | H | H | H | $CH_2CH_2CN$ | 321 |
| 4-10 | H | H | H | H | H | $CH_2CH_2OH$ | 312 |
| 4-11 | H | H | H | H | H | $CH(CH_2OH)_2$ | 342 |
| 4-12 | H | H | H | H | H | a-3 | 366 |
| 4-13 | H | H | H | H | H | $CH_2CH_2OMe$ | 326 |
| 4-14 | H | H | H | H | H | $CH_2CH_2OCOCMe_3$ | 396 |
| 4-15 | H | H | H | H | H | $CH_2CH_2SCOMe$ | 370 |
| 4-16 | H | H | H | H | H | $CH_2CH_2SH$ | 328 |
| 4-17 | H | H | H | H | H | $CH_2CH_2NH_2$ | 311 |
| 4-18 | H | H | H | H | H | $CH_2CH_2CH_2NH_2$ | 325 |
| 4-19 | H | H | H | H | H | $CH_2CH_2CH_2NHMe$ | 339 |
| 4-20 | H | H | H | H | H | $CH(CH_2NHCOMe)_2$ | 424 |
| 4-21 | H | H | H | H | H | $CH(CH_2NH_2)_2$ | 340 |
| 4-22 | H | H | H | H | H | $CH_2CH_2NHCOMe$ | 353 |
| 4-23 | H | H | H | H | H | a-4 | 366 |
| 4-24 | H | H | H | H | H | $CH_2CH_2NHCOCH_2NHCOMe$ | 410 |
| 4-25 | H | H | H | H | H | $CH_2CH_2NHCOCH_2NMe_2$ | 396 |
| 4-26 | H | H | H | H | H | $CH_2CH_2NHSO_2Me$ | 389 |
| 4-27 | H | H | H | H | H | a-5 | 365 |
| 4-28 | H | H | H | H | H | a-6 | 407 |
| 4-29 | H | H | H | H | H | $CH_2CH_2CH_2NHCONHCH_2CH_2OH$ | 412 |
| 4-30 | H | H | H | H | H | $CH_2CH_2CH_2NHCONHBn$ | 458 |
| 4-31 | H | H | H | H | H | $CH_2CH_2NHCSNHCH_2CH_2OH$ | 414 |
| 4-32 | H | H | H | H | H | $CH_2CH_2NHCSNHC_3H_5$ | 446 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-33 | H | H | H | H | H | $CH_2CH_2NHCOOCH_2CH_2NMe_2$ | 426 |
| 4-34 | H | H | H | H | H | a-7 | 423 |
| 4-35 | H | H | H | H | H | a-8 | 310 |
| 4-36 | H | H | H | H | H | a-9 | 365 |
| 4-37 | H | H | H | H | H | COMe | 310 |
| 4-38 | H | H | H | H | H | a-10 | 322 |
| 4-39 | H | H | H | H | H | a-11 | 336 |
| 4-40 | H | H | H | H | H | a-12 | 350 |
| 4-41 | H | H | H | H | H | Bn | 358 |
| 4-42 | H | H | H | H | H | a-13 | 392 |
| 4-43 | H | H | H | H | H | a-14 | 374 |
| 4-44 | H | H | H | H | H | a-15 | 418 |
| 4-45 | H | H | H | H | H | a-16 | 432 |
| 4-46 | H | H | H | H | H | a-17 | 448 |
| 4-47 | H | H | H | H | H | a-18 | 425 |
| 4-48 | H | H | H | H | H | a-19 | 443 |
| 4-49 | H | H | H | H | H | a-20 | 434 |
| 4-50 | H | H | H | H | H | a-21 | 373 |
| 4-51 | H | H | H | H | H | a-22 | 387 |
| 4-52 | H | H | H | H | H | a-23 | 401 |
| 4-53 | H | H | H | H | H | a-24 | 417 |
| 4-54 | H | H | H | H | H | a-25 | 431 |
| 4-55 | H | H | H | H | H | a-26 | 461 |
| 4-56 | H | H | H | H | H | a-27 | 415 |
| 4-57 | H | H | H | H | H | a-28 | 451 |
| 4-58 | H | H | H | H | H | a-29 | 460 |
| 4-59 | H | H | H | H | H | a-30 | 402 |
| 4-60 | H | H | H | H | H | a-31 | 418 |
| 4-61 | H | H | H | H | H | a-32 | 388 |
| 4-62 | H | H | H | H | H | a-33 | 388 |
| 4-63 | H | H | H | H | H | a-34 | 418 |
| 4-64 | H | H | H | H | H | a-35 | 418 |
| 4-65 | H | H | H | H | H | a-36 | 448 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-66 | H | H | H | H | H | a-37 | 448 |
| 4-67 | H | H | H | H | H | a-38 | 346 |
| 4-68 | H | H | H | H | H | a-39 | 348 |
| 4-69 | H | H | H | H | H | a-40 | 364 |
| 4-70 | H | H | H | H | H | a-41 | 416 |
| 4-71 | H | H | H | H | H | a-42 | 403 |
| 4-72 | H | H | H | H | H | a-43 | 359 |
| 4-73 | H | H | H | H | H | a-44 | 345 |
| 4-74 | H | H | H | H | H | a-45 | 360 |
| 4-75 | H | H | H | H | H | $CH_2CH_2CH_2COOH$ | 354 |
| 4-76 | H | H | H | H | H | $CH_2CH_2CH_2CH_2COOH$ | 368 |
| 4-77 | H | H | H | H | H | $CH_2CH_2CH_2CN$ | 335 |
| 4-78 | H | H | H | H | H | $CH_2CH_2CH_2CH_2CN$ | 349 |
| 4-79 | H | H | H | H | H | $CH_2CH$ (Me) OH | 326 |
| 4-80 | H | H | H | H | H | CH (Me) $CH_2OH$ | 326 |
| 4-81 | H | H | H | H | H | $CH_2CH_2CH_2OH$ | 326 |
| 4-82 | H | H | H | H | H | $CH_2CH$ (OH) $CH_2OH$ | 342 |
| 4-83 | H | H | H | H | H | $CH_2CH$ ($NH_2$) $CH_2OH$ | 341 |
| 4-84 | H | H | H | H | H | $CH_2CH$ (NHMe) $CH_2OH$ | 355 |
| 4-85 | H | H | H | H | H | $CH_2CH$ ($NMe_2$) $CH_2OH$ | 369 |
| 4-86 | H | H | H | H | H | $CH_2CH$ (Me) $CH_2OH$ | 340 |
| 4-87 | H | H | H | H | H | $CH_2CH_2CH_2CH_2OH$ | 340 |
| 4-88 | H | H | H | H | H | $CH_2CH_2CH_2CH_2CH_2OH$ | 354 |
| 4-89 | H | H | H | H | H | a-46 | 480 |
| 4-90 | H | H | H | H | H | CH ($CH_2OH$) $CH_2CH_2NHCONHC_6H_5$ | 474 |
| 4-91 | H | H | H | H | H | a-47 | 466 |
| 4-92 | H | H | H | H | H | $CH_2CH(OH)CH_2NHCONHC_6H_5$ | 460 |
| 4-93 | H | H | H | H | H | $CH_2CH_2OCONHMe$ | 369 |
| 4-94 | H | H | H | H | H | $CH_2CH_2OCONHC_6H_5$ | 431 |
| 4-95 | H | H | H | H | H | $CH_2CH_2CH_2OCONHMe$ | 383 |
| 4-96 | H | H | H | H | H | a-48 | 451 |
| 4-97 | H | H | H | H | H | $CH_2CH_2CH_2OCONHC_6H_5$ | 445 |
| 4-98 | H | H | H | H | H | $CH_2CH_2CH_2CHOCONHMe$ | 397 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-99 | H | H | H | H | H | a-49 | 465 |
| 4-100 | H | H | H | H | H | $CH_2CH_2CH_2CH_2OCONHC_6H_5$ | 459 |
| 4-101 | H | H | H | H | H | $CH_2CH_2CH_2SH$ | 342 |
| 4-102 | H | H | H | H | H | $CH_2CH_2CH_2CH_2SH$ | 356 |
| 4-103 | H | H | H | H | H | $CH_2CH_2SCOC_6H_5$ | 432 |
| 4-104 | H | H | H | H | H | $CH_2CH_2CH_2SCOMe$ | 384 |
| 4-105 | H | H | H | H | H | $CH_2CH_2CH_2SCOC_6H_5$ | 446 |
| 4-106 | H | H | H | H | H | $CH_2CH_2CH_2OMe$ | 340 |
| 4-107 | H | H | H | H | H | $CH_2CH_2CH_2CH_2OMe$ | 354 |
| 4-108 | H | H | H | H | H | $CH_2CH_2CH_2CH_2CH_2OMe$ | 368 |
| 4-109 | H | H | H | H | H | $CH_2CH_2OCOMe$ | 354 |
| 4-110 | H | H | H | H | H | $CH_2CH_2CH_2OCOMe$ | 368 |
| 4-111 | H | H | H | H | H | $CH_2CH_2CH_2CH_2OCOMe$ | 382 |
| 4-112 | H | H | H | H | H | $CH_2CH_2OCOCH_2Me$ | 368 |
| 4-113 | H | H | H | H | H | $CH_2CH_2CH_2OCOCH_2Me$ | 382 |
| 4-114 | H | H | H | H | H | $CH_2CH_2CH_2CH_2OCOCH_2Me$ | 396 |
| 4-115 | H | H | H | H | H | $CH_2CH_2OCOCHMe_2$ | 382 |
| 4-116 | H | H | H | H | H | $CH_2CH_2CH_2OCOCHMe_2$ | 396 |
| 4-117 | H | H | H | H | H | $CH_2CH_2CH_2CH_2OCOCHMe_2$ | 410 |
| 4-118 | H | H | H | H | H | $CH_2CH_2CH_2OCOCMe_3$ | 410 |
| 4-119 | H | H | H | H | H | $CH_2CH_2CH_2CH_2OCOCMe_3$ | 424 |
| 4-120 | H | H | H | H | H | $CH_2CH_2OCOC_6H_5$ | 416 |
| 4-121 | H | H | H | H | H | $CH_2CH_2CH_2OCOC_6H_5$ | 430 |
| 4-122 | H | H | H | H | H | $CH_2CH_2CH_2CH_2OCOC_6H_5$ | 444 |
| 4-123 | H | H | H | H | H | $CH_2CH_2OCOCH_2OH$ | 370 |
| 4-124 | H | H | H | H | H | $CH_2CH_2OCOCH_2NH_2$ | 369 |
| 4-125 | H | H | H | H | H | $CH_2CH_2OCH_2CH_2OMe$ | 370 |
| 4-126 | H | H | H | H | H | $CH_2CH_2OCH_2CH_2OH$ | 356 |
| 4-127 | H | H | H | H | H | $CH_2CH_2CH_2OCH_2CH_2OH$ | 370 |
| 4-128 | H | H | H | H | H | $CH_2CH_2CH_2CH_2OCH_2CH_2OH$ | 384 |
| 4-129 | H | H | H | H | H | $CH_2CH$ ($CH_2OH$) $NH_2$ | 341 |
| 4-130 | H | H | H | H | H | $CH_2CH$ ($CH_2OH$) NHCOMe | 383 |
| 4-131 | H | H | H | H | H | a-50 | 466 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-132 | H | H | H | H | H | $CH_2CH\ (CH_2OH)\ NHCONHC_6H_5$ | 460 |
| 4-133 | H | H | H | H | H | $CH(CH_2OH)CH_2NH_2$ | 341 |
| 4-134 | H | H | H | H | H | $CH\ (CH_2OH)\ CH_2NHCOMe$ | 383 |
| 4-135 | H | H | H | H | H | a-51 | 466 |
| 4-136 | H | H | H | H | H | $CH(CH_2OH)CH_2NHCONHC_6H_5$ | 460 |
| 4-137 | H | H | H | H | H | $CH_2CH\ (OH)\ CH_2NH_2$ | 341 |
| 4-138 | H | H | H | H | H | $CH_2CH(OH)CH_2NHMe$ | 355 |
| 4-139 | H | H | H | H | H | $CH_2CH(OH)CH_2NMe_2$ | 369 |
| 4-140 | H | H | H | H | H | $CH_2CH_2CH_2CH_2NH_2$ | 339 |
| 4-141 | H | H | H | H | H | $CH_2CH_2CH_2CH_2CH_2NH_2$ | 353 |
| 4-142 | H | H | H | H | H | $CH_2CH_2NHMe$ | 325 |
| 4-143 | H | H | H | H | H | $CH_2CH_2CH_2CH_2NHMe$ | 353 |
| 4-144 | H | H | H | H | H | $CH_2CH_2CH_2CH_2CH_2NHMe$ | 367 |
| 4-145 | H | H | H | H | H | $CH_2CH_2NMe_2$ | 339 |
| 4-146 | H | H | H | H | H | $CH_2CH_2CHNMe_2$ | 353 |
| 4-147 | H | H | H | H | H | $CH_2CH_2CH_2CH_2NMe_2$ | 367 |
| 4-148 | H | H | H | H | H | $CH_2CH_2CH_2CH_2CH_2NMe_2$ | 381 |
| 4-149 | H | H | H | H | H | $CH_2CH_2NHCH_2CH_2OH$ | 355 |
| 4-150 | H | H | H | H | H | $CH_2CH_2CH_2NHCH_2CH_2OH$ | 369 |
| 4-151 | H | H | H | H | H | $CH_2CH_2CH_2CH_2NHCH_2CH_2OH$ | 383 |
| 4-152 | H | H | H | H | H | $CH_2CONH_2$ | 325 |
| 4-153 | H | H | H | H | H | $CH_2CH_2CONH_2$ | 339 |
| 4-154 | H | H | H | H | H | $CH_2CH_2CH_2CONH_2$ | 353 |
| 4-155 | H | H | H | H | H | $CH_2CH_2NHCOCH_2Me$ | 367 |
| 4-156 | H | H | H | H | H | $CH_2CH_2NHCOCH_2CH_2Me$ | 381 |
| 4-157 | H | H | H | H | H | $CH_2CH_2NHCOCHMe_2$ | 381 |
| 4-158 | H | H | H | H | H | $CH_2CH_2NHCOCMe_3$ | 395 |
| 4-159 | H | H | H | H | H | $CH_2CH_2NHCOCH_2OH$ | 369 |
| 4-160 | H | H | H | H | H | $CH_2CH_2NHCOCH_2OMe$ | 383 |
| 4-161 | H | H | H | H | H | $CH_2CH_2NHCOC_6H_5$ | 415 |
| 4-162 | H | H | H | H | H | a-52 | 416 |
| 4-163 | H | H | H | H | H | a-53 | 421 |
| 4-164 | H | H | H | H | H | a-54 | 405 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-165 | H | H | H | H | H | a-55 | 417 |
| 4-166 | H | H | H | H | H | $CH_2CH_2NHCOCH_2NHCOCH_2CHMe_2$ | 452 |
| 4-167 | H | H | H | H | H | $CH_2CH_2NHCOCH_2NHCOC_6H_5$ | 472 |
| 4-168 | H | H | H | H | H | $CH_2CH_2CH_2NHCOMe$ | 367 |
| 4-169 | H | H | H | H | H | $CH_2CH_2CH_2NHCOCH_2Me$ | 381 |
| 4-170 | H | H | H | H | H | $CH_2CH_2CH_2NHCOCH_2CH_2Me$ | 395 |
| 4-171 | H | H | H | H | H | $CH_2CH_2CH_2NHCOCHMe_2$ | 395 |
| 4-172 | H | H | H | H | H | $CH_2CH_2CH_2NHCOCMe_3$ | 409 |
| 4-173 | H | H | H | H | H | $CH_2CH_2CH_2CH_2NHCOMe$ | 381 |
| 4-174 | H | H | H | H | H | $CH_2CH_2CH_2CH_2NHCOCH_2Me$ | 395 |
| 4-175 | H | H | H | H | H | $CH_2CH_2CH_2CH_2NHCOCH_2CH_2Me$ | 409 |
| 4-176 | H | H | H | H | H | $CH_2CH_2CH_2CH_2NHCOCHMe_2$ | 409 |
| 4-177 | H | H | H | H | H | $CH_2CH_2CH_2CH_2NHCOCMe_3$ | 423 |
| 4-178 | H | H | H | H | H | $CH_2CH_2N$ (Me) COMe | 367 |
| 4-179 | H | H | H | H | H | $CH_2CH_2N$ (Me) $COC_6H_5$ | 429 |
| 4-180 | H | H | H | H | H | $CH_2CH_2CH_2N$ (Me) COMe | 381 |
| 4-181 | H | H | H | H | H | $CH_2CH_2CH_2N$ (Me) $COC_6H_5$ | 443 |
| 4-182 | H | H | H | H | H | $CH_2CH_2CH_2CH_2N$ (Me) COMe | 395 |
| 4-183 | H | H | H | H | H | $CH_2CH_2N$ ($CH_2CH_2OH$) COMe | 397 |
| 4-184 | H | H | H | H | H | $CH_2CH_2CH_2N$ ($CH_2CH_2OH$) COMe | 411 |
| 4-185 | H | H | H | H | H | $CH_2CH_2N$ ($CH_2CN$) COMe | 392 |
| 4-186 | H | H | H | H | H | $CH_2CH_2CH_2N$ ($CH_2CN$) COMe | 406 |
| 4-187 | H | H | H | H | H | a-56 | 338 |
| 4-188 | H | H | H | H | H | a-57 | 337 |
| 4-189 | H | H | H | H | H | a-58 | 379 |
| 4-190 | H | H | H | H | H | a-59 | 441 |
| 4-191 | H | H | H | H | H | a-60 | 469 |
| 4-192 | H | H | H | H | H | a-61 | 352 |
| 4-193 | H | H | H | H | H | a-62 | 365 |
| 4-194 | H | H | H | H | H | a-63 | 351 |
| 4-195 | H | H | H | H | H | $CH_2CH_2NHSO_2C_6H_5$ | 451 |
| 4-196 | H | H | H | H | H | $CH_2CH_2NHSO_2NMe_2$ | 418 |
| 4-197 | H | H | H | H | H | $CH_2CH_2N(Me)SO_2Me$ | 403 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-198 | H | H | H | H | H | $CH_2CH_2N$ (Me) $SO_2C_6H_5$ | 465 |
| 4-199 | H | H | H | H | H | $CH_2CH_2N$ (Me) $SO_2NMe_2$ | 432 |
| 4-200 | H | H | H | H | H | $CH_2CH_2N$ ($CH_2CH_2OH$) $SO_2Me$ | 419 |
| 4-201 | H | H | H | H | H | $CH_2CH_2CH_2NHSO_2Me$ | 403 |
| 4-202 | H | H | H | H | H | $CH_2CH_2CH_2NHSO_2C_6H_5$ | 465 |
| 4-203 | H | H | H | H | H | $CH_2CH_2CH_2NHSO_2NMe_2$ | 432 |
| 4-204 | H | H | H | H | H | $CH_2CH_2CH_2N$ (Me) $SO_2Me$ | 417 |
| 4-205 | H | H | H | H | H | $CH_2CH_2CH_2N$ (Me) $SO_2C_6H_5$ | 479 |
| 4-206 | H | H | H | H | H | $CH_2CH_2CH_2N$ (Me) $SO_2NMe_2$ | 446 |
| 4-207 | H | H | H | H | H | $CH_2CH_2CH_2N$ ($CH_2CH_2OH$) $SO_2Me$ | 433 |
| 4-208 | H | H | H | H | H | $CH_2CH_2CH_2CH_2NHSO_2Me$ | 417 |
| 4-209 | H | H | H | H | H | $CH_2CH_2CH_2CH_2NHSO_2C_6H_5$ | 479 |
| 4-210 | H | H | H | H | H | $CH_2CH_2CH_2CH_2NHSO_2NMe_2$ | 446 |
| 4-211 | H | H | H | H | H | $CH_2CH_2CH_2CH_2N$ (Me) $SO_2Me$ | 431 |
| 4-212 | H | H | H | H | H | $CH_2CH_2CH_2CH_2N$ (Me) $SO_2C_6H_5$ | 493 |
| 4-213 | H | H | H | H | H | $CH_2CH_2CH_2CH_2N$ (Me) $SO_2NMe_2$ | 460 |
| 4-214 | H | H | H | H | H | $CH_2CH_2CH_2CH_2N$ ($CH_2CH_2OH$) $SO_2Me$ | 447 |
| 4-215 | H | H | H | H | H | a-64 | 415 |
| 4-216 | H | H | H | H | H | a-65 | 379 |
| 4-217 | H | H | H | H | H | a-66 | 443 |
| 4-218 | H | H | H | H | H | a-67 | 393 |
| 4-219 | H | H | H | H | H | $CH_2CH_2NHCONHMe$ | 368 |
| 4-220 | H | H | H | H | H | $CH_2CH_2NHCONHCH_2Me$ | 382 |
| 4-221 | H | H | H | H | H | $CH_2CH_2NHCONHCH_2CH_2Me$ | 396 |
| 4-222 | H | H | H | H | H | $CH_2CH_2NHCONHCHMe_2$ | 396 |
| 4-223 | H | H | H | H | H | $CH_2CH_2NHCONHCMe_3$ | 410 |
| 4-224 | H | H | H | H | H | a-68 | 422 |
| 4-225 | H | H | H | H | H | a-69 | 436 |
| 4-226 | H | H | H | H | H | a-70 | 450 |
| 4-227 | H | H | H | H | H | a-71 | 464 |
| 4-228 | H | H | H | H | H | a-72 | 488 |
| 4-229 | H | H | H | H | H | $CH_2CH_2NHCONHC_6H_5$ | 430 |
| 4-230 | H | H | H | H | H | a-73 | 460 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-231 | H | H | H | H | H | a-74 | 446 |
| 4-232 | H | H | H | H | H | a-75 | 445 |
| 4-233 | H | H | H | H | H | a-76 | 473 |
| 4-234 | H | H | H | H | H | a-77 | 480 |
| 4-235 | H | H | H | H | H | a-78 | 480 |
| 4-236 | H | H | H | H | H | a-79 | 455 |
| 4-237 | H | H | H | H | H | a-80 | 459 |
| 4-238 | H | H | H | H | H | a-81 | 464 |
| 4-239 | H | H | H | H | H | $CH_2CH_2NHCONHCOC_6H_5$ | 458 |
| 4-240 | H | H | H | H | H | $CH_2CH_2NHCONHSO_2C_6H_5$ | 494 |
| 4-241 | H | H | H | H | H | a-82 | 372 |
| 4-242 | H | H | H | H | H | a-83 | 372 |
| 4-243 | H | H | H | H | H | a-84 | 487 |
| 4-244 | H | H | H | H | H | a-85 | 474 |
| 4-245 | H | H | H | H | H | a-86 | 486 |
| 4-246 | H | H | H | H | H | a-87 | 436 |
| 4-247 | H | H | H | H | H | $CH_2CH_2NHCONHCMe_2CH_2CMe_3$ | 466 |
| 4-248 | H | H | H | H | H | a-88 | 449 |
| 4-249 | H | H | H | H | H | $CH_2CH_2N(Me)CONHC_6H_5$ | 444 |
| 4-250 | H | H | H | H | H | $CH_2CH_2N$ (Me) CON (Me) $C_6H_5$ | 458 |
| 4-251 | H | H | H | H | H | $CH_2CH_2NHCONHCH_2CH_2OH$ | 398 |
| 4-252 | H | H | H | H | H | $CH_2CH_2NHCONHCH_2CH_2OMe$ | 412 |
| 4-253 | H | H | H | H | H | $CH_2CH_2NHCONHCH_2CH_2NMe_2$ | 425 |
| 4-254 | H | H | H | H | H | $CH_2CH_2NHCON$ (Me) $CH_2CH_2OH$ | 412 |
| 4-255 | H | H | H | H | H | $CH_2CH_2NHCON$ $(CH_2CH_2OH)_2$ | 442 |
| 4-256 | H | H | H | H | H | a-89 | 423 |
| 4-257 | H | H | H | H | H | $CH_2CH_2NHCONMe_2$ | 382 |
| 4-258 | H | H | H | H | H | $CH_2CH_2CH_2NHCONHMe$ | 382 |
| 4-259 | H | H | H | H | H | $CH_2CH_2CH_2NHCONHCH_2Me$ | 396 |
| 4-260 | H | H | H | H | H | $CH_2CH_2CH_2NHCONHCH_2CH_2Me$ | 410 |
| 4-261 | H | H | H | H | H | $CH_2CH_2CH_2NHCONHCHMe_2$ | 410 |
| 4-262 | H | H | H | H | H | $CH_2CH_2CH_2NHCONHCMe_3$ | 424 |
| 4-263 | H | H | H | H | H | a-90 | 436 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-264 | H | H | H | H | H | a-91 | 450 |
| 4-265 | H | H | H | H | H | a-92 | 464 |
| 4-266 | H | H | H | H | H | a-93 | 478 |
| 4-267 | H | H | H | H | H | a-94 | 502 |
| 4-268 | H | H | H | H | H | $CH_2CH_2CH_2NHCONHC_6H_5$ | 444 |
| 4-269 | H | H | H | H | H | a-95 | 474 |
| 4-270 | H | H | H | H | H | a-96 | 460 |
| 4-271 | H | H | H | H | H | a-97 | 459 |
| 4-272 | H | H | H | H | H | a-98 | 487 |
| 4-273 | H | H | H | H | H | a-99 | 494 |
| 4-274 | H | H | H | H | H | a-100 | 494 |
| 4-275 | H | H | H | H | H | a-101 | 469 |
| 4-276 | H | H | H | H | H | a-102 | 473 |
| 4-277 | H | H | H | H | H | a-103 | 478 |
| 4-278 | H | H | H | H | H | $CH_2CH_2CH_2NHCONHCOC_6H_5$ | 472 |
| 4-279 | H | H | H | H | H | a-104 | 520 |
| 4-280 | H | H | H | H | H | a-105 | 520 |
| 4-281 | H | H | H | H | H | a-106 | 501 |
| 4-282 | H | H | H | H | H | a-107 | 488 |
| 4-283 | H | H | H | H | H | a-108 | 500 |
| 4-284 | H | H | H | H | H | a-109 | 450 |
| 4-285 | H | H | H | H | H | $CH_2CH_2CH_2NHCONHCMe_2CH_2CMe_3$ | 480 |
| 4-286 | H | H | H | H | H | a-110 | 463 |
| 4-287 | H | H | H | H | H | $CH_2CH_2CH_2N$ (Me) $CONHC_6H_5$ | 458 |
| 4-288 | H | H | H | H | H | $CH_2CH_2CH_2N$ (Me) CON (Me) $C_6H_5$ | 472 |
| 4-289 | H | H | H | H | H | $CH_2CH_2CH_2NHCONHCH_2CH_2OMe$ | 426 |
| 4-290 | H | H | H | H | H | $CH_2CH_2CH_2NHCONHCH_2CH_2NMe_2$ | 439 |
| 4-291 | H | H | H | H | H | $CH_2CH_2CH_2NHCON$ (Me) $CH_2CH_2OH$ | 426 |
| 4-292 | H | H | H | H | H | $CH_2CH_2CH_2NHCON$ $(CH_2CH_2OH)_2$ | 456 |
| 4-293 | H | H | H | H | H | a-111 | 437 |
| 4-294 | H | H | H | H | H | $CH_2CH_2CH_2NHCONMe_2$ | 396 |
| 4-295 | H | H | H | H | H | $CH_2CH_2NHCSNHCH_2Me$ | 398 |
| 4-296 | H | H | H | H | H | $CH_2CH_2NHCSNHCH_2CH_2Me$ | 412 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-297 | H | H | H | H | H | $CH_2CH_2NHCSNHCHMe_2$ | 412 |
| 4-298 | H | H | H | H | H | $CH_2CH_2NHCSNHCMe_3$ | 426 |
| 4-299 | H | H | H | H | H | a-112 | 372 |
| 4-300 | H | H | H | H | H | $CH_2CH_2NHCSNHCH_2CH_2OMe$ | 428 |
| 4-301 | H | H | H | H | H | $CH_2CH_2NHCSNHCH_2CH_2NMe_2$ | 441 |
| 4-302 | H | H | H | H | H | $CH_2CH_2NHCSN(Me)CH_2CH_2OH$ | 428 |
| 4-303 | H | H | H | H | H | $CH_2CH_2NHCSN(CH_2CH_2OH)_2$ | 458 |
| 4-304 | H | H | H | H | H | a-113 | 439 |
| 4-305 | H | H | H | H | H | $CH_2CH_2NHCSNMe_2$ | 398 |
| 4-306 | H | H | H | H | H | $CH_2CH_2NHCOOMe$ | 369 |
| 4-307 | H | H | H | H | H | $CH_2CH_2NHCOOCH_2Me$ | 383 |
| 4-308 | H | H | H | H | H | $CH_2CH_2NHCOOCH_2CH_2Me$ | 397 |
| 4-309 | H | H | H | H | H | $CH_2CH_2NHCOOCHMe_2$ | 397 |
| 4-310 | H | H | H | H | H | a-114 | 437 |
| 4-311 | H | H | H | H | H | $CH_2CH_2NHCOOC_6H_5$ | 431 |
| 4-312 | H | H | H | H | H | $CH_2CH_2NHCOOCH_2CH_2OH$ | 399 |
| 4-313 | H | H | H | H | H | $CH_2CH_2NHCOOCH_2CH_2OMe$ | 413 |
| 4-314 | H | H | H | H | H | a-115 | 392 |
| 4-315 | H | H | H | H | H | a-116 | 392 |
| 4-316 | H | H | H | H | H | a-117 | 379 |
| 4-317 | H | H | H | H | H | a-118 | 393 |
| 4-318 | H | H | H | H | H | a-119 | 368 |
| 4-319 | H | H | H | H | H | a-120 | 393 |
| 4-320 | H | H | H | H | H | $COC_6H_5$ | 372 |
| 4-321 | Cl | H | H | H | H | H | 302 |
| 4-322 | Br | H | H | H | H | H | 346 |
| 4-323 | OH | H | H | H | H | H | 284 |
| 4-324 | Me | H | H | H | H | H | 282 |
| 4-325 | $CH=CH_2$ | H | H | H | H | H | 294 |
| 4-326 | $C\equiv CC_6H_5$ | H | H | H | H | H | 368 |
| 4-327 | OMe | H | H | H | H | H | 298 |
| 4-328 | $NH_2$ | H | H | H | H | H | 283 |
| 4-329 | NHMe | H | H | H | H | H | 297 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-330 | $NMe_2$ | H | H | H | H | H | 311 |
| 4-331 | $NHC_6H_5$ | H | H | H | H | H | 359 |
| 4-332 | OH | H | H | H | H | Me | 298 |
| 4-333 | OH | H | H | H | H | a-1 | 366 |
| 4-334 | OH | H | H | H | H | a-2 | 404 |
| 4-335 | OH | H | H | H | H | $C_6H_5$ | 360 |
| 4-336 | OH | H | H | H | H | $CH_2COOH$ | 342 |
| 4-337 | OH | H | H | H | H | $CH_2CH_2COOH$ | 356 |
| 4-338 | OH | H | H | H | H | $CH_2CN$ | 323 |
| 4-339 | OH | H | H | H | H | $CH_2CH_2CN$ | 337 |
| 4-340 | OH | H | H | H | H | $CH_2CH_2OH$ | 328 |
| 4-341 | OH | H | H | H | H | $CH(CH_2OH)_2$ | 358 |
| 4-342 | OH | H | H | H | H | a-3 | 382 |
| 4-343 | OH | H | H | H | H | $CH_2CH_2OMe$ | 342 |
| 4-344 | OH | H | H | H | H | $CH_2CH_2OCOCMe_3$ | 412 |
| 4-345 | OH | H | H | H | H | $CH_2CH_2SCOMe$ | 386 |
| 4-346 | OH | H | H | H | H | $CH_2CH_2SH$ | 344 |
| 4-347 | OH | H | H | H | H | $CH_2CH_2NH_2$ | 327 |
| 4-348 | OH | H | H | H | H | $CH_2CH_2CH_2NH_2$ | 341 |
| 4-349 | OH | H | H | H | H | $CH_2CH_2CH_2NHMe$ | 355 |
| 4-350 | OH | H | H | H | H | $CH\ (CH_2NHCOMe)_2$ | 440 |
| 4-351 | OH | H | H | H | H | $CH(CH_2NH_2)_2$ | 356 |
| 4-352 | OH | H | H | H | H | $CH_2CH_2NHCOMe$ | 369 |
| 4-353 | OH | H | H | H | H | a-4 | 382 |
| 4-354 | OH | H | H | H | H | $CH_2CH_2NHCOCH_2NHCOMe$ | 426 |
| 4-355 | OH | H | H | H | H | $CH_2CH_2NHCOCH_2NMe_2$ | 412 |
| 4-356 | OH | H | H | H | H | $CH_2CHNHSO_2Me$ | 405 |
| 4-357 | OH | H | H | H | H | a-5 | 381 |
| 4-358 | OH | H | H | H | H | a-6 | 423 |
| 4-359 | OH | H | H | H | H | $CH_2CH_2CH_2NHCONHCH_2CH_2OH$ | 428 |
| 4-360 | OH | H | H | H | H | $CH_2CH_2CH_2NBCONHBn$ | 474 |
| 4-361 | OH | H | H | H | H | $CH_2CH_2NHCSNHCH_2CH_2OH$ | 430 |
| 4-362 | OH | H | H | H | H | $CH_2CH_2NHCSNHC_6H_5$ | 462 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-363 | OH | H | H | H | H | $CH_2CH_2NHCOOCH_2CH_2NMe_2$ | 442 |
| 4-364 | OH | H | H | H | H | a-7 | 439 |
| 4-365 | OH | H | H | H | H | a-8 | 326 |
| 4-366 | OH | H | H | H | H | a-9 | 381 |
| 4-367 | OH | H | H | H | H | COMe | 326 |
| 4-368 | OH | H | H | H | H | a-10 | 338 |
| 4-369 | OH | H | H | H | H | a-11 | 352 |
| 4-370 | OH | H | H | H | H | a-12 | 366 |
| 4-371 | OH | H | H | H | H | Bn | 374 |
| 4-372 | OH | H | H | H | H | a-13 | 408 |
| 4-373 | OH | H | H | H | H | a-14 | 390 |
| 4-374 | OH | H | H | H | H | a-15 | 434 |
| 4-375 | OH | H | H | H | H | a-16 | 448 |
| 4-376 | OH | H | H | H | H | a-17 | 464 |
| 4-377 | OH | H | H | H | H | a-18 | 441 |
| 4-378 | OH | H | H | H | H | a-19 | 459 |
| 4-379 | OH | H | H | H | H | a-20 | 450 |
| 4-380 | OH | H | H | H | H | a-21 | 389 |
| 4-381 | OH | H | H | H | H | a-22 | 403 |
| 4-382 | OH | H | H | H | H | a-23 | 417 |
| 4-383 | OH | H | H | H | H | a-24 | 433 |
| 4-384 | OH | H | H | H | H | a-25 | 447 |
| 4-385 | OH | H | H | H | H | a-26 | 477 |
| 4-386 | OH | H | H | H | H | a-27 | 431 |
| 4-387 | OH | H | H | H | H | a-28 | 467 |
| 4-388 | OH | H | H | H | H | a-29 | 476 |
| 4-389 | OH | H | H | H | H | a-30 | 418 |
| 4-390 | OH | H | H | H | H | a-31 | 434 |
| 4-391 | OH | H | H | H | H | a-32 | 404 |
| 4-392 | OH | H | H | H | H | a-33 | 404 |
| 4-393 | OH | H | H | H | H | a-34 | 434 |
| 4-394 | OH | H | H | H | H | a-35 | 434 |
| 4-395 | OH | H | H | H | H | a-36 | 464 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-396 | OH | H | H | H | H | a-37 | 464 |
| 4-397 | OH | H | H | H | H | a-38 | 362 |
| 4-398 | OH | H | H | H | H | a-39 | 364 |
| 4-399 | OH | H | H | H | H | a-40 | 380 |
| 4-400 | OH | H | H | H | H | a-41 | 432 |
| 4-401 | OH | H | H | H | H | a-42 | 419 |
| 4-402 | OH | H | H | H | H | a-43 | 375 |
| 4-403 | OH | H | H | H | H | a-44 | 361 |
| 4-404 | OH | H | H | H | H | a-45 | 376 |
| 4-405 | OH | H | H | H | H | $CH_2CH_2CH_2COOH$ | 370 |
| 4-406 | OH | H | H | H | H | $CH_2CH_2CH_2CH_2COOH$ | 384 |
| 4-407 | OH | H | H | H | H | $CH_2CH_2CH_2CN$ | 351 |
| 4-408 | OH | H | H | H | H | $CH_2CH_2CH_2CH_2CN$ | 365 |
| 4-409 | OH | H | H | H | H | $CH_2CH$ (Me) OH | 342 |
| 4-410 | OH | H | H | H | H | CH(Me)$CH_2OH$ | 342 |
| 4-411 | OH | H | H | H | H | $CH_2CH_2CH_2OH$ | 342 |
| 4-412 | OH | H | H | H | H | $CH_2CH$ (OH) $CH_2OH$ | 358 |
| 4-413 | OH | H | H | H | H | $CH_2CH$ ($NH_2$) $CH_2OH$ | 357 |
| 4-414 | OH | H | H | H | H | $CH_2CH$(NHMe)$CH_2OH$ | 371 |
| 4-415 | OH | H | H | H | H | $CH_2CH(NMe_2)CH_2OH$ | 385 |
| 4-416 | OH | H | H | H | H | $CH_2CH$ (Me) $CH_2OH$ | 356 |
| 4-417 | OH | H | H | H | H | $CH_2CH_2CH_2CH_2OH$ | 356 |
| 4-418 | OH | H | H | H | H | $CH_2CH_2CH_2CH_2CH_2OH$ | 370 |
| 4-419 | OH | H | H | H | H | a-46 | 496 |
| 4-420 | OH | H | H | H | H | CH ($CH_2OH$) $CH_2CH_2NHCONHC_6H_5$ | 490 |
| 4-421 | OH | H | H | H | H | a-47 | 482 |
| 4-422 | OH | H | H | H | H | $CH_2CH$ (OH) $CH_2NHCONHC_6H_5$ | 476 |
| 4-423 | OH | H | H | H | H | $CH_2CH_2OCONHMe$ | 385 |
| 4-424 | OH | H | H | H | H | $CH_2CH_2OCONHC_6H_5$ | 447 |
| 4-425 | OH | H | H | H | H | $CH_2CH_2CH_2OCONHMe$ | 399 |
| 4-426 | OH | H | H | H | H | a-48 | 467 |
| 4-427 | OH | H | H | H | H | $CH_2CH_2CH_2OCONHC_6H_5$ | 461 |
| 4-428 | OH | H | H | H | H | $CH_2CH_2CH_2CH_2OCONHMe$ | 413 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-429 | OH | H | H | H | H | a-49 | 481 |
| 4-430 | OH | H | H | H | H | $CH_2CH_2CH_2CH_2OCONHC_6H_5$ | 475 |
| 4-431 | OH | H | H | H | H | $CH_2CH_2CH_2SH$ | 358 |
| 4-432 | OH | H | H | H | H | $CH_2CH_2CH_2CH_2SH$ | 372 |
| 4-433 | OH | H | H | H | H | $CH_2CH_2SCOC_6H_5$ | 448 |
| 4-434 | OH | H | H | H | H | $CH_2CH_2CH_2SCOMe$ | 400 |
| 4-435 | OH | H | H | H | H | $CH_2CH_2CH_2SCOC_6H_5$ | 462 |
| 4-436 | OH | H | H | H | H | $CH_2CH_2CH_2OMe$ | 356 |
| 4-437 | OH | H | H | H | H | $CH_2CH_2CH_2CH_2OMe$ | 370 |
| 4-438 | OH | H | H | H | H | $CH_2CH_2CH_2CH_2CH_2OMe$ | 384 |
| 4-439 | OH | H | H | H | H | $CH_2CH_2OCOMe$ | 370 |
| 4-440 | OH | H | H | H | H | $CH_2CH_2CH_2OCOMe$ | 384 |
| 4-441 | OH | H | H | H | H | $CH_2CH_2CH_2CH_2OCOMe$ | 398 |
| 4-442 | OH | H | H | H | H | $CH_2CH_2OCOCH_2Me$ | 384 |
| 4-443 | OH | H | H | H | H | $CH_2CH_2CH_2OCOCH_2Me$ | 398 |
| 4-444 | OH | H | H | H | H | $CH_2CH_2CH_2CH_2OCOCH_2Me$ | 412 |
| 4-445 | OH | H | H | H | H | $CH_2CH_2OCOCHMe_2$ | 398 |
| 4-446 | OH | H | H | H | H | $CH_2CH_2CH_2OCOCHMe_2$ | 412 |
| 4-447 | OH | H | H | H | H | $CH_2CH_2CH_2CH_2OCOCHMe_2$ | 426 |
| 4-448 | OH | H | H | H | H | $CH_2CH_2CH_2OCOCMe_3$ | 426 |
| 4-449 | OH | H | H | H | H | $CH_2CH_2CH_2CH_2OCOCMe_3$ | 440 |
| 4-450 | OH | H | H | H | H | $CH_2CH_2OCOC_6H_5$ | 432 |
| 4-451 | OH | H | H | H | H | $CH_2CH_2CH_2OCOC_6H_5$ | 446 |
| 4-452 | OH | H | H | H | H | $CH_2CH_2CH_2CH_2OCOC_6H_5$ | 460 |
| 4-453 | OH | H | H | H | H | $CH_2CH_2OCOCH_2OH$ | 386 |
| 4-454 | OH | H | H | H | H | $CH_2CH_2OCOCH_2NH_2$ | 385 |
| 4-455 | OH | H | H | H | H | $CH_2CH_2OCH_2CH_2OMe$ | 386 |
| 4-456 | OH | H | H | H | H | $CH_2CH_2OCH_2CH_2OH$ | 372 |
| 4-457 | OH | H | H | H | H | $CH_2CH_2CH_2OCH_2CH_2OH$ | 386 |
| 4-458 | OH | H | H | H | H | $CH_2CH_2CH_2CH_2OCH_2CH_2OH$ | 400 |
| 4-459 | OH | H | H | H | H | $CH_2CH(CH_2OH)NH_2$ | 357 |
| 4-460 | OH | H | H | H | H | $CH_2CH\ (CH_2OH)\ NHCOMe$ | 399 |
| 4-461 | OH | H | H | H | H | a-50 | 482 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-462 | OH | H | H | H | H | $CH_2CH(CH_2OH)NHCONHC_6H_5$ | 476 |
| 4-463 | OH | H | H | H | H | $CH (CH_2OH) CH_2NH_2$ | 357 |
| 4-464 | OH | H | H | H | H | $CH (CH_2OH) CH_2NHCOMe$ | 399 |
| 4-465 | OH | H | H | H | H | a-51 | 482 |
| 4-466 | OH | H | H | H | H | $CH (CH_2OH) CH_2NHCONHC_6H_5$ | 476 |
| 4-467 | OH | H | H | H | H | $CH_2CH (OH) CH_2NH_2$ | 357 |
| 4-468 | OH | H | H | H | H | $CH_2CH(OH)CH_2NHMe$ | 371 |
| 4-469 | OH | H | H | H | H | $CH_2CH (OH) CH_2NMe_2$ | 385 |
| 4-470 | OH . | H | H | H | H | $CH_2CH_2CH_2CH_2NH_2$ | 355 |
| 4-471 | OH | H | H | H | H | $CH_2CH_2CH_2CH_2CH_2NH_2$ | 369 |
| 4-472 | OH | H | H | H | H | $CH_2CH_2NHMe$ | 341 |
| 4-473 | OH | H | H | H | H | $CH_2CH_2CH_2CH_2NHMe$ | 369 |
| 4-474 | OH | H | H | H | H | $CH_2CH_2CH_2CH_2CH_2NHMe$ | 383 |
| 4-475 | OH | H | H | H | H | $CH_2CH_2NMe_2$ | 355 |
| 4-476 | OH | H | H | H | H | $CH_2CH_2CH_2NMe_2$ | 369 |
| 4-477 | OH | H | H | H | H | $CH_2CH_2CH_2CH_2NMe_2$ | 383 |
| 4-478 | OH | H | H | H | H | $CH_2CH_2CH_2CH_2CH_2NMe_2$ | 397 |
| 4-479 | OH | H | H | H | H | $CH_2CH_2NHCH_2CH_2OH$ | 371 |
| 4-480 | OH | H | H | H | H | $CH_2CH_2CH_2NHCH_2CH_2OH$ | 385 |
| 4-481 | OH | H | H | H | H | $CH_2CH_2CH_2CH_2NHCH_2CH_2OH$ | 399 |
| 4-482 | OH | H | H | H | H | $CH_2CONH_2$ | 341 |
| 4-483 | OH | H | H | H | H | $CH_2CH_2CONH_2$ | 355 |
| 4-484 | OH | H | H | H | H | $CH_2CH_2CH_2CONH_2$ | 369 |
| 4-485 | OH | H | H | H | H | $CH_2CH_2NHCOCH_2Me$ | 383 |
| 4-486 | OH | H | H | H | H | $CH_2CH_2NHCOCH_2CH_2Me$ | 397 |
| 4-487 | OH | H | H | H | H | $CH_2CH_2NHCOCHMe_2$ | 397 |
| 4-488 | OH | H | H | H | H | $CH_2CH_2NHCOCMe_3$ | 411 |
| 4-489 | OH | H | H | H | H | $CH_2CH_2NHCOCH_2OH$ | 385 |
| 4-490 | OH | H | H | H | H | $CH_2CH_2NHCOCH_2OMe$ | 399 |
| 4-491 | OH | H | H | H | H | $CH_2CH_2NHCOC_6H_5$ | 431 |
| 4-492 | OH | H | H | H | H | a-52 | 432 |
| 4-493 | OH | H | H | H | H | a-53 | 437 |
| 4-494 | OH | H | H | H | H | a-54 | 421 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-495 | OH | H | H | H | H | a-55 | 433 |
| 4-496 | OH | H | H | H | H | $CH_2CH_2NHCOCH_2NHCOCH_2CHMe_2$ | 468 |
| 4-497 | OH | H | H | H | H | $CH_2CH_2NHCOCH_2NHCOC_6H_5$ | 488 |
| 4-498 | OH | H | H | H | H | $CH_2CH_2CH_2NHCOMe$ | 383 |
| 4-499 | OH | H | H | H | H | $CH_2CH_2CH_2NHCOCH_2Me$ | 397 |
| 4-500 | OH | H | H | H | H | $CH_2CH_2CH_2NHCOCH_2CH_2Me$ | 411 |
| 4-501 | OH | H | H | H | H | $CH_2CH_2CH_2NHCOCHMe_2$ | 411 |
| 4-502 | OH | H | H | H | H | $CH_2CH_2CH_2NHCOCMe_3$ | 425 |
| 4-503 | OH | H | H | H | H | $CH_2CH_2CH_2CH_2NHCOMe$ | 397 |
| 4-504 | OH | H | H | H | H | $CH_2CH_2CH_2CH_2NHCOCH_2Me$ | 411 |
| 4-505 | OH | H | H | H | H | $CH_2CH_2CH_2CH_2NHCOCH_2CH_2Me$ | 425 |
| 4-506 | OH | H | H | H | H | $CH_2CH_2CH_2CH_2NHCOCHMe_2$ | 425 |
| 4-507 | OH | H | H | H | H | $CH_2CH_2CH_2CH_2NHCOCMe_3$ | 439 |
| 4-508 | OH | H | H | H | H | $CH_2CH_2N$ (Me) COMe | 383 |
| 4-509 | OH | H | H | H | H | $CH_2CH_2N$ (Me) $COC_6H_5$ | 445 |
| 4-510 | OH | H | H | H | H | $CH_2CH_2CH_2N$ (Me) COMe | 397 |
| 4-511 | OH | H | H | H | H | $CH_2CH_2CH_2N$ (Me) $COC_6H_5$ | 459 |
| 4-512 | OH | H | H | H | H | $CH_2CH_2CH_2CH_2N$ (Me) COMe | 411 |
| 4-513 | OH | H | H | H | H | $CH_2CH_2N$ ($CH_2CH_2OH$) COMe | 413 |
| 4-514 | OH | H | H | H | H | $CH_2CH_2CH_2N$ ($CH_2CH_2OH$) COMe | 427 |
| 4-515 | OH | H | H | H | H | $CH_2CH_2N(CH_2CN)$ COMe | 408 |
| 4-516 | OH | H | H | H | H | $CH_2CH_2CH_2N$ ($CH_2CN$) COMe | 422 |
| 4-517 | OH | H | H | H | H | a-56 | 354 |
| 4-518 | OH | H | H | H | H | a-57 | 353 |
| 4-519 | OH | H | H | H | H | a-58 | 395 |
| 4-520 | OH | H | H | H | H | a-59 | 457 |
| 4-521 | OH | H | H | H | H | a-60 | 485 |
| 4-522 | OH | H | H | H | H | a-61 | 368 |
| 4-523 | OH | H | H | H | H | a-62 | 381 |
| 4-524 | OH | H | H | H | H | a-63 | 367 |
| 4-525 | OH | H | H | H | H | $CH_2CH_2NHSO_2C_6H_5$ | 467 |
| 4-526 | OH | H | H | H | H | $CH_2CH_2NHSO_2NMe_2$ | 434 |
| 4-527 | OH | H | H | H | H | $CH_2CH_2N$ (Me) $SO_2Me$ | 419 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-528 | OH | H | H | H | H | $CH_2CH_2N$ (Me) $SO_2C_6H_5$ | 481 |
| 4-529 | OH | H | H | H | H | $CH_2CH_2N$ (Me) $SO_2NMe_2$ | 448 |
| 4-530 | OH | H | H | H | H | $CH_2CH_2N$ ($CH_2CH_2OH$) $SO_2Me$ | 435 |
| 4-531 | OH | H | H | H | H | $CH_2CH_2CH_2NHSO_2Me$ | 419 |
| 4-532 | OH | H | H | H | H | $CH_2CH_2CH_2NHSO_2C_6H_5$ | 481 |
| 4-533 | OH | H | H | H | H | $CH_2CH_2CH_2NHSO_2NMe_2$ | 448 |
| 4-534 | OH | H | H | H | H | $CH_2CH_2CH_2N$ (Me) $SO_2Me$ | 433 |
| 4-535 | OH | H | H | H | H | $CH_2CH_2CH_2N(Me)SO_2C_6H_5$ | 495 |
| 4-536 | OH | H | H | H | H | $CH_2CH_2CH_2N$ (Me) $SO_2NMe_2$ | 462 |
| 4-537 | OH | H | H | H | H | $CH_2CH_2CH_2N$ ($CH_2CH_2OH$) $SO_2Me$ | 449 |
| 4-538 | OH | H | H | H | H | $CH_2CH_2CH_2CH_2NHSO_2Me$ | 433 |
| 4-539 | OH | H | H | H | H | $CH_2CH_2CH_2CH_2NHSO_2C_6H_5$ | 495 |
| 4-540 | OH | H | H | H | H | $CH_2CH_2CH_2CH_2NHSO_2NMe_2$ | 462 |
| 4-541 | OH | H | H | H | H | $CH_2CH_2CH_2CH_2N$ (Me) $SO_2Me$ | 447 |
| 4-542 | OH | H | H | H | H | $CH_2CH_2CH_2CH_2N$ (Me) $SO_2C_6H_5$ | 509 |
| 4-543 | OH | H | H | H | H | $CH_2CH_2CH_2CH_2N$ (Me) $SO_2NMe_2$ | 476 |
| 4-544 | OH | H | H | H | H | $CH_2CH_2CH_2CH_2N$ ($CH_2CH_2OH$) $SO_2Me$ | 463 |
| 4-545 | OH | H | H | H | H | a-64 | 431 |
| 4-546 | OH | H | H | H | H | a-65 | 395 |
| 4-547 | OH | H | H | H | H | a-66 | 459 |
| 4-548 | OH | H | H | H | H | a-67 | 409 |
| 4-549 | OH | H | H | H | H | $CH_2CH_2NHCONHMe$ | 384 |
| 4-550 | OH | H | H | H | H | $CH_2CH_2NHCONHCH_2Me$ | 398 |
| 4-551 | OH | H | H | H | H | $CH_2CH_2NHCONHCH_2CH_2Me$ | 412 |
| 4-552 | OH | H | H | H | H | $CH_2CH_2NHCONHCHMe_2$ | 412 |
| 4-553 | OH | H | H | H | H | $CH_2CH_2NHCONHCMe_3$ | 426 |
| 4-554 | OH | H | H | H | H | a-68 | 438 |
| 4-555 | OH | H | H | H | H | a-69 | 452 |
| 4-556 | OH | H | H | H | H | a-70 | 466 |
| 4-557 | OH | H | H | H | H | a-71 | 480 |
| 4-558 | OH | H | H | H | H | a-72 | 504 |
| 4-559 | OH | H | H | H | H | $CH_2CH_2NHCONHC_6H_5$ | 446 |
| 4-560 | OH | H | H | H | H | a-73 | 476 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-561 | OH | H | H | H | H | a-74 | 462 |
| 4-562 | OH | H | H | H | H | a-75 | 461 |
| 4-563 | OH | H | H | H | H | a-76 | 489 |
| 4-564 | OH | H | H | H | H | a-77 | 496 |
| 4-565 | OH | H | H | H | H | a-78 | 496 |
| 4-566 | OH | H | H | H | H | a-79 | 471 |
| 4-567 | OH | H | H | H | H | a-80 | 475 |
| 4-568 | OH | H | H | H | H | a-81 | 480 |
| 4-569 | OH | H | H | H | H | $CH_2CH_2NHCONHCOC_6H_5$ | 474 |
| 4-570 | OH | H | H | H | H | $CH_2CH_2NHCONHSO_2C_6H_5$ | 510 |
| 4-571 | OH | H | H | H | H | a-82 | 388 |
| 4-572 | OH | H | H | H | H | a-83 | 388 |
| 4-573 | OH | H | H | H | H | a-84 | 503 |
| 4-574 | OH | H | H | H | H | a-85 | 490 |
| 4-575 | OH | H | H | H | H | a-86 | 502 |
| 4-576 | OH | H | H | H | H | a-87 | 452 |
| 4-577 | OH | H | H | H | H | $CH_2CH_2NHCONHCMe_2CH_2CMe_3$ | 482 |
| 4-578 | OH | H | H | H | H | a-88 | 465 |
| 4-579 | OH | H | H | H | H | $CH_2CH_2N$ (Me) $CONHC_6H_5$ | 460 |
| 4-580 | OH | H | H | H | H | $CH_2CH_2N$ (Me) CON (Me) $C_6H_5$ | 474 |
| 4-581 | OH | H | H | H | H | $CH_2CH_2NHCONHCH_2CH_2OH$ | 414 |
| 4-582 | OH | H | H | H | H | $CH_2CH_2NHCONHCH_2CH_2OMe$ | 428 |
| 4-583 | OH | H | H | H | H | $CH_2CH_2NHCONHCH_2CH_2NMe_2$ | 441 |
| 4-584 | OH | H | H | H | H | $CH_2CH_2NHCON(Me)CH_2CH_2OH$ | 428 |
| 4-585 | OH | H | H | H | H | $CH_2CH_2NHCON$ $(CH_2CH_2OH)_2$ | 458 |
| 4-586 | OH | H | H | H | H | a-89 | 439 |
| 4-587 | OH | H | H | H | H | $CH_2CH_2NHCONMe_2$ | 398 |
| 4-588 | OH | H | H | H | H | $CH_2CH_2CH_2NHCONHMe$ | 398 |
| 4-589 | OH | H | H | H | H | $CH_2CH_2CH_2NHCONHCH_2Me$ | 412 |
| 4-590 | OH | H | H | H | H | $CH_2CH_2CH_2NHCONHCH_2CH_2Me$ | 426 |
| 4-591 | OH | H | H | H | H | $CH_2CH_2CH_2NHCONHCHMe_2$ | 426 |
| 4-592 | OH | H | H | H | H | $CH_2CH_2CH_2NHCONHCMe_3$ | 440 |
| 4-593 | OH | H | H | H | H | a-90 | 452 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-594 | OH | H | H | H | H | a-91 | 466 |
| 4-595 | OH | H | H | H | H | a-92 | 480 |
| 4-596 | OH | H | H | H | H | a-93 | 494 |
| 4-597 | OH | H | H | H | H | a-94 | 518 |
| 4-598 | OH | H | H | H | H | $CH_2CH_2CH_2NHCONHC_6H_5$ | 460 |
| 4-599 | OH | H | H | H | H | a-95 | 490 |
| 4-600 | OH | H | H | H | H | a-96 | 476 |
| 4-601 | OH | H | H | H | H | a-97 | 475 |
| 4-602 | OH | H | H | H | H | a-98 | 503 |
| 4-603 | OH | H | H | H | H | a-99 | 510 |
| 4-604 | OH | H | H | H | H | a-100 | 510 |
| 4-605 | OH | H | H | H | H | a-101 | 485 |
| 4-606 | OH | H | H | H | H | a-102 | 489 |
| 4-607 | OH | H | H | H | H | a-103 | 494 |
| 4-608 | OH | H | H | H | H | $CH_2CH_2CH_2NHCONHCOC_6H_5$ | 488 |
| 4-609 | OH | H | H | H | H | a-104 | 536 |
| 4-610 | OH | H | H | H | H | a-105 | 536 |
| 4-611 | OH | H | H | H | H | a-106 | 517 |
| 4-612 | OH | H | H | H | H | a-107 | 504 |
| 4-613 | OH | H | H | H | H | a-108 | 516 |
| 4-614 | OH | H | H | H | H | a-109 | 466 |
| 4-615 | OH | H | H | H | H | $CH_2CH_2CH_2NHCONHCMe_2CH_2CMe_3$ | 496 |
| 4-616 | OH | H | H | H | H | a-110 | 479 |
| 4-617 | OH | H | H | H | H | $CH_2CH_2CH_2N$ (Me) $CONHC_6H_5$ | 474 |
| 4-618 | OH | H | H | H | H | $CH_2CH_2CH_2N$ (Me) CON (Me) $C_6H_5$ | 488 |
| 4-619 | OH | H | H | H | H | $CH_2CH_2CH_2NHCONHCH_2CH_2OMe$ | 442 |
| 4-620 | OH | H | H | H | H | $CH_2CH_2CH_2NHCONHCH_2CH_2NMe_2$ | 455 |
| 4-621 | OH | H | H | H | H | $CH_2CH_2CH_2NHCON$ (Me) $CH_2CH_2OH$ | 442 |
| 4-622 | OH | H | H | H | H | $CH_2CH_2CH_2NHCON$ $(CH_2CH_2OH)_2$ | 472 |
| 4-623 | OH | H | H | H | H | a-111 | 453 |
| 4-624 | OH | H | H | H | H | $CH_2CH_2CH_2NHCONMe_2$ | 412 |
| 4-625 | OH | H | H | H | H | $CH_2CH_2NHCSNHCH_2Me$ | 414 |
| 4-626 | OH | H | H | H | H | $CH_2CH_2NHCSNHCH_2CH_2Me$ | 428 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-627 | OH | H | H | H | H | $CH_2CH_2NHCSNHCHMe_2$ | 428 |
| 4-628 | OH | H | H | H | H | $CH_2CH_2NHCSNHCMe_3$ | 442 |
| 4-629 | OH | H | H | H | H | a-112 | 388 |
| 4-630 | OH | H | H | H | H | $CH_2CH_2NHCSNHCH_2CH_2OMe$ | 444 |
| 4-631 | OH | H | H | H | H | $CH_2CH_2NHCSNHCH_2CH_2NMe_2$ | 457 |
| 4-632 | OH | H | H | H | H | $CH_2CH_2NHCSN$ (Me) $CH_2CH_2OH$ | 444 |
| 4-633 | OH | H | H | H | H | $CH_2CH_2NHCSN(CH_2CH_2OH)_2$ | 474 |
| 4-634 | OH | H | H | H | H | a-113 | 455 |
| 4-635 | OH | H | H | H | H | $CH_2CH_2NHCSNMe_2$ | 414 |
| 4-636 | OH | H | H | H | H | $CH_2CH_2NHCOOMe$ | 385 |
| 4-637 | OH | H | H | H | H | $CH_2CH_2NHCOOCH_2Me$ | 399 |
| 4-638 | OH | H | H | H | H | $CH_2CH_2NHCOOCH_2CH_2Me$ | 413 |
| 4-639 | OH | H | H | H | H | $CH_2CH_2NHCOOCHMe_2$ | 413 |
| 4-640 | OH | H | H | H | H | a-114 | 453 |
| 4-641 | OH | H | H | H | H | $CH_2CH_2NHCOOC_6H_5$ | 447 |
| 4-642 | OH | H | H | H | H | $CH_2CH_2NHCOOCH_2CH_2OH$ | 415 |
| 4-643 | OH | H | H | H | H | $CH_2CH_2NHCOOCH_2CH_2OMe$ | 429 |
| 4-644 | OH | H | H | H | H | a-115 | 408 |
| 4-645 | OH | H | H | H | H | a-116 | 408 |
| 4-646 | OH | H | H | H | H | a-117 | 395 |
| 4-647 | OH | H | H | H | H | a-118 | 409 |
| 4-648 | OH | H | H | H | H | a-119 | 384 |
| 4-649 | OH | H | H | H | H | a-120 | 409 |
| 4-650 | OH | H | H | H | H | $COC_6H_5$ | 388 |
| 4-651 | H | Cl | H | H | H | H | 302 |
| 4-652 | H | Br | H | H | H | H | 346 |
| 4-653 | H | OH | H | H | H | H | 284 |
| 4-654 | H | Me | H | H | H | H | 282 |
| 4-655 | H | $CH=CH_2$ | H | H | H | H | 294 |
| 4-656 | H | $C\equiv CC_6H_5$ | H | H | H | H | 368 |
| 4-657 | H | OMe | H | H . | H | H | 298 |
| 4-658 | H | $NH_2$ | H | H | H | H | 283 |
| 4-659 | H | NHMe | H | H | H | H | 297 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-660 | H | $NMe_2$ | H | H | H | H | 311 |
| 4-661 | H | $NHC_6H_5$ | H | H | H | H | 359 |
| 4-662 | H | H | Cl | H | H | H | 302 |
| 4-663 | H | H | Br | H | H | H | 346 |
| 4-664 | H | H | OH | H | H | H | 284 |
| 4-665 | H | H | Me | H | H | H | 282 |
| 4-666 | H | H | $CH=CH_2$ | H | H | H | 294 |
| 4-667 | H | H | $C\equiv CC_6H_5$ | H | H | H | 368 |
| 4-668 | H | H | 0Me | H | H | H | 298 |
| 4-669 | H | H | $NH_2$ | H | H | H | 283 |
| 4-670 | H | H | NHMe | H | H | H | 297 |
| 4-671 | H | H | $NMe_2$ | H | H | H | 311 |
| 4-672 | H | H | $NHC_6H_5$ | H | H | H | 359 |
| 4-673 | H | H | H | Cl | H | H | 302 |
| 4-674 | H | H | H | Br | H | H | 346 |
| 4-675 | H | H | H | OH | H | H | 284 |
| 4-676 | H | H | H | Me | H | H | 282 |
| 4-677 | H | H | H | $CH=CH_2$ | H | H | 294 |
| 4-678 | H | H | H | $C\equiv CC_6H_5$ | H | H | 368 |
| 4-679 | H | H | H | OMe | H | H | 298 |
| 4-680 | H | H | H | $NH_2$ | H | H | 283 |
| 4-681 | H | H | H | NHMe | H | H | 297 |
| 4-682 | H | H | H | $NMe_2$ | H | H | 311 |
| 4-683 | H | H | H | $NHC_6H_5$ | H | H | 359 |
| 4-684 | H | H | H | H | Cl | H | 302 |
| 4-685 | H | H | H | H | Br | H | 346 |
| 4-686 | H | H | H | H | OH | H | 284 |
| 4-687 | H | H | H | H | Me | H | 282 |
| 4-688 | H | H | H | H | $CH=CH_2$ | H | 294 |
| 4-689 | H | H | H | H | $C\equiv CC_6H_5$ | H | 368 |
| 4-690 | H | H | H | H | OMe | H | 298 |
| 4-691 | H | H | H | H | $NH_2$ | H | 283 |
| 4-692 | H | H | H | H | NHMe | H | 297 |

(continued)

| Exp. No. | $R^1$ | $R^5$ | $R^8$ | $R^7$ | $R^6$ | $A^{61}$ | Mass ($MH^+$) |
|---|---|---|---|---|---|---|---|
| 4-693 | H | H | H | H | $NMe_2$ | H | 311 |
| 4-694 | H | H | H | H | $NHC_6H_5$ | H | 359 |

Example 5-1: 1-Amino-4-(2,3-dihydro-1H-1,5-diazaphenalen-1-yl)cyclohexane

Step A

5-(1,4-Dioxa-spiro[4.5]dec-8-yl)amino-4-vinylisoquinoline (Intermediate 41)

By using 5-amino-4-vinylisoquinoline (Intermediate 1) obtained in Example 1-1, Step A, and 1,4-cyclohexanedione monoethylene ketal (Tokyo Kasei Kogyo), the title compound was obtained according to the method described in Example 1-1, Step B.
MS (m/z): 311 (MH+)

Step B

2,3-Dihydro-1-(1,4-dioxa-spiro[4.5]dec-8-yl)-1,5-diazaphenalene (Intermediate 42)

By using 5-(1,4-dioxa-spiro[4.5]dec-8-yl)amino-4-vinylisoquinoline (Intermediate 41) obtained in Step A, the title compound was obtained according to the method described in Example 1-1, Step C.
MS (m/z): 311 (MH+)

Step C

4-(2,3-Dihydro-1H-1,5-diazaphenalen-1-yl)cyclohexanone (Intermediate 43)

A solution of 2,3-dihydro-1-(1,4-dioxa-spiro[4.5]dec-8-yl)-1,5-diazaphenalene (Intermediate 42, 3.60 g) obtained in Step B in 3 N hydrochloric acid (68 ml) was refluxed by heating for 18 hours. The reaction mixture was left to cool to room temperature, and then carefully added with 2 N aqueous sodium hydroxide (50 ml) under cooling on an ice bath for neutralization. The reaction mixture was extracted with ethyl acetate (100 ml), and then the organic layer was washed twice with saturated brine (150 ml for each time), and dried over anhydrous magnesium sulfate. The organic layer was filtered through Celite, and then the solvent was evaporated under reduced pressure. The residue was purified by silica gel column chromatography (n-hexane:ethyl acetate = 1:2) to obtain the title compound (2.85 g). MS (m/z): 267 (MH+)

Step D

1-Benzylamino-4-(2,3-dihydro-1H-1,5-diazaphenalen-1-yl)cyclohexane (Intermediate 44)

A solution of 4-(2,3-dihydro-1H-1,5-diazaphenalen-1-yl)cyclohexanone (Intermediate 43, 133 mg) obtained in Step C, benzylamine (220 mg, Tokyo Kasei Kogyo), and glacial acetic acid (57 μl) in 1,2-dichloroethane (5 ml) was added with sodium triacetoxyborohydride (212 mg), and stirred at room temperature for 15 hours. The reaction mixture was washed with saturated aqueous sodium hydrogencarbonate (5 ml), and dried over anhydrous magnesium sulfate. The organic layer was filtered through Celite, and then the solvent was evaporated under reduced pressure. The residue was purified by silica gel column chromatography (chloroform:methanol = 10:1) to obtain the title compound (155 mg).
MS (m/z) : 358 (MH+)

Step E

1-Amino-4-(2,3-dihydro-1H-1,5-diazaphenalen-1-yl)cyclohexane

A solution of 1-benzylamino-4-(2,3-dihydro-1H-1,5-diazaphenalen-1-yl)cyclohexane (Intermediate 44, 90 mg) obtained in Step D in methanol (3 ml) was added with 10% palladium/carbon catalyst (10 mg, Wako Pure Chemical Industries), and stirred at room temperature for 12 hours under hydrogen atmosphere of ordinary pressure. The catalyst was separated by filtration through Celite, and the solvent was evaporated under reduced pressure. The residue was purified by silica gel column chromatography (chloroform:methanol = 10:1) to obtain the title compound (32 mg).
MS (m/z): 268 (MH+)

Example 5-2: 5-[4-(2,3-Dihydro-1H-1,5-diazaphenalen-1-yl)cyclohexylamino]pentanoic acid

By using 1-amino-4-(2,3-dihydro-1H-1,5-diazaphenalen-1-yl)cyclohexane obtained in Example 5-1, alkylation

was performed with tert-butyl 5-bromopentanoate according to the method described in Example 1-3, and then deprotection was performed for the protective group according to the method described in Example 1-1, Step D to obtain the title compound as a hydrochloride.
MS (m/z): 368 (MH+)

Example 5-3: 5-[4-(2,3-Dihydro-1H-1,5-diazaphenalen-1-yl)cyclohexylamino]pentanol

By using 4-(2,3-dihydro-1H-1,5-diazaphenalen-1-yl)cyclohexanone (Intermediate 43) obtained in Example 5-1, Step C, reductive amination was performed with 5-amino-1-pentanol according to the method described in Example 1-12 to obtain the title compound.
MS (m/z): 354 (MH+)

Example 5-4: Thioacetic acid

S-{2-[4-(2,3-dihydro-1H-1,5-diazaphenalen-1-yl)cyclohexylamino]ethyl} ester

By using 4-(2,3-dihydro-1H-1,5-diazaphenalen-1-yl)cyclohexanone (Intermediate 43) obtained in Example 5-1, Step C, reductive amination was performed with thioacetic acid S-(2-aminoethyl) ester (Matrix) according to the method described in Example 1-12 to obtain the title compound.
MS (m/z): 370 (MH+)

Example 5-5:

2-[4-(2,3-Dihydro-1H-1,5-diazaphenalen-1-yl)cyclohexylamino]ethanethiol

Deprotection was performed for the acetyl group of thioacetic acid S-{2-[4-(2,3-dihydro-1H-1,5-diazaphenalen-1-yl)cyclohexylamino]ethyl} ester obtained in Example 5-4 according to the method described in Example 1-16 to obtain the title compound.
MS (m/z): 328 (MH+)

Example 5-6: 1-(Acetamino)-4-(2,3-dihydro-1H-1,5-diazaphenalen-1-yl)cyclohexane

By using 1-amino-4-(2,3-dihydro-1H-1,5-diazaphenalen-1-yl)cyclohexane obtained in Example 5-1, acetylation was performed with acetic anhydride according to the method described in Example 1-22 to obtain the title compound.
MS (m/z): 310 (MH+)

Example 5-7:

1-(Methanesulfonamino)-4-(2,3-dihydro-1H-1,5-diazaphenalen-1-yl)cyclohexane

By using 1-amino-4-(2,3-dihydro-1H-1,5-diazaphenalen-1-yl)cyclohexane obtained in Example 5-1, mesylation was performed with methanesulfonic anhydride according to the method described in Example 1-22 to obtain the title compound.
MS (m/z): 346 (MH+)

Example 5-8:

N-{3-[4-(2,3-Dihydro-1H-1,5-diazaphenalen-1-yl)cyclohexylamino]propyl}-N'-phenylurea

By using 4-(2,3-dihydro-1H-1,5-diazaphenalen-1-yl)cyclohexanone (Intermediate 43) obtained in Example 5-1, Step C, reductive amination was performed with tert-butyl N-(3-aminopropyl)carbamate (Tokyo Kasei Kogyo) according to the method described in Example 1-12" and then deprotection was performed for the protective group according to the method described in Example 1-1, Step D to obtain 1-[(3-aminopropyl)amino]-4-(2,3-dihydro-1H-1,5-diazaphenalen-1-yl)cyclohexane as a hydrochloride. Then, the resultant was reacted with 4-nitrophenyl carbonate and then with aniline according to the method described in Example 1-29 to obtain the title compound.
MS (m/z): 444 (MH+)

Example 5-9:

N-{3-[4-(2,3-Dihydro-1H-1,5-diazaphenalen-1-yl)cyclohexylamino]propyl}-N'-cyclohexylurea

By using 4-(2,3-dihydro-1H-1,5-diazaphenalen-1-yl)cyclohexanone (Intermediate 43) obtained in Example 5-1, Step C, reductive amination was performed with N-(3-aminopropyl)-N'-cyclohexylurea according to the method described in Example 5-3 to obtain the title compound.
MS (m/z): 450 (MH+)

Example 5-10: 4-(2,3-Dihydro-1H-1,5-diazaphenalen-1-yl)cyclohexanol

A solution of 4-(2,3-dihydro-1H-1,5-diazaphenalen-1-yl)cyclohexanone (Intermediate 43, 266 mg) obtained in Example 5-1, Step C in methanol (8 ml) was added with sodium borohydride (38 mg) under cooling on an ice bath, and stirred at the same temperature for 2 hours. The reaction mixture was carefully added with saturated brine (15 ml) under cooling on an ice bath, and stirred at the same temperature for 1 hour. The reaction mixture was returned to room temperature, and then extracted 3 times with ethyl acetate (10 ml), and the combined organic layer was washed with saturated brine (20 ml), and then dried over anhydrous magnesium sulfate. The organic layer was filtered through Celite, and then the solvent was evaporated under reduced pressure. The residue was purified by silica gel column chromatography (chloroform:methanol = 10:1) to obtain the title compound (232 mg).
MS (m/z): 269 (MH+)

Examples 5-11 to 5-16

The compounds of Examples 5-11 to 5-16 were obtained by using 4-(2,3-dihydro-1H-1,5-diazaphenalen-1-yl) cyclohexanone (Intermediate 43) obtained in Example 5-1, Step C to perform reductive amination according to the method described in Example 5-3.

Example 5-17

The compound of Example 5-17 was obtained by using 1-amino-4-(2,3-dihydro-1H-1,5-diazaphenalen-1-yl) cyclohexane obtained in Example 5-1 to perform esterification according to the method described in Example 1-22.

Example 5-18

The compound of Example 5-18 was obtained by using 1-amino-4-(2,3-dihydro-1H-1,5-diazaphenalen-1-yl) cyclohexane obtained in Example 5-1 to perform sulfonylation according to the method described in Example 1-22.

Examples 5-19 to 5-32

The compounds of Examples 5-19 to 5-32 were obtained by using 1-amino-4-(2,3-dihydro-1H-1,5-diazaphenalen-1-yl)cyclohexane obtained in Example 5-1 to perform alkylation according to the method described in Example 5-2, or by using 4-(2,3-dihydro-1H-1,5-diazaphenalen-1-yl)cyclohexanone (Intermediate 43) obtained in Example 5-1, Step C to perform reductive amination according to the method described in Example 5-3.

Examples 5-33 to 5-41

The compounds of Examples 5-33 to 5-41 were obtained by using 2-[4-(2,3-dihydro-1H-1,5-diazaphenalen-1-yl)cyclohexylamino]ethanol obtained in Example 5-26 to perform active esterification, and then reacting the resultant with an amine according to the method described in Example 1-34.

Example 5-42

The compound of Example 5-42 was obtained by performing deprotection for the acetyl group of thioacetic acid S-{3-[4-(2,3-dihydro-1H-1,5-diazaphenalen-1-yl)cyclohexylamino]propyl} ester obtained in Example 5-44 mentioned below according to the method described in Example 1-16.

Examples 5-43 to 5-45

The compounds of Examples 5-43 to 5-45 were obtained by using 4-(2,3-dihydro-1H-1,5-diazaphenalen-1-yl) cyclohexanone (Intermediate 43) obtained in Example 5-1, Step C to perform reductive amination according to the method described in Example 1-12.

Examples 5-46 to 5-49

The compounds of Examples 5-46 to 5-49 were obtained by using 1-amino-4-(2,3-dihydro-1H-1,5-diazaphenalen-1-yl)cyclohexane obtained in Example 5-1 to perform alkylation according to the method described in Example 5-2.

Examples 5-50 to 5-53

The compounds of Examples 5-50 to 5-53 were obtained by using 4-(2,3-dihydro-1H-1,5-diazaphenalen-1-yl) cyclohexanone (Intermediate 43) obtained in Example 5-1, Step C to perform reductive amination according to the method described in Example 1-12.

Example 5-54

The compound of Example 5-54 was obtained by using 4-(2,3-dihydro-1H-1,5-diazaphenalen-1-yl)cyclohexanone (Intermediate 43) obtained in Example 5-1, Step C to perform reductive amination, and then acetylation according to the methods described in Examples 1-12 and 5-6,.

Examples 5-55 and 5-56

The compounds of Examples 5-55 and 5-56 were obtained by using 4-(2,3-dihydro-1H-1,5-diazaphenalen-1-yl)cyclohexanone (Intermediate 43) obtained in Example 5-1, Step C to perform reductive amination, and then ureation according to the methods described in Examples 1-12 and 1-30.

Examples 5-57 to 5-74

The compounds of Examples 5-57 to 5-74 were obtained by using 4-(2,3-dihydro-1H-1,5-diazaphenalen-1-yl) cyclohexanone (Intermediate 43) obtained in Example 5-1, Step C to perform reductive amination according to the method described in Example 1-12.

Examples 5-75 to 5-77

The compounds of Examples 5-75 to 5-77 were obtained by using 1-amino-4-(2,3-dihydro-1H-1,5-diazaphenalen-1-yl)cyclohexane obtained in Example 5-1 to perform alkylation according to the method described in Example 1-3, or Example 1-7.

Examples 5-78 to 5-122

The compounds of Examples 5-78 to 5-122 were obtained by using 4-(2,3-dihydro-1H-1,5-diazaphenalen-1-yl)cyclohexanone (Intermediate 43) obtained in Example 5-1, Step C to perform reductive amination, and then esterification according to the methods described in Example 1-12 and 1-24.

Example 5-123

The compound of Example 5-123 was obtained by using 1-amino-4-(2,3-dihydro-1H-1,5-diazaphenalen-1-yl) cyclohexane obtained in Example 5-1 and 1,4-cyclohexanedione monoethylene ketal to perform reductive alkylation, deprotection for the protective group, and reduction according to the method described in Example 1-12.

Example 5-124

The compound of Example 5-124 was obtained by using 1-amino-4-(2,3-dihydro-1H-1,5-diazaphenalen-1-yl) obtained in Example 5-1 to perform reductive alkylation, and deprotection for the protective group according to the method described in Example 1-27.

Example 5-125

The compound of Example 5-125 was obtained by using 1-(4-aminocyclohexyl)amino-4-(2,3-dihydro-1H-1,5-diazaphenalen-1-yl)cyclohexane obtained in Example 5-124 to perform acetylation according to the method described in Example 1-22.

Examples 5-126 to 5-146

The compounds of Examples 5-126 to 5-146 were obtained by using 4-(2,3-dihydro-1H-1,5-diazaphenalen-1-yl)cyclohexanone (Intermediate 43) obtained in Example 5-1, Step C to perform reductive amination, and then sulfonylation according to the methods described in Examples 1-12 and 1-22.

Examples 5-147 to 5-167 and 5-170 to 5-259

The compounds of Examples 5-147 to 5-167 and 5-170 to 5-259 were obtained by using
1-(2-aminoethyl)amino-4-(2,3-dihydro-1H-1,5-diazaphenalen-1-yl)cyclohexane obtained in Example 5-57,
1- [2-(methylamino)ethyl]amino-4-(2,3-dihydro-1H-1,5-diazaphenalen-1-yl)cyclohexane obtained in Example 5-64,
1-(3-aminopropyl)amino-4-(2,3-dihydro-1H-1,5-diazaphenalen-1-yl)cyclohexane obtained in Example 5-58,
1-[3-(methylamino)propyl]amino-4-(2,3-dihydro-1H-1,5-diazaphenalen-1-yl)cyclohexan e obtained in Example 5-65,
1-(4-aminobutyl)amino-4-(2,3-dihydro-1H-1,5-diazaphenalen-1-yl)cyclohexane obtained in Example 5-62, or
1-[4-(methylamino)butyl]amino-4-(2,3-dihydro-1H-1,5-diazaphenalen-1-yl)cyclohexane obtained in Example 5-66 to perform ureation according to the method described in Example 1-29.

Examples 5-168 and 5-169

The compounds of Examples 5-168 and 5-169 were obtained by using 4-(2,3-dihydro-1H-1,5-diazaphenalen-1-yl)cyclohexanone (Intermediate 43) obtained in Example 5-1, Step C to perform reductive amination according to the method described in Example 5-3.

Examples 5-260 to 5-263 and 5-265 to 5-272

The compounds of Examples 5-260 to 5-263 and 5-265 to 5-272 were obtained by using
1-(2-aminoethyl)amino-4-(2,3-dihydro-1H-1,5-diazaphenalen-1-yl)cyclohexane obtained in Example 5-57 to perform thioureation according to the method described in Example 1-31.

Example 5-264

The compound of Example 5-264 was obtained by using 4-(2,3-dihydro-1H-1,5-diazaphenalen-1-yl)cyclohexanone (Intermediate 43) obtained in Example 5-1, Step C to perform reductive amination according to the method described in Example 5-3.

Examples 5-273 to 5-281

The compounds of Examples 5-273 to 5-281 were obtained by using 1-(2-aminoethyl) amino-4-(2, 3-dihydro-1H-1,5-diazaphenalen-1-yl)cyclohexane obtained in Example 5-57 to perform carbamation according to the method described in Example 1-33.

Examples 5-282 and 5-283

The compounds of Examples 5-282 and 5-283 were obtained by using 1-amino-4-(2,3-dihydro-1H-1,5-diazaphenalen-1-yl)cyclohexane obtained in Example 5-1 to perform alkylation according to the method described in Example 1-3.

Examples 5-284 to 5-286

The compounds of Examples 5-284 to 5-286 were obtained by using 1-(2-aminoethyl)amino-4-(2,3-dihydro-1H-1,5-diazaphenalen-1-yl)cyclohexane obtained in Example 5-57 to perform alkylation, deprotection for the protective group, and then lactam cyclization according to the method described in Example 1-36.

Example 5-287

The compound of Example 5-287 was obtained by using 1-amino-4-(2,3-dihydro-1H-1,5-diazaphenalen-1-yl) cyclohexane obtained in Example 5-1 to perform guanidylation according to the method described in Example 1-35.

Example 5-288

The compound of Example 5-288 was obtained by using 1-amino-4-(2,3-dihydro-1H-1,5-diazaphenalen-1-yl) cyclohexane obtained in Example 5-1 to perform alkylation according to the method described in Example 5-2.

Examples 5-289 and 5-290

The compounds of Examples 5-289 and 5-290 were obtained by using 1-amino-4-(2,3-dihydro-1H-1,5-diazaphenalen-1-yl)cyclohexane obtained in Example 5-1 to perform reductive alkylation according to the method described in Example 1-11.

Example 5-291:

1-Amino-4-(6-chloro-2,3-dihydro-1H-1,5-diazaphenalen-1-yl)cyclohexane

Step A

1-Chloro-5-(1,4-dioxa-spiro[4.5]dec-8-yl)amino-4-vinylisoquinoline (Intermediate 45)

A solution of 5-amino-1-chloro-4-vinylisoquinoline (Intermediate 8, 16.38 g) obtained in Example 1-321, Step D, and 1,4-cyclohexanedione monoethylene ketal (25.00 g) in 1,2-dichloroethane (820 ml) was added with glacial acetic acid (9.07 ml) and then with sodium triacetoxyborohydride (33.93 g), and stirred at room temperature for 48 hours. The reaction mixture was cooled on an ice bath, and slowly added with saturated aqueous sodium hydrogencarbonate (500 ml) for neutralization. The organic layer was separated, and the aqueous layer was extracted twice.with ethyl acetate (400 ml for each time). The combined organic layer was washed with saturated brine, and dried over anhydrous magnesium sulfate. The organic layer was filtered through Celite, and then the solvent was evaporated under reduced pressure. The residue was purified by silica gel column chromatography (n-hexane:ethyl acetate = 4:1) to obtain the title compound (26.20 g).
MS (m/z): 346 (MH+)

Step B

6-Chloro-2,3-dihydro-1-(1,4-dioxa-spiro[4.5]dec-8-yl)-1,5-diazaphenalene (Intermediate 46)

A solution of 1-chloro-5-(1,4-dioxa-spiro[4.5]dec-8-ylamino)-4-vinylisoquinoline (Intermediate 45, 13.64 g) obtained in Step A mentioned above in tetrahydrofuran (400 ml) was added with potassium tert-butoxide (44.38 g), and stirred at 40°C for 2 hours. The reaction mixture was left to cool to room temperature, and then added with toluene (800 ml), and then with a saturated solution of citric acid monohydrate (41.55 g) in tetrahydrofuran under cooling on an ice bath for neutralization (pH 7). After insoluble solids were removed by filtration through Celite, the organic layer was separated, and the aqueous layer was extracted twice with ethyl acetate (500 ml for each time). The combined organic layer was washed with saturated brine (500 ml), and dried over anhydrous magnesium sulfate. The organic layer was filtered through Celite, and then the solvent was evaporated under reduced pressure. The residue was purified by silica gel column chromatography (n-hexane:ethyl acetate = 3:1) to obtain the title compound (5.05 g).
MS (m/z) : 346 (MH+)

Step C

4-(6-Chloro-2,3-dihydro-1H-1,5-diazaphenalen-1-yl)cyclohexanone (Intermediate 47)

A mixture of 2 N hydrochloric acid (40 ml) and tetrahydrofuran (20 ml) was added with 6-chloro-2,3-dihydro-1-(1,4-dioxa-spiro[4.5]dec-8-yl)-1,5-diazaphenalene (Intermediate 46, 5.0 g) obtained in Step B mentioned above, and stirred at room temperature for 12 hours. The solvent was evaporated under reduced pressure, and the residue was added with 2 N aqueous sodium hydroxide for neutralization. The mixture was extracted 3 times with chloroform (100

ml for each time), and the combined organic layer was washed with saturated brine (200 ml), and dried over anhydrous magnesium sulfate. The organic layer was filtered through Celite, and then the solvent was evaporated under reduced pressure to obtain the title compound (4.30 g).
MS (m/z): 301 (MH+)

Step D

1-(4-Methoxybenzylamino)-4-(6-chloro-2,3-dihydro-1H-1,5-diazaphenalen-1-yl)cyclohexane (Intermediate 48)

By using 4-(6-chloro-2,3-dihydro-1H-1,5-diazaphenalen-1-yl)cyclohexanone (Intermediate 47, 4.00 g) obtained in Step C mentioned above and 4-methoxybenzylamine (Tokyo Kasei Kogyo), the title compound (4.83 mg) was obtained according to the method described in Example 5-1, Step D.
MS (m/z): 422 (MH+)

Step E

1-Amino-4-(6-chloro-2,3-dihydro-1H-1,5-diazaphenalen-1-yl)cyclohexane

A solution of 1-(4-methoxybenzylamino)-4-(6-chloro-2,3-dihydro-1H-1,5-diazaphenalen-1-yl)cyclohexane (Intermediate 48, 105 mg) obtained in Step D mentioned above in 95% trifluoroacetic acid (1 ml) was stirred at room temperature for 3 hours. The solvent was evaporated under reduced pressure to obtain the title compound as a trifluoroacetate (100 mg).
MS (m/z): 302 (MH+)

Example 5-292

The compound of Example 5-292 was obtained by referring to the method used for the compound of Example 5-291.

Example 5-293:

1-Amino-4-(6-hydroxy-2,3-dihydro-1H-1,5-diazaphenalen-1-yl)cyclohexane

Step A

4-(6-Hydroxy-2,3-dihydro-1H-1,5-diazaphenalen-1-yl)cyclohexanone (Intermediate 49)

6-Chloro-2,3-dihydro-1-(1,4-dioxa-spiro[4.5]dec-8-yl)-1,5-diazaphenalene (Intermediate 46, 3.02 g) obtained in Example 5-291, Step B was added with concentrated hydrochloric acid (50 ml), and stirred at 70°C for 17 hours. The reaction mixture was left to cool to room temperature, and then the solvent was evaporated under reduced pressure to obtain the title compound (2.33 g).
MS (m/z): 283 (MH+)

Step B

1-Amino-4-(6-hydroxy-2,3-dihydro-1H-1,5-diazaphenalen-1-yl)cyclohexane

By using 4-(6-hydroxy-2,3-dihydro-1H-1,5-diazaphenalen-1-yl)cyclohexanone (Intermediate 49) obtained in Step A mentioned above, the title compound was obtained according to the methods described in Example 5-1, Steps D and E.
MS (m/z): 284 (MH+)

Example 5-294:

1-Amino-4-(6-methyl-2, 3-dihydro-1H-1, 5-diazaphenalen-1-yl)cyclohexane

Step A

6-Methyl-2,3-dihydro-1-(1,4-dioxa-spiro[4.5]dec-8-yl)-1,5-diazaphenalene (Intermediate 50)

The title compound was obtained by using 6-chloro-2,3-dihydro-1-(1,4-dioxa-spiro[4.5]dec-8-yl)-1,5-diazaphenalene (Intermediate 46) obtained in Example 5-291, Step B with referring to a known publication (for example, Hollenbaugh, Dian L. et al., Bioorg. Med. Chem. Lett., 13, 1345 (2003) and the like).
MS (m/z): 325

Step B

4-(6-Methyl-2,3-dihydro-1H-1,5-diazaphenalen-1-yl)cyclohexanone (Intermediate 51)

By using 6-methyl-2,3-dihydro-1-(1,4-dioxa-spiro[4.5]dec-8-yl)-1,5-diazaphenalene (Intermediate 50) obtained in Step A mentioned above, deprotection was performed for the protective group according to the method described in Example 5-1, Step C to obtain the title compound.
MS (m/z): 281 (MH+)

Step C

1-(Benzylamino)-4-(6-methyl-2,3-dihydro-1H-1,5-diazaphenalen-1-yl)cyclohexane (Intermediate 52)

By using 4-(6-methyl-2,3-dihydro-1H-1,5-diazaphenalen-1-yl)cyclohexanone (Intermediate 51) obtained in Step B mentioned above, reductive amination was performed with benzylamine according to the method described in Example 5-1, Step D, to obtain the title compound.
MS (m/z): 372 (MH+)

Step D

1-Amino-4-(6-methyl-2, 3-dihydro-1H-1, 5-diazaphenalen-1-yl)cyclohexane

By using 1-(benzylamino)-4-(6-methyl-2,3-dihydro-1H-1,5-diazaphenalen-1-yl)cyclohexane (Intermediate 52) obtained in Step C mentioned above, the title compound was obtained according to the method described in Example 5-1, Step E.
MS (m/z): 282 (MH+)

Example 5-295:

1-Amino-4-(6-vinyl-2,3-dihydro-1H-1,5-diazaphenalen-1-yl)cyclohexane

Step A

6-Vinyl-2,3-dihydro-1-(1,4-dioxa-spiro[4.5]dec-8-yl)-1,5-diazaphenalene (Intermediate 53)

By using 6-chloro-2,3-dihydro-1-(1,4-dioxa-spiro[4.5]dec-8-yl)-1,5-diazaphenalene (Intermediate 46) obtained in Example 5-291, Step B, the title compound was obtained with referring to a known publication (for example, Carreno, M.C., Mahugo, R.H-S.J., J. Org. Chem., 64, 1387 (1999) and the like).
MS (m/z): 337 (MH+)

Step B

4-(6-Vinyl-2,3-dihydro-1H-1,5-diazaphenalen-1-yl)cyclohexanone (Intermediate 54)

By using 6-vinyl-2,3-dihydro-1-(1,4-dioxa-spiro[4.5]dec-8-yl)-1,5-diazaphenalene (Intermediate 53) obtained

in Step A mentioned above, deprotection was performed for the protective group according to the method described in Example 5-1, Step C to obtain the title compound.
MS (m/z): 293 (MH+)

Step C

1-(4-Methoxybenzylamino)-4-(6-vinyl-2,3-dihydro-1H-1,5-diazaphenalen-1-yl)cyclohexane (Intermediate 55)

By using 4-(6-vinyl-2,3-dihydro-1H-1,5-diazaphenalen-1-yl)cyclohexanone (Intermediate 54) obtained in Step B mentioned above, reductive amination was performed with 4-methoxybenzylamine according to the method described in Example 5-1, Step D to obtain the title compound.
MS (m/z): 414 (MH+)

Step D

1-Amino-4-(6-methyl-2,3-dihydro-1H-1,5-diazaphenalen-1-yl)cyclohexane

By using 1-(4-methoxybenzylamino)-4-(6-vinyl-2, 3-dihydro-1H-1,5-diazaphenalen-1-yl)cyclohexane (Intermediate 55) obtained in Step C mentioned above, the title compound was obtained as a trifluoroacetate according to the method described in Example 5-291, Step E.
MS (m/z): 294 (MH+)

Example 5-296:

1-Amino-4-[6-(phenylethynyl)-2, 3-dihydro-1H-1,5-diazaphenalen-1-yl]cyclohexane

Step A

6-(Phenylethynyl)-2,3-dihydro-1-(1,4-dioxa-spiro[4.5]dec-8-yl)-1,5-diazaphenalene (Intermediate 56)

By using 6-chloro-2,3-dihydro-1-(1,4-dioxa-spiro[4.5]dec-8-yl)-1,5-diazaphenalene (Intermediate 46) obtained in Example 5-291, Step B, the title compound was obtained with referring to a known publication (for example, Hundertmark, T., Littke, A.F., Buchwald, S.L., Fu, G.C., Org. Lett., 2, 1729 (2000) and the like).
MS (m/z): 411 (MH+)

Step B

4-[6-(Phenylethynyl)-2,3-dihydro-1H-1,5-diazaphenalen-1-yl]cyclohexanone (Intermediate 57)

By using 6-(phenylethynyl)-2,3-dihydro-1-(1,4-dioxa-spiro[4.5]dec-8-yl)-1,5-diazaphenalene (Intermediate 56) obtained in Step A mentioned above, deprotection was performed for the protective group according to the method described in Example 5-1, Step C to obtain the title compound.
MS (m/z): 367 (MH+)

Step C

1-(4-Methoxybenzylamino)-4-[6-(phenylethynyl)-2,3-dihydro-1H-1,5-diazaphenalen-1-yl]cyclohexane (Intermediate 58)

By using 4-[6-(phenylethynyl)-2,3-dihydro-1H-1,5-diazaphenalen-1-yl]cyclohexanone (Intermediate 57) obtained in Step B mentioned above, reductive amination was performed with 4-methoxybenzylamine according to the method described in Example 5-1, Step D to obtain the title compound.
MS (m/z): 488 (MH+)

Step D

1-Amino-4-[6-(phenylethynyl)-2,3-dihydro-1H-1,5-diazaphenalen-1-yl]cyclohexane

By using 1-(4-methoxybenzylamino)-4-[6-(phenylethynyl)-2,3-dihydro-1H-1,5-diazaphenalen-1-yl]cyclohexane (Intermediate 58) obtained in Step C mentioned above, the title compound was obtained as a trifluoroacetate according to the method described in Example 5-291, Step E.
MS (m/z): 368 (MH+)

Example 5-297:

1-Amino-4-(6-methoxy-2,3-dihydro-1H-1, 5-diazaphenalen-1-yl)cyclohexane

Step A

6-Methoxy-2,3-dihydro-1-(1,4-dioxa-spiro[4.5]dec-8-yl)-1,5-diazaphenalene (Intermediate 59)

By using 6-chloro-2,3-dihydro-1-(1,4-dioxa-spiro[4.5]dec-8-yl)-1,5-diazaphenalene (Intermediate 46) obtained in Example 5-291, Step B, the title compound was obtained with referring to a known publication (for example, Toracca, K.E., Huang, X., Parrish, C.A., Buchwald, S.L., J. Am. Chem. Soc., 123, 10770 (2001) and the like).
MS (m/z): 341 (MH+)

Step B

4-(6-Methoxy-2,3-dihydro-1H-1,5-diazaphenalen-1-yl)cyclohexanone (Intermediate 60)

By using 6-methoxy-2,3-dihydro-1-(1,4-dioxa-spiro[4.5]dec-8-yl)-1,5-diazaphenalene (Intermediate 59) obtained in Step A mentioned above, deprotection was performed for the protective group according to the method described in Example 5-1, Step C to obtain the title compound.
MS (m/z): 297 (MH+)

Step C

1-(Benzylamino)-4-(6-methoxy-2,3-dihydro-1H-1,5-diazaphenalen-1-yl)cyclohexane (Intermediate 61)

By using 4-(6-methoxy-2,3-dihydro-1H-1,5-diazaphenalen-1-yl)cyclohexanone (Intermediate 60) obtained in Step B mentioned above, reductive amination was performed with benzylamine according to the method described in Example 5-1, Step D to obtain the title compound.
MS (m/z): 388 (MH+)

Step D

1-Amino-4-(6-methoxy-2,3-dihydro-1H-1,5-diazaphenalen-1-yl)cyclohexane

By using 1-(benzylamino)-4-(6-methoxy-2,3-dihydro-1H-1,5-diazaphenalen-1-yl)cyclohexane (Intermediate 61) obtained in Step C mentioned above, the title compound was obtained according to the method described in Example 5-1, Step E. MS (m/z): 298 (MH+)

Example 5-298:

1-Amino-4-(6-amino-2,3-dihydro-1H-1,5-diazaphenalen-1-yl)cyclohexane

Step A

6-Amino-2,3-dihydro-1-(1,4-dioxa-spiro[4.5]dec-8-yl)-1,5-diazaphenalene (Intermediate 62)

By using 6-chloro-2,3-dihydro-1-(1,4-dioxa-spiro[4.5]dec-8-yl)-1,5-diazaphenalene (Intermediate 46) obtained in Example 5-291, Step B, the title compound was obtained with referring to a known publication (for example, Klapars, A., Huang, X., Buchwald, S.L., J. Am. Chem. Soc., 124, 7421 (2002) and the like).
MS (m/z): 326 (MH+)

Step B

4-(6-Amino-2,3-dihydro-1H-1,5-diazaphenalen-1-yl)cyclohexanone (Intermediate 63)

By using 6-amino-2,3-dihydro-1-(1,4-dioxa-spiro[4.5]dec-8-yl)-1,5-diazaphenalene (Intermediate 62) obtained in Step A mentioned above, deprotection was performed for the protective group according to the method described in Example 5-1, Step C to obtain the title compound.
MS (m/z): 282 (MH+)

Step C

1-(Benzylamino)-4-(6-amino-2, 3-dihydro-1H-1,5-diazaphenalen-1-yl)cyclohexane (Intermediate 64)

By using 4-(6-amino-2,3-dihydro-1H-1,5-diazaphenalen-1-yl)cyclohexanone (Intermediate 63) obtained in Step B mentioned above, reductive amination was performed with benzylamine according to the method described in Example 5-1, Step D to obtain the title compound.
MS (m/z): 373 (MH+)

Step D

1-Amino-4-(6-amino-2,3-dihydro-1H-1,5-diazaphenalen-1-yl)cyclohexane

By using 1-(benzylamino)-4-(6-amino-2,3-dihydro-1H-1,5-diazaphenalen-1-yl)cyclohexane (Intermediate 64) obtained in Step C mentioned above, the title compound was obtained according to the method described in Example 5-1, Step E.
MS (m/z): 283 (MH+)

Examples 5-299 to 5-300

The compounds of Examples 5-299 to 5-300 were obtained by referring to the method used for the compound of Example 5-301 mentioned below.

Example 5-301:

1-Amino-4-(6-anilino-2,3-dihydro-1H-1,5-diazaphenalen-1-yl)cyclohexane

Step A

6-Anilino-2,3-dihydro-1-(1,4-dioxa-spiro[4.5]dec-8-yl)-1,5-diazaphenalene (Intermediate 65)

By using 6-chloro-2,3-dihydro-1-(1,4-dioxa-spiro[4.5]dec-8-yl)-1,5-diazaphenalene (Intermediate 46) obtained in Example 5-291, Step B, the title compound was obtained with referring to a known publication (for example, Buchwald, S.L., Zim, D., Org. Lett., 5, 2413 (2003) and the like).

Step B

1-Amino-4-(6-anilino-2,3-dihydro-1H-1,5-diazaphenalen-1-yl)cyclohexane

By using 6-anilino-2,3-dihydro-1-(1,4-dioxa-spiro[4.5]dec-8-yl)-1,5-diazaphenalene (Intermediate 65) obtained in Step A mentioned above, the title compound was obtained according to the methods described in Example 5-1, Steps C to E.
MS (m/z): 359 (MH+)

Example 5-302:

5- [4-(6-Hydroxy-2,3-dihydro-1H-1,5-diazaphenalen-1-yl)cyclohexylamino]pentanoic acid

By using 1-amino-4-(6-hydroxy-2,3-dihydro-1H-1,5-diazaphenalen-1-yl)cyclohexane obtained in Example

5-293, alkylation was performed with tert-butyl 5-bromopentanoate according to the method described in Example 1-3, and then deprotection was performed for the protective group according to the method described in Example 1-1, Step D to obtain the title compound as a hydrochloride.
MS (m/z) : 384 (MH+)

Example 5-303:

5-[4-(6-Hydroxy-2,3-dihydro-1H-1,5-diazaphenalen-1-yl)cyclohexylamino]pentanol

By using 4-(6-hydroxy-2,3-dihydro-1H-1,5-diazaphenalen-1-yl)cyclohexanone (Intermediate 49) obtained in Example 5-293, Step A, reductive amination was performed with 5-amino-1-pentanol according to the method described in Example 1-12 to obtain the title compound.
MS (m/z): 370 (MH+)

Example 5-304: Thioacetic acid S-{2-[4-(6-hydroxy-2,3-dihydro-1H-1,5-diazaphenalen-1-yl)cyclohexylamino]ethyl} ester

By using 4-(6-hydroxy-2,3-dihydro-1H-1,5-diazaphenalen-1-yl)cyclohexanone (Intermediate 49) obtained in Example 5-293, Step A, reductive amination was performed with thioacetic acid S-(2-aminoethyl) ester according to the method described in Example 1-12 to obtain the title compound.
MS (m/z): 386 (MH+)

Example 5-305: 2-[4-(6-Hydroxy-2,3-dihydro-1H-1,5-diazaphenalen-1-yl)cyclohexylamino] ethane thiol

Deprotection was performed for the acetyl group of thioacetic acid S-{2-[4-(6-hydroxy-2, 3-dihydro-1H-1,5-diazaphenalen-1-yl)cyclohexylamino]ethyl} ester obtained in Example 5-304 according to the method described in Example 1-16 to obtain the title compound.
MS (m/z): 344 (MH+)

Example 5-306: 1-(Acetamino)-4-(6-hydroxy-2,3-dihydro-1H-1,5-diazaphenalen-1-yl)cyclohexane

By using 1-amino-4-(6-hydroxy-2,3-dihydro-1H-1,5-diazaphenalen-1-yl)cyclohexane obtained in Example 5-293, acetylation was performed with acetic anhydride according to the method described in Example 1-22 to obtain the title compound.
MS (m/z): 326 (MH+)

Example 5-307: 1-(Methanesulfonamino)-4-(6-hydroxy-2,3-dihydro-1H-1,5-diazaphenalen- 1-yl)cyclohexane

By using 1-amino-4-(6-hydroxy-2,3-dihydro-1H-1,5-diazaphenalen-1-yl)cyclohexane obtained in Example 5-293, mesylation was performed with methanesulfonic anhydride according to the method described in Example 1-22 to obtain the title compound.
MS (m/z) : 362 (MH+)

Example 5-308: N-{3-[4-(6-Hydroxy-2,3-dihydro-1H-1,5-diazaphenalen-1-yl)cyclohexylamino]propyl}-N'-phenylurea

By using 4-(6-hydroxy-2,3-dihydro-1H-1,5-diazaphenalen-1-yl)cyclohexanone (Intermediate 49) obtained in Example 5-293, Step A, reductive amination was performed with tert-butyl N-(3-aminopropyl)carbamate (Tokyo Kasei Kogyo) according to the method described in Example 1-12, and then deprotection was performed for the protective group according to the method described in Example 1-1, Step D to obtain 1-[(3-aminopropyl)amino]-4-(6-hydroxy-2,3-dihydro-1H-1,5-diazaphenalen-1-yl)cyclohexane as a hydrochloride. Then, the resultant was reacted with 4-nitrophenyl carbonate, and then with aniline according to the method described in Example 1-29 to obtain the title compound.
MS (m/z): 460 (MH+)

Example 5-309: N-{3-[4-(6-Hydroxy-2,3-dihydro-1H-1,5-diazaphenalen-1-yl)cyclohexylamino]propyl}-N'-cyclohexylurea

By using 4-(6-hydroxy-2,3-dihydro-1H-1,5-diazaphenalen-1-yl)cyclohexanone (Intermediate 49) obtained in Example 5-293, Step A, reductive amination was performed with N-(3-aminopropyl)-N'-cyclohexylurea according to the method described in Example 5-3 to obtain the title compound.

MS (m/z): 466 (MH+)

Example 5-310: 4-(6-Hydroxy-2,3-dihydro-1H-1,5-diazaphenalen-1-yl)cyclohexanol

By using 4-(6-hydroxy-2,3-dihydro-1H-1,5-diazaphenalen-1-yl)cyclohexanone (Intermediate 49) obtained in Example 5-293, reduction of the carbonyl group was performed according to the method described in Example 5-10 to obtain the title compound.
MS (m/z): 285 (MH+)

Examples 5-311 to 5-316

The compounds of Examples 5-311 to 5-316 were obtained by using 4-(6-hydroxy-2,3-dihydro-1H-1,5-diazaphenalen-1-yl)cyclohexanone (Intermediate 49) obtained in Example 5-293 to perform reductive amination according to the method described in Example 5-3.

Example 5-317

The compound of Example 5-317 was obtained by using 1-amino-4-(6-hydroxy-2,3-dihydro-1H-1,5-diazaphenalen-1-yl)cyclohexane obtained in Example 5-293 to perform esterification according to the method described in Example 5-6.

Example 5-318

The compound of Example 5-318 was obtained by using 1-amino-4-(6-hydroxy-2,3-dihydro-1H-1,5-diazaphenalen-1-yl)cyclohexane obtained in Example 5-293 to perform sulfonylation according to the method described in Example 5-7.

Examples 5-319 to 5-332

The compounds of Examples 5-319 to 5-332 were obtained by using 1-amino-4-(6-hydroxy-2,3-dihydro-1H-1,5-diazaphenalen-1-yl)cyclohexane obtained in Example 5-293 to perform alkylation according to the method described in Example 5-2, or by using 4-(6-hydroxy-2,3-dihydro-1H-1,5-diazaphenalen-1-yl)cyclohexanone (Intermediate 49) obtained in Example 5-293 to perform reductive amination according to the method described in Example 5-3.

Examples 5-333 to 5-341

The compounds of Examples 5-333 to 5-341 were obtained by using 2- [4-(6-hydroxy-2,3-dihydro-1H-1,5-diazaphenalen-1-yl)cyclohexylamino]ethanol obtained in Example 5-326 to perform active esterification and then react the resultant with an amine according to the method described in Example 1-34.

Example 5-342

The compound of Example 5-342 was obtained by performing deprotection for the acetyl group of the thioacetic acid S-{3-[4-(6-hydroxy-2,3-dihydro-1H-1,5-diazaphenalen-1-yl)cyclohexylamino]propyl} ester obtained in Example 5-344 mentioned below according to the method described in Example 1-16.

Examples 5-343 to 5-345

The compounds of Examples 5-343 to 5-345 were obtained by using 4-(6-hydroxy-2,3-dihydro-1H-1,5-diazaphenalen-1-yl)cyclohexanone (Intermediate 49) obtained in Example 5-293 to perform reductive amination according to the method described in Example 1-12.

Examples 5-346 to 5-349

The compounds of Examples 5-346 to 5-349 were obtained by using 1-amino-4-(6-hydroxy-2,3-dihydro-1H-1,5-diazaphenalen-1-yl)cyclohexane obtained in Example 5-293 to perform alkylation according to the method described in Example 5-2.

Examples 5-350 to 5-353

The compounds of Examples 5-350 to 5-353 were obtained by using 4-(6-hydroxy-2,3-dihydro-1H-1,5-diazaphenalen-1-yl)cyclohexanone (Intermediate 49) obtained in Example 5-293 to perform reductive amination according to the method described in Example 1-12.

Example 5-354

The compound of Example 5-354 was obtained by using 4-(6-hydroxy-2,3-dihydro-1H-1,5-diazaphenalen-1-yl)cyclohexanone (Intermediate 49) obtained in Example 5-293 to perform reductive amination, and then acetylation according to the methods described in Examples 5-3 and 1-22.

Examples 5-355 and 5-356

The compounds of Examples 5-355 and 5-356 were obtained by using 4-(6-hydroxy-2,3-dihydro-1H-1,5-diazaphenalen-1-yl)cyclohexanone (Intermediate 49) obtained in Example 5-293 to perform reductive amination, and then ureation according to the methods described in Example 1-12 and 1-30.

Examples 5-357 to 5-374

The compounds of Examples 5-357 to 5-374 were obtained by using 4-(6-hydroxy-2,3-dihydro-1H-1,5-diazaphenalen-1-yl)cyclohexanone (Intermediate 49) obtained in Example 5-293 to perform reductive amination according to the method described in Example 1-12.

Examples 5-375 to 5-377

The compounds of Examples 5-375 to 5-377 were obtained by using 1-amino-4-(6-hydroxy-2,3-dihydro-1H-1,5-diazaphenalen-1-yl)cyclohexane obtained in Example 5-293 to perform alkylation according to the method described in Example 1-3 or Example 1-7.

Examples 5-378 to 5-422

The compounds of Examples 5-378 to 5-422 were obtained by using 4-(6-hydroxy-2,3-dihydro-1H-1,5-diazaphenalen-1-yl)cyclohexanone (Intermediate 49) obtained in Example 5-293 to perform reductive amination, and then esterification according to the methods described in Example 1-12 and 1-24.

Example 5-423

The compound of Example 5-423 was obtained by using 1-amino-4-(6-hydroxy-2,3-dihydro-1H-1,5-diazaphenalen-1-yl)cyclohexane obtained in Example 5-293 to perform reductive alkylation, deprotection for the protective group, and reduction according to the method described in Example 1-12.

Example 5-424

The compound of Example 5-424 was obtained by using 1-amino-4-(6-hydroxy-2,3-dihydro-1H-1,5-diazaphenalen-1-yl)cyclohexane obtained in Example 5-293 to perform reductive alkylation, and deprotection for the protective group according to the method described in Example 1-27.

Example 5-425

The compound of Example 5-425 was obtained by using 1-(4-aminocyclohexyl)amino-4-(6-hydroxy-2,3-dihydro-1H-1,5-diazaphenalen-1-yl)cyclohexane obtained in Example 5-424 to perform acetylation according to the method described in Example 1-22.

Example 5-426 to 5-446

The compounds of Examples 5-426 to 5-446 were obtained by using 4-(6-hydroxy-2,3-dihydro-1H-1,5-diazaphenalen-1-yl)cyclohexanone (Intermediate 49) obtained in Example 5-293 to perform reductive amination, and then

sulfonylation according to the methods described in Examples 1-12 and 1-22.

Examples 5-447 to 5-467 and 5-470 to 5-559

The compounds of Examples 5-447 to 5-467 and 5-470 to 5-559 were obtained by using 1-(2-aminoethyl) amino-4-(6-hydroxy-2,3-dihydro-1H-1,5-diazaphenalen-1-yl)cyclohexane obtained in Example 5-357,
1-[2-(methylamino)ethyl]amino-4-(6-hydroxy-2,3-dihydro-1H-1,5-diazaphenalen-1-yl)cyclohexane obtained in Example 5-364,
1-(3-aminopropyl)amino-4-(6-hydroxy-2,3-dihydro-1H-1,5-diazaphenalen-1-yl)cyclohexane obtained in Example 5-358,
1-[3-(methylamino)propyl]amino-4-(6-hydroxy-2,3-dihydro-1H-1,5-diazaphenalen-1-yl)cyclohexane obtained in Example 5-365,
1-(4-aminobutyl)amino-4-(6-hydroxy-2,3-dihydro-1H-1,5-diazaphenalen-1-yl)cyclohexane obtained in Example 5-362, or
1-[4-(methylamino)butyl]amino-4-(6-hydroxy-2,3-dihydro-1H-1,5-diazaphenalen-1-yl)cyclohexane obtained in Example 5-366 to perform ureation according to the method described in Example 1-29.

Examples 5-468 and 5-469

The compounds of Examples 5-468 and 5-469 were obtained by using 4-(6-hydroxy-2,3-dihydro-1H-1,5-diazaphenalen-1-yl)cyclohexanone (Intermediate 49) obtained in Example 5-293 to perform reductive amination according to the method described in Example 5-3.

Examples 5-560 to 5-563 and 5-565 to 5-572

The compounds of Examples 5-560 to 5-563 and 5-565 to 5-572 were obtained by using 1-(2-aminoethyl) amino-4-(6-hydroxy-2,3-dihydro-1H-1,5-diazaphenalen-1-yl)cyclohexane obtained in Example 5-357 to perform thioureation according to the method described in Example 1-31.

Example 5-564

The compound of Example 5-564 was obtained by using 4-(6-hydroxy-2,3-dihydro-1H-1,5-diazaphenalen-1-yl)cyclohexanone (Intermediate 49) obtained in Example 5-293 to perform reductive amination according to the method described in Example 5-3.

Examples 5-573 to 5-581

The compounds of Examples 5-573 to 5-581 were obtained by using 1-(2-aminoethyl)amino-4-(6-hydroxy-2,3-dihydro-1H-1,5-diazaphenalen-1-yl)cyclohexane obtained in Example 5-357 to perform carbamation according to the method described in 1-33.

Examples 5-582 and 5-583

The compounds of Examples 5-582 and 5-583 were obtained by using 1-amino-4-(6-hydroxy-2,3-dihydro-1H-1,5-diazaphenalen-1-yl)cyclohexane obtained in Example 5-293 to perform alkylation according to the method described in Example 1-3.

Examples 5-584 to 5-586

The compounds of Examples 5-584 to 5-586 were obtained by using 1-(2-aminoethyl)amino-4-(6-hydroxy-2,3-dihydro-1H-1,5-diazaphenalen-1-yl)cyclohexane obtained in Example 5-357 to perform alkylation, deprotection for the protective group, and then lactam cyclization according to the method described in Example 1-36.

Example 5-587

The compound of Example 5-587 was obtained by using 1-amino-4-(6-hydroxy-2,3-dihydro-1H-1,5-diazaphenalen-1-yl)cyclohexane obtained in Example 5-293 to perform guanidylation according to the method described in Example 1-35.

Example 5-588

The compound of Example 5-588 was obtained by using 1-amino-4-(6-hydroxy-2,3-dihydro-1H-1,5-diazaphenalen-1-yl)cyclohexane obtained in Example 5-293 to perform alkylation according to the method described in Example 1-3.

Examples 5-589 and 5-59

The compounds of Examples 5-589 and 5-590 were obtained by using 1-amino-4-(6-hydroxy-2,3-dihydro-1H-1,5-diazaphenalen-1-yl)cyclohexane obtained in Example 5-293 to perform reductive alkylation according to the method described in Example 1-11.

Example 5-591:

1-Amino-4-(6-chloro-2,3-dihydro-1H-1,5-diazaphenalen-1-yl)cyclohexane

By using 3-chloro-5-nitro-4-vinylisoquinoline (Intermediate 7) obtained in Example 1-321, Step C, reduction of the nitro group, reductive alkylation with 1,4-cyclohexanedione monoethylene ketal, and cyclization were performed in the same manner as that of Example 5-291 to obtain 4-chloro-2,3-dihydro-1-(1,4-dioxaspiro[4.5]dec-8-yl)-1,5-diazaphenalene, and for this compound, deprotection for the protective group, reductive amination with 4-methoxybenzylamine, and debenzylation were further performed to obtain the title compound.
MS (m/z): 302 (MH+)

Example 5-592

The compound of Example 5-592 was obtained by referring to the method used for the compound of Example 5-591.

Examples 5-593 to 5-601

The compounds of Examples 5-593 to 5-601 were obtained by using 4-chloro-2,3-dihydro-1-(1,4-dioxa-spiro[4.5]dec-8-yl)-1,5-diazaphenalene according to the methods described in Examples 5-293 to 5-301.

Example 5-602

The compound of Example 5-602 was obtained by referring to the method used for the compound of Example 5-603 mentioned below.

Example 5-603: 1-Amino-4-(7-bromo-2,3-dihydro-1H-1,5-diazaphenalen-1-yl)cyclohexane

By using 2,3-dihydro-1-(1,4-dioxa-spiro[4.5]dec-8-yl)-1,5-diazaphenalene (Intermediate 42) obtained in Example 5-1, Step B, bromation was performed according to the method described in Example 1-663 to obtain 7-bromo-2,3-dihydro-1-(1,4-dioxa-spiro[4.5]dec-8-yl)-1,5-diazaphenalene, and then deprotection for the protective group, reductive amination with 4-methoxybenzylamine, and further debenzylation using trifluoroacetic acid were performed according to the method described in Example 5-291 to obtain the title compound as a trifluoroacetate. MS (m/z): 346 (MH+)

Examples 5-604 to 5-612

The compounds of Examples 5-604 to 5-612 were obtained by using 7-bromo-2,3-dihydro-1-(1,4-dioxa-spiro[4.5]dec-8-yl)-1,5-diazaphenalene obtained in Example 5-603 in the same manner as that used for the compounds of Examples 5-293 to 5-301.

Example 5-613

The compound of Example 5-613 was obtained by referring to the method used for the compound of Example 5-614 mentioned below.

Example 5-614

The compound of Example 5-614 was obtained by using 2,3-dihydro-1-(1,4-dioxa-spiro[4.5]dec-8-yl)-1,5-diazaphenalene (Intermediate 42) obtained in Example 5-1, Step B to perform bromation according to the method described in Example 1-663 to obtain 8-bromo-2,3-dihydro-1-(1,4-dioxaspiro[4.5]dec-8-yl)-1,5-diazaphenalene, and then using the same step as that used for the compound of Example 5-603.

Examples 5-615 to 5-623

The compounds of Examples 5-615 to 5-623 were obtained by using 8-bromo-2,3-dihydro-1-(1,4-dioxa-spiro[4.5]dec-8-yl)-1,5-diazaphenalene obtained in Example 5-614 in the same manner as that used for the compounds of Examples 5-293 to 5-301.

Example 5-624

The compound of Example 5-624 was obtained by referring to the method used for the compound of Example 5-625 mentioned below.

Example 5-625

The compound of Example 5-625 was obtained by using 9-amino-2,3-dihydro-1-(1,4-dioxa-spiro[4.5]dec-8-yl)-1,5-diazaphenalene obtained in Example 5-631 mentioned below to perform known diazotization-bromination referring to the method used for the compound of Example 1-685 and thereby obtain 9-bromo-2,3-dihydro-1-(1,4-dioxa-spiro[4.5]dec-8-yl)-1,5-diazaphenalene, and then using the same step as that used for the compound of Example 5-603.

Examples 5-626 to 5-630

The compounds of Examples 5-626 to 5-630 were obtained by using 9-bromo-2,3-dihydro-1-(1,4-dioxa-spiro[4.5]dec-8-yl)-1,5-diazaphenalene obtained in Example 5-625 in the same manner as that used for the compounds of Examples 5-293 to 5-297.

Example 5-631

The compound of Example 5-631 was obtained by using 7-bromo-2,3-dihydro-1-(1,4-dioxa-spiro[4.5]dec-8-yl)-1,5-diazaphenalene obtained in Example 5-603 to perform nitration (9-position), and reduction (bromine atom→ hydrogen atom at the 7-position, nitro group→amino group at the 9-position) in the same manner as that of Example 1-691 to obtain 9-amino-2,3-dihydro-1-(1,4-dioxa-spiro[4.5]dec-8-yl)-1,5-diazaphenalene, and then performing deprotection for the protective group, reductive amination with benzylamine, and debenzylation according to the method described in Example 5-1. Examples 5-632 to 5-634

The compounds of Examples 5-632 to 5-634 were obtained by using 9-bromo-2,3-dihydro-1-(1,4-dioxa-spiro[4.5]dec-8-yl)-1,5-diazaphenalene obtained in Example 5-625 in the same manner as that used for the compounds of Examples 5-299 to 5-301.

[Table 5]

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-1 | H | H | H | H | H | H | H | 268 |
| 5-2 | H | H | H | H | H | H | $CH_2CH_2CH_2CH_2COOH$ | 368 |
| 5-3 | H | H | H | H | H | H | $CH_2CH_2CH_2CH_2CH_2OH$ | 354 |
| 5-4 | H | H | H | H | H | H | $CH_2CH_2SCOMe$ | 370 |
| 5-5 | H | H | H | H | H | H | $CH_2CH_2SH$ | 328 |
| 5-6 | H | H | H | H | H | H | COMe | 310 |
| 5-7 | H | H | H | H | H | H | $SO_2Me$ | 346 |
| 5-8 | H | H | H | H | H | H | $CH_2CH_2CH_2NHCONHC_6H_5$ | 444 |
| 5-9 | H | H | H | H | H | H | a-91 | 450 |
| 5-11 | H | H | H | H | H | H | Me | 282 |
| 5-12 | H | H | H | H | H | H | a-121 | 322 |
| 5-13 | H | H | H | H | H | H | a-122 | 307 |
| 5-14 | H | H | H | H | H | H | Bn | 358 |
| 5-15 | H | H | H | H | H | H | a-2 | 388 |
| 5-16 | H | H | H | H | H | Me | Me | 296 |
| 5-17 | H | H | H | H | H | H | $COC_6H_5$ | 372 |
| 5-18 | H | H | H | H | H | H | $SO_2C_6H_5$ | 408 |
| 5-19 | H | H | H | H | H | H | $CH_2COOH$ | 326 |
| 5-20 | H | H | H | H | H | H | $CH_2CH_2COOH$ | 340 |
| 5-21 | H | H | H | H | H | H | $CH_2CH_2CH_2COOH$ | 354 |
| 5-22 | H | H | H | H | H | H | $CH_2CN$ | 307 |
| 5-23 | H | H | H | H | H | H | $CH_2CH_2CN$ | 321 |
| 5-24 | H | H | H | H | H | H | $CH_2CH_2CH_2CN$ | 335 |
| 5-25 | H | H | H | H | H | H | $CH_2CH_2CH_2CH_2CN$ | 349 |
| 5-26 | H | H | H | H | H | H | $CH_2CH_2OH$ | 312 |
| 5-27 | H | H | H | H | H | H | $CH_2CH$ (Me) OH | 326 |
| 5-28 | H | H | H | H | H | H | $CH(Me)CH_2OH$ | 326 |
| 5-29 | H | H | H | H | H | H | $CH_2CH_2CH_2OH$ | 326 |
| 5-30 | H | H | H | H | H | H | $CH_2CH$ (OH) $CH_2OH$ | 342 |
| 5-31 | H | H | H | H | H | H | $CH_2CH$ (Me) $CH_2OH$ | 340 |
| 5-32 | H | H | H | H | H | H | $CH_2CH_2CH_2CH_2OH$ | 340 |
| 5-33 | H | H | H | H | H | H | $CH_2CH_2OCONHMe$ | 369 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-34 | H | H | H | H | H | H | a-7 | 423 |
| 5-35 | H | H | H | H | H | H | $CH_2CH_2OCONHC_6H_5$ | 431 |
| 5-36 | H | H | H | H | H | H | $CH_2CH_2CH_2OCONHMe$ | 383 |
| 5-37 | H | H | H | H | H | H | a-48 | 437 |
| 5-38 | H | H | H | H | H | H | $CH_2CH_2CH_2OCONHC_6H_5$ | 445 |
| 5-39 | H | H | H | H | H | H | $CH_2CH_2CH_2CH_2OCONHMe$ | 397 |
| 5-40 | H | H | H | H | H | H | a-49 | 451 |
| 5-41 | H | H | H | H | H | H | $CH_2CH_2CH_2CH_2OCONHC_6H_5$ | 459 |
| 5-42 | H | H | H | H | H | H | $CH_2CH_2CH_2SH$ | 342 |
| 5-43 | H | H | H | H | H | H | $CH_2CH_2SCOC_6H.$ | 432 |
| 5-44 | H | H | H | H | H | H | $CH_2CH_2CH_2SCOMe$ | 384 |
| 5-45 | H | H | H | H | H | H | $CH_2CH_2CH_2SCOC_6H_s$ | 446 |
| 5-46 | H | H | H | H | H | H | $CH_2CH_2OMe$ | 326 |
| 5-47 | H | H | H | H | H | H | $CH_2CH_2CH_2OMe$ | 340 |
| 5-48 | H | H | H | H | H | H | $CH_2CH_2CH_2CH_2OMe$ | 354 |
| 5-49 | H | H | H | H | H | H | $CH_2CH_2CH_2CH_2CH_2OMe$ | 368 |
| 5-50 | H | H | H | H | H | H | $CH_2CH_2OCH_2CH_2OH$ | 356 |
| 5-51 | H | H | H | H | H | H | H $CH_2CH_2CH_2OCH_2CH_2OH$ | 370 |
| 5-52 | H | H | H | H | H | H | $CH_2CH_2CH_2CH_2OCH_2CH_2OH$ | 384 |
| 5-53 | H | H | H | H | H | H | CH ($CH_2OH$) $CH_2NH_2$ | 341 |
| 5-54 | H | H | H | H | H | H | CH ($CH_2OH$) $CH_2NHCOMe$ | 383 |
| 5-55 | H | H | H | H | H | H | a-51 | 466 |
| 5-56 | H | H | H | H | H | H | $CH(CH_2OH)CH_2NHCO$-H $NHC_6H_5$ | 460 |
| 5-57 | H | H | H | H | H | H | $CH_2CH_2NH_2$ | 311 |
| 5-58 | H | H | H | H | H | H | $CH_2CH_2CH_2NH_2$ | 325 |
| 5-59 | H | H | H | H | H | H | $CH_2CH$ (OH) $CH_2NH_2$ | 341 |
| 5-60 | H | H | H | H | H | H | $CH_2CH$ (OH) $CH_2NHMe$ | 355 |
| 5-61 | H | H | H | H | H | H | $CH_2CH$ (OH) $CH_2NMe_2$ | 369 |
| 5-62 | H | H | H | H | H | H | $CH_2CH_2CH_2CH_2NH_2$ | 339 |
| 5-63 | H | H | H | H | H | H | $CH_2CH_2CH_2CH_2CH_2NH_2$ | 353 |
| 5-64 | H | H | H | H | H | H | $CH_2CH_2NHMe$ | 325 |
| 5-65 | H | H | H | H | H | H | $CH_2CH_2CH_2NHMe$ | 339 |
| 5-66 | H | H | H | H | H | H | $CH_2CH_2CH_2CH_2NHMe$ | 353 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-67 | H | H | H | H | H | H | $CH_2CH_2CH_2CH_2CH_2NHMe$ | 367 |
| 5-68 | H | H | H | H | H | H | $CH_2CH_2NMe_2$ | 339 |
| 5-69 | H | H | H | H | H | H | $CH_2CH_2CH_2NMe_2$ | 353 |
| 5-70 | H | H | H | H | H | H | $CH_2CH_2CH_2CH_2NMe_2$ | 367 |
| 5-71 | H | H | H | H | H | H | $CH_2CH_2CH_2CH_2CH_2NMe_2$ | 381 |
| 5-72 | H | H | H | H | H | H | $CH_2CH_2NHCH_2CH_2OH$ | 355 |
| 5-73 | H | H | H | H | H | H | $CH_2CH_2CH_2NHCH_2CH_2OH$ | 369 |
| 5-74 | H | H | H | H | H | H | $CH_2CH_2CH_2CH_2NH$-H $CH_2CH_2OH$ | 383 |
| 5-75 | H | H | H | H | H | H | $CH_2CONH_2$ | 325 |
| 5-76 | H | H | H | H | H | H | $CH_2CH_2CONH_2$ | 339 |
| 5-77 | H | H | H | H | H | H | $CH_2CH_2CH_2CONH_2$ | 353 |
| 5-78 | H | H | H | H | H | H | $CH_2CH_2NHCOMe$ | 353 |
| 5-79 | H | H | H | H | H | H | CH $(CH_2NHCOMe)_2$ | 424 |
| 5-80 | H | H | H | H | H | H | $CH_2CH_2NHCOCH_2Me$ | 367 |
| 5-81 | H | H | H | H | H | H | $CH_2CH_2NHCOCH_2CH_2Me$ | 381 |
| 5-82 | H | H | H | H | H | H | $CH_2CH_2NHCOCHMe_2$ | 381 |
| 5-83 | H | H | H | H | H | H | $CH_2CH_2NHCOCMe_3$ | 395 |
| 5-84 | H | H | H | H | H | H | $CH_2CH_2NHCOCH_2OH$ | 369 |
| 5-85 | H | H | H | H | H | H | $CH_2CH_2NHCOCH_2OMe$ | 383 |
| 5-86 | H | H | H | H | H | H | $CH_2CH_2NHCOCH_2NMe_2$ | 396 |
| 5-87 | H | H | H | H | H | H | $CH_2CH_2NHCOC_6H_5$ | 415 |
| 5-88 | H | H | H | H | H | H | a-52 | 416 |
| 5-89 | H | H | H | H | H | H | a-53 | 421 |
| 5-90 | H | H | H | H | H | H | a-54 | 405 |
| 5-91 | H | H | H | H | H | H | a-55 | 417 |
| 5-92 | H | H | H | H | H | H | $CH_2CH_2NHCOCH_2NHCOMe$ | 410 |
| 5-93 | H | H | H | H | H | H | $CH_2CH_2NHCOCH_2NHCO$- $CH_2CHMe_2$ | 452 |
| 5-94 | H | H | H | H | H | H | $CH_2CH_2NHCOCH_2NHCOC_6H_5$ | 472 |
| 5-95 | H | H | H | H | H | H | $CH_2CH_2CH_2NHCOMe$ | 367 |
| 5-96 | H | H | H | H | H | H | $CH_2CH_2CH_2NHCOCH_2Me$ | 381 |
| 5-97 | H | H | H | H | H | H | $CH_2CH_2CH_2NHCOCH_2CH_2Me$ | 395 |
| 5-98 | H | H | H | H | H | H | $CH_2CH_2CH_2NHCOCHMe_2$ | 395 |
| 5-99 | H | H | H | H | H | H | $CH_2CH_2CH_2NHCOCMe_3$ | 409 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-100 | H | H | H | H | H | H | $CH_2CH_2CH_2CH_2NHCOMe$ | 381 |
| 5-101 | H | H | H | H | H | H | $CH_2CH_2CH_2CH_2NHCOCH_2Me$ | 395 |
| 5-102 | H | H | H | H | H | H | $CH_2CH_2CH_2CH_2NHCO\text{-}CH_2CH_2Me$ | 409 |
| 5-103 | H | H | H | H | H | H | $CH_2CH_2CH_2CH_2NHCOCHMe_2$ | 409 |
| 5-104 | H | H | H | H | H | H | $CH_2CH_2CH_2CH_2NHCOCMe_3$ | 423 |
| 5-105 | H | H | H | H | H | H | $CH_2CH_2N$ (Me) COMe | 367 |
| 5-106 | H | H | H | H | H | H | $CH_2CH_2N$ (Me) $COC_6H_5$ | 429 |
| 5-107 | H | H | H | H | H | H | $CH_2CH_2CH_2N(Me)COMe$ | 381 |
| 5-108 | H | H | H | H | H | H | $CH_2CH_2CH_2N$ (Me) $COC_6H_5$ | 443 |
| 5-109 | H | H | H | H | H | H | $CH_2CH_2CH_2CH_2N(Me)\text{-}COMe$ | 395 |
| 5-110 | H | H | H | H | H | H | $CH_2CH_2CH_2CH_2N$ (Me)-$COC_6H_5$ | 457 |
| 5-111 | H | H | H | H | H | H | $CH_2CH_2N(CH_2CH_2OH)\text{-}COMe$ | 397 |
| 5-112 | H | H | H | H | H | H | $CH_2CH_2N(CH_2CH_2OH)\text{-}COC_6H_5$ | 460 |
| 5-113 | H | H | H | H | H | H | $CH_2CH_2CH_2N$ ($CH_2CH_2OH$)-COMe | 411 |
| 5-114 | H | H | H | H | H | H | $CH_2CH_2CH_2N$ ($CH_2CH_2OH$)-$COC_6H_5$ | 474 |
| 5-115 | H | H | H | H | H | H | $CH_2CH_2CH_2CH_2$ $N(CH_2CH_2OH)COMe$ | 425 |
| 5-116 | H | H | H | H | H | H | $CH_2CH_2CH_2CH_2$-N ($CH_2CH_2OH$) $COC_6H_5$ | 488 |
| 5-117 | H | H | H | H | H | H | $CH_2CH_2N$ ($CH_2CN$) COMe | 392 |
| 5-118 | H | H | H | H | H | H | $CH_2CH_2N$ ($CH_2CN$) $COC_6H_5$ | 455 |
| 5-119 | H | H | H | H | H | H | $CH_2CH_2CH_2N$ ($CH_2CN$)-COMe | 406 |
| 5-120 | H | H | H | H | H | H | $CH_2CH_2CH_2N$ ($CH_2CN$)-$COC_6H_5$ | 469 |
| 5-121 | H | H | H | H | H | H | $CH_2CH_2CH_2CH_2N$ ($CH_2CN$)-COMe | 420 |
| 5-122 | H | H | H | H | H | H | $CH_2CH_2CH_2CH_2N$ ($CH_2CN$)-$COC_6H_5$ | 483 |
| 5-123 | H | H | H | H | H | H | a-3 | 366 |
| 5-124 | H | H | H | H | H | H | a-5 | 365 |
| 5-125 | H | H | H | H | H | H | a-6 | 407 |
| 5-126 | H | H | H | H | H | H | $CH_2CH_2NHSO_2Me$ | 389 |
| 5-127 | H | H | H | H | H | H | $CH_2CH_2NHSO_2NMe_2$ | 418 |
| 5-128 | H | H | H | H | H | H | $CH_2CH_2N$ (Me) $SO_2Me$ | 403 |
| 5-129 | H | H | H | H | H | H | $CH_2CH_2N$ (Me) $SO_2C_6H_5$ | 465 |
| 5-130 | H | H | H | H | H | H | $CH_2CH_2N$ (Me) $SO_2NMe_2$ | 432 |
| 5-131 | H | H | H | H | H | H | $CH_2CH_2N$ ($CH_2CH_2OH$)-$SO_2Me$ | 419 |
| 5-132 | H | H | H | H | H | H | $CH_2CH_2CH_2NHSO_2Me$ | 403 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-133 | H | H | H | H | H | H | $CH_2CH_2CH_2NHSO_2C_6H_5$ | 465 |
| 5-134 | H | H | H | H | H | H | $CH_2CH_2CH_2NHSO_2NMe_2$ | 432 |
| 5-135 | H | H | H | H | H | H | $CH_2CH_2CH_2N(Me)SO_2Me$ | 417 |
| 5-136 | H | H | H | H | H | H | $CH_2CH_2CH_2N(Me)SO_2C_6H_5$ | 479 |
| 5-137 | H | H | H | H | H | H | $CH_2CH_2CH_2N(Me)SO_2NMe_2$ | 446 |
| 5-138 | H | H | H | H | H | H | $CH_2CH_2CH_2$-$N(CH_2CH_2OH)SO_2Me$ | 433 |
| 5-139 | H | H | H | H | H | H | $CH_2CH_2CH_2CH_2NHSO_2Me$ | 417 |
| 5-140 | H | H | H | H | H | H | $CH_2CH_2CH_2CH_2NHSO_2C_6H_5$ | 479 |
| 5-141 | H | H | H | H | H | H | $CH_2CH_2CH_2CH_2NHSO_2NMe_2$ | 446 |
| 5-142 | H | H | H | H | H | H | $CH_2CH_2CH_2CH_2N$ (Me)-$SO_2Me$ | 431 |
| 5-143 | H | H | H | H | H | H | $CH_2CH_2CH_2CH_2$-$N(Me)SO_2C_6H_5$ | 493 |
| 5-144 | H | H | H | H | H | H | $CH_2CH_2CH_2CH_2$-$N(Me)SO_2NMe_2$ | 460 |
| 5-145 | H | H | H | H | H | H | $CH_2CH_2CH_2CH_2$-$N(CH_2CH_2OH)SO_2Me$ | 447 |
| 5-146 | H | H | H | H | H | H | a-66 | 443 |
| 5-147 | H | H | H | H | H | H | $CH_2CH_2NHCONHMe$ | 368 |
| 5-148 | H | H | H | H | H | H | $CH_2CH_2NHCONHCH_2Me$ | 382 |
| 5-149 | H | H | H | H | H | H | $CH_2CH_2NHCONHCH_2CH_2Me$ | 396 |
| 5-150 | H | H | H | H | H | H | $CH_2CH_2NHCONHCHMe_2$ | 396 |
| 5-151 | H | H | H | H | H | H | $CH_2CH_2NHCONHCMe_3$ | 410 |
| 5-152 | H | H | H | H | H | H | a-68 | 422 |
| 5-153 | H | H | H | H | H | H | a-69 | 436 |
| 5-154 | H | H | H | H | H | H | a-70 | 450 |
| 5-155 | H | H | H | H | H | H | a-71 | 464 |
| 5-156 | H | H | H | H | H | H | a-72 | 488 |
| 5-157 | H | H | H | H | H | H | $CH_2CH_2NHCONHC_6H_5$ | 430 |
| 5-158 | H | H | H | H | H | H | a-73 | 460 |
| 5-159 | H | H | H | H | H | H | a-74 | 446 |
| 5-160 | H | H | H | H | H | H | a-75 | 445 |
| 5-161 | H | H | H | H | H | H | a-76 | 473 |
| 5-162 | H | H | H | H | H | H | a-77 | 480 |
| 5-163 | H | H | H | H | H | H | a-78 | 480 |
| 5-164 | H | H | H | H | H | H | a-79 | 455 |
| 5-165 | H | H | H | H | H | H | a-80 | 459 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-166 | H | H | H | H | H | H | a-81 | 464 |
| 5-167 | H | H | H | H | H | H | $CH_2CH_2NHCONHCOC_6H_5$ | 458 |
| 5-168 | H | H | H | H | H | H | a-82 | 372 |
| 5-169 | H | H | H | H | H | H | a-83 | 372 |
| 5-170 | H | H | H | H | H | H | a-84 | 487 |
| 5-171 | H | H | H | H | H | H | a-85 | 474 |
| 5-172 | H | H | H | H | H | H | a-86 | 486 |
| 5-173 | H | H | H | H | H | H | a-87 | 436 |
| 5-174 | H | H | H | H | H | H | a-88 | 449 |
| 5-175 | H | H | H | H | H | H | $CH_2CH_2N(Me)CONHC_6H_5$ | 444 |
| 5-176 | H | H | H | H | H | H | $CH_2CH_2N$ (Me) CO-N(Me)$C_6H_5$ | 458 |
| 5-177 | H | H | H | H | H | H | $CH_2CH_2NHCONHCH_2CH_2OH$ | 398 |
| 5-178 | H | H | H | H | H | H | $CH_2CH_2NHCONHCH_2CH_2$-OMe | 412 |
| 5-179 | H | H | H | H | H | H | $CH_2CH_2NHCONH$-$CH_2CH_2NMe_2$ | 425 |
| 5-180 | H | H | H | H | H | H | $CH_2CH_2NHCON(Me)$-$CH_2CH_2OH$ | 412 |
| 5-181 | H | H | H | H | H | H | $CH_2CH_2NHCO$-$N(CH_2CH_2OH)_2$ | 442 |
| 5-182 | H | H | H | H | H | H | a-89 | 423 |
| 5-183 | H | H | H | H | H | H | $CH_2CH_2NHCONMe_2$ | 382 |
| 5-184 | H | H | H | H | H | H | $CH_2CH_2CH_2NHCONHMe$ | 382 |
| 5-185 | H | H | H | H | H | H | $CH_2CH_2CH_2NHCONHCH_2Me$ | 396 |
| 5-186 | H | H | H | H | H | H | $CH_2CH_2CH_2NHCONH$- | 410 |
| 5-187 | H | H | H | H | H | H | $CH_2CH_2Me$ $CH_2CH_2CH_2NHCONHCHMe_2$ | 410 |
| 5-188 | H | H | H | H | H | H | $CH_2CH_2CH_2NHCONHCMe_3$ | 424 |
| 5-189 | H | H | H | H | H | H | a-90 | 436 |
| 5-190 | H | H | H | H | H | H | a-92 | 450 |
| 5-191 | H | H | H | H | H | H | a-93 | 464 |
| 5-192 | H | H | H | H | H | H | a-94 | 478 |
| 5-193 | H | H | H | H | H | H | a-95 | 502 |
| 5-194 | H | H | H | H | H | H | a-96 | 444 |
| 5-195 | H | H | H | H | H | H | a-97 | 474 |
| 5-196 | H | H | H | H | H | H | a-98 | 460 |
| 5-197 | H | H | H | H | H | H | a-99 | 459 |
| 5-198 | H | H | H | H | H | H | a-100 | 487 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-199 | H | H | H | H | H | H | a-101 | 494 |
| 5-200 | H | H | H | H | H | H | a-102 | 494 |
| 5-201 | H | H | H | H | H | H | a-103 | 469 |
| 5-202 | H | H | H | H | H | H | $CH_2CH_2CH_2NHCONHCOC_6H_5$ | 473 |
| 5-203 | H | H | H | H | H | H | $CH_2CH_2CH_2NHCONH-SO_2C_6H_5$ | 478 |
| 5-204 | H | H | H | H | H | H | a-104 | 472 |
| 5-205 | H | H | H | H | H | H | a-105 | 520 |
| 5-206 | H | H | H | H | H | H | a-106 | 520 |
| 5-207 | H | H | H | H | H | H | a-107 | 501 |
| 5-208 | H | H | H | H | H | H | a-108 | 488 |
| 5-209 | H | H | H | H | H | H | a-109 | 500 |
| 5-210 | H | H | H | H | H | H | $CH_2CH_2CH_2NHCONH-CMe_2CH_2CMe_3$ | 450 |
| 5-211 | H | H | H | H | H | H | a-110 | 463 |
| 5-212 | H | H | H | H | H | H | $CH_2CH_2CH_2N$ (Me) CO-$NHC_6H_5$ | 458 |
| 5-213 | H | H | H | H | H | H | $CH_2CH_2CH_2N$ (Me) CO-N (Me) $C_6H_5$ | 472 |
| 5-214 | H | H | H | H | H | H | $CH_2CH_2CH_2NHCONH-CH_2CH_2OH$ | 412 |
| 5-215 | H | H | H | H | H | H | $CH_2CH_2CH_2NHCONH-CH_2CH_2OMe$ | 426 |
| 5-216 | H | H | H | H | H | H | $CH_2CH_2CH_2NHCONH-CH_2CH_2NMe_2$ | 439 |
| 5-217 | H | H | H | H | H | H | $CH_2CH_2CH_2NHCO-N(Me)CH_2CH_2OH$ | 426 |
| 5-218 | H | H | H | H | H | H | $CH_2CH_2CH_2NHCO-N(CH_2CH_2OH)_2$ | 456 |
| 5-219 | H | H | H | H | H | H | a-111 | 437 |
| 5-220 | H | H | H | H | H | H | $CH_2CH_2CH_2NHCONMe_2$ | 396 |
| 5-221 | H | H | H | H | H | H | $CH_2CH_2CH_2CH_2NHCONHMe$ | 396 |
| 5-222 | H | H | H | H | H | H | $CH_2CH_2CH_2CH_2NHCONH-CH_2Me$ | 410 |
| 5-223 | H | H | H | H | H | H | $CH_2CH_2CH_2CH_2NHCONH-CH_2CH_2Me$ | 424 |
| 5-224 | H | H | H | H | H | H | $CH_2CH_2CH_2CH_2NHCO-NHCHMe_2$ | 424 |
| 5-225 | H | H | H | H | H | H | $CH_2CH_2CH_2CH_2NHCO-NHCMe_3$ | 438 |
| 5-226 | H | H | H | H | H | H | a-123 | 450 |
| 5-227 | H | H | H | H | H | H | a-124 | 464 |
| 5-228 | H | H | H | H | H | H | a-125 | 478 |
| 5-229 | H | H | H | H | H | H | a-126 | 492 |
| 5-230 | H | H | H | H | H | H | a-127 | 516 |
| 5-231 | H | H | H | H | H | H | $CH_2CH_2CH_2CH_2NHCO-NHC_6H_5$ | 458 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-232 | H | H | H | H | H | H | a-128 | 488 |
| 5-233 | H | H | H | H | H | H | a-129 | 474 |
| 5-234 | H | H | H | H | H | H | a-130 | 473 |
| 5-235 | H | H | H | H | H | H | a-131 | 501 |
| 5-236 | H | H | H | H | H | H | a-132 | 508 |
| 5-237 | H | H | H | H | H | H | a-133 | 508 |
| 5-238 | H | H | H | H | H | H | a-134 | 483 |
| 5-239 | H | H | H | H | H | H | a-135 | 487 |
| 5-240 | H | H | H | H | H | H | a-136 | 492 |
| 5-241 | H | H | H | H | H | H | $CH_2CH_2CH_2CH_2NHCONH\text{-}COC_6H_5$ | 486 |
| 5-242 | H | H | H | H | H | H | $CH_2CH_2CH_2CH_2NHCONH\text{-}SO_2C_6H_5$ | 534 |
| 5-243 | H | H | H | H | H | H | a-137 | 534 |
| 5-244 | H | H | H | H | H | H | a-138 | 515 |
| 5-245 | H | H | H | H | H | H | a-139 | 502 |
| 5-246 | H | H | H | H | H | H | a-140 | 514 |
| 5-247 | H | H | H | H | H | H | a-141 | 464 |
| 5-248 | H | H | H | H | H | H | a-142 | 477 |
| 5-249 | H | H | H | H | H | H | $CH_2CH_2CH_2CH_2NHCONH\text{-}CMe_2CH_2CMe_3$ | 472 |
| 5-250 | H | H | H | H | H | H | a-143 | 486 |
| 5-251 | H | H | H | H | H | H | $CH_2CH_2CH_2CH_2N$ (Me) CO-$NHC_6H_5$ | 426 |
| 5-252 | H | H | H | H | H | H | $CH_2CH_2CH_2CH_2N$ (Me) CO-N (Me) $C_6H_5$ | 440 |
| 5-253 | H | H | H | H | H | H | $CH_2CH_2CH_2CH_2NHCONH\text{-}CH_2CH_2OH$ | 453 |
| 5-254 | H | H | H | H | H | H | $CH_2CH_2CH_2CH_2NHCONH\text{-}CH_2CH_2OMe$ | 440 |
| 5-255 | H | H | H | H | H | H | $CH_2CH_2CH_2CH_2NHCONH\text{-}CH_2CH_2NMe_2$ | 470 |
| 5-256 | H | H | H | H | H | H | $CH_2CH_2CH_2CH_2NHCO$-N (Me) $CH_2CH_2OH$ | 451 |
| 5-257 | H | H | H | H | H | H | $CH_2CH_2CH_2CH_2NHCON\text{-}(CH_2CH_2OH)_2$ | 410 |
| 5-258 | H | H | H | H | H | H | a-144 | 451 |
| 5-259 | H | H | H | H | H | H | $CH_2CH_2CH_2CH_2NHCONMe_2$ | 410 |
| 5-260 | H | H | H | H | H | H | $CH_2CH_2NHCSNHCH_2Me$ | 398 |
| 5-261 | H | H | H | H | H | H | $CH_2CH_2NHCSNHCH_2CH_2Me$ | 412 |
| 5-262 | H | H | H | H | H | H | $CH_2CH_2NHCSNHCHMe_2$ | 412 |
| 5-263 | H | H | H | H | H | H | $CH_2CH_2NHCSNHCMe_3$ | 426 |
| 5-264 | H | H | H | H | H | H | a-112 | 372 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-265 | H | H | H | H | H | H | $CH_2CH_2NHCSNHC_6H_5$ | 446 |
| 5-266 | H | H | H | H | H | H | $CH_2CH_2NHCSNHCH_2CH_2OH$ | 414 |
| 5-267 | H | H | H | H | H | H | $CH_2CH_2NHCSNHCH_2CH_2$-OMe | 428 |
| 5-268 | H | H | H | H | H | H | $CH_2CH_2NHCSNHCH_2CH_2$-$NMe_2$ | 441 |
| 5-269 | H | H | H | H | H | H | $CH_2CH_2NHCSN$ (Me)-$CH_2CH_2OH$ | 428 |
| 5-270 | H | H | H | H | H | H | $CH_2CH_2NHCS$-N $(CH_2CH_2OH)_2$ | 458 |
| 5-271 | H | H | H | H | H | H | a-113 | 439 |
| 5-272 | H | H | H | H | H | H | $CH_2CH_2NHCSNMe_2$ | 398 |
| 5-273 | H | H | H | H | H | H | $CH_2CH_2NHCOOMe$ | 369 |
| 5-274 | H | H | H | H | H | H | $CH_2CH_2NHCOOCH_2Me$ | 383 |
| 5-275 | H | H | H | H | H | H | $CH_2CH_2NHCOOCH_2CH_2Me$ | 397 |
| 5-276 | H | H | H | H | H | H | $CH_2CH_2NHCOOCHMe_2$ | 397 |
| 5-277 | H | H | H | H | H | H | a-114 | 437 |
| 5-278 | H | H | H | H | H | H | $CH_2CH_2NHCOOC_6H_5$ | 431 |
| 5-279 | H | H | H | H | H | H | $CH_2CH_2NHCOOCH_2CH_2OH$ | 399 |
| 5-280 | H | H | H | H | H | H | $CH_2CH_2NHCOOCH_2CH_2OMe$ | 413 |
| 5-281 | H | H | H | H | H | H | $CH_2CH_2NHCOOCH_2CH_2NMe_2$ | 426 |
| 5-282 | H | H | H | H | H | H | a-115 | 392 |
| 5-283 | H | H | H | H | H | H | a-116 | 392 |
| 5-284 | H | H | H | H | H | H | a-9 | 365 |
| 5-285 | H | H | H | H | H | H | a-117 | 379 |
| 5-286 | H | H | H | H | H | H | a-118 | 393 |
| 5-287 | H | H | H | H | H | H | a-8 | 310 |
| 5-288 | H | H | H | H | H | H | $CH_2CH_2OCH_2CH_2OMe$ | 370 |
| 5-289 | H | H | H | H | H | H | CH $(CH_2OH)_2$ | 341 |
| 5-290 | H | H | H | H | H | H | a-4 | 366 |
| 5-291 | Cl | H | H | H | H | H | H | 302 |
| 5-292 | Br | H | H | H | H | H | H | 346 |
| 5-293 | OH | H | H | H | H | H | H | 284 |
| 5-294 | Me | H | H | H | H | H | H | 282 |
| 5-295 | $CH{=}CH_2$ | H | H | H | H | H | H | 294 |
| 5-296 | $C{\equiv}CC_6H_5$ | H | H | H | H | H | H | 368 |
| 5-297 | OMe | H | H | H | H | H | H | 298 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-298 | $NH_2$ | H | H | H | H | H | H | 283 |
| 5-299 | NHMe | H | H | H | H | H | H | 297 |
| 5-300 | $NMe_2$ | H | H | H | H | H | H | 311 |
| 5-301 | $NHC_6H_5$ | H | H | H | H | H | H | 359 |
| 5-302 | OH | H | H | H | H | H | $CH_2CH_2CH_2CH_2COOH$ | 384 |
| 5-303 | OH | H | H | H | H | H | $CH_2CH_2CH_2CH_2CH_2OH$ | 370 |
| 5-304 | OH | H | H | H | H | H | $CH_2CH_2SCOMe$ | 386 |
| 5-305 | OH | H | H | H | H | H | $CH_2CH_2SH$ | 344 |
| 5-306 | OH | H | H | H | H | H | COMe | 326 |
| 5-307 | OH | H | H | H | H | H | $SO_2Me$ | 362 |
| 5-308 | OH | H | H | H | H | H | $CH_2CH_2CH_2NHCONHC_6H_5$ | 460 |
| 5-309 | OH | H | H | H | H | H | a-91 | 466 |
| 5-311 | OH | H | H | H | H | H | Me | 298 |
| 5-312 | OH | H | H | H | H | H | a-121 | 338 |
| 5-313 | OH | H | H | H | H | H | a-122 | 323 |
| 5-314 | OH | H | H | H | H | H | Bn | 374 |
| 5-315 | OH | H | H | H | H | H | a-2 | 404 |
| 5-316 | OH | H | H | H | H | Me | Me | 312 |
| 5-317 | OH | H | H | H | H | H | $COC_6H_5$ | 388 |
| 5-318 | OH | H | H | H | H | H | $SO_2C_6H_5$ | 424 |
| 5-319 | OH | H | H | H | H | H | $CH_2COOH$ | 342 |
| 5-320 | OH | H | H | H | H | H | $CH_2CH_2COOH$ | 356 |
| 5-321 | OH | H | H | H | H | H | $CH_2CH_2CH_2COOH$ | 370 |
| 5-322 | OH | H | H | H | H | H | $CH_2CN$ | 323 |
| 5-323 | OH | H | H | H | H | H | $CH_2CH_2CN$ | 337 |
| 5-324 | OH | H | H | H | H | H | $CH_2CH_2CH_2CN$ | 351 |
| 5-325 | OH | H | H | H | H | H | $CH_2CH_2CH_2CH_2CN$ | 365 |
| 5-326 | OH | H | H | H | H | H | $CH_2CH_2OH$ | 328 |
| 5-327 | OH | H | H | H | H | H | $CH_2CH$ (Me) OH | 342 |
| 5-328 | OH | H | H | H | H | H | CH (Me) $CH_2OH$ | 342 |
| 5-329 | OH | H | H | H | H | H | $CH_2CH_2CH_2OH$ | 342 |
| 5-330 | OH | H | H | H | H | H | $CH_2CH$ (OH) $CH_2OH$ | 358 |
| 5-331 | OH | H | H | H | H | H | $CH_2CH$ (Me) $CH_2OH$ | 356 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-332 | OH | H | H | H | H | H | $CH_2CH_2CH_2CH_2OH$ | 356 |
| 5-333 | OH | H | H | H | H | H | $CH_2CH_2OCONHMe$ | 385 |
| 5-334 | OH | H | H | H | H | H | a-7 | 439 |
| 5-335 | OH | H | H | H | H | H | $CH_2CH_2OCONHC_6H_5$ | 447 |
| 5-336 | OH | H | H | H | H | H | $CH_2CH_2CH_2OCONHMe$ | 399 |
| 5-337 | OH | H | H | H | H | H | a-48 | 453 |
| 5-338 | OH | H | H | H | H | H | $CH_2CH_2CH_2OCONHC_6H_5$ | 461 |
| 5-339 | OH | H | H | H | H | H | $CH_2CH_2CH_2CH_2OCONHMe$ | 413 |
| 5-340 | OH | H | H | H | H | H | a-49 | 467 |
| 5-341 | OH | H | H | H | H | H | $CH_2CH_2CH_2CH_2OCONHC_6H_5$ | 475 |
| 5-342 | OH | H | H | H | H | H | $CH_2CH_2CH_2SH$ | 358 |
| 5-343 | OH | H | H | H | H | H | $CH_2CH_2SCOC_6H_5$ | 448 |
| 5-344 | OH | H | H | H | H | H | $CH_2CH_2CH_2SCOMe$ | 400 |
| 5-345 | OH | H | H | H | H | H | $CH_2CH_2CH_2SCOC_6H_5$ | 462 |
| 5-346 | OH | H | H | H | H | H | $CH_2CH_2OMe$ | 342 |
| 5-347 | OH | H | H | H | H | H | $CH_2CH_2CH_2OMe$ | 356 |
| 5-348 | OH | H | H | H | H | H | $CH_2CH_2CH_2CH_2OMe$ | 370 |
| 5-349 | OH | H | H | H | H | H | $CH_2CH_2CH_2CH_2CH_2OMe$ | 384 |
| 5-350 | OH | H | H | H | H | H | $CH_2CH_2OCH_2CH_2OH$ | 372 |
| 5-351 | OH | H | H | H | H | H | $CH_2CH_2CH_2OCH_2CH_2OH$ | 386 |
| 5-352 | OH | H | H | H | H | H | $CH_2CH_2CH_2CH_2OCH_2CH_2OH$ | 400 |
| 5-353 | OH | H | H | H | H | H | $CH\ (CH_2OH)CH_2NH_2$ | 357 |
| 5-354 | OH | H | H | H | H | H | $CH\ (CH_2OH)\ CH_2NHCOMe$ | 399 |
| 5-355 | OH | H | H | H | H | H | a-51 | 482 |
| 5-356 | OH | H | H | H | H | H | $CH(CH_2OH)CH_2NHCO$-<br>$NHC_6H_5$ | 476 |
| 5-357 | OH | H | H | H | H | H | $CH_2CH_2NH_2$ | 327 |
| 5-358 | OH | H | H | H | H | H | $CH_2CH_2CH_2NH_2$ | 341 |
| 5-359 | OH | H | H | H | H | H | $CH_2CH\ (OH)\ CH_2NH_2$ | 357 |
| 5-360 | OH | H | H | H | H | H | $CH_2CH\ (OH)\ CH_2NHMe$ | 371 |
| 5-361 | OH | H | H | H | H | H | $CH_2CH\ (OH)\ CH_2NMe_2$ | 385 |
| 5-362 | OH | H | H | H | H | H | $CH_2CH_2CH_2CH_2NH_2$ | 355 |
| 5-363 | OH | H | H | H | H | H | $CH_2CH_2CH_2CH_2CH_2NH_2$ | 369 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-364 | OH | H | H | H | H | H | $CH_2CH_2NHMe$ | 341 |
| 5-365 | OH | H | H | H | H | H | $CH_2CH_2CH_2NHMe$ | 355 |
| 5-366 | OH | H | H | H | H | H | $CH_2CH_2CH_2CH_2NHMe$ | 369 |
| 5-367 | OH | H | H | H | H | H | $CH_2CH_2CH_2CH_2CH_2NHMe$ | 383 |
| 5-368 | OH | H | H | H | H | H | $CH_2CH_2NMe_2$ | 355 |
| 5-369 | OH | H | H | H | H | H | $CH_2CH_2CH_2NMe_2$ | 369 |
| 5-370 | OH | H | H | H | H | H | $CH_2CH_2CH_2CH_2NMe_2$ | 383 |
| 5-371 | OH | H | H | H | H | H | $CH_2CH_2CH_2CH_2CH_2NMe_2$ | 397 |
| 5-372 | OH | H | H | H | H | H | $CH_2CH_2NHCH_2CH_2OH$ | 371 |
| 5-373 | OH | H | H | H | H | H | $CH_2CH_2CH_2NHCH_2CH_2OH$ | 385 |
| 5-374 | OH | H | H | H | H | H | $CH_2CH_2CH_2CH_2NHCH_2CH_2$-OH | 399 |
| 5-375 | OH | H | H | H | H | H | $CH_2CONH_2$ | 341 |
| 5-376 | OH | H | H | H | H | H | $CH_2CH_2CONH_2$ | 355 |
| 5-377 | OH | H | H | H | H | H | $CH_2CH_2CH_2CONH_2$ | 369 |
| 5-378 | OH | H | H | H | H | H | $CH_2CH_2NHCOMe$ | 369 |
| 5-379 | OH | H | H | H | H | H | $CH(CH_2NHCOMe)_2$ | 440 |
| 5-380 | OH | H | H | H | H | H | $CH_2CH_2NHCOCH_2Me$ | 383 |
| 5-381 | OH | H | H | H | H | H | $CH_2CH_2NHCOCH_2CH_2Me$ | 397 |
| 5-382 | OH | H | H | H | H | H | $CH_2CH_2NHCOCHMe_2$ | 397 |
| 5-383 | OH | H | H | H | H | H | $CH_2CH_2NHCOCMe_3$ | 411 |
| 5-384 | OH | H | H | H | H | H | $CH_2CH_2NHCOCH_2OH$ | 385 |
| 5-385 | OH | H | H | H | H | H | $CH_2CH_2NHCOCH_2OMe$ | 399 |
| 5-386 | OH | H | H | H | H | H | $CH_2CH_2NHCOCH_2NMe_2$ | 412 |
| 5-387 | OH | H | H | H | H | H | $CH_2CH_2NHCOC_6H_5$ | 431 |
| 5-388 | OH | H | H | H | H | H | a-52 | 432 |
| 5-389 | OH | H | H | H | H | H | a-53 | 437 |
| 5-390 | OH | H | H | H | H | H | a-54 | 421 |
| 5-391 | OH | H | H | H | H | H | a-55 | 433 |
| 5-392 | OH | H | H | H | H | H | $CH_2CH_2NHCOCH_2NHCOMe$ | 426 |
| 5-393 | OH | H | H | H | H | H | $CH_2CH_2NHCOCH_2NHCO$-$CH_2CHMe_2$ | 468 |
| 5-394 | OH | H | H | H | H | H | $CH_2CH_2NHCOCH_2NHCOC_6H_5$ | 488 |
| 5-395 | OH | H | H | H | H | H | $CH_2CH_2CH_2NHCOMe$ | 383 |
| 5-396 | OH | H | H | H | H | H | $CH_2CH_2CH_2NHCOCH_2Me$ | 397 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-397 | OH | H | H | H | H | H | $CH_2CH_2CH_2NHCOCH_2CH_2Me$ | 411 |
| 5-398 | OH | H | H | H | H | H | $CH_2CH_2CH_2NHCOCHMe_2$ | 411 |
| 5-399 | OH | H | H | H | H | H | $CH_2CH_2CH_2NHCOCMe_3$ | 425 |
| 5-400 | OH | H | H | H | H | H | $CH_2CH_2CH_2CH_2NHCOMe$ | 397 |
| 5-401 | OH | H | H | H | H | H | $CH_2CH_2CH_2CH_2NHCOCH_2Me$ | 411 |
| 5-402 | OH | H | H | H | H | H | $CH_2CH_2CH_2CH_2NHCO\text{-}CH_2CH_2Me$ | 425 |
| 5-403 | OH | H | H | H | H | H | $CH_2CH_2CH_2CH_2NHCOCHMe_2$ | 425 |
| 5-404 | OH | H | H | H | H | H | $CH_2CH_2CH_2CH_2NHCOCMe_3$ | 439 |
| 5-405 | OH | H | H | H | H | H | $CH_2CH_2N(Me)$ COMe | 383 |
| 5-406 | OH | H | H | H | H | H | $CH_2CH_2N$ (Me) $COC_6H_5$ | 445 |
| 5-407 | OH | H | H | H | H | H | $CH_2CH_2CH_2N$ (Me) COMe | 397 |
| 5-408 | OH | H | H | H | H | H | $CH_2CH_2CH_2N$ (Me) $COC_6H_5$ | 459 |
| 5-409 | OH | H | H | H | H | H | $CH_2CH_2CH_2CH_2N$ (Me) -COMe | 411 |
| 5-410 | OH | H | H | H | H | H | $CH_2CH_2CH_2CH_2N$ (Me) $-COC_6H_5$ | 473 |
| 5-411 | OH | H | H | H | H | H | $CH_2CH_2N(CH_2CH_2OH)$-COMe | 413 |
| 5-412 | OH | H | H | H | H | H | $CH_2CH_2N(CH_2CH_2OH)\text{-}COC_6H_5$ | 476 |
| 5-413 | OH | H | H | H | H | H | $CH_2CH_2CH_2N(CH_2CH_2OH)$-COMe | 427 |
| 5-414 | OH | H | H | H | H | H | $CH_2CH_2CH_2N$ (CH2CH2OH)-$COC_6H_5$ | 490 |
| 5-415 | OH | H | H | H | H | H | $CH_2CH_2CH_2CH_2$-N $(CH_2CH_2OH)$ COMe | 441 |
| 5-416 | OH | H | H | H | H | H | $CH_2CH_2CH_2CH_2\text{-}N(CH_2CH_2OH)COC_6H_5$ | 504 |
| 5-417 | OH | H | H | H | H | H | $CH_2CH_2N$ $(CH_2CN)$ COMe | 408 |
| 5-418 | OH | H | H | H | H | H | $CH_2CH_2N(CH_2CN)COC_6H_5$ | 471 |
| 5-419 | OH | H | H | H | H | H | $CH_2CH_2CH_2N$ $(CH_2CN)$-COMe | 422 |
| 5-420 | OH | H | H | H | H | H | $CH_2CH_2CH_2N$ $(CH_2CN)\text{-}COC_6H_5$ | 485 |
| 5-421 | OH | H | H | H | H | H | $CH_2CH_2CH_2CH_2N(CH_2CN\text{-})COMe$ | 436 |
| 5-422 | OH | H | H | H | H | H | $CH_2CH_2CH_2CH_2N$ $(CH_2CN)\text{-}COC_6H_5$ | 499 |
| 5-423 | OH | H | H | H | H | H | a-3 | 382 |
| 5-424 | OH | H | H | H | H | H | a-5 | 381 |
| 5-425 | OH | H | H | H | H | H | a-6 | 423 |
| 5-426 | OH | H | H | H | H | H | $CH_2CH_2NHSO_2Me$ | 405 |
| 5-427 | OH | H | H | H | H | H | $CH_2CH_2NHSO_2NMe_2$ | 434 |
| 5-428 | OH | H | H | H | H | H | $CH_2CH_2N$ (Me) $SO_2Me$ | 419 |
| 5-429 | OH | H | H | H | H | H | $CH_2CH_2N(Me)SO_2C_6H_5$ | 481 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-430 | OH | H | H | H | H | H | $CH_2CH_2N$ (Me) $SO_2NMe_2$ | 448 |
| 5-431 | OH | H | H | H | H | H | $CH_2CH_2N$ ($CH_2CH_2OH$)-$SO_2Me$ | 435 |
| 5-432 | OH | H | H | H | H | H | $CH_2CH_2CH_2NHSO_2Me$ | 419 |
| 5-433 | OH | H | H | H | H | H | $CH_2CH_2CH_2NHSO_2C_6H_5$ | 481 |
| 5-434 | OH | H | H | H | H | H | $CH_2CH_2CH_2NHSO_2NMe_2$ | 448 |
| 5-435 | OH | H | H | H | H | H | $CH_2CH_2CH_2N(Me)SO_2Me$ | 433 |
| 5-436 | OH | H | H | H | H | H | $CH_2CH_2CH_2N$ (Me) $SO_2C_6H_5$ | 495 |
| 5-437 | OH | H | H | H | H | H | $CH_2CH_2CH_2N$ (Me) $SO_2NMe_2$ | 462 |
| 5-438 | OH | H | H | H | H | H | $CH_2CH_2CH_2$- | 449 |
| 5-439 | OH | H | H | H | H | H | $N(CH_2CH_2OH)SO_2Me$ $CH_2CH_2CH_2CH_2NHSO_2Me$ | 433 |
| 5-440 | OH | H | H | H | H | H | $CH_2CH_2CH_2CH_2NHSO_2C_6H_5$ | 495 |
| 5-441 | OH | H | H | H | H | H | $CH_2CH_2CH_2CH_2NHSO_2NMe_2$ | 462 |
| 5-442 | OH | H | H | H | H | H | $CH_2CH_2CH_2CH_2N(Me)$-$SO_2Me$ | 447 |
| 5-443 | OH | H | H | H | H | H | $CH_2CH_2CH_2CH_2$-$N(Me)SO_2C_6H_5$ | 509 |
| 5-444 | OH | H | H | H | H | H | $CH_2CH_2CH_2CH_2$ -$N(Me)SO_2NMe_2$ | 476 |
| 5-445 | OH | H | H | H | H | H | $CH_2CH_2CH_2CH_2$-N ($CH_2CH_2OH$) $SO_2Me$ | 463 |
| 5-446 | OH | H | H | H | H | H | a-66 | 459 |
| 5-447 | OH | H | H | H | H | H | $CH_2CH_2NHCONHMe$ | 384 |
| 5-448 | OH | H | H | H | H | H | $CH_2CH_2NHCONHCH_2Me$ | 398 |
| 5-449 | OH | H | H | H | H | H | $CH_2CH_2NHCONHCH_2CH_2Me$ | 412 |
| 5-450 | OH | H | H | H | H | H | $CH_2CH_2NHCONHCHMe_2$ | 412 |
| 5-451 | OH | H | H | H | H | H | $CH_2CH_2NHCONHCMe_3$ | 426 |
| 5-452 | OH | H | H | H | H | H | a-68 | 438 |
| 5-453 | OH | H | H | H | H | H | a-69 | 452 |
| 5-454 | OH | H | H | H | H | H | a-70 | 466 |
| 5-455 | OH | H | H | H | H | H | a-71 | 480 |
| 5-456 | OH | H | H | H | H | H | a-72 | 504 |
| 5-457 | OH | H | H | H | H | H | $CH_2CH_2NHCONHC_6H_5$ | 446 |
| 5-458 | OH | H | H | H | H | H | a-73 | 476 |
| 5-459 | OH | H | H | H | H | H | a-74 | 462 |
| 5-460 | OH | H | H | H | H | H | a-75 | 461 |
| 5-461 | OH | H | H | H | H | H | a-76 | 489 |
| 5-462 | OH | H | H | H | H | H | a-77 | 496 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-463 | OH | H | H | H | H | H | a-78 | 496 |
| 5-464 | OH | H | H | H | H | H | a-79 | 471 |
| 5-465 | OH | H | H | H | H | H | a-80 | 475 |
| 5-466 | OH | H | H | H | H | H | a-81 | 480 |
| 5-467 | OH | H | H | H | H | H | $CH_2CH_2NHCONHCOC_6H_5$ | 474 |
| 5-468 | OH | H | H | H | H | H | a-82 | 388 |
| 5-469 | OH | H | H | H | H | H | a-83 | 388 |
| 5-470 | OH | H | H | H | H | H | a-84 | 503 |
| 5-471 | OH | H | H | H | H | H | a-85 | 490 |
| 5-472 | OH | H | H | H | H | H | a-86 | 502 |
| 5-473 | OH | H | H | H | H | H | a-87 | 452 |
| 5-474 | OH | H | H | H | H | H | a-88 | 465 |
| 5-475 | OH | H | H | H | H | H | $CH_2CH_2N$ (Me) $CONHC_6H_5$ | 460 |
| 5-476 | OH | H | H | H | H | H | $CH_2CH_2N$ (Me) CO- N (Me) $C_6H_5$ | 474 |
| 5-477 | OH | H | H | H | H | H | $CH_2CH_2NHCONHCH_2CH_2OH$ | 414 |
| 5-478 | OH | H | H | H | H | H | $CH_2CH_2NHCONHCH_2CH_2$- OMe | 428 |
| 5-479 | OH | H | H | H | H | H | $CH_2CH_2NHCONH$-$CH_2CH_2NMe_2$ | 441 |
| 5-480 | OH | H | H | H | H | H | $CH_2CH_2NHCON(Me)$-$CH_2CH_2OH$ | 428 |
| 5-481 | OH | H | H | H | H | H | $CH_2CH_2NHCO$-$N(CH_2CH_2OH)_2$ | 458 |
| 5-482 | OH | H | H | H | H | H | a-89 | 439 |
| 5-483 | OH | H | H | H | H | H | $CH_2CH_2NHCONMe_2$ | 398 |
| 5-484 | OH | H | H | H | H | H | $CH_2CH_2CH_2NHCONHMe$ | 398 |
| 5-485 | OH | H | H | H | H | H | $CH_2CH_2CH_2NHCONHCH_2Me$ | 412 |
| 5-486 | OH | H | H | H | H | H | $CH_2CH_2CH_2NHCONH$-$CH_2CH_2Me$ | 426 |
| 5-487 | OH | H | H | H | H | H | $CH_2CH_2CH_2NHCONHCHMe_2$ | 426 |
| 5-488 | OH | H | H | H | H | H | $CH_2CH_2CH_2NHCONHCMe_3$ | 440 |
| 5-489 | OH | H | H | H | H | H | a-90 | 452 |
| 5-490 | OH | H | H | H | H | H | a-92 | 466 |
| 5-491 | OH | H | H | H | H | H | a-93 | 480 |
| 5-492 | OH | H | H | H | H | H | a-94 | 494 |
| 5-493 | OH | H | H | H | H | H | a-95 | 518 |
| 5-494 | OH | H | H | H | H | H | a-96 | 460 |
| 5-495 | OH | H | H | H | H | H | a-97 | 490 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-496 | OH | H | H | H | H | H | a-98 | 476 |
| 5-497 | OH | H | H | H | H | H | a-99 | 475 |
| 5-498 | OH | H | H | H | H | H | a-100 | 503 |
| 5-499 | OH | H | H | H | H | H | a-101 | 510 |
| 5-500 | OH | H | H | H | H | H | a-102 | 510 |
| 5-501 | OH | H | H | H | H | H | a-103 | 485 |
| 5-502 | OH | H | H | H | H | H | $CH_2CH_2CH_2NHCONHCOC_6H_5$ | 489 |
| 5-503 | OH | H | H | H | H | H | $CH_2CH_2CH_2NHCONH\text{-}SO_2C_6H_5$ | 494 |
| 5-504 | OH | H | H | H | H | H | a-104 | 488 |
| 5-505 | OH | H | H | H | H | H | a-105 | 536 |
| 5-506 | OH | H | H | H | H | H | a-106 | 536 |
| 5-507 | OH | H | H | H | H | H | a-107 | 517 |
| 5-508 | OH | H | H | H | H | H | a-108 | 504 |
| 5-509 | OH | H | H | H | H | H | a-109 | 516 |
| 5-510 | OH | H | H | H | H | H | $CH_2CH_2CH_2NHCONH\text{-}CMe_2CH_2CMe_3$ | 466 |
| 5-511 | OH | H | H | H | H | H | a-110 | 479 |
| 5-512 | OH | H | H | H | H | H | $CH_2CH_2CH_2N(Me)CO\text{-}NHC_6H_5$ | 474 |
| 5-513 | OH | H | H | H | H | H | $CH_2CH_2CH_2N(Me)CO\text{-}N(Me)C_6H_5$ | 488 |
| 5-514 | OH | H | H | H | H | H | $CH_2CH_2CH_2NHCONH\text{-}CH_2CH_2OH$ | 428 |
| 5-515 | OH | H | H | H | H | H | $CH_2CH_2CH_2NHCONH\text{-}CH_2CH_2OMe$ | 442 |
| 5-516 | OH | H | H | H | H | H | $CH_2CH_2CH_2NHCONH\text{-}CH_2CH_2NMe_2$ | 455 |
| 5-517 | OH | H | H | H | H | H | $CH_2CH_2CH_2NHCON$ $(Me)\text{-}CH_2CH_2OH$ | 442 |
| 5-518 | OH | H | H | H | H | H | $CH_2CH_2CH_2NHCO\text{-}$ | 472 |
| 5-519 | OH | H | H | H | H | H | N $(CH_2CH_2OH)_2$ a-111 | 453 |
| 5-520 | OH | H | H | H | H | H | $CH_2CH_2CH_2NHCONMe_2$ | 412 |
| 5-521 | OH | H | H | H | H | H | $CH_2CH_2CH_2CH_2NHCONHMe$ | 412 |
| 5-522 | OH | H | H | H | H | H | $CH_2CH_2CH_2CH_2NHCO\text{-}NHCH_2Me$ | 426 |
| 5-523 | OH | H | H | H | H | H | $CH_2CH_2CH_2CH_2NHCONHCH_2$ $CH_2Me$ | 440 |
| 5-524 | OH | H | H | H | H | H | $CH_2CH_2CH_2CH_2NHCO\text{-}NHCHMe_2$ | 440 |
| 5-525 | OH | H | H | H | H | H | $CH_2CH_2CH_2CH_2NHCO\text{-}NHCMe_3$ | 454 |
| 5-526 | OH | H | H | H | H | H | a-123 | 466 |
| 5-527 | OH | H | H | H | H | H | a-124 | 480 |
| 5-528 | OH | H | H | H | H | H | a-125 | 494 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-529 | OH | H | H | H | H | H | a-126 | 508 |
| 5-530 | OH | H | H | H | H | H | a-127 | 532 |
| 5-531 | OH | H | H | H | H | H | $CH_2CH_2CH_2CH_2NHCO\text{-}NHC_6H_5$ | 474 |
| 5-532 | OH | H | H | H | H | H | a-128 | 504 |
| 5-533 | OH | H | H | H | H | H | a-129 | 490 |
| 5-534 | OH | H | H | H | H | H | a-130 | 489 |
| 5-535 | OH | H | H | H | H | H | a-131 | 517 |
| 5-536 | OH | H | H | H | H | H | a-132 | 524 |
| 5-537 | OH | H | H | H | H | H | a-133 | 524 |
| 5-538 | OH | H | H | H | H | H | a-134 | 499 |
| 5-539 | OH | H | H | H | H | H | a-135 | 503 |
| 5-540 | OH | H | H | H | H | H | a-136 | 508 |
| 5-541 | OH | H | H | H | H | H | $CH_2CH_2CH_2CH_2NHCONH\text{-}COC_6H_5$ | 502 |
| 5-542 | OH | H | H | H | H | H | $CH_2CH_2CH_2CH_2NHCONH\text{-}SO_2C_6H_5$ | 550 |
| 5-543 | OH | H | H | H | H | H | a-137 | 550 |
| 5-544 | OH | H | H | H | H | H | a-138 | 531 |
| 5-545 | OH | H | H | H | H | H | a-139 | 518 |
| 5-546 | OH | H | H | H | H | H | a-140 | 530 |
| 5-547 | OH | H | H | H | H | H | a-141 | 480 |
| 5-548 | OH | H | H | H | H | H | a-142 | 493 |
| 5-549 | OH | H | H | H | H | H | $CH_2CH_2CH_2CH_2NHCONH\text{-}CMe_2CH_2CMe_3$ | 488 |
| 5-550 | OH | H | H | H | H | H | a-143 | 502 |
| 5-551 | OH | H | H | H | H | H | $CH_2CH_2CH_2CH_2N$ (Me) $CO\text{-}NHC_6H_5$ | 442 |
| 5-552 | OH | H | H | H | H | H | $CH_2CH_2CH_2CH_2N$ (Me) CO-N (Me) $C_6H_5$ | 456 |
| 5-553 | OH | H | H | H | H | H | $CH_2CH_2CH_2CH_2NHCO\text{-}NHCH_2CH_2OH$ | 469 |
| 5-554 | OH | H | H | H | H | H | $CH_2CH_2CH_2CH_2NHCONH\text{-}CH_2CH_2OMe$ | 456 |
| 5-555 | OH | H | H | H | H | H | $CH_2CH_2CH_2CH_2NHCONH\text{-}CH_2CH_2NMe_2$ | 486 |
| 5-556 | OH | H | H | H | H | H | $CH_2CH_2CH_2CH_2NHCO\text{-}N$ (Me) $CH_2CH_2OH$ | 467 |
| 5-557 | OH | H | H | H | H | H | $CH_2CH_2CH_2CH_2NHCO\text{-}N$ $(CH_2CH_2OH)_2$ | 426 |
| 5-558 | OH | H | H | H | H | H | a-144 | 467 |
| 5-559 | OH | H | H | H | H | H | $CH_2CH_2CH_2CH_2NHCONMe_2$ | 426 |
| 5-560 | OH | H | H | H | H | H | $CH_2CH_2NHCSNHCH_2Me$ | 414 |
| 5-561 | OH | H | H | H | H | H | $CH_2CH_2NHCSNHCH_2CH_2Me$ | 428 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-562 | OH | H | H | H | H | H | $CH_2CH_2NHCSNHCHMe_2$ | 428 |
| 5-563 | OH | H | H | H | H | H | $CH_2CH_2NHCSNHCMe_3$ | 442 |
| 5-564 | OH | H | H | H | H | H | a-112 | 388 |
| 5-565 | OH | H | H | H | H | H | $CH_2CH_2NHCSNHC_6H_5$ | 462 |
| 5-566 | OH | H | H | H | H | H | $CH_2CH_2NHCSNHCH_2CH_2OH$ $CH_2CH_2NHCSNH$- | 430 |
| 5-567 | OH | H | H | H | H | H | $CH_2CH_2OMe$ | 444 |
| 5-568 | OH | H | H | H | H | H | $CH_2CH_2NHCSNH$-$CH_2CH_2NMe_2$ | 457 |
| 5-569 | OH | H | H | H | H | H | $CH_2CH_2NHCSN$ (Me)-$CH_2CH_2OH$ | 444 |
| 5-570 | OH | H | H | H | H | H | $CH_2CH_2NHCS$- N $(CH_2CH_2OH)_2$ | 474 |
| 5-571 | OH | H | H | H | H | H | a-113 | 455 |
| 5-572 | OH | H | H | H | H | H | $CH_2CH_2NHCSNMe_2$ | 414 |
| 5-573 | OH | H | H | H | H | H | $CH_2CH_2NHCOOMe$ | 385 |
| 5-574 | OH | H | H | H | H | H | $CH_2CH_2NHCOOCH_2Me$ | 399 |
| 5-575 | OH | H | H | H | H | H | $CH_2CH_2NHCOOCH_2CH_2Me$ | 413 |
| 5-576 | OH | H | H | H | H | H | $CH_2CH_2NHCOOCHMe_2$ | 413 |
| 5-577 | OH | H | H | H | H | H | a-114 | 453 |
| 5-578 | OH | H | H | H | H | H | $CH_2CH_2NHCOOC_6H_5$ | 447 |
| 5-579 | OH | H | H | H | H | H | $CH_2CH_2NHCOOCH_2CH_2OH$ | 415 |
| 5-580 | OH | H | H | H | H | H | $CH_2CH_2NHCOOCH_2CH_2OMe$ | 429 |
| 5-581 | OH | H | H | H | H | H | $CH_2CH_2NHCOOCH_2CH_2NMe_2$ | 442 |
| 5-582 | OH | H | H | H | H | H | a-115 | 408 |
| 5-583 | OH | H | H | H | H | H | a-116 | 408 |
| 5-584 | OH | H | H | H | H | H | a-9 | 381 |
| 5-585 | OH | H | H | H | H | H | a-117 | 395 |
| 5-586 | OH | H | H | H | H | H | a-118 | 409 |
| 5-587 | OH | H | H | H | H | H | a-8 | 326 |
| 5-588 | OH | H | H | H | H | H | $CH_2CH_2OCH_2CH_2OMe$ | 386 |
| 5-589 | OH | H | H | H | H | H | $CH(CH_2OH)_2$ | 357 |
| 5-590 | OH | H | H | H | H | H | a-4 | 382 |
| 5-591 | H | Cl | H | H | H | H | H | 302 |
| 5-592 | H | Br | H | H | H | H | H | 346 |
| 5-593 | H | OH | H | H | H | H | H | 284 |
| 5-594 | H | Me | H | H | H | H | H | 282 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-595 | H | $CH=CH_2$ | H | H | H | H | H | 294 |
| 5-596 | H | $C≡CC_6H_5$ | H | H | H | H | H | 368 |
| 5-597 | H | OMe | H | H | H | H | H | 298 |
| 5-598 | H | $NH_2$ | H | H | H | H | H | 283 |
| 5-599 | H | NHMe | H | H | H | H | H | 297 |
| 5-600 | H | $NMe_2$ | H | H | H | H | H | 311 |
| 5-601 | H | $NHC_6H_5$ | H | H | H | H | H | 359 |
| 5-602 | H | H | Cl | H | H | H | H | 302 |
| 5-603 | H | H | Br | H | H | H | H | 346 |
| 5-604 | H | H | OH | H | H | H | H | 284 |
| 5-605 | H | H | Me | H | H | H | H | 282 |
| 5-606 | H | H | $CH=CH_2$ | H | H | H | H | 294 |
| 5-607 | H | H | $C≡CC_6H_5$ | H | H | H | H | 368 |
| 5-608 | H | H | OMe | H | H | H | H | 298 |
| 5-609 | H | H | $NH_2$ | H | H | H | H | 283 |
| 5-610 | H | H | NHMe | H | H | H | H | 297 |
| 5-611 | H | H | $NMe_2$ | H | H | H | H | 311 |
| 5-612 | H | H | $NHC_6H_5$ | H | H | H | H | 359 |
| 5-613 | H | H | H | Cl | H | H | H | 302 |
| 5-614 | H | H | H | Br | H | H | H | 346 |
| 5-615 | H | H | H | OH | H | H | H | 284 |
| 5-616 | H | H | H | Me | H | H | H | 282 |
| 5-617 | H | H | H | $CH=CH_2$ | H | H | H | 294 |
| 5-618 | H | H | H | $C=C-C_6H_5$ | H | H | H | 368 |
| 5-619 | H | H | H | OMe | H | H | H | 298 |
| 5-620 | H | H | H | $NH_2$ | H | H | H | 283 |
| 5-621 | H | H | H | NHMe | H | H | H | 297 |
| 5-622 | H | H | H | $NMe_2$ | H | H | H | 311 |
| 5-623 | H | H | H | $NHC_6H_5$ | H | H | H | 359 |
| 5-624 | H | H | H | H | Cl | H | H | 302 |
| 5-625 | H | H | H | H | Br | H | H | 346 |
| 5-626 | H | H | H | H | OH | H | H | 284 |
| 5-627 | H | H | H | H | Me | H | H | 282 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 5-628 | H | H | H | H | $CH=CH_2$ | H | H | 294 |
| 5-629 | H | H | H | H | $C≡C-C_6H_5$ | H | H | 368 |
| 5-630 | H | H | H | H | 0Me | H | H | 298 |
| 5-631 | H | H | H | H | $NH_2$ | H | H | 283 |
| 5-632 | H | H | H | H | NHMe | H | H | 297 |
| 5-633 | H | H | H | H | $NMe_2$ | H | H | 311 |
| 5-634 | H | H | H | H | $NHC_6H_5$ | H | H | 359 |

Example 6-1: 1-Amino-4-[(2,3-dihydro-1H-1,5-diazaphenalen-1-yl)methyl]cyclohexane

Step A

1,4-Dioxa-spiro[4.5]dec-8-carbaldehyde

The title compound was obtained from commercially available 4-oxo-cyclohexanecarboxylic acid ethyl ester (Tokyo Kasei Kogyo) by referring to a known publication (Ferrino, S.A., Maldonado, L.A., Synth. Commun.14, 925 (1984)).

Step B

5-(1,4-Dioxa-spiro[4.5]dec-8-ylmethyl)amino-4-vinylisoquinoline (Intermediate 66)

By using 5-amino-4-vinylisoquinoline (Intermediate 1) obtained in Example 1-1, Step A, and 1,4-dioxa-spiro [4.5]dec-8-carbaldehyde obtained in Step A mentioned above, reductive alkylation was performed according to the method described in Example 1-1, Step B to obtain the title compound.
MS (m/z) : 325 (MH+)

Step C

2,3-Dihydro-1-(1,4-dioxa-spiro[4.5]dec-8-yl)methyl-1,5-diazaphenalene (Intermediate 67)

By using 5-(1,4-dioxa-spiro[4.5]dec-8-ylmethyl)amino-4-vinylisoquinoline (Intermediate 66) obtained in Step B mentioned above, cyclization was performed according to the method described in Example 1-1, Step C to obtain the title compound.
MS (m/z): 325 (MH+)

Step D

4-[(2,3-Dihydro-1H-1,5-diazaphenalen-1-yl)methyl]cyclohexanone (Intermediate 68)

By using 2,3-dihydro-1-(1,4-dioxa-spiro[4.5]dec-8-yl)methyl-1,5-diazaphenalene (Intermediate 67) obtained in Step C mentioned above, deprotection was performed for the protective group according to the method described in Example 5-1, Step C to obtain the title compound.
MS (m/z): 281 (MH+)

Step E

1-Benzylamino-4-[(2, 3-dihydro-1H-1,5-diazaphenalen-1-yl)methyl]cyclohexane (Intermediate 69)

By using 4-[(2,3-dihydro-1H-1,5-diazaphenalen-1-yl)methyl]cyclohexanone (Intermediate 68) obtained in Step D mentioned above, and benzylamine, reductive amination was performed according to the method described in Example 5-1, Step D to obtain the title compound.
MS (m/z): 372 (MH+)

Step F

1-Amino-4- [(2,3-dihydro-1H-1,5-diazaphenalen-1-yl)methyl]cyclohexane

By using 1-benzylamino-4-[(2,3-dihydro-1H-1,5-diazaphenalen-1-yl)methyl]cyclohexane (Intermediate 69) obtained in Step E mentioned above, debenzylation was performed according to the method described in Example 5-1, Step E to obtain the title compound.
MS (m/z): 282 (MH+)

Examples 6-2 to 6-290

The compounds of Examples 6-2 to 6-290 were obtained by using 4-[(2,3-dihydro-1H-1,5-diazaphenalen-1-yl) methyl]cyclohexanone (Intermediate 68) obtained in Example 6-1, Step D, or

1-amino-4-[(2,3-dihydro-1H-1,5-diazaphenalen-1-yl)methyl]cyclohexane obtained in Example 6-1 according to the methods described in Examples 5-2 to 5-320.

Example 6-291: 1-Amino-4-[(6-chloro-2,3-dihydro-1H-1,5-diazaphenalen-1-yl)methyl]cyclohexane

Step A

1-Chloro-5-(1,4-dioxa-spiro[4.5]dec-8-ylmethyl)amino-4-vinylisoquinoline (Intermediate 70)

By using 5-amino-1-chloro-4-vinylisoquinoline (Intermediate 8) obtained in Example 1-321, Step D, and 1,4-dioxa-spiro[4.5]dec-8-carbaldehyde, reductive alkylation was performed according to the method described in Example 5-291, Step A to obtain the title compound.
MS (m/z) : 359 (MH+)

Step B

6-Chloro-2,3-dihydro-1-(1,4-dioxa-spiro[4.5]dec-8-yl)methyl-1,5-diazaphenalene (Intermediate 71)

By using 1-chloro-5-(1,4-dioxa-spiro[4.5]dec-8-ylmethyl)amino-4-vinylisoquinoline (Intermediate 70) obtained in Step A mentioned above, cyclization was performed according to the method described in Example 5-291, Step B to obtain the title compound.
MS (m/z): 359 (MH+)

Step C

4-[(6-Chloro-2,3-dihydro-1H-1,5-diazaphenalen-1-yl)methyl]cyclohexanone (Intermediate 72)

By using 6-chloro-2,3-dihydro-1-(1,4-dioxa-spiro[4.5]dec-8-yl)methyl-1,5-diazaphenalene (Intermediate 71) obtained in Step B mentioned above, deprotection was performed for the protective group according to the method described in Example 5-291, Step C to obtain the title compound.
MS (m/z): 315 (MH+)

Step D

1-(4-Methoxybenzylamino)-4-[(6-chloro-2,3-dihydro-1H-1,5-diazaphenalen-1-yl)methyl]cyclohexane (Intermediate 73)

By using 4-[(6-chloro-2,3-dihydro-1H-1,5-diazaphenalen-1-yl)methyl]cyclohexanone (Intermediate 72) obtained in Step C mentioned above, and 4-methoxybenzylamine, reductive amination was performed according to the method described in Example 5-1, Step D to obtain the title compound.
MS (m/z): 436 (MH+)

Step E

1-Amino-4-[(6-chloro-2,3-dihydro-1H-1,5-diazaphenalen-1-yl)methyl]cyclohexane

By using 1-(4-methoxybenzylamino)-4-[(6-chloro-2,3-dihydro-1H-1,5-diazaphenalen-1-yl)methyl]cyclohexane (Intermediate 73) obtained in Step D mentioned above, debenzylation was performed according to the method described in Example 5-291, Step E to obtain the title compound as a trifluoroacetate .
MS (m/z): 316 (MH+)

Example 6-292

The compound of Example 6-292 was obtained by referring to the method used for the compound of Example 6-291.

Example 6-293

The compound of Example 6-293 was obtained in the same manner as that used for the compound of Example

5-293.

Examples 6-294 to 6-301

The compounds of Examples 6-294 to 6-301 were obtained by using 6-chloro-2,3-dihydro-1-(1,4-dioxa-spiro [4.5]dec-8-yl)methyl-1,5-diazaphenalene (Intermediate 71) obtained in Example 6-291, Step B according to the methods described in Examples 5-294 to 5-301;

Examples 6-302 to 6-590

The compounds of Examples 6-302 to 6-590 were obtained by using 4-[(6-chloro-2,3-dihydro-1H-1,5-diazaphenalen-1-yl)methyl]cyclohexanone (Intermediate 72) obtained in Example 6-291, Step C, or 1-amino-4- [(6-chloro-2,3-dihydro-1H-1,5-diazaphenalen-1-yl)methyl]cyclohexane obtained in Example 6-291 in the same manner as that used for the compounds of Examples 5-302 to 5-590.

Example 6-591: 1-Amino-4-[(4-chloro-2,3-dihydro-1H-1,5-diazaphenalen-1-yl)methyl]cyclohexane

By using 5-amino-3-chloro-4-vinylisoquinoline, reductive alkylation, and cyclization were performed according to the method described in Example 5-291 to obtain 4-chloro-2,3-dihydro-1-(1,4-dioxa-spiro[4.5]dec-8-yl)methyl-1,5-diazaphenalene, and then deprotection for the protective group, reductive amination with 4-methoxybenzylamine, and debenzylation were performed to obtain the title compound as a trifluoroacetate .
MS (m/z): 316 (MH+)

Example 6-592

The compound of Example 6-592 was obtained by referring to the method used for the compound of Example 6-591.

Examples 6-593 to 6-601

The compounds of Examples 6-593 to 6-601 were obtained by using 4-chloro-2,3-dihydro-1-(1,4-dioxa-spiro [4.5]dec-8-yl)methyl-1,5-diazaphenalene obtained in Example 6-591 according to the methods described in Example 5-593 to 5-601.

Example 6-602

The compound of Example 6-602 was obtained by referring to the method used for the compound of Example 6-603 mentioned below.

Example 6-603: 1-Amino-4-[(7-bromo-2,3-dihydro-1H-1,5-diazaphenalen-1-yl)methyl]cyclohexane

Step A

7-Bromo-2,3-dihydro-1-(1,4-dioxa-spiro[4.5]dec-8-yl)methyl-1,5-diazaphenalene (Intermediate 74)

By using 2,3-dihydro-1-(1,4-dioxa-spiro[4.5]dec-8-yl)methyl-1,5-diazaphenalene (Intermediate 67) obtained in Example 6-1, Step C, bromination was performed according to the method described in Example 1-663, Step A to obtain the title compound.
MS (m/z): 403 (MH+)

Step B

1-Amino-4-[(7-bromo-2,3-dihydro-1H-1,5-diazaphenalen-1-yl)methyl]cyclohexane

By using 7-bromo-2,3-dihydro-1-(1,4-dioxa-spiro[4.5]dec-8-yl)methyl-1,5-diazaphenalene (Intermediate 74) obtained in Step A mentioned above, deprotection for the protective group, reductive amination with 4-methoxybenzylamine, and debenzylation were performed according to the method described in Example 5-291 to obtain the title compound as a trifluoroacetate .

MS (m/z): 360 (MH+)

Examples 6-604 to 6-612

The compounds of Examples 6-604 to 6-612 were obtained by using 7-bromo-2,3-dihydro-1-(1,4-dioxa-spiro [4.5]dec-8-yl)methyl-1,5-diazaphenalene (Intermediate 74) obtained in Example 6-603, Step A in the same manner as that used for the compounds of Examples 5-293 to 5-301.

Example 6-61

The compound of Example 6-613 was obtained by referring to the method used for the compound of Example 6-614 mentioned below.

Example 6-614: 1-Amino-4-[(8-bromo-2,3-dihydro-1H-1,5-diazaphenalen-1-yl)methyl]cyclohexane

Step A

8-Bromo-2,3-dihydro-1-(1,4-dioxa-spiro[4,5]dec-8-yl)methyl-1,5-diazaphenalene (Intermediate 75)

By using 2,3-dihydro-1-(1,4-dioxa-spiro[4.5]dec-8-yl)methyl-1,5-diazaphenalene (Intermediate 67) obtained in Example 6-1, Step C, bromination was performed according to the method described in Example 1-663, Step A to obtain the title compound.
MS (m/z): 403 (MH+)

Step B

1-Amino-4-[(7-bromo-2,3-dihydro-1H-1,5-diazaphenalen-1-yl)methyl]cyclohexane

By using 8-bromo-2,3-dihydro-1-(1,4-dioxa-spiro[4.5]dec-8-yl)methyl-1,5-diazaphenalene (Intermediate 75) obtained in Step A mentioned above, deprotection for the protective group, reductive amination with 4-methoxybenzylamine, and debenzylation were performed according to the method described in Example 5-291 to obtain the title compound as a trifluoroacetate.
MS (m/z) : 360 (MH+)

Examples 6-615 to 6-623

The compounds of Examples 6-615 to 6-623 were obtained by using 8-bromo-2,3-dihydro-1-(1,4-dioxa-spiro [4.5]dec-8-yl)methyl-1,5-diazaphenalene (Intermediate 75) obtained in Example 6-614, Step A in the same manner as that used for the compounds of Examples 5-615 to 5-623.

Example 6-624

The compound of Example 6-624 was obtained by referring to the method used for the compound of Example 6-625 mentioned below.

Example 6-625

The compound of Example 6-625 was obtained by using 9-amino-2,3-dihydro-1-(1,4-dioxa-spiro[4.5]dec-8-yl) methyl-1,5-diazaphenalene obtained in Example 6-631 mentioned below to perform known diazotization-bromination with referring to the method used for the compound of Example 1-685 to obtain 9-bromo-2,3-dihydro-1-(1,4-dioxa-spiro [4.5]dec-8-yl)methyl-1,5-diazaphenalene, and then using the same step as that used for the compound of Example 5-603.

Examples 6-626 to 6-630

The compounds of Examples 6-626 to 6-630 were obtained by using 9-bromo-2,3-dihydro-1-(1,4-dioxa-spiro [4.5]dec-8-yl)methyl-l,5-diazaphenalene obtained in Example 6-625 in the same manner as that used for the compounds of Examples 5-293 to 5-297.

Example 6-631

The compound of Example 6-631 was obtained by using 7-bromo-2,3-dihydro-1-(1,4-dioxa-spiro[4.5]dec-8-yl)methyl-1,5-diazaphenalene (Intermediate 74) obtained in Example 6-603, Step A to perform nitration (9-position), and reduction (bromine atom→hydrogen atom at the 7-position, nitro group→amino group at the 9-position) in the same manner as that for Example 1-691 to obtain 9-amino-2,3-dihydro-1-(1,4-dioxa-spiro[4.5]dec-8-yl)methyl-1,5-diazaphenalene, and then performing deprotection for the protective group, reductive amination with benzylamine, and debenzylation according to the method described in Example 6-1.

Examples 6-632 to 6-634

The compounds of Examples 6-632 to 6-634 were obtained by using 9-bromo-2,3-dihydro-1-(1,4-dioxa-spiro[4.5]dec-8-yl)methyl-1,5-diazaphenalene obtained in Example 6-625 in the same manner as that used for the compounds of Examples 5-299 to 5-301.

[Table 6]

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-1 | H | H | H | H | H | H | H | 282 |
| 6-2 | H | H | H | H | H | H | $CH_2CH_2CH_2CH_2COOH$ | 382 |
| 6-3 | H | H | H | H | H | H | $CH_2CH_2CH_2CH_2CH_2OH$ | 368 |
| 6-4 | H | H | H | H | H | H | $CH_2CH_2SCOMe$ | 384 |
| 6-5 | H | H | H | H | H | H | $CH_2CH_2SH$ | 342 |
| 6-6 | H | H | H | H | H | H | COMe | 324 |
| 6-7 | H | H | H | H | H | H | $SO_2Me$ | 360 |
| 6-8 | H | H | H | H | H | H | $CH_2CH_2CH_2NHCONHC_6H_5$ | 458 |
| 6-9 | H | H | H | H | H | H | a-91 | 464 |
| 6-11 | H | H | H | H | H | H | Me | 296 |
| 6-12 | H | H | H | H | H | H | a-121 | 336 |
| 6-13 | H | H | H | H | H | H | a-122 | 321 |
| 6-14 | H | H | H | H | H | H | Bn | 372 |
| 6-15 | H | H | H | H | H | H | a-2 | 402 |
| 6-16 | H | H | H | H | H | Me | Me | 310 |
| 6-17 | H | H | H | H | H | H | $COC_6H_5$ | 386 |
| 6-18 | H | H | H | H | H | H | $SO_2C_6H_5$ | 422 |
| 6-19 | H | H | H | H | H | H | $CH_2COOH$ | 340 |
| 6-20 | H | H | H | H | H | H | $CH_2CH_2COOH$ | 354 |
| 6-21 | H | H | H | H | H | H | $CH_2CH_2CH_2COOH$ | 368 |
| 6-22 | H | H | H | H | H | H | $CH_2CN$ | 321 |
| 6-23 | H | H | H | H | H | H | $CH_2CH_2CN$ | 335 |
| 6-24 | H | H | H | H | H | H | $CH_2CH_2CH_2CN$ | 349 |
| 6-25 | H | H | H | H | H | H | $CH_2CH_2CH_2CH_2CN$ | 363 |
| 6-26 | H | H | H | H | H | H | $CH_2CH_2OH$ | 326 |
| 6-27 | H | H | H | H | H | H | $CH_2CH$ (Me) OH | 340 |
| 6-28 | H | H | H | H | H | H | CH (Me) $CH_2OH$ | 340 |
| 6-29 | H | H | H | H | H | H | $CH_2CH_2CH_2OH$ | 340 |
| 6-30 | H | H | H | H | H | H | $CH_2CH(OH)CH_2OH$ | 356 |
| 6-31 | H | H | H | H | H | H | $CH_2CH$ (Me) $CH_2OH$ | 354 |
| 6-32 | H | H | H | H | H | H | $CH_2CH_2CH_2CH_2OH$ | 354 |
| 6-33 | H | H | H | H | H | H | $CH_2CH_2OCONHMe$ | 383 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-34 | H | H | H | H | H | H | a-7 | 437 |
| 6-35 | H | H | H | H | H | H | $CH_2CH_2OCONHC_6H_5$ | 445 |
| 6-36 | H | H | H | H | H | H | $CH_2CH_2CH_2OCONHMe$ | 397 |
| 6-37 | H | H | H | H | H | H | a-48 | 451 |
| 6-38 | H | H | H | H | H | H | $CH_2CH_2CH_2OCONHC_6H_5$ | 459 |
| 6-39 | H | H | H | H | H | H | $CH_2CH_2CH_2CH_2OCONHMe$ | 411 |
| 6-40 | H | H | H | H | H | H | a-49 | 465 |
| 6-41 | H | H | H | H | H | H | $CH_2CH_2CH_2CH_2OCONH-C_6H_5$ | 473 |
| 6-42 | H | H | H | H | H | H | $CH_2CH_2CH_2SH$ | 356 |
| 6-43 | H | H | H | H | H | H | $CH_2CH_2SCOC_6H_5$ | 446 |
| 6-44 | H | H | H | H | H | H | $CH_2CH_2CH_2SCOMe$ | 398 |
| 6-45 | H | H | H | H | H | H | $CH_2CH_2CH_2SCOC_6H_5$ | 460 |
| 6-46 | H | H | H | H | H | H | $CH_2CH_2OMe$ | 340 |
| 6-47 | H | H | H | H | H | H | $CH_2CH_2CH_2OMe$ | 354 |
| 6-48 | H | H | H | H | H | H | $CH_2CH_2CH_2CH_2OMe$ | 368 |
| 6-49 | H | H | H | H | H | H | $CH_2CH_2CH_2CH_2CH_2OMe$ | 382 |
| 6-50 | H | H | H | H | H | H | $CH_2CH_2OCH_2CH_2OH$ | 370 |
| 6-51 | H | H | H | H | H | H | $CH_2CH_2CH_2OCH_2CH_2OH$ | 384 |
| 6-52 | H | H | H | H | H | H | $CH_2CH_2CH_2CH_2OCH_2CH_2-OH$ | 398 |
| 6-53 | H | H | H | H | H | H | $CH (CH_2OH) CH_2NH_2$ | 355 |
| 6-54 | H | H | H | H | H | H | $CH(CH_2OH)CH_2NHCOMe$ | 397 |
| 6-55 | H | H | H | H | H | H | a-51 | 480 |
| 6-56 | H | H | H | H | H | H | $CH (CH_2OH) CH_2NHCO-NHC_6H_5$ | 474 |
| 6-57 | H | H | H | H | H | H | $CH_2CH_2NH_2$ | 325 |
| 6-58 | H | H | H | H | H | H | $CH_2CH_2CH_2NH_2$ | 339 |
| 6-59 | H | H | H | H | H | H | $CH_2CH (OH) CH_2NH_2$ | 355 |
| 6-60 | H | H | H | H | H | H | $CH_2CH (OH) CH_2NHMe$ | 369 |
| 6-61 | H | H | H | H | H | H | $CH_2CH (OH) CH_2NMe_2$ | 383 |
| 6-62 | H | H | H | H | H | H | $CH_2CH_2CH_2CH_2NH_2$ | 353 |
| 6-63 | H | H | H | H | H | H | $CH_2CH_2CH_2CH_2CH_2NH_2$ | 367 |
| 6-64 | H | H | H | H | H | H | $CH_2CH_2NHMe$ | 339 |
| 6-65 | H | H | H | H | H | H | $CH_2CH_2CH_2NHMe$ | 353 |
| 6-66 | H | H | H | H | H | H | $CH_2CH_2CH_2CH_2NHMe$ | 367 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-67 | H | H | H | H | H | H | $CH_2CH_2CH_2CH_2CH_2NHMe$ | 381 |
| 6-68 | H | H | H | H | H | H | $CH_2CH_2NMe_2$ | 353 |
| 6-69 | H | H | H | H | H | H | $CH_2CH_2CH_2NMe_2$ | 367 |
| 6-70 | H | H | H | H | H | H | $CH_2CH_2CH_2CH_2NMe_2$ | 381 |
| 6-71 | H | H | H | H | H | H | $CH_2CH_2CH_2CH_2CH_2NMe_2$ | 395 |
| 6-72 | H | H | H | H | H | H | $CH_2CH_2NHCH_2CH_2OH$ | 369 |
| 6-73 | H | H | H | H | H | H | $CH_2CH_2CH_2NHCH_2CH_2OH$ | 383 |
| 6-74 | H | H | H | H | H | H | $CH_2CH_2CH_2CH_2NH$-$CH_2CH_2OH$ | 397 |
| 6-75 | H | H | H | H | H | H | $CH_2CONH_2$ | 339 |
| 6-76 | H | H | H | H | H | H | $CH_2CH_2CONH_2$ | 353 |
| 6-77 | H | H | H | H | H | H | $CH_2CH_2CH_2CONH_2$ | 367 |
| 6-78 | H | H | H | H | H | H | $CH_2CH_2NHCOMe$ | 367 |
| 6-79 | H | H | H | H | H | H | $CH\ (CH_2NHCOMe)_2$ | 438 |
| 6-80 | H | H | H | H | H | H | $CH_2CH_2NHCOCH_2Me$ | 381 |
| 6-81 | H | H | H | H | H | H | $CH_2CH_2NHCOCH_2CH_2Me$ | 395 |
| 6-82 | H | H | H | H | H | H | $CH_2CH_2NHCOCHMe_2$ | 395 |
| 6-83 | H | H | H | H | H | H | $CH_2CH_2NHCOCMe_3$ | 409 |
| 6-84 | H | H | H | H | H | H | $CH_2CH_2NHCOCH_2OH$ | 383 |
| 6-85 | H | H | H | H | H | H | $CH_2CH_2NHCOCH_2OMe$ | 397 |
| 6-86 | H | H | H | H | H | H | $CH_2CH_2NHCOCH_2NMe_2$ | 410 |
| 6-87 | H | H | H | H | H | H | $CH_2CH_2NHCOC_6H_5$ | 429 |
| 6-88 | H | H | H | H | H | H | a-52 | 430 |
| 6-89 | H | H | H | H | H | H | a-53 | 435 |
| 6-90 | H | H | H | H | H | H | a-54 | 419 |
| 6-91 | H | H | H | H | H | H | a-55 | 431 |
| 6-92 | H | H | H | H | H | H | $CH_2CH_2NHCOCH_2NHCOMe$ | 424 |
| 6-93 | H | H | H | H | H | H | $CH_2CH_2NHCOCH_2NHCO$- $CH_2CHMe_2$ | 466 |
| 6-94 | H | H | H | H | H | H | $CH_2CH_2NHCOCH_2NHCO$-$C_6H_5$ | 486 |
| 6-95 | H | H | H | H | H | H | $CH_2CH_2CH_2NHCOMe$ | 381 |
| 6-96 | H | H | H | H | H | H | $CH_2CH_2CH_2NHCOCH_2Me$ | 395 |
| 6-97 | H | H | H | H | H | H | $CH_2CH_2CH_2NHCO$- $CH_2CH_2Me$ | 409 |
| 6-98 | H | H | H | H | H | H | $CH_2CH_2CH_2NHCOCHMe_2$ | 409 |
| 6-99 | H | H | H | H | H | H | $CH_2CH_2CH_2NHCOCMe_3$ | 423 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-100 | H | H | H | H | H | H | $CH_2CH_2CH_2CH_2NHCOMe$ | 395 |
| 6-101 | H | H | H | H | H | H | $CH_2CH_2CH_2CH_2NHCO\text{-}CH_2Me$ | 409 |
| 6-102 | H | H | H | H | H | H | $CH_2CH_2CH_2CH_2NHCO\text{-}CH_2CH_2Me$ | 423 |
| 6-103 | H | H | H | H | H | H | $CH_2CH_2CH_2CH_2CHCO\text{-}CHMe_2$ | 423 |
| 6-104 | H | H | H | H | H | H | $CH_2CH_2CH_2CH_2NHCOCMe_3$ | 437 |
| 6-105 | H | H | H | H | H | H | $CH_2CH_2N(Me)COMe$ | 381 |
| 6-106 | H | H | H | H | H | H | $CH_2CH_2N(Me)COC_6H_5$ | 443 |
| 6-107 | H | H | H | H | H | H | $CH_2CH_2CH_2N(Me)COMe$ | 395 |
| 6-108 | H | H | H | H | H | H | $CH_2CH_2CH_2N(Me)COC_6H_5$ | 457 |
| 6-109 | H | H | H | H | H | H | $CH_2CH_2CH_2CH_2N(Me)\text{-}COMe$ | 409 |
| 6-110 | H | H | H | H | H | H | $CH_2CH_2CH_2CH_2N(Me)\ \text{-}COC_6H_5$ | 471 |
| 6-111 | H | H | H | H | H | H | $CH_2CH_2N(CH_2CH_2OH)\text{-}COMe$ | 411 |
| 6-112 | H | H | H | H | H | H | $CH_2CH_2N(CH_2CH_2OH)\text{-}COC_6H_5$ | 474 |
| 6-113 | H | H | H | H | H | H | $CH_2CH_2CH_2\text{-}N(CH_2CH_2OH)COMe$ | 425 |
| 6-114 | H | H | H | H | H | H | $CH_2CH_2CH_2\text{-}N(CH_2CH_2OH)COC_6H_5$ | 488 |
| 6-115 | H | H | H | H | H | H | $CH_2CH_2CH_2CH_2N(CH_2CH_2\ OH)\ COMe$ | 439 |
| 6-116 | H | H | H | H | H | H | $CH_2CH_2CH_2CH_2\text{-}N(CH_2CH_2OH)COC_6H_5$ | 502 |
| 6-117 | H | H | H | H | H | H | $CH_2CH_2N(CH_2CN)COMe$ | 406 |
| 6-118 | H | H | H | H | H | H | $CH_2CH_2N(CH_2CN)COC_6H_5$ | 469 |
| 6-119 | H | H | H | H | H | H | $CH_2CH_2CH_2\text{-}N(CH_2CN)COMe$ | 420 |
| 6-120 | H | H | H | H | H | H | $CH_2CH_2CH_2N(CH_2CN)COC_6H_5$ | 483 |
| 6-121 | H | H | H | H | H | H | $CH_2CH_2CH_2CH_2\text{-}N(CH_2CN)COMe$ | 434 |
| 6-122 | H | H | H | H | H | H | $CH_2CH_2CH_2CH_2\text{-}N(CH_2CN)COC_6H_5$ | 497 |
| 6-123 | H | H | H | H | H | H | a-3 | 380 |
| 6-124 | H | H | H | H | H | H | a-5 | 379 |
| 6-125 | H | H | H | H | H | H | a-6 | 421 |
| 6-126 | H | H | H | H | H | H | $CH_2CH_2NHSO_2Me$ | 403 |
| 6-127 | H | H | H | H | H | H | $CH_2CH_2NHSO_2NMe_2$ | 432 |
| 6-128 | H | H | H | H | H | H | $CH_2CH_2N\ (Me)\ SO_2Me$ | 417 |
| 6-129 | H | H | H | H | H | H | $CH_2CH_2N(Me)SO_2C_6H_5$ | 479 |
| 6-130 | H | H | H | H | H | H | $CH_2CH_2N(Me)SO_2NMe_2$ | 446 |
| 6-131 | H | H | H | H | H | H | $CH_2CH_2N\ (CH_2CH_2OH)\text{-}SO_2Me$ | 433 |
| 6-132 | H | H | H | H | H | H | $CH_2CH_2CH_2NHSO_2Me$ | 417 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-133 | H | H | H | H | H | H | $CH_2CH_2CH_2NHSO_2C_6H_5$ | 479 |
| 6-134 | H | H | H | H | H | H | $CH_2CH_2CH_2NHSO_2NMe_2$ | 446 |
| 6-135 | H | H | H | H | H | H | $CH_2CH_2CH_2N$ (Me) $SO_2Me$ | 431 |
| 6-136 | H | H | H | H | H | H | $CH_2CH_2CH_2N$ (Me) $-SO_2C_6H_5$ | 493 |
| 6-137 | H | H | H | H | H | H | $CH_2CH_2CH_2N$ (Me) $-SO_2NMe_2$ | 460 |
| 6-138 | H | H | H | H | H | H | $CH_2CH_2CH_2$-N ($CH_2CH_2OH$) $SO_2Me$ | 447 |
| 6-139 | H | H | H | H | H | H | $CH_2CH_2CH_2CH_2NHSO_2Me$ | 431 |
| 6-140 | H | H | H | H | H | H | $CH_2CH_2CH_2CH_2NH-SO_2C_6H_5$ | 493 |
| 6-141 | H | H | H | H | H | H | $CH_2CH_2CH_2CH_2NH-SO_2NMe_2$ | 460 |
| 6-142 | H | H | H | H | H | H | $CH_2CH_2CH_2CH_2N$ (Me)-$SO_2Me$ | 445 |
| 6-143 | H | H | H | H | H | H | $CH_2CH_2CH_2CH_2N$ (Me) $-SO_2C_6H_5$ | 507 |
| 6-144 | H | H | H | H | H | H | $CH_2CH_2CH_2CH_2N$ (Me) $-SO_2NMe_2$ | 474 |
| 6-145 | H | H | H | H | H | H | $CH_2CH_2CH_2CH_2$-N ($CH_2CH_2OH$) $SO_2Me$ | 461 |
| 6-146 | H | H | H | H | H | H | a-66 | 457 |
| 6-147 | H | H | H | H | H | H | $CH_2CH_2NHCONHMe$ | 382 |
| 6-148 | H | H | H | H | H | H | $CH_2CH_2NHCONHCH_2Me$ | 396 |
| 6-149 | H | H | H | H | H | H | $CH_2CH_2NHCONH-CH_2CH_2Me$ | 410 |
| 6-150 | H | H | H | H | H | H | $CH_2CH_2NHCONHCHMe_2$ | 410 |
| 6-151 | H | H | H | H | H | H | $CH_2CH_2NHCONHCMe_3$ | 424 |
| 6-152 | H | H | H | H | H | H | a-68 | 436 |
| 6-153 | H | H | H | H | H | H | a-69 | 450 |
| 6-154 | H | H | H | H | H | H | a-70 | 464 |
| 6-155 | H | H | H | H | H | H | a-71 | 478 |
| 6-156 | H | H | H | H | H | H | a-72 | 502 |
| 6-157 | H | H | H | H | H | H | $CH_2CH_2NHCONHC_6H_5$ | 444 |
| 6-158 | H | H | H | H | H | H | a-73 | 474 |
| 6-159 | H | H | H | H | H | H | a-74 | 460 |
| 6-160 | H | H | H | H | H | H | a-75 | 459 |
| 6-161 | H | H | H | H | H | H | a-76 | 487 |
| 6-162 | H | H | H | H | H | H | a-77 | 494 |
| 6-163 | H | H | H | H | H | H | a-78 | 494 |
| 6-164 | H | H | H | H | H | H | a-79 | 469 |
| 6-165 | H | H | H | H | H | H | a-80 | 473 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-166 | H | H | H | H | H | H | a-81 | 478 |
| 6-167 | H | H | H | H | H | H | $CH_2CH_2NHCONHCOC_6H_5$ | 472 |
| 6-168 | H | H | H | H | H | H | a-82 | 386 |
| 6-169 | H | H | H | H | H | H | a-83 | 386 |
| 6-170 | H | H | H | H | H | H | a-84 | 501 |
| 6-171 | H | H | H | H | H | H | a-85 | 488 |
| 6-172 | H | H | H | H | H | H | a-86 | 500 |
| 6-173 | H | H | H | H | H | H | a-87 | 450 |
| 6-174 | H | H | H | H | H | H | a-88 | 463 |
| 6-175 | H | H | H | H | H | H | $CH_2CH_2N(Me)CONHC_6H_5$ | 458 |
| 6-176 | H | H | H | H | H | H | $CH_2CH_2N$ (Me) CO-N(Me)$C_6H_5$ | 472 |
| 6-177 | H | H | H | H | H | H | $CH_2CH_2NHCONHCH_2CH_2$-OH | 412 2 |
| 6-178 | H | H | H | H | H | H | $CH_2CH_2NHCONHCH_2CH_2$-OMe | 426 |
| 6-179 | H | H | H | H | H | H | $CH_2CH_2NHCONHCH_2CH_2$-$NMe_2$ | 439 |
| 6-180 | H | H | H | H | H | H | $CH_2CH_2NHCON(Me)$ -$CH_2CH_2OH$ | 426 |
| 6-181 | H | H | H | H | H | H | $CH_2CH_2NHCO$-$N(CH_2CH_2OH)_2$ | 456 |
| 6-182 | H | H | H | H | H | H | a-89 | 437 |
| 6-183 | H | H | H | H | H | H | $CH_2CH_2NHCONMe_2$ | 396 |
| 6-184 | H | H | H | H | H | H | $CH_2CH_2CH_2NHCONHMe$ | 396 |
| 6-185 | H | H | H | H | H | H | $CH_2CH_2CH_2NHCONH$-$CH_2Me$ | 410 |
| 6-186 | H | H | H | H | H | H | $CH_2CH_2CH_2NHCONH$-$CH_2CH_2Me$ | 424 |
| 6-187 | H | H | H | H | H | H | $CH_2CH_2CH_2NHCONH$-$CHMe_2$ | 424 |
| 6-188 | H | H | H | H | H | H | $CH_2CH_2CH_2NHCONHCMe_3$ | 438 |
| 6-189 | H | H | H | H | H | H | a-90 | 450 |
| 6-190 | H | H | H | H | H | H | a-92 | 464 |
| 6-191 | H | H | H | H | H | H | a-93 | 478 |
| 6-192 | H | H | H | H | H | H | a-94 | 492 |
| 6-193 | H | H | H | H | H | H | a-95 | 516 |
| 6-194 | H | H | H | H | H | H | a-96 | 458 |
| 6-195 | H | H | H | H | H | H | a-97 | 488 |
| 6-196 | H | H | H | H | H | H | a-98 | 474 |
| 6-197 | H | H | H | H | H | H | a-99 | 473 |
| 6-198 | H | H | H | H | H | H | a-100 | 501 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-199 | H | H | H | H | H | H | a-101 | 508 |
| 6-200 | H | H | H | H | H | H | a-102 | 508 |
| 6-201 | H | H | H | H | H | H | a-103 | 483 |
| 6-202 | H | H | H | H | H | H | $CH_2CH_2CH_2NHCONH\text{-}COC_6H_5$ | 487 |
| 6-203 | H | H | H | H | H | H | $CH_2CH_2CH_2NHCONH\text{-}SO_2C_6H_5$ | 492 |
| 6-204 | H | H | H | H | H | H | a-104 | 486 |
| 6-205 | H | H | H | H | H | H | a-105 | 534 |
| 6-206 | H | H | H | H | H | H | a-106 | 534 |
| 6-207 | H | H | H | H | H | H | a-107 | 515 |
| 6-208 | H | H | H | H | H | H | a-108 | 502 |
| 6-209 | H | H | H | H | H | H | a-109 | 514 |
| 6-210 | H | H | H | H | H | H | $CH_2CH_2CH_2NHCONH\text{-}CMe_2CH_2CMe_3$ | 464 |
| 6-211 | H | H | H | H | H | H | a-110 | 477 |
| 6-212 | H | H | H | H | H | H | $CH_2CH_2CH_2N(Me)CO\text{-}NHC_6H_5$ | 472 |
| 6-213 | H | H | H | H | H | H | $CH_2CH_2CH_2N(Me)CO\text{-}N(Me)C_6H_5$ | 486 |
| 6-214 | H | H | H | H | H | H | $CH_2CH_2CH_2NHCONH\text{-}CH_2CH_2OH$ | 426 |
| 6-215 | H | H | H | H | H | H | $CH_2CH_2CH_2NHCONH\text{-}CH_2CH_2OMe$ | 440 |
| 6-216 | H | H | H | H | H | H | $CH_2CH_2CH_2NHCONH\text{-}CH_2CH_2NMe_2$ | 453 |
| 6-217 | H | H | H | H | H | H | $CH_2CH_2CH_2NHCO\text{-}N(Me)CH_2CH_2OH$ | 440 |
| 6-218 | H | H | H | H | H | H | $CH_2CH_2CH_2NHCO\text{-}N(CH_2CH_2OH)_2$ | 470 |
| 6-219 | H | H | H | H | H | H | a-111 | 451 |
| 6-220 | H | H | H | H | H | H | $CH_2CH_2CH_2NHCONMe_2$ | 410 |
| 6-221 | H | H | H | H | H | H | $CH_2CH_2CH_2CH_2NHCO\text{-}NHMe$ | 410 |
| 6-222 | H | H | H | H | H | H | $CH_2CH_2CH_2CH_2NHCO\text{-}NHCH_2Me$ | 424 |
| 6-223 | H | H | H | H | H | H | $CH_2CH_2CH_2CH_2NHCO\text{-}NHCH_2CH_2Me$ | 438 |
| 6-224 | H | H | H | H | H | H | $CH_2CH_2CH_2CH_2NHCO\text{-}NHCHMe_2$ | 438 |
| 6-225 | H | H | H | H | H | H | $CH_2CH_2CH_2CH_2NHCO\text{-}NHCMe_3$ | 452 |
| 6-226 | H | H | H | H | H | H | a-123 | 464 |
| 6-227 | H | H | H | H | H | H | a-124 | 478 |
| 6-228 | H | H | H | H | H | H | a-125 | 492 |
| 6-229 | H | H | H | H | H | H | a-126 | 506 |
| 6-230 | H | H | H | H | H | H | a-127 | 530 |
| 6-231 | H | H | H | H | H | H | $CH_2CH_2CH_2CH_2NHCO\text{-}NHC_6H_5$ | 472 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-232 | H | H | H | H | H | H | a-128 | 502 |
| 6-233 | H | H | H | H | H | H | a-129 | 488 |
| 6-234 | H | H | H | H | H | H | a-130 | 487 |
| 6-235 | H | H | H | H | H | H | a-131 | 515 |
| 6-236 | H | H | H | H | H | H | a-132 | 522 |
| 6-237 | H | H | H | H | H | H | a-133 | 522 |
| 6-238 | H | H | H | H | H | H | a-134 | 497 |
| 6-239 | H | H | H | H | H | H | a-135 | 501 |
| 6-240 | H | H | H | H | H | H | a-136 | 506 |
| 6-241 | H | H | H | H | H | H | $CH_2CH_2CH_2CH_2NHCO\text{-}NHCOC_6H_5$ | 500 |
| 6-242 | H | H | H | H | H | H | $CH_2CH_2CH_2CH_2NHCO\text{-}NHSO_2C_6H_5$ | 548 |
| 6-243 | H | H | H | H | H | H | a-137 | 548 |
| 6-244 | H | H | H | H | H | H | a-138 | 529 |
| 6-245 | H | H | H | H | H | H | a-139 | 516 |
| 6-246 | H | H | H | H | H | H | a-140 | 528 |
| 6-247 | H | H | H | H | H | H | a-141 | 478 |
| 6-248 | H | H | H | H | H | H | a-142 | 491 |
| 6-249 | H | H | H | H | H | H | $CH_2CH_2CH_2CH_2NHCO\text{-}NHCMe_2CH_2CMe_3$ | 486 |
| 6-250 | H | H | H | H | H | H | a-143 | 500 |
| 6-251 | H | H | H | H | H | H | $CH_2CH_2CH_2CH_2\text{-}N(Me)CONHC_6H_5$ | 440 |
| 6-252 | H | H | H | H | H | H | $CH_2CH_2CH_2CH_2\text{-}N (Me) CON (Me) C_6H_5$ | 454 |
| 6-253 | H | H | H | H | H | H | $CH_2CH_2CH_2CH_2NHCO\text{-}NHCH_2CH_2OH$ | 467 |
| 6-254 | H | H | H | H | H | H | $CH_2CH_2CH_2CH_2NHCO\text{-}NHCH_2CH_2OMe$ | 454 |
| 6-255 | H | H | H | H | H | H | $CH_2CH_2CH_2CH_2NHCO\text{-}NHCH_2CH_2NMe_2$ | 484 |
| 6-256 | H | H | H | H | H | H | $CH_2CH_2CH_2CH_2NHCO\text{-}N (Me) CH_2CH_2OH$ | 465 |
| 6-257 | H | H | H | H | H | H | $CH_2CH_2CH_2CH_2NHCO\text{-}N(CH_2CH_2OH)_2$ | 424 |
| 6-258 | H | H | H | H | H | H | a-144 | 465 |
| 6-259 | H | H | H | H | H | H | $CH_2CH_2CH_2CH_2NHCONMe_2$ | 424 |
| 6-260 | H | H | H | H | H | H | $CH_2CH_2NHCSNHCH_2Me$ | 412 |
| 6-261 | H | H | H | H | H | H | $CH_2CH_2NHCSNH\text{-}CH_2CH_2Me$ | 426 |
| 6-262 | H | H | H | H | H | H | $CH_2CH_2NHCSNHCHMe_2$ | 426 |
| 6-263 | H | H | H | H | H | H | $CH_2CH_2NHCSNHCMe_3$ | 440 |
| 6-264 | H | H | H | H | H | H | a-112 | 386 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-265 | H | H | H | H | H | H | $CH_2CH_2NHCSNHC_6H_5$ | 460 |
| 6-266 | H | H | H | H | H | H | $CH_2CH_2NHCSNHCH_2CH_2O$ H | 428 |
| 6-267 | H | H | H | H | H | H | $CH_2CH_2NHCSNH$-$CH_2CH_2OMe$ | 442 |
| 6-268 | H | H | H | H | H | H | $CH_2CH_2NHCSNH$-$CH_2CH_2NMe_2$ | 455 |
| 6-269 | H | H | H | H | H | H | $CH_2CH_2NHCS$-N (Me) $CH_2CH_2OH$ | 442 |
| 6-270 | H | H | H | H | H | H | $CH_2CH_2NHCS$-N $(CH_2CH_2OH)_2$ | 472 |
| 6-271 | H | H | H | H | H | H | a-113 | 453 |
| 6-272 | H | H | H | H | H | H | $CH_2CH_2NHCSNMe_2$ | 412 |
| 6-273 | H | H | H | H | H | H | $CH_2CH_2NHCOOMe$ | 383 |
| 6-274 | H | H | H | H | H | H | $CH_2CH_2NHCOOCH_2Me$ | 397 |
| 6-275 | H | H | H | H | H | H | $CH_2CH_2NHCOOCH_2CH_2Me$ | 411 |
| 6-276 | H | H | H | H | H | H | $CH_2CH_2NHCOOCHMe_2$ | 411 |
| 6-277 | H | H | H | H | H | H | a-114 | 451 |
| 6-278 | H | H | H | H | H | H | $CH_2CH_2NHCOOC_6H_5$ | 445 |
| 6-279 | H | H | H | H | H | H | $CH_2CH_2NHCOOCH_2CH_2OH$ | 413 |
| 6-280 | H | H | H | H | H | H | $CH_2CH_2NHCOOCH_2CH_2$-OMe | 427 |
| 6-281 | H | H | H | H | H | H | $CH_2CH_2NHCOOCH_2CH_2$-$NMe_2$ | 440 |
| 6-282 | H | H | H | H | H | H | a-115 | 406 |
| 6-283 | H | H | H | H | H | H | a-116 | 406 |
| 6-284 | H | H | H | H | H | H | a-9 | 379 |
| 6-285 | H | H | H | H | H | H | a-117 | 393 |
| 6-286 | H | H | H | H | H | H | a-118 | 407 |
| 6-287 | H | H | H | H | H | H | a-8 | 324 |
| 6-288 | H | H | H | H | H | H | $CH_2CH_2OCH_2CH_2OMe$ | 384 |
| 6-289 | H | H | H | H | H | H | $CH(CH_2OH)_2$ | 355 |
| 6-290 | H | H | H | H | H | H | a-4 | 380 |
| 6-291 | Cl | H | H | H | H | H | H | 316 |
| 6-292 | Br | H | H | H | H | H | H | 360 |
| 6-293 | OH | H | H | H | H | H | H | 298 |
| 6-294 | Me | H | H | H | H | H | H | 296 |
| 6-295 | $CH=CH_2$ | H | H | H | H | H | H | 308 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-296 | $C\equiv CC_6H_5$ | H | H | H | H | H | H | 382 |
| 6-297 | OMe | H | H | H | H | H | H | 312 |
| 6-298 | $NH_2$ | H | H | H | H | H | H | 297 |
| 6-299 | NHMe | H | H | H | H | H | H | 311 |
| 6-300 | $NMe_2$ | H | H | H | H | H | H | 325 |
| 6-301 | $NHC_6H_5$ | H | H | H | H | H | H | 373 |
| 6-302 | OH | H | H | H | H | H | $CH_2CH_2CH_2CH_2COOH$ | 398 |
| 6-303 | OH | H | H | H | H | H | $CH_2CH_2CH_2CH_2CH_2OH$ | 384 |
| 6-304 | OH | H | H | H | H | H | $CH_2CH_2SCOMe$ | 400 |
| 6-305 | OH | H | H | H | H | H | $CH_2CH_2SH$ | 358 |
| 6-306 | OH | H | H | H | H | H | COMe | 340 |
| 6-307 | OH | H | H | H | H | H | $SO_2Me$ | 376 |
| 6-308 | OH | H | H | H | H | H | $CH_2CH_2CH_2NHCONHC_6H_5$ | 474 |
| 6-309 | OH | H | H | H | H | H | a-91 | 480 |
| 6-311 | OH | H | H | H | H | H | Me | 312 |
| 6-312 | OH | H | H | H | H | H | a-121 | 352 |
| 6-313 | OH | H | H | H | H | H | a-122 | 337 |
| 6-314 | OH | H | H | H | H | H | Bn | 388 |
| 6-315 | OH | H | H | H | H | H | a-2 | 418 |
| 6-316 | OH | H | H | H | H | Me | Me | 326 |
| 6-317 | OH | H | H | H | H | H | $COC_6H_5$ | 402 |
| 6-318 | OH | H | H | H | H | H | $SO_2C_6H_5$ | 438 |
| 6-319 | OH | H | H | H | H | H | $CH_2COOH$ | 356 |
| 6-320 | OH | H | H | H | H | H | $CH_2CH_2COOH$ | 370 |
| 6-321 | OH | H | H | H | H | H | $CH_2CH_2CH_2COOH$ | 384 |
| 6-322 | OH | H | H | H | H | H | $CH_2CN$ | 337 |
| 6-323 | OH | H | H | H | H | H | $CH_2CH_2CN$ | 351 |
| 6-324 | OH | H | H | H | H | H | $CH_2CH_2CH_2CN$ | 365 |
| 6-325 | OH | H | H | H | H | H | $CH_2CH_2CH_2CH_2CN$ | 379 |
| 6-326 | OH | H | H | H | H | H | $CH_2CH_2OH$ | 342 |
| 6-327 | OH | H | H | H | H | H | $CH_2CH$ (Me) OH | 356 |
| 6-328 | OH | H | H | H | H | H | $CH(Me)CH_2OH$ | 356 |
| 6-329 | OH | H | H | H | H | H | $CH_2CH_2CH_2OH$ | 356 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-330 | OH | H | H | H | H | H | $CH_2CH$ (OH) $CH_2OH$ | 372 |
| 6-331 | OH | H | H | H | H | H | $CH_2CH(Me)CH_2OH$ | 370 |
| 6-332 | OH | H | H | H | H | H | $CH_2CH_2CH_2CH_2OH$ | 370 |
| 6-333 | OH | H | H | H | H | H | $CH_2CH_2OCONHMe$ | 399 |
| 6-334 | OH | H | H | H | H | H | a-7 | 453 |
| 6-335 | OH | H | H | H | H | H | $CH_2CH_2OCONHC_6H_5$ | 461 |
| 6-336 | OH | H | H | H | H | H | $CH_2CH_2CH_2OCONHMe$ | 413 |
| 6-337 | OH | H | H | H | H | H | a-48 | 467 |
| 6-338 | OH | H | H | H | H | H | $CH_2CH_2CH_2OCONHC_6H_5$ | 475 |
| 6-339 | OH | H | H | H | H | H | $CH_2CH_2CH_2CH_2OCONHMe$ | 427 |
| 6-340 | OH | H | H | H | H | H | a-49 | 481 |
| 6-341 | OH | H | H | H | H | H | $CH_2CH_2CH_2CH_2OCONH-C_6H_5$ | 489 |
| 6-342 | OH | H | H | H | H | H | $CH_2CH_2CH_2SH$ | 372 |
| 6-343 | OH | H | H | H | H | H | $CH_2CH_2SCOC_6H_5$ | 462 |
| 6-344 | OH | H | H | H | H | H | $CH_2CH_2CH_2SCOMe$ | 414 |
| 6-345 | OH | H | H | H | H | H | $CH_2CH_2CH_2SCOC_6H_5$ | 476 |
| 6-346 | OH | H | H | H | H | H | $CH_2CH_2OMe$ | 356 |
| 6-347 | OH | H | H | H | H | H | $CH_2CH_2CH_2OMe$ | 370 |
| 6-348 | OH | H | H | H | H | H | $CH_2CH_2CH_2CH_2OMe$ | 384 |
| 6-349 | OH | H | H | H | H | H | $CH_2CH_2CH_2CH_2CH_2OMe$ | 398 |
| 6-350 | OH | H | H | H | H | H | $CH_2CH_2OCH_2CH_2OH$ | 386 |
| 6-351 | OH | H | H | H | H | H | $CH_2CH_2CH_2OCH_2CH_2OH$ | 400 |
| 6-352 | OH | H | H | H | H | H | $CH_2CH_2CH_2CH_2OCH_2$CH2 -OH | 414 |
| 6-353 | OH | H | H | H | H | H | $CH(CH_2OH)CH_2NH_2$ | 371 |
| 6-354 | OH | H | H | H | H | H | $CH(CH_2OH)CH_2NHCOMe$ | 413 |
| 6-355 | OH | H | H | H | H | H | a-51 | 496 |
| 6-356 | OH | H | H | H | H | H | $CH(CH_2OH)CH_2NHCO-NHC_6H_5$ | 490 |
| 6-357 | OH | H | H | H | H | H | $CH_2CH_2NH_2$ | 341 |
| 6-358 | OH | H | H | H | H | H | $CH_2CH_2CH_2NH_2$ | 355 |
| 6-359 | OH | H | H | H | H | H | $CH_2CH(OH)CH_2NH_2$ | 371 |
| 6-360 | OH | H | H | H | H | H | $CH_2CH$ (OH) $CH_2NHMe$ | 385 |
| 6-361 | OH | H | H | H | H | H | $CH_2CH(OH)CH_2NMe_2$ | 399 |
| 6-362 | OH | H | H | H | H | H | $CH_2CH_2CH_2CH_2NH_2$ | 369 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-363 | OH | H | H | H | H | H | $CH_2CH_2CH_2CH_2CH_2NH_2$ | 383 |
| 6-364 | OH | H | H | H | H | H | $CH_2CH_2NHMe$ | 355 |
| 6-365 | OH | H | H | H | H | H | $CH_2CH_2CH_2NHMe$ | 369 |
| 6-366 | OH | H | H | H | H | H | $CH_2CH_2CH_2CH_2NHMe$ | 383 |
| 6-367 | OH | H | H | H | H | H | $CH_2CH_2CH_2CH_2CH_2NHMe$ | 397 |
| 6-368 | OH | H | H | H | H | H | $CH_2CH_2NMe_2$ | 369 |
| 6-369 | OH | H | H | H | H | H | $CH_2CH_2CH_2NMe_2$ | 383 |
| 6-370 | OH | H | H | H | H | H | $CH_2CH_2CH_2CH_2NMe_2$ | 397 |
| 6-371 | OH | H | H | H | H | H | $CH_2CH_2CH_2CH_2CH_2NMe_2$ | 411 |
| 6-372 | OH | H | H | H | H | H | $CH_2CH_2NHCH_2CH_2OH$ | 385 |
| 6-373 | OH | H | H | H | H | H | $CH_2CH_2CH_2NHCH_2CH_2OH$ | 399 |
| 6-374 | OH | H | H | H | H | H | $CH_2CH_2CH_2CH_2NHCH_2$-$CH_2OH$ | 413 |
| 6-375 | OH | H | H | H | H | H | $CH_2CONH_2$ | 355 |
| 6-376 | OH | H | H | H | H | H | $CH_2CH_2CONH_2$ | 369 |
| 6-377 | OH | H | H | H | H | H | $CH_2CH_2CH_2CONH_2$ | 383 |
| 6-378 | OH | H | H | H | H | H | $CH_2CH_2NHCOMe$ | 383 |
| 6-379 | OH | H | H | H | H | H | $CH\ (CH_2NHCOMe)_2$ | 454 |
| 6-380 | OH | H | H | H | H | H | $CH_2CH_2NHCOCH_2Me$ | 397 |
| 6-381 | OH | H | H | H | H | H | $CH_2CH_2NHCOCH_2CH_2Me$ | 411 |
| 6-382 | OH | H | H | H | H | H | $CH_2CH_2NHCOCHMe_2$ | 411 |
| 6-383 | OH | H | H | H | H | H | $CH_2CH_2NHCOCMe_3$ | 425 |
| 6-384 | OH | H | H | H | H | H | $CH_2CH_2NHCOCH_2OH$ | 399 |
| 6-385 | OH | H | H | H | H | H | $CH_2CH_2NHCOCH_2OMe$ | 413 |
| 6-386 | OH | H | H | H | H | H | $CH_2CH_2NHCOCH_2NMe_2$ | 426 |
| 6-387 | O | H | H | H | H | H | $CH_2CH_2NHCOC_6H_5$ | 445 |
| 6-388 | OH | H | H | H | H | H | a-52 | 446 |
| 6-389 | OH | H | H | H | H | H | a-53 | 451 |
| 6-390 | OH | H | H | H | H | H | a-54 | 435 |
| 6-391 | OH | H | H | H | H | H | a-55 | 447 |
| 6-392 | OH | H | H | H | H | H | $CH_2CH_2NHCOCH_2NHCOMe$ | 440 |
| 6-393 | OH | H | H | H | H | H | $CH_2CH_2NHCOCH_2NHCO$-$CH_2CHMe_2$ | 482 |
| 6-394 | OH | H | H | H | H | H | $CH_2CH_2NHCOCH_2NHCO$-$C_6H_5$ | 502 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-395 | OH | H | H | H | H | H | $CH_2CH_2CH_2NHCOMe$ | 397 |
| 6-396 | OH | H | H | H | H | H | $CH_2CH_2CH_2NHCOCH_2Me$ | 411 |
| 6-397 | OH | H | H | H | H | H | $CH_2CH_2CH_2NHCO\text{-}CH_2CH_2Me$ | 425 |
| 6-398 | OH | H | H | H | H | H | $CH_2CH_2CH_2NHCOCHMe_2$ | 425 |
| 6-399 | OH | H | H | H | H | H | $CH_2CH_2CH_2NHCOCMe_3$ | 439 |
| 6-400 | OH | H | H | H | H | H | $CH_2CH_2CH_2CH_2NHCOMe$ | 411 |
| 6-401 | OH | H | H | H | H | H | $CH_2CH_2CH_2CH_2NHCO\text{-}CH_2Me$ | 425 |
| 6-402 | OH | H | H | H | H | H | $CH_2CH_2CH_2CH_2NHCO\text{-}CH_2CH_2Me$ | 439 |
| 6-403 | OH | H | H | H | H | H | $CH_2CH_2CH_2CH_2NHCO\text{-}CHMe_2$ | 439 |
| 6-404 | OH | H | H | H | H | H | $CH_2CH_2CH_2CH_2NHCOCMe$ | 453 |
| 6-405 | OH | H | H | H | H | H | $CH_2CH_2N$ (Me) C0Me | 397 |
| 6-406 | OH | H | H | H | H | H | $CH_2CH_2N$ (Me) $COC_6H_5$ | 459 |
| 6-407 | OH | H | H | H | H | H | $CH_2CH_2CH_2N$ (Me) COMe | 411 |
| 6-408 | OH | H | H | H | H | H | $CH_2CH_2CH_2N(Me)COC_6H_5$ | 473 |
| 6-409 | OH | H | H | H | H | H | $CH_2CH_2CH_2CH_2N$ (Me) -COMe | 425 |
| 6-410 | OH | H | H | H | H | H | $CH_2CH_2CH_2CH_2N$ (Me) $-COC_6H_5$ | 487 |
| 6-411 | OH | H | H | H | H | H | $CH_2CH_2N$ ($CH_2CH_2OH$) -COMe | 427 |
| 6-412 | OH | H | H | H | H | H | $CH_2CH_2N(CH_2CH_2OH)\text{-}COC_6H_5$ | 490 |
| 6-413 | OH | H | H | H | H | H | $CH_2CH_2CH_2$- | 441 |
| 6-414 | OH | H | H | H | H | H | N ($CH_2CH_2OH$) COMe $CH_2CH_2CH_2$-N ($CH_2CH_2OH$) $COC_6H_5$ | 504 |
| 6-415 | OH | H | H | H | H | H | $CH_2CH_2CH_2CH_2$ -N ($CH_2CH_2OH$) COMe | 455 |
| 6-416 | OH | H | H | H | H | H | $CH_2CH_2CH_2CH_2$ -N ($CH_2CH_2OH$) $COC_6H_5$ | 518 |
| 6-417 | OH | H | H | H | H | H | $CH_2CH_2N(CH_2CN)COMe$ | 422 |
| 6-418 | OH | H | H | H | H | H | $CH_2CH_2N(CH_2CN)COC_6H_5$ | 485 |
| 6-419 | OH | H | H | H | H | H | $CH_2CH_2CH_2$-N ($CH_2CN$)COMe | 436 |
| 6-420 | OH | H | H | H | H | H | $CH_2CH_2CH_2N(CH_2CN)CO$ $C_6H_5$ | 499 |
| 6-421 | OH | H | H | H | H | H | $CH_2CH_2CH_2CH_2$-N ($CH_2CN$) COMe | 450 |
| 6-422 | OH | H | H | H | H | H | $CH_2CH_2CH_2CH_2N(CH_2CN)$ $COC_6H_5$ | 513 |
| 6-423 | OH | H | H | H | H | H | a-3 | 396 |
| 6-424 | OH | H | H | H | H | H | a-5 | 395 |
| 6-425 | OH | H | H | H | H | H | a-6 | 437 |
| 6-426 | OH | H | H | H | H | H | $CH_2CH_2NHSO_2Me$ | 419 |
| 6-427 | OH | H | H | H | H | H | $CH_2CH_2NHSO_2NMe_2$ | 448 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-428 | OH | H | H | H | H | H | $CH_2CH_2N$ (Me) $SO_2Me$ | 433 |
| 6-429 | OH | H | H | H | H | H | $CH_2CH_2N$ (Me) $SO_2C_6H_5$ | 495 |
| 6-430 | OH | H | H | H | H | H | $CH_2CH_2N(Me)SO_2NMe_2$ | 462 |
| 6-431 | OH | H | H | H | H | H | $CH_2CH_2N(CH_2CH_2OH)$-$SO_2Me$ | 449 |
| 6-432 | OH | H | H | H | H | H | $CH_2CH_2CH_2NHSO_2Me$ | 433 |
| 6-433 | OH | H | H | H | H | H | $CH_2CH_2CH_2NHSO_2C_6H_5$ | 495 |
| 6-434 | OH | H | H | H | H | H | $CH_2CH_2CH_2NHSO_2NMe_2$ | 462 |
| 6-435 | OH | H | H | H | H | H | $CH_2CH_2CH_2N$ (Me) $SO_2Me$ | 447 |
| 6-436 | OH | H | H | H | H | H | $CH_2CH_2CH_2N$ (Me)-$SO_2C_6H_5$ | 509 |
| 6-437 | OH | H | H | H | H | H | $CH_2CH_2CH_2N$ (Me)-$SO_2NMe_2$ | 476 |
| 6-438 | OH | H | H | H | H | H | $CH_2CH_2CH_2$-N ($CH_2CH_2OH$) $SO_2Me$ | 463 |
| 6-439 | OH | H | H | H | H | H | $CH_2CH_2CH_2CH_2NHSO_2Me$ | 447 |
| 6-440 | OH | H | H | H | H | H | $CH_2CH_2CH_2CH_2NH$-$SO_2C_6H_5$ | 509 |
| 6-441 | OH | H | H | H | H | H | $CH_2CH_2CH_2CH_2NH$-$SO_2NMe_2$ | 476 |
| 6-442 | OH | H | H | H | H | H | $CH_2CH_2CH_2CH_2N$ (Me)-$SO_2Me$ | 461 |
| 6-443 | OH | H | H | H | H | H | $CH_2CH_2CH_2CH_2N$ (Me)-$SO_2C_6H_5$ | 523 |
| 6-444 | OH | H | H | H | H | H | $CH_2CH_2CH_2CH_2N$ (Me) -$SO_2NMe_2$ | 490 |
| 6-445 | OH | H | H | H | H | H | $CH_2CH_2CH_2CH_2$-N ($CH_2CH_2OH$) $SO_2Me$ | 477 |
| 6-446 | OH | H | H | H | H | H | a-66 | 473 |
| 6-447 | OH | H | H | H | H | H | $CH_2CH_2NHCONHMe$ | 398 |
| 6-448 | OH | H | H | H | H | H | $CH_2CH_2NHCONHCH_2Me$ | 412 |
| 6-449 | OH | H | H | H | H | H | $CH_2CH_2NHCONH$-$CH_2CH_2Me$ | 426 |
| 6-450 | OH | H | H | H | H | H | $CH_2CH_2NHCONHCHMe_2$ | 426 |
| 6-451 | OH | H | H | H | H | H | $CH_2CH_2NHCONHCMe_3$ | 440 |
| 6-452 | OH | H | H | H | H | H | a-68 | 452 |
| 6-453 | OH | H | H | H | H | H | a-69 | 466 |
| 6-454 | OH | H | H | H | H | H | a-70 | 480 |
| 6-455 | OH | H | H | H | H | H | a-71 | 494 |
| 6-456 | OH | H | H | H | H | H | a-72 | 518 |
| 6-457 | OH | H | H | H | H | H | $CH_2CH_2NHCONHC_6H_5$ | 460 |
| 6-458 | OH | H | H | H | H | H | a-73 | 490 |
| 6-459 | OH | H | H | H | H | H | a-74 | 476 |
| 6-460 | OH | H | H | H | H | H | a-75 | 475 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-461 | OH | H | H | H | H | H | a-76 | 503 |
| 6-462 | OH | H | H | H | H | H | a-77 | 510 |
| 6-463 | OH | H | H | H | H | H | a-78 | 510 |
| 6-464 | OH | H | H | H | H | H | a-79 | 485 |
| 6-465 | OH | H | H | H | H | H | a-80 | 489 |
| 6-466 | OH | H | H | H | H | H | a-81 | 494 |
| 6-467 | OH | H | H | H | H | H | $CH_2CH_2NHCONHCOC_6H_5$ | 488 |
| 6-468 | OH | H | H | H | H | H | a-82 | 402 |
| 6-469 | OH | H | H | H | H | H | a-83 | 402 |
| 6-470 | OH | H | H | H | H | H | a-84 | 517 |
| 6-471 | OH | H | H | H | H | H | a-85 | 504 |
| 6-472 | OH | H | H | H | H | H | a-86 | 516 |
| 6-473 | OH | H | H | H | H | H | a-87 | 466 |
| 6-474 | OH | H | H | H | H | H | a-88 | 479 |
| 6-475 | OH | H | H | H | H | H | $CH_2CH_2N$ (Me) $CONHC_6H_5$ | 474 |
| 6-476 | OH | H | H | H | H | H | $CH_2CH_2N$ (Me)-CON (Me) $C_6H_5$ | 488 |
| 6-477 | OH | H | H | H | H | H | $CH_2CH_2NHCONH-CH_2CH_2OH$ | 428 |
| 6-478 | OH | H | H | H | H | H | $CH_2CH_2NHCONH-CH_2CH_2OMe$ | 442 |
| 6-479 | OH | H | H | H | H | H | $CH_2CH_2NHCONH-CH_2CH_2NMe_2$ | 455 |
| 6-480 | OH | H | H | H | H | H | $CH_2CH_2NHCON$ (Me) $CH_2C\ H_2OH$ | 442 |
| 6-481 | OH | H | H | H | H | H | $CH_2CH_2NHCO-\ N(CH_2CH_2OH)_2$ | 472 |
| 6-482 | OH | H | H | H | H | H | a-89 | 453 |
| 6-483 | OH | H | H | H | H | H | $CH_2CH_2NHCONMe_2$ | 412 |
| 6-484 | OH | H | H | H | H | H | $CH_2CH_2CH_2NHCONHMe$ | 412 |
| 6-485 | OH | H | H | H | H | H | $CH_2CH_2CH_2NHCO-NHCH_2Me$ | 426 |
| 6-486 | OH | H | H | H | H | H | $CH_2CH_2CH_2NHCO-NHCH_2CH_2Me$ | 440 |
| 6-487 | OH | H | H | H | H | H | $CH_2CH_2CH_2NHCO-NHCHMe_2$ | 440 |
| 6-488 | OH | H | H | H | H | H | $CH_2CH_2CH_2NHCONHCMe_3$ | 454 |
| 6-489 | OH | H | H | H | H | H | a-90 | 466 |
| 6-490 | OH | H | H | H | H | H | a-92 | 480 |
| 6-491 | OH | H | H | H | H | H | a-93 | 494 |
| 6-492 | OH | H | H | H | H | H | a-94 | 508 |
| 6-493 | OH | H | H | H | H | H | a-95 | 532 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-494 | OH | H | H | H | H | H | a-96 | 474 |
| 6-495 | OH | H | H | H | H | H | a-97 | 504 |
| 6-496 | OH | H | H | H | H | H | a-98 | 490 |
| 6-497 | OH | H | H | H | H | H | a-99 | 489 |
| 6-498 | OH | H | H | H | H | H | a-100 | 517 |
| 6-499 | OH | H | H | H | H | H | a-101 | 524 |
| 6-500 | OH | H | H | H | H | H | a-102 | 524 |
| 6-501 | OH | H | H | H | H | H | a-103 | 499 |
| 6-502 | 0H | H | H | H | H | H | $CH_2CH_2CH_2NHCO\text{-}NHCOC_6H_5$ | 503 |
| 6-503 | OH | H | H | H | H | H | $CH_2CH_2CH_2NHCO\text{-}NHSO_2C_6H_5$ | 508 |
| 6-504 | OH | H | H | H | H | H | a-104 | 502 |
| 6-505 | OH | H | H | H | H | H | a-105 | 550 |
| 6-506 | OH | H | H | H | H | H | a-106 | 550 |
| 6-507 | OH | H | H | H | H | H | a-107 | 531 |
| 6-508 | OH | H | H | H | H | H | a-108 | 518 |
| 6-509 | OH | H | H | H | H | H | a-109 | 530 |
| 6-510 | OH | H | H | H | H | H | $CH_2CH_2CH_2NHCO\text{-}NHCMe_2CH_2CMe_3$ | 480 |
| 6-511 | OH | H | H | H | H | H | a-110 | 493 |
| 6-512 | OH | H | H | H | H | H | $CH_2CH_2CH_2N$ (Me) - $CONHC_6H_5$ | 488 |
| 6-513 | OH | H | H | H | H | H | $CH_2CH_2CH_2N$ (Me) - | 502 |
| 6-514 | OH | H | H | H | H | H | CON (Me) $C_6H_5$ $CH_2CH_2CH_2NHCONH\text{-}CH_2CH_2OH$ | 442 |
| 6-515 | OH | H | H | H | H | H | $CH_2CH_2CH_2NHCONH\text{-}CH_2CH_2OMe$ | 456 |
| 6-516 | OH | H | H | H | H | H | $CH_2CH_2CH_2NHCONH\text{-}CH_2CH_2NMe_2$ | 469 |
| 6-517 | OH | H | H | H | H | H | $CH_2CH_2CH_2NHCO\text{-}N$ (Me) $CH_2CH_2OH$ | 456 |
| 6-518 | OH | H | H | H | H | H | $CH_2CH_2CH_2NHCO\text{-}N$ $(CH_2CH_2OH)_2$ | 486 |
| 6-519 | OH | H | H | H | H | H | a-111 | 467 |
| 6-520 | OH | H | H | H | H | H | $CH_2CH_2CH_2NHCONMe_2$ | 426 |
| 6-521 | OH | H | H | H | H | H | $CH_2CH_2CH_2CH_2NHCONHMe$ | 426 |
| 6-522 | OH | H | H | H | H | H | $CH_2CH_2CH_2CH_2NHCO\text{-}NHCH_2Me$ | 440 |
| 6-523 | OH | H | H | H | H | H | $CH_2CH_2CH_2CH_2NHCO\text{-}NHCH_2CH_2Me$ | 454 |
| 6-524 | OH | H | H | H | H | H | $CH_2CH_2CH_2CH_2NHCO\text{-}NHCHMe_2$ | 454 |
| 6-525 | OH | H | H | H | H | H | $CH_2CH_2CH_2CH_2NHCO\text{-}NHCMe_3$ | 468 |
| 6-526 | OH | H | H | H | H | H | a-123 | 480 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-527 | OH | H | H | H | H | H | a-124 | 494 |
| 6-528 | OH | H | H | H | H | H | a-125 | 508 |
| 6-529 | OH | H | H | H | H | H | a-126 | 522 |
| 6-530 | OH | H | H | H | H | H | a-127 | 546 |
| 6-531 | OH | H | H | H | H | H | $CH_2CH_2CH_2CH_2NHCO-NHC_6H_5$ | 488 |
| 6-532 | OH | H | H | H | H | H | a-128 | 518 |
| 6-533 | OH | H | H | H | H | H | a-129 | 504 |
| 6-534 | OH | H | H | H | H | H | a-130 | 503 |
| 6-535 | OH | H | H | H | H | H | a-131 | 531 |
| 6-536 | OH | H | H | H | H | H | a-132 | 538 |
| 6-537 | OH | H | H | H | H | H | a-133 | 538 |
| 6-538 | OH | H | H | H | H | H | a-134 | 513 |
| 6-539 | OH | H | H | H | H | H | a-135 | 517 |
| 6-540 | OH | H | H | H | H | H | a-136 | 522 |
| 6-541 | OH | H | H | H | H | H | $CH_2CH_2CH_2CH_2NHCO-NHCOC_6H_5$ | 516 |
| 6-542 | OH | H | H | H | H | H | $CH_2CH_2CH_2CH_2NHCO-NHSO_2C_6H_5$ | 564 |
| 6-543 | OH | H | H | H | H | H | a-137 | 564 |
| 6-544 | OH | H | H | H | H | H | a-138 | 545 |
| 6-545 | OH | H | H | H | H | H | a-139 | 532 |
| 6-546 | OH | H | H | H | H | H | a-140 | 544 |
| 6-547 | OH | H | H | H | H | H | a-141 | 494 |
| 6-548 | OH | H | H | H | H | H | a-142 | 507 |
| 6-549 | OH | H | H | H | H | H | $CH_2CH_2CH_2CH_2NHCONH-CMe_2CH_2CMe_3$ | 502 |
| 6-550 | OH | H | H | H | H | H | a-143 | 516 |
| 6-551 | OH | H | H | H | H | H | $CH_2CH_2CH_2CH_2N$ (Me)-$CONHC_6H_5$ | 456 |
| 6-552 | OH | H | H | H | H | H | $CH_2CH_2CH_2CH_2N$ (Me) -CON (Me) $C_6H_5$ | 470 |
| 6-553 | OH | H | H | H | H | H | $CH_2CH_2CH_2CH_2NHCONH-CH_2CH_2OH$ | 483 |
| 6-554 | OH | H | H | H | H | H | $CH_2CH_2CH_2CH_2NHCONH-CH_2CH_2OMe$ | 470 |
| 6-555 | OH | H | H | H | H | H | $CH_2CH_2CH_2CH_2NHCONH-CH_2CH_2NMe_2$ | 500 |
| 6-556 | OH | H | H | H | H | H | $CH_2CH_2CH_2CH_2NHCO$-N (Me) $CH_2CH_2OH$ | 481 |
| 6-557 | OH | H | H | H | H | H | $CH_2CH_2CH_2CH_2NHCO$-N $(CH_2CH_2OH)_2$ | 440 |
| 6-558 | OH | H | H | H | H | H | a-144 | 481 |
| 6-559 | OH | H | H | H | H | H | $CH_2CH_2CH_2CH_2NHCONMe_2$ | 440 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-560 | OH | H | H | H | H | H | $CH_2CH_2NHCSNHCH_2Me$ | 428 |
| 6-561 | OH | H | H | H | H | H | $CH_2CH_2NHCSNH-CH_2CH_2Me$ | 442 |
| 6-562 | OH | H | H | H | H | H | $CH_2CH_2NHCSNHCHMe_2$ | 442 |
| 6-563 | OH | H | H | H | H | H | $CH_2CH_2NHCSNHCMe_3$ | 456 |
| 6-564 | OH | H | H | H | H | H | a-112 | 402 |
| 6-565 | OH | H | H | H | H | H | $CH_2CH_2NHCSNHC_6H_5$ | 476 |
| 6-566 | OH | H | H | H | H | H | $CH_2CH_2NHCSNH-CH_2CH_2OH$ | 444 |
| 6-567 | OH | H | H | H | H | H | $CH_2CH_2NHCSNH-CH_2CH_2OMe$ | 458 |
| 6-568 | OH | H | H | H | H | H | $CH_2CH_2NHCSNH-CH_2CH_2NMe_2$ | 471 |
| 6-569 | OH | H | H | H | H | H | $CH_2CH_2NHCSN$ (Me)-$CH_2CH_2OH$ | 458 |
| 6-570 | OH | H | H | H | H | H | $CH_2CH_2NHCS-N(CH_2CH_2OH)_2$ | 488 |
| 6-571 | OH | H | H | H | H | H | a-113 | 469 |
| 6-572 | OH | H | H | H | H | H | $CH_2CH_2NHCSNMe_2$ | 428 |
| 6-573 | OH | H | H | H | H | H | $CH_2CH_2NHCOOMe$ | 399 |
| 6-574 | OH | H | H | H | H | H | $CH_2CH_2NHCOOCH_2Me$ | 413 |
| 6-575 | OH | H | H | H | H | H | $CH_2CH_2NHCOOCH_2CH_2Me$ | 427 |
| 6-576 | OH | H | H | H | H | H | $CH_2CH_2NHCOOCHMe_2$ | 427 |
| 6-577 | OH | H | H | H | H | H | a-114 | 467 |
| 6-578 | OH | H | H | H | H | H | $CH_2CH_2NHCOOC_6H_5$ | 461 |
| 6-579 | OH | H | H | H | H | H | $CH_2CH_2NHCOOCH_2CH_2OH$ | 429 |
| 6-580 | OH | H | H | H | H | H | $CH_2CH_2NHCOO-CH_2CH_2OMe$ | 443 |
| 6-581 | OH | H | H | H | H | H | $CH_2CH_2NHCOO-CH_2CH_2NMe_2$ | 456 |
| 6-582 | OH | H | H | H | H | H | a-115 | 422 |
| 6-583 | OH | H | H | H | H | H | a-116 | 422 |
| 6-584 | OH | H | H | H | H | H | a-9 | 395 |
| 6-585 | OH | H | H | H | H | H | a-117 | 409 |
| 6-586 | OH | H | H | H | H | H | a-118 | 423 |
| 6-587 | OH | H | H | H | H | H | a-8 | 340 |
| 6-588 | OH | H | H | H | H | H | $CH_2CH_2OCH_2CH_2OMe$ | 400 |
| 6-589 | OH | H | H | H | H | H | $CH(CH_2OH)_2$ | 371 |
| 6-590 | OH | H | H | H | H | H | a-4 | 396 |
| 6-591 | H | Cl | H | H | H | H | H | 316 |
| 6-592 | H | Br | H | H | H | H | H | 360 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-593 | H | OH | H | H | H | H | H | 298 |
| 6-594 | H | Me | H | H | H | H | H | 296 |
| 6-595 | H | $CH=CH_2$ | H | H | H | H | H | 308 |
| 6-596 | H | $C\equiv CC_6H_5$ | H | H | H | H | H | 382 |
| 6-597 | H | OMe | H | H | H | H | H | 312 |
| 6-598 | H | $NH_2$ | H | H | H | H | H | 297 |
| 6-599 | H | NHMe | H | H | H | H | H | 311 |
| 6-600 | H | $NMe_2$ | H | H | H | H | H | 325 |
| 6-601 | H | $NHC_6H_5$ | H | H | H | H | H | 373 |
| 6-602 | H | H | Cl | H | H | H | H | 316 |
| 6-603 | H | H | Br | H | H | H | H | 360 |
| 6-604 | H | H | OH | H | H | H | H | 298 |
| 6-605 | H | H | Me | H | H | H | H | 296 |
| 6-606 | H | H | $CH=CH_2$ | H | H | H | H | 308 |
| 6-607 | H | H | $C\equiv CC_6H_5$ | H | H | H | H | 382 |
| 6-608 | H | H | OMe | H | H | H | H | 312 |
| 6-609 | H | H | $NH_2$ | H | H | H | H | 297 |
| 6-610 | H | H | NHMe | H | H | H | H | 311 |
| 6-611 | H | H | $NMe_2$ | H | H | H | H | 325 |
| 6-612 | H | H | $NHC_6H_5$ | H | H | H | H | 373 |
| 6-613 | H | H | H | C1 | H | H | H | 316 |
| 6-614 | H | H | H | Br | H | H | H | 360 |
| 6-615 | H | H | H | OH | H | H | H | 298 |
| 6-616 | H | H | H | Me | H | H | H | 296 |
| 6-617 | H | H | H | $CH=CH_2$ | H | H | H | 308 |
| 6-618 | H | H | H | $C\equiv CC_6H_5$ | H | H | H | 382 |
| 6-619 | H | H | H | OMe | H | H | H | 312 |
| 6-620 | H | H | H | $NH_2$ | H | H | H | 297 |
| 6-621 | H | H | H | NHMe | H | H | H | 311 |
| 6-622 | H | H | H | $NMe_2$ | H | H | H | 325 |
| 6-623 | H | H | H | $NHC_6H_5$ | H | H | H | 373 |
| 6-624 | H | H | H | H | Cl | H | H | 316 |
| 6-625 | H | H | H | H | Br | H | H | 360 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 6-626 | H | H | H | H | OH | H | H | 298 |
| 6-627 | H | H | H | H | Me | H | H | 296 |
| 6-628 | H | H | H | H | $CH=CH_2$ | H | H | 308 |
| 6-629 | H | H | H | H | $C\equiv CC_6H_5$ | H | H | 382 |
| 6-630 | H | H | H | H | OMe | H | H | 312 |
| 6-631 | H | H | H | H | $NH_2$ | H | H | 297 |
| 6-632 | H | H | H | H | NHMe | H | H | 311 |
| 6-633 | H | H | H | H | $NMe_2$ | H | H | 325 |
| 6-634 | H | H | H | H | $NHC_6H_5$ | H | H | 373 |

Example 7-1: 4-(2,3-Dihydro-1H-1,5-diazaphenalen-1-yl)cyclohexylmethylamine

Step A

5-[4-([1,3]Dioxolan-2-yl)cyclohexylamino]-4-vinylisoquinoline (Intermediate 76)

By using 5-amino-4-vinylisoquinoline (Intermediate 1) obtained in Example 1-1, Step A, and 4-([1,3]dioxolan-2-yl)cyclohexanone obtained by referring to a known publication (T. Yamada, T. Mukaiyama, Chem. Lett., 515 (1989)), reductive alkylation was performed according to the method described in Example 1-1, Step B to obtain the title compound. MS (m/z): 325 (MH+)

Step B

1-(2, 3-Dihydro-1H-1,5-diazaphenalen-1-yl)-4-([1,3]dioxolan-2-yl)cyclohexane (Intermediate 77)

By using 5-[4-([1,3]dioxolan-2-yl)cyclohexylamino]-4-vinylisoquinoline (Intermediate 76) obtained in Step A mentioned above, cyclization was performed according to the method described in Example 1-1, Step C to obtain the title compound. MS (m/z): 325 (MH+)

Step C

4-(2,3-Dihydro-1H-1,5-diazaphenalen-1-yl)cyclohexanecarbaldehyde (Intermediate 78)

By using 1-(2,3-dihydro-1H-1,5-diazaphenalen-1-yl)-4-([1,3]dioxolan-2-yl)cyclohexane (Intermediate 77) obtained in Step B mentioned above, deprotection was performed for the protective group according to the method described in Example 5-1, Step C to obtain the title compound.
MS (m/z): 281 (MH+)

Step D

N-[4-(2,3-Dihydro-1H-1,5-diazaphenalen-1-yl)cyclohexylmethyl]-N-benzylamine (Intermediate 79)

By using 4-(2,3-dihydro-1H-1,5-diazaphenalen-1-yl)cyclohexanecarbaldehyde (Intermediate 78) obtained in Step C mentioned above, and benzylamine, reductive amination was performed according to the method described in Example 5-1, Step D to obtain the title compound.
MS (m/z): 372 (MH+)

Step E

4-(2,3-Dihydro-1H-1,5-diazaphenalen-1-yl)cyclohexylmethylamine

By using N-[4-(2,3-dihydro-1H-1,5-diazaphenalen-1-yl)cyclohexylmethyl]-N-benzylamine (Intermediate 79) obtained in Step D mentioned above, debenzylation was performed according to the method described in Example 5-1, Step E to obtain the title compound.
MS (m/z): 282 (MH+)

The compounds of Examples 7-2 to 7-290 were obtained by using 4-(2,3-dihydro-1H-1,5-diazaphenalen-1-yl) cyclohexanecarbaldehyde (Intermediate 78) obtained in Example 7-1, Step C, or 4-(2,3-dihydro-1H-1,5-diazaphenalen-1-yl)cyclohexylmethylamine obtained in Example 7-1 according to the methods described in Examples 5-2 to 5-320.

Example 7-291:

4-(6-Chloro-2,3-dihydro-1H-1,5-diazaphenalen-1-yl)cyclohexylmethylamine

Step A

1-Chloro-5-[4-([1,3]dioxolan-2-yl)cyclohexylamino]-4-vinylisoquinoline (Intermediate 80)

By using 5-amino-1-chloro-4-vinylisoquinoline (Intermediate 8) obtained in Example 1-321; Step D, and 4-([1,3]dioxolan-2-yl)cyclohexanone, reductive alkylation was performed according to the method described in Example 5-291, Step A to obtain the title compound.
MS (m/z): 359 (MH+)

Step B

1-(6-Chloro-2,3-dihydro-1H-1,5-diazaphenalen-1-yl)-4-([1,3]dioxolan-2-yl)cyclohexane (Intermediate 81)

By using 1-chloro-5-[4-([1,3]dioxolan-2-yl)cyclohexylamino]-4-vinylisoquinoline (Intermediate 80) obtained in Step A mentioned above, cyclization was performed according to the method described in Example 5-291, Step B to obtain the title compound.
MS (m/z): 359 (MH+)

Step C

4-(6-Chloro-2,3-dihydro-1H-1,5-diazaphenalen-1-yl)cyclohexanecarbaldehyde (Intermediate 82)

By using 1-(6-chloro-2,3-dihydro-1H-1,5-diazaphenalen-1-yl)-4-([1,3]dioxolan-2-yl)cyclohexane (Intermediate 81) obtained in Step B mentioned above, deprotection was performed for the protective group according to the method described in Example 5-291, Step C to obtain the title compound.
MS (m/z): 315 (MH+)

Step D

N- [4-(6-Chloro-2,3-dihydro-1H-1,5-diazaphenalen-1-yl)cyclohexylmethyl]-N-(4-methox ybenzyl)amine (Intermediate 83)

By using 4-(6-chloro-2,3-dihydro-1H-1,5-diazaphenalen-1-yl)cyclohexanecarbaldehyde (Intermediate 82) obtained in Step C mentioned above, and 4-methoxybenzylamine, reductive amination was performed according to the method described in Example 5-1, Step D to obtain the title compound.
MS (m/z): 436 (MH+)

Step E

4-(6-Chloro-2,3-dihydro-1H-1,5-diazaphenalen-1-yl)cyclohexylmethylamine

By using N-[4-(6-chloro-2,3-dihydro-1H-1,5-diazaphenalen-1-yl)cyclohexylmethyl]-N-(4-methoxybenzyl)amine (Intermediate 83) obtained in Step D mentioned above, debenzylation was performed according to the method described in Example 5-291, Step E to obtain the title compound as a trifluoroacetate.
MS (m/z): 316 (MH+)

Example 7-292

The compound of Example 7-292 was obtained by referring to the method used for the compound of Example 7-291.

Example 7-293

The compound of Example 7-293 was obtained in the same manner as that used for the compound of Example 5-293.

Examples 7-294 to 7-301

The compounds of Examples 7-294 to 7-301 were obtained by using 1-(6-chloro-2,3-dihydro-1H-1,5-diazaphenalen-1-yl)-4-([1,3]dioxolan-2-yl)cyclohexane (Intermediate 81) obtained in Example 7-291, Step B according to the methods described in Examples 5-294 to 5-301.

Examples 7-302 to 7-590

The compounds of Examples 7-302 to 7-590 were obtained by using 4-(6-chloro-2,3-dihydro-1H-1,5-diazaphenalen-1-yl)cyclohexanecarbaldehyde (Intermediate 82) obtained in Example 7-291, Step C, or 4-(6-chloro-2,3-dihydro-1H-1,5-diazaphenalen-1-yl)cyclohexylmethylamine obtained in Example 7-291 in the same manner as that used for the compounds of Examples 5-302 to 5-590.

Example 7-591:

4-(4-Chloro-2,3-dihydro-1H-1,5-diazaphenalen-1-yl)cyclohexylmethylamine

By using 5-amino-3-chloro-4-vinylisoquinoline, reductive alkylation, and cyclization were performed according to the method described in Example 5-291 to obtain
1-(4-chloro-2,3-dihydro-1H-1,5-diazaphenalen-1-yl)-4-([1,3]dioxolan-2-yl)cyclohexane, and then deprotection for the protective group, reductive amination with 4-methoxybenzylamine, and debenzylation were performed to obtain the title compound as a trifluoroacetate.
MS (m/z): 316 (MH+)

Example 7-592

The compound of Example 7-592 was obtained by referring to the method used for the compound of Example 7-591.

Examples 7-593 to 7-601

The compounds of Examples 7-593 to 7-601 were obtained by using 1-(4-chloro-2,3-dihydro-1H-1,5-diazaphenalen-1-yl)-4-([1,3]dioxo-lan-2-yl)cyclohexane obtained in Example 7-591 according to the methods described in Example 5-593 to 5-601.

Example 7-602

The compound of Example 7-602 was obtained by referring to the method used for the compound of Example 7-603 mentioned below.

Example 7-603:

4-(7-Bromo-2,3-dihydro-1H-1,5-diazaphenalen-1-yl)cyclohexylmethylamine

Step A

1-(7-Bromo-2,3-dihydro-1H-1,5-diazaphenalen-1-yl)-4-([1,3]dioxolan-2-yl)cyclohexane (Intermediate 84)

By using 1-(2,3-dihydro-1H-1,5-diazaphenalen-1-yl)-4-([1,3]dioxolan-2-yl)cyclohexane (Intermediate 77) obtained in Example 7-1, Step B, bromation was performed according to the method described in Example 1-663, Step A to obtain the title compound.
MS (m/z): 403 (MH+)

Step B

4-(7-Bromo-2,3-dihydro-1H-1,5-diazaphenalen-1-yl)cyclohexylmethylamine

By using 1-(7-bromo-2,3-dihydro-1H-1,5-diazaphenalen-1-yl)-4-([1,3]dioxolan-2-yl)cyclohexane (Intermediate 84) obtained in Step A mentioned above, deprotection for the protective group, reductive amination with 4-methoxybenzylamine, and debenzylation were performed according to the method described in Example 5-291 to obtain the title compound.
MS (m/z): 360 (MH+)

Examples 7-604 to 7-612

The compounds of Examples 7-604 to 7-612 were obtained by using 1-(7-bromo-2,3-dihydro-1H-1,5-diazaphenalen-1-yl)-4-([1,3]dioxolan-2-yl)cyclohexane (Intermediate 84) obtained in Example 7-603, Step A in the same manner as that used for the compounds of Examples 5-293 to 5-301.

Example 7-613

The compound of Example 7-613 was obtained by referring to the method used for the compound of Example 7-614 mentioned below.

Example 7-614:

4-(8-Bromo-2,3-dihydro-1H-1,5-diazaphenalen-1-yl)cyclohexylmethylamine

Step A

1-(8-Bromo-2,3-dihydro-1H-1,5-diazaphenalen- 1-yl)-4-([1,3]dioxolan-2-yl)cyclohexane (Intermediate 85)

By using 1-(2,3-dihydro-1H-1,5-diazaphenalen-1-yl)-4-([1,3]dioxolan-2-yl)cyclohexane (Intermediate 77) obtained in Example 7-1, Step B, bromation was performed according to the method described in Example 1-663, Step A to obtain the title compound.
MS (m/z): 403 (MH+)

Step B

1-Amino-4-[(7-bromo-2,3-dihydro-1H-1,5-diazaphenalen-1-yl)methyl]cyclohexane

By using 1-(8-bromo-2,3-dihydro-1H-1,5-diazaphenalen-1-yl)-4-([1,3]dioxolan-2-yl)cyclohexane (Intermediate 85) obtained in Step A mentioned above, deprotection for the protective group, reductive amination with 4-methoxybenzylamine, and debenzylation were performed according to the method described in Example 5-291 to obtain the title compound.
MS (m/z): 360 (MH+)

Examples 7-615 to 7-623

The compounds of Examples 7-615 to 7-623 were obtained by using 1-(8-bromo-2,3-dihydro-1H-1,5-diazaphenalen-1-yl)-4-([1,3]dioxolan-2-yl)cyclohexane (Intermediate 85) obtained in Example 7-614, Step A in the same manner as that used for the compounds of Examples 5-615 to 5-623.

Example 7-624

The compound of Example 7-624 was obtained by referring to the method used for the compound of Example 7-625 mentioned below.

Example 7-625

The compound of Example 7-625 was obtained by using 1-(9-amino-2,3-dihydro-1H-1,5-diazaphenalen-1-yl)-4-([1,3]dioxolan-2-yl)cyclohexane obtained in Example 7-631 mentioned below to perform known diazotization-bromation with referring to the method used for the compound of Example 1-685 and thereby obtain 1-(9-bromo-2,3-dihydro-1H-1,5-diazaphenalen-1-yl)-4-([1,3]dioxolan-2-yl)cyclohexane, and then using the same step as that used for the compound of Example 5-603.

Examples 7-626 to 7-630

The compounds of Examples 7-626 to 7-630 were obtained by using 1-(9-bromo-2,3-dihydro-1H-1,5-diazaphenalen-1-yl)-4-([1,3]dioxolan-2-yl)cyclohexane obtained in Example 7-625 in the same manner as that used for the compounds of Examples 5-293 to 5-297.

Example 7-631

The compound of Example 7-631 was obtained by using 1-(7-bromo-2,3-dihydro-1H-1,5-diazaphenalen-1-yl)-4-([1,3]dioxolan-2-yl)cyclohexane (Intermediate 84) obtained in Example 7-603, Step A to perform nitration (9-position), and reduction (bromine atom→hydrogen atom at the 7-position, nitro group→amino group at the 9-position) in the same manner as that of Example 1-691 to obtain 1-(9-amino-2,3-dihydro-1H-1,5-diazaphenalen-1-yl)-4-([1,3] dioxolan-2-yl) cyclohexane, and then performing deprotection for the protective group, reductive amination with benzylamine, and debenzylation according to the method described in Example 5-1.

Examples 7-632 to 7-634

The compounds of Examples 7-632 to 7-634 were obtained by using 1-(9-bromo-2,3-dihydro-1H-1,5-diazaphenalen-1-yl)-4-([1,3]dioxolan-2-yl)cyclohexane obtained in Example 7-625 in the same manner as that used for the compounds of Examples 5-299 to 5-301.

[Table 7]

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-1 | H | H | H | H | H | H | H | 282 |
| 7-2 | H | H | H | H | H | H | $CH_2CH_2CH_2CH_2COOH$ | 382 |
| 7-3 | H | H | H | H | H | H | $CH_2CH_2CH_2CH_2CH_2OH$ | 368 |
| 7-4 | H | H | H | H | H | H | $CH_2CH_2SCOMe$ | 384 |
| 7-5 | H | H | H | H | H | H | $CH_2CH_2SH$ | 342 |
| 7-6 | H | H | H | H | H | H | COMe | 324 |
| 7-7 | H | H | H | H | H | H | $SO_2Me$ | 360 |
| 7-8 | H | H | H | H | H | H | $CH_2CH_2CH_2NHCONHC_6H_5$ | 458 |
| 7-9 | H | H | H | H | H | H | a-91 | 464 |
| 7-11 | H | H | H | H | H | H | Me | 296 |
| 7-12 | H | H | H | H | H | H | a-121 | 336 |
| 7-13 | H | H | H | H | H | H | a-122 | 321 |
| 7-14 | H | H | H | H | H | H | Bn | 372 |
| 7-15 | H | H | H | H | H | H | a-2 | 402 |
| 7-16 | H | H | H | H | H | Me | Me | 310 |
| 7-17 | H | H | H | H | H | H | $COC_6H_5$ | 386 |
| 7-18 | H | H | H | H | H | H | $SO_2C_6H_5$ | 422 |
| 7-19 | H | H | H | H | H | H | $CH_2COOH$ | 340 |
| 7-20 | H | H | H | H | H | H | $CH_2CH_2COOH$ | 354 |
| 7-21 | H | H | H | H | H | H | $CH_2CH_2CH_2COOH$ | 368 |
| 7-22 | H | H | H | H | H | H | $CH_2CN$ | 321 |
| 7-23 | H | H | H | H | H | H | $CH_2CH_2CN$ | 335 |
| 7-24 | H | H | H | H | H | H | $CH_2CH_2CH_2CN$ | 349 |
| 7-25 | H | H | H | H | H | H | $CH_2CH_2CH_2CH_2CN$ | 363 |
| 7-26 | H | H | H | H | H | H | $CH_2CH_2OH$ | 326 |
| 7-27 | H | H | H | H | H | H | $CH_2CH$ (Me) OH | 340 |
| 7-28 | H | H | H | H | H | H | CH (Me) $CH_2OH$ | 340 |
| 7-29 | H | H | H | H | H | H | $CH_2CH_2CH_2OH$ | 340 |
| 7-30 | H | H | H | H | H | H | $CH_2CH$ (OH) $CH_2OH$ | 356 |
| 7-31 | H | H | H | H | H | H | $CH_2CH$ (Me) $CH_2OH$ | 354 |
| 7-32 | H | H | H | H | H | H | $CH_2CH_2CH_2CH_2OH$ | 354 |
| 7-33 | H | H | H | H | H | H | $CH_2CH_2OCONHMe$ | 383 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-34 | H | H | H | H | H | H | a-7 | 437 |
| 7-35 | H | H | H | H | H | H | $CH_2CH_2OCONHC_6H_5$ | 445 |
| 7-36 | H | H | H | H | H | H | $CH_2CH_2CH_2OCONHMe$ | 397 |
| 7-37 | H | H | H | H | H | H | a-48 | 451 |
| 7-38 | H | H | H | H | H | H | $CH_2CH_2CH_2OCONHC_6H_5$ | 459 |
| 7-39 | H | H | H | H | H | H | $CH_2CH_2CH_2CH_2OCONHMe$ | 411 |
| 7-40 | H | H | H | H | H | H | a-49 | 465 |
| 7-41 | H | H | H | H | H | H | $CH_2CH_2CH_2CH_2OCO\text{-}NHC_6H_5$ | 473 |
| 7-42 | H | H | H | H | H | H | $CH_2CH_2CH_2SH$ | 356 |
| 7-43 | H | H | H | H | H | H | $CH_2CH_2SCOC_6H_5$ | 446 |
| 7-44 | H | H | H | H | H | H | $CH_2CH_2CH_2SCOMe$ | 398 |
| 7-45 | H | H | H | H | H | H | $CH_2CH_2CH_2SCOC_6H_5$ | 460 |
| 7-46 | H | H | H | H | H | H | $CH_2CH_2OMe$ | 340 |
| 7-47 | H | H | H | H | H | H | $CH_2CH_2CH_2OMe$ | 354 |
| 7-48 | H | H | H | H | H | H | $CH_2CH_2CH_2CH_2OMe$ | 368 |
| 7-49 | H | H | H | H | H | H | $CH_2CH_2CH_2CH_2CH_2OMe$ | 382 |
| 7-50 | H | H | H | H | H | H | $CH_2CH_2OCH_2CH_2OH$ | 370 |
| 7-51 | H | H | H | H | H | H | $CH_2CH_2CH_2OCH_2CH_2OH$ | 384 |
| 7-52 | H | H | H | H | H | H | $CH_2CH_2CH_2CH_2O\text{-}CH_2CH_2OH$ | 398 |
| 7-53 | H | H | H | H | H | H | $CH(CH_2OH)CH_2NH_2$ | 355 |
| 7-54 | H | H | H | H | H | H | $CH\ (CH_2OH)\ CH_2NHCOMe$ | 397 |
| 7-55 | H | H | H | H | H | H | a-51 | 480 |
| 7-56 | H | H | H | H | H | H | $CH\ (CH_2OH)\ CH_2NHCO\text{-}NHC_6H_5$ | 474 |
| 7-57 | H | H | H | H | H | H | $CH_2CH_2NH_2$ | 325 |
| 7-58 | H | H | H | H | H | H | $CH_2CH_2CH_2NH_2$ | 339 |
| 7-59 | H | H | H | H | H | H | $CH_2CH\ (OH)\ CH_2NH_2$ | 355 |
| 7-60 | H | H | H | H | H | H | $CH_2CH\ (OH)\ CH_2NHMe$ | 369 |
| 7-61 | H | H | H | H | H | H | $CH_2CH\ (OH)\ CH_2NMe_2$ | 383 |
| 7-62 | H | H | H | H | H | H | $CH_2CH_2CH_2CH_2NH_2$ | 353 |
| 7-63 | H | H | H | H | H | H | $CH_2CH_2CH_2CH_2CH_2NH_2$ | 367 |
| 7-64 | H | H | H | H | H | H | $CH_2CH_2NHMe$ | 339 |
| 7-65 | H | H | H | H | H | H | $CH_2CH_2CH_2NHMe$ | 353 |
| 7-66 | H | H | H | H | H | H | $CH_2CH_2CH_2CH_2NHMe$ | 367 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-67 | H | H | H | H | H | H | $CH_2CH_2CH_2CH_2CH_2NHMe$ | 381 |
| 7-68 | H | H | H | H | H | H | $CH_2CH_2NMe_2$ | 353 |
| 7-69 | H | H | H | H | H | H | $CH_2CH_2CH_2NMe_2$ | 367 |
| 7-70 | H | H | H | H | H | H | $CH_2CH_2CH_2CH_2NMe_2$ | 381 |
| 7-71 | H | H | H | H | H | H | $CH_2CH_2CH_2CH_2CH_2NMe_2$ | 395 |
| 7-72 | H | H | H | H | H | H | $CH_2CH_2NHCH_2CH_2OH$ | 369 |
| 7-73 | H | H | H | H | H | H | $CH_2CH_2CH_2NHCH_2CH_2OH$ | 383 |
| 7-74 | H | H | H | H | H | H | $CH_2CH_2CH_2CH_2NH\text{-}CH_2CH_2OH$ | 397 |
| 7-75 | H | H | H | H | H | H | $CH_2CONH_2$ | 339 |
| 7-76 | H | H | H | H | H | H | $CH_2CH_2CONH_2$ | 353 |
| 7-77 | H | H | H | H | H | H | $CH_2CH_2CH_2CONH_2$ | 367 |
| 7-78 | H | H | H | H | H | H | $CH_2CH_2NHCOMe$ | 367 |
| 7-79 | H | H | H | H | H | H | $CH\ (CH_2NHCOMe)_2$ | 438 |
| 7-80 | H | H | H | H | H | H | $CH_2CH_2NHCOCH_2Me$ | 381 |
| 7-81 | H | H | H | H | H | H | $CH_2CH_2NHCOCH_2CH_2Me$ | 395 |
| 7-82 | H | H | H | H | H | H | $CH_2CH_2NHCOCHMe_2$ | 395 |
| 7-83 | H | H | H | H | H | H | $CH_2CH_2NHCOCMe_3$ | 409 |
| 7-84 | H | H | H | H | H | H | $CH_2CH_2NHCOCH_2OH$ | 383 |
| 7-85 | H | H | H | H | H | H | $CH_2CH_2NHCOCH_2OMe$ | 397 |
| 7-86 | H | H | H | H | H | H | $CH_2CH_2NHCOCH_2NMe_2$ | 410 |
| 7-87 | H | H | H | H | H | H | $CH_2CH_2NHCOC_6H_5$ | 429 |
| 7-88 | H | H | H | H | H | H | a-52 | 430 |
| 7-89 | H | H | H | H | H | H | a-53 | 435 |
| 7-90 | H | H | H | H | H | H | a-54 | 419 |
| 7-91 | H | H | H | H | H | H | a-55 | 431 |
| 7-92 | H | H | H | H | H | H | $CH_2CH_2NHCOCH_2NHCOMe$ | 424 |
| 7-93 | H | H | H | H | H | H | $CH_2CH_2NHCOCH_2NHCO\text{-}CH_2CHMe_2$ | 466 |
| 7-94 | H | H | H | H | H | H | $CH_2CH_2NHCOCH_2NHCO\text{-}C_6H_5$ | 486 |
| 7-95 | H | H | H | H | H | H | $CH_2CH_2CH_2NHCOMe$ | 381 |
| 7-96 | H | H | H | H | H | H | $CH_2CH_2CH_2NHCOCH_2Me$ | 395 |
| 7-97 | H | H | H | H | H | H | $CH_2CH_2CH_2NHCO\text{-}CH_2CH_2Me$ | 409 |
| 7-98 | H | H | H | H | H | H | $CH_2CH_2CH_2NHCOCHMe_2$ | 409 |
| 7-99 | H | H | H | H | H | H | $CH_2CH_2CH_2NHCOCMe_3$ | 423 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-100 | H | H | H | H | H | H | $CH_2CH_2CH_2CH_2NHCOMe$ | 395 |
| 7-101 | H | H | H | H | H | H | $CH_2CH_2CH_2CH_2NHCO-CH_2Me$ | 409 |
| 7-102 | H | H | H | H | H | H | $CH_2CH_2CH_2CH_2NHCO-CH_2CH_2Me$ | 423 |
| 7-103 | H | H | H | H | H | H | $CH_2CH_2CH_2CH_2NHCO-CHMe_2$ | 423 |
| 7-104 | H | H | H | H | H | H | $CH_2CH_2CH_2CH_2NHCOCMe_3$ | 437 |
| 7-105 | H | H | H | H | H | H | $CH_2CH_2N(Me)COMe$ | 381 |
| 7-106 | H | H | H | H | H | H | $CH_2CH_2N(Me)COC_6H_5$ | 443 |
| 7-107 | H | H | H | H | H | H | $CH_2CH_2CH_2N(Me)COMe$ | 395 |
| 7-108 | H | H | H | H | H | H | $CH_2CH_2CH_2N(Me)COC_6H_5$ | 457 |
| 7-109 | H | H | H | H | H | H | $CH_2CH_2CH_2CH_2N(Me)-COMe$ | 409 |
| 7-110 | H | H | H | H | H | H | $CH_2CH_2CH_2CH_2N(Me)-COC_6H_5$ | 471 |
| 7-111 | H | H | H | H | H | H | $CH_2CH_2N(CH_2CH_2OH)-COMe$ | 411 |
| 7-112 | H | H | H | H | H | H | $CH_2CH_2N(CH_2CH_2OH)-COC_6H_5$ | 474 |
| 7-113 | H | H | H | H | H | H | $CH_2CH_2CH_2$-N ($CH_2CH_2OH$) COMe | 425 |
| 7-114 | H | H | H | H | H | H | $CH_2CH_2CH_2$-N ($CH_2CH_2OH$) $COC_6H_5$ | 488 |
| 7-115 | H | H | H | H | H | H | $CH_2CH_2CH_2CH_2$-N ($CH_2CH_2OH$) COMe | 439 |
| 7-116 | H | H | H | H | H | H | $CH_2CH_2CH_2CH_2-N(CH_2CH_2OH)COC_6H_5$ | 502 |
| 7-117 | H | H | H | H | H | H | $CH_2CH_2N$ ($CH_2CN$) COMe | 406 |
| 7-118 | H | H | H | H | H | H | $CH_2CH_2N$ ($CH_2CN$) $COC_6H_5$ | 469 |
| 7-119 | H | H | H | H | H | H | $CH_2CH_2CH_2N$ ($CH_2CN$) CO Me | 420 |
| 7-120 | H | H | H | H | H | H | $CH_2CH_2CH_2N$ ($CH_2CN$) -$COC_6H_5$ | 483 |
| 7-121 | H | H | H | H | H | H | $CH_2CH_2CH_2CH_2$-N ($CH_2CN$) COMe | 434 |
| 7-122 | H | H | H | H | H | H | $CH_2CH_2CH_2CH_2-N(CH_2CN)COC_6H_5$ | 497 |
| 7-123 | H | H | H | H | H | H | a-3 | 380 |
| 7-124 | H | H | H | H | H | H | a-5 | 379 |
| 7-125 | H | H | H | H | H | H | a-6 | 421 |
| 7-126 | H | H | H | H | H | H | $CH_2CH_2NHSO_2Me$ | 403 |
| 7-127 | H | H | H | H | H | H | $CH_2CH_2NHSO_2NMe_2$ | 432 |
| 7-128 | H | H | H | H | H | H | $CH_2CH_2N(Me)$ $SO_2Me$ | 417 |
| 7-129 | H | H | H | H | H | H | $CH_2CH_2N(Me)SO_2C_6H_5$ | 479 |
| 7-130 | H | H | H | H | H | H | $CH_2CH_2N$ $(Me)SO_2NMe_2$ | 446 |
| 7-131 | H | H | H | H | H | H | $CH_2CH_2N(CH_2CH_2OH)-SO_2Me$ | 433 |
| 7-132 | H | H | H | H | H | H | $CH_2CH_2CH_2NHSO_2Me$ | 417 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-133 | H | H | H | H | H | H | $CH_2CH_2CH_2NHSO_2C_6H_5$ | 479 |
| 7-134 | H | H | H | H | H | H | $CH_2CH_2CH_2NHSO_2NMe_2$ | 446 |
| 7-135 | H | H | H | H | H | H | $CH_2CH_2CH_2N(Me)SO_2Me$ | 431 |
| 7-136 | H | H | H | H | H | H | $CH_2CH_2CH_2N$ (Me) -$SO_2C_6H_5$ | 493 |
| 7-137 | H | H | H | H | H | H | $CH_2CH_2CH_2N$ (Me)-$SO_2NMe_2$ | 460 |
| 7-138 | H | H | H | H | H | H | $CH_2CH_2CH_2$-N ($CH_2CH_2OH$)$SO_2Me$ | 447 |
| 7-139 | H | H | H | H | H | H | $CH_2CH_2CH_2CH_2NHSO_2Me$ | 431 |
| 7-140 | H | H | H | H | H | H | $CH_2CH_2CH_2CH_2NH$-$SO_2C_6H_5$ | 493 |
| 7-141 | H | H | H | H | H | H | $CH_2CH_2CH_2CH_2NH$-$SO_2NMe_2$ | 460 |
| 7-142 | H | H | H | H | H | H | $CH_2CH_2CH_2CH_2N$ (Me) $S0_2$ Me | 445 |
| 7-143 | H | H | H | H | H | H | $CH_2CH_2CH_2CH_2N$ (Me) -$SO_2C_6H_5$ | 507 |
| 7-144 | H | H | H | H | H | H | $CH_2CH_2CH_2CH_2N$ (Me) $SO_2$ $NMe_2$ | 474 |
| 7-145 | H | H | H | H | H | H | $CH_2CH_2CH_2CH_2$-N($CH_2CH_2OH$)$SO_2Me$ | 461 |
| 7-146 | H | H | H | H | H | H | a-66 | 457 |
| 7-147 | H | H | H | H | H | H | $CH_2CH_2NHCONHMe$ | 382 |
| 7-148 | H | H | H | H | H | H | $CH_2CH_2NHCONHCH_2Me$ | 396 |
| 7-149 | H | H | H | H | H | H | $CH_2CH_2NHCONH$-$CH_2CH_2Me$ | 410 |
| 7-150 | H | H | H | H | H | H | $CH_2CH_2NHCONHCHMe_2$ | 410 |
| 7-151 | H | H | H | H | H | H | $CH_2CH_2NHCONHCMe_3$ | 424 |
| 7-152 | H | H | H | H | H | H | a-68 | 436 |
| 7-153 | H | H | H | H | H | H | a-69 | 450 |
| 7-154 | H | H | H | H | H | H | a-70 | 464 |
| 7-155 | H | H | H | H | H | H | a-71 | 478 |
| 7-156 | H | H | H | H | H | H | a-72 | 502 |
| 7-157 | H | H | H | H | H | H | $CH_2CH_2NHCONHC_6H_5$ | 444 |
| 7-158 | H | H | H | H | H | H | a-73 | 474 |
| 7-159 | H | H | H | H | H | H | a-74 | 460 |
| 7-160 | H | H | H | H | H | H | a-75 | 459 |
| 7-161 | H | H | H | H | H | H | a-76 | 487 |
| 7-162 | H | H | H | H | H | H | a-77 | 494 |
| 7-163 | H | H | H | H | H | H | a-78 | 494 |
| 7-164 | H | H | H | H | H | H | a-79 | 469 |
| 7-165 | H | H | H | H | H | H | a-80 | 473 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-166 | H | H | H | H | H | H | a-81 | 478 |
| 7-167 | H | H | H | H | H | H | $CH_2CH_2NHCONHCOC_6H_5$ | 472 |
| 7-168 | H | H | H | H | H | H | a-82 | 386 |
| 7-169 | H | H | H | H | H | H | a-83 | 386 |
| 7-170 | H | H | H | H | H | H | a-84 | 501 |
| 7-171 | H | H | H | H | H | H | a-85 | 488 |
| 7-172 | H | H | H | H | H | H | a-86 | 500 |
| 7-173 | H | H | H | H | H | H | a-87 | 450 |
| 7-174 | H | H | H | H | H | H | a-88 | 463 |
| 7-175 | H | H | H | H | H | H | $CH_2CH_2N(Me)CONHC_6H_5$ | 458 |
| 7-176 | H | H | H | H | H | H | $CH_2CH_2N$ (Me) CO-N(Me)$C_6H_5$ | 472 |
| 7-177 | H | H | H | H | H | H | $CH_2CH_2NHCONH\text{-}CH_2CH_2OH$ | 412 |
| 7-178 | H | H | H | H | H | H | $CH_2CH_2NHCONH\text{-}CH_2CH_2OMe$ | 426 |
| 7-179 | H | H | H | H | H | H | $CH_2CH_2NHCONH\text{-}CH_2CH_2NMe_2$ | 439 |
| 7-180 | H | H | H | H | H | H | $CH_2CH_2NHCON$ (Me)-$CH_2CH_2OH$ | 426 |
| 7-181 | H | H | H | H | H | H | $CH_2CH_2NHCO\text{-}N(CH_2CH_2OH)_2$ | 456 |
| 7-182 | H | H | H | H | H | H | a-89 | 437 |
| 7-183 | H | H | H | H | H | H | $CH_2CH_2NHCONMe_2$ | 396 |
| 7-184 | H | H | H | H | H | H | $CH_2CH_2CH_2NHCONHMe$ | 396 |
| 7-185 | H | H | H | H | H | H | $CH_2CH_2CH_2NHCONH\text{-}CH_2Me$ | 410 |
| 7-186 | H | H | H | H | H | H | $CH_2CH_2CH_2NHCONH\text{-}CH_2CH_2Me$ | 424 |
| 7-187 | H | H | H | H | H | H | $CH_2CH_2CH_2NHCONH\text{-}$ $CHMe_2$ | 424 |
| 7-188 | H | H | H | H | H | H | $CH_2CH_2CH_2NHCONHCMe_3$ | 438 |
| 7-189 | H | H | H | H | H | H | a-90 | 450 |
| 7-190 | H | H | H | H | H | H | a-92 | 464 |
| 7-191 | H | H | H | H | H | H | a-93 | 478 |
| 7-192 | H | H | H | H | H | H | a-94 | 492 |
| 7-193 | H | H | H | H | H | H | a-95 | 516 |
| 7-194 | H | H | H | H | H | H | a-96 | 458 |
| 7-195 | H | H | H | H | H | H | a-97 | 488 |
| 7-196 | H | H | H | H | H | H | a-98 | 474 |
| 7-197 | H | H | H | H | H | H | a-99 | 473 |
| 7-198 | H | H | H | H | H | H | a-100 | 501 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-199 | H | H | H | H | H | H | a-101 | 508 |
| 7-200 | H | H | H | H | H | H | a-102 | 508 |
| 7-201 | H | H | H | H | H | H | a-103 | 483 |
| 7-202 | H | H | H | H | H | H | $CH_2CH_2CH_2NHCO$- $NHCOC_6H_5$ | 487 |
| 7-203 | H | H | H | H | H | H | $CH_2CH_2CH_2NHCO$- $NHSO_2C_6H_5$ | 492 |
| 7-204 | H | H | H | H | H | H | a-104 | 486 |
| 7-205 | H | H | H | H | H | H | a-105 | 534 |
| 7-206 | H | H | H | H | H | H | a-106 | 534 |
| 7-207 | H | H | H | H | H | H | a-107 | 515 |
| 7-208 | H | H | H | H | H | H | a-108 | 502 |
| 7-209 | H | H | H | H | H | H | a-109 | 514 |
| 7-210 | H | H | H | H | H | H | $CH_2CH_2CH_2NHCO-NHCMe_2CH_2CMe_3$ | 464 |
| 7-211 | H | H | H | H | H | H | a-110 | 477 |
| 7-212 | H | H | H | H | H | H | $CH_2CH_2CH_2N(Me)CO$- $NHC_6H_5$ | 472 |
| 7-213 | H | H | H | H | H | H | $CH_2CH_2CH_2N$ (Me) CO- $N(Me)C_6H_5$ | 486 |
| 7-214 | H | H | H | H | H | H | $CH_2CH_2CH_2NHCONH-CH_2CH_2OH$ | 426 |
| 7-215 | H | H | H | H | H | H | $CH_2CH_2CH_2NHCONH-CH_2CH_2OMe$ | 440 |
| 7-216 | H | H | H | H | H | H | $CH_2CH_2CH_2NHCONH-CH_2CH_2NMe_2$ | 453 |
| 7-217 | H | H | H | H | H | H | $CH_2CH_2CH_2NHCO-N(Me)CH_2CH_2OH$ | 440 |
| 7-218 | H | H | H | H | H | H | $CH_2CH_2CH_2NHCO-N(CH_2CH_2OH)_2$ | 470 |
| 7-219 | H | H | H | H | H | H | a-111 | 451 |
| 7-220 | H | H | H | H | H | H | $CH_2CH_2CH_2NHCONMe_2$ | 410 |
| 7-221 | H | H | H | H | H | H | $CH_2CH_2CH_2CH_2NHCO-NHMe$ | 410 |
| 7-222 | H | H | H | H | H | H | $CH_2CH_2CH_2CH_2NHCO-NHCH_2Me$ | 424 |
| 7-223 | H | H | H | H | H | H | $CH_2CH_2CH_2CH_2NHCO-NHCH_2CH_2Me$ | 438 |
| 7-224 | H | H | H | H | H | H | $CH_2CH_2CH_2CH_2NHCO-NHCHMe_2$ | 438 |
| 7-225 | H | H | H | H | H | H | $CH_2CH_2CH_2CH_2NHCO-NHCMe_3$ | 452 |
| 7-226 | H | H | H | H | H | H | a-123 | 464 |
| 7-227 | H | H | H | H | H | H | a-124 | 478 |
| 7-228 | H | H | H | H | H | H | a-125 | 492 |
| 7-229 | H | H | H | H | H | H | a-126 | 506 |
| 7-230 | H | H | H | H | H | H | a-127 | 530 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-231 | H | H | H | H | H | H | $CH_2CH_2CH_2CH_2NHCO\text{-}NHC_6H_5$ | 472 |
| 7-232 | H | H | H | H | H | H | a-128 | 502 |
| 7-233 | H | H | H | H | H | H | a-129 | 488 |
| 7-234 | H | H | H | H | H | H | a-130 | 487 |
| 7-235 | H | H | H | H | H | H | a-131 | 515 |
| 7-236 | H | H | H | H | H | H | a-132 | 522 |
| 7-237 | H | H | H | H | H | H | a-133 | 522 |
| 7-238 | H | H | H | H | H | H | a-134 | 497 |
| 7-239 | H | H | H | H | H | H | a-135 | 501 |
| 7-240 | H | H | H | H | H | H | a-136 | 506 |
| 7-241 | H | H | H | H | H | H | $CH_2CH_2CH_2CH_2NHCO\text{-}NHCOC_6H_5$ | 500 |
| 7-242 | H | H | H | H | H | H | $CH_2CH_2CH_2CH_2NHCO\text{-}NHSO_2C_6H_5$ | 548 |
| 7-243 | H | H | H | H | H | H | a-137 | 548 |
| 7-244 | H | H | H | H | H | H | a-138 | 529 |
| 7-245 | H | H | H | H | H | H | a-139 | 516 |
| 7-246 | H | H | H | H | H | H | a-140 | 528 |
| 7-247 | H | H | H | H | H | H | a-141 | 478 |
| 7-248 | H | H | H | H | H | H | a-142 | 491 |
| 7-249 | H | H | H | H | H | H | $CH_2CH_2CH_2CH_2NHCO\text{-}NHCMe_2CH_2CMe_3$ | 486 |
| 7-250 | H | H | H | H | H | H | a-143 | 500 |
| 7-251 | H | H | H | H | H | H | $CH_2CH_2CH_2CH_2N(Me)\text{-}CONHC_6H_5$ | 440 |
| 7-252 | H | H | H | H | H | H | $CH_2CH_2CH_2CH_2N(Me)\text{-}CON\ (Me)\ C_6H_5$ | 454 |
| 7-253 | H | H | H | H | H | H | $CH_2CH_2CH_2CH_2NHCO\text{-}NHCH_2CH_2OH$ | 467 |
| 7-254 | H | H | H | H | H | H | $CH_2CH_2CH_2CH_2NHCO\text{-}NHCH_2CH_2OMe$ | 454 |
| 7-255 | H | H | H | H | H | H | $CH_2CH_2CH_2CH_2NHCO\text{-}NHCH_2CH_2NMe_2$ | 484 |
| 7-256 | H | H | H | H | H | H | $CH_2CH_2CH_2CH_2NHCO\text{-}N\ (Me)\ CH_2CH_2OH$ | 465 |
| 7-257 | H | H | H | H | H | H | $CH_2CH_2CH_2CH_2NHCO\text{-}N(CH_2CH_2OH)_2$ | 424 |
| 7-258 | H | H | H | H | H | H | a-144 | 465 |
| 7-259 | H | H | H | H | H | H | $CH_2CH_2CH_2CH_2NHCONMe_2$ | 424 |
| 7-260 | H | H | H | H | H | H | $CH_2CH_2NHCSNHCH_2Me$ | 412 |
| 7-261 | H | H | H | H | H | H | $CH_2CH_2NHCSNH\text{-}CH_2CH_2Me$ | 426 |
| 7-262 | H | H | H | H | H | H | $CH_2CH_2NHCSNHCHMe_2$ | 426 |
| 7-263 | H | H | H | H | H | H | $CH_2CH_2NHCSNHCMe_3$ | 440 |

(continued)

| Exp. N o. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-264 | H | H | H | H | H | H | a-112 | 386 |
| 7-265 | H | H | H | H | H | H | $CH_2CH_2NHCSNHC_6H_5$ | 460 |
| 7-266 | H | H | H | H | H | H | $CH_2CH_2NHCSNH-CH_2CH_2OH$ | 428 |
| 7-267 | H | H | H | H | H | H | $CH_2CH_2NHCSNH-CH_2CH_2OMe$ | 442 |
| 7-268 | H | H | H | H | H | H | $CH_2CH_2NHCSNH-CH_2CH_2NMe_2$ | 455 |
| 7-269 | H | H | H | H | H | H | $CH_2CH_2NHCSN (Me) -CH_2CH_2OH$ | 442 |
| 7-270 | H | H | H | H | H | H | $CH_2CH_2NHCS-N (CH_2CH_2OH)\ _2$ | 472 |
| 7-271 | H | H | H | H | H | H | a-113 | 453 |
| 7-272 | H | H | H | H | H | H | $CH_2CH_2NHCSNMe_2$ | 412 |
| 7-273 | H | H | H | H | H | H | $CH_2CH_2NHCOOMe$ | 383 |
| 7-274 | H | H | H | H | H | H | $CH_2CH_2NHCOOCH_2Me$ | 397 |
| 7-275 | H | H | H | H | H | H | $CH_2CH_2NHCOOCH_2CH_2Me$ | 411 |
| 7-276 | H | H | H | H | H | H | $CH_2CH_2NHCOOCHMe_2$ | 411 |
| 7-277 | H | H | H | H | H | H | a-114 | 451 |
| 7-278 | H | H | H | H | H | H | $CH_2CH_2NHCOOC_6H_5$ | 445 |
| 7-279 | H | H | H | H | H | H | $CH_2CH_2NHCOOCH_2CH_2OH$ | 413 |
| 7-280 | H | H | H | H | H | H | $CH_2CH_2NHCOO-CH_2CH_2OMe$ | 427 |
| 7-281 | H | H | H | H | H | H | $CH_2CH_2NHCOO-CH_2CH_2NMe_2$ | 440 |
| 7-282 | H | H | H | H | H | H | a-115 | 406 |
| 7-283 | H | H | H | H | H | H | a-116 | 406 |
| 7-284 | H | H | H | H | H | H | a-9 | 379 |
| 7-285 | H | H | H | H | H | H | a-117 | 393 |
| 7-286 | H | H | H | H | H | H | a-118 | 407 |
| 7-287 | H | H | H | H | H | H | a-8 | 324 |
| 7-288 | H | H | H | H | H | H | $CH_2CH_2OCH_2CH_2OMe$ | 384 |
| 7-289 | H | H | H | H | H | H | $CH(CH_2OH)_2$ | 355 |
| 7-290 | H | H | H | H | H | H | a-4 | 380 |
| 7-291 | Cl | H | H | H | H | H | H | 316 |
| 7-292 | Br | H | H | H | H | H | H | 360 |
| 7-293 | OH | H | H | H | H | H | H | 298 |
| 7-294 | Me | H | H | H | H | H | H | 296 |
| 7-295 | $CH=CH_2$ | H | H | H | H | H | H | 308 |
| 7-296 | $C\equiv CC_6H_5$ | H | H | H | H | H | H | 382 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-297 | OMe | H | H | H | H | H | H | 312 |
| 7-298 | $NH_2$ | H | H | H | H | H | H | 297 |
| 7-299 | NHMe | H | H | H | H | H | H | 311 |
| 7-300 | $NMe_2$ | H | H | H | H | H | H | 325 |
| 7-301 | $NHC_6H_5$ | H | H | H | H | H | H | 373 |
| 7-302 | OH | H | H | H | H | H | $CH_2CH_2CH_2CH_2COOH$ | 398 |
| 7-303 | OH | H | H | H | H | H | $CH_2CH_2CH_2CH_2CH_2OH$ | 384 |
| 7-304 | OH | H | H | H | H | H | $CH_2CH_2SCOMe$ | 400 |
| 7-305 | OH | H | H | H | H | H | $CH_2CH_2SH$ | 358 |
| 7-306 | OH | H | H | H | H | H | COMe | 340 |
| 7-307 | OH | H | H | H | H | H | $SO_2Me$ | 376 |
| 7-308 | OH | H | H | H | H | H | $CH_2CH_2CH_2NHCONHC_6H_5$ | 474 |
| 7-309 | OH | H | H | H | H | H | a-91 | 480 |
| 7-311 | OH | H | H | H | H | H | Me | 312 |
| 7-312 | OH | H | H | H | H | H | a-121 | 352 |
| 7-313 | OH | H | H | H | H | H | a-122 | 337 |
| 7-314 | OH | H | H | H | H | H | Bn | 388 |
| 7-315 | OH | H | H | H | H | H | a-2 | 418 |
| 7-316 | OH | H | H | H | H | Me | Me | 326 |
| 7-317 | OH | H | H | H | H | H | $COC_6H_5$ | 402 |
| 7-318 | OH | H | H | H | H | H | $SO_2C_6H_5$ | 438 |
| 7-319 | OH | H | H | H | H | H | $CH_2COOH$ | 356 |
| 7-320 | OH | H | H | H | H | H | $CH_2CH_2COOH$ | 370 |
| 7-321 | OH | H | H | H | H | H | $CH_2CH_2CH_2COOH$ | 384 |
| 7-322 | OH | H | H | H | H | H | $CH_2CN$ | 337 |
| 7-323 | OH | H | H | H | H | H | $CH_2CH_2CN$ | 351 |
| 7-324 | OH | H | H | H | H | H | $CH_2CH_2CH_2CN$ | 365 |
| 7-325 | OH | H | H | H | H | H | $CH_2CH_2CH_2CH_2CN$ | 379 |
| 7-326 | OH | H | H | H | H | H | $CH_2CH_2OH$ | 342 |
| 7-327 | OH | H | H | H | H | H | $CH_2CH$ (Me) OH | 356 |
| 7-328 | OH | H | H | H | H | H | CH (Me) $CH_2OH$ | 356 |
| 7-329 | OH | H | H | H | H | H | $CH_2CH_2CH_2OH$ | 356 |
| 7-330 | OH | H | H | H | H | H | $CH_2CH$ (OH) $CH_2OH$ | 372 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-331 | OH | H | H | H | H | H | $CH_2CH$ (Me) $CH_2OH$ | 370 |
| 7-332 | OH | H | H | H | H | H | $CH_2CH_2CH_2CH_2OH$ | 370 |
| 7-333 | OH | H | H | H | H | H | $CH_2CH_2OCONHMe$ | 399 |
| 7-334 | OH | H | H | H | H | H | a-7 | 453 |
| 7-335 | OH | H | H | H | H | H | $CH_2CH_2OCONHC_6H_5$ | 461 |
| 7-336 | OH | H | H | H | H | H | $CH_2CH_2CH_2OCONHMe$ | 413 |
| 7-337 | OH | H | H | H | H | H | a-48 | 467 |
| 7-338 | OH | H | H | H | H | H | $CH_2CH_2CH_2OCONHC_6H_5$ | 475 |
| 7-339 | OH | H | H | H | H | H | $CH_2CH_2CH_2CH_2OCONHMe$ | 427 |
| 7-340 | OH | H | H | H | H | H | a-49 | 481 |
| 7-341 | OH | H | H | H | H | H | $CH_2CH_2CH_2CH_2OCO$-$NHC_6H_5$ | 489 |
| 7-342 | OH | H | H | H | H | H | $CH_2CH_2CH_2SH$ | 372 |
| 7-343 | OH | H | H | H | H | H | $CH_2CH_2SCOC_6H_5$ | 462 |
| 7-344 | OH | H | H | H | H | H | $CH_2CH_2CH_2SCOMe$ | 414 |
| 7-345 | OH | H | H | H | H | H | $CH_2CH_2CH_2SCOC_6H_5$ | 476 |
| 7-346 | OH | H | H | H | H | H | $CH_2CH_2OMe$ | 356 |
| 7-347 | OH | H | H | H | H | H | $CH_2CH_2CH_2OMe$ | 370 |
| 7-348 | OH | H | H | H | H | H | $CH_2CH_2CH_2CH_2OMe$ | 384 |
| 7-349 | OH | H | H | H | H | H | $CH_2CH_2CH_2CH_2CH_2OMe$ | 398 |
| 7-350 | OH | H | H | H | H | H | $CH_2CH_2OCH_2CH_2OH$ | 386 |
| 7-351 | OH | H | H | H | H | H | $CH_2CH_2CH_2OCH_2CH_2OH$ | 400 |
| 7-352 | OH | H | H | H | H | H | $CH_2CH_2CH_2CH_2O$-$CH_2CH_2OH$ | 414 |
| 7-353 | OH | H | H | H | H | H | $CH(CH_2OH)CH_2NH_2$ | 371 |
| 7-354 | OH | H | H | H | H | H | CH ($CH_2OH$) $CH_2NHCOMe$ | 413 |
| 7-355 | OH | H | H | H | H | H | a-51 | 496 |
| 7-356 | OH | H | H | H | H | H | CH ($CH_2OH$) $CH_2NHCO$-$NHC_6H_5$ | 490 |
| 7-357 | OH | H | H | H | H | H | $CH_2CH_2NH_2$ | 341 |
| 7-358 | OH | H | H | H | H | H | $CH_2CH_2CH_2NH_2$ | 355 |
| 7-359 | OH | H | H | H | H | H | $CH_2CH(OH)CH_2NH_2$ | 371 |
| 7-360 | OH | H | H | H | H | H | $CH_2CH$ (OH) $CH_2NHMe$ | 385 |
| 7-361 | OH | H | H | H | H | H | $CH_2CH$ (OH) $CH_2NMe_2$ | 399 |
| 7-362 | OH | H | H | H | H | H | $CH_2CH_2CH_2CH_2NH_2$ | 369 |
| 7-363 | OH | H | H | H | H | H | $CH_2CH_2CH_2CH_2CH_2NH_2$ | 383 |

(continued)

| Exp. N o. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-364 | OH | H | H | H | H | H | $CH_2CH_2NHMe$ | 355 |
| 7-365 | OH | H | H | H | H | H | $CH_2CH_2CH_2NHMe$ | 369 |
| 7-366 | OH | H | H | H | H | H | $CH_2CH_2CH_2CH_2NHMe$ | 383 |
| 7-367 | OH | H | H | H | H | H | $CH_2CH_2CH_2CH_2CH_2NHMe$ | 397 |
| 7-368 | OH | H | H | H | H | H | $CH_2CH_2NMe_2$ | 369 |
| 7-369 | OH | H | H | H | H | H | $CH_2CH_2CH_2NMe_2$ | 383 |
| 7-370 | OH | H | H | H | H | H | $CH_2CH_2CH_2CH_2NMe_2$ | 397 |
| 7-371 | OH | H | H | H | H | H | $CH_2CH_2CH_2CH_2CH_2NMe_2$ | 411 |
| 7-372 | OH | H | H | H | H | H | $CH_2CH_2NHCH_2CH_2OH$ | 385 |
| 7-373 | OH | H | H | H | H | H | $CH_2CH_2CH_2NHCH_2CH_2OH$ | 399 |
| 7-374 | OH | H | H | H | H | H | $CH_2CH_2CH_2CH_2NH$- $CH_2CH_2OH$ | 413 |
| 7-375 | OH | H | H | H | H | H | $CH_2CONH_2$ | 355 |
| 7-376 | OH | H | H | H | H | H | $CH_2CH_2CONH_2$ | 369 |
| 7-377 | OH | H | H | H | H | H | $CH_2CH_2CH_2CONH_2$ | 383 |
| 7-378 | OH | H | H | H | H | H | $CH_2CH_2NHCOMe$ | 383 |
| 7-379 | OH | H | H | H | H | H | $CH(CH_2NHCOMe)_2$ | 454 |
| 7-380 | OH | H | H | H | H | H | $CH_2CH_2NHCOCH_2Me$ | 397 |
| 7-381 | OH | H | H | H | H | H | $CH_2CH_2NHCOCH_2CH_2Me$ | 411 |
| 7-382 | OH | H | H | H | H | H | $CH_2CH_2NHCOCHMe_2$ | 411 |
| 7-383 | OH | H | H | H | H | H | $CH_2CH_2NHCOCMe_3$ | 425 |
| 7-384 | OH | H | H | H | H | H | $CH_2CH_2NHCOCH_2OH$ | 399 |
| 7-385 | OH | H | H | H | H | H | $CH_2CH_2NHCOCH_2OMe$ | 413 |
| 7-386 | OH | H | H | H | H | H | $CH_2CH_2NHCOCH_2NMe_2$ | 426 |
| 7-387 | OH | H | H | H | H | H | $CH_2CH_2NHCOC_6H_5$ | 445 |
| 7-388 | OH | H | H | H | H | H | a-52 | 446 |
| 7-389 | OH | H | H | H | H | H | a-53 | 451 |
| 7-390 | OH | H | H | H | H | H | a-54 | 435 |
| 7-391 | OH | H | H | H | H | H | a-55 | 447 |
| 7-392 | OH | H | H | H | H | H | $CH_2CH_2NHCOCH_2NHCOMe$ | 440 |
| 7-393 | OH | H | H | H | H | H | $CH_2CH_2NHCOCH_2NH$-$COCH_2CHMe_2$ | 482 |
| 7-394 | OH | H | H | H | H | H | $CH_2CH_2NHCOCH_2NHCO$-$C_6H_5$ | 502 |

(continued)

| Exp. N o. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-395 | OH | H | H | H | H | H | $CH_2CH_2CH_2NHCOMe$ | 397 |
| 7-396 | OH | H | H | H | H | H | $CH_2CH_2CH_2NHCOCH_2Me$ | 411 |
| 7-397 | OH | H | H | H | H | H | $CH_2CH_2CH_2NHCO\text{-}CH_2CH_2Me$ | 425 |
| 7-398 | OH | H | H | H | H | H | $CH_2CH_2CH_2NHCOCHMe_2$ | 425 |
| 7-399 | OH | H | H | H | H | H | $CH_2CH_2CH_2NHCOCMe_3$ | 439 |
| 7-400 | OH | H | H | H | H | H | $CH_2CH_2CH_2CH_2NHCOMe$ | 411 |
| 7-401 | OH | H | H | H | H | H | $CH_2CH_2CH_2CH_2NHCO\text{-}CH_2Me$ | 425 |
| 7-402 | OH | H | H | H | H | H | $CH_2CH_2CH_2CH_2NHCO\text{-}CH_2CH_2Me$ | 439 |
| 7-403 | OH | H | H | H | H | H | $CH_2CH_2CH_2CH_2NHCO\text{-}CHMe_2$ | 439 |
| 7-404 | OH | H | H | H | H | H | $CH_2CH_2CH_2CH_2NHCOCMe_3$ | 453 |
| 7-405 | OH | H | H | H | H | H | $CH_2CH_2N$ (Me) COMe | 397 |
| 7-406 | OH | H | H | H | H | H | $CH_2CH_2N$ (Me) $COC_6H_5$ | 459 |
| 7-407 | OH | H | H | H | H | H | $CH_2CH_2CH_2N$ (Me) COMe | 411 |
| 7-408 | OH | H | H | H | H | H | $CH_2CH_2CH_2N$ (Me) $COC_6H_5$ | 473 |
| 7-409 | OH | H | H | H | H | H | $CH_2CH_2CH_2CH_2N$ (Me) -COMe | 425 |
| 7-410 | OH | H | H | H | H | H | $CH_2CH_2CH_2CH_2N$ (Me) - $COC_6H_5$ | 487 |
| 7-411 | OH | H | H | H | H | H | $CH_2CH_2N$ ($CH_2CH_2OH$)-COMe | 427 |
| 7-412 | OH | H | H | H | H | H | $CH_2CH_2N$ ($CH_2CH_2OH$)-$COC_6H_5$ | 490 |
| 7-413 | OH | H | H | H | H | H | $CH_2CH_2CH_2\text{-}N(CH_2CH_2OH)COMe$ | 441 |
| 7-414 | OH | H | H | H | H | H | $CH_2CH_2CH_2$-N ($CH_2CH_2OH$) $COC_6H_5$ | 504 |
| 7-415 | OH | H | H | H | H | H | $CH_2CH_2CH_2CH_2\text{-}N(CH_2CH_2OH)COMe$ | 455 |
| 7-416 | OH | H | H | H | H | H | $CH_2CH_2CH_2CH_2\text{-}N(CH_2CH_2OH)COC_6H_5$ | 518 |
| 7-417 | OH | H | H | H | H | H | $CH_2CH_2N$ ($CH_2CN$) COMe | 422 |
| 7-418 | OH | H | H | H | H | H | $CH_2CH_2N$ ($CH_2CN$) $COC_6H_5$ | 485 |
| 7-419 | OH | H | H | H | H | H | $CH_2CH_2CH_2N$ ($CH_2CN$) CO Me | 436 |
| 7-420 | OH | H | H | H | H | H | $CH_2CH_2CH_2$-N ($CH_2CN$) $COC_6H_5$ | 499 |
| 7-421 | OH | H | H | H | H | H | $CH_2CH_2CH_2CH_2$-N ($CH_2CN$) COMe | 450 |
| 7-422 | OH | H | H | H | H | H | $CH_2CH_2CH_2CH2\text{-}N(CH_2CN)COC_6H_5$ | 513 |
| 7-423 | OH | H | H | H | H | H | a-3 | 396 |
| 7-424 | OH | H | H | H | H | H | a-5 | 395 |
| 7-425 | OH | H | H | H | H | H | a-6 | 437 |
| 7-426 | OH | H | H | H | H | H | $CH_2CH_2NHSO_2Me$ | 419 |
| 7-427 | OH | H | H | H | H | H | $CH_2CH_2NHSO_2NMe_2$ | 448 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-428 | OH | H | H | H | H | H | $CH_2CH_2N(Me)SO_2Me$ | 433 |
| 7-429 | OH | H | H | H | H | H | $CH_2CH_2N\ (Me)SO_2C_6H_5$ | 495 |
| 7-430 | OH | H | H | H | H | H | $CH_2CH_2N(Me)SO_2NMe_2$ | 462 |
| 7-431 | OH | H | H | H | H | H | $CH_2CH_2N(CH_2CH_2OH)\text{-}SO_2Me$ | 449 |
| 7-432 | OH | H | H | H | H | H | $CH_2CH_2CH_2NHSO_2Me$ | 433 |
| 7-433 | OH | H | H | H | H | H | $CH_2CH_2CH_2NHSO_2C_6H_5$ | 495 |
| 7-434 | OH | H | H | H | H | H | $CH_2CH_2CH_2NHSO_2NMe_2$ | 462 |
| 7-435 | OH | H | H | H | H | H | $CH_2CH_2CH_2N\ (Me)\ SO_2Me$ | 447 |
| 7-436 | OH | H | H | H | H | H | $CH_2CH_2CH_2N(Me)\text{-}SO_2C_6H_5$ | 509 |
| 7-437 | OH | H | H | H | H | H | $CH_2CH_2CH_2N\ (Me)\text{-}SO_2NMe_2$ | 476 |
| 7-438 | OH | H | H | H | H | H | $CH_2CH_2CH_2\text{-}N(CH_2CH_2OH)SO_2Me$ | 463 |
| 7-439 | OH | H | H | H | H | H | $CH_2CH_2CH_2CH_2NHSO_2Me$ | 447 |
| 7-440 | OH | H | H | H | H | H | $CH_2CH_2CH_2CH_2NH\text{-}SO_2C_6H_5$ | 509 |
| 7-441 | OH | H | H | H | H | H | $CH_2CH_2CH_2CH_2NH\text{-}SO_2NMe_2$ | 476 |
| 7-442 | OH | H | H | H | H | H | $CH_2CH_2CH_2CH_2N\ (Me)\text{-}SO_2Me$ | 461 |
| 7-443 | OH | H | H | H | H | H | $CH_2CH_2CH_2CH_2N\ (Me)\text{-}SO_2C_6H_5$ | 523 |
| 7-444 | OH | H | H | H | H | H | $CH_2CH_2CH_2CH_2N\ (Me)\ \text{-}SO_2NMe_2$ | 490 |
| 7-445 | OH | H | H | H | H | H | $CH_2CH_2CH_2CH_2\ \text{-}N(CH_2CH_2OH)SO_2Me$ | 477 |
| 7-446 | OH | H | H | H | H | H | a-66 | 473 |
| 7-447 | OH | H | H | H | H | H | $CH_2CH_2NHCONHMe$ | 398 |
| 7-448 | OH | H | H | H | H | H | $CH_2CH_2NHCONHCH_2Me$ | 412 |
| 7-449 | OH | H | H | H | H | H | $CH_2CH_2NHCONH\text{-}CH_2CH_2Me$ | 426 |
| 7-450 | OH | H | H | H | H | H | $CH_2CH_2NHCONHCHMe_2$ | 426 |
| 7-451 | OH | H | H | H | H | H | $CH_2CH_2NHCONHCMe_3$ | 440 |
| 7-452 | OH | H | H | H | H | H | a-68 | 452 |
| 7-453 | OH | H | H | H | H | H | a-69 | 466 |
| 7-454 | OH | H | H | H | H | H | a-70 | 480 |
| 7-455 | OH | H | H | H | H | H | a-71 | 494 |
| 7-456 | OH | H | H | H | H | H | a-72 | 518 |
| 7-457 | OH | H | H | H | H | H | $CH_2CH_2NHCONHC_6H_5$ | 460 |
| 7-458 | OH | H | H | H | H | H | a-73 | 490 |
| 7-459 | OH | H | H | H | H | H | a-74 | 476 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-460 | OH | H | H | H | H | H | a-75 | 475 |
| 7-461 | OH | H | H | H | H | H | a-76 | 503 |
| 7-462 | OH | H | H | H | H | H | a-77 | 510 |
| 7-463 | OH | H | H | H | H | H | a-78 | 510 |
| 7-464 | OH | H | H | H | H | H | a-79 | 485 |
| 7-465 | OH | H | H | H | H | H | a-80 | 489 |
| 7-466 | OH | H | H | H | H | H | a-81 | 494 |
| 7-467 | OH | H | H | H | H | H | $CH_2CH_2NHCONHCOC_6H_5$ | 488 |
| 7-468 | OH | H | H | H | H | H | a-82 | 402 |
| 7-469 | OH | H | H | H | H | H | a-83 | 402 |
| 7-470 | OH | H | H | H | H | H | a-84 | 517 |
| 7-471 | OH | H | H | H | H | H | a-85 | 504 |
| 7-472 | OH | H | H | H | H | H | a-86 | 516 |
| 7-473 | OH | H | H | H | H | H | a-87 | 466 |
| 7-474 | OH | H | H | H | H | H | a-88 | 479 |
| 7-475 | OH | H | H | H | H | H | $CH_2CH_2N(Me)CONHC_6H_5$ | 474 |
| 7-476 | OH | H | H | H | H | H | $CH_2CH_2N$ (Me) CO- $N(Me)C_6H_5$ | 488 |
| 7-477 | OH | H | H | H | H | H | $CH_2CH_2NHCONH-CH_2CH_2OH$ | 428 |
| 7-478 | OH | H | H | H | H | H | $CH_2CH_2NHCONH-CH_2CH_2OMe$ | 442 |
| 7-479 | OH | H | H | H | H | H | $CH_2CH_2NHCONH-CH_2CH_2NMe_2$ | 455 |
| 7-480 | OH | H | H | H | H | H | $CH_2CH_2NHCON$ (Me) $-CH_2CH_2OH$ | 442 |
| 7-481 | OH | H | H | H | H | H | $CH_2CH_2NHCO-N$ $(CH_2CH_2OH)_2$ | 472 |
| 7-482 | OH | H | H | H | H | H | a-89 | 453 |
| 7-483 | OH | H | H | H | H | H | $CH_2CH_2NHCONMe_2$ | 412 |
| 7-484 | OH | H | H | H | H | H | $CH_2CH_2CH_2NHCONHMe$ | 412 |
| 7-485 | OH | H | H | H | H | H | $CH_2CH_2CH_2NHCO-NHCH_2Me$ | 426 |
| 7-486 | OH | H | H | H | H | H | $CH_2CH_2CH_2NHCO-NHCH_2CH_2Me$ | 440 |
| 7-487 | OH | H | H | H | H | H | $CH_2CH_2CH_2NHCO-NHCHMe_2$ | 440 |
| 7-488 | OH | H | H | H | H | H | $CH_2CH_2CH_2NHCONHCMe_3$ | 454 |
| 7-489 | OH | H | H | H | H | H | a-90 | 466 |
| 7-490 | OH | H | H | H | H | H | a-92 | 480 |
| 7-491 | OH | H | H | H | H | H | a-93 | 494 |
| 7-492 | OH | H | H | H | H | H | a-94 | 508 |

(continued)

| Exp. N o. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-493 | OH | H | H | H | H | H | a-95 | 532 |
| 7-494 | OH | H | H | H | H | H | a-96 | 474 |
| 7-495 | OH | H | H | H | H | H | a-97 | 504 |
| 7-496 | OH | H | H | H | H | H | a-98 | 490 |
| 7-497 | OH | H | H | H | H | H | a-99 | 489 |
| 7-498 | OH | H | H | H | H | H | a-100 | 517 |
| 7-499 | OH | H | H | H | H | H | a-101 | 524 |
| 7-500 | OH | H | H | H | H | H | a-102 | 524 |
| 7-501 | OH | H | H | H | H | H | a-103 | 499 |
| 7-502 | OH | H | H | H | H | H | $CH_2CH_2CH_2NHC0\text{-}NHCOC_6H_5$ | 503 |
| 7-503 | OH | H | H | H | H | H | $CH_2CH_2CH_2NHCO\text{-}NHSO_2C_6H_5$ | 508 |
| 7-504 | OH | H | H | H | H | H | a-104 | 502 |
| 7-505 | OH | H | H | H | H | H | a-105 | 550 |
| 7-506 | OH | H | H | H | H | H | a-106 | 550 |
| 7-507 | OH | H | H | H | H | H | a-107 | 531 |
| 7-508 | OH | H | H | H | H | H | a-108 | 518 |
| 7-509 | OH | H | H | H | H | H | a-109 | 530 |
| 7-510 | OH | H | H | H | H | H | $CH_2CH_2CH_2NHCO\text{-}NHCMe_2CH_2CMe_3$ | 480 |
| 7-511 | OH | H | H | H | H | H | a-110 | 493 |
| 7-512 | OH | H | H | H | H | H | $CH_2CH_2CH_2N$ (Me) CO-<br>$NHC_6H_5$ | 488 |
| 7-513 | OH | H | H | H | H | H | $CH_2CH_2CH_2N$ (Me) CO-N(Me)$C_6H_5$ | 502 |
| 7-514 | OH | H | H | H | H | H | $CH_2CH_2CH_2NHCONH\text{-}CH_2CH_2OH$ | 442 |
| 7-515 | OH | H | H | H | H | H | $CH_2CH_2CH_2NHCONH\text{-}CH_2CH_2OMe$ | 456 |
| 7-516 | OH | H | H | H | H | H | $CH_2CH_2CH_2NHCONH\text{-}CH_2CH_2NMe_2$ | 469 |
| 7-517 | OH | H | H | H | H | H | $CH_2CH_2CH_2NHCO$-N (Me) $CH_2CH_2OH$ | 456 |
| 7-518 | OH | H | H | H | H | H | $CH_2CH_2CH_2NHCO\text{-}N(CH_2CH_2OH)_2$ | 486 |
| 7-519 | OH | H | H | H | H | H | a-111 | 467 |
| 7-520 | OH | H | H | H | H | H | $CH_2CH_2CH_2NHCONMe_2$ | 426 |
| 7-521 | OH | H | H | H | H | H | $CH_2CH_2CH_2CH_2NHCO\text{-}NHMe$ | 426 |
| 7-522 | OH | H | H | H | H | H | $CH_2CH_2CH_2CH_2NHCO\text{-}NHCH_2Me$ | 440 |
| 7-523 | OH | H | H | H | H | H | $CH_2CH_2CH_2CH_2NHCO\text{-}NHCH_2CH_2Me$ | 454 |
| 7-524 | OH | H | H | H | H | H | $CH_2CH_2CH_2CH_2NHCO\text{-}NHCHMe_2$ | 454 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-525 | OH | H | H | H | H | H | $CH_2CH_2CH_2CH_2NHCO\text{-}NHCMe_3$ | 468 |
| 7-526 | OH | H | H | H | H | H | a-123 | 480 |
| 7-527 | OH | H | H | H | H | H | a-124 | 494 |
| 7-528 | OH | H | H | H | H | H | a-125 | 508 |
| 7-529 | OH | H | H | H | H | H | a-126 | 522 |
| 7-530 | OH | H | H | H | H | H | a-127 | 546 |
| 7-531 | OH | H | H | H | H | H | $CH_2CH_2CH_2CH_2NHCO\text{-}NHC_6H_5$ | 488 |
| 7-532 | OH | H | H | H | H | H | a-128 | 518 |
| 7-533 | OH | H | H | H | H | H | a-129 | 504 |
| 7-534 | OH | H | H | H | H | H | a-130 | 503 |
| 7-535 | OH | H | H | H | H | H | a-131 | 531 |
| 7-536 | OH | H | H | H | H | H | a-132 | 538 |
| 7-537 | OH | H | H | H | H | H | a-133 | 538 |
| 7-538 | OH | H | H | H | H | H | a-134 | 513 |
| 7-539 | OH | H | H | H | H | H | a-135 | 517 |
| 7-540 | OH | H | H | H | H | H | a-136 | 522 |
| 7-541 | OH | H | H | H | H | H | $CH_2CH_2CH_2CH_2NHCO\text{-}NHCOC_6H_5$ | 516 |
| 7-542 | OH | H | H | H | H | H | $CH_2CH_2CH_2CH_2NHCO\text{-}NHSO_2C_6H_5$ | 564 |
| 7-543 | OH | H | H | H | H | H | a-137 | 564 |
| 7-544 | OH | H | H | H | H | H | a-138 | 545 |
| 7-545 | OH | H | H | H | H | H | a-139 | 532 |
| 7-546 | OH | H | H | H | H | H | a-140 | 544 |
| 7-547 | OH | H | H | H | H | H | a-141 | 494 |
| 7-548 | OH | H | H | H | H | H | a-142 | 507 |
| 7-549 | OH | H | H | H | H | H | $CH_2CH_2CH_2CH_2NHCO\text{-}NHCMe_2CH_2CMe_3$ | 502 |
| 7-550 | OH | H | H | H | H | H | a-143 | 516 |
| 7-551 | OH | H | H | H | H | H | $CH_2CH_2CH_2CH_2N\ (Me)\text{-}CONHC_6H_5$ | 456 |
| 7-552 | OH | H | H | H | H | H | $CH_2CH_2CH_2CH_2N(Me)\text{-}CON(Me)C_6H_5$ | 470 |
| 7-553 | OH | H | H | H | H | H | $CH_2CH_2CH_2CH_2NHCO\text{-}NHCH_2CH_2OH$ | 483 |
| 7-554 | OH | H | H | H | H | H | $CH_2CH_2CH_2CH_2NHCO\text{-}NHCH_2CH_2OMe$ | 470 |
| 7-555 | OH | H | H | H | H | H | $CH_2CH_2CH_2CH_2NHCO\text{-}NHCH_2CH_2NMe_2$ | 500 |
| 7-556 | OH | H | H | H | H | H | $CH_2CH_2CH_2CH_2NH\text{-}CON\ (Me)\ CH_2CH_2OH$ | 481 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-557 | OH | H | H | H | H | H | $CH_2CH_2CH_2CH_2NHCO\text{-}N(CH_2CH_2OH)_2$ | 440 |
| 7-558 | OH | H | H | H | H | H | a-144 | 481 |
| 7-559 | OH | H | H | H | H | H | $CH_2CH_2CH_2CH_2NHCONMe_2$ | 440 |
| 7-560 | OH | H | H | H | H | H | $CH_2CH_2NHCSNHCH_2Me$ | 428 |
| 7-561 | OH | H | H | H | H | H | $CH_2CH_2NHCSNH\text{-}CH_2CH_2Me$ | 442 |
| 7-562 | OH | H | H | H | H | H | $CH_2CH_2NHCSNHCHMe_2$ | 442 |
| 7-563 | OH | H | H | H | H | H | $CH_2CH_2NHCSNHCMe_3$ | 456 |
| 7-564 | OH | H | H | H | H | H | a-112 | 402 |
| 7-565 | OH | H | H | H | H | H | $CH_2CH_2NHCSNHC_6H_5$ | 476 |
| 7-566 | OH | H | H | H | H | H | $CH_2CH_2NHCSNH\text{-}CH_2CH_2OH$ | 444 |
| 7-567 | OH | H | H | H | H | H | $CH_2CH_2NHCSNHCH_2CH_2O$ Me | 458 |
| 7-568 | OH | H | H | H | H | H | $CH_2CH_2NHCSNH\text{-}CH_2CH_2NMe_2$ | 471 |
| 7-569 | OH | H | H | H | H | H | $CH_2CH_2NHCSN(Me)\text{-}CH_2CH_2OH$ | 458 |
| 7-570 | OH | H | H | H | H | H | $CH_2CH_2NHCS\text{-}N(CH_2CH_2OH)_2$ | 488 |
| 7-571 | OH | H | H | H | H | H | a-113 | 469 |
| 7-572 | OH | H | H | H | H | H | $CH_2CH_2NHCSNMe_2$ | 428 |
| 7-573 | OH | H | H | H | H | H | $CH_2CH_2NHCOOMe$ | 399 |
| 7-574 | OH | H | H | H | H | H | $CH_2CH_2NHCOOCH_2Me$ | 413 |
| 7-575 | OH | H | H | H | H | H | $CH_2CH_2NHCOOCH_2CH_2Me$ | 427 |
| 7-576 | OH | H | H | H | H | H | $CH_2CH_2NHCOOCHMe_2$ | 427 |
| 7-577 | OH | H | H | H | H | H | a-114 | 467 |
| 7-578 | OH | H | H | H | H | H | $CH_2CH_2NHCOOC_6H_5$ | 461 |
| 7-579 | OH | H | H | H | H | H | $CH_2CH_2NHCOOCH_2CH_2OH$ | 429 |
| 7-580 | OH | H | H | H | H | H | $CH_2CH_2NHCOO\text{-}CH_2CH_2OMe$ | 443 |
| 7-581 | OH | H | H | H | H | H | $CH_2CH_2NHCOO\text{-}CH_2CH_2NMe_2$ | 456 |
| 7-582 | OH | H | H | H | H | H | a-115 | 422 |
| 7-583 | OH | H | H | H | H | H | a-116 | 422 |
| 7-584 | OH | H | H | H | H | H | a-9 | 395 |
| 7-585 | OH | H | H | H | H | H | a-117 | 409 |
| 7-586 | OH | H | H | H | H | H | a-118 | 423 |
| 7-587 | OH | H | H | H | H | H | a-8 | 340 |
| 7-588 | OH | H | H | H | H | H | $CH_2CH_2OCH_2CH_2OMe$ | 400 |
| 7-589 | OH | H | H | H | H | H | $CH(CH_2OH)_2$ | 371 |

(continued)

| Exp. N o. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-590 | OH | H | H | H | H | H | a-4 | 396 |
| 7-591 | H | Cl | H | H | H | H | H | 316 |
| 7-592 | H | Br | H | H | H | H | H | 360 |
| 7-593 | H | OH | H | H | H | H | H | 298 |
| 7-594 | H | Me | H | H | H | H | H | 296 |
| 7-595 | H | $CH=CH_2$ | H | H | H | H | H | 308 |
| 7-596 | H | $C{\equiv}CC_6H_5$ | H | H | H | H | H | 382 |
| 7-597 | H | OMe | H | H | H | H | H | 312 |
| 7-598 | H | $NH_2$ | H | H | H | H | H | 297 |
| 7-599 | H | NHMe | H | H | H | H | H | 311 |
| 7-600 | H | $NMe_2$ | H | H | H | H | H | 325 |
| 7-601 | H | $NHC_6H_5$ | H | H | H | H | H | 373 |
| 7-602 | H | H | Cl | H | H | H | H | 316 |
| 7-603 | H | H | Br | H | H | H | H | 360 |
| 7-604 | H | H | OH | H | H | H | H | 298 |
| 7-605 | H | H | Me | H | H | H | H | 296 |
| 7-606 | H | H | $CH=CH_2$ | H | H | H | H | 308 |
| 7-607 | H | H | $C{\equiv}CC_6H_5$ | H | H | H | H | 382 |
| 7-608 | H | H | OMe | H | H | H | H | 312 |
| 7-609 | H | H | $NH_2$ | H | H | H | H | 297 |
| 7-610 | H | H | NHMe | H | H | H | H | 311 |
| 7-611 | H | H | $NMe_2$ | H | H | H | H | 325 |
| 7-612 | H | H | $NHC_6H_5$ | H | H | H | H | 373 |
| 7-613 | H | H | H | Cl | H | H | H | 316 |
| 7-614 | H | H | H | Br | H | H | H | 360 |
| 7-615 | H | H | H | OH | H | H | H | 298 |
| 7-616 | H | H | H | Me | H | H | H | 296 |
| 7-617 | H | H | H | $CH=CH_2$ | H | H | H | 308 |
| 7-618 | H | H | H | $C{\equiv}CC_6H_5$ | H | H | H | 382 |
| 7-619 | H | H | H | OMe | H | H | H | 312 |
| 7-620 | H | H | H | $NH_2$ | H | H | H | 297 |
| 7-621 | H | H | H | NHMe | H | H | H | 311 |
| 7-622 | H | H | H | $NMe_2$ | H | H | H | 325 |

(continued)

| Exp. No. | $R^1$ | $R^5$ | $R^8$ | $R^7$ | $R^6$ | $A^6$ | $A^{61}$ | Mass ($MH^+$) |
|---|---|---|---|---|---|---|---|---|
| 7-623 | H | H | H | $NHC_6H_5$ | H | H | H | 373 |
| 7-624 | H | H | H | H | C1 | H | H | 316 |
| 7-625 | H | H | H | H | Br | H | H | 360 |
| 7-626 | H | H | H | H | OH | H | H | 298 |
| 7-627 | H | H | H | H | Me | H | H | 296 |
| 7-628 | H | H | H | H | $CH=CH_2$ | H | H | 308 |
| 7-629 | H | H | H | H | $C\equiv CC_6H_5$ | H | H | 382 |
| 7-630 | H | H | H | H | OMe | H | H | 312 |
| 7-631 | H | H | H | H | $NH_2$ | H | H | 297 |
| 7-632 | H | H | H | H | NHMe | H | H | 311 |
| 7-633 | H | H | H | H | $NMe_2$ | H | H | 325 |
| 7-634 | H | H | H | H | $NHC_6H_5$ | H | H | 373 |

Test Example 1: Inhibitory action on phosphorylation of myosin regulatory light chain

A volume of 50 to 100 ml of peripheral blood collected from healthy volunteers was centrifuged by using Mono-Poly separator solution (Dainippon Pharmaceutical) to prepare a neutrophil containing fraction. The neutrophils were washed with PBS(-) and resuspended in Hanks' Balanced Salt Solution (HBSS+, Gibco) to prepare a cell suspension ($8 \times 10^6$/ml). The cell suspension was diluted to $5 \times 10^6$/ml, introduced into Eppendorf tubes in a volume of 0.4 ml each, then 0.1 ml each of solutions of a test compound at various concentrations were added to the suspension and allowed to react at 25°C for 5 minutes. After the reaction, 0.1 ml of trichloroacetic acid solution was added to each reaction, the reaction mixture was gently shaken and centrifuged at 12,000 rpm (4°C, 5 minutes), and the supernatant was removed. Subsequently, 3 μl of 1 M Tris solution was added to the residue, the mixture was further mixed with 50 μl of extraction buffer (8 M urea, 0.02% 2-mercaptoethanol, 0.002% bromophenol blue) and left stand at room temperature for 1 hour. Then, the reaction mixture was loaded on a spin column (0.45 μm, Millipore) to remove the insoluble solids and a sample buffer for SDS polyacrylamide gel electrophoresis (25 mM, Tris-HCl pH 6.8, 2.5% 2-mercaptoethanol, 2% sodium dodecylsulfate, 5% sucrose, 0.002% bromophenol blue as final concentrations) was added, and 10 μ of each sample was subjected to electrophoresis.
The gel after the electrophoresis was blotted on a nitrocellulose membrane (BioRad), blocked with 5% skim milk, and reacted successively with antibodies pLC1 (Sakurada K. et al, Am. J. Physiol., 274, C1563-C1572 (1998)), which specifically recognize the phosphorylated myosin regulatory light chain, and donkey anti-mouse IgG (Chemicon) conjugated with horseradish peroxidase. The band of the phosphorylated myosin regulatory light chain was detected on a film by using ECL Plus Kit (Amersham Pharmacia Biotech). This band was subjected to quantification using a densitometer. By using this value, the inhibitory ratio (%) for phosphorylation of the myosin regulatory light chain was calculated by using the following equation.

**Phosphorylation inhibition ratio (%) = 1 - (Band intensity of phosphorylated myosin regulatory light chain with addition of the test compound /Band intensity of phosphorylated myosin regulatory light chain without addition of the test compound) x**

**100**

Further, the phosphorylation inhibition ratio was calculated with changing the concentrations of the test compound, and a compound concentration providing an inhibition ratio of 50% was obtained as $IC_{50}$. Table 8 mentioned below lists compounds having an $IC_{50}$ of 40 μM or lower ($IC_{50} \leqq 40$ μ M), and compounds having an $IC_{50}$ of 10 μM or lower ($IC_{50} \leqq 10$ μM) by referring to respective example numbers thereof. Sequential example numbers, for example, 1-1, 1-2, 1-3, ...1-100, are indicated as, for example, 1-1 ~ 1-100.
It was revealed that the compounds of the present invention listed in Table 8 inhibited phosphorylation of the myosin regulatory light chain.
1-(3-Fluoropiperidin-4-yl)-2,3-dihydro-1H-1,5-diazaphenalene, and 2-{N-[4-(2,3-dihydro-1H-1,5-diazaphenalen-1-yl)cyclohexyl]-N-methylamino}ethanol also inhibited phosphorylation of the myosin regulatory light chain at a concentration below 40 μ M.
[Table 8]

| $IC_{50} \leqq 40$ μM | | | | | | | |
|---|---|---|---|---|---|---|---|
| 1-1. | 1-4, | 1-10, | 1-17, | 1-22. | 1 - 41 ~ | 1-44, | 1-50. |
| 1-52, | 1-54, | 1-59 ~ | 1-65, | 1 - 67 ~ | 1-69, | 1-161, | 1-229, |
| 1-299, | 1 - 314, | 1-315, | 1-323, | 1-334, | 1-340, | 1-347, | 1-352. |
| 1-371 ~ | 1-374. | 1-380, | 1-382, | 1-384, | 1-389 ~ | 1-395, | 1-397 ~ |
| 1-399. | 1 - 491, | 1-559, | 1-629, | 1-644, | 1-645. | 2-1, | 2-2, |
| 2-4, | 2-6 ~ | 2-8, | 2 - 10 ~ | 2-13, | 2 - 17, | 2-18, | 2-20, |
| 2-22, | 2-24 ~ | 2-29, | 2-31 ~ | 2-33. | 2-35, | 2-36, | 2-40 ~ |

(continued)

| IC50≦40 μM | | | | | | | |
|---|---|---|---|---|---|---|---|
| 2-42, | 2-60, | 2-70, | 2-74, | 2-79, | 2-81, | 2-87, | 2-109, |
| 2-130, | 2-142, | 2-145, | 2-152, | 2-155~ | 2-157, | 2-159~ | 2-169, |
| 2-171, | 2-172, | 2-178, | 2-192, | 2-194, | 2-202, | 2-219, | 2-220, |
| 2-222, | 2-223, | 2-225, | 2-228, | 2-229, | 2-251~ | 2-259, | 2-261~ |
| 2-264, | 2-268, | 2-269, | 2-272~ | 2-275, | 2-277~ | 2-280, | 2-282~ |
| 2-286, | 2-289, | 2-290, | 2-292, | 2-294, | 2-295, | 2-297, | 2-298, |
| 2-300~ | 2-307, | 2-309~ | 2-313, | 2-315~ | 2-317, | 2-321, | 2-323, |
| 2-332, | 2-334, | 2-336~ | 2-338, | 2-340~ | 2-343, | 2-347, | 2-348, |
| 2-350, | 2-352, | 2-354~ | 2-359, | 2-361~ | 2-363, | 2-365, | 2-366, |
| 2-370~ | 2-372, | 2-390, | 2-400, | 2-404, | 2-409, | 2-411, | 2-417, |
| 2-439, | 2-460, | 2-472, | 2-475, | 2-482, | 2-485~ | 2-487, | 2-489~ |
| 2-502, | 2-508, | 2-522, | 2-524, | 2-532, | 2-549, | 2-550, | 2-552, |
| 2-553, | 2-555, | 2-558, | 2-559, | 2-581~ | 2-589, | 2-591~ | 2-594, |
| 2-598, | 2-599, | 2-602~ | 2-605, | 2-607~ | 2-610, | 2-612~ | 2-616, |
| 2-619, | 2-620, | 2-622, | 2-624, | 2-625, | 2-627, | 2-628, | 2-630~ |
| 2-637, | 2-639~ | 2-643, | 2-645~ | 2-647, | 2-691, | 3-1, | 3-4, |
| 3-10, | 3-17, | 3-18, | 3-41, | 3-42, | 3-60, | 3-67~ | 3-69, |
| 3-161. | 3-229. | 3-264. | 3-268. | 3-314. | 3-315. | 3-323. | 3-334. |
| 3-340. | 3-347. | 3-348. | 3 -371. | 3-372. | 3-390. | 3-397~ | 3-399. |
| 3-491. | 3-559. | 3-594. | 3-598. | 3-644. | 3-645. | 5-1. | 5-6. |
| 5-8~ | 5-11. | 5-13~ | 5-16. | 5-26. | 5-29. | 5-32. | 5-57. |
| 5-78. | 5-153. | 5-157. | 5-227. | 5-282. | 5-283. | 5-293. | 5-306. |
| 5-308. | 5-309~ | 5-311. | 5-313~ | 5-316. | 5-326. | 5-329. | 5-332. |
| 5-357. | 5-358. | 5-378. | 5-380~ | 5-383. | 5-395~ | 5-399. | 5-453. |
| 5-457. | 5-527. | 5-582. | 5-583. | 7-1. | 7-293 | | |
| I C50≦10 μM | | | | | | | |
| 1-1, | 1-4, | 1-41~ | 1-43, | 1-50, | 1-54, | 1-59, | 1-61, |
| 1-62, | 1-67~ | 1-69, | 1-161, | 1-299, | 1-314, | 1-323, | 1-334, |
| 1-371~ | 1-373, | 1-380, | 1-384, | 1-389, | 1-391, | 1-392, | 1-397~ |
| 1-399, | 1-491, | 1-629, | 1-644, | 2-1, | 2-10~ | 2-12, | 2-17, |
| 2-18, | 2-25, | 2-27, | 2-33, | 2-35, | 2-81, | 2-87, | 2-142, |
| 2-145, | 2-169, | 2-192, | 2-194, | 2-253, | 2-256, | 2-264, | 2-268, |
| 2-269, | 2-272, | 2-273, | 2-275, | 2-277, | 2-282, | 2-284, | 2-323, |
| 2-340~ | 2-342, | 2-347, | 2-348, | 2-355, | 2-357, | 2-363, | 2-365, |
| 2-411, | 2-417, | 2-472, | 2-475, | 2-499, | 2-522, | 2-524, | 2-583, |
| 2-586, | 2-594, | 2-598, | 2-599, | 2-602, | 2-603, | 2-605, | 2-607, |
| 2-612, | 2-614, | 3-4, | 3-41 | 3-42, | 3-60, | 3-67, | 3-69, |
| 3-161, | 3-314, | 3-315, | 3-334, | 3-371, | 3 -372, | 3-390, | 3-397, |
| 3-399, | 3-491, | 3-644, | 3-645, | 5-1, | 5-8, | 5-9. | 5-11, |
| 5-16, | 5-26, | 5-29, | 5-57, | 5-153, | 5-157, | 5-227, | 5-293, |
| 5-308, | 5-309, | 5-311, | 5-316, | 5-326, | 5-329, | 5-357, | 5-453, |
| 5-457, | 5-527, | 7-1, | 7-293 | | | | |

Test Example 2: Vasoconstriction inhibitory action

Rats (Wistar, 11-week old) were bleeded to death and laparotomized to take out the thoracic aorta. That aorta was cut into a ring of a length of about 3 mm in a conventional manner (Asano,T., et al., J. Pharmacol. Exp. Ther., 241,

pp.1033-1040 (1987)) and hung in 10-ml organ bath filled with Krebs-Hensright nutrient solution bubbled with a mixed gas of 95% $O_2$ and 5% $CO_2$. One end of the blood vessel was connected to an isometric transducer (FD Pickup TB-912T, Nihon Kohden) and applied with 2.5 g of resting tension, and constriction and relaxation reactions of the aorta were recorded.
The aorta was constricted with phenylephrine (1 μ M, Sigma) and then added with a test compound (1 μ M), and the vasoconstriction inhibitory action thereof was observed. The vasoconstriction inhibitory actions of the test compounds were calculated as relaxation ratios, which were based on the vasoconstriction with phenylephrine observed immediately before the addition of the test compounds taken as 100%.
Table 9 mentioned below lists the compounds of the present invention by referring to example numbers thereof. Sequential example numbers, for example, 1-1, 1-2, 1-3, ...1-100, are indicated as, for example, 1-1 ~ 1-100.
It was revealed that the compounds listed in Table 9 had vasoconstriction inhibitory action. It was also revealed that 1-(3-fluoropiperidin-4-yl)-2,3-dihydro-1H-1,5-diazaphenalene, and 2-{N-[4-(2,3-dihydro-1H-1,5-diazaphenalen-1-yl)cyclohexyl]-N-methylamino}ethanol also had vasoconstriction inhibitory action.
Thus, it was confirmed that the compounds of the present invention were useful as medicaments for prophylactic and/or therapeutic treatment of hypertension and the like.
[Table 9]

| | | | | | | | |
|---|---|---|---|---|---|---|---|
| 1-1, | 1-4, | 1-17, | 1-41~ | 1-44, | 1-50, | 1-52, | 1-54, |
| 1-59~ | 1-65 | 1-67~ | 1-69 | 1-161 | 1-229 | 1-299. | 1-314. |
| 1-315. | 1-323. | 1-334. | 1-347. | 1-371~ | 1-374. | 1-380. | 1-382. |
| 1-384. | 1-389~ | 1-395. | 1-397~ | 1-399. | 1-491. | 1-559. | 1-629. |
| 1-644. | 1-645. | 2-1. | 2-2. | 2-4. | 2-6~ | 2-8. | 2-10~ |
| 2-13, | 2-17. | 2-18. | 2-20. | 2-22. | 2-24~ | 2-29. | 2-31. |
| 2-33. | 2-35. | 2-36. | 2-40. | 2-41. | 2-70. | 2-79. | 2-81. |
| 2-87. | 2-109. | 2-130. | 2-142. | 2-145. | 2-152. | 2-155. | 2-156. |
| 2-159~ | 2-166. | 2-168. | 2-169. | 2-171. | 2-172. | 2-178. | 2-192. |
| 2-194. | 2-202. | 2-219. | 2-220. | 2-222. | 2-223. | 2-225. | 2-228. |
| 2-229, | 2-251~ | 2-259, | 2-261~ | 2-264 | 2-268 | 2-269 | 2-272~ |
| 2-275, | 2-277~ | 2-280, | 2-282~ | 2-286 | 2-289, | 2-290. | 2-292, |
| 2-294, | 2-295, | 2-297, | 2-301, | 2-304, | 2-306, | 2-307, | 2-310~ |
| 2-313, | 2-316, | 2-323, | 2-332, | 2-334, | 2-336~ | 2-338, | 2-340~ |
| 2-343, | 2-347, | 2-348, | 2-350, | 2-352, | 2-354~ | 2-359, | 2-361. |
| 2-363, | 2-365, | 2-366, | 2-370, | 2-371, | 2-400, | 2-409, | 2-411, |
| 2-417, | 2-439, | 2-460, | 2-472, | 2-475, | 2-482, | 2-485, | 2-486, |
| 2-489. | 2-490~ | 2-496, | 2-498, | 2-499, | 2-501, | 2-502, | 2-508, |
| 2-522, | 2-524, | 2-532, | 2-549, | 2-550, | 2-552, | 2-553, | 2-555, |
| 2-558, | 2-559, | 2-581~ | 2-589, | 2-591~ | 2-594, | 2-598, | 2-599, |
| 2-62~ | 2-605, | 2-607~ | 2-610, | 2-612~ | 2-616, | 2-619, | 2-620, |
| 2-622, | 2-624, | 2-625, | 2-627, | 2-631, | 2-634, | 2-636, | 2-637, |
| 2-640~ | 2-643, | 2-646, | 3-1, | 3-4, | 3-17, | 3-18, | 3-41, |
| 3-42, | 3-60, | 3-67~ | 3-69, | 3-161, | 3-229, | 3-314, | 3-315, |
| 3-323, | 3-334, | 3-347, | 3-348, | 3-371, | 3-372, | 3-390, | 3-397~ |
| 3-399, | 3-491, | 3-559, | 3-644, | 3-645, | 5-1, | 5-8, | 5-9~ |
| 5-11, | 5-13~ | 5-16, | 5-26, | 5-29, | 5-32, | 5-57, | 5-78, |
| 5-153, | 5-157, | 5-227, | 5-282, | 5-283, | 5-293, | 5-308, | 5-309~ |
| 5-311, | 5-313~ | 5-316, | 5-326, | 5-329, | 5-332, | 5-357, | 5-378, |
| 5-453, | 5-457, | 5-527, | 5-582, | 5-583, | 7-1, | 7-293 | |

Test Example 3: Action of suppressing respiratory tract constriction induced by antigen stimulation

Four-week old Hartley guinea pigs (male) were immunized by intraperitoneal administration of ovalbumin (Sigma, Grade V) in amounts of 1 mg for each animal on the day on which the experiment was started, 3 mg for each animal after 2 days, and 10 mg for each animal after 4 days.

Twelve to fourteen days after the final immunization, the ovalbumin-immunized guinea pigs were anesthetized by intraperitoneal administration of about 40 mg/kg of pentobarbital (Somnopentyl), and the tracheas were taken out. Subsequently, a cannula (SP-110, Natsume) was inserted into each trachea, and one end of the cannula was connected to an artificial respirator (Model-b83, Harvard). The aeration conditions were set at 6 ml per kilogram and 60 times per 1 minute. Further, a cannula for medicament administration (JMS wing needle 23G 3/4) was inserted into a hind leg vein. Myoblock (Organon Technica) was administered in an amount of 0.5 mg/kg from the cannula inserted into the hind leg vein to stop the spontaneous breathing, and after 2 or 3 minutes, 0.3 mg/kg of ovalbumin was administered to induce constriction of respiratory tract. The increase of airway resistance value 2 minutes after the induction (measurement apparatuses: pressure transducer TR-603T, respiratory amplifier AR-601G, and recorder RTA-3100, Nihon Kohden Corp.) was confirmed to be above 80 cm $H_2O$ or higher, and then, a solution of a test medicament was administered from the cannula inserted into the hind leg vein, and the airway resistance value was continuously measured for 15 minutes after the administration to determine the effect. Table 10 mentioned below lists the compounds of the present invention by referring to example numbers thereof, and sequential example numbers, for example, 1-1, 1-2, 1-3, ...1-100, are indicated as, for example, 1-1 ~ 1-100.

The compounds listed in Table 10 exhibited significant respiratory tract constriction suppressing action. 1-(3-Fluoropiperidin-4-yl)-2,3-dihydro-1H-1,5-diazaphenalene, and 2-{N-[4-(2,3-dihydro-1H-1,5-diazaphenalen-1-yl)cyclohexyl]-N-methylamino}ethanol also exhibited significant respiratory tract constriction suppressing action.

Further, the respiratory tract constriction suppression action of the test compounds was also evaluated by inhalation. Twelve to fourteen days after the final sensitization, 10 mg/kg of pyrilamine (Sigma), 5 mg/kg of indomethacin (Wako), or 0.1 mg/kg of propranolol (Sigma) was intraperitoneally administered. Then, 30 minutes after the administration, a 0.1% ovalbumin aqueous solution was inhaled by using a pressurization type nebulizer (PARI-IS2) to induce constriction of the respiratory tract. Ten minutes after the induction, 2 ml of a solution of a test compound prepared at various concentrations was filled in the aforementioned nebulizer, and administered by inhalation over 5 minutes. After the administration of pyrilamine and other drugs, airway resistance value was continuously measured to determine effectiveness. As a result, the compounds of the present invention listed in Table 10 significantly improved the constriction of respiratory tract. Further, 1-(3-fluoropiperidin-4-yl)-2,3-dihydro-1H-1,5-diazaphenalene, and 2-{N-[4-(2,3-dihydro-1H-1,5-diazaphenalen-1-yl)cyclohexyl]-N-methylamino}ethanol also significantly improved the constriction of respiratory tract.

Therefore, it was confirmed that the compounds of the present invention were useful as medicaments for prophylactic and/or therapeutic treatment of bronchial asthma and/or chronic obstructive pulmonary disease (COPD).

[Table 10]

| | | | | | | | |
|---|---|---|---|---|---|---|---|
| 1-1, | 1-4, | 1-10, | 1-17, | 1-41, | 1-44, | 1-50, | 1-54. |
| 1-61, | 1-68, | 1-69, | 1-299, | 1-323, | 1-334, | 1-340, | 1-347, |
| 1-371, | 1-374, | 1-380, | 1-384, | 1-391, | 1-398, | 1-399, | 1-629, |
| 2-1, | 2-2, | 2-7, | 2-8, | 2-10~ | 2-13, | 2-17, | 2-18, |
| 2-20, | 2-22, | 2-24 ~ | 2-28, | 2-35, | 2-36, | 2-79, | 2-81, |
| 2-87, | 2-109, | 2-130, | 2-142, | 2-145, | 2-152, | 2-155 ~ | 2-162, |
| 2-164 ~ | 2-166, | 2-168, | 2-169, | 2-171, | 2-172, | 2-178, | 2-192. |
| 2-194, | 2-219, | 2-220, | 2-222, | 2-223, | 2-225, | 2-228, | 2-229, |
| 2-251~ | 2-257, | 2-263, | 2-264, | 2-268, | 2-269, | 2-272, | 2-282, |
| 2-285, | 2-286, | 2-301, | 2-304 | 2-306, | 2-307, | 2-312, | 2-313, |
| 2-323, | 2-332, | 2-337. | 2-338, | 2-340~ | 2-343 | 2-347, | 2-348. |
| 2-350, | 2-352, | 2-354~ | 2-358, | 2-365, | 2-366, | 2-409, | 2-411, |
| 2-417, | 2-439, | 2-460, | 2-472, | 2-475, | 2-482, | 2-485- | 2-492, |
| 2-494~ | 2-496, | 2-498, | 2-499, | 2-501, | 2-502, | 2-508, | 2-522, |
| 2-524, | 2-549, | 2-550, | 2-552, | 2-553, | 2-555, | 2-558, | 2-559, |
| 2-581~ | 2-587, | 2-593, | 2-594, | 2-598, | 2-599, | 2-602, | 2-612, |
| 2-615, | 2-616, | 2-631, | 2-634, | 2-636, | 2-637, | 2-642, | 2-643, |
| 2-691, | 3-1, | 3-4, | 3-10, | 3-17, | 3-18, | 3-41, | 3-60, |
| 3-67, | 3-69, | 3-161, | 3-229, | 3-323, | 3-334, | 3-340, | 3-347, |
| 3-348, | 3-371, | 3-390, | 3-397, | 3-399, | 3-491, | 3-559, | 5-1, |
| 5-9, | 5-11, | 5-16, | 5-26, | 5-29, | 5-78, | 5-153, | 5-227, |
| 5-293, | 5-309, | 5-311, | 5-316, | 5-326, | 5-329, | 5-378, | 5-453, |

(continued)

| | | |
|---|---|---|
| 5-527, | 7-1, | 7-293 |

Test Example 4: Intraocular pressure reducing action

A Japanese white rabbit having a body weight of about 2 kg was placed in a positioner and naturalized for one week before the experiment. An ophthalmologic local anesthesant (Benoxil) was administered to the left eye, and then intraocular pressure was measured by using a tonometer (Classic 30, Solan). The initial value of the intraocular pressure was measured, then 50 $\mu$l of an aqueous solution of a test compound was dropped to the left eye at various concentrations, and the intraocular pressure was measured with passage of time.
Table 11 mentioned below lists the compounds of the present invention by referring to example numbers thereof, and sequential example numbers, for example, 1-1, 1-2, 1-3, ...1-100, are indicated as, for example, 1-1 ~ 1-100.
The compounds of the present invention listed in Table 11 exhibited significant intraocular pressure reducing action. 1-(3-Fluoropiperidin-4-yl)-2,3-dihydro-1H- 1,5-diazaphenalene, and 2-{N- [4-(2,3-dihydro-1H- 1,5-diazaphenalen-1-yl) cyclohexyl]-N-methylamino}ethanol also exhibited significant intraocular pressure reducing action.
Thus, it was confirmed that the compounds of the present invention were useful as medicaments for prophylactic and/or therapeutic treatment of glaucoma.
[Table 11]

| | | | | | | | |
|---|---|---|---|---|---|---|---|
| 1-1, | 1-4, | 1-10, | 1-17, | 1-44, | 1-50, | 1-5 4, | 1-61, |
| 1-299, | 1-323, | 1-334, | 1-340, | 1-347, | 1-374, | 1-380, | 1-384, |
| 1-391, | 1-629, | 2-1, | 2-2, | 2-7, | 2-8, | 2-10~ | 2-13, |
| 2-17, | 2-18, | 2-20, | 2-22, | 2-24~ | 2-28, | 2-35, | 2-36, |
| 2-79. | 2-81. | 2-87, | 2-109, | 2-130, | 2-142. | 2-145. | 2-152, |
| 2-155~ | 2-162, | 2-164~ | 2-166. | 2-168, | 2-169. | 2-171, | 2-172, |
| 2-178, | 2-192, | 2-194. | 2-219, | 2-220, | 2-222. | 2-223, | 2-225, |
| 2-228. | 2-229, | 2-251~ | 2-257, | 2-263, | 2-269, | 2-272, | 2-282, |
| 2-285. | 2-286, | 2-306. | 2-307. | 2-312. | 2-313. | 2-323, | 2-332, |
| 2-337. | 2-338, | 2-340~ | 2-343, | 2-347, | 2-348. | 2-350, | 2-354~ |
| 2-358. | 2-365. | 2-366. | 2-409, | 2-411. | 2-417. | 2-439, | 2-460, |
| 2-472. | 2-475. | 2-482. | 2-485~ | 2-492. | 2-4 94~ | 2-496, | 2-498~ |
| 2-502. | 2-508, | 2-522. | 2-524, | 2-549, | 2-550, | 2-552, | 2-553, |
| 2-555. | 2-558, | 2-559, | 2-581~ | 2-587, | 2-593, | 2-599, | 2-602. |
| 2-612, | 2-615, | 2-616, | 2-636, | 2-637, | 2-642, | 2-643, | 2-691, |
| 3-1, | 3-10, | 3-17, | 3-18, | 3-67, | 3-161, | 3-229, | 3-323, |
| 3-340, | 3-347, | 3-348, | 3-397, | 3-491. | 3-559. | 5-1, | 5-9, |
| 5-11, | 5-16, | 5-26, | 5-29. | 5-78, | 5-153, | 5-227, | 5-293, |
| 5-309, | 5-311, | 5-316, | 5-326, | 5-329, | 5-378, | 5-380, | 5-381, |
| 5-395, | 5-396, | 5-399, | 5-453, | 5-527, | 7-1 | 7-293 | |

Test Example 5: Neurite outgrowth action

From 18 day-old Sprague-Dawley rats, cerebral hippocampal neurons were prepared according to the method of Neuman et al. (Neuman, H.R. et al., J. Neurosci., 22, pp.854-862, 2002). The prepared neurons were cultured for 24 hours according to the method of Tanaka et al. (Tanaka, H. et al., J. Cell Biol., 158 (2), pp.321-329, 2002), then the medium was exchanged with fresh medium, and test medicaments of various concentrations or equivalent amounts of vehicle were added. Twenty-four hours after the addition of the medicaments, neurite length of each neuron was measured for the medicament-added group and the no-addition group, and compared. The neurite length was evaluated according to the method of Neuman et al. (Neuman, H.R. et al., J.Neurosci., 22, pp.854-862, 2002).
As a result, the compounds of the present invention listed in Table 9 induced significant neurite outgrowth. 1-(3-Fluoropiperidin-4-yl)-2,3-dihydro-1H-1,5-diazaphenalene, and 2-{N-[4-(2,3-dihydro-1H-1,5-diazaphenalen-1-yl)cyclohexyl]-N-methylamino}ethanol also induced significant neurite outgrowth.
Thus, it was confirmed that the compounds of the present invention were useful as medicaments for prophylactic and/or

therapeutic treatment of spinal cord injury.

Test Example 6: Neutrophil migration inhibitory action

Neutrophils were isolated from 50 to 100 ml of peripheral blood collected from healthy volunteers by the method described in Test Example 1 to obtain a cell suspension (8 x $10^6$/ml). Subsequently, solutions of a test compound at various concentrations were introduced into wells of a 96-well plate in a volume of 125 $\mu$ l per well, the cell suspension of an equivalent volume was added to it and the plate was preincubated at room temperature for 5 minutes. During the preincubation, FMLP (1 $\mu$M, Sigma) solution was added to the lower chamber to set Boyden Chamber, the preincubated cell suspension was added to the upper chamber in a volume of 200 $\mu$l per well, and the cells were allowed to migrate at 37°C under 5% carbon dioxide for 30 minutes. The filter after the migration was collected, and the non-migrated cells adhered to the surface that faced the upper chamber were carefully wiped off. Then, the migrated cells on the back surface were stained with DifQuick dye solution (International Reagents), washed with water and dried, and then absorbance was measured at 595 nm. The inhibition ratio against migration (%) of a test compound was calculated by using the following equation:

$$\text{Migration inhibition ratio (\%)} = (1 - \text{Absorbance of the group with addition of test compound} / \text{Absorbance of the group without addition of test compound}) \times 100$$

Further, the migration inhibitory ratio was calculated with changing the test compound concentration, and a compound concentration providing an inhibition ratio of 50% was obtained as $IC_{50}$. Table 12 mentioned below lists compounds exhibiting an $IC_{50}$ of 40 $\mu$ M or lower ($IC_{50} \leqq 40\ \mu M$), and compounds exhibiting an $IC_{50}$ of 10 $\mu$ M or lower ($IC_{50} \leqq 10\ \mu M$) by referring to respective example numbers thereof, and sequential example numbers, for example, 1-1, 1-2, 1-3, ...1-100, are indicated as, for example, 1-1 ~ 1-100.
The compounds of the present invention listed in Table 12 inhibited the migration of neutrophils. Further, 1-(3-fluoropiperidin-4-yl)-2,3-dihydro-1H-1,5-diazaphenalene, and 2-{N-[4-(2,3-dihydro-1H-1,5-diazaphenalen-1-yl)cyclohexyl]-N-methylamino}ethanol also inhibited the migration of neutrophils at a concentration of 40 $\mu$M or less.
Thus, it was confirmed that the compounds of the present invention were useful for prophylactic and/or therapeutic treatment of bronchial asthma and/or a chronic obstructive pulmonary disease and the like.
[Table 12]

| $IC_{50} \leqq 40\ \mu M$ | | | | | | | |
|---|---|---|---|---|---|---|---|
| 1-1, | 1-4, | 1-17, | 1-41~ | 1-44, | 1-50, | 1-52, | 1-54, |
| 1-59~ | 1-65 | 1-67~ | 1-69, | 1-161, | 1-229, | 1-299, | 1-314, |
| 1-315, | 1-323, | 1-334, | 1-347, | 1-371~ | 1-374, | 1-380, | 1-382. |
| 1-384, | 1-389~ | 1-395, | 1-397~ | 1-399, | 1-491, | 1-559, | 1-629. |
| 1-644, | 1-645, | 2-1, | 2-2, | 2-4, | 2-6~ | 2-8, | 2-10~ |
| 2-13, | 2-17, | 2-18, | 2-20, | 2-22, | 2-24~ | 2-29, | 2-31. |
| 2-33, | 2-35, | 2-36, | 2-40, | 2-41, | 2-70, | 2-79, | 2-81, |
| 2-87, | 2-109, | 2-130, | 2-142, | 2-145, | 2-152, | 2-155, | 2-156, |
| 2-159~ | 2-166, | 2-168, | 2-169, | 2-171, | 2-172, | 2-178, | 2-192, |
| 2-194, | 2-202, | 2-219, | 2-220, | 2-222, | 2-223, | 2-225, | 2-228, |
| 2-229, | 2-251~ | 2-259, | 2-261~ | 2-264, | 2-268, | 2-269, | 2-272~ |
| 2-275, | 2-277~ | 2-280, | 2-282~ | 2-286, | 2-289, | 2-290, | 2-292, |
| 2-294, | 2-295, | 2-297, | 2-301, | 2-304, | 2-306, | 2-307, | 2-310~ |
| 2-313, | 2-316, | 2-323, | 2-332, | 2-334, | 2-336~ | 2-338, | 2-340~ |
| 2-343, | 2-347, | 2-348, | 2-350, | 2-352, | 2-354~ | 2-359, | 2-361, |
| 2-363, | 2-365, | 2-366, | 2-370, | 2-371, | 2-400, | 2-409, | 2-411, |
| 2-417, | 2-439, | 2-460, | 2-472, | 2-475, | 2-482, | 2-485, | 2-486, |
| 2-489, | 2-490~ | 2-496, | 2-498~ | 2-502, | 2-508, | 2-522, | 2-524, |
| 2-532, | 2-549, | 2-550, | 2-552, | 2-553, | 2-555, | 2-558, | 2-559, |
| 2-5 81 ~ | 2-589, | 2-591 ~ | 2-594, | 2-598, | 2-599, | 2-602 ~ | 2-605, |

(continued)

| I $C_{50}$≦40 μM | | | | | | | |
|---|---|---|---|---|---|---|---|
| 2-607 ~ | 2-610, | 2-612 ~ | 2-616, | 2-619, | 2-620, | 2-622, | 2-624, |
| 2-625, | 2-627, | 2-631. | 2-634, | 2-636, | 2-637, | 2-640~ | 2-643, |
| 2-646, | 3-1, | 3-4, | 3-17, | 3-18, | 3-41, | 3-42, | 3-60, |
| 3-67~ | 3-69, | 3-161, | 3-229, | 3-314, | 3-315, | 3-323, | 3-334. |
| 3-347, | 3-348, | 3-371, | 3-372, | 3-390, | 3-397~ | 3-399, | 3-491, |
| 3-559. | 3-644. | 3-645. | 5-1, | 5-8. | 5-9~ | 5-11. | 5-13~ |
| 5-16. | 5-26. | 5-29. | 5-32. | 5-57. | 5-78. | 5-153. | 5-157. |
| 5-227. | 5-282. | 5-283. | 5-293. | 5-308, | 5-309~ | 5-311. | 5-313~ |
| 5-316. | 5-326. | 5-329. | 5-332. | 5-357. | 5-358. | 5-378. | 5-380. |
| 5-381. | 5-395~ | 5-399. | 5-453. | 5-457. | 5-527. | 5-582. | 5-583, |
| 7-1. | 7-293 | | | | | | |
| $IC_{50}$ ≦ 10 μM | | | | | | | |
| 1-1. | 1-4. | 1-41~ | 1-43. | 1-50, | 1-54. | 1-59. | 1-61. |
| 1-62. | 1-67~ | 1-69. | 1-161. | 1-299. | 1-314. | 1-323. | 1-334. |
| 1-371~ | 1-373, | 1-380, | 1-384, | 1-389, | 1-391, | 1-392, | 1-397~ |
| 1-399, | 1-491, | 1-629, | 1-644, | 2-1, | 2-10~ | 2-12, | 2-17, |
| 2-18, | 2-25, | 2-27, | 2-33, | 2-35, | 2-81, | 2-87, | 2-142, |
| 2-145, | 2-169, | 2-192, | 2-194, | 2-253, | 2-256, | 2-264, | 2-268, |
| 2-269, | 2-272, | 2-273, | 2-275, | 2-277, | 2-282, | 2-284, | 2-323, |
| 2-340~ | 2-342, | 2-347, | 2-348, | 2-355, | 2-357, | 2-363, | 2-365, |
| 2-411, | 2-417, | 2-472, | 2-475, | 2-499, | 2-522, | 2-524, | 2-583, |
| 2-586, | 2-594, | 2-598, | 2-599, | 2-602, | 2-603, | 2-605, | 2-607, |
| 2-612, | 2-614, | 3-4, | 3-41, | 3-42, | 3-60, | 3-67, | 3-69, |
| 3-161, | 3-314, | 3-315, | 3-334, | 3-371, | 3-372, | 3-390, | 3-397, |
| 3-399 | 3-491. | 3-6 44, | 3-645, | 5-1. | 5-8, | 5-9, | 5-11, |
| 5-16, | 5-26, | 5-29, | 5-57, | 5-153, | 5-157, | 5-227. | 5-293. |
| 5-308, | 5-309. | 5-311. | 5-316. | 5-326, | 5-329. | 5-357, | 5-453, |
| 5-457, | 5-527, | 7-1, | 7-293 | | | | |

Test Example 7: Chronic respiratory tract inflammation suppressing action

According to Henderson, W.R., et al., Am. J. Respir. Cric. Care Med., 165(1), 108-116 (2002), suppressing action on bronchial inflammation was confirmed. BALB/c female mice (7-week old) were used for the test, which were grouped into a negative control group, a positive control group, and a drug administration group. The mice of each group were intraperitoneally administered with ovalbumin (OVA, 100 ng, Sigma) and 1 mg of aluminum hydroxide for initial immunization, and after 2 weeks, they are subcutaneously administered with 10 μ g of OVA as additional immunization. In the following experiments, test compounds are used as a solution obtained by dissolving each compound in water containing 0.5% carboxymethylcellulose.
For the drug administration group, a procedure of oral administering a solution of test compound once a day and inhalation of 1 or 2% OVA for 10 minutes 1 hour later to induce respiratory tract inflammation was repeated for 5 days from 1 week after the additional immunization. At the same time, for the positive control group, the same procedure was repeated for 5 days by using water containing 0.5% carboxymethylcellulose instead of the solution of test compound. Further, for the negative control group, the same procedure was repeated for 5 days by using water containing 0.5% carboxymethylcellulose instead of the solution of test compound, and physiological saline instead of 1 or 2% OVA..
Twenty four hours after the final OVA inhalation or inhalation of physiological saline, pulmonary cavities of the test animals were washed with physiological saline containing 1% BSA, and the total infiltrated white blood cells (WBC) were counted.
As a result, the compounds of the present invention significantly improved the pathological conditions.
Further, BALB/c female mice were grouped into a negative control group, a positive control group, and a drug administration group, and subjected to the initial immunization, and the additional immunization. Then, for the drug administration group, a procedure of administering a solution of test compound once a day by using an infusion pump

immediately after the induction of respiratory tract inflammation was repeated for 5 days from 1 week after the additional immunization. For the positive control group, the same procedure was repeated for 5 days by using 0.5% carboxymethylcellulose instead of the test compound after the induction of respiratory tract inflammation. Further, for the negative control group, the same procedure as mentioned above was repeated for 5 days by using physiological saline instead of OVA induction, and 0.5% carboxymethylcellulose instead of the test compound.
Twenty four hours after the final drug administration, pulmonary cavities of the test animals were washed with physiological saline, and the total infiltrated white blood cells (WBC) were counted. As a result, the compounds of the present invention listed in Table 10 mentioned above significantly improved the pathological conditions. Further, 1-(3-fluoropiperidin-4-yl)-2,3-dihydro-1H-1,5-diazaphenalene, and 2-{N-[4-(2,3-dihydro-1H-1,5-diazaphenalen-1-yl)cyclohexyl]-N-methylamino}ethanol also significantly improved the pathological conditions.
Thus, it was confirmed that the compounds of the present invention were useful as medicaments for prophylactic and/or therapeutic treatment of bronchial asthma, and/or chronic obstructive pulmonary disease. Further, no abnormality was observed in the test animals (mice) in a 5 consecutive day administration test, and the administration test using an infusion pump with these compounds, and thus they were safe compounds.

Test Example 8 : Acute respiratory tract inflammation suppressing action

According to Gonzales de Moraes, VL., et al., Br. J. Pharmacol., 123, pp.631-6, 1998, suppressing action on pulmonary inflammation was studied. BALB/c female mice (7-week old) were used for the test, as the compound administration group and positive control group, each consisting of 7 mice, as well as the negative control group consisting of 5 mice. For induction of inflammation, a 0.03% physiological saline solution of a lipopolysaccharide (LPS, a mixture derived from *Escherichia coli* O55 and B5 strains, Sigma) was used. A test compound was dissolved in physiological saline to prepare solutions of various concentrations. The test animals were first inhaled with the aforementioned lipopolysaccharide solution for 10 minutes by using a pressurization type nebulizer (PARI-IS2) to induce inflammation. Then, 1 minute after the completion of the inhalation of the lipopolysaccharide solution, the animals were administered with a solution of test compound at various concentrations over 10 minutes by inhalation using the aforementioned nebulizer. The mice of the positive control group were administered with the same volume of physiological saline instead of the test compound solution over 10 minutes by inhalation. The mice of the negative control group were inhaled with physiological saline instead of the lipopolysaccharide solution by inhalation, and then administered with the same volume of physiological saline instead of the test compound solution by inhalation. Three hours after the administration of the test compound, pulmonary cavities of the test animals were washed with physiological saline, and the total infiltrated leucocyte (WBC) number was counted. As a result, the compounds of the present invention listed in Table 10 mentioned above significantly improved the pathological condition. Further, 1-(3-fluoropiperidin-4-yl)-2,3-dihydro-1H-1,5-diazaphenalene, and 2-{N-[4-(2,3-dihydro-1H-1,5-diazaphenalen-1-yl)cyclohexyl]-N-methylamino}ethanol also significantly improved the pathological conditions.
Further, after starting the administration of the test compound solution by using an infusion pump, the mice of the drug administration group was inhaled with the lipopolysaccharide solution over 10 minutes by the aforementioned method to induce inflammation. At the same time, the mice of the positive control group were administered with the same volume of physiological saline instead of the test compound, and inflammation was induced by the aforementioned method. For the mice of the negative control group, the same procedure was performed by using physiological saline instead of the test compound, and physiological saline instead of the lipopolysaccharide solution. Three hours after the induction of respiratory tract inflammation, pulmonary cavities of the test animals were washed with physiological saline, and the total infiltrated leucocyte number (WBC) was counted. As a result, the compounds of the present invention listed in Table 10 mentioned above significantly improved the pathological condition. Further, 1-(3-fluoropiperidin-4-yl)-2,3-dihydro-1H-1,5-diazaphenalene, and 2-{N-[4-(2,3-dihydro-1H-1,5-diazaphenalen-1-yl)cyclohexyl]-N-methylamino}ethanol also significantly improved the pathological conditions.
Thus, it was confirmed that the compounds of the present invention were useful as medicaments for prophylactic and/or therapeutic treatment of bronchial asthma, and/or chronic obstructive pulmonary disease.

Test Example 9: Action on increase of intracellular calcium concentration

According to the method described in Test Example 1, a neutrophil containing fraction was prepared. Fura2-AM (Sigma) at a final concentration of 3 μM was added to the human neutrophil fraction and the mixture was incubated at 37°C for 1 hour. After centrifugation (250 g for 5 minutes), the supernatant was discarded, and the neutrophils were resuspended in Hanks' Balanced Salt Solution (HBSS , Gibco) to prepare a cell suspension (8 x $10^6$/ml) for measurement of intracellular calcium concentration. The cell suspension for measurement of intracellular calcium concentration was left stand at room temperature for 30 minutes. Then, 490 μl of the cell suspension for measurement of intracellular calcium concentration was placed in a cuvette, 10 μl of calcium chloride solution at a final concentration of 1 μM was

added to it and the cuvette was set in an intracellular calcium concentration analyzer (CAF110, Nippon Bunko). fMLP (Sigma) solution at a final concentration of 1 μM was added to the cell suspension, and F340 and F380, which are fluorescence intensity at 340 nm and 380 nm, respectively, were measured to obtain an R value (F340/F380) as an index of the intracellular calcium concentration. A test compound (1 μ M) was added 3 minutes before the addition of fMLP, and the action on the intracellular calcium concentration was observed. The ratios of the maximum R value obtained with addition of each test compound relative to the maximum R value obtained without addition of test compound and taken as 100% were obtained.
It was revealed that the compounds of the present invention had almost no effect on the increase of the intracellular calcium concentration caused by the fMLP stimulation.

Test Example 10: Action on myosin light chain kinase (MLCK) activity

A myosin light chain kinase (MLCK) was purified from chicken gizzard smooth muscle by a conventional method (Yoshida, M., et al., J. Biochem., 99, 1027-1036 (1986)). The myosin regulatory light chain as a substrate was purified from the chicken gizzard smooth muscle by a conventional method (Grand, R. J., et al., Biochem. J., 211, 267-272 (1983)). The MLCK activity was measured by ELISA (Sakurada, K., et al., J. Biochem., 115, 18-21 (1994)) using anti-phosphorylated myosin regulatory light chain-recognizing antibodies (Sakurada, K., et al., Am. J. Physiol., 274, C1563-C1572, 1998). The myosin regulatory light chain was diluted in phosphate-buffered saline (PBS, Sigma) to a concentration of 5.0 g/ml, added to 96-well Immunoplate (Nunc) in a volume of 100 μl per well and left stand overnight at 4°C. Each well was washed with PBS, and 25 mM Tris/HCl buffer containing 100 μ M ATP, 3 mM $MgCl_2$, 1 mM $CaCl_2$, 100 ng/ml of calmodulin (Sigma) and 100 ng/ml of MLCK (pH 7.4, Buffer A) was added to each well and incubated at 30°C for 10 minutes. In a volume of 100 μ l each of 20% aqueous phosphoric acid solution was added, to each well to terminate the enzymatic reaction. Each well was washed with 25 mM Tris/HCl buffer (TTBS) containing 0.1% Tween 20, and then 100 μ l of antibodies specifically recognizing phosphorylated myosin regulatory light chain (Sakurada, K., et al., Am. J. Physiol., 274, C1563-C1572, 1998) was added to each well and incubated at room temperature for 90 minutes.
Each well was washed with TTBS, and then 100 μl of the HRP-labeled anti-mouse IgG antibodies (Bio-Rad) were added to each well and incubated at room temperature for 90 minutes. Each well was washed with TTBS, and then 25 mM citrate buffer (pH 5.0) containing orthophenylenediamine (Sigma) as a substrate of HRP and aqueous hydrogen peroxide (0.03%) was added in a volume of 100 μl per well and incubated at room temperature for 5 minutes. 50 μl of 4 N sulfuric acid was added to each well to terminate the reaction, and then absorbance was measured by using an immunoplate reader (Bio-Rad). The MLCK activity inhibition ratio was calculated by adding the test compound to Buffer A at various concentrations to obtain a compound concentration providing an inhibition ratio of 50% as $IC_{50}$.
It was revealed that the compounds of the present invention had almost no inhibitory effect on MLCK.

Test Example 11: Tracheal relaxation action

Hartley guinea pigs (male, 300 to 600 g) were bled to death, and each trachea was extracted. The trachea was first cut on the pair of smooth muscles side along the direction parallel to the smooth muscles, then cut along the cartilage to prepare a sample in the form of a strip having a width of about 2 to 3 mm. After removing vascular endothelium, the sample was suspended with a static load of 1 kg in a Magnus bath containing Krebs-Henseleit solution of 37°C bubbled with a mixed gas of 95% $O_2$ and 5% $CO_2$. Generated tension was recorded on a recorder via an isometric transducer (FD Pickup TB-612T, Nihon Kohden).
After suspending the sample, and waiting for 1 hour or more until a stable ground line was obtainable, a constriction substance was administered, and then the experiment was started after reproducible constriction was obtainable. During this procedure, the solution in the Magnus bath was changed every about 30 minutes. The sample was constricted with 10 μ M histamine, and after the time point that the generated tension was stabilized, a test drug was cumulatively administered at various concentrations to examine change in tension of the sample. After completion of the measurement, 10 μ M salbutamol was administered to obtain a tension of the maximum relaxation, and a drug concentration inducing relaxation comparable to the maximum relaxation was determined for each test compound.
As a result, the compounds of the present invention listed in Table 9 mentioned above significantly suppressed constriction of the trachea sample. Further, 1-(3-fluoropiperidin-4-yl)-2,3-dihydro-1H-1,5-diazaphenalene, and 2-{N-[4-(2,3-dihydro-1H-1,5-diazaphenalen-1-yl)cyclohexyl]N-methylamino}ethanol also significantly suppressed constriction of the trachea sample.
Thus, it was confirmed that the compounds of the present invention were useful as medicaments for prophylactic and/or therapeutic treatment of bronchial asthma and/or chronic obstructive pulmonary disease.

Test Example 12: Action of suppressing respiratory tract constriction induced by constriction elicitor

Hartley guinea pigs of 4-week old (male) were anesthetized by intraperitoneal administration of about 45 mg/kg of pentobarbital (Somnopentyl), and the tracheas were taken out. Subsequently, a cannula (SP-110, Natsume) was inserted into each trachea, and one end of the cannula was connected to an artificial respirator (Model-b83, Harvard). The aeration conditions were set at 6 ml per kilogram and 60 times per 1 minute. Further, a cannula for medicament administration (JMS wing needle 23G 3/4) was inserted into a hind leg vein. Myoblock (Organon Technica) was administered in an amount of 0.5 mg/kg from the cannula inserted into the hind leg vein to stop the spontaneous breathing, and after 5 minutes, 50 to 60 μg/kg of histamine was administered to induce constriction of respiratory tract. A histamine level inducing constriction corresponding to a delta value of 40 to 60 cm $H_2O$ as an indicator of airway resistance was determined for each individual animal (measurement apparatuses: pressure transducer TR-603T, respiratory amplifier: AR-601G, and recorder: RTA-3100, Nihon Kohden Corp.).
Then, 100 μ 1 of a test compound solution was sprayed with various concentrations onto the respiratory tract using Microsprayer™ (IA-1B, Penn Century), and after 2 minutes, histamine was sprayed at the concentration determined above to include constriction of the respiratory tract. The airway resistance value was continuously measured after the induction to determine the effect of each test medicament. As a result, the compounds of the present invention listed in Table 10 mentioned above significantly improved constriction of respiratory tract. Further, 1-(3-fluoropiperidin-4-yl)-2,3-dihydro-1H-1,5-diazaphenalene, and 2-{N-[4-(2,3-dihydro-1H-1,5-diazaphenalen-1-yl)cyclohexyl]-N-methylamino}ethanol also significantly suppressed the constriction of respiratory tract.
Thus, it was confirmed that the compounds of the present invention were useful as medicaments for prophylactic and/or therapeutic treatment of bronchial asthma and/or chronic obstructive pulmonary disease.

Test Example 13: Intraocular pressure reduction enhancing action

For evaluation of intraocular pressure reduction enhancing action of a combination of the compound of the present invention and a jointly-used drug other than latanoprost, rabbits were used as experimental animals, and for evaluation of intraocular pressure reduction enhancing action of a combination of the compound of the present invention and latanoprost, monkeys were used.
Naturalization of the rabbits, method for measuring intraocular pressure, and preparation of liquid for eye drop were according to those of Test Example 4. The rabbits were divided into a control group, instillation group for test compound solution alone (single active ingredient preparation group 1), instillation group for jointly-used drug alone (single active ingredient preparation group 2), and instillation group for jointly-used drug and test compound solution (combination use group). After initial values of intraocular pressure of the rabbits in each group were measured, 50 μL of a test compound solution in physiological saline at various concentrations was dropped to the eyes of the rabbits of the single active ingredient preparation group 1 and the combination use group, and physiological saline of the same volume was dropped to the eyes of the rabbits of the control group and the single active ingredient preparation group 2. Then, 5 minutes after the instillation of the test compound solution or physiological saline, 50 μL of a jointly-used drug was dropped to the eyes of the rabbits of the single active ingredient preparation group 2 and the combination use group, and 50 μL of physiological saline was dropped to the eyes of the rabbits of the control group and the single active ingredient preparation group 1. Then, the intraocular pressure was measured in time course for each group. As the jointly-used drug, isopropylunoprostone (trade name: Rescula, Fujisawa Pharmaceutical), dorzolamide hydrochloride (trade name: Trusopt, Banyu Pharmaceutical), bunazosin hydrochloride (trade name: Detantol, Santen Pharmaceutical), and timolol maleate (trade name: Timoptol, Santen Pharmaceutical) were used as marketed preparations, per se.
As a result, the compounds of the present invention listed in Table 11 mentioned above exhibited the intraocular pressure reducing action for the single active ingredient preparation group 1 in a degree exceeding that observed for the control group, as in Test Example 4, and further exhibited the intraocular pressure reducing action for the combination use group in a degree exceeding both of those observed for the single active ingredient preparation group 1 and the single active ingredient preparation group 2. The degree of the action was comparable to or more than the sum of the degrees of the intraocular pressure reducing actions observed for the single active ingredient preparation group 1 and the single active ingredient preparation group 2, and thus enhancement of the intraocular pressure reducing action was confirmed. Enhancement of the intraocular pressure reducing action was also confirmed for 1-(3-fluoropiperidin-4-yl)-2,3-dihydro-1H-1,5-diazaphenalene, and 2-{N-[4-(2,3-dihydro-1H-1,5-diazaphenalen-1-yl)cyclohexyl]-N-methylamino}ethanol.
Moreover, when any of the compositions A, B, and C described in Formulation Example 1, the compositions E, F, G, and H described in Formulation Example 2, or a composition comprising a combination of any of the compounds of the examples mentioned above and any of the jointly-used drugs was dropped instead of successively dropping a solution of a compound of the present invention and a jointly-used drug as the combination use group, the combination use group exhibited higher intraocular pressure reducing action than both of those observed in the single active ingredient preparation group 1 and the single active ingredient preparation group 2.

In the following experiment, the commercially available medicament, Xalatan (Pfizer, Inc.), was used as latanoprost.

Naturalized monkeys were divided into a control group, instillation group for test compound solution alone (single active ingredient preparation group 1), instillation group for Xalatan alone (single active ingredient preparation group 2), and instillation group for jointly-used drug and test compound solution (combination use group).

After initial values of intraocular pressure of the monkeys in each group were measured, 20 μ L of a test compound solution in physiological saline at various concentrations was dropped to the eyes of the monkeys of the single active ingredient preparation group 1 and the combination use group, and physiological saline of the same volume was dropped to the eyes of the monkeys of the control group and the single active ingredient preparation group 2. Then, 5 minutes after the instillation of the test compound solution or physiological saline, 20 μL of Xalatan was administered to the eyes of the monkeys of the single active ingredient preparation group 2 and the combination use group, and 20 μ L of physiological saline was dropped to the eyes of the monkeys of the control group and the single active ingredient preparation group 1. Then the intraocular pressure was measured in time course for each group.

As a result, the compounds of the examples mentioned above exhibited the intraocular pressure reducing action for the single active ingredient preparation group 1 in a degree exceeding that observed for the control group, and further exhibited the intraocular pressure reducing action for the combination use group in a degree exceeding both of those observed for the single active ingredient preparation group 1 and the single active ingredient preparation group 2. The degree of the action was comparable to or more than the sum of the degrees of the intraocular pressure reducing actions observed for the single active ingredient preparation group 1 and the single active ingredient preparation group 2, and thus enhancement of the intraocular pressure reducing action was confirmed.

Moreover, when any of the composition D described in Formulation Example 1, the composition I described in Formulation Example 2, or a composition comprising a combination of any of the compounds of the examples mentioned above and any of the jointly-used drugs was dropped instead of successively dropping a solution of a compound of the present invention and a jointly-used drug as the combination use group, the combination use group exhibited higher intraocular pressure reducing action than both of those observed in the single active ingredient preparation group 1 and the single active ingredient preparation group 2.

Thus, it was revealed that a medicament comprising a combination of a compound of the present invention and a jointly-used drug is useful as a medicament for prophylactic and/or therapeutic treatment of glaucoma.

Formulation Example 1

Preparation examples of eye drop compositions in which the compound of Example 5-11 and a jointly-used drug are dissolved in physiological saline in combination are mentioned below. However, the present invention is not limited to these examples, and compositions based on a combination with a jointly-used drug can be prepared for the compounds other than the compound of Example 5-11. Furthermore, by suitably changing the type and amount of the jointly-used drug, the type and amount of additives and the like, an eye drop based on a desired combination having desired concentrations can be prepared.

Physiological saline containing 0.1% by weight of the compound of Example 5-11, 0.12% by weight of isopropylunoprostone (Funakoshi), and 0.005% by weight of benzalkonium chloride is designated as composition A.

Physiological saline containing 0.1% by weight of the compound of Example 5-11, 1.0% by weight of dorzolamide hydrochloride (HYDRUS CHEMICAL), and 0.005% by weight of benzalkonium chloride is designated as composition B.

Physiological saline containing 0.1% by weight of the compound of Example 5-11, 0.25% by weight of timolol maleate (Sigma-Aldrich), and 0.005% by weight of benzalkonium chloride is designated as composition C.

Physiological saline containing 0.1% by weight of the compound of Example 5-11, 0.005% by weight of latanoprost (Funakoshi), and 0.005% by weight of benzalkonium chloride is designated as composition D.

Formulation Example 2

Preparation examples of eye drop compositions based on a combination of the compound of Example 5-11 and a jointly-used drug in which the compound of Example 5-11 is dissolved in a marketed eye drop are mentioned below. However, the present invention is not limited to these examples, and compositions based on a combination with a jointly-used drug can be prepared for the compounds other than the compound of Example 5-11. Furthermore, by suitably changing the type and amount of the jointly-used drug, the type and amount of additives and the like, an eye drop having a desired combination with desired concentrations can be prepared.

A solution in which the compound of Example 5-11 is dissolved at a concentration of 0.1 % by weight in an eye drop, Rescula (active ingredient: isopropylunoprostone, Fujisawa Pharmaceutical), is designated as composition E.

A solution in which the compound of Example 5-11 is dissolved at a concentration of 0.1 % by weight in an eye drop, Trusopt (active ingredient: dorzolamide hydrochloride, Banyu Pharmaceutical), is designated as composition F.

A solution in which the compound of Example 5-11 is dissolved at a concentration of 0.1 % by weight in an eye drop, Detantol (active ingredient: bunazosin hydrochloride, Santen Pharmaceutical), is designated as composition G.
A solution in which the compound of Example 5-11 is dissolved at a concentration of 0.1 % by weight in an eye drop, Timoptol (active ingredient: timolol maleate, Santen Pharmaceutical), is designated as composition H.
A solution in which the compound of Example 5-11 is dissolved at a concentration of 0.1 % by weight in an eye drop, Xalatan (active ingredient: latanoprost, Pfizer), is designated as composition I.

[Industrial Applicability]

The compounds of the present invention represented by the formula (1) have inhibitory activity against the myosin regulatory light chain phosphorylation, and are useful as active ingredients of medicaments for prophylactic and/or therapeutic treatment of, for example, diseases relating to contraction of various cells, diseases relating to morphological change of various cells, diseases relating to migration of various cells, diseases relating to release of various cells, diseases relating to aggregation of various cells, diseases relating to apoptosis of various cells, diseases relating to abnormal gene expression in various cells and the like.

## Claims

**1.** A compound represented by the following formula (1) or a salt thereof:

[Formula 1]

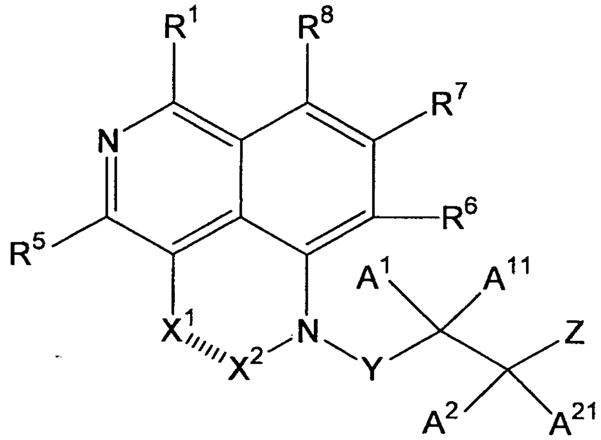

(1)

[wherein $R^1$, $R^5$, $R^6$, $R^7$, and $R^8$ independently represent hydrogen atom, a halogen atom, hydroxyl group, an alkyl group, an alkenyl group, an alkynyl group, an alkoxy group, amino group, an alkylamino group, or an arylamino group;
$X^1 \cdots X^2$ represents $-CH(R^2)-CH(R^3)-$, $-CH(R^2)-CH(R^3)-CH(R^4)-$, $-C(R^2)=C(R^3)-$, or $-C(R^2)=C(R^3)-CH(R^4)-$;
$R^2$, $R^3$, and $R^4$ independently represent hydrogen atom, or an alkyl group;
$A^1$, $A^{11}$, $A^2$, and $A^{21}$ independently represent hydrogen atom, or an alkyl group;
Y represents $-CH(A^3)-$, $-CH(A^3)-C(A^4)(A^{41})-$, $-CH(A^3)-C(A^4)(A^{41})-C(A^5)(A^{51})-$, or a single bond;
$A^3$, $A^4$, $A^{41}$, $A^5$, and $A^{51}$ independently represent hydrogen atom, or an alkyl group;
Z represents hydroxyl group, or $-N(A^6)(A^{61})$;
$A^6$ represents hydrogen atom, or an alkyl group, $A^{61}$ represents hydrogen atom, an alkyl group, an alkyl group substituted with an aryl group, an alkyl group substituted with an aromatic heterocyclic group, an alkyl group substituted with carboxyl group, an alkyl group substituted with cyano group, an alkyl group substituted with hydroxyl group, an alkyl group substituted with an alkoxy group, an alkyl group substituted with a substituted alkoxy group, an alkyl group substituted with amino group, an alkyl group substituted with an alkylamino group, an alkyl group substituted with aminocarbonyl group, an alkyl group substituted with an alkylaminocarbonyloxy group, an alkyl group substituted with a cycloalkylaminocarbonyloxy group, an alkyl group substituted with an arylaminocarbonyloxy group, an alkyl group substituted with mercapto group, an alkyl group substituted with an alkylthio group, an alkyl group substituted with an acylthio group, a cycloalkyl group, a substituted cycloalkyl group, carbamimidoyl group, an alkylcarbonyl group, an arylcarbonyl group, a non-aromatic heterocyclic group, a substituted non-aromatic heterocyclic group, or an alkyl group of which end is substituted with $N(A^7)(-X^3-A^{71})$, $-X^3-$ represents carbonyl group, $-SO_2-$, -C(=O)-O-, $-C(=O)-N(A^8)-$, or $-C(=S)-N(A^8)-$, $A^7$ represents hydrogen atom, an alkyl group, an alkyl group substituted with hydroxyl group, an alkyl group substituted with amino group, an alkyl group substituted with an alkylamino group, an alkyl group substituted with aminocarbonyl group, an alkyl group substituted with an acylamino group, or an alkyl group substituted with cyano group, $A^{71}$ and $A^8$ independently represent hydrogen atom, an alkyl group, an alkyl group substituted with an aryl group, an alkyl group substituted with a heterocyclic group, an alkyl group substituted with hydroxyl group, an alkyl group substituted with amino group, an alkyl group substituted with an alkylamino group, an alkyl group substituted with an alkoxy group, an alkyl group substituted with an acylamino group, a cycloalkyl group, an aryl group, or an aromatic heterocyclic group, or $A^7$ and $A^{71}$ may combine together to become an alkylene group, or an alkylene group substituted with an alkyl group to form a ring, or $A^{71}$ and $A^8$ may combine together to become an alkylene group, -alkylene-O-alkylene-, -alkylene-NH-alkylene-, or -alkylene-N(alkyl)-alkylene- to form a ring (wherein said alkylene moiety may be substituted with an alkyl group) ; and groups in each of one or more combinations selected from the group consisting of combinations of $A^6$ and $A^3$, $A^6$ and $A^4$, $A^6$ and $A^1$, $A^6$ and $A^2$, $A^2$ and $A^3$, $A^2$ and $A^4$, $A^6$ and $A^5$, $A^3$ and $A^1$, and $A^5$ and $A^1$ may bind to each other to form a 5- or 6-membered ring].

**2.** The compound or a salt thereof according to claim 1, wherein a compound represented by the formula (1) mentioned in claim 1 wherein:
$R^1$ is hydrogen atom, chlorine atom, or hydroxyl group,
$R^5$, $R^6$, $R^7$, and $R^8$ are hydrogen atoms, and
$A^{61}$ is hydrogen atom, an alkyl group, an alkyl group substituted with an aryl group, an alkyl group substituted with carboxyl group, an alkyl group substituted with cyano group, an alkyl group substituted with hydroxyl group, an alkyl group substituted with an alkoxy group, an alkyl group substituted with amino group, an alkyl group substituted with an alkylamino group, an alkyl group substituted with aminocarbonyl group, or an alkyl group of which end is substituted with $N(A^7)(-X^3-A^{71})$ (where $-X^3-$ is carbonyl group, and $A^{71}$ is an alkyl group, or an alkyl group substituted with an aryl group) is excluded.

**3.** A medicament comprising the compound or a physiologically acceptable salt thereof according to claim 1 or 2 as an active ingredient, and one or more kinds of pharmaceutical additives as required.

**4.** The medicament according to claim 3, which is for inhibiting phosphorylation of myosin regulatory light chain.

**5.** The medicament according to claim 3, which is for inhibiting the Rho/Rho kinase pathway.

**6.** The medicament according to claim 3, which is used for prophylactic and/or therapeutic treatment of glaucoma.

**7.** The medicament according to claim 3, which is used for prophylactic and/or therapeutic treatment of bronchial asthma and/or chronic obstructive pulmonary disease.

**8.** The medicament according to claim 3, which is used for prophylactic and/or therapeutic treatment of a neurological disorder.

**9.** An inhibitor of phosphorylation of myosin regulatory light chain, which comprises the compound or a physiologically acceptable salt thereof according to claim 1 or 2.

**10.** A Rho/Rho kinase pathway inhibitor, which comprises the compound or a physiologically acceptable salt thereof according to claim 1 or 2.

**11.** A medicament comprising a combination of the compound or a physiologically acceptable salt thereof according to claim 1 or 2 and a jointly-used drug.

**12.** A medicament comprising a combination of the compound or a physiologically acceptable salt thereof according to claim 1 or 2 and a drug having an intraocular pressure reducing action and/or an optic nerve protective action.

**13.** A medicament comprising a combination of the compound or a physiologically acceptable salt thereof according to claim 1 or 2 and one or more of a prostaglandin-related agent, a carbonic anhydrase inhibitor, an adrenergic receptor brocker, and an NMDA receptor blocker.

**14.** The medicament according to claim 12 or 13, which is used for prophylactic and/or therapeutic treatment of glaucoma.

**15.** A medicament comprising a combination of the compound or a physiologically acceptable salt thereof according to claim 1 or 2 and a drug having a tracheal dilational action and/or an anti-inflammatory action.

**16.** A medicament comprising a combination of the compound or a physiologically acceptable salt thereof according to claim 1 or 2 and one or more of a β 2-adrenergic receptor stimulant, a xanthine derivative, an anticholinergic agent, a phosphodiesterase inhibitor, a steroid, and a leukotriene receptor antagonist.

**17.** The medicament according to claim 15 or 16, which is used for prophylactic and/or therapeutic treatment of chronic obstructive pulmonary disease.

**18.** The medicament according to claim 15 or 16, which is used for prophylactic and/or therapeutic treatment of bronchial asthma.

**19.** A medicament comprising a combination of the compound or a physiologically acceptable salt thereof according to claim 1 or 2 and a drug having a central nerve regeneration action.

**20.** The medicament according to claim 19, which is used for prophylactic and/or therapeutic treatment of spinal cord injury.

## INTERNATIONAL SEARCH REPORT

International application No.
PCT/JP2005/021522

A. CLASSIFICATION OF SUBJECT MATTER
***C07D471/06***(2006.01), ***A61K31/4375***(2006.01), ***A61K31/444***(2006.01), ***A61K31/4545***(2006.01), ***A61K31/455***(2006.01), ***A61K31/496***(2006.01), ***A61K31/497***(2006.01), ***A61K31/513***(2006.01), ***A61K31/5377***(2006.01), ***A61K45/00***(2006.01),
According to International Patent Classification (IPC) or to both national classification and IPC

B. FIELDS SEARCHED
Minimum documentation searched (classification system followed by classification symbols)
***C07D471/06***(2006.01), ***A61K31/4375***(2006.01), ***A61K31/444***(2006.01), ***A61K31/4545***(2006.01), ***A61K31/455***(2006.01), ***A61K31/496***(2006.01), ***A61K31/497***(2006.01), ***A61K31/513***(2006.01), ***A61K31/5377***(2006.01), ***A61K45/00***(2006.01),

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,X | WO 2004/108724 A1 (Asahi Kasei Fama Kabushiki Kaisha), 16 December, 2004 (16.12.04), & US 2005/096310 A1 | 1-20 |
| A | WO 2004/009555 A1 (Asahi Kasei Fama Kabushiki Kaisha), 29 January, 2004 (29.01.04), & EP 1541559 A1 | 1-20 |
| A | WO 1995/005824 A1 (SMITHKLINE BEECHAM CORP.), 02 March, 1995 (02.03.95), (Family: none) | 1-20 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 December, 2005 (19.12.05) | 27 December, 2005 (27.12.05) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2005/021522

Continuation of A. CLASSIFICATION OF SUBJECT MATTER
(International Patent Classification (IPC))

***A61P1/02***(2006.01), ***A61P1/04***(2006.01), ***A61P1/08***(2006.01), ***A61P1/16***(2006.01), ***A61P1/18***(2006.01), ***A61P3/14***(2006.01), ***A61P5/00***(2006.01), ***A61P7/02***(2006.01), ***A61P7/06***(2006.01), ***A61P7/12***(2006.01), ***A61P9/00***(2006.01), ***A61P9/04***(2006.01), ***A61P9/06***(2006.01), ***A61P9/08***(2006.01), ***A61P9/10***(2006.01), ***A61P9/12***(2006.01), ***A61P9/14***(2006.01), ***A61P11/00***(2006.01), ***A61P11/02***(2006.01), ***A61P11/04***(2006.01), ***A61P11/06***(2006.01), ***A61P11/08***(2006.01), ***A61P11/16***(2006.01), ***A61P13/00***(2006.01), ***A61P13/02***(2006.01), ***A61P13/08***(2006.01), ***A61P13/10***(2006.01), ***A61P13/12***(2006.01), ***A61P15/00***(2006.01), ***A61P15/06***(2006.01), ***A61P15/10***(2006.01), ***A61P17/00***(2006.01), ***A61P17/02***(2006.01), ***A61P17/14***(2006.01), ***A61P19/00***(2006.01), ***A61P19/02***(2006.01), ***A61P19/06***(2006.01), ***A61P19/08***(2006.01), ***A61P19/10***(2006.01), ***A61P25/00***(2006.01), ***A61P25/02***(2006.01), ***A61P25/08***(2006.01), ***A61P25/14***(2006.01), ***A61P25/16***(2006.01), ***A61P25/18***(2006.01), ***A61P25/28***(2006.01), ***A61P25/30***(2006.01), ***A61P27/00***(2006.01), ***A61P27/02***(2006.01), ***A61P27/06***(2006.01), ***A61P27/16***(2006.01), ***A61P29/00***(2006.01), ***A61P31/00***(2006.01), ***A61P31/12***(2006.01), ***A61P31/14***(2006.01), ***A61P31/18***(2006.01), ***A61P31/20***(2006.01), ***A61P35/00***(2006.01), ***A61P35/02***(2006.01), ***A61P35/04***(2006.01), ***A61P37/00***(2006.01), ***A61P37/06***(2006.01), ***A61P37/08***(2006.01), ***A61P41/00***(2006.01), ***A61P43/00***(2006.01)

According to International Patent Classification (IPC) or to both national classification and IPC)

Continuation of B. FIELDS SEARCHED
Minimum documentation searched (International Patent Classification (IPC))

***A61P1/02***(2006.01), ***A61P1/04***(2006.01), ***A61P1/08***(2006.01), ***A61P1/16***(2006.01), ***A61P1/18***(2006.01), ***A61P3/14***(2006.01), ***A61P5/00***(2006.01), ***A61P7/02***(2006.01), ***A61P7/06***(2006.01), ***A61P7/12***(2006.01), ***A61P9/00***(2006.01), ***A61P9/04***(2006.01), ***A61P9/06***(2006.01), ***A61P9/08***(2006.01), ***A61P9/10***(2006.01), ***A61P9/12***(2006.01), ***A61P9/14***(2006.01), ***A61P11/00***(2006.01), ***A61P11/02***(2006.01), ***A61P11/04***(2006.01), ***A61P11/06***(2006.01), ***A61P11/08***(2006.01), ***A61P11/16***(2006.01), ***A61P13/00***(2006.01), ***A61P13/02***(2006.01), ***A61P13/08***(2006.01), ***A61P13/10***(2006.01), ***A61P13/12***(2006.01), ***A61P15/00***(2006.01), ***A61P15/06***(2006.01), ***A61P15/10***(2006.01), ***A61P17/00***(2006.01), ***A61P17/02***(2006.01), ***A61P17/14***(2006.01), ***A61P19/00***(2006.01), ***A61P19/02***(2006.01), ***A61P19/06***(2006.01), ***A61P19/08***(2006.01), ***A61P19/10***(2006.01), ***A61P25/00***(2006.01), ***A61P25/02***(2006.01), ***A61P25/08***(2006.01), ***A61P25/14***(2006.01), ***A61P25/16***(2006.01), ***A61P25/18***(2006.01), ***A61P25/28***(2006.01), ***A61P25/30***(2006.01), ***A61P27/00***(2006.01), ***A61P27/02***(2006.01), ***A61P27/06***(2006.01), ***A61P27/16***(2006.01), ***A61P29/00***(2006.01), ***A61P31/00***(2006.01), ***A61P31/12***(2006.01), ***A61P31/14***(2006.01), ***A61P31/18***(2006.01), ***A61P31/20***(2006.01), ***A61P35/00***(2006.01), ***A61P35/02***(2006.01), ***A61P35/04***(2006.01), ***A61P37/00***(2006.01), ***A61P37/06***(2006.01), ***A61P37/08***(2006.01), ***A61P41/00***(2006.01), ***A61P43/00***(2006.01)

Minimum documentation searched (classification system followed by classification symbols)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004009555 A
- WO 0156988 A
- US 3809714 A
- US 4517199 A
- US 5627209 A
- US 5296504 A
- US 5510383 A
- US 6403649 B
- EP 296879 A
- US 5378703 A
- US 2554816 A
- GB 1398455 A
- US 5354860 A
- US 3953456 A
- US 4515977 A
- US 4252984 A
- JP 60054317 A
- US 5426227 A
- US 4791107 A
- US 255416 A
- JP 60222472 A
- WO 02083175 A
- US 5616599 A
- US 4122193 A
- JP 3258763 A
- WO 9501324 A
- US 5811553 A
- US 5610163 A
- US 3505337 A
- GB 2088877 A
- JP 2000128897 A
- EP 0435811 A
- JP 62081380 A
- JP 52029318 A
- JP 5097825 A
- JP 2003073378 A
- EP 242518 A
- US 4226878 A
- EP 0173516 A
- EP 480717 A
- US 4859692 A
- JP 3066311 A
- WO 9723213 A
- JP 62031707 A
- GB 2225321 A
- US 5116863 A
- JP 7215968 A
- JP 10237070 A
- JP 2024821 A
- JP 3070698 A
- US 6288069 B


### Non-patent literature cited in the description


- **BARANY, K. et al.** *Biochemistry of Smooth Muscle Contraction,* 1996, 21-35
- **BARANY, M. et al.** *Biochemistry of Smooth Muscle Contraction,* 1996, 321-339
- **KAMM, K. et al.** *Annu. Rev. Physiol.,* 1989, vol. 51, 299-313
- **SCHMIDT, J.T. et al.** *J, Neurobiol.,* 2002, vol. 52 (3), 175-188
- **NIGGLI, V.** *FEBS Lett.,* 1999, vol. 445, 69-72
- **KITANI, S. et al.** *Biochem. Biophys. Res. Commun.,* 1992, vol. 183, 48-54
- **ITOH, K. et al.** *Biochim. Biophys. Acta,* 1992, vol. 1136, 52-56
- **MILLS, J.C. et al.** *J. Cell Biol.,* 1998, vol. 140 (3), 627-636
- **SAMLYO, A.P. et al.** *Rev. Physiol. Biochem. Pharmacol.,* 1999, vol. 134, 209-234
- **SHIMOKAWA et al.** *Cardiovasc. Res.,* 1999, vol. 43 (4), 1029-1039
- **SATOH, H. et al.** *Jpn. J. Pharmacol.,* 1999, vol. 79, 211
- **M. SATOH et al.** *the 57th General Meeting of Japan Neurosurgical Society, Collection of Abstracts,* 1998, 153
- **N. ONO et al.** *Pharmacol. Ther.,* 1991, vol. 82 (2-3), 123-131
- **ANDERSSON, KE. et al.** *World J. Vrol.,* 1997, vol. 15, 14-20
- **K. IIDZUKA.** *Allergy,* 1998, vol. 47, 943
- **K. IIDZUKA et al.** *Jpn. J. Respirology Society,* 1999, vol. 37, 196
- **ARAKAWA, Y. et al.** *BIO Clinica,* 2002, vol. 17 (13), 26-28
- **ITOH, K. et al.** *Nat. Med.,* 1999, vol. 5 (2), 221-5

- **KEELY, P. et al.** *Trends Cell Biol.,* 1998, vol. 8 (3), 101-6
- **FUJIMOTO, O. et al.** *Journal of Japanese Society of Internal Medicine,* 1998, vol. 88 (1), 148-58
- **KEANE-MYERS A. et al.** *Curr. Allergy Asthma Rep.,* 2001, vol. 1 (6), 550-557
- **NAKAI, K. et al.** *Blood,* 1997, vol. 90 (10), 3736-42
- **THOMPSON, C.B.** *Science,* 1995, vol. 267, 1456-1462
- **SUZUKI, A. et al.** *Br. J. Pharmacol.,* 1993, vol. 109, 703-712
- **HOWE, P.H. et al.** *Biochem J.,* 1988, vol. 255, 423-429
- **MOBLEY P.L. et al.** *Exp. Cell Res.,* 1994, vol. 214, 55-66
- **BERGE et al.** *J. Pharm. Sci.,* 1977, vol. 66, 1-19
- **GREENE, T.W. ; WUTS, P.G.M.** Protective Groups in Organic Synthesis. John Wiley and Sons Inc,
- **KOCIENSKI, P.J.** Protecting Groups. Georg Thieme Verlag, 1994
- **G.F. HOLLAND ; L.A. COHEN.** *J. Am. Chem. Soc.,* 1958, vol. 80, 3765
- **K. HAMMERSTROM ; W. LUNKENHEIMER ; H. ZAHN.** *Makromol. Chem.,* 1970, vol. 41, 133
- **KUCZNIERZ, R. ; DICKHAUT, J ; LEINERT, H. ; VON DER SAAL, W.** *Synth. Commun.,* 1999, vol. 29, 1617
- **PING CHEN.** *Bioorganic & Medicinal Chemistry Letters,* 2003, vol. 13, 1345
- **DENNY, WILLIAM. et al.** *J. Med. Chem.,* 2002, 740
- **KULKA.** *J. Am. Chem. Soc.,* 1953, vol. 75, 3597
- **KIRCHHOFF, J.H. ; NETHERTON, M.R. ; HILLS, I.D. ; FU, G.C.** *J. Am. Chem. Soc.,* 2002, vol. 124, 13662
- **DAI, C. ; FU, G.C.** *J. Am. Chem. Soc.,* 2001, vol. 123, 2719
- **CARRENO, M.C. ; MAHUGO, R.H-S.J.** *J. Org. Chem.,* 1999, vol. 64, 1387
- **SELVAKUMAR, K. ; ZAPF, A. ; BELLER, M.** *Org. Lett.,* 2002, vol. 4, 3031
- **RAGGON, J.W. ; SNYDER, W.M.** *Organic Process Research & Development,* 2002, vol. 6, 67
- **HUNDERTMARK, T. ; LITTKE, A.F. ; BUCHWALD, S.L. ; FU, G.C.** *Org. Lett.,* 2000, vol. 2, 1729
- **KLAPARS, A. ; HUANG, X. ; BUCHWALD, S.L.** *J. Am. Chem. Soc.,* 2002, vol. 124, 7421
- **BUCHWALD, S.L. ; ZIM, D.** *Org. Lett.,* 2003, vol. 5, 2413
- **TORACCA, KE. ; HUANG, X. ; PARRISH, C.A. ; BUCHWALD, S.L.** *J. Am. Chem. Soc.,* 2001, vol. 123, 10770
- **TSUNODA et al.** *Chemistry Letters,* 1994, 539
- **SEYFERTH et al.** *Chem. Ind.,* 1959, 402
- *J. Amer. Chem. Soc.,* 1962, 361
- *J. Amer. Chem. Soc.,* 1957, 515
- **GREGORY, C, FU et al.** *Angew. Chem. Int. Ed.,* 1999, 2411
- **FUKATA, Y. et al.** *Trends Pharmacol. Sci.,* 2001, vol. 22, 32-39
- **FENG, J. et al.** *J. Biol. Chem.,* 1999, vol. 274, 37385-37390
- **FUKATA, Y. et al.** *Trends in Pharmacological Sciences,* 2001, vol. 22, 32-39
- **MURATA T. et al.** *J. Hepatotol.,* 2001, vol. 35, 474-481
- **OHNAKA, K. et al.** *Biochem. Biophys. Res. Commun.,* 2001, vol. 287, 337-342
- **YUHONG, S. et al.** *Exp. Cell Res.,* 2002, vol. 278, 45-52
- **INOUE, M. et al.** *Nat. Med.,* 2004, vol. 10, 712-718
- **JIKKEN IGAKU.** *Experimental Medicine,* 1999, vol. 17, 7
- **UEKI, J. et al.** *Gendai Iryo,* 2002, vol. 34 (9), 87-92
- *Japanese Journal of Ophthalmology,* 2003, vol. 107 (3
- Vitreoretinal Diseases. Shin Zusetsu Rinsho Ganka Koza. MEDICAL VIEW, 2003, vol. 5
- **YUGE T. et al.** Medical Care Practice of Anesthesiology 6. *Current Situation of Neuropathic Pain, Bunko-do,* 2002
- **UEHATA, M. et al.** *Nature,* 1997, vol. 389, 990-994
- **ITO, K.M. et al.** *Am. J. Physiol.,* 2000, vol. 279, H1786-H1795
- *Cir. Res.,* 2001, vol. 89 (5), 415-21
- **SAWADA N. et al.** *Circulation,* 2000, vol. 101 (17), 2030-3
- **KIRINO et al.** *Brain Res.,* 1982, vol. 239, 57-69
- **XIA Q.G. et al.** *Cardiovasc. Res.,* 2001, vol. 49 (1), 110-7
- **PIERCE S.M. et al.** *Am. J. Physiol. Heart Circ. Physiol.,* 2001, vol. 281 (1), H67-74
- *Jpn. J. Ophthalmol.,* 2001, vol. 45 (4), 359-62
- *Kiso to Rinsho,* 1996, vol. 30, 511-524
- **HENDERSON, W.R. et al.** *Am. J. Respir. Cric. Care Med.,* 2002, vol. 165 (1), 108-116
- **DANIELA, S. et al.** *J. Pharmacol. Exp. Ther.,* 2001, vol. 297 (1), 280-290
- **MIYATA, K. et al.** *J. Pharmacol. Exp. Ther.,* 1991, vol. 259, 815-819
- **MIYATA, K. et al.** *Am. J. Physiol.,* 1998, vol. 274, G827-831

- *Surv. Ophthalmol.,* 1996, vol. 41, S9-S18
- **OSHIMA, Y. et al.** *Gene Ther.,* 2002, vol. 9 (18), 1214-20
- **ITO, S. et al.** *Graefes Arch. Clin. Exp. Ophthalmol.,* vol. 237 (8), 691-6
- **KANEKO S. et al.** *Folia Pharmacol. Japon,* 1989, vol. 93 (2), 55-60
- **NOMURA N. et al.** *Folia Pharmacol. Japon,* 1989, vol. 94 (3), 173
- *J. Uro.,* 1994, vol. 151, 797-800
- *Cancer Res.,* 1995, vol. 55, 3551-3557
- **GRIFFITH, M.M. et al.** *Arthritis Rheumatism,* 1981, vol. 24, 781
- **WOOLEY, P.H. et al.** *J. Exp. Med.,* 1981, vol. 154, 688
- **KOJIMA et al.** *Folia. Pharmacol. Jpn.,* 2001, vol. 118, 123-130
- **SAYER F.T. et al.** *Exp. Neurol.,* 2002, vol. 175 (1), 282-96
- **HENDERSON W.R. et al.** *Am. J. Respir. Crit. Care Med.,* 2002, vol. 165 (1), 108-116
- **GONZALES DE MORAES, VL. et al.** *Br. J. Pharmacol.,* 1998, vol. 123, 631-6
- *J. Immunol.,* 1997, vol. 159, 3961-3967
- **NIEDIRAU, C. et al.** *Gastroenterology,* 1985, vol. 88 (5 Pt 1), 1192-1204
- **OCHIAI T. et al.** *Transplant. Proc.,* 1987, vol. 19, 1284-1286
- **FUCHIGAMI J. et al.** *73rd Meeting of Japanese Pharmacological Society, Collection of Abstracts,* 2000
- *J. Hepatol.,* 2001, vol. 35 (4), 474-481
- *Am. J. Respir. Crit. Care Med.,* 2001, vol. 163 (1), 210-217
- *Allergy,* 2001, vol. 50 (12), 1152-1162
- **MAEKAWA, T. et al.** *Trombos. Diathes. Haemorrh.,* 1974, vol. 60, 363-370
- **YANKNER, B.A. et al.** *Science,* 1990, vol. 250, 279-282
- **OVX MOUSE ; GOLUB, L.M. et al.** *Ann. N.Y. Acad. Sci.,* 1999, vol. 878, 290-310
- **CRUB S. et al.** *Acta Neuropathol.,* 2001, vol. 101 (2), 85-91
- **ORENGO I.F. et al.** *Arch Dermatol.,* 2002, vol. 138 (6), 823-824
- **KI D.W. et al.** *Anticancer Res.,* 2002, vol. 22 (2A), 777-788
- **BENNETT, GJ. et al.** *Pain,* 1988, vol. 33 (1), 87-107
- *AI Report, Cima Science Journal,* 2002
- **HONJO, M. et al.** *Invest. Ophthalmol. Vis. Sci.,* 2001, vol. 42 (1), 137-44
- **HONJO, M. et al.** *Arch. Ophthalmol.,* 2001, vol. 119 (8), 1171-8
- **INOUE, T. et al.** *Current Eye Res.,* 2001, vol. 23 (2), 133-8
- **HAHN et al.** *Proc. Natl. Acad. Sci. USA,* 1998, vol. 85, 6556
- **TAMAKI, Y. et al.** *Surv. Ophthalmol.,* 1997, vol. 42 (1), S52-S63
- **WHEELER, LA. et al.** *Eur. J. Ophthalmol.,* 2001, vol. 11 (2), 403-11
- **WOOD, JP. et al.** *Exp. Eye Res.,* 2003, vol. 76 (4), 505-16
- **TORIU, N. et al.** *Exp. Eye Res.,* 2000, vol. 70 (4), 475-84
- **KIM, TW. et al.** *Korean J. Ophthalmol.,* 2002, vol. 16 (1), 1-7
- **TAMAKI, Y. et al.** *J. Ocul. Pharmacol. Ther.,* 2001, vol. 17 (5), 403-11
- **HARRIS, A. et al.** *J. Ocul. Pharmacol. Ther.,* 1999, vol. 15, 189-197
- **INOUE, T. et al.** *Ophthalmic Res.,* 2003, vol. 35, 351-4
- **KUROSE et al.** *Protein Nucleic Acid Enzyme,* 1997, vol. 42 (3), 316-26
- **RICHARD, MB. et al.** *Annu. Rev. Pharmacol. Toxicol.,* 2001, vol. 41, 661-90
- **NAGUMO, H. et al.** *Am. J. Physiol. Cell Physiol.,* 2000, vol. 278 (1), C57-65
- **IIZUKA, K. et al.** *Eur. J. Pharmacol.,* 2000, vol. 406 (2), 273-9
- **GIZYCKI, ML. et al.** *Thorax,* 2002, vol. 57 (9), 799-803
- **CULPITT, SV. et al.** *Am. J. Respir. Crit. Care Med.,* 2002, vol. 165, 1371-76
- **PROFITA, M. et al.** *Thorax,* 2003, vol. 58 (7), 573-9
- **MASAKAZU ICHINOSE.** Respiratory Disease New Approach 8. *Obstructive Pulmonary Disease,* 2003, 68-76
- **BANHOLZER, VR. et al.** *Arzneimittelforschung,* 1985, vol. 35 (1A), 217-28
- **GRISWOLD, DE et al.** *J. Pharmacol. Exp. Ther.,* 1998, vol. 287 (2), 705-11
- **BUNDSCHUH, DS. et al.** *J. Pharmacol. Exp. Ther.,* 2001, vol. 297 (1), 280-90
- **KAWABATA, K. et al.** *Biochem. Biophys. Res. Commun.,* 1991, vol. 177 (2), 814-20
- **BAICI, A. et al.** *Biochem. Pharmacol.,* 1990, vol. 39 (5), 919-24
- **OGURA T. et al.** *Key Word 1994-'95, Sentan Igaku-Sha,* 1994
- **IZUMI, T. et al.** *Biochem.,* 2002, vol. 132, 1-6
- **TANAKA, T. et al.** *J. Cell Biol.,* 2002, vol. 158 (2), 321-329
- **KISHINO, A. et al.** *Exp. Neurol.,* 1997, vol. 144 (2), 273-86
- **RABCHEVSKY, AG. et al.** *J. Neurotrauma.,* 1999, vol. 16 (9), 817-30
- **NAKAMURA M. et al.** *Journal of Spine and Spinal Cord,* 2003, vol. 16 (4), 284-290
- **HOLLENBAUGH, DIAN L. et al.** *Bioorg. Med. Chem. Lett.,* 2003, vol. 13, 1345

- **TORACCA, K.E. ; HUANG, X. ; PARRISH, C.A. ; BUCHWALD, S.L.** *J. Am. Chem. Soc.,* 2001, vol. 123, 10770
- **DENNY, WILLIAM et al.** *J. Med. Chem.,* 2002, 740
- **DENNY ; WILLIAM et al.** *J. Med. Chem.,* 2002, 740
- **FERRINO, S.A. ; MALDONADO, L.A.** *Synth. Commun.,* 1984, vol. 14, 925
- **T. YAMADA ; T. MUKAIYAMA.** *Chem. Lett.,* 1989, 515
- **SAKURADA K. et al.** *Am. J. Physiol.,* 1998, vol. 274, C1563-C1572
- **ASANO,T. et al.** *J. Pharmacol. Exp. Ther.,* 1987, vol. 241, 1033-1040
- **NEUMAN, H.R. et al.** *J. Neurosci.,* 2002, vol. 22, 854-862
- **TANAKA, H. et al.** *J. Cell Biol.,* 2002, vol. 158 (2), 321-329
- **YOSHIDA, M. et al.** *Biochem.,* 1986, vol. 99, 1027-1036
- **GRAND, R. J. et al.** *Biochem. J.,* 1983, vol. 211, 267-272
- **SAKURADA, K. et al.** *J. Biochem.,* 1994, vol. 115, 18-21
- **SAKURADA, K. et al.** *Am. J. Physiol.,* 1998, vol. 274, C1563-C1572